(19) Europäisches Patentamt
European Patent Office
Office européen des brevets

(11) EP 2 468 865 B1

(12) **EUROPEAN PATENT SPECIFICATION**

(45) Date of publication and mention of the grant of the patent:
**03.09.2014 Bulletin 2014/36**

(51) Int Cl.:
***C12N 15/113*** *(2010.01)* ***A61K 31/712*** *(2006.01)*

(21) Application number: **12156572.5**

(22) Date of filing: **30.09.2004**

(54) **Antisense oligonucleotide modulation of stat3 expression**

Antisense-Oligonukleotid-Modulation einer STAT3-Expression

Modulation d'oligonucléotides antisens de l'expression stat3

(84) Designated Contracting States:
**AT BE BG CH CY CZ DE DK EE ES FI FR GB GR HU IE IT LI LU MC NL PL PT RO SE SI SK TR**

(30) Priority: **06.02.2004 US 773678**

(43) Date of publication of application:
**27.06.2012 Bulletin 2012/26**

(60) Divisional application:
**12175203.4 / 2 527 444**

(62) Document number(s) of the earlier application(s) in accordance with Art. 76 EPC:
**04789332.6 / 1 718 762**

(73) Proprietor: **Isis Pharmaceuticals, Inc.**
**Carlsbad, CA 92010 (US)**

(72) Inventor: **Karras, James G.**
**San Marcos, CA 92069 (US)**

(74) Representative: **Gainey, Laurence David Scott**
**AstraZeneca PLC**
**Global Intellectual Property**
**Mereside**
**Alderley Park**
**Macclesfield, Cheshire SK10 4TG (GB)**

(56) References cited:
**WO-A1-00/61602**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 468 865 B1

SEQUENCE LISTING

<110> Isis Pharmaceuticals, Inc.

<120> Antisense Oligonucleotide Modulation of STAT3 Expression

<130> ISPH-0883

<140>
<141>

<150> 10/773,678
<151> 2004-02-06

<150> 10/713,139
<151> 2003-11-14

<150> 09/758,881
<151> 2001-01-11

<150> PCT/US00/09054
<151> 2000-04-06

<150> 09/288,461
<151> 1999-04-08

<160> 511

<170> PatentIn Ver. 2.1 and FastSeq Ver. 4.0

<210> 1
<211> 2787
<212> DNA
<213> Homo sapiens

<400> 1

```
cagctggaat tcggggcggc ggcgcagact gggaggggga gccgggggtt ccgacgtcgc 60
agccgaggga acaagcccca accggatcct ggacaggcac cccggcttgg cgctgtctct 120
ccccctcggc tcggagaggc ccttcggcct gagggagcct cgccgcccgt ccccggcaca 180
cgcgcagccc cggcctctcg gcctctgccg gagaaacagg atggcccaat ggaatcagct 240
acagcagctt gacacacggt acctggagca gctccatcag ctctacagtg acagcttccc 300
aatggagctg cggcagtttc tggccccttg gattgagagt caagattggg catatgcggc 360
cagcaaagaa tcacatgcca ctttggtgtt tcataatctc ctgggagaga ttgaccagca 420
gtatagccgc ttcctgcaag agtcgaatgt tctctatcag cacaatctac gaagaatcaa 480
gcagtttctt cagagcaggt atcttgagaa gccaatggag attgcccgga ttgtggcccg 540
gtgcctgtgg gaagaatcac gccttctaca gactgcagcc actgcggccc agcaaggggg 600
ccaggccaac caccccacag cagccgtggt gacggagaag cagcagatgc tggagcagca 660
ccttcaggat gtccggaaga gagtgcagga tctagaacag aaaatgaaag tggtagagaa 720
tctccaggat gactttgatt tcaactataa aaccctcaag agtcaaggag acatgcaaga 780
tctgaatgga aacaaccagt cagtgaccag gcagaagatg cagcagctgg aacagatgct 840
cactgcgctg gaccagatgc ggagaagcat cgtgagtgag ctggcggggc ttttgtcagc 900
gatggagtac gtgcagaaaa ctctcacgga cgaggagctg gctgactgga agaggcggca 960
acagattgcc tgcattggag gcccgcccaa catctgccta gatcggctag aaaactggat 1020
aacgtcatta gcagaatctc aacttcagac ccgtcaacaa attaagaaac tggaggagtt 1080
gcaccaaaaa gtttcctaca aaggggaccc cattgtacag caccggccga tgctggagga 1140
gaggatcgtg gagctgttca gaaacttaat gaaaagtgcc tttgtggtgg agcggcagcc 1200
ctgcatgccc atgcatcctg accggcccct cgtcatcaag accggcgtcc agttcactac 1260
taaagtcagg ttgctggtca agttccctga gttgaattat cagcttaaaa ttaaagtgtg 1320
cattgacaaa gactctgggg acgttgcagc tctcagagga tcccggaaat ttaacattct 1380
gggcacaaac acaaaagtga tgaacatgga agaatccaac aacggcagcc tctctgcaga 1440
attcaaacac ttgaccctga gggagcagag atgtgggaat gggggccgag ccaattgtga 1500
tgcttccctg attgtgactg aggagctgca cctgatcacc tttgagaccg aggtgtatca 1560
ccaaggtctc aagattgacc tagagaccca ctccttgtca gttgtggtga tctccaacat 1620
ctgtcagatg ccaaatgcct gggcgtccat cctgtggtac aacatgctga ccaacaatcc 1680
caagaatgtg aacttcttca ctaagccgcc aattggaacc tgggaccaag tggccgaggt 1740

gctcagctgg cagttctcgt ccaccaccaa gcgggggctg agcatcgagc agctgacaac 1800
gctggctgag aagctcctag ggcctggtgt gaactactca gggtgtcaga tcacatgggc 1860
taacttctgc aaagaaaaca tggctggcaa gggcttctcc tactgggtct ggctagacaa 1920
tatcatcgac cttgtgaaaa agtatatctt ggccctttgg aatgaagggt acatcatggg 1980
tttcatcagc aaggagcggg agcgggccat cttgagcact aagcccccag gcaccttcct 2040
gctgcgcttc agtgaaagca gcaaagaagg aggcgtcact ttcacttggg tggagaagga 2100
catcagcggt aagacccaga tccagtccgt ggaaccatac acaaagcagc agctgaacaa 2160
catgtcattt gctgaaatca tcatgggcta taagatcatg gatgctacca atatcctgtt 2220
gtctccactt gtctatctct atcctgacat tcccaaggag gaggcattcg ggaagtattg 2280
tcggccagag agccaggagc atcctgaagc tgacccaggt agcgctgccc catacctgaa 2340
gaccaagttt atctgtgtga caccaacgac ctgcagcaat accattgacc tgccgatgtc 2400
cccccgcgct ttagattcat tgatgcagtt tggaaataat ggtgaaggtg ctgaaccctc 2460
agcaggaggg cagtttgagt ccctcacctt tgacatggag ttgacctcgg agtgcgctac 2520
ctcccccatg tgaggagctg agaacggaag ctgcagaaag atacgactga ggcgcctacc 2580
tgcattctgc cacccctcac acagccaaac cccagatcat ctgaaactac taactttgtg 2640
gttccagatt ttttttaatc tcctacttct gctatctttg agcaatctgg gcacttttaa 2700
aaatagagaa atgagtgaat gtgggtgatc tgcttttatc taaatgcaaa taaggatgtg 2760
ttctctgaga cccatgatca ggggatg                                     2787
```

<210> 2
<211> 20
<212> DNA
<213> Artificial Sequence

<220>
<223> Antisense Oligonucleotide

<400> 2
gtctgcgccg ccgccccgaa 20

<210> 3
<211> 20
<212> DNA
<213> Artificial Sequence

<220>
<223> Antisense Oligonucleotide

<400> 3
ggccgaaggg cctctccgag 20

<210> 4
<211> 20
<212> DNA
<213> Artificial Sequence

<220>
<223> Antisense Oligonucleotide

<400> 4
tcctgtttct ccggcagagg 20

<210> 5
<211> 20
<212> DNA
<213> Artificial Sequence

<220>
<223> Antisense Oligonucleotide

<400> 5

<210> 6
<211> 20
<212> DNA
<213> Artificial Sequence

<220>
<223> Antisense Oligonucleotide

<400> 6
gccatcctgt ttctccggca 20

<210> 7
<211> 20
<212> DNA
<213> Artificial Sequence

<220>
<223> Antisense Oligonucleotide

<400> 7
gggccatcct gtttctccgg 20

<210> 8
<211> 20
<212> DNA
<213> Artificial Sequence

<220>
<223> Antisense Oligonucleotide

<400> 8
ttgggccatc ctgtttctcc 20

<210> 9
<211> 20
<212> DNA
<213> Artificial Sequence

<220>
<223> Antisense Oligonucleotide

<400> 9
cattgggcca tcctgtttct 20

<210> 10
<211> 20
<212> DNA
<213> Artificial Sequence

<220>
<223> Antisense Oligonucleotide

<400> 10
tccattgggc catcctgttt 20

<210> 11
<211> 20
<212> DNA
<213> Artificial Sequence

<220>
<223> Antisense Oligonucleotide

<400> 11
attccattgg gccatcctgt 20

<210> 12
<211> 20
<212> DNA
<213> Artificial Sequence

<220>
<223> Antisense Oligonucleotide

<400> 12
tgattccatt gggccatcct 20

<210> 13
<211> 20
<212> DNA
<213> Artificial Sequence

<220>
<223> Antisense Oligonucleotide

<400> 13
gctgattcca ttgggccatc 20

<210> 14
<211> 20
<212> DNA
<213> Artificial Sequence

<220>
<223> Antisense Oligonucleotide

<400> 14
tagctgattc cattgggcca 20

<210> 15
<211> 20
<212> DNA
<213> Artificial Sequence

<220>
<223> Antisense Oligonucleotide

<400> 15
tgtagctgat tccattgggc 20

<210> 16
<211> 20
<212> DNA
<213> Artificial Sequence

<220>
<223> Antisense Oligonucleotide

<400> 16
ctgtagagct gatggagctg 20

<210> 17
<211> 20
<212> DNA
<213> Artificial Sequence

<220>
<223> Antisense Oligonucleotide

<400> 17
cccaatcttg actctcaatc 20

<210> 18
<211> 20
<212> DNA
<213> Artificial Sequence

<220>
<223> Antisense Oligonucleotide

<400> 18
cccaggagat tatgaaacac 20

<210> 19
<211> 20
<212> DNA
<213> Artificial Sequence

<220>
<223> Antisense Oligonucleotide

<400> 19
acattcgact cttgcaggaa 20

<210> 20
<211> 20
<212> DNA
<213> Artificial Sequence

<220>
<223> Antisense Oligonucleotide

<400> 20
tctgaagaaa ctgcttgatt 20

<210> 21
<211> 20
<212> DNA
<213> Artificial Sequence

<220>
<223> Antisense Oligonucleotide

<400> 21
ggccacaatc cgggcaatct 20

<210> 22
<211> 20
<212> DNA
<213> Artificial Sequence

<220>
<223> Antisense Oligonucleotide

<400> 22
tggctgcagt ctgtagaagg 20

<210> 23
<211> 20
<212> DNA
<213> Artificial Sequence

<220>
<223> Antisense Oligonucleotide

<400> 23
ctgctccagc atctgctgct 20

<210> 24
<211> 20
<212> DNA

<213> Artificial Sequence

<220>
<223> Antisense Oligonucleotide

<400> 24
tttctgttct agatcctgca 20

<210> 25
<211> 20
<212> DNA
<213> Artificial Sequence

<220>
<223> Antisense Oligonucleotide

<400> 25
tagttgaaat caaagtcatc 20

<210> 26
<211> 20
<212> DNA
<213> Artificial Sequence

<220>
<223> Antisense Oligonucleotide

<400> 26
ttccattcag atcttgcatg 20

<210> 27
<211> 20
<212> DNA
<213> Artificial Sequence

<220>
<223> Antisense Oligonucleotide

<400> 27
tctgttccag ctgctgcatc 20

<210> 28
<211> 20
<212> DNA
<213> Artificial Sequence

<220>
<223> Antisense Oligonucleotide

<400> 28
tcactcacga tgcttctccg 20

<210> 29
<211> 20
<212> DNA
<213> Artificial Sequence

<220>

<223> Antisense Oligonucleotide

<400> 29
gagttttctg cacgtactcc 20

<210> 30
<211> 20
<212> DNA
<213> Artificial Sequence

<220>
<223> Antisense Oligonucleotide

<400> 30
atctgttgcc gcctcttcca 20

<210> 31
<211> 20
<212> DNA
<213> Artificial Sequence

<220>
<223> Antisense Oligonucleotide

<400> 31
ctagccgatc taggcagatg 20

<210> 32
<211> 20
<212> DNA
<213> Artificial Sequence

<220>
<223> Antisense Oligonucleotide

<400> 32
cgggtctgaa gttgagattc 20

<210> 33
<211> 20
<212> DNA
<213> Artificial Sequence

<220>
<223> Antisense Oligonucleotide

<400> 33
cggccggtgc tgtacaatgg 20

<210> 34
<211> 20
<212> DNA
<213> Artificial Sequence

<220>
<223> Antisense Oligonucleotide

<400> 34

tttcattaag tttctgaaca 20

<210> 35
<211> 20
<212> DNA
<213> Artificial Sequence

<220>
<223> Antisense Oligonucleotide

<400> 35
aggatgcatg ggcatgcagg 20

<210> 36
<211> 20
<212> DNA
<213> Artificial Sequence

<220>
<223> Antisense Oligonucleotide

<400> 36
gaccagcaac ctgactttag 20

<210> 37
<211> 20
<212> DNA
<213> Artificial Sequence

<220>
<223> Antisense Oligonucleotide

<400> 37
atgcacactt taattttaag 20

<210> 38
<211> 20
<212> DNA
<213> Artificial Sequence

<220>
<223> Antisense Oligonucleotide

<400> 38
ttccgggatc ctctgagagc 20

<210> 39
<211> 20
<212> DNA
<213> Artificial Sequence

<220>
<223> Antisense Oligonucleotide

<400> 39
ttccatgttc atcacttttg 20

<210> 40

<211> 20
<212> DNA
<213> Artificial Sequence

<220>
<223> Antisense Oligonucleotide

<400> 40
gtcaagtgtt tgaattctgc 20

<210> 41
<211> 20
<212> DNA
<213> Artificial Sequence

<220>
<223> Antisense Oligonucleotide

<400> 41
caatcaggga agcatcacaa 20

<210> 42
<211> 20
<212> DNA
<213> Artificial Sequence

<220>
<223> Antisense Oligonucleotide

<400> 42
tacacctcgg tctcaaaggt 20

<210> 43
<211> 20
<212> DNA
<213> Artificial Sequence

<220>
<223> Antisense Oligonucleotide

<400> 43
tgacaaggag tgggtctcta 20

<210> 44
<211> 20
<212> DNA
<213> Artificial Sequence

<220>
<223> Antisense Oligonucleotide

<400> 44
cgcccaggca tttggcatct 20

<210> 45
<211> 20
<212> DNA
<213> Artificial Sequence

<220>
<223> Antisense Oligonucleotide

<400> 45
cattcttggg attgttggtc 20

<210> 46
<211> 20
<212> DNA
<213> Artificial Sequence

<220>
<223> Antisense Oligonucleotide

<400> 46
cacttggtcc caggttccaa 20

<210> 47
<211> 20
<212> DNA
<213> Artificial Sequence

<220>
<223> Antisense Oligonucleotide

<400> 47
cccgcttggt ggtggacgag 20

<210> 48
<211> 20
<212> DNA
<213> Artificial Sequence

<220>
<223> Antisense Oligonucleotide

<400> 48
agttcacacc aggccctagg 20

<210> 49
<211> 20
<212> DNA
<213> Artificial Sequence

<220>
<223> Antisense Oligonucleotide

<400> 49
gttttctttg cagaagttag 20

<210> 50
<211> 20
<212> DNA
<213> Artificial Sequence

<220>
<223> Antisense Oligonucleotide

<400> 50
atattgtcta gccagaccca 20

<210> 51
<211> 20
<212> DNA
<213> Artificial Sequence

<220>
<223> Antisense Oligonucleotide

<400> 51
aacccatgat gtacccttca 20

<210> 52
<211> 20
<212> DNA
<213> Artificial Sequence

<220>
<223> Antisense Oligonucleotide

<400> 52
gcttagtgct caagatggcc 20

<210> 53
<211> 20
<212> DNA
<213> Artificial Sequence

<220>
<223> Antisense Oligonucleotide

<400> 53
gctgctttca ctgaagcgca 20

<210> 54
<211> 20
<212> DNA
<213> Artificial Sequence

<220>
<223> Antisense Oligonucleotide

<400> 54
gtgaaagtga cgcctccttc 20

<210> 55
<211> 20
<212> DNA
<213> Artificial Sequence

<220>
<223> Antisense Oligonucleotide

<400> 55
ctgatgtcct tctccaccca 20

<210> 56
<211> 20
<212> DNA
<213> Artificial Sequence

<220>
<223> Antisense Oligonucleotide

<400> 56
actggatctg ggtcttaccg 20

<210> 57
<211> 20
<212> DNA
<213> Artificial Sequence

<220>
<223> Antisense Oligonucleotide

<400> 57
aaatgacatg ttgttcagct 20

<210> 58
<211> 20
<212> DNA
<213> Artificial Sequence

<220>
<223> Antisense Oligonucleotide

<400> 58
gcccatgatg atttcagcaa 20

<210> 59
<211> 20
<212> DNA
<213> Artificial Sequence

<220>
<223> Antisense Oligonucleotide

<400> 59
tattggtagc atccatgatc 20

<210> 60
<211> 20
<212> DNA
<213> Artificial Sequence

<220>
<223> Antisense Oligonucleotide

<400> 60
atagacaagt ggagacaaca 20

<210> 61
<211> 20
<212> DNA

<213> Artificial Sequence

<220>
<223> Antisense Oligonucleotide

<400> 61
ttgggaatgt caggatagag 20

<210> 62
<211> 20
<212> DNA
<213> Artificial Sequence

<220>
<223> Antisense Oligonucleotide

<400> 62
ctcctggctc tctggccgac 20

<210> 63
<211> 20
<212> DNA
<213> Artificial Sequence

<220>
<223> Antisense Oligonucleotide

<400> 63
acctgggtca gcttcaggat 20

<210> 64
<211> 20
<212> DNA
<213> Artificial Sequence

<220>
<223> Antisense Oligonucleotide

<400> 64
cacagataaa cttggtcttc 20

<210> 65
<211> 20
<212> DNA
<213> Artificial Sequence

<220>
<223> Antisense Oligonucleotide

<400> 65
atcggcaggt caatggtatt 20

<210> 66
<211> 20
<212> DNA
<213> Artificial Sequence

<220>

<223> Antisense Oligonucleotide

<400> 66
ccaaactgca tcaatgaatc 20

<210> 67
<211> 20
<212> DNA
<213> Artificial Sequence

<220>
<223> Antisense Oligonucleotide

<400> 67
ggttcagcac cttcaccatt 20

<210> 68
<211> 20
<212> DNA
<213> Artificial Sequence

<220>
<223> Antisense Oligonucleotide

<400> 68
gagggactca aactgccctc 20

<210> 69
<211> 20
<212> DNA
<213> Artificial Sequence

<220>
<223> Antisense Oligonucleotide

<400> 69
caactccatg tcaaaggtga 20

<210> 70
<211> 20
<212> DNA
<213> Artificial Sequence

<220>
<223> Antisense Oligonucleotide

<400> 70
ttctcagctc ctcacatggg 20

<210> 71
<211> 20
<212> DNA
<213> Artificial Sequence

<220>
<223> Antisense Oligonucleotide

<400> 71

cgttctcagc tcctcacatg 20

<210> 72
<211> 20
<212> DNA
<213> Artificial Sequence

<220>
<223> Antisense Oligonucleotide

<400> 72
tccgttctca gctcctcaca 20

<210> 73
<211> 20
<212> DNA
<213> Artificial Sequence

<220>
<223> Antisense Oligonucleotide

<400> 73
cttccgttct cagctcctca 20

<210> 74
<211> 20
<212> DNA
<213> Artificial Sequence

<220>
<223> Antisense Oligonucleotide

<400> 74
agcttccgtt ctcagctcct 20

<210> 75
<211> 20
<212> DNA
<213> Artificial Sequence

<220>
<223> Antisense Oligonucleotide

<400> 75
agaatgcagg taggcgcctc 20

<210> 76
<211> 20
<212> DNA
<213> Artificial Sequence

<220>
<223> Antisense Oligonucleotide

<400> 76
accacaaagt tagtagtttc 20

<210> 77

<211> 20
<212> DNA
<213> Artificial Sequence

<220>
<223> Antisense Oligonucleotide

<400> 77
tgctcaaaga tagcagaagt 20

<210> 78
<211> 20
<212> DNA
<213> Artificial Sequence

<220>
<223> Antisense Oligonucleotide

<400> 78
attcactcat ttctctattt 20

<210> 79
<211> 20
<212> DNA
<213> Artificial Sequence

<220>
<223> Antisense Oligonucleotide

<400> 79
catttagata aaagcagatc 20

<210> 80
<211> 20
<212> DNA
<213> Artificial Sequence

<220>
<223> Antisense Oligonucleotide

<400> 80
acatccttat ttgcatttag 20

<210> 81
<211> 20
<212> DNA
<213> Artificial Sequence

<220>
<223> Antisense Oligonucleotide

<400> 81
gatcatgggt ctcagagaac 20

<210> 82
<211> 2869
<212> DNA
<213> Mus musculus

<400> 82

```
gccgcgacca gccaggccgg ccagtcgggc tcagcccgga gacagtcgag acccctgact 60
gcagcaggat ggctcagtgg aaccagctgc agcagctgga cacacgctac ctgaagcagc 120
tgcaccagct gtacagcgac acgttcccca tggagctgcg gcagttcctg gcaccttgga 180
ttgagagtca agactgggca tatgcagcca gcaaagagtc acatgccacg ttggtgtttc 240
ataatctctt gggtgaaatt gaccagcaat atagccgatt cctgcaagag tccaatgtcc 300
tctatcagca caaccttcga agaatcaagc agtttctgca gagcaggtat cttgagaagc 360
caatggaaat tgcccggatc gtggcccgat gcctgtggga agagtctcgc ctcctccaga 420
cggcagccac ggcagcccag caagggggcc aggccaacca cccaacagcc gccgtagtga 480
cagagaagca gcagatgttg gagcagcatc ttcaggatgt ccggaagcga gtgcaggatc 540
tagaacagaa aatgaaggtg gtggagaacc tccaggacga ctttgatttc aactacaaaa 600
ccctcaagag ccaaggagac atgcaggatc tgaatggaaa caaccagtct gtgaccagac 660
agaagatgca gcagctggaa cagatgctca cagccctgga ccagatgcgg agaagcattg 720
tgagtgagct ggcggggctc ttgtcagcaa tggagtacgt gcagaagaca ctgactgatg 780
aagagctggc tgactggaag aggcggcagc agatcgcgtg catcggaggc cctcccaaca 840
tctgcctgga ccgtctggaa aactggataa cttcattagc agaatctcaa cttcagaccc 900
gccaacaaat taagaaactg gaggagctgc agcagaaagt gtcctacaag ggcgacccta 960
tcgtgcagca ccggcccatg ctggaggaga ggatcgtgga gctgttcaga aacttaatga 1020
agagtgcctt cgtggtggag cggcagccct gcatgcccat gcacccggac cggcccttag 1080
tcatcaagac tggtgtccag tttaccacga aagtcaggtt gctggtcaaa tttcctgagt 1140
tgaattatca gcttaaaatt aaagtgtgca ttgataaaga ctctggggat gttgctgccc 1200
tcagagggtc tcggaaattt aacattctgg gcacgaacac aaaagtgatg aacatggagg 1260
agtctaacaa cggcagcctg tctgcagagt tcaagcacct gacccttagg gagcagagat 1320
gtgggaatgg aggccgtgcc aattgtgatg cctccttgat cgtgactgag gagctgcacc 1380
tgatcacctt cgagactgag gtgtaccacc aaggcctcaa gattgaccta gagacccact 1440
ccttgccagt tgtggtgatc tccaacatct gtcagatgcc aaatgcttgg gcatcaatcc 1500
tgtggtataa catgctgacc aataacccca agaacgtgaa cttcttcact aagccgccaa 1560
ttggaacctg ggaccaagtg gccgaggtgc tcagctggca gttctcgtcc accaccaagc 1620
gagggctgag catcgagcag ctgacaacgc tggctgagaa gctcctaggg cctggtgtga 1680
actactcagg gtgtcagatc acatgggcta aattctgcaa agaaaacatg gctggcaagg 1740
gcttctcctt ctgggtctgg ctagacaata tcatcgacct tgtgaaaaag tatatcttgg 1800
ccctttggaa tgaagggtac atcatgggtt tcatcagcaa ggagcgggag cggccatcc 1860
taagcacaaa gcccccgggc accttcctac tgcgcttcag cgagagcagc aaagaaggag 1920
gggtcacttt cacttgggtg gaaaaggaca tcagtggcaa gacccagatc cagtctgtag 1980
agccatacac caagcagcag ctgaacaaca tgtcatttgc tgaaatcatc atgggctata 2040
agatcatgga tgcgaccaac atcctggtgt ctccacttgt ctacctctac cccgacattc 2100
ccaaggagga ggcatttgga aagtactgta ggcccgagag ccaggagcac cccgaagccg 2160
acccaggtag tgctgccccg tacctgaaga ccaagttcat ctgtgtgaca ccaacgacct 2220
gcagcaatac cattgacctg ccgatgtccc cccgcacttt agattcattg atgcagtttg 2280
gaaataacgg tgaaggtgct gagccctcag caggagggca gtttgagtcg ctcacgtttg 2340
acatggatct gacctcggag tgtgctacct cccccatgtg aggagctgaa accagaagct 2400
gcagagacgt gacttgagac acctgccccg tgctccaccc ctaagcagcc gaaccccata 2460
tcgtctgaaa ctcctaactt tgtggttcca gatttttttt tttaatttcc tacttctgct 2520
atctttgggc aatctgggca ctttttaaaa gagagaaatg agtgagtgtg ggtgataaac 2580
tgttatgtaa agaggagaga cctctgagtc tggggatggg gctgagagca gaagggaggc 2640
aaaggggaac acctcctgtc ctgcccgcct gccctccttt ttcagcagct cgggggttgg 2700
ttgttagaca agtgcctcct ggtgcccatg gctacctgtt gccccactct gtgagctgat 2760
accccattct gggaactcct ggctctgcac tttcaacctt gctaatatcc acatagaagc 2820
taggactaag cccaggaggt tcctctttaa attaaaaaaa aaaaaaaaa            2869
```

<210> 83
<211> 20
<212> DNA
<213> Artificial Sequence

<220>
<223> Antisense Oligonucleotide

<400> 83
gttccactga gccatcctgc 20

<210> 84
<211> 20
<212> DNA
<213> Artificial Sequence

<220>
<223> Antisense Oligonucleotide

<400> 84
ttcaggtagc gtgtgtccag 20

<210> 85
<211> 20
<212> DNA
<213> Artificial Sequence

<220>
<223> Antisense Oligonucleotide

<400> 85
atgtgactct ttgctggctg 20

<210> 86
<211> 20
<212> DNA
<213> Artificial Sequence

<220>
<223> Antisense Oligonucleotide

<400> 86
ccaagagatt atgaaacacc 20

<210> 87
<211> 20
<212> DNA
<213> Artificial Sequence

<220>
<223> Antisense Oligonucleotide

<400> 87
gctccaacat ctgctgcttc 20

<210> 88
<211> 20
<212> DNA
<213> Artificial Sequence

<220>
<223> Antisense Oligonucleotide

<400> 88
gctcttcatc agtcagtgtc 20

<210> 89
<211> 20
<212> DNA

<213> Artificial Sequence

<220>
<223> Antisense Oligonucleotide

<400> 89
atctgacacc ctgagtagtt 20

<210> 90
<211> 20
<212> DNA
<213> Artificial Sequence

<220>
<223> Antisense Oligonucleotide

<400> 90
gccagaccca gaaggagaag 20

<210> 91
<211> 20
<212> DNA
<213> Artificial Sequence

<220>
<223> Antisense Oligonucleotide

<400> 91
cgctccttgc tgatgaaacc 20

<210> 92
<211> 20
<212> DNA
<213> Artificial Sequence

<220>
<223> Antisense Oligonucleotide

<400> 92
aacttggtct tcaggtacgg 20

<210> 93
<211> 20
<212> DNA
<213> Artificial Sequence

<220>
<223> Antisense Oligonucleotide

<400> 93
atcaatgaat ctaaagtgcg 20

<210> 94
<211> 20
<212> DNA
<213> Artificial Sequence

<220>

<223> Antisense Oligonucleotide

<400> 94
tcagcacctt caccgttatt 20

<210> 95
<211> 20
<212> DNA
<213> Artificial Sequence

<220>
<223> Antisense Oligonucleotide

<400> 95
actcaaactg ccctcctgct 20

<210> 96
<211> 20
<212> DNA
<213> Artificial Sequence

<220>
<223> Antisense Oligonucleotide

<400> 96
ggtttcagct cctcacatgg 20

<210> 97
<211> 20
<212> DNA
<213> Artificial Sequence

<220>
<223> Antisense Oligonucleotide

<400> 97
taaaaaaaaa aatctggaac 20

<210> 98
<211> 20
<212> DNA
<213> Artificial Sequence

<220>
<223> Antisense Oligonucleotide

<400> 98
aagatagcag aagtaggaaa 20

<210> 99
<211> 20
<212> DNA
<213> Artificial Sequence

<220>
<223> Antisense Oligonucleotide

<400> 99

aaaaagtgcc cagattgccc 20

<210> 100
<211> 20
<212> DNA
<213> Artificial Sequence

<220>
<223> Antisense Oligonucleotide

<400> 100
atcacccaca ctcactcatt 20

<210> 101
<211> 20
<212> DNA
<213> Artificial Sequence

<220>
<223> Antisense Oligonucleotide

<400> 101
cctttgcctc ccttctgctc 20

<210> 102
<211> 20
<212> DNA
<213> Artificial Sequence

<220>
<223> Antisense Oligonucleotide

<400> 102
tgaaaaagga gggcaggcgg 20

<210> 103
<211> 20
<212> DNA
<213> Artificial Sequence

<220>
<223> Antisense Oligonucleotide

<400> 103
caccaggagg cacttgtcta 20

<210> 104
<211> 20
<212> DNA
<213> Artificial Sequence

<220>
<223> Antisense Oligonucleotide

<400> 104
aacctcctgg gcttagtcct 20

<210> 105

<211> 20
<212> DNA
<213> Artificial Sequence

<220>
<223> Antisense Oligonucleotide

<400> 105
aaaaagtgcg cagattgccc 20

<210> 106
<211> 20
<212> DNA
<213> Artificial Sequence

<220>
<223> Antisense Oligonucleotide

<400> 106
aaaaagtccg ctgattgccc 20

<210> 107
<211> 20
<212> DNA
<213> Artificial Sequence

<220>
<223> Antisense Oligonucleotide

<400> 107
aaaaactccg ctgaatgccc 20

<210> 108
<211> 20
<212> DNA
<213> Artificial Sequence

<220>
<223> Antisense Oligonucleotide

<400> 108
atgtgattct ttgctggccg 20

<210> 109
<211> 20
<212> DNA
<213> Artificial Sequence

<220>
<223> Antisense Oligonucleotide

<400> 109
agctgattcc attgggccat 20

<210> 110
<211> 20
<212> DNA
<213> Artificial Sequence

<220>
<223> Antisense Oligonucleotide

<400> 110
ccaggagatt atgaaacacc 20

<210> 111
<211> 20
<212> DNA
<213> Artificial Sequence

<220>
<223> Antisense Oligonucleotide

<400> 111
accgtgtgtc aagctgctgt 20

<210> 112
<211> 20
<212> DNA
<213> Artificial Sequence

<220>
<223> Antisense Oligonucleotide

<400> 112
ccattgggaa gctgtcactg 20

<210> 113
<211> 20
<212> DNA
<213> Artificial Sequence

<220>
<223> Antisense Oligonucleotide

<400> 113
tgtgattctt tgctggccgc 20

<210> 114
<211> 20
<212> DNA
<213> Artificial Sequence

<220>
<223> Antisense Oligonucleotide

<400> 114
gcggctatac tgctggtcaa 20

<210> 115
<211> 20
<212> DNA
<213> Artificial Sequence

<220>
<223> Antisense Oligonucleotide

<400> 115
gctccagcat ctgctgcttc 20

<210> 116
<211> 20
<212> DNA
<213> Artificial Sequence

<220>
<223> Antisense Oligonucleotide

<400> 116
gattcttccc acaggcaccg 20

<210> 117
<211> 20
<212> DNA
<213> Artificial Sequence

<220>
<223> Antisense Oligonucleotide

<400> 117
tgattcttcc cacaggcacc 20

<210> 118
<211> 20
<212> DNA
<213> Artificial Sequence

<220>
<223> Antisense Oligonucleotide

<400> 118
atcctgaagg tgctgctcca 20

<210> 119
<211> 20
<212> DNA
<213> Artificial Sequence

<220>
<223> Antisense Oligonucleotide

<400> 119
cggacatcct gaaggtgctg 20

<210> 120
<211> 20
<212> DNA
<213> Artificial Sequence

<220>
<223> Antisense Oligonucleotide

<400> 120
cccgccagct cactcacgat 20

<210> 121
<211> 20
<212> DNA
<213> Artificial Sequence

<220>
<223> Antisense Oligonucleotide

<400> 121
agtcagccag ctcctcgtcc 20

<210> 122
<211> 20
<212> DNA
<213> Artificial Sequence

<220>
<223> Antisense Oligonucleotide

<400> 122
ccagtcagcc agctcctcgt 20

<210> 123
<211> 20
<212> DNA
<213> Artificial Sequence

<220>
<223> Antisense Oligonucleotide

<400> 123
cgcctcttcc agtcagccag 20

<210> 124
<211> 20
<212> DNA
<213> Artificial Sequence

<220>
<223> Antisense Oligonucleotide

<400> 124
ggccggtgct gtacaatggg 20

<210> 125
<211> 20
<212> DNA
<213> Artificial Sequence

<220>
<223> Antisense Oligonucleotide

<400> 125
atcctctcct ccagcatcgg 20

<210> 126
<211> 20

<212> DNA
<213> Artificial Sequence

<220>
<223> Antisense Oligonucleotide

<400> 126
ccgctccacc acaaaggcac 20

<210> 127
<211> 20
<212> DNA
<213> Artificial Sequence

<220>
<223> Antisense Oligonucleotide

<400> 127
cgtccccaga gtctttgtca 20

<210> 128
<211> 20
<212> DNA
<213> Artificial Sequence

<220>
<223> Antisense Oligonucleotide

<400> 128
ttgtgtttgt gcccagaatg 20

<210> 129
<211> 20
<212> DNA
<213> Artificial Sequence

<220>
<223> Antisense Oligonucleotide

<400> 129
gctcggcccc cattcccaca 20

<210> 130
<211> 20
<212> DNA
<213> Artificial Sequence

<220>
<223> Antisense Oligonucleotide

<400> 130
aggcatttgg catctgacag 20

<210> 131
<211> 20
<212> DNA
<213> Artificial Sequence

<220>
<223> Antisense Oligonucleotide

<400> 131
cttgggattg ttggtcagca 20

<210> 132

<211> 20
<212> DNA
<213> Artificial Sequence

<220>
<223> Antisense Oligonucleotide

<400> 132
ctcggccact tggtcccagg 20

<210> 133
<211> 20
<212> DNA
<213> Artificial Sequence

<220>
<223> Antisense Oligonucleotide

<400> 133
ccccgcttgg tggtggacga 20

<210> 134
<211> 20
<212> DNA
<213> Artificial Sequence

<220>
<223> Antisense Oligonucleotide

<400> 134
cccccgcttg gtggtggacg 20

<210> 135
<211> 20
<212> DNA
<213> Artificial Sequence

<220>
<223> Antisense Oligonucleotide

<400> 135
ggagaagccc ttgccagcca 20

<210> 136
<211> 20
<212> DNA
<213> Artificial Sequence

<220>
<223> Antisense Oligonucleotide

<400> 136
ttcattccaa agggccaaga 20

<210> 137
<211> 20
<212> DNA
<213> Artificial Sequence

<220>
<223> Antisense Oligonucleotide

<400> 137
cccgctcctt gctgatgaaa 20

<210> 138
<211> 20
<212> DNA
<213> Artificial Sequence

<220>
<223> Antisense Oligonucleotide

<400> 138
gtgctcaaga tggcccgctc 20

<210> 139
<211> 20
<212> DNA
<213> Artificial Sequence

<220>
<223> Antisense Oligonucleotide

<400> 139
cccaagtgaa agtgacgcct 20

<210> 140
<211> 20
<212> DNA
<213> Artificial Sequence

<220>
<223> Antisense Oligonucleotide

<400> 140
acccaagtga aagtgacgcc 20

<210> 141
<211> 20
<212> DNA
<213> Artificial Sequence

<220>
<223> Antisense Oligonucleotide

<400> 141
ccgaatgcct cctccttggg 20

<210> 142
<211> 20
<212> DNA
<213> Artificial Sequence

<220>
<223> Antisense Oligonucleotide

<400> 142
gccgacaata cttcccgaat 20

<210> 143
<211> 20
<212> DNA
<213> Artificial Sequence

<220>
<223> Antisense Oligonucleotide

<400> 143
gatgctcctg gctctctggc 20

<210> 144
<211> 20
<212> DNA
<213> Artificial Sequence

<220>
<223> Antisense Oligonucleotide

<400> 144
tcaatgaatc taaagcgcgg 20

<210> 145
<211> 20
<212> DNA
<213> Artificial Sequence

<220>
<223> Antisense Oligonucleotide

<400> 145
gactcaaact gccctcctgc 20

<210> 146
<211> 20
<212> DNA
<213> Artificial Sequence

<220>
<223> Antisense Oligonucleotide

<400> 146
atcacccaca ttcactcatt 20

<210> 147
<211> 17
<212> DNA

<213> Artificial Sequence

<220>
<223> Antisense Oligonucleotide

<400> 147
aaaagtgccc agattgc 17

<210> 148
<211> 20
<212> DNA
<213> Artificial Sequence

<220>
<223> Antisense Oligonucleotide

<400> 148
aaaagtgccc agattgctca 20

<210> 149
<211> 20
<212> DNA
<213> Artificial Sequence

<220>
<223> Antisense Oligonucleotide

<400> 149
taaaagtgcc cagattgctc 20

<210> 150
<211> 20
<212> DNA
<213> Artificial Sequence

<220>
<223> Antisense Oligonucleotide

<400> 150
aagcagatca cccacattca 20

<210> 151
<211> 20
<212> DNA
<213> Artificial Sequence

<220>
<223> Antisense Oligonucleotide

<400> 151
aaaaagaggc ctgattgccc 20

<210> 152
<211> 20
<212> DNA
<213> Artificial Sequence

<220>

<223> Antisense Oligonucleotide

<400> 152
tctggcaaag tgtcagtatg 20

<210> 153
<211> 74424
<212> DNA
<213> H. sapiens

<220>
<223> Antisense oligonucleotide

<400> 153

```
agagcgggca ggagggagct gtatcagggg catttaaagt gccttgacgt cacgcactgc 60
caggaactca gctgagtttt cagcaggaca ttccggtcat cttccctccc tccccccggg 120
cttctgtgcc caagtcctcg gctcttccct cgctgtggcg gagggaggag caccgaactg 180
tcggaacagc cagcacaggg gcgtatcagt ctcctcttgg ctccgccctt tctcctagct 240
gctctcctca ttggtcagtg ggcggggctt cggctgtacc gcacacgcac tgggacctct 300
gggtggccga acgagctggc ctttcatgaa ttatgcatga cggcgtgcct cggccaggct 360
ggggctgggc gaggattggc tgaaggggct gtaattcagc ggtttccgga gctgcggcgg 420
cgcagactgg gagggggagc cgggggttcc gacgtcgcag ccgagggaac aagccccaac 480
cggatcctgg acaggcaccc cggcttggcg ctgtctctcc ccctcggctc ggagaggccc 540
ttcggcctga gggagcctcg ccgcccgtcc ccggcacacg cgcagccccg gcctctcggc 600
ctctgccgga gaaacaggtg aagggggtgc agggtggggc cgttggggag gcctggggac 660
ccgggggctc cgcagcggca gggggcctct gggaccttgg ggatgttgtg atggacgctg 720
cagtggggcc gggagagatg aagagacgcg gagggtcgcc ctgagggaag actcttcggg 780
atgacaggag cggcctcgg aagggactcg gggcgctgga gggaagtttc gttcttcgga 840
gaaacagaac gcgctcgagg gggcaccgtg gggcgagggc gcactcggtt gcggcggcag 900
gagtgaggga cagtcccccg atttcctgct ccctggggcc ctggggacgt tccggccacc 960
ggagcgactg tcacgccgac ggggatcacc ggcgcgagtg gggggtcgga aagcgcctcc 1020
tccccgcccg gtcggcggct cccgctgagc cacttcctcc gcttgccctg ttcccgctcc 1080
ttcaggagac agctgtgccc ttttggaggc aggaataggt gtgtctgtcg cctgcagcct 1140
tacgggctgg ctggtcgtgg gtaggcttta ttgcataaga atcaagtttc ctgtagggaa 1200
attgacagac cggtactctt tctaaattcc ctcgcatctt tttctaggtt aaattatgct 1260
ccccccacgt ccccgccttg taaaaaagag aaaaaaagac aaaataaaat ccccatcaac 1320
ccgtcaagcc agctctagag agagaaataa acctcttgac attgtccttt tccaaatacc 1380
tggtaaagtc ggccagaaga taaataattg agccattgca tttactggat tgtggtgttg 1440
cttaattgca taggacggaa tgaaccaatt gagagtggga gttttctgtc tcagagccaa 1500
gatcttgggt aaatgcagag gagagggaaa caaagacagg ctggccttga aaaaaccatg 1560
tgtgcaaact ttacatgcat ttgggggtg tggttgcact gaagttaaca agattcaaac 1620
cgtcgcccaa gttggtattt ccatgtttgg tacacatcac tctgtgccat atcaggtcgt 1680
tgttaagtgt ggtgacaaaa tcagtggtta gtcatttttt taattaaaaa tgtgtatagt 1740
gtgtacctgc tggtcttact gtatgtgcaa ctaaaggttt acatagtctg tgtatgggtt 1800
gtaaattttt ggctggctgt gctgataaag cattgggctt gaataaagca aagcagaaaa 1860
tcatctcaat cttttatatg tggatttaga ctgtgttatg acttggttca gccagttttc 1920
tatcttattt tatattaaat atgtctgtgt tctctgagtc agcacattta tttccttatt 1980
acatgttcca gacaggagtg ctagcccagt ttttgttcag tttgcacagt gggatgggga 2040
aacaagtctg gaatttaaaa aaaaatgttt tagaggttgg agccttgatt ttagtctcta 2100
tattagcaca tccatcacaa agaaccatta gtaaattcat gaatcttttg ttttttatgt 2160
agttcatttg agaagaataa tcacttagaa atatccacag tgccaggcat ggtggtgcac 2220
acctctgatc ccagctaatt gaaggctgag gtgggaggat tccttgagtc caggagttga 2280
gtctggtctg ggcaacatgg tgagaggcca ggaattgggt ctagagtcta gtctaagcac 2340
cataatgaga acccatcttt aagaaagaaa gaaaggaaag gaggaagaaa ggaaagaaaa 2400
agaaataccc acagcacagt tatgaattaa cccacaaagg acttgtgagg tgggtagttc 2460
acataacaat taccctaata tcgtagataa gaaaattgag gccaaaggat caagacactt 2520
ggccaacgca gcagagtgcc atagtggtgg aatttgtgcc tccttctgta tattttgtga 2580
aaagtatcag tgaaattctt tttttttttt tttttgagtc agagtcttgc tctgttgccc 2640
aggctagagt gcagtggcgc aatcttggct cactgcaacc tctgcctcct gggttcaagc 2700
gattctcctg cctcagcctc ccaagtagct gggactacag gcgtgcgcca ccacgcccag 2760
ctaatttttg tatttttagt agagaccggg gttttaccat attggccagg ctggtcttga 2820
actcctgacc ttgtgatttg cccacctcta tctcccaaag tgctgggatt acaggtgtga 2880
gccaccgcgc ccagtaagta tcagtgaaat tctaacatat atctgaacag taaaatacca 2940
ccaataggct gaaagacttc atgggaggta aatattcaat aaacaggtga aaaaagaaat 3000
acaaatggag cttgcttaga ttatttttct aattgctatg tctaacttgg gaagtgagga 3060
actgtttttg gtcagcataa tttaccatca gaatttagct atttactaat gaaaagaaat 3120
actaatctag gtttgtttta gattaaggac agtcatgacc taaatgtcat ttaaaccaga 3180
gtgcattgtg gcttgatcag tggtcatttc tgtctctaga aagttgcttt aacttctctg 3240
cctctacgtg tctcttgaca ttcagatatg aggtggggta gaggtggtga ccaactttcc 3300
agacgcctga gtccaaacct tcttagctta tggttttctt aggtgatgtg caaatcaaca 3360
aatatatact tttttttttt ttttttgag ttggagttgc actctatcac ccaggctgga 3420
gtgcagtggc atgatcgtgg ctcactgcaa cctcctcctc ccgggttcaa gtgattctcg 3480
cacctcagcc tcctgagtag ctgggattac aggtgcccgc cactacgccc ggctaatttt 3540
tgtattttta gtagagataa ggtttcacta tattgaccag gctggtctca aactcctgac 3600
ctcaagagat ctgcccacct cagcctccca aagtgctggg attacaggcg tgaaccacct 3660
tgcctggcca acatatatat acctttgca actttgtcag agttgctatg aagaataagt 3720
tgtatcttgt tcacagaaat tgcagtctac tgggggagct gataaatgtt ttaaccatcc 3780
aatgtaacat gttgtcatca aagagatggt gagactttac acttgtgcta acaaggtagc 3840
tgttctacat aaaagaacat acagtacaga tgtagaactt ttctgttatc atagaacgtt 3900
```

```
ctattggaca gtgctaggct gaatgctaca gatcttcaga gaaaggagag gttatgaggc 3960
ctggagttgt ctagaaagtc tttttgccaa agagggattt caactgggtc ccaaataatg 4020
ggtggaattt gataggtgta aagaatttgc ggtggtttat gcctgtaatc ccagcacttt 4080
gggaggctga ggcaggagga ttgcttgagc ccaggagttt gagaccagct tgggcaacgt 4140
ggtaaaactc cctctcccct aaaaataaaa aaaattagcc aggcctggtg gcgtggacct 4200
gtagtcccag ctactggtga gactaaggtg ggaggatcac ccaagccccg ggggttaagg 4260
ctgcagtgag ccgtgatccc gccaccgcac tccagcctgg gtgacagagt gagaccctgt 4320
ctccaaaaaa aaaaaaaatt cctggtagcc cggtagacta ggagggtaag taggggagaa 4380
gtgattactt acaaaagaca ttgaatacag gaccaaggaa tttcagttct gttcttttgt 4440
aggggaagct tttaaaactt tcggggcgcc gggcgcggtg gctcacgcct gtaatcccag 4500
cactttggga ggcccagacg cgcggatcac gaggccagga gatcgagacc atcctggcta 4560
acacggtgaa accccatctc tactaaaaat acaaaaaaaa gtagccgggc gttgtggcgg 4620
gcgtctgtag tcccagctac tcgggaggct gaggcaggag aagagcgtga actcgggagg 4680
cggagcttgc agtgagccga tatcgcacca ctgcactcca gcctgggcga cagagcgaga 4740
ctccgtctca aaaaaaaaaa aaataaataa ataaataaat aaataaaact ttggagccga 4800
agcactgatg tttaatcata gagtgcttac tatgtgttag gcacaggcct gattgcctga 4860
tgctggttaa tttgtacaaa gtaaatcagt gcatatgccc tctgccctag gggagttatt 4920
aactggagtc tgacattgta caaaggtagg tatcctgact agtttgattt ggtactttgg 4980
gtgaaaaaag tatagtgtgc ttaagtgcag aagtgttttt tgaggatttt tgattggata 5040
caaaccacca ctcatatttt atgtctttgg cacttaaaaa tttcaccata acttttgagt 5100
catttataaa aaccactgaa agagtacttg agggacatcc ccgaatcctg aagaacttct 5160
ggtgttctgg agcagcctca gtgagatcca ggaggatggc attgctgggc tggcccagcc 5220
cttattgatt atggtgtaaa gaattaatat ggtggtttata tactctttgt tagacacctt 5280
ggcttacaag acgtaagcgt aaagtgtagt gcgctttagt cagtatggcc acatggtcct 5340
ttggtggtaa attgtttgag atgcctccag tttttaaaag gagtagcata tcgggccagg 5400
agcagtggct catgcctata atcccagcac tttggaaggc cgaggcaaga ggattgcttg 5460
agcccaggag ttcaagacca gcctgggcaa catagtgaga ccactttgtt tctttaaaaa 5520
aaaaaaaaag gcaaaaacag gctgggcatg gtggctgatg cctgtaatcc cagcgctttg 5580
tgaggcagag gtgagcggat cacttgaggt caggagtttg agaccagcct ggccaacatg 5640
gtaaaacccc gtctctacta aaaatacaaa aattagccag gtgtggtggc acacgcctgt 5700
agttccagct actctggagg ctgagccagg agaattgctt gaacctggga ggtggaggct 5760
gcagtgagcc aagatcctgc cactgcactc cagactgggg gacagagtga gacattctga 5820
cagtgctaca ctgaatgcta catgtcttca gaggaaggag aggttatgag gcctgggaat 5880
aacatatgga agaatgaatt tctgttatgg tcagttctca tttgtcatgt taggattact 5940
gcaactctta cccagccggg tgtggtggct catgcctgta attccagcac tttgggaggc 6000
tgtgggcgga tcacgaggtc aggagatcga gaccatcctg gctaacacgg tgaaactccg 6060
cctctactaa aaatacaaaa aattagccca gcgtggtggc agacgcctgt agtcccagct 6120
actcaggagg ctgaggcagg agaatggcat gagtcctgga ggcggagctt gcagtgagct 6180
gagatcgtgc cactgcactc cagcctgggc aacagagtgg gactccatct caaaaaaaaa 6240
agaaaaaaaa aaggattacc gcaactcttt aattcagatc agcaaacatg ttgagagcca 6300
ggtattgcgt caggcaggat ccaaggataa tgaaatattg tccgttttca tgaaactgga 6360
gatgttgcag ggaccgaggt gtgtgctatg ccagtatgga agtaggacag gggagacgac 6420
agggcagtga gtggttcaag actctggctc tgaagtcaaa cagatctggg actgaatcct 6480
ggatctgcca cttcctagtc agaatctgag cctctatttt cttatctgta aaagaagatt 6540
ataacagtgc ttatcttgta ggtactgttg acgattcaat aagataatgt ggataaaatg 6600
cttagcatag tgcctggcac atagtaagag ctcggtaaat ctaagttctt actaaatatc 6660
caagaaaaga gattaattct tttcaggagt gagagaaagt catcattatt gaggggcttt 6720
atcagatggg aacacctgaa tagggtttta taggatgaat aggaattctt tccacgaagt 6780
tgcgttacaa aaagttgcat tcaaggctga aggaacatga gggtgcagag gcttaaaaca 6840
gccttgtgtg ttcagggagc tataagtaga agttcttaat ttaggagaac taaaccaagg 6900
ggaaaggagg ccaaggaacc acagttctta tccctttcct gttaataatt gggtttaaat 6960
gtcattaaaa taagttattt tgtccttttt agaaaagtaa taacatgcta ttataaaaaa 7020
aaagacttgt aggaatataa aatgtgtgtt ttacatgtat cctgttaatt gacttgcttt 7080
tattcagatt ttttgcagcc ctttctgttt accaggttat cttggagaca tatttattcc 7140
aaattccttt tttttttttt ttttgagatg gagtctcgct ctgtcgccca ggctggagtg 7200
cagtggcgct atcttggctc attgcaagct ccgcctcccg ggttcacgcc actctcctgc 7260
ctcagcctcc cgagtagctg ggactacagg cgcccgccac cacgcccagc taatgttttt 7320
tttttttatat ttttagtagc gacagggttt caccgtgtta gccaggatgg tctcaatctc 7380
ctcacattgt gatccgcctg cctcggcctc ccaaagtgct gggattacag gcgtgagcca 7440
gcacgcctgg ccttccaaat tcctttttaac agcctagcaa aagaataata aggaaggtaa 7500
atctgcccct acaagaaaat aatgcttcga cgatccggct ttccttcctg ctaccccccag 7560
ccataagaat aaatgacctt gctcatcact gaaattttac ctgacctttg aatttttaac 7620
tgcgtcagcc aaagaactta tattttgagt attcctaagg tgattgctat tgtagttttg 7680
aaacacttgg ttggtatgtt tgagggtttc atggtccaaa gttactatag cagttaaaag 7740
agtggactat caggtcagac ctattgggct ttaatcccag ttctgccttc tcttagacct 7800
```

tgggcctgtt gttttcactt ctctggtttt cagtttctct gtccacaatt gtggaaacga 7860
ggtccacttg tagagtaatt gagaggatga agcaagatga tgcatatcaa gtactttgca 7920
tagtgccggg cagacaggta acattcaagt gctaataatt actattatta ctatttattt 7980
tttgagacag gttctcactc tgtcacctag gctggagtgc agcggtgaga tcacagctca 8040
tgacagcctt gacctcctag gctcaagtga tcctcctgcc tcagccttcg ggtagctgg 8100
ggctacaggt gtgtgctacc accctcagct aattttctaa ttttttgag tcaggatctc 8160
gtcacgttgc ctaggctgaa ttactcttat taaaaactat aatatcaggc cgagtgcggt 8220
ggctcacgcc tgtaatccca gcactttggg aggccaaggc gggtggatca cctgaggtca 8280
ggagttcaag accagcctgc ccaacagagt gagacccccc ccgtctctac taaaaatata 8340
aaaattagcc agttgtggtg gtgggcacct gtaatcccag ctactcggga ggctgaggca 8400
ggataatcgc ttgaacccgg gaggcggagg ttgcggtgaa ccgagatcgt gccactgcac 8460
tacagcctgg gtgacagagt gagactctgt ctcaaaaaaa ccgaaaaaca aaaagcataa 8520
ttagggtggt aacgcttata catagggca ggtggaataa aacataatta ggaggtcggg 8580
catggtggct cacgcctgta attccagcac tttgggaggc cgaggcgggt caggagttca 8640
agaccagcct gcccaacata gtgagacccc gtctctacta aaaatataaa atttagcctg 8700
ttgtggtggc gggtgcctgt agtcccagct acccgggagg ctgaggcagg agaattgctt 8760
ttgaacccag gaggtggggg ttgcagtgag ctgagatcgc gccgctgcac tccagcctgg 8820
gagacagagc aagactccgt cacaaaaaca aaaaacaaaa aactgtcata tcaaaaacta 8880
aactaaaatg gtaatatctg ttagatatta caaagtcagg caaattatga ttcatggcag 8940
ccactaatga cccaaaggag agaaagaata attagcagat tctaacctaa tgggaaaaaa 9000
actaaatgaa tagggatggg ggacttacat tctgttagag gaaattgagg ctgtcatata 9060
aaaggaatag gtaaggcaaa ctgtaaattc ctgtttacac aaatgccctt ctgataaatc 9120
tctgcattgc ccacagtcca tgattacctc tcccttattt taagtaatat ttaacacatt 9180
aaaaatggat taccacccaa ggaattgctc ccgacccaga aagtgcaggt agtgttgaag 9240
gtttgagggg aagaggaatg attagagttg gttgtgtctc aggaagaagc caacaggagg 9300
aaccttattt tgagtcaggt aaagaaggtg ggagtgagga ggcatcccgg tggccaggta 9360
tgaagctggg agctgattgc tgcacattac tcagctgaat taaatgtgcc ctcacatctg 9420
tgtgtgtgcg tacatgcaaa tgtacatgtg tatgagttag ttggaggggt agacctttat 9480
tttcctgtcc tgtaactttc ctttgcaaac taatctgtat tcagaacagt gttgcagtta 9540
agaaccaccc agcttgtcca tgaaacaggt tctctcaccc catctcccca gttttagaga 9600
aggcaggaaa gaaaaggcag tgcttttctt ttttcctggc cgtatgcggg gcaggaagaa 9660
gccagcagag cttgaaagag aaagtaaacc ttctgggaaa taaacggctt ggcttcccta 9720
ttgtggagga ggagtgcaaa ttattagggg gatgtttggg tagtttttgt agaagccatt 9780
tctgaaaact gatttggatt agtgaaggta agcccaattt aggaaaaccc tgcccagtct 9840
ggtgtcagcc acctgtttcc cgctttgttt gattgatttg attagtttgt ggtattctga 9900
cctctcattt ttattacaag agttggaaga tttgagtctg aacttgagca cctgcttcgg 9960
tgaaagcttc ctaaaatgca tgttttttca cattttttct catgttcatt ttgttttgct 10020
ttttagcaaa cacttttct gacagaatct aaaagcatta gacttttctt gttttcccct 10080
tctctcccca caatgtaatc ttgaaaaccc aaatgttagc tgtgtaaatt acctctcccg 10140
taaaccaaac aaagtgcaat attgcattga gttagcattg aaatagtcgg cctttgaatt 10200
tttttctact tgtggtttag acataataaa tatttcatct cagactgact ttctcgacaa 10260
atcagttttg catttggccc tcttttcatc agtatgttta gggaaagcac atttattgaa 10320
acattaacca aaatgaaaca taattaggag gccgggagcg atggctcacg cctgtaatcc 10380
cagcactttg ggagaccaag gcatgtggat tgcttgaggt caggagttca agaccatcct 10440
tgccgacttg gtgaaatcct gtttctactg aaaatacaaa aaactagctg ggtgtggtga 10500
cgcgtgcctg taatcccagc tactctggag gctaaagcaa gagaatcgct tgaacctggg 10560
aggcagaggt tgcagtgagt cgagatcgtg ccactgcact ccagcctggg caacagagac 10620
tccgtctcaa acaaccaaaa aaacaaaaac aagcataatt agggtggtaa cgcttataca 10680
taggggcagg tggaataatt gaagcattct ggagccagaa ataatcaact gattaagaat 10740
aatctggctg ggtgcggtgg ctcacgcctg taatcccagc tactcaggag gctgaggcag 10800
gagaatcgct tgaacctggg aggtggaggt tgcagtgagc cgagatcgcg ccattgcact 10860
ccagcctggg ctatggagca agactccatc tcaaaaaaaa aaaaaaaaaa aatcctgttt 10920
ctgcagaaat atcccaggtg tcctgggtca gcagtgcccc atagattcca cggacgttta 10980
ccctaagttt tccaatggga gttcatacct ctatacccag tgagaatatt ttctgagtaa 11040
tgggaatgag attggagatg tagggtagag aagatccata cagtctttgg gttaaacttt 11100
ttcctctttg cctaggaaag attaatgcta atcttaacca cagatttgta gtaagaatgt 11160
atcagttttg tcattcagtt ctagactcca gttttcttta ttgtaatacc aatattttag 11220
agtaaatttt gaaatgaatc agtacaaaag atatgtagta agtggaaagt tagtccgcac 11280
cttatccttg ggactctttc ccagggacag ctagttacct actatttatc tctcctgagt 11340
tacttcatat gtatgcatgc aaacatgtta ttctctgggt gttgttcctt ccatatatag 11400
cagcaaatac accaaactct gtattttgct ttttgtcact ttatcttaga gaatactcaa 11460
tgcaaataca tgtgtatata cctcatgttt aaaaaatcta catagtaaaa ttagccaggc 11520
atggtagtgt gtgcctgtaa tcccagctac tcgggaggct gtggtgggag aatcacttga 11580
accctgagat cacaccactg gactccagcc tgggccacag agcaagattc tgtctcaaaa 11640
aacaaaaaca aaaacaaaaa aactacagag tagtattcta ggctatgcat atcataaatt 11700

tgatttccta atgataggca tagatgattt gcctgggcgg caaattagcg ttggctgtgt 11760
ctcaggaaga agccaacagg aggaacctta ttttgagtca ggttccaaag acagaaacat 11820
tgtctgacat ttgtttttgg gcttatatga ataaatctgt acacatatat ttttaatgtt 11880
ttaatcgtaa tatgtatact atttggaaat gtggcttttt agttaacaga gtgcatgttt 11940
taccccattg cacttaaaca ttaacttggg gataattaaa tgagtctgtc acttggacag 12000
gcaggaattg tacccccac aaacccataa accgccaatt ttttttttt gagacagagc 12060
ctcattctgt tgcccaggct ggagtgcagt ggtgcgatct gggcccactg taagctcagc 12120
ctcccgggtt catgccattc tcctgcctca gcctcccaag tagctgggac tacaggcgcc 12180
cgtcacaatg cccggctaat tttttgtatt tttagtagag tcggggtttc accatgttag 12240
ccaggatggt ctctatctcc tgaccttgtg atccgcccgc tttggcctcc caaagtgctg 12300
gaattacagg tgtgagccac cgcacctggc cggttttttt tttttttttt gagatggagt 12360
cttgctctgt tgccaggctg gagtgcaatg gcatgatctc cgctcactgc aacctccacc 12420
tcccgggttc aagtgattct cctgcctcag cctcctgagt agctgggact acaggcgtgt 12480
gccaccacgc acagctaatt tttgtaattt tagtagagat ggggtttcat taataatcat 12540
taatattaga caactgtcag actcacagtg gtggatacaa actttctcaa attctgattt 12600
ttactctaaa gctcaaattt tatcattggc aacaaatatt gtcagttgct ttccctgaac 12660
agacagcttc ccttctttca tttttgagaa aatatctgcc agtatcccag ttggtttatc 12720
aatcattctt tctctttttt tttttgagac ggagtctcac tctgtcaccc aggctggagt 12780
gcagtggcat gatctcggct cactgcaacc tccacctccc aggttccagc aattctcctg 12840
cctcagcctc ccgagtagct gggattacag gggctagcag ccacacctgg ctaattttg 12900
catttttagt agagacaggg ttttaccatg ttggccaggc tgatcttgaa ctcctgacct 12960
catgatatgc ccaccttggc ctcccaaagt gctgggatta caggtgtgag ccattgcgcc 13020
cggctctatt atttcttttc tttctttctt tttctttttt ttttttgaga tggagtttcg 13080
ctcttgttgc ccaggctgga gtgcaatggc gcgatctcgg ctcaccacaa cctccgcctc 13140
ccgaattcaa gtgattctct tgcctaagcc tcccgagtag ctgggattac aggcatgtgc 13200
caccacaccc gtctagtttt gtatttttat tagagatggg ggtttctcca tgttggtcag 13260
gctggtctcg aactcccaac ctcaggagat ctgcctgcct cagcctccca aagtactggg 13320
attacagttt tgagccacct gacccggttt gcttattatt tcttttaaat ttaaaaaata 13380
ataaataaag gggccatgag agcgaagagt ttgagaaagg ttggtctaaa ggttttaaca 13440
taagaatccc tgggttattt gcttaaaaag aagaaagaat ctatggatct gcctgagagg 13500
gtctgatgta gtttatctgg ggtcatcctc acaggcatag cagatattct gattcagatg 13560
gtccttggtc cttagtttga gaaatgtggc tttacaaggc ccatagaata taaagtcttc 13620
tttggattag tgaagtcatg tccacagggt ttagaaaatg tttttgtttt agagataaag 13680
gtaagtggaa gagtagacat gtagtgaatg agggaaaatg ttttagagat ttctttttat 13740
tctgtttact cttcttggta tgcacgtacc tgaatattaa ggatatttta tgaagtcatg 13800
acattaccag attaatgttg gttttgtttt aaggtacttt ctgactgctg gggttaattc 13860
ctacagacga ttctggtaaa gaatagcctt taagttttaa aagtgttgac ttatttcaga 13920
tgtcttaata aagttaactt ccagttatta catgtaacgt atataaagct ctcattttcc 13980
tttattctcg ttaattgttt gcataacaaa ttcaaaggga aatttgcttg gcagagatca 14040
gatagcagag atgagattta aaaacaggta atttggctac tagcctggga gtttgaagat 14100
tccaagtttg catccatgtg tagtcactta acatttctgt ccttatctgt aaatgggaat 14160
aacacctact tgatagggtt gttacattat cttggccacc tcaggttctc tttggctgag 14220
tgattgactg gaaaacgcaa tgtgaattca tgcttcagac tgggttcttt tttttttttt 14280
tttttgagat ggagtttcac tcttattgcc caggctggag tgcaatggca cgatctcagc 14340
tcactgcaac ctctgcctcc caggttcaag cgattctcct gcctcaggct cccgagtagc 14400
tgggattaca ggcatgcacc accatgcctg gctaattttt ttgtattttt agtagagacg 14460
gggtttcact gtgttggtca gactggtttc aaactcctga cctcaggtga tccacctgct 14520
tcagtctccc aaagtgctgg gattacaggc atgagccacc gcacccagcc caggctaggt 14580
tctatatggg tgtgcttttt agaatttaga tcatgggcta tccccaacac aaactggata 14640
atgtttcttt ctagattctc tctaagcgtg tattctcttt ctttcctagg cacagccacc 14700
acttcactta cattgtggga ttataatttc atgagtagtg gaatttcctt aaccttctct 14760
tgtgtgggag ctgaaggaca aaatgagata ttctctgaag agtggttaca tcatgcaaaa 14820
ctatgatgtg taatgaggtc acttagtttt ctaagtacat tatacatttt gataagattt 14880
tcatagaaaa gcttgtctcc ttggggagat cactcatctt ccatcttgac tattatttaa 14940
actttatggg tcagatttat ctttttaaaa acttaaccat aaagctcaat taattttttt 15000
tttttttttt tgagacggag tctcgctctg ttgcccaggc tggagtgtag tggcgcgatc 15060
tcggctcact gcaagctctg cctcccaggt tcatgccatt ctcctgcctc agcctcctga 15120
ctagatggga ctacaggcgc ccgccacgat gcccggctaa tttttgtat ttttagtaga 15180
gacggggttt caccgtgtta ggatggtctc gatctcctga cctcgtgatc cacccgcctc 15240
ggcctcccaa agtgctggga ttacaagcgt gagccaccgc gcccggctca attaatatat 15300
tttaaaaatt aatagacttt attattttta ttttatttta tttttgaggc agagtctcgc 15360
tctgtcaccc aggctgagtg cagtggtgtg atcttggctc actgcaaact ccacctcccg 15420
ggctcaagtg attctcctgc ctcagcctcc taagtagcta ggattacagg tgcctgccac 15480
catacccggc tagtttttgt aattttagta gatacgtgtt ttctttcttt tcttttcttt 15540
tttttgagat ggagtttcac tctttttgcc caggctggag tgcaatggca tgatctcggc 15600

tcactgcaac ctccgcctcc caggttcaag tgattctcct gcctcagcct cccaagtagc 15660
tgagattata gttgtctgcc accacgcctg gctaattttt tgtatgtttg atagagacag 15720
ggtttcacta tgttagccag gatgtctcga tctcttgacc tcgtgatccg cctgccttgg 15780
cctcccaaag tgctgggatt acaggcgtga gccactgcgg ccagtctaga ctttattttt 15840
taaagcagtg ttagttttac agaaaaatta tgtggaaagt acagagagtt tccatatacc 15900
ccttactttc tcccacaact tctattatta acatcttgca ttagtatagt acgtccctta 15960
caactaatga accaactcga tacattatta ttaaccaaat tcctgagttt attttatttc 16020
tatttttatt ttattattat tattttttag aggtagggtc tcactgtgtt gtccaggcca 16080
ggttgcagtg gcatcatcat agcttgctat agcctgaaac tcctgggctc aagcaatcct 16140
cctgcctcag tctcccaaag tgttggaatt acaggtgtga gccactctgt ccagcctgaa 16200
gtccatagtt tacattacat ttcactctgt tgagcattct atggattttg acaaatgtgt 16260
gatgatgtat atttgccagt acacaattat ataaaatagt tttactgccc tagaaacccc 16320
ctgtgctcca cctattcatt cctctgctga accactggca accactgatc ttttataata 16380
tctccatagt tttgtctttt ccagaatgtc atatagttgg acatacagtg tgtagccttt 16440
tcagattggc ttctttcagt aaatgatatg catttcaggt ttcttcatgt ttttttgtgg 16500
cttgataggt tgtttctttt cattggtgag taatactcta ttgtatggat ataccacatg 16560
ttgtttatca aacattcacc tgaaggatag acatcttggt tgcttccaag tttgagcagt 16620
tatgaataaa gctgctataa acattccagt gcaggacttt tcacctcctc tggataaata 16680
tcaaggagtg caattgctag atcatatggt aagagtatgt ttagttttgt aagaagctat 16740
caaactatat tcaaagtgac tgtaccatta tacattccca tcagcagtga gtgagagttc 16800
ctgttactcc acatcttcac cagcatttag tggtgtcagt gttttggatt ttagccattt 16860
taatgggtgt ataatggtat acctattaaa attggttttt tttggagaca gagtttcaca 16920
gtttcactct tgttgccctg gctggagtgc aatggcgcaa tctcggctca ctgcagcctc 16980
cgcctcccag tttcaagtga ttctcctgcc tcagcctccc aagtagctgg gattacaggt 17040
gcacgccacc atgttctgct aattttttttg tattttagta gagatggggt ttcactgtgt 17100
tacccaggct ggtcttgaac tcctgagctc aggtaatcca cctgcctcag cttcccaaag 17160
tgttaggatt acaggcatga gccaccgcac ctggcctcaa tttttttttt tttttttttg 17220
agacagagtt ttgctcctgt tgaccaggct ggagtgcagt ggcacaatct cggctcactg 17280
caacctccgc ctcctgagtt caagcgattc tcctgccaca gcctcctgag tagctgggat 17340
tataggcgcc cgccactacg cctggctaat tttttttttt tttttaatta gagacgaggt 17400
ttctccatgt tggtcaggct ggtcttgaac tccccgttct caggtgatcc gcctgcctca 17460
gcctcccaaa gtgctgagat tacaggtgtg agccaccgtg ccccgcctgt tttggctttt 17520
actgtgaaga cgtgttagcc gctgtgatga ctagcaagtg tggccctcca cccagtcgct 17580
ctgggctccc agctcctgca tcctgctgca aacttgacat cttccctcaa gtaacttgta 17640
gttgtctcct gtctacttgc ccaaaatata actcttaaac ttttctctct gcaagtttgt 17700
gcctctctcc ctgtctgact tccccatcta aataaatggt agaccaccat ctactccttt 17760
gtgcaagcca gaaatctagg aatcatcctt aaattccctg ttctgtctta tctctgcttt 17820
cattcaaagc atcagcaaat cctgttggtt ctacctctga agttttctca aatactgtta 17880
cttgactcat cctgactttt gtttctgctt tatgttaggc taaatgccct gaaaactctt 17940
ttgtacaaaa cacctagaaa tactggataa actgggctta acagggaggc ccggtgtggt 18000
ggctcacgcc tgtaatccca gaactttggg aggccaaggt gggtggatca cctgaggtca 18060
ggagttccag accagcctgg ccaatacgta gtgaaacccc acctctacta aaaaaaaaaa 18120
aaaaaattag ctgggtgttg tggtgcacac ctgtaggtgg tgcatgcttg aacttgggag 18180
gcggaggttg cagcgagctg agatcgcgcc actgcacttc agcctgggtg acagagcagg 18240
attctgtctc ttaaaaaaaa aaacaaaaaa agaaaaacag gaaaatcttc agaagcaaaa 18300
accaaacaat ctcaccaaag aaatgagaag atggctgggc gcggtggctc acgcctgtaa 18360
tcccagcact ttgggaggcc gaggcgggca gatcacccga gatgggcaga tcacccgagg 18420
tcaggaattc gagaccagcc tggccaatat ggtgaaaccc cgtctctgct aaaaatacaa 18480
aaattagcca ggtgtggtgg caggcgcctg taatcccagc tactcaggag gctgaggcag 18540
gagaatcgct tgaacctggg aggcggaggt tgcagtgagc cgagatcatg ccactgtact 18600
ctagcctgga cgacagagca agactctgtc tcaaaaaaaa aaaaggctgg gtgtggtggc 18660
tcatgcctat aatcctagca ctttgggagg ccaaggtggg cggatcactt gaggccaggt 18720
gaacatggcg aaaccccatc tctactaaaa atactaaagt tagctgggca tggtggtggg 18780
tgcctgtaat cccagctact cgggaggcga ggcaggagaa tcgcttgaac caggaggtgg 18840
aggttacagt gaaccgagat ctcgccaccg cactctagtc tgggcgacag agcaagactc 18900
cgtctcaaaa aacaacaaca aaaaaccaac acatggccaa agtgcagtga cttacatctg 18960
tataatccca atgttttggg aggctgaggc aggaggatcg cttgagtcca ggaatttgag 19020
accagcctgg gcaacataga cctcatcacc aaaaaaaaaa tattttttaa ttagctgggt 19080
ttggcagcat gtacctgtag tcctagctac tcaggaggct gaggtgggag gatcacttag 19140
gcccaggagt ttgatagttc gaggttatag tgagctatga tcctgccact gcactccagc 19200
ctgggccaca gagtgagacc ctgtctctta gaaacaaaac aaaacaaaaa aaagaaactg 19260
aattaaaaac aacaagaaca aaaatgctgc tttttgttat tgagttgtag cccaagtttc 19320
ttgagggtaa agcattgaaa agcaggcagt aatagatttg ctgtttaaag agatttactt 19380
gcagcactat tcacaatagc aaagacatgg aatcaaccta aatgcccatc agtgacaaat 19440
tggataaaga aaatgtggta catacactgt ggaatactat gcagccataa aaaacaacga 19500

gatcatgttt ttgtttgttt gtttgtttgt ttgtttttga gatggagtct tgctctattg 19560
cccaggctgg aatgcaggtg gcacgatttc agctcactgc aacctccgcc tcccaggttc 19620
aagcaattct ctgcctcagc ctcccgagta gctgggatta caggtgccct ccaccatgcc 19680
tggctaattt ttgtatttct agtagagatg gggtttcacc gtgttgggca ggctgttctt 19740
gaactcctga cctcatgatc ctcccacctc ggcctcccaa agtgccggga ttacgtgtga 19800
gccaccgtgc tcggctgaga tcatgttttt gcaggaacat ggatggagct ggaggctatt 19860
atccttagca aagtaatgca ggaacagaaa accgaagacc acgtgttctc acttataagt 19920
gggagctaaa tgataaggac ttgtgaacac aaagaaggaa accacagata ctggggttta 19980
cttgagggtg gagagtggga ggagggagag gaacagaaaa gataactatt gggtattggg 20040
cttaatactt aatattttat caaaataagc tgtacaacaa acccctctga catgagttta 20100
cctatataac aaacttgcac gtgtaacccc aaacctaaaa taaaagttaa aaaaaaaaaa 20160
aaaggctggt tgcattggga ggctgaggca ggcagagcac ttgaggccag gaattcgaga 20220
ccagcttggc taacgtggag aaaccctgtc tctactaaaa attcaaaaat tagccaggtg 20280
tggtggtgca tgcctgcagt cccagctacc agggaggctg aggcaggaga attgcttgaa 20340
ctcaggaggc agaggttgca gtgagctgag attgcaccac tgcattccag cctgggcgac 20400
agggcgagac cttgtctcaa aaaacaaaac aaaacaaaac aaaaacctgt cactttggga 20460
atatctcaaa cctagtcatc caagtggttg tacgatttta gtgtctgcat atcaatattt 20520
agtgtgatct actttcttag attctcaaat actgccaatg ggcacatgtc atgaaataat 20580
gtcttttaga ggacaagaga gtgctaaagt ctcattattg cagtttaaga aaaacaattc 20640
tgtaacagtt taactttata ggaaatgcct tttgtttatt tatttttttt cttttgaggc 20700
ttagattttt atttttatgt ttttagagat ggggtcttcc tatgttaccc aggctggcct 20760
tgaattcctg ggctcaagtg atcttcctgc ttcagcctcc tgagtagctg ggactagacg 20820
tccactactg ctcctggctg gaagtttaga ttttaattta aactcttcta ttgggaaact 20880
ttgtatgttt gctttaccac ttaacatttg catgcattat tgtacctatt gtctcctact 20940
taaggaaggg cagtttatgc tgttatatga agtgaattaa cctcctatgg tacttcagtt 21000
ttctctatgc taaaagtgtg ttctagattt ttgaaaaact tacttaattt tcattcattt 21060
attcaaatat ttgagcattc tgtagttgct ggggaaatag cagtgaactg aagaatgtct 21120
ttgttcttat ggggcttaag ttcctagttg atcatattgg aaggagatac atgaaaaaag 21180
aaatatatga acaatggagg gcgatgagta ctgtaaagga gaattcagca ggggagatgt 21240
tgctgtttta gatagagggg tgtcaagaga cattgtgcag agacctgaac gaagtgaggg 21300
agcaagccat ggagatatct agggaaagag cctatcaggt ggagagaaga gtcctagggc 21360
agaaacgggc aaggtgtgtt ccaggagcag agaggggaca gctgtgagca aggggagagt 21420
tgtagggaag gaggcaaaga gagacatctg gggcaaaatg gattgactgg tgggccgtgg 21480
taggactttg gattttttcc tgagtgggtt ttgagcaggg gaatgaaatg atctgactct 21540
ggtttttttt tttttggag acaaaatctt gctctgttgc cgaggctgaa gtgcagtggc 21600
gcaatctcgg ctcattgcaa catctacttc ctgggttcaa gctatgctcc tgcctcagcc 21660
tcccgagtag ctaggattac aggcttgggc caccatgccg gcgaatttct gtttttattt 21720
ttattttta tttattttta tgtttatgtt ttttgagacg gagtctcgct gtgtcaccca 21780
ggctggagtg cagtggcgcg atctcagctc actgcaacct ctgcctcccc ggttcaagca 21840
acttctcctg cctcagcctc ccgagtagct gagattacag gcgcctgcca ctacacctgg 21900
ctaatttttg tatttttagt agaaacggga tttcaccttg ttggccaggc tggtctcgaa 21960
ctcctgacct taatttatct gctcgccttg gcctcccaaa gtgctgggat gacaggtttg 22020
agccaccgtg ccagccagga ctcttatttt gaaaggatct gtaatgtgga gaatagaagg 22080
tagagggaca aggatgaaag catccaggcc agttagccta gtccagctat ctaggtaaga 22140
gatgctggtg gcctggatta aggctgcgtc agtgggaggt tgtgagaaag gctcaccttc 22200
cttttttttt tttttttttt tttttgagac aggatcttac tctgtctccc aggctggagt 22260
gcagtggtgc aatctcagct tactacaacc tccgcctcct gggctcaagt gataccccca 22320
cctcagcctc ccaagtagct gggatcacag gcttgcgcca ctatatccgg ctaattttg 22380
tatatttcgt agagacaggg ttttgccatg ttgcctaggc tggtctcaaa ctcctgagct 22440
caagtgatcc acccgcctca gcctcctaaa gtgctgggat tataggcctg agccattgtg 22500
cccggtcact tccagatttt gaagacagag ccaacaggat ttgttaatgg attaggtgtg 22560
gcaggaggag gggaggaag agagagagag actggagttg aagttaaggc tcatttcaag 22620
gtttttagcc tcaacatgtg caggaatgga gttgtcactt gctagaatgg gggagactgg 22680
aggagaagcc ggctgggaga ggtttttaat gaaggggttg gctttggata cattaagttt 22740
gacatgcatt ttagacatcc aggtggagat attgaagagg cagttggcta taagtgtctg 22800
atgttcatat tagcggatgg ggctagagac ataaatttga gaattgtcag tgtataaacg 22860
ttgttttgaa agaaagtggg gctgaataat ttagaaagga gtgcatagag aaaataagtt 22920
tactattaaa atagctttaa caggccgggc acggtggctc atgcctgtaa tcccagcact 22980
ttgggaggct ggggtgggca gatcaaaagg tcaggagttt gagaccagcc tggccaatat 23040
ggtgaaaccc tgtctctact gaaaatacaa aaattagcca ggcgttgtac cgggcacctg 23100
tagtcccagc tacttgggag gttgaggcag gagaatcact tcaacccggg aggtggaggt 23160
tgcagtgagc caagatcacg ccactgcact ccatcctggg caacagagca agactccgtc 23220
tcaaaaaaaa aaacaaaaaa aaacaaaaaa aaaaaacttt aacagcaaag cctcttcctt 23280
taaaattatg aatttttttc ttatggaagt tggactcttt cattattaag tctacattca 23340
atcactatgt tagtaaaaat gttgttctag ttgccgaatg caataaacca gctcagactt 23400

agtggcctaa agcagcaatc atttgactat gttcgaagat gccgtgggca ggaatttaga 23460
taacagcagg gatggcttgt ctttgctctg cgatgtctga ggtctcactg agaaaactca 23520
agcggctggg ggtaataatc atctggaatt ttctttactc ctgtatctga tgtctgggct 23580
gcgatgactc aaaggctgat ttcagctgag actgtagacc acgtgcctac ttgtggcctc 23640
cccttttgcc ttgggtttct cacagaatgt ggctggttct ggagaatgag acttccaatg 23700
aaatcaggtg gaaatgacat ctcgccgctt tcagcatgct ctattggttg gaacagttat 23760
ggacttagct agattcaaag gaagggaaca aagacccccct cctctcagag agtggggcat 23820
aatgagagaa tttagggcca tgttatccaa ccaccacaaa tgccttctga atttgaggtt 23880
ctgcctcaaa agttcatagt tcctttgact gaaggacttc tatatatcca agcatcgtca 23940
gccccaggta tattgttcca tgtaagtgac caggactacc ttagtatttc gtatagggaa 24000
agtgacctga ataaatttga gaaaagaatc ttccttctct ccagtaagca ctgaggtaag 24060
cattgagcca tattataggt ttatgacttt gagactcaga aatttaaatt cttggccagg 24120
cgcagtggct cacgcctgta accccaacac tttgggaggc caaggcaggc agatcacttg 24180
aggtcaggag tttgagacca acctggccaa aatggtgaaa ctccatctct acgaaaaata 24240
caaaaattag ccaggtgtgg tggcgggcac ctgtaatccc agctacttgg gaggctgagg 24300
taagagaatg gcttaagttc tctttatctg ctttatttca gttgcctctc ttagatgaat 24360
attaatgact tacatagcat tttagatcag tggatgtttt tgtgattctt ttatttgagc 24420
tttggccaaa gataacagta cccacaggtt ttttccagct actcgctctt ctcccttcag 24480
tggccctcga gcctggaaaa tctgacatga caatgtgctt gctcaaccta ccactgtttt 24540
tcttttgaaa agtttggcag cctgtttctg actcctatga aggtgaattc ctcagcattc 24600
acagtttatt agaaaaatac tttgcttctc tccaaactcg aaattcaaga taaccaaacc 24660
tatatatagg ctgatctttc aggatgcagt tgtcatgttg atgccatgct tttcagtatc 24720
gtggccatca tctgttcagt aggggaggtg tacttctgta atgggaggtg gtggttatgt 24780
gtgtgtgcaa gtgtttattt ggtgtcttaa gttagcctgt gggaagttct aaatcaggat 24840
ggtacgtggt tgccagcaga gagctgctcc tcaagtgaag gaggtagaat caaagccaat 24900
aggaaagagc ctcagatgct tatatatgta ccgtggggat tcagagtgaa agcagtcatt 24960
ggactagggg tggggttagg gagagcctgt ctgacagaca caagaaaggg atggataacg 25020
ccacccagag aaaaaagcat tttaggcaag aacaaatatg aaaaaggaac aaagtctgtg 25080
ggtggggggc aaggaggaga taagttgact tgaaggaaga caacacttat gaaagtcacc 25140
tggaggctgg gtgccatggc tcatgcctat aatcgcagca ctttgggagg ccgaggtagg 25200
aggacaactt gagcccagga gttcgagacc atcctgggca acatggtgag actgagtctc 25260
taccaaaaaa aaaaaaaaaa gaaaattatc cagacatggt ggcatgtgcc tgtaatccca 25320
gttactcagg aggctgaggt gggagggttg cttgagccca ggaggttgag gctgcagtga 25380
gctgtgatcg tattattgca ctccagcctg ggtaacagag caagaccctg tctcaaaaaa 25440
tgaaagtcat ctgtaggctg gagagaggaa ctggaagggg ctaaagttgg ctgagtagtt 25500
acagagcctg agataagggt aaagattttg cattggacaa tgagatgtta gtgtgtgttt 25560
ttgagctggg gagtgctgtg attttactct tattgaagaa tcactgaagg attattcttg 25620
aatcagtgat tcttgatcat tcttgaattt ttcaaacagc aaaactggaa gagttggcct 25680
attcctcaga atattttcta attgggcgca gtgtcctcac ttgggagaac ctggctacac 25740
actttagttg taattcactc cagtcgttca ttcattcaat acctattttt tcagcaccta 25800
ttatgagcca gacactatgc tggatgccag ggttcagggt aggacacgct agtgagcaaa 25860
agccaagact cttcttgtct tcatggggct ttcagtccag catagtggtt atgagtccaa 25920
gttaatggag tcacagtact tgggtgcaag tcatggtgat ggtgatagaa ggaaggcatg 25980
tgtgagggcc agtggcaggc aggagcctgg tgtttttgag gacctgaaga aggagcagag 26040
tgagtgccag gaacttagcc accagctggt accagccata cgagaggggc agagccagcc 26100
aggatgtcgg tcatgctagt aatgagtaca aacacttaca tgctgcacgc tattgggctc 26160
ctgagtgcta cgtgttcatt agctcgatga atttgtacag caaccctgtg aggtaagcac 26220
tgttctctcc cctttctata gatgaggaaa ttaaggcaca aagaggataa ataactggca 26280
ccagctacac gctaagtgat cgaagtggtg gaaccaggat tcaaatccat gctattctgc 26340
cttaagataa caaatcttgt tttttagcct aagaacagag cagtcatcag gagggtttta 26400
agtaggggtg tggcaagatc aagtttgtgt cttgaaaagg tctctctacc cacagtgtgg 26460
aaaatggcct ggaggcaagc acacagatgt tgggagacag ttaacagctc ttgccatggc 26520
cccctatgca ttttggctct gatgtttctg cctgattttt ctcttgcctc tgcctctttt 26580
cctgagggga tggcaggttt taccattcag ctggagtaca aaccctgaac cctttttggt 26640
taaatatcta cttgcttttc ctacagtatt attttgagtt gctgtggctg taatgtcttg 26700
agggaatcga gcttgacagt aatttataga acaaacagtt tttagagact gtgtggccca 26760
attgccctct caatgttggc actcctgcca tgacatttac catgctgagc atgtgaccgc 26820
catctgaata ccaaatgcca caggaacctg ggaggttgtc acttactcct ccctttctct 26880
gagtcacctt tgcccttcag tcagtcacca agtcccatca catgtagctc tgtaatgtca 26940
cagaagatgg atgtctgcct caaaacactt acaatgctgc tacctaaatt gggcagccac 27000
gacctcccac caggattatt gcagcctgag ggatcttttt gaaatgtaaa tcaaactatc 27060
acttgtctgt ttaaagcttt tcaaagactt accccattgc ccttggaaga aagtgcagat 27120
atcttgacag gagagccttc tccagcctcc tcttctgccg tggtctcctt gtacagtctc 27180
tacagtgtac tgcttcatta gaaccctgga gattattatt tgctagttct gggctaagaa 27240
ctggcacctg gctttgtaga gctcctcagg agattctgag gcgtattcag agttgagccc 27300

tgatctctgc tctgatttcg aggttctcgt tatatttatt aatgatcacg aaaaaattta 27360
ttattattct ttggcctcac tttagcatca tctgaggaat tttttttttt ttttgacaga 27420
gttttgctct tgttgcccag gctggagtgc aatggcgtga tctcagctca ctgcaacctc 27480
cgcctcccgg gttcaagaga ttctcctgcc tcagcctccc aagtagctga aattacaggc 27540
atccaccacc atgcctgcta atgtttttgt attttttagt agaggtgggc tttcacagtg 27600
ttggtcaggc tggttttgaa ctcctgacgt cagctgatcc acccacctag gccccccaga 27660
gtgctgggat tacaggtgtg agccaccgtg cccagccgta gctttcgaaa tttgaaacct 27720
ggtcccactg tcagaggttc caatttggca ctggtttggt tcccaggcat ctttcttgct 27780
gtatatattt tttagtgtca gccagggtgg agacctctgt attacttcat ggggaagaat 27840
ttgggagaag atgttgtgag gagacaggtt ctagtcctag agtgatttat cctttctcgt 27900
acagatttcc aggtatttga ggggccactc ttctgtaatt catgtttttc tctcctaacc 27960
tcactcctgt tgcctgcatc ttcttgctga gcaaaatatt caaggtcttc aactcctcac 28020
accctggttg tccctccctg gatgtgtttg gttgttttag tgttccattt caattttgat 28080
acacagaatt agaatagcat ccagatgtgg gtctgttaca gctagactac tagatccttc 28140
aaaatccaag tactagtatg tctattaaaa taccataaga tcacattggc tagttacaat 28200
ggttggtttg tgggttactt aaaaatcaac taaaattctt tttttttttt tgagatggag 28260
ttttgctctt gttgcctagg ctggaatgca atgacacaat cttggctcac tgccacctct 28320
gcctcccagg ttcaagcaat tcccctgcct tagcctcctg agtagctggg attacaggca 28380
tgtgccacca tggccagcta attctgtatt tttagtagag atgaggtttt tccatgttgg 28440
tcaggctggt ctcgaactcc cgacctcagg tgatccacct gcctcagcct cccaaagtgc 28500
tgggattaca ggcgtgagcc actgagcctg gccaaaattc ccactttcta atactcctgt 28560
agtagctggg tacggtgggt cacatctgta atcccagcac ttttggaggc tgaggctgga 28620
ggatcgcttg agcctaggag ttcgagacca gcctgggcaa gatggccaga cgccatctct 28680
aatttaaaaa aaagaaaaaa caagactcct atagtggtga agaacagaca ttccgaaaac 28740
agactgtgcg ttatgattcc agctccatgc ctttactacc tgtgttgtga ctttggataa 28800
atcacttaaa aatctttttt tttttttttt ttttttgaga cggagtcttg ctctgccgcc 28860
caggctggag tgcagtggcg cgatctcggc tcactgcaag ctctgcctcc caggttcaca 28920
ccattctcct gcctcagcct cccaagtagc tgggactgca ggtgcccgcc actacacctg 28980
gctaattttt tgtattttta gtagagacgg ggtttcaccg tgttagccag gatggtctcg 29040
atctcctgtc ctcgtgatcc acccgcctca gcctctcaaa gtgttgggat tacaggcgtg 29100
agccaccgca cccggccaaa tcacttaaaa ttctgtgcct cagtttctcc tctgtaaagt 29160
gggataaaaa tagtacctat ctgatagggt tgttacaatt atgaaatgag caaataagta 29220
tgtcaagtgt ttaaaacagc gcctggcttc ttgtaaaaag tgctatataa atcatagcta 29280
taatcattac ttatttcgac tgctctttaa ccaaggttct tatttttcat ctttttcttt 29340
tgttttgaat atcacttagt gttttcacct tttactcttt ttaggaccta gagccatcct 29400
aggtgaaata cgtatggaga tatttgatca ggtcaccacc cagctctcct gacctcccttt 29460
ctctccttaa attaacatgc caaatcacag catcactgac tccttccctc ccgatatgat 29520
aagagtgtgc attgaaatgc atgtatttta cttagcaggg aaagctgatt agtgattatc 29580
acacttaacc cctagtgaat ctgatggatt aacctgcttt ccaggacact aaggaaatgg 29640
gtttaagata agaaatatct ggctgggtgc ggtggcttta cgcctgtaat cccagcactt 29700
tgggaggccg aggtgggcag atcacgaggt caggtgattg agatcatcct ggctaacacg 29760
atgaaacccc ctctttacta aaaatacaaa aaattagccg ggtgtggtgg cgggcgcctg 29820
gagtcccagc tactcgggag gctgaggcaa gagaatggtg tgaacccagg aggcagagct 29880
tgcagtgagc tgagattgtg cccaccgcat tccagcctgg gcaacagagt gagactacat 29940
ctcaaaaaaa aaaaaaaaaa aagtaagaaa tgtccatgaa agggagaccc tgggggaaag 30000
gaacaataac tgcagctctg aggatctggc accagcagca ccagcacaga gggatgctgt 30060
acaaccatta ttgattttaa ctttacaaca gttcttcaaa ggagagagag ttccctgttt 30120
tactgaagag aaagcccatt tggtagtgaa ataccattcc caaagacaaa tagctaataa 30180
atgtcaggca gggttttgca cccaggccca tccagctccc gtctctactg tcctttcccc 30240
cacaccacac tgatacagag gaatgtgtct ggttggggaa gtggaagtgt tcccaagtgg 30300
ggaggtcatc tgatgcacaa atttggtctg ttttgtgggt tttcttgttt tagttttagt 30360
ttttgtagag ctcagacctg ttcttaggca gctttaacaa tcaactgtgc actcagtaat 30420
tgacaaatca tgtttgttac ttttaattta gagggaatta ggtttgttaa gctcttgctc 30480
cttctttaga gatggggtct agctctgtca cccaggctgg agcgcagtgg tatgatcaca 30540
gctcactgca gtctcaatct gctcaagtga tcctcctgcc tcagcctcca tgggactaca 30600
agcatgggcc accatgctag gctaatttta aaaaaatttt tttgtagagg caaggtctca 30660
cggtgttgcc caggctggtc ttgaactcct gagctcaagc aatccctctt ccaccttggc 30720
ctctcaaagt gctagaatta taggcatgag ccaccatgcc tggcctttac ttctttcata 30780
tattcaaatt ttgtcatatt agtagggaac tataactcaa gttttcttat agattgatgt 30840
tcatttttac aagcttgatc gtcattggtt tttaatttta aagcaaatcc tgttatatgt 30900
aattgaacat tacagtaatt atagtaattt gtttcagatt gggcactcaa gtgttaatat 30960
tttgtctctt taggaaatca aaactagatt tatatataga cttcttattg caagtatcta 31020
gtcttaaatc ttacaaaggt actatttgga cttaaaacta tgaaattgtg tgcttactat 31080
ataagtgtac ttattttgag ttatgtttta aacttgaaat tccattctta atgtctagag 31140
taattatgaa tggttaaatt atgaatgact ctaatagttt aaagctacag tatttattta 31200

tttatttatt taatttattt tttgagatgg agtttcgctc ttgtcgccca ggctggagtg 31260
tagtggcacc atcttggttc actgcaacct ctgcctcgcg ggttcaagtg attctcctgc 31320
ctcagcctcc caagtggctg ggattacagg tgtatttcac catgcctggc taatttttgt 31380
atttttagta gagacaggct tttgccatgt tagcctggct ggtctcgaac tcctgacctc 31440
aggtgaccta ccctcctcag cctcccaagg attacaagca tgagccacca cacctggcct 31500
acagtatttt aatgtggact ctctgtcatc cattatgctg tttatcctgt ggtgaaaatt 31560
ttatgaagat tgaatgtttt tctctagcgt gaattgcttt ctcttacttt tctcattttt 31620
ttccttccta atctacttgc agatacttca gattattttt agaacgtggt atggtgagaa 31680
caaataaatt ggggtttcca aatcttaata aattatgtgg ccctcagtgg gattagcagg 31740
gttgtattga aaacaccaat agaaacaaaa tagttctttt atgcgcttta aataaaaatt 31800
tcttttcagg ccaggcgcag tggctcacac ctgtaatccc agcaccctgg gaggctgagg 31860
caggcagatc acctcaggtc aggagtttaa gacaagcctg gccaacatgg tgaagcgccg 31920
tctctactaa aaatacaaaa attagccggg tatgatggcg catgcctgta atcccagcta 31980
ctccagaggc tgaggcatga gaatcacttg aactcaggag atggaggttg cagtgagctg 32040
agatggtgcc actgcactct agcctgggca acagagtgag attctgtctc aaacaacaac 32100
aacaacaata acaaaacatc tcttttcagg ccaggtactg tggctcacgc ctgtaatccc 32160
agcactttgg gaggccaaaa caggagggtc gcttgagacc aggagtttga gaccagcttg 32220
ggcagctggt ctctatttga acaaacaaac aaacaaacac aatactcttt tcatagaaaa 32280
atgtttacta cacaataaac tttaaaagaa tatgcagctg tattaatgct atgactccaa 32340
tgtaaaaaaa aaaaaatata tatatatata tacacacaca caaacacatt ctgaaataga 32400
tttaaaggaa ttacatcaac atgtcaattt ttattttttc ggagacaggg tctcgctgtg 32460
tcacccaagc tggagtacag tggtgcaatc acagctcact gcagccttga cttcctggcc 32520
tcaagtgatc ctcccccctc agcctcccaa agtgctgggg tcacagacca ccacacctgg 32580
caacatgtca gtttttgttc tgcatagtgg gatggtggga tatggatgtt tttatctttt 32640
attttctttt ttatattttt ctaaatttte cacattgaac attattttat aatctttcaa 32700
acatatctct taaaaggact ggttcctata gaattcagtg caagaaatct tctgtgtttc 32760
tttatacttt ggttgccttg atcactgggc ctttcctgac agcaaagaag aggttagtgt 32820
aggcagcaga taaaacacag gtatgctcta tttaaaatgc atgtatttat aataaaagta 32880
taggtggtac ccaaaggaaa atgtcatgac acattgcaaa gtggaacaga agttatcttt 32940
agatcacttt ctgttctgga ttattgtatg agcctgattt tcgtctctct ttccgccttc 33000
cctcaccctc gttgtaaatc cactagtgca tggatgtgaa gtacaagtct taactttaaa 33060
aagttttatg aagctgtgta gtaaatccct tttgtaagtg gtcttgactg cgtttctcaa 33120
tatatctttt ggtttcatta gattcaagta tataaatgag aactgtaact ttggacagac 33180
tttttcagtc atctttacgg taataagttc ccaattagac aatagttatt tgttttatga 33240
cttgctgttg gtaggttatc cccaagggac tgagaaattc ctgttttgaa aagtccaaaa 33300
agtctttgat gacttgctgt ttcatttttt tcttttctct tcagttatag aaaacaggat 33360
tacacccacc ttgcctttgt acagtgcatc tactatctgc tgacttaacc tgagtaaatg 33420
ctttgaattg agccccatat aatgtcctaa ggcagcctat atggagtaat gaattgtctt 33480
ctctcttatg cacccagagt ggtagttggc actcaagttg ttcctcagat aactttgtgt 33540
gttctggggc tcaatgaagt agttattaag tcacaggctt ggggagaaca ttcatcctat 33600
ggcattgaat gaagtgttgc ccaattctag aatgtctaat aaaatttttt taaaaaccca 33660
caggcttaga attattccgt agatatgaag taatgtagtt agaacttagt ggagttcttt 33720
agattaactt gtaatttgaa aaaccaaaat tgaaattgtg aaataacatg ggctctttga 33780
ggtcttttcc agtaaaacag ttacagtaaa gctgcttggc agtgatttte ctagacactt 33840
tggctagtca tctcctgtga ctgctgttaa ttaaatatgg tttgtagcta agcagcctgt 33900
aaggagaaga ctatggaagt atttgcatat tctctccttg aaaatactac ctggtctttg 33960
gctttaagtt atacttttat tttcccctgt agaataacta ttaaagtatt acctatggtg 34020
attagactaa gaagtaaaac atgaaatcag tcattgttgg tgccctggtg ccttcttttt 34080
tttttttttg agacagagtc tcactctgtt gcccaggctg gagtgcaatg gcacgatctt 34140
ggctcactgc aacctctgcc tcccaggttc aagcgattct cctgcctcag cctcccaagt 34200
agctgagact acaggcgccc accaccacgc ctggctaatt tttgaatttt tagtagagac 34260
agggtttcac tatattggct aggctggtct caaactcctg accttgtgat ccgcccacct 34320
cagcctccca aagtgctggg attataggtg ttagccactg tgcccagcct ggtgctttaa 34380
ttttatggaa aaaactacta gctggtttct gttttaagaa ataacacagg ccgggtgcca 34440
tgacttgcgc ttgtactccc agcagtttgg gaggccgagg cgggcggatc acgaggtcag 34500
gagtttgaga ccagcctggc caacatagtg aaaccccgtc tctactaaaa atacaaaaat 34560
tagccgggcg tggtggggca tgcctgtagt cccagctact cgggaggctg aggcaggaga 34620
atcgcttgaa cctgggaggt ggaggctgca gtgagccaag atcgccccac tgcacaccag 34680
cccgggtgac agtatttcat ctcaaaaaaa aaaaaaaaaa aaagaacaca attattgtac 34740
tacttactag ccctcctctg tccccagcta aaaataagaa cagcaacaac caaaaaatcc 34800
ttagttatgt actggaaatg aattagataa ttttcaataa cttacacgtt tttaggatat 34860
gttagtttga aaatgcaaat attcatgcat gaccccagtg ttaatctatg atggagcagg 34920
tatagtggga tgctgtttca tgatttaatt tggaccttca gggagtagac tgtgatgcct 34980
ctgcatttgt atccaagaca aataattaaa tagtctattt ttggctgggc atgatgcctc 35040
atgcctgcag tcccagcact ttgggaggct gaggtgggag gatcgcttga ggccaggagt 35100

tcaagatcag tctgggcaac aaaatgagac cttgtctcta caaaaactac aaaaaattag 35160
ctgaacattg tggcttgtgc ccctagtccc agctactcag gtccctgagt taggaggatt 35220
gcttgagccc aggagttgga ggttacagtg atctatattt gccactgcac tccagcctgg 35280
gtgacagaga gagaccctgt ctcaaaaaat aaagtctgtt tttaaaatta attttaaaca 35340
ctggagttta ttacaaaaag cagttggttc tttttttaaa tcattttttt ttaggagaac 35400
caccgctttt tggctacatt gtctagagta gcagtgttca ataaaaataa gatccaagtc 35460
acatatgtaa tgttaagttt tcttttagtt tctttttctt ttcttttctt ttctcttctt 35520
tctttctttc tttcttttt ttttttttga tatgcagtct cactctgttg cccaggctgg 35580
agtgcagtgg cacgatctcg gcccactgca acctccgcct cccgggttca agcaattctc 35640
ctgcctcagc ctcccgagta gctgggacta caggcatgtg ccaccatacc cagctaattt 35700
ttgtattttt agtagagatg gagctttgcc atgttggcca gtctggtctc aaactcctga 35760
cctcgggtga tccacatgct ttggcctccc taagtgctgg gattacaggc atgagccacc 35820
atgccctacc aatgttaagt tttctagtag ccatattaaa agaagtaaaa agaaatgggt 35880
gaagttaatt ttaataatat attttattta acccaatata tctaaaatat tatcatttca 35940
acatgaacaa gatactttac attcttttgt ttttcactaa gtcctcaaaa tccagtgtgt 36000
attttatatt gacagcatag ttcagtttga agcagccaca tttcaagtgc tcagtagcca 36060
catgtggcta gtgactccat actggactgt gtaggtttag agtttcagta aatttgtatg 36120
caatagaatc tacataaatt ggcatattat gcagatttct ttgtatgcac atcagttctt 36180
gcatagcata agtcaggtca tgatgctttt agtctatgag gcagattttt tttttttttt 36240
ttttgagaca gagtctcact tggtcaccca ggctggagtg tagatgcaca atcttggctc 36300
actgcaacct ccatgtgagg cagattttaa cttggcccta atgcaaatat tgtaagagag 36360
atctaatggc ctttgatttc ttacagaggg caatcaatac atgccatggt tacaatgctt 36420
cagcatatag tatgcacgtc agccactgct tttactctgg ctagtgctta gtgtacctgt 36480
accactgccc aggcagcatt tgtcctgtgg caggtgaatc ttagggtgga aggtggcaag 36540
taacattgct tttttttgag agggagtctt gctgtattgc ccaggctgga gtgcagtggt 36600
gcgatctcgg ctcactacaa cctccacctc ccgggttcaa gtgattctcc tgcctcagcc 36660
tcctgagtag ctgggattac agacggccac caccatgctc ggctaatttt tgtattttta 36720
gtagagacgg ggtttcacta tgttggccag gctggtctcg aactcctgac ctcgtgatcc 36780
acccgcctcg gcctcccaaa gttctgggat tacaggtgtg agccaccgtg cccagcctac 36840
atttttaaat taattaatta taagcaggat ctcactgtgt tggccagact ggtcttgaac 36900
tgataagagt tcaagaccag cctaggcaac atggtaaaac cctgtctact aaaaaataca 36960
aaaaaaaaaa ttagctgggc atggtggtgc gtgcctataa tcccagctac ttgggaggct 37020
gaggcaggaa aatcgcttga acccgggaga ctgaagttgc agtgaggtga gattgcacca 37080
ctgcactcca gcctaggcga ttccatctca aaaacaataa caacaaaata acattgttgg 37140
aatatttagt taatttatag aagcgtattg gcctaattgg ggcaaatacc ttattctgac 37200
attctctcta tttgctttac tgagcttttt caccagtgga atttaagccc ttgatacatg 37260
aggagggaaa ataccttgga gctgtgctgc acatgtaaag tacacaggag atttagaaaa 37320
cttcgtagca aaaaaaagag tgtaaagtat ctcattaata gtttttgtgg gctggacacg 37380
gtggctcaag cctatactct tggcacattg ggaggctgag atgcatgagt ctaggagttt 37440
gagaccagcc tgggcaacac agtaggaccc cgtctctaca aaaataatca gccagatgtg 37500
gtgcgcatct gtagtcccag ttacttgaga ggctgaggtg ggaggatcgt ttgagctggg 37560
aagttgaggc tacagtgagc tgtgattgaa ccactgcact ccagcctggg tgacagagtg 37620
cctgtctcca aaaaataaat aaataaataa taatatgttt tgtatgttca tatgttgcaa 37680
taacattttg gatatattaa atgaaataaa atacattaaa attaatttca cctgtttctt 37740
ttcttttctt tttttttttt ttttttgaga tggagtctcg ctatgtcatc aggctggagt 37800
gcagtggcac gatctcggct cactgcaacc tcctcctcct gggttcaagc gattcttctg 37860
cctcagcctc cctagtagct gggattaaag gcatgtgcca ccacacccag ctaatttttg 37920
tatttttagt agagacgggg tttcaccata ttggccagga tggtctcgat ctcctgacct 37980
catgatccgc ctgccttggc ctcccaaagt tctgggatta caggcgtgag ccactgcacc 38040
cagcctcttt taacttttta agtatggcta ccagaaaatt taaaatgcat gtgtggcctg 38100
tattctattt ctgttggatg ctgctgcctt agattattaa ttattcaatg taaagactgc 38160
tgggaggtac tacctgcact tccctgaata tatgcttgag agctccacca gccgtcttca 38220
cagtagcaag aggggtattc tgagtctgtc cccccaaagag ggagggagaa gtgcagccct 38280
ctcaggttct gtcagaaaac ctgatcccag gccaggcgtg gtagcttacg cctgtaatcc 38340
cagcactttg ggaggttgag gcaggaggat tgcttaagcc caggagttcg agaccagcct 38400
gggcaacaca gtgaagaccc tatctctaca aaaatttttt taaaaaaatt agccaggtgc 38460
agcaatgctg cctgtactcc cagctgcttg ggaggctgag gtaggaggat tgcctgagcc 38520
caggagttag aggttgcagg agttagaggt tccacgatcg cacctttcat tccgttacat 38580
ttgctgcctt gagaacagaa gacctgctgg ttttgttgcc agtttgctca gtcattttta 38640
tgaaaaagcc agtgctaact aggtgcttct tcgtgccttc tctgagaatc aagaactcta 38700
gtatgtttgc gtgtgttcag tctctcatta aatgttctca ctatcccaga gaaccatctc 38760
attggacctt ggtctgtaca taccttcatc tttggctctg acttgtaatt atttttagaa 38820
cttctctttt tttttttttg gagacagagt tttgctctag ttgccagact ggaatgcagt 38880
ggcacgatct cagctcacct caacctctgc cttccaggtt caagcaattc tcctgcctca 38940
acctcttgag tagctgtaat tacaggcatg tgccaccacg cctggctaat tttgtgtttt 39000

tagtagagac agggtttctc caagttggtc aggctggtct caaactcccg acctcaggtg 39060
atctgcccgc cttggcctcc caaagtgctg ggattacagg cgtaagccac tgcgcctggc 39120
ctaattttag aacttgttaa aacaacttgg cctctattga tatttccatg acccatgcta 39180
ttcagaaaga ggattacagg taattagctg gctgggtttc tcataccaga gcatttcact 39240
gggatgttcc tgaacctggg acaactttta tgcctggcat ttttctttcc ttctctgttg 39300
tcccagacta agcaattttt aaaatagtta ttatttgttg agtaggagaa tctcaggcag 39360
atcttcctgg atcctcattt atacttttaa acctgtagtc ttggaattag tgctctgtcc 39420
cccaacccca aacatccaat ttctacattt tggctacagt acaggtttac tgtgtataac 39480
taaaagggct gtggaggaga aagaaaggaa ccgacatttg ttgggcatct gttatgtgcc 39540
atgcactgag ctggatgctg taggaatatc tcaatacctc tgaggagtgg gaattattat 39600
ctctatttta tagacaaggg aatagaaatc tgggagttaa gtaatttttt aatttcacac 39660
acttctggta gataatggat tctagaacct ggcataatag ccacttgtca tcccagtgta 39720
aaagagatgt gtggccagat ggggtggctc acatatgtaa tcccagcact ttgggaagcc 39780
gaggcaggag gatgacttga gcccaggagt tcaagaccag cctgggcatg ttttgtttgt 39840
ctcacgaaac atttttttaaa aaatgagtgt ggcatggtgt tgtgtgccta tagtcccagc 39900
tcctcgggag gctgaggtgg gaggatctct tgagcccatg atcatgccat tgcactctag 39960
cctgggccac agagcaagac tctgtcttca aaaaataata aaaaggagct gtgattatcc 40020
caaggtgggg attgtgaatg tgtttgtatt gttctaaact gggagaaaca ggctgggtgt 40080
gttggcttat gcctgtaatc tcagcacttt gggaggccaa ggtgggagga tcacttgagt 40140
ccaggagttc aaggccaccc tgggcaacag gcaaaaaata gagaccccat ctctattttt 40200
taaaaataaa ataaactggg agaaagaagc agggtcctcc ccagagcatc tttatcccta 40260
gtcacagacc tgacacctgt gttgggcaat ggctacttct agattgttta cccctactgg 40320
gacttgtggt gaacatatgc acactttggt ttacagttgg gacccctgat tttagcagga 40380
tggcccaatg gaatcagcta cagcagcttg acacacggta cctggagcag ctccatcagc 40440
tctacagtga cagcttccca atggagctgc ggcagtttct ggccccttgg attgagagtc 40500
aagattggta agtccttctt aagtgactct ccaaattgtt aggtttcagt ttgagtcaag 40560
agacatgaac tcttaatgtc atgccttgct gttccattaa aaaatgtatg ggtacaggtg 40620
atggggaaaa tgagatcagg agataaaggg gcaccctttg gtcttgtaaa gccttttta 40680
tcttagaagg gcatgtgggc aactgtcttt gacacattga aaccgcctgt atggtggtgg 40740
atgtcttgaa ggttgatttg gacctcattt acttgggcag atcctctata tattctgata 40800
atccagtgat gtggtagaca tattttttct ctgaatgtga attctgtcat agctagaact 40860
ttgggttgat acttgtaatt cccctttagt taaaggaagg agccacaggg gtgtattagt 40920
ctgttctcaa tttgctataa agaaataccT gagactgggt aatttataag aaaagaggtt 40980
taatcggctc atagttctgc aggctatata ggaagcatag cagcatctgc tgctggggag 41040
gcctcagcaa gcttccaatc atggcggaag gcagagaggg agcaggcagg tcacatggcc 41100
acagcaagag caagagagca agggggaggt gccacacact tttaaactat cagatctcac 41160
aagaactcac tgtctcgagg acagtatcaa cagggatggt attaaaccat tcatgagaaa 41220
cccaccccca tgatccagtc accttccacc aggcccccacc tcaaacagtg ggggttacat 41280
ttcagtatga gatttgggca gggatgtaga tccaaactag atcacaggat aagggaagta 41340
gattccattc atagagcaga taatggcaca gatgtccagc aactattttc ttcactttaa 41400
tatgctcagg ctcactactg attttggttt aattcaggcc agtgttaata tgacctggtt 41460
tttccagaat gcatactctg atttggtgaa gggccaggag gtgattcaca gatgttggag 41520
ataggccatc ccagcctggg attacttatt tgtactaata aatctgacca gagttaattg 41580
agggtttaaa gcaaaacagc atatctgtct actttgctca aatattttac aaatacaaca 41640
gattatgaga gtgggtaata atatctggaa taattgtttt tttgttttgt ggtttttttt 41700
tttttttttt tgagatggag tctggctgta gcccaggctg gagtgcagtg gtacagtctc 41760
ggctcactgc acctctgcct cttggattca agcgattctc ccgcctcagc ctcccgagta 41820
gctgggatta caggtgccca ccaccacacc tggctaattt tttattttta gtagagacag 41880
cgtttcacca tgttggccag gttggtctgg aactcctgac ctcaggtgat ccgcctgcct 41940
cagcctccca aagtgctggg attacaggca tgagccacca tgcctggcct ggaataattg 42000
ttaataatta ttacattgat ggcattttat tgctgagcaa gaagaatcta acatgatgaa 42060
tgggttatag catcaggttt gctttgtttt tttgtttttt tcctctttct tgatggtgat 42120
ttctgtgttt gtgtgtatgc gtcggcttca gagccattct ttatcattct tccttttcct 42180
agggcatatg cggccagcaa agaatcacat gccactttgg tgtttcataa tctcctggga 42240
gagattgacc agcagtatag ccgcttcctg caagagtcga atgttctcta tcagcacaat 42300
ctacgaagaa tcaagcagtt tcttcaggta tgatgagaaa ctgaggacaa ggagaaacag 42360
gacccgcaga gtcgggtgtt agtgttcttt cctggaagca tctcttttct catttggcta 42420
agtaacgaga atctatcttg tattttcaat cacaggagaa gtaattagcc ctttctcaaa 42480
gctctgtata cttacccgtg agcatcatta cctgagaatc acttctcttg tcacagttga 42540
agtaataaag tgattgttat gttaatcata catgttagca tgttaacgcg gtccactgat 42600
aggaagatga ctctcactgt tacatgttaa atgtttgacc ataatgggat acttcttgac 42660
taagtcagta gcttccctgc aagaccagga tagtatactg tgtaaagact cagacaaggc 42720
caggcatggt ggctcacgcc tgtaatccca acaccttagg aggttgaggt gggaggattg 42780
cttgagcctg ggagttttga gaccagcttg ggcaacataa caagacacca tctctacaga 42840
aattttttttt aaaaactagc tgattgtggt ggcatgcacc tgtagtccca gctactcaga 42900

aggctgaggt gagaaaattg tttgagcctg ggaggtcgaa gctgcaataa gccgtgattg 42960
cgccactgca ctccagcctg gcggacagag tgagagccag tctcaaaaaa aaaaaaaaaa 43020
gactcaggct aatgtgcctt ctgttacaga aatagtaacg acctcccctt cgcccccccgc 43080
cgacagagag ccttcaccca ggctctgaag cctttgttcc gttgtttcct agaataaatg 43140
ctttccttga tgaatacatt agttttaagg tgccacagtt cagtccacat ctccatggtc 43200
tgctgctgat ttttattctc tttctctcct acttatagag caggtatctt gagaagccaa 43260
tggagattgc ccggattgtg gcccggtgcc tgtgggaaga atcacgcctt ctacagactg 43320
cagccactgc ggcccaggtg agacctgaga caaaacaaat ccctggtctg ggaggaatgg 43380
aaaatcaaac aactttataa tgagataaat tattagatct actaaaaaag aaggaaaaga 43440
aattaaatag atcaataatc ataaaaatac attgaaaaac tctaaaaaaa aagaaagttc 43500
cacccccccaa aatacattga aaactctaa aaaaaagaaa gttccaccaa aagaatccaa 43560
cagacccaat ggtttaaaag ttttgttttg ttctgacaaa ttttctttgt ttttcttttt 43620
ttttttttct gagacagagt tttgctcttg ttacccaggc tagagtgcaa tggcgcgatc 43680
ttggctcact gcaacctcca cctccagggt tcaagtgatt ctcctgcctc agcctcaaga 43740
gtagctggga ttataggcgt gtgccaccac acccagctaa ttttgtattt ttagtagaga 43800
cggggtttct tcatgttggt caggctggtc tcgaactcct gacctcaggt gatccgcccg 43860
cctcagcctc ccacagtgct gggattacag gcgtgagcca ctgtgcccgg cctgttctga 43920
caaactttca tagtacagat tattccaata tcattcaaac ttttccaaag tataggaaaa 43980
caagggatgt tttcagctta ttttatgagg ctggaaaaat cctcatatca aaacctaaaa 44040
aacagccagg tgtagtagct cacgcctgta atcccagcac tttgggaggc tgagacgggc 44100
agattgcctg agcctcagga gttcgagacc agctggggca atgtagcgag acctcatctc 44160
tctttttttt ttttttgag acagagtctc tctctgtcgt ccaggctgga gtgcagtggt 44220
gccatcttag ctcactgcaa cctccgcctc ccaggttcaa gcgattctct tgcctcagcc 44280
tcccgactag ctgggactac aggtgtgtgc caccaagcct ggctaatttt ttgtattttt 44340
tttagtagag atggggtttc accttgttag gcaggatggt cttgatctcc tgacttcatg 44400
atccaccggc cacagcctcc caaagtgctg ggattatagg catgagccac cacgcccagc 44460
cttttttttt tttttgagac agagtcttgc tctgttgcca ggctggagtg cagtggcgtg 44520
atctcagctc actgcaactt ctgcctccca ggttcaagct attcccctgc ctcagcctcc 44580
caagtagctg ggactacagg cgcgcgccac cacacccagc taattttttg tgtttttagt 44640
agagatgggg tttcactgtg ttagccagga tggtctcgat ctcttgacct cgtgatccgc 44700
ccgcctcggc ctcccaaagt gctgggatta caggcgtgag caaccgcacc tggcttaatt 44760
aaggatcttt ctaaacacaa gaaagaatat ttatcagaaa ccaaagggag catgatgcac 44820
agtggtgaaa cactattctc agtaaaaaca gcaaaagata aggatgtctt ttaccattga 44880
tacttttctg agggatccag cctatgcaaa aagaaaaaga aatgagggta caaatattgg 44940
aaagcaaggg acagaactct tattatttac agatagatag gtcttcctcg aagatccaag 45000
agaaacaaaa ctaacaataa caattggaac tagcaaggtt tagaaaggcc attgtataca 45060
agataaatat ttttagaatc tgcagttccc ctaatcagta gcagcagtaa cctgttagaa 45120
gatgtaatga aagtaaagat ctgggccagg cacgatgtct cacgcctgta atccaagcac 45180
tttgggaggc caaggtgggc agatcatgag gtcaagagat tgagaccatc ctggccaaca 45240
tgatgaaacc ccatctctac taaaaataca aaaattagct gggtgtggtg gtacgcgcct 45300
gtagtcccag ctactcggga agctggggca ggagaatcgc ttgaacctgg gaggcggagg 45360
ttgtagtgaa ccaagattgc gccactgcac tcctgggcga cagagcgaga ctccgactga 45420
aaaaaaaaaa aaaaaaaaaa gaaagatctg attcatagta gtaaaactaa atgtatgcaa 45480
tttgcatata ctattggtat gtatgggaaa atatctggaa acacatatac taaatcatta 45540
aagtagtcgg tcataggaga ctttttttact ttctgtgagg ggttttaccg tctttaatat 45600
cctataatca gggacatttt ttctttttct ccgtgacccc ctgcttttta aaaaattgtg 45660
gtgaaataca cataacatta catttcaaat ttacctttgt aacctttgtt tttttttttt 45720
tttttgaga cagtctcact ctgtcaccca ggctggagtg cagtggtgtg atcacagctc 45780
actgcagcct caaccacctg ggccctagcg atcctcctgc ctcagcctta tgagtagctg 45840
ggactacagg cacatgccac catgcccagc taattttttt tttttttttt tttggtagag 45900
atgggctctt gccatgtttc ccaggctggt gttgaactcc tgggctcatc aactgatgag 45960
aaagagctct ccaggcagaa agaagatcat gttcaaagac agaaacagaa atgtgtattc 46020
ttgggagaag tgtagaaagt tcagcatctg attgggtcgg ggaagacaag ctagtcaagg 46080
ccacatgatg ttttaattag tcatgcctaa cagtggggcc ctggaagagc agtttaccac 46140
aaggggccaa ctgcttcggt ttgaacccgc agccctgcca cttgctctgt aaccttaagt 46200
aaacaatttt tactctctct gttcctccaa tgggagtgat aacaatacct tcttcataga 46260
attaattcat acatgtaaaa tgcttagaac agtatctgac acataaatgc aaaataattt 46320
aactgctttc tgctgctgct gacatcacta tcatcaccct caccattact gtaggaaatg 46380
gggacccagt gaagaatttt tttttttct tttgagacag agtctcactc tgtcacccag 46440
gccggagtgc agtgacgcga tttcggccca ctgcaacctc tgcctctcag gttcaagcga 46500
ttctcatgtc tcagcttccc aagtagctgg gattacaggc atgagccacc acactgggct 46560
aattttttgt atttttagtg agatagggtt tcaccatatt ggccaggctg gtctcaaact 46620
cctgacctca ggtgatccat ccacctcggc ctcccaaagt gctgggatta caggcataag 46680
ccactgtgtc cggccctagt gaggaatttt aagcagaaaa ctgatatgct caggtgtgag 46740
cgaggtggta ggtaacactt actgtgcagt gccctgtagc ccaagaggtt agcacacagg 46800

catttgctca ggcagcacta ggattttctg ctgtggaaaa cctttgtatt ttatcctgct 46860
ccacaagata aaaataagtg gtttaagtca atttggatag aggctccaac ttaccatggg 46920
aggtaggaaa gccaaagtta tcccaaggat gttttcaatc gtacggatta ggggtctgca 46980
aactgtgagc gtggcccaaa tccagcctgc tgcttgtttt tgtaaatgag gtttttttcgg 47040
aacccagcca cactcattta tttatgcatt atctgtggct gctttggtgc tgcagtggca 47100
gggctatttg tggcagggac tgtatgaccc aggaaaccaa aaatatttac cctctgtccc 47160
ttagagaaaa agtttgcaac ccctgatata aagctataag ttggttattt gtggcctcaa 47220
cccaggcctc actgctattt tttctgttta caatacctgg catgctctta agtgtctaga 47280
attggttaaa gatagaagag tggatgtaat ccctgctacc aagggctgtc aggctagttg 47340
ggattataag tacacaaaca ctcaaagtga gaaaaacaca gaaaaggatg tgtgtcattt 47400
tgtctaagga agttgaataa gatttctcag gaaaagaaac atttgaactg aatttgaagg 47460
tgagtgagtt caggtgtgtt tgggctgaag cccaggccat gctgagtgga tagcgggtgg 47520
gaagagagtg tggaaacaca ctgcatgcag ggaagagttg ggagtctggg gtgaccaagg 47580
cacagggagg gaaagttgaa gttatcaatt gtgtgaaaca gctttctgtg ttggcctgag 47640
atgtttatag ctggaagcag tggggagcca atacagtttt ttacgaaggt attagaggtg 47700
ggtttctgtg ggtgatcgtt aatcatgttt tctcccttta agtgtagtcc tgcttgagaa 47760
atagacatga gaaaggaatg aaggttaaaa catcagctgt attgttggta aaactagaat 47820
ggaaagtgtg gcttgagctg gtaaccatag gggctttcca atgcctgtgc cctgagttag 47880
atcttggggt agagagactg gatgtgcaga gcagcacccc caccccaccc cagccatcca 47940
tatggagctt cagctgccat agaccaacaa ggcagaggga taggcctcta gacctgcttc 48000
tagaaaccag gctgctgctc ttgcttatgg tgggccctag gaaggcaaga gtgagaggag 48060
ggaggcacca gcttaggtgc tgggttcttt gaagatctgt gtgtacacag agtctttctc 48120
tccatcttac caatcagatg agtcactgtc actgtgggaa gaagtagggg catgggtcac 48180
cttcccaaaa cttctaagaa gtttgtattc tgtgggcttg gatagggacc atgggaaagg 48240
aagagaatgg ttgcccataa aactggctgt agtgtggcct caaacttctg gacttaaatg 48300
atcctcccac ctcagcctcc caagtagcta gaactacagg tatatgccac catgcccagc 48360
tagttaaaaa aaaatttttt tttttttttg gttgagatga ggtctctttc tatgttccct 48420
gggccggtct caaactccca gcctcaagtg atcctcctgc cttggtttcc caaagtgcta 48480
ggattatagg tgggagctac catgcctagc ccaagcctgt aatttttttt tttttttttt 48540
gagatggagt ttcacttttg ttgctcaggc tggagtgcat ggcgcagtct tggctcacca 48600
caacctccac ctcccgggtt caggcgattc tccttcctca gcctcccgag tagctgggat 48660
tacaggcatg caccaccaag ctcagctaac tttgtatttt tagtagagat gggtttctcc 48720
gtgtcggtca ggctggtctc aaactcctga cctcaggtaa tctgcccacc ttggcctccc 48780
aaagtgctgg gattacaggc atcagccacc gcacctggca cgaacctgta atttttaagt 48840
ttcatatgct atttattttt tgttatttct ttaattcatt cattcattta ttcattcgag 48900
atggggcctc actatgttga ctaggctagt tttgaactcc tggcctcaag cagtcctccc 48960
acttcagcct tcccaagtgc tgatattata ggtgtgagct gctacatcca gccttctttc 49020
ttctttttct ttttccatgt gctatttgac attttccaag gtaccagcct ccccttctcc 49080
ccaagataat atcttttaat atggaatttc atccctaggg caggactttt tttttattat 49140
ccctcagaaa tatactggac accacgttta agtagacatc caacatctgc tgtcataaat 49200
tgttttgaat tttttgacat acttgcccat gaggtttttg aaggcataga ccatgtctta 49260
gctgaacatg tggtctctta gtgccataaa gggggtttat ggtatgacct gtgtagtgtc 49320
acctgtgtag tgacagcacc actgcctctg tttcccttcc tcttgtgatg gcagcagcgt 49380
ctcaagccaa acaagaaggg tagttagggt gggatggaag ctgggtagag gtattcctct 49440
ccccatagtt ctgtgttcac atgtgcattg acctcctttt tggcagcaag ggggccaggc 49500
caaccacccc acagcagccg tggtgacgga gaagcagcag atgctggagc agcaccttca 49560
ggatgtccgg aagagagtgc aggtgatgca agttacaagc ctcgggcagg gagctttcat 49620
taattttttt tttttttttt gagacagggt cttgctctgc cactcaggct gggctgcagt 49680
ggcatgatca cagctcactg cagcctcgac ctctcaggcc caagcgatcc tcctacctca 49740
tcctcccaag tagccgggac cacaggcatg caccaccacg cccagctaat taaaaaaaaa 49800
aaatttgtag agatgggggt ctccctgtgt tgtccaggct gatcatgaac tcctgggctc 49860
aagtgatcct cccaactcag cctctcaaag tgctggcatt acaggcgtga gccactgcac 49920
ctggccaaca gggagccttc tcttggggat actgcctgca ggtcctgcat gtatcttttt 49980
tgaggttttg gcttcatttg aattctcctc agaaacttta tattttctgt tcccaaggaa 50040
atctttcttt acttctgttt ttttgtttgc ttattttaaa caggatctag aacagaaaat 50100
gaaagtggta gagaatctcc aggatgactt tgatttcaac tataaaaccc tcaagagtca 50160
aggaggcaag tgaatattag agatgttaaa atctctagaa agtgagtttg tgttgttgag 50220
ttgaaagact catttgtctt aactctgttt agatcttaag gcgggcgggg cgcaagggag 50280
gtacgggtcc tcaaaggagc ctggtcatta aggacaggag tattccctca ggtccaggag 50340
tattccctca ggtccaggag tattccctca ggtcaaggag tattccctca ggtcaaggag 50400
tattccctca ggtccaggag tattccctca ggtccaggag tattccctca ggtccaggag 50460
tattccctca ggtccaggag tattccctca ggtccaggag tattccctca ggtcaaggag 50520
tattccctca ggtccaggag tattccctca ggtccaggag tattccctca ggtccaggag 50580
tattccctca ggtccaggag tattccctca ggtccaggag tattccctca ggtcaaggag 50640
tattccctca ggtccaggag tattccctca ggtccaggag tattccctca ggtccaggag 50700

tattccctca ggtcaaggag tattccctca ggtccaggag tattccctca ggtccaggag 50760
tattccctca ggtccaggag tattccctca ggtccaggag tattccctca ggtccaggag 50820
tattccctca ggtccaggag tattccctca ggtccaggag tattccctca ggtccaggag 50880
tattccctca ggtcaaggag tattccctca ggtccaggag tattccctca ggtccaggag 50940
tattccctca ggtccaggag tattccctca ggtcaaggag tattccctca ggtcaaggag 51000
tattccctca ggtcaaggag ttttttcttc cttcgcagac atgcaagatc tgaatggaaa 51060
caaccagtca gtgaccaggc agaagatgca gcagctggaa cagatgctca ctgcgctgga 51120
ccagatgcgg agagtaaggg cataggtcgg accacttccc ccatgtgtct cgctcacttg 51180
cgggatttca gcgtcttgtg gcagaacttg cttggtttct aagaagttcc tgctctggag 51240
ttgactaaag aatgtggtta gagacagtct gaggaaatgt tttctgactt tgttttggtt 51300
tccaaccaga gcatcgtgag tgagctggcg gggcttttgt cagcgatgga gtacgtgcag 51360
aaaactctca cggacgagga gctggctgac tggaagaggc ggcaacagat tgcctgcatt 51420
ggaggcccgc ccaacatctg cctagatcgg ctagaaaact ggtaaaggat gaaagaagct 51480
tttcctttct ttctcgaaag ctagattgaa ttctgatctt aactgcaggc ccacagaatt 51540
ggtactatat ctccaacgtg gggacttttc catattcaaa tttagcccaa gaattaaagt 51600
ttttacttta tttcggccag gcgctgtggc tcacacctgt aatcccagca ctttgggaga 51660
ccaagatggg cggatcactt gaggtcagga gtttgagacc agcctggcca acatggtgaa 51720
aacacatctc tactaaaaac ataaaaaaat tagccgggcg tggtggtgcg cacctgtagt 51780
cccagctact ctgggcggct gaggcaggag aatcacttga acctgggata tggaagttgc 51840
agtgagcgga gatcttacta ccgcacacca accagcctgg gagacagagt gagactccat 51900
ctcaaaaaaa taaaaataaa ataaagtttt tactttattt ggagaaactt tgttttaaaa 51960
aatgtattta tattattata ttttaagtat attttactta ataattcaat taaggctttt 52020
ggtttaactg tatttaacag atagacaaac cttttaattt tagttatttt agtaatctaa 52080
aatgacacat gcccttttta agggaaaaaa ttcaaataca gaaaattaat caagagaaga 52140
aaaaattttt aaatgaaatc atcagcagta ctagtagtta aaatttagtt gatgctcaat 52200
ctagacatct gtcattatgt atatacacat tatgtatata cacataaaga tagaaattta 52260
tacagtttat attaggatca tttttttttc tttttttgga gtcagggtct cactgtgtta 52320
cccagtctag agtacagtta tgcagtcatg gctcactgga gccttgacct cctgggctca 52380
ggcagtcttc ccaccttagc cttctcagta gctgggacta caggcatgca ccaccacacc 52440
tggctaattt ttaaattttt tatagagaca gggtcttact ttgttgcctg ggctggtctc 52500
aaattcctgg gctcaaggga tcatcccact tcggcctctc aaaagctctg gaattataga 52560
tgtgagctgc cgtgcccagc ccaggatctt cctttatatg cttttctgta atttgcactt 52620
ttaccttcat ccagcatatc ttactgcaac ccttcctgtg caaggcccta tagtgagcat 52680
gttgcaccag cttgccttag gagaaacttg agatacagag cctgcactgg aaatttagcg 52740
caactctaca tgagaatgcc tgtctattca tatcctcact aaccctgagt gttgttaatt 52800
tactgaaagc agttttaaat gcttcctgac cagggaacga agaagcttaa gttctgggaa 52860
tgggaggata gaagtgccag aaaagagctc aggagttcag aaatccctgc agcggtcccc 52920
ctccctctcc tttcactttc tgtctttctg gtcttttggt ctttgttaca ctagtgataa 52980
accatcaaag aatgatggaa tgatgctaac ttctctcttt ttttaatttt tttgagacag 53040
agtctcactc tgtcacccag gttagagtgc agtggcatga tcttggctta ctgcaacctc 53100
ctcctcccag gttcaagcga ttcttagtca caaccttcca agtagctggg attacaggcc 53160
catgccacca tgcctggcta tttttttgta ttttagtaga tcgacctgcc tcggcctctc 53220
aaatttttgg gattacaggt gtcagccact gcacctggcc taatatctct attcttggag 53280
atagatttaa tgagcttttt ctccctctct attcacttat tccttgtgca tgttatcaat 53340
attttgaaac ataatgtcat gtcctttgat cagttgaagg ctgacattga aaaggcttat 53400
ggggattggg tgttgtggct cacgcctgta aatcccaatg ctttgggagg cagagtcggg 53460
aggatcactt gaacccagga gtttgagacc agcctgggca acaaagtgag atcccatccc 53520
tacaaaaatt taaaaaacta gacatgtgcc attacacttc agcctgggtg acagagtgag 53580
actccatctc aaaaaactaa actaaactaa acaggcatgg tggcacacac ctatagttct 53640
agctactcag gaagctgagg taggaggatc actcatgtcc aggagttgga ggaggcagtg 53700
agctatgatc atgccattgc actgcactag gccacagagt gggaccctgt ctcaaaaaaa 53760
aaaaaagaaa gaaagaaaag aaagggctca tgtagttcaa gcccttctct tcatgcaagg 53820
ggatgctaag gcccatgatg gtgaagggcc tggcaaagct tgcacagata gtgtgtgaca 53880
gagctggctc aaacccatct ttgggagctg tctaatctct ttttctgagt ctttatgttc 53940
atagacaagt taggatgagt aaagtaagtg ctaaattcca tatttcgtgt tctgcatatc 54000
tgggctcaga tgcttgtcat tttccagtga taactccatc aatgcctcct agtggtataa 54060
atttaatac ttcttgtgtg cccagccccc tcttagaaat ttgagatttt aggaagggac 54120
tagtaataaa aggtaaaata aattattttc tggccaggca tggtggctca cacctgtaat 54180
gccagtactt cgggaggtcg aggcagatgg atcacctgag gtcaggagtt caagaccagc 54240
ctggccaaca aggcaaaatc ccatctctac taaaaatgca aaaattatcc gggagtggtg 54300
gtgggtgcct gtaatcccag ctacttggga ggctgaggca ggagaatcac ttgaacttgg 54360
gaggcggagg ttgcagtgag ctgagactgt gccactgcac tccagcctgg gcaacagagt 54420
aagactctat ctcaaaaaaa aaaaaaaaaa aaaaaaggcc aggcgcagtg gcttacacct 54480
gtaatctctc aggaggctca ggcaggagaa tcacttgaac ccgggaaatg gaggttgcag 54540
tgagccgaga ttgcaccact gcactccagc tcaaaaaata aataaataaa taaattattt 54600

tctttttta tttatttttt cagcatccac ccaacatggt gaaaaattcc tcttttctta 54660
atgtcactga actgtaaact taagatgaaa aattgtaaat ttcatgctat atatatttca 54720
ccacaataaa aaaattcctt gttcttattg tagtggtctc catgtcttca gtatttcctt 54780
ccccttctcc atctcacctg tatacattca ctttggtaat tagcatcttt cttaatttat 54840
tggcaggata acgtcattag cagaatctca acttcagacc cgtcaacaaa ttaagaaact 54900
ggaggagttg cagcaaaaag tttcctacaa aggggacccc attgtacagc accggccgat 54960
gctggaggag agaatcgtgg agctgtttag aaacttaatg aaaaggtaat ttagcatcct 55020
tgtccctttc cctcatctaa aaaataccta aagactcacg tggtagagtg agaggcgggc 55080
tgacttctgg tcatggccgt ggcgcgtgag cccatcttct ctttcctcag tgcctttgtg 55140
gtggagcggc agccctgcat gcccatgcat cctgaccggc ccctcgtcat caagaccggc 55200
gtccagttca ctactaaagt caggtaggcc atgccacttc catttccagt agagatttta 55260
ctgagggaca ctgttagggt gagggtagag ttggtggcca gggtcattct ttccaggtgt 55320
ggtgtcacag gcagtacact gttgcggggt tgaaatttgt tgccatacta tctgcttgct 55380
ctctgattct gatgtcaaaa gcaaaagagc agtcatcttt ttgaaggtac ctgggcatat 55440
tcctatgatt gtagacctgg agtctcaggc cacagcttct ccttctgccc aagggacaaa 55500
ataatgtcat ctattttctg ttctttgagg ctactcttcc ctgtggattt taagggaaag 55560
agtaaggctt agtgatgggg aagctgagag gccccagggc aggtgggtgg tgggcctgta 55620
gggtgaggtg ttactttcac actcaagtca gaacaggtgt gctggggttt tgaccttctg 55680
cagcaaaatt tccctcctca gaaacttagt atggtgttcg gtttcaggat taatagaaca 55740
aaatgccagc tgcacagcat gtgttcctgt aatatttttc attatatggc tttgattatc 55800
cttttgtgaa tctctcacaa ctttaagttg ttagttctta gatgttttct cagtaccttt 55860
ggcttgaagg agtgatactc atcttttgtt tttgtttgag acagggtctc actctcaccc 55920
aggctggtgt gcagtggcat gatctcagct cactgcaacc tccatctccc aggttcaagt 55980
gattcttgtg cctcagcctc ctgagtaact gggaatagag gtgcgtgcca ccacacccgg 56040
ctaatttttt ttttttgag acagagtctc gctctttcgg ccaggccaga gtgcgtgttg 56100
caatctcaac tcactgcaac ctccacctcc caggttcaag cgattctcct gccttagcct 56160
ccctgagtag ctggaccggc acactccacc atgcccggct aatttttgta tttttagtag 56220
agacagggtt tctccatgtt ggccaggctg gtctcaaaac tcctgacctc agtaatccac 56280
ccacccccggc ctccaaaagt gctgggatta cagatgtgag ccaccacgct cggccttttt 56340
tttttttttt tttttttga gatggagtct ttctctatca cccaggctag agtgctgagg 56400
tgtgatctcg gatcactgca gcctctgcct cctgggttca agtgattctc ctgcctcagc 56460
ctcccaagta gctgggatta caggtacctg ccaccatgcc cggctgattt ttgtattttt 56520
agtagagacg gggtttcacc atcttggcca ggctggtctc gaactcctga ccttgtgatc 56580
cacctgcctt ggcctcccaa agtgctggga ttacaggtgt gagtcaccgc acccagccct 56640
attttaattt ttttaaagag agagataggg gccaggcacg gtggctctcg cctgtaatcc 56700
cagcactttg ggaggccaag gtgggtggat cacctgaggt cgggagttcg agaccatcct 56760
gaccaacatg gagaaactct gtctctacta aaaatacaaa attagctgag cgtggtggcg 56820
cgcgcctgta atcccagcta cttgagaggc tgaggcagga gaatcacttg aacccaggag 56880
gcggaggttg cggtgaacgg agattgcgcc attgcactcc agcctgggta acgagagaaa 56940
ctgtctcaaa aaaaaaaaaa gagaaagaga gataggatct cgctctgtca tctaggctag 57000
agtgcagtgg catgatcata gatcactgta gccttgaact cctgggcaca agtgatcctc 57060
ttgcctcagc ctcccgagta actgcgacta caggtacatg ctaccacacc ccgctaattt 57120
ttaaattttt tatagatgtg ggctctcact ttgttgccca gactgttatg gaactcctgg 57180
gctcaaggga tcctcccagc ttggcctccc acagtgctga gattatagat gtgagcctgt 57240
aattatagac agcttggcct atttacctgt tggaaatgaa gaattatgaa ttttacattt 57300
cttcaagaaa aggttatggg agagttactg actttttttc cttggatttt ttctttttaa 57360
ataggttgct ggtcaaattc cctgagttga attatcagct taaaattaaa gtgtgcattg 57420
acaagtaagt actcctatct tagctctgtt tttcaaatga ggaatagaaa aatgagaact 57480
ttgacagaca tcatttgaac tagagactct gtctttattc agagatcttc attttgtgga 57540
caaaagtttt caaaagcctt ggggtgcatt gtcatttacg tgtctgaaca aagccacaaa 57600
gctgggggta cagatttgat ttgtggttgc tattgtgaca accagtccct cttttccttg 57660
tttagttttt tacttgtaca tgtcattcat gcatattata tataagactg agatcatgtg 57720
ttaattaacg actgggatac gttctgcaaa atgtatcatt aggcaatttt gttgtgcaaa 57780
tgttgtagag tatatagtcc ttacacaaac ctgggtggca gaacctactg cacacctacg 57840
ctatgtggca gagcctactg gtcgtaggct gtaaacctgt acagtatgtt actgtgctga 57900
ataccgtagg caattgtaac acatctcaat gaagtaggaa tttttcagct ccatgataat 57960
cttatgggac caccatcata tatgcatttt gttgttgacc gaaacgtcgt tatatattct 58020
ttccatacat agcatgtgga aagaatagat ctcttttttt taattgttcc acactttacc 58080
atataatgga atacgcaaaa tttcacaata cctttcagga tgtaaaatac atatacccctt 58140
tgacgacatt agaaaagaga aaatgtgggc cgggcgcggt ggctcatgcc tgtaatccca 58200
gcactttggg aggccgaggc gggcggatca cgaggtcagg agatcgagac catcctgggt 58260
aacacggtga aaccccgtct ctactaaaaa tacaaaaaaa ctagctgggc gtggtggcgg 58320
gcacctgtag tcccagctac tcaggaggct gaggcaggag aatggcatga acctgggagg 58380
tggagtttgc agtgagccaa gatcacacca ctgcactcca gcctgggcga cagagactcc 58440
atctcaaaaa aaaaaaaaaa gaaaagaaaa gagaaaatgt ggctgggcgc ggtggctcac 58500

gcctgtaatc tcagcacttt gggaggctga ggtgggcaga tcacctgagg tcgagagttc 58560
gaaaccagcc cgaccaacat ggagaaacct tgtctctact aaaaatacaa aattagccag 58620
gtgtgttggc gcatgcccgt aatcccagct acacgggagg ctgaggcagg agaatcactt 58680
gaactcagga ggtggaggtt gtggtgagcc gagatcacac cattgcactc cagcctgggc 58740
aacaagagcg aaactatctc aaaaaaaaaa aaagaaaaaa gaaaagataa aatgcattct 58800
tatttttagt tgatgtaatt atgtggaaat ttcatgagga tgcactggaa aataatgaaa 58860
taagggagtt gacgaaggtg gtaggtttaa taagtacata tgcaatatga aacataggtt 58920
ccccttccta tggggaggca accaactgtg cctgctacgc agaggtgtta tgttgcgctg 58980
atcaactgta actgaatagt ttaaagaaat gcccaggagc acagaggttt tttcatgaca 59040
gtaaataaca ggtggtcaaa gtaggctttt tgaagaaaca cagagcctat tttattaaca 59100
acagtctgtg ttcttacaga gactctgggg acgttgcagc tctcagaggg taagttcagc 59160
ctagaggctt ccttttgttc cgtttaacct aacttcatcc tccggctact tggtcaccta 59220
catagttgat tgttcccctg tgattcagat cccggaaatt taacattctg ggcacaaaca 59280
caaaagtgat gaacatggaa gaatccaaca acggcagcct ctctgcagaa ttcaaacact 59340
tggtatgtgg gaggagctcc ccttcacaaa gggcctctgg ctgccggaga gggctaggga 59400
gagcctcaca ggacacctgc ctttttcttt tcttacagac cctgagggag cagagatgtg 59460
ggaatggggg ccgagccaat tgtgatgtaa gttttgttgg ggatgaaaga caactggggt 59520
gttttccttg agggagagag gggtaaagat ccttcttaat ccccagaatt agaaacatca 59580
acctgttctt tcagctgtag ttattccaaa aagtcacttc aggccaaagt gacatgaaca 59640
gaagttccat gtgccatgga gctctctggc ttggaacatt tccgtgaata tctgggagtt 59700
ggctcctcct taaggagaag tggaaagtcc cttgctgagt tgttctccac acccatgtgg 59760
tataaagcag ctttccacct tgcctggggc tttccaaatt ccccatccag ctcctgcggc 59820
tgaccctgct tggctccatt tttagtgccc tgtttttctc tcccactgag gtgggataga 59880
gggtgtaaaa gcaacagatt tgagttaaac tttaaaataa atgaccacct tgcattagct 59940
tgcttaggaa aagagtacat aaaataaaat gaacaaacaa aaacccatct tgttctttat 60000
cccccttatt ttctgctttt cattgattca gattattgga ttcttattgt caagaataaa 60060
ctttaaacaa acaaacaaaa aaaggtaaat gtgacggaag gctagttttc agtcattttt 60120
aaaaattgtg atgccccgtt ctttttctta catttgtccc ctgaacaatt cttcctcttt 60180
aaaatgtagc agtcctagct gggcgtgctg gctcacaccc cgtactttgg gatgccaagg 60240
caggctggtc acttgaggtc aggagttcaa gaccagcctg gccaacatgg tgaaagcccg 60300
tctctactaa agatacaaaa attagctggg tgtggtggtg cacgcctgta gtcccagtta 60360
ctggggaggc tgaggcatga gaatcgcttg aacctgagag gtggagcttg cagtgagcca 60420
agatttgcc actgcactct agcctgggca acagagtgaa actctgtctc aaaaaaataa 60480
ataaaataaa atgtagcagt cctttttaaa aatgtggaat tttacttgac agtagagtga 60540
agtagcctgt atgcaatgat atgggaaaat gtacatgaca tattaagaaa aagcaaaatg 60600
taaaataatt tgaatagtat tattagtata tgtgttttaa aaatacacta tactcttatg 60660
tgtattcata tgtatattaa gaaattctgg aggaatatac cagcagtgct atgtgtatta 60720
gtgctgctgt tggtatccat ggctattcta gactgtctct gtgatatttg cattttaaac 60780
tgaatatatt acttttataa tcagaaaaat agtattaaaa atgaattata atttaatttc 60840
ttttttcttt tttttttttg agtcggagtc tcgttctatc ggattgcagt ggtgcgatct 60900
cagctcactg caacctctgc ctcctaggtt caagcgattc tcctgtctca gcctcccaag 60960
tagctgggac gataggtgca tgccaccacg cctggctaat ttttgcattt ttagtagaga 61020
cagggtttca ccatattggt caggctggtc ttgaactcct gacctcgtga tccacccatc 61080
tcggcctccg aaagtgctgg gattacaggc atgagccgct gtgcccagac tagaattcaa 61140
tttttgagaa ttcattgaca actcttactt aaaataaggt tgctgtactg atgtgagaca 61200
ttgttgtagt cagtttggaa aacaatttgg cagtataaaa atgaacatac ctgtaaacca 61260
acggtgccat tcccaggatt taatagcaga gaaatctttg catatatgtc ccaggagaca 61320
tatataaagt ggacatcagc ctgattataa gctctaaatg caacccaaat aaatacccat 61380
caacattaga atgaatacat tatttgtggt atagacacaa tggaatactc cgcagctgtg 61440
aaaaggaata cactgcagat acacataacc atgtggattc atttcacatc aagtgaaaag 61500
tgaatcccaa aagaattcat tggagtccat aagtgtaagg ttcacaaatg tcccaaacta 61560
aacaatacct gcattgctta gataaacaaa tatggtaaaa ctgtaaaaaa acaaaacaaa 61620
acaagacaaa aagggctagg aaatgataaa cccaaaagac aaaatagcag ttatttctga 61680
gggaggaggg aaggggatgg ggttggggaa gggcacccag agaattttag gagtgatgga 61740
cttttcctta aattgaatgg tgggttcata ttgttttgtt attctttgtg ccttacgtat 61800
tttacaaata accaattgga tctatgtaat attataatac aaactgagta aaggattagg 61860
ttgaggatca cagcattgga agttcttggt gttgaagaga gtaagtgccg agcaagttgt 61920
gtccctggca gtttgtttgt gaccacctgg tggcttaccc ttcttggtgt ggtgaggctt 61980
ggcatgtcac tttccttggc tgtggctgtt agtactgaat gccattctct ctgaggaaaa 62040
gtgtccttct ctttttttatt gattgactga ttgattgaga cagagtctca ctctgtcacc 62100
caggctggag tgcagtggcg tgatctcggc tcactgcatc ctctgcctcc tgggttcaag 62160
cgattctcct gcctcagcct cctgagtagc tgggactaca ggcgcccact accacaccca 62220
gctaattttt gtattcttag tagaaacggg gtttcaccaa attattggcc aggctggtct 62280
cgaactcctg accatgtgat ccacctgcct cggcctccca aaatgctggg attgtaggtg 62340
tgagccatca cgctcagcct ttttttttat ttaatttaat tttttttttaa gacagggtct 62400

cactctgtca ccccagctag agtgcagtgg cacaatcata gctcgctgca gcctccatct 62460
cctaggctca agccatcctc ccacctcagc ctctcgagta gctggggcta taggtgtgca 62520
ccaccacacc cagctaattt ttgtattttt tgcagagatg gagttttgct gtgctgctta 62580
gactggtctc gaactcctgg gctcaggcaa tcctcctgcc ttggcctccc aaagtgctgg 62640
gattacaggc atgagccacc acacctggcc taagagtgtc cttctcgtta ctgtaggctt 62700
ccctgattgt gactgaggag ctgcacctga tcacctttga gaccgaggtg tatcaccaag 62760
gcctcaagat tgacctagag gtaagttctg cagcagaatc ggtgagaggc tacgtacagg 62820
ggtgactcag gacaaaaact tccactggga tttttacaag agaaggtgga atgattactg 62880
tttgcttaac actgtgttta tttttgctta cttttctcca aaaaaatcct tggcatccca 62940
tctggcaata aagtcttgct tgaatgctta gaagatgtgt gtatattcag ctttcagcaa 63000
acttgatatg aaaatctcta tttagaaatt gattggccgg gcgcggtggc tcacgcctgt 63060
aatcccagca ctttgggagg ctgaggcggg tggatcacga ggtcaggagt tcgagaccag 63120
cctggccaac atgacgaaac cccgtctcta ctaaaataca aaaattagct gggtatggtg 63180
gcggacgcct ataatcccag ctactcggga ggctgaggca ggagaatcac ttgaacctgg 63240
gaggcagagg ttgcagtgag ctgagattgt gccattgcac tccagcctgg gtgacagagt 63300
gagactccgt ctcaaaaaaa aaaaaaagaa attagaactg actttataaa gtttgggcat 63360
aagagtctta gcagccagtg tgtttagtat acagaaaatt gtggcaatga cattctcctt 63420
tcccaacttt cttgattttt aaattaagat atacctagaa aagcaggaat cctggtcttt 63480
gattcctgag acctccctgt ttcatgtgaa gatacagctt caagtcttgg agaatgcctc 63540
caaggtctta aaaatgggga atctgtggat tgtgagtcaa gctttgagca agtcaggttt 63600
tacaagggac cggtatattc cgactgcagc ctgagttgtg tggccacgct gggcattctt 63660
tccactatga gtgctcactg agctgactca ctcacactcc tcgcctagag ttggcagcag 63720
gtgtggttta tggcatgtcc tttcattctg agccccgtga gatgcgggtg aagagatttc 63780
caaggctgtg agagcccctc tgcctcccca gctcagtccc cactccctcc gcagacccac 63840
tccttgccag ttgtggtgat ctccaacatc tgtcagatgc caaatgcctg ggcgtccatc 63900
ctgtggtaca acatgctgac caacaatccc aaggttagtg ccccctcctt ttagttggtg 63960
ccccgggatc tcttgcgact taggggtacc tagtatagac aatgagcacc atccctcatc 64020
taaacaagca aatgtgttct ttccaataga atgtaaactt ttttaccaag cccccaattg 64080
gaacctggga tcaagtggcc gaggtcctga gctggcagtt ctcctccacc accaagcgag 64140
gactgagcat cgagcagctg actacactgg cagagaaact cttgggtccg catttcaccc 64200
cttctccctc ccgcccaccc gcccagaaaa gggatccggc ccatagggct gttcatttgg 64260
gccatgtcta ctgagcatta ggccatgttt ctttcctgag caaggcgctg tgctggtgcc 64320
aggaaacagg ggagttgggg agttggggtg cagagacagt ttgcagtttt cagtcgaggt 64380
gatcattttt gaggtgggag gtagatttct tttctcctgg ttgctgtctc attcacccac 64440
tctatctaac tttagaagat cttttaagtg tgtgttggaa ggtggcacta aaggcttgac 64500
attccctgtc cattttttta ataaactata ggctagttgg tttttttttgt cttattttat 64560
ttatttattt atttttttga gacgagtctt gctctgttgc ccaggctgga gtgcagtagt 64620
gtgatctcgg ctcactgcaa cctccgcctt ctgggttcaa gcgattcttc tgcctcggcc 64680
tcccgagtag ctgagactac aggtgctcac caccacgccc agctaatttt tgtattttta 64740
gtagagacgg ggttttacca tgttggccag gatagtctcc gtctcttcac ctcgtgatcc 64800
gcccacctcg gcctcctaaa gtgctgggat tacaggcttg agccactgtg cccagcgtag 64860
gctagttttt aaaaaagaat tagtggaata ttttatgtgc cacctgggct agaagtagct 64920
ttgttctaat aaagctgttg ccaccaaata cacctgtctg acacccgatg tcagcttgtt 64980
agtgagtgct gctgttggtt cccagcctac caccccgaggt tgggaagagc agggggactt 65040
gttatatcac cctccatccc tgctgggcta cccagcaaca caagtgagtc aaatgatggg 65100
atagtgtttg tcctcatgtg cacacacaca acagtgccta ccttcaaaga tgtgaaagct 65160
gattattttg tggcccattg tgggatgaat gtgtgtgtgt tctgttttaa gaaataacct 65220
cttgacccca agctgaaaat gtactacttg actctttttc tttccttcag gacctggtgt 65280
gaattattca gggtgtcaga tcacatgggc taaattttgc aaagtaagca atcttgttaa 65340
attctcgtgg gaatgggaat gctcacctgc acggctgtcg ttgagggctc tggcttgaag 65400
gccctgaact cttggtccag cggccagtag gacctgcctg aaggtagacg ggcctgagga 65460
tttgggtgat gcactgcacc cctaggaagg gaagggctgg gatggcagta gacttggctt 65520
tcccattact cttttctcca ggaaaacatg gctggcaagg gcttctcctt ctgggtctgg 65580
ctggacaata tcattgacct tgtgaaaaag tacatcctgg ccctttggaa cgaagggtag 65640
gttggacaga gtgtgcacag atgtaaccaa gtcccctgct ctcagcaagc cagtggcagg 65700
ggatggatgc cctgttagca ataacaacat tgttcctcct ccttggctcc aggtacatca 65760
tgggctttat cagtaaggag cgggagcggg ccatcttgag cactaagcct ccaggcacct 65820
tcctgctaag attcagtgaa agcagcaaag aaggaggcgt cactttcact tgggtggaga 65880
aggacatcag cggtaaggga ggctcccacc caccccacct gctggtggct gctgaggcct 65940
catcactgct tctagttgca agcacctact gccccctggt gggtggagat ggccttgact 66000
ccctgtttca ctcagactcg caaaacacat ttgcgtgact tctaaatcct tccagctgaa 66060
ggattggttt gctttgtttt gcttgctcca gtgactattt gttgagaatt ttgcaattta 66120
aattgtattc ttcatctctt tttctactta accctgttaa tatatcttac gcaagtagtt 66180
atattcaagt ttatttteta tgacccaact agtagcctct tcttaattag aagccagcct 66240
gaatatttcc acagtgccag gccactgaac agggtgttca gggtctcaac actagggtgg 66300

cttaagtctt ttccccttcg aggaaagaaa aaatgggcag ttttctctga gatgacctag 66360
ctgtaggttc catgatcttt ccttcccatg tcctgtgaca ggtaagaccc agatccagtc 66420
cgtggaacca tacacaaagc agcagctgaa caacatgtca tttgctgaaa tcatcatggg 66480
ctataagatc atggatgcta ccaatatcct ggtgtctcca ctggtctatc tctatcctga 66540
cattcccaag gaggaggcat tcggaaagta ttgtcggcca gagagccagg agcatcctga 66600
agctgaccca ggtagttgtt gattttccat gttcctggca tttaattttt gggaaaagtt 66660
ggaaattttg ggatccttgg aggatagata ggcaaatgcc tgaataacct gggggataat 66720
tatttctcct tatgggaaag aattgtagtg agtgcttttg ttggggtgac cgatgggatt 66780
tgagaggaga atcagaatca cttagagtag tgtagttcct gctccacaga gagtgcatga 66840
gtctaaagag gggatacagc ctgggcaata tggtgaaacc tcgtctctac aaaaaatcca 66900
aaaaaattac ccggtgtggt ggcacgcatt tgtagtcgta gctacttggg aggctgaggt 66960
gggaggatca cctgagccaa ggagttcaag gctgtagtga gcggtgatca tgccaccgca 67020
ctccagcctg gctgatagag tgagatactg tgtcaaaaaa taaaaataaa gaggggatca 67080
atacacatac gtcccccaaa acatgcctga aacacgagaa gggaaagtga gggcagttaa 67140
caggatgccc tgctggcaca gtgcttctta gtagatgcta gaaggtttga ggcccagatt 67200
tcagcccagc atatggcttt ttgcctgtaa ctgaaccatg tcagtgtgcc agatggtctg 67260
aagaaagggt ttctggagga aattattatt agctgcatgg gagtatggtt tacactagag 67320
tagaagagct gggagcatca cgtttgaagg ggaagacagt gactgggtgg aggggcaagg 67380
gattagtatt tagagtgtgc aactattgaa aataaggtat attttaatgt gtaagaggac 67440
atgtacttat atgttatata taaattattt tagctgggtg aagtggctca tgcctatagt 67500
cctagcactt tgggaggccc aggcgggagg atcacttgag cctgggagtt tgagaacagc 67560
ctagacaaca tagtgagacc ctatctatac aaaaataatt ttttttaaat tagccacgtg 67620
tggtggtttg tgcctgtagt cctagctact cgggaggctg aggtgggagg attgcttaag 67680
cccaggaggt tgaggctgca gtgagccatg atcgcaccac tgcactccag cctgggtgac 67740
agagcaagac cttgactcac caaaaaaaaa aaaaaaaaaa agagagagaa attaaaaata 67800
ctgtaatctc agctgggcat gggggttcac acctgcagtc ctagcacttt gggaggctga 67860
agcaggagga tcacttgagg ccaggaactc aagaccagcc tggcaacata gcaagacccc 67920
actacacaca cacacacaca cacacacaaa gaagagaaag aaaaaaacga aacaaaactg 67980
taatctctgc agctgtcctc agtgtggagg gggtagccct gtctgttccc cttcagcact 68040
tgctgttttg actctctggg ttctttgtgc aggtcttgat ggggagtctc tggtttgcca 68100
ttctttgttt gatttaactt tctgtaatca taaagccaat gatgggcttt tttttttttt 68160
ttttttttag actaagtctt gctctatcac ccaagctgga gtgcagtggc accatctcgg 68220
ctcactgcaa cctccacctc ccggttcaag caattctcct gcctcagcct cccgagtagc 68280
tgggattatg ggcttgtgcc accatgccca gctgattttt gtattttttg tagagaaagg 68340
gtttcgccat gttggccagg ctggcctcga actcctgacc tcaggtgatc tgcccacttc 68400
agcctcccaa agtgctggga ttacaggcgt gagccactgt gcctggccta atgatgggct 68460
ctttaatgtg atcctttagg gttggcgcct tgccctagtt gctgttgaaa aaactatttt 68520
tgtccaaata gcacacacac agaaacctac caacttccct cccacttttt cctaggaatt 68580
ccttctgagg gatttcttga gatggggcag aatggggctt ggaagaggga gttggagcta 68640
attgaccgtt gcctttctcc tttgttgggg tcctgagtct tgttcctgct gtaagagtta 68700
ctcacttcct gtctgccacc tatctccctt tgcatgtgtg cttcagttgg gagatctgtt 68760
tatcagcccc tgccacacgg ctctttgttc cttctgcaga ggacgttggg gtcccacggc 68820
tggtcctttt gactcatttt gctttcaagg tcccacctcc cagtctgagg ctgcatcctc 68880
cattaccatc gcccttcctg tgggctggga ggccaggtcc tttcctgccc agcgatgtca 68940
gcgtttcctc aggggccagg cactcatcag gagaaaggaa ctaattactt gagtaatttg 69000
ccttgccttg ctgagaggag tgtgccctga gggactccat gtgagtgtgg tgacgggtgt 69060
gggggtgtcc ctgtgttatt ttaaaatggg tgccttcagg acgatgagca tgtgaccatt 69120
tcctctctat ttccatcaca agagtattat ggtatgaggg tctcaggtta gattatcctc 69180
ccaagactct tctctcttcc ttctctactg gaagcccaca tagcatttcc ttatggcttg 69240
agggagaggt tcggagccac ttacaaatta gataaagtac atttacaatc ttgtacaaag 69300
ccacacaatg aagtcatttt tctcagcttt tttttttttt tttttttttt ttttgagcct 69360
gagtctcgct ctatcgtcca gactggagtg cagtggcgcg atcttgcttc actgaaacct 69420
ctgcctccca ggttcaagag attctcatac ctcagcctcc tgagtagctg ggattacaga 69480
catgcaccac tatgcctggc taatttttgg atttttagta gagaccgggt ttcaccctgt 69540
tggccaggct ggtctcgaac ccctgacctc aagtgatctt cccgcctggg cctcccaaag 69600
tgctgggatt ataggtgtga gccacagtgc ccagccttgt ttttgttttt gttttgtttt 69660
gacagtctgt cactctgtca cccaggctgg agtgcagtgg tgcgatctca cctcacttca 69720
gcctctgcct cccaggttca agtgattctc ctgtctcagc ctcctgagta gctgggatta 69780
caggcgtgcc accacgccca gctattttg taatttcatt aaagacaggg tttccccatg 69840
ttggtgaggc tggtcttgaa ctcctggcct caagtgatcc acctgcttca gcctcccaaa 69900
gtgcagggat tacaggcatg agccactgtg cctggcctca gctatcttga atgctggaga 69960
attaaatcct tttctgtcta gggtgtcagc tccctaaggg ctgggccaaa acagttggat 70020
ttataagaca ctagagtctt gcctcagtag ctcctttgaa ttctgcactg aattgatcag 70080
tttcttggcc caaagtaaac tcagatggca gcccaagagc cactctgcag tgccttcttt 70140
cacatggtca tcatgctctc tgatccctca ggttctgtct aagcctcatg ttttatgacc 70200

gtgctgttct cagcccacct caccctgccc catgccttct caatggtttg ttcacctgaa 70260
ttccccagat ttcatgccag tatccccaag gttccttgac ctcttggtgt aagcattcag 70320
catctaaaat tcattttatt cccgtcaacg catttctaac tgtagaacaa gaattataaa 70380
tgacaaagct catagaaaat tggcaccttg tcttcccct ccctcttatt ttatacataa 70440
aagagaatat gggctgggca ttgtggccaa ggctgggcat gatagctcat acttgtaatc 70500
cagcactttg ggagggtgag gcagatggat cacctgaggt caggagttca agaccagcct 70560
ggccaacatg gtgaaacctc atctctacta aaattacaaa aaaaaaatta gctaggcatg 70620
gtggcagatg cctgtaatcc agctactcag gaggctgatg aaggagaatc acttgaaccc 70680
tggaggcaga ggttgtagag agccaagatg gcgctactgc actccaacct gggcgaaaga 70740
gagcaagact ccgtctcaaa aaaaaaaaag acaaaaatta gccaggcatg gtggtgccac 70800
ctgtagtccc agctgcttgg gagcctaagg caggagaatc gttttgacct gggagtagga 70860
ggttgcggta accgagattg tgccactgca cttgagcctg ggcaacagag tgagactctg 70920
tctcaaaaca ataagaacaa cagcaacaaa agagagagac catgccttgc tccaggtctc 70980
ttagctattg aagatgtacc tggacccagg tctccggtct tctagttgaa gcaattgtac 71040
tgccttacaa agtcacattc tctttggtgc tttttgattg acgtatttat ccaactagaa 71100
agttactcat gccctcatcc aaaaatgtgg tagaggccag attagtgctg gtaggaataa 71160
gagatataac ctttggcttt ggaaccacaa gcattagcag tctccatgtt ctttaaagac 71220
ttggtgatat tggtatttag gctggacacc atgcaaagac tacacaggct cggttcctgc 71280
atgcagagaa gttatctaag agatatgacc aggccggaat agaatgctca gaccacgtgg 71340
aggctgttaa acttttacat aatctaggga aagaagggac acaaggtggc attagtctag 71400
ggtcaggtgg gaaaaggtta tgctgaaaag tctctgcagc tcaggacagc tttgtgcaaa 71460
gaactgaagt tcacagctgc tagtgcctgg gagatcaaat agtataaatg agggcagaca 71520
accctgaggg gcagatggag ctttccagac aatcttggca tgaggatgag tgagtttcaa 71580
atcagtcctg ccgaggcaga tggcttcctc cagctctgct tactgaatgc gaagtcacag 71640
tcagtaagaa aactggtttt cttcttccca ggcgctgccc catacctgaa gaccaagttt 71700
atctgtgtga caccgtaagt ggcttccttt ccccgttttg ccttcatttc taatatcctc 71760
agttatccct gggaatggga cactgggtga gagttaatct gccaaaggtt ggaagcccct 71820
gggctatgtt tagtactcaa agtgaccttg tgtgtttaaa aagcttgagc ttttattttt 71880
ctgttggaga ccagagtttg atggcttgtg tgtgtgtgtt ttgttctttt tttttttcc 71940
attgtgtctt gtcaaccccc cgtttcccct cctgctgccc cccatttcct acagaacgac 72000
ctgcagcaat accattgacc tgccgatgtc cccccgcact ttagattcat tgatgcagtt 72060
tggaaataat ggtgaaggtg ctgaaccctc agcaggaggg cagtttggtg agtatttggt 72120
tgacagactt tgtccctata agggaagttg gtcccctttg tgtgatgctc tcacatgtac 72180
acaccgagag ctggtcactc ggaatggtag gagattctag agctttgctt tccaaaagag 72240
atggtatgaa tgccacatgt gtgagtataa atcttctagc agccacactg gaaatagacg 72300
aacttaattt ttacaatata ttttatttaa cccactaaat ccaacatact ctcaatttaa 72360
catttcagaa aaagttgagg ctgggtgagt ggctcatgcc tgtaatccca gcactttggg 72420
aggccgaggt gggtggatca cttgaggtca ggagttcgag accagtctga ccaaaatctc 72480
taaaatataa aaattagctg ggcatggtgg cgcatacctg taatcccagc tactcaagaa 72540
gctgaggtgg gaggatcgct tgagcctggg aggtggaggt tgcagtgagc agagatcgtg 72600
ccactgcact ccagcctggg cgacagagtg agactccatc tcaaataaac aaaactaaac 72660
taaaaagaaa aagttgagac cttttttttat tctttttttt catactaagc ctttaaaatc 72720
cagtgggctt ttgacagcca cagcacagct cagtttggac aaaccaaatc tcaaatgctt 72780
ggtggccacg tgtgtctcgg ggctcctgaa ttaaacagta gatcaagggc agaagatctc 72840
aggacagcct tagagcttct gtaaacatgg agctctggga atcagttaag gtgggaatga 72900
gaaaggaccc ttcccgaggc agggtcctcc agggaggagg gtaaatctgg cttttctgac 72960
catccctggg ccttaagggg caggagattg gatagcagtg gtagcctggg ccctgtcctc 73020
tgaagggctg ggggcgtggc ctgccagttg cagagggtgg acaactgaac tagttttccc 73080
tgtctgtccc tccagagtcc ctcacctttg acatggagtt gacctcggag tgcgctacct 73140
cccccatgtg aggagctgag aacggaagct gcagaaagat acgactgagg cgcctacctg 73200
cattctgcca cccctcacac agccaaaccc cagatcatct gaaactacta actttgtggt 73260
tccagatttt ttttaatctc ctacttctgc tatctttgag caatctgggc acttttaaaa 73320
atagagaaat gagtgaatgt gggtgatctg cttttatcta aatgcaaata aggatgtgtt 73380
ctctgagacc catgatcagg ggatgtggcg gggggtggct agagggagaa aaaggaaatg 73440
tcttgtgttg ttttgttccc ctgccctcct ttctcagcag ctttttgtta ttgttgttgt 73500
tgttcttaga caagtgcctc ctggtgcctg cggcatcctt ctgcctgttt ctgtaagcaa 73560
atgccacagg ccacctatag ctacatactc ctggcattgc actttttaac cttgctgaca 73620
tccaaataga agataggact atctaagccc taggtttctt tttaaattaa gaaataataa 73680
caattaaagg gcaaaaaaca ctgtatcagc atagcctttc tgtatttaag aaacttaagc 73740
agccgggcat ggtggctcac gcctgtaatc ccagcacttt gggaggccga ggcggatcat 73800
aaggtcagga gatcaagacc atcctggcta acacggtgaa accccgtctc tactaaaagt 73860
acaaaaaatt agctgggtgt ggtggtgggc gcctgtagtc ccagctactc gggaggctga 73920
ggcaggagaa tcgcttgaac ctgagaggcg gaggttgcag tgagccaaaa ttgcaccact 73980
gcacactgca ctccatcctg ggcgacagtc tgagactctg tctcaaaaaa aaaaaaaaaa 74040
aaaagaaact tcagttaaca gcctccttgg tgctttaagc attcagcttc cttcaggctg 74100

```
gtaatttata taatccctga aacgggcttc aggtcaaacc cttaagacat ctgaagctgc 74160
aacctggcct ttggtgttga aataggaagg tttaaggaga atctaagcat tttagacttt 74220
tttttataaa tagacttatt ttcctttgta atgtattggc cttttagtga gtaaggctgg 74280
gcagagggtg cttacaacct tgactccctt tctccctgga cttgatctgc tgtttcagag 74340
gctaggttgt ttctgtgggt gccttatcag ggctgggata cttctgattc tggcttcctt 74400
cctgccccac cctcccgacc ccag                                        74424
```

<210> 154
<211> 3455
<212> DNA
<213> H. sapiens

<220>
<221> CDS
<222> (241)...(2553)

<400> 154

```
ggtttccgga gctgcggcgg cgcagactgg gagggggagc cgggggttcc gacgtcgcag 60
ccgagggaac aagccccaac cggatcctgg acaggcaccc cggcttggcg ctgtctctcc 120
ccctcggctc ggagaggccc ttcggcctga gggagcctcg ccgcccgtcc ccggcacacg 180
cgcagccccg gcctctcggc ctctgccgga gaaacagttg ggacccctga ttttagcagg 240
atg gcc caa tgg aat cag cta cag cag ctt gac aca cgg tac ctg gag   288
Met Ala Gln Trp Asn Gln Leu Gln Gln Leu Asp Thr Arg Tyr Leu Glu
 1               5                  10                  15

cag ctc cat cag ctc tac agt gac agc ttc cca atg gag ctg cgg cag   336
Gln Leu His Gln Leu Tyr Ser Asp Ser Phe Pro Met Glu Leu Arg Gln
            20                  25                  30

ttt ctg gcc cct tgg att gag agt caa gat tgg gca tat gcg gcc agc   384
Phe Leu Ala Pro Trp Ile Glu Ser Gln Asp Trp Ala Tyr Ala Ala Ser
        35                  40                  45

aaa gaa tca cat gcc act ttg gtg ttt cat aat ctc ctg gga gag att   432
Lys Glu Ser His Ala Thr Leu Val Phe His Asn Leu Leu Gly Glu Ile
    50                  55                  60

gac cag cag tat agc cgc ttc ctg caa gag tcg aat gtt ctc tat cag   480
Asp Gln Gln Tyr Ser Arg Phe Leu Gln Glu Ser Asn Val Leu Tyr Gln
65                  70                  75                  80

cac aat cta cga aga atc aag cag ttt ctt cag agc agg tat ctt gag   528
His Asn Leu Arg Arg Ile Lys Gln Phe Leu Gln Ser Arg Tyr Leu Glu
                85                  90                  95

aag cca atg gag att gcc cgg att gtg gcc cgg tgc ctg tgg gaa gaa   576
Lys Pro Met Glu Ile Ala Arg Ile Val Ala Arg Cys Leu Trp Glu Glu
            100                 105                 110

tca cgc ctt cta cag act gca gcc act gcg gcc cag caa ggg ggc cag   624
Ser Arg Leu Leu Gln Thr Ala Ala Thr Ala Ala Gln Gln Gly Gly Gln
        115                 120                 125

gcc aac cac ccc aca gca gcc gtg gtg acg gag aag cag cag atg ctg   672
Ala Asn His Pro Thr Ala Ala Val Val Thr Glu Lys Gln Gln Met Leu
    130                 135                 140

gag cag cac ctt cag gat gtc cgg aag aga gtg cag gat cta gaa cag   720
Glu Gln His Leu Gln Asp Val Arg Lys Arg Val Gln Asp Leu Glu Gln
145                 150                 155                 160

aaa atg aaa gtg gta gag aat ctc cag gat gac ttt gat ttc aac tat   768
Lys Met Lys Val Val Glu Asn Leu Gln Asp Asp Phe Asp Phe Asn Tyr
```

```
                    165                 170                 175

aaa acc ctc aag agt caa gga gac atg caa gat ctg aat gga aac aac      816
Lys Thr Leu Lys Ser Gln Gly Asp Met Gln Asp Leu Asn Gly Asn Asn
            180                 185                 190

cag tca gtg acc agg cag aag atg cag cag ctg gaa cag atg ctc act      864
Gln Ser Val Thr Arg Gln Lys Met Gln Gln Leu Glu Gln Met Leu Thr
        195                 200                 205

gcg ctg gac cag atg cgg aga agc atc gtg agt gag ctg gcg ggg ctt      912
Ala Leu Asp Gln Met Arg Arg Ser Ile Val Ser Glu Leu Ala Gly Leu
    210                 215                 220

ttg tca gcg atg gag tac gtg cag aaa act ctc acg gac gag gag ctg      960
Leu Ser Ala Met Glu Tyr Val Gln Lys Thr Leu Thr Asp Glu Glu Leu
225                 230                 235                 240

gct gac tgg aag agg cgg caa cag att gcc tgc att gga ggc ccg ccc     1008
Ala Asp Trp Lys Arg Arg Gln Gln Ile Ala Cys Ile Gly Gly Pro Pro
                245                 250                 255

aac atc tgc cta gat cgg cta gaa aac tgg ata acg tca tta gca gaa     1056
Asn Ile Cys Leu Asp Arg Leu Glu Asn Trp Ile Thr Ser Leu Ala Glu
            260                 265                 270

tct caa ctt cag acc cgt caa caa att aag aaa ctg gag gag ttg cag     1104
Ser Gln Leu Gln Thr Arg Gln Gln Ile Lys Lys Leu Glu Glu Leu Gln
        275                 280                 285

caa aaa gtt tcc tac aaa ggg gac ccc att gta cag cac cgg ccg atg     1152
Gln Lys Val Ser Tyr Lys Gly Asp Pro Ile Val Gln His Arg Pro Met
    290                 295                 300

ctg gag gag aga atc gtg gag ctg ttt aga aac tta atg aaa agt gcc     1200
Leu Glu Glu Arg Ile Val Glu Leu Phe Arg Asn Leu Met Lys Ser Ala
305                 310                 315                 320

ttt gtg gtg gag cgg cag ccc tgc atg ccc atg cat cct gac cgg ccc     1248
Phe Val Val Glu Arg Gln Pro Cys Met Pro Met His Pro Asp Arg Pro
                325                 330                 335

ctc gtc atc aag acc ggc gtc cag ttc act act aaa gtc agg ttg ctg     1296
Leu Val Ile Lys Thr Gly Val Gln Phe Thr Thr Lys Val Arg Leu Leu
            340                 345                 350

gtc aaa ttc cct gag ttg aat tat cag ctt aaa att aaa gtg tgc att     1344
Val Lys Phe Pro Glu Leu Asn Tyr Gln Leu Lys Ile Lys Val Cys Ile
        355                 360                 365

gac aaa gac tct ggg gac gtt gca gct ctc aga gga tcc cgg aaa ttt     1392
Asp Lys Asp Ser Gly Asp Val Ala Ala Leu Arg Gly Ser Arg Lys Phe
    370                 375                 380

aac att ctg ggc aca aac aca aaa gtg atg aac atg gaa gaa tcc aac     1440
Asn Ile Leu Gly Thr Asn Thr Lys Val Met Asn Met Glu Glu Ser Asn
385                 390                 395                 400

aac ggc agc ctc tct gca gaa ttc aaa cac ttg acc ctg agg gag cag     1488
Asn Gly Ser Leu Ser Ala Glu Phe Lys His Leu Thr Leu Arg Glu Gln
                405                 410                 415

aga tgt ggg aat ggg ggc cga gcc aat tgt gat gct tcc ctg att gtg     1536
Arg Cys Gly Asn Gly Gly Arg Ala Asn Cys Asp Ala Ser Leu Ile Val
            420                 425                 430
```

```
act gag gag ctg cac ctg atc acc ttt gag acc gag gtg tat cac caa   1584
Thr Glu Glu Leu His Leu Ile Thr Phe Glu Thr Glu Val Tyr His Gln
        435                 440                 445

ggc ctc aag att gac cta gag acc cac tcc ttg cca gtt gtg gtg atc   1632
Gly Leu Lys Ile Asp Leu Glu Thr His Ser Leu Pro Val Val Val Ile
    450                 455                 460

tcc aac atc tgt cag atg cca aat gcc tgg gcg tcc atc ctg tgg tac   1680
Ser Asn Ile Cys Gln Met Pro Asn Ala Trp Ala Ser Ile Leu Trp Tyr
465                 470                 475                 480

aac atg ctg acc aac aat ccc aag aat gta aac ttt ttt acc aag ccc   1728
Asn Met Leu Thr Asn Asn Pro Lys Asn Val Asn Phe Phe Thr Lys Pro
                485                 490                 495

cca att gga acc tgg gat caa gtg gcc gag gtc ctg agc tgg cag ttc   1776
Pro Ile Gly Thr Trp Asp Gln Val Ala Glu Val Leu Ser Trp Gln Phe
            500                 505                 510

tcc tcc acc acc aag cga gga ctg agc atc gag cag ctg act aca ctg   1824
Ser Ser Thr Thr Lys Arg Gly Leu Ser Ile Glu Gln Leu Thr Thr Leu
        515                 520                 525

gca gag aaa ctc ttg gga cct ggt gtg aat tat tca ggg tgt cag atc   1872
Ala Glu Lys Leu Leu Gly Pro Gly Val Asn Tyr Ser Gly Cys Gln Ile
    530                 535                 540

aca tgg gct aaa ttt tgc aaa gaa aac atg gct ggc aag ggc ttc tcc   1920
Thr Trp Ala Lys Phe Cys Lys Glu Asn Met Ala Gly Lys Gly Phe Ser
545                 550                 555                 560

ttc tgg gtc tgg ctg gac aat atc att gac ctt gtg aaa aag tac atc   1968
Phe Trp Val Trp Leu Asp Asn Ile Ile Asp Leu Val Lys Lys Tyr Ile
                565                 570                 575

ctg gcc ctt tgg aac gaa ggg tac atc atg ggc ttt atc agt aag gag   2016
Leu Ala Leu Trp Asn Glu Gly Tyr Ile Met Gly Phe Ile Ser Lys Glu
            580                 585                 590

cgg gag cgg gcc atc ttg agc act aag cct cca ggc acc ttc ctg cta   2064
Arg Glu Arg Ala Ile Leu Ser Thr Lys Pro Pro Gly Thr Phe Leu Leu
        595                 600                 605

aga ttc agt gaa agc agc aaa gaa gga ggc gtc act ttc act tgg gtg   2112
Arg Phe Ser Glu Ser Ser Lys Glu Gly Gly Val Thr Phe Thr Trp Val
    610                 615                 620

gag aag gac atc agc ggt aag acc cag atc cag tcc gtg gaa cca tac   2160
Glu Lys Asp Ile Ser Gly Lys Thr Gln Ile Gln Ser Val Glu Pro Tyr
625                 630                 635                 640

aca aag cag cag ctg aac aac atg tca ttt gct gaa atc atc atg ggc   2208
Thr Lys Gln Gln Leu Asn Asn Met Ser Phe Ala Glu Ile Ile Met Gly
                645                 650                 655

tat aag atc atg gat gct acc aat atc ctg gtg tct cca ctg gtc tat   2256
Tyr Lys Ile Met Asp Ala Thr Asn Ile Leu Val Ser Pro Leu Val Tyr
            660                 665                 670

ctc tat cct gac att ccc aag gag gag gca ttc gga aag tat tgt cgg   2304
Leu Tyr Pro Asp Ile Pro Lys Glu Glu Ala Phe Gly Lys Tyr Cys Arg
        675                 680                 685
```

```
cca gag agc cag gag cat cct gaa gct gac cca ggt agc gct gcc cca    2352
Pro Glu Ser Gln Glu His Pro Glu Ala Asp Pro Gly Ser Ala Ala Pro
    690                 695                 700

tac ctg aag acc aag ttt atc tgt gtg aca cca acg acc tgc agc aat    2400
Tyr Leu Lys Thr Lys Phe Ile Cys Val Thr Pro Thr Thr Cys Ser Asn
705                 710                 715                 720

acc att gac ctg ccg atg tcc ccc cgc act tta gat tca ttg atg cag    2448
Thr Ile Asp Leu Pro Met Ser Pro Arg Thr Leu Asp Ser Leu Met Gln
                725                 730                 735

ttt gga aat aat ggt gaa ggt gct gaa ccc tca gca gga ggg cag ttt    2496
Phe Gly Asn Asn Gly Glu Gly Ala Glu Pro Ser Ala Gly Gly Gln Phe
            740                 745                 750

gag tcc ctc acc ttt gac atg gag ttg acc tcg gag tgc gct acc tcc    2544
Glu Ser Leu Thr Phe Asp Met Glu Leu Thr Ser Glu Cys Ala Thr Ser
        755                 760                 765

ccc atg tga ggagctgaga acggaagctg cagaaagata cgactgaggc            2593
Pro Met  *
    770

gcctacctgc attctgccac ccctcacaca gccaaacccc agatcatctg aaactactaa 2653
ctttgtggtt ccagattttt tttaatctcc tacttctgct atctttgagc aatctgggca 2713
cttttaaaaa tagagaaatg agtgaatgtg ggtgatctgc ttttatctaa atgcaaataa 2773
ggatgtgttc tctgagaccc atgatcaggg gatgtggcgg ggggtggcta gagggagaaa 2833
aaggaaatgt cttgtgttgt tttgttcccc tgccctcctt tctcagcagc ttttgttat 2893
tgttgttgtt gttcttagac aagtgcctcc tggtgcctgc ggcatccttc tgcctgtttc 2953
tgtaagcaaa tgccacaggc cacctatagc tacatactcc tggcattgca ctttttaacc 3013
ttgctgacat ccaaatagaa gataggacta tctaagccct aggtttcttt ttaaattaag 3073
aaataataac aattaaaggg caaaaaacac tgtatcagca tagcctttct gtatttaaga 3133
aacttaagca gccgggcatg gtggctcacg cctgtaatcc cagcactttg ggaggccgag 3193
gcggatcata aggtcaggag atcaagacca tcctggctaa cacggtgaaa ccccgtctct 3253
actaaaagta caaaaaatta gctgggtgtg gtggtgggcg cctgtagtcc cagctactcg 3313
ggaggctgag gcaggagaat cgcttgaacc tgagaggcgg aggttgcagt gagccaaaat 3373
tgcaccactg cacactgcac tccatcctgg gcgacagtct gagactctgt ctcaaaaaaa 3433
aaaaaaaaaa aaaaaaaaaa aa                                          3455
```

<210> 155
<211> 20
<212> DNA
<213> Artificial Sequence

<220>
<223> Antisense oligonucleotide

<400> 155
gtgcgcgcga gcccgaaatc 20

<210> 156
<211> 24
<212> DNA
<213> Artificial Sequence

<220>
<223> PCR Primer

<400> 156

acatgccact ttggtgtttc ataa 24

<210> 157
<211> 27
<212> DNA
<213> Artificial Sequence

<220>
<223> PCR Primer

<400> 157
tcttcgtaga ttgtgctgat agagaac 27

<210> 158
<211> 29
<212> DNA
<213> Artificial Sequence

<220>
<223> PCR Probe

<400> 158
cagtatagcc gcttcctgca agagtcgaa 29

<210> 159
<211> 20
<212> DNA
<213> Artificial Sequence

<220>
<223> Antisense oligonucleotide

<400> 159
agcctctgca ccctcatgtt 20

<210> 160
<211> 20
<212> DNA
<213> Artificial Sequence

<220>
<223> Antisense oligonucleotide

<400> 160
ctcctaaatt aagaacttct 20

<210> 161
<211> 20
<212> DNA
<213> Artificial Sequence

<220>
<223> Antisense oligonucleotide

<400> 161
ttttgcatga tgtaaccact 20

<210> 162

<211> 20
<212> DNA
<213> Artificial Sequence

<220>
<223> Antisense oligonucleotide

<400> 162
tattgaaaat tatctaattc 20

<210> 163
<211> 20
<212> DNA
<213> Artificial Sequence

<220>
<223> Antisense oligonucleotide

<400> 163
ttgggccatc ctgctaaaat 20

<210> 164
<211> 20
<212> DNA
<213> Artificial Sequence

<220>
<223> Antisense oligonucleotide

<400> 164
attcacttgc ctccttgact 20

<210> 165
<211> 20
<212> DNA
<213> Artificial Sequence

<220>
<223> Antisense oligonucleotide

<400> 165
atgcccttac tctccgcatc 20

<210> 166
<211> 20
<212> DNA
<213> Artificial Sequence

<220>
<223> Antisense oligonucleotide

<400> 166
ctgaacttac cctctgagag 20

<210> 167
<211> 20
<212> DNA
<213> Artificial Sequence

<220>
<223> Antisense oligonucleotide

<400> 167
aaatgcggac ccaagagttt 20

<210> 168
<211> 20
<212> DNA
<213> Artificial Sequence

<220>
<223> Antisense oligonucleotide

<400> 168
cttgttccct cggctgcgac 20

<210> 169
<211> 20
<212> DNA
<213> Artificial Sequence

<220>
<223> Antisense oligonucleotide

<400> 169
gcctgtccag gatccggttg 20

<210> 170
<211> 20
<212> DNA
<213> Artificial Sequence

<220>
<223> Antisense oligonucleotide

<400> 170
gaagggcctc tccgagccga 20

<210> 171
<211> 20
<212> DNA
<213> Artificial Sequence

<220>
<223> Antisense oligonucleotide

<400> 171
ggcggcgagg ctccctcagg 20

<210> 172
<211> 20
<212> DNA
<213> Artificial Sequence

<220>
<223> Antisense oligonucleotide

<400> 172
tccggcagag gccgagaggc 20

<210> 173
<211> 20
<212> DNA
<213> Artificial Sequence

<220>
<223> Antisense oligonucleotide

<400> 173
ccatcctgct aaaatcaggg 20

<210> 174
<211> 20
<212> DNA
<213> Artificial Sequence

<220>
<223> Antisense oligonucleotide

<400> 174
ccattgggcc atcctgctaa 20

<210> 175
<211> 20
<212> DNA
<213> Artificial Sequence

<220>
<223> Antisense oligonucleotide

<400> 175
tgtcaagctg ctgtagctga 20

<210> 176
<211> 20
<212> DNA
<213> Artificial Sequence

<220>
<223> Antisense oligonucleotide

<400> 176
aactgccgca gctccattgg 20

<210> 177
<211> 20
<212> DNA
<213> Artificial Sequence

<220>
<223> Antisense oligonucleotide

<400> 177
tcttgactct caatccaagg 20

<210> 178
<211> 20
<212> DNA
<213> Artificial Sequence

<220>
<223> Antisense oligonucleotide

<400> 178
cgcatatgcc caatcttgac 20

<210> 179
<211> 20
<212> DNA
<213> Artificial Sequence

<220>
<223> Antisense oligonucleotide

<400> 179
cgactcttgc aggaagcggc 20

<210> 180
<211> 20
<212> DNA
<213> Artificial Sequence

<220>
<223> Antisense oligonucleotide

<400> 180
tcgtagattg tgctgataga 20

<210> 181
<211> 20
<212> DNA
<213> Artificial Sequence

<220>
<223> Antisense oligonucleotide

<400> 181
agaaactgct tgattcttcg 20

<210> 182
<211> 20
<212> DNA
<213> Artificial Sequence

<220>
<223> Antisense oligonucleotide

<400> 182
gatacctgct ctgaagaaac 20

<210> 183
<211> 20
<212> DNA

<213> Artificial Sequence

<220>
<223> Antisense oligonucleotide

<400> 183
ttctcaagat acctgctctg 20

<210> 184
<211> 20
<212> DNA
<213> Artificial Sequence

<220>
<223> Antisense oligonucleotide

<400> 184
ttggcttctc aagatacctg 20

<210> 185
<211> 20
<212> DNA
<213> Artificial Sequence

<220>
<223> Antisense oligonucleotide

<400> 185
gtgattcttc ccacaggcac 20

<210> 186
<211> 20
<212> DNA
<213> Artificial Sequence

<220>
<223> Antisense oligonucleotide

<400> 186
atctgctgct tctccgtcac 20

<210> 187
<211> 20
<212> DNA
<213> Artificial Sequence

<220>
<223> Antisense oligonucleotide

<400> 187
ccagcatctg ctgcttctcc 20

<210> 188
<211> 20
<212> DNA
<213> Artificial Sequence

<220>

<223> Antisense oligonucleotide

<400> 188
tgaaggtgct gctccagcat 20

<210> 189
<211> 20
<212> DNA
<213> Artificial Sequence

<220>
<223> Antisense oligonucleotide

<400> 189
ttctgttcta gatcctgcac 20

<210> 190
<211> 20
<212> DNA
<213> Artificial Sequence

<220>
<223> Antisense oligonucleotide

<400> 190
ctggagattc tctaccactt 20

<210> 191
<211> 20
<212> DNA
<213> Artificial Sequence

<220>
<223> Antisense oligonucleotide

<400> 191
aagtcatcct ggagattctc 20

<210> 192
<211> 20
<212> DNA
<213> Artificial Sequence

<220>
<223> Antisense oligonucleotide

<400> 192
aatcaaagtc atcctggaga 20

<210> 193
<211> 20
<212> DNA
<213> Artificial Sequence

<220>
<223> Antisense oligonucleotide

<400> 193

gttgaaatca aagtcatcct 20

<210> 194
<211> 20
<212> DNA
<213> Artificial Sequence

<220>
<223> Antisense oligonucleotide

<400> 194
ttatagttga aatcaaagtc 20

<210> 195
<211> 20
<212> DNA
<213> Artificial Sequence

<220>
<223> Antisense oligonucleotide

<400> 195
gggttttata gttgaaatca 20

<210> 196
<211> 20
<212> DNA
<213> Artificial Sequence

<220>
<223> Antisense oligonucleotide

<400> 196
cttgagggtt ttatagttga 20

<210> 197
<211> 20
<212> DNA
<213> Artificial Sequence

<220>
<223> Antisense oligonucleotide

<400> 197
tgactcttga gggttttata 20

<210> 198
<211> 20
<212> DNA
<213> Artificial Sequence

<220>
<223> Antisense oligonucleotide

<400> 198
ctccttgact cttgagggtt 20

<210> 199

<211> 20
<212> DNA
<213> Artificial Sequence

<220>
<223> Antisense oligonucleotide

<400> 199
catgtctcct tgactcttga 20

<210> 200
<211> 20
<212> DNA
<213> Artificial Sequence

<220>
<223> Antisense oligonucleotide

<400> 200
attcagatct tgcatgtctc 20

<210> 201
<211> 20
<212> DNA
<213> Artificial Sequence

<220>
<223> Antisense oligonucleotide

<400> 201
tggttgtttc cattcagatc 20

<210> 202
<211> 20
<212> DNA
<213> Artificial Sequence

<220>
<223> Antisense oligonucleotide

<400> 202
tggtcactga ctggttgttt 20

<210> 203
<211> 20
<212> DNA
<213> Artificial Sequence

<220>
<223> Antisense oligonucleotide

<400> 203
tccagctgct gcatcttctg 20

<210> 204
<211> 20
<212> DNA
<213> Artificial Sequence

<220>
<223> Antisense oligonucleotide

<400> 204
gagcatctgt tccagctgct 20

<210> 205
<211> 20
<212> DNA
<213> Artificial Sequence

<220>
<223> Antisense oligonucleotide

<400> 205
cttctccgca tctggtccag 20

<210> 206
<211> 20
<212> DNA
<213> Artificial Sequence

<220>
<223> Antisense oligonucleotide

<400> 206
ttctgcacgt actccatcgc 20

<210> 207
<211> 20
<212> DNA
<213> Artificial Sequence

<220>
<223> Antisense oligonucleotide

<400> 207
cagccagctc ctcgtccgtg 20

<210> 208
<211> 20
<212> DNA
<213> Artificial Sequence

<220>
<223> Antisense oligonucleotide

<400> 208
ctcttccagt cagccagctc 20

<210> 209
<211> 20
<212> DNA
<213> Artificial Sequence

<220>
<223> Antisense oligonucleotide

<400> 209
tgccgcctct tccagtcagc 20

<210> 210
<211> 20
<212> DNA
<213> Artificial Sequence

<220>
<223> Antisense oligonucleotide

<400> 210
ccagttttct agccgatcta 20

<210> 211
<211> 20
<212> DNA
<213> Artificial Sequence

<220>
<223> Antisense oligonucleotide

<400> 211
acgttatcca gttttctagc 20

<210> 212
<211> 20
<212> DNA
<213> Artificial Sequence

<220>
<223> Antisense oligonucleotide

<400> 212
agttgagatt ctgctaatga 20

<210> 213
<211> 20
<212> DNA
<213> Artificial Sequence

<220>
<223> Antisense oligonucleotide

<400> 213
tctgaagttg agattctgct 20

<210> 214
<211> 20
<212> DNA
<213> Artificial Sequence

<220>
<223> Antisense oligonucleotide

<400> 214
cctttgtagg aaactttttg 20

<210> 215
<211> 20
<212> DNA
<213> Artificial Sequence

<220>
<223> Antisense oligonucleotide

<400> 215
aggcactttt cattaagttt 20

<210> 216
<211> 20
<212> DNA
<213> Artificial Sequence

<220>
<223> Antisense oligonucleotide

<400> 216
ttgaccagca acctgacttt 20

<210> 217
<211> 20
<212> DNA
<213> Artificial Sequence

<220>
<223> Antisense oligonucleotide

<400> 217
agctgataat tcaactcagg 20

<210> 218
<211> 20
<212> DNA
<213> Artificial Sequence

<220>
<223> Antisense oligonucleotide

<400> 218
ttttaagctg ataattcaac 20

<210> 219
<211> 20
<212> DNA
<213> Artificial Sequence

<220>
<223> Antisense oligonucleotide

<400> 219
ctttaatttt aagctgataa 20

<210> 220
<211> 20
<212> DNA

<213> Artificial Sequence

<220>
<223> Antisense oligonucleotide

<400> 220
gcacacttta attttaagct 20

<210> 221
<211> 20
<212> DNA
<213> Artificial Sequence

<220>
<223> Antisense oligonucleotide

<400> 221
tcaatgcaca ctttaatttt 20

<210> 222
<211> 20
<212> DNA
<213> Artificial Sequence

<220>
<223> Antisense oligonucleotide

<400> 222
cccagaatgt taaatttccg 20

<210> 223
<211> 20
<212> DNA
<213> Artificial Sequence

<220>
<223> Antisense oligonucleotide

<400> 223
agaggctgcc gttgttggat 20

<210> 224
<211> 20
<212> DNA
<213> Artificial Sequence

<220>
<223> Antisense oligonucleotide

<400> 224
aagtgtttga attctgcaga 20

<210> 225
<211> 20
<212> DNA
<213> Artificial Sequence

<220>

<223> Antisense oligonucleotide

<400> 225
tctctgctcc ctcagggtca 20

<210> 226
<211> 20
<212> DNA
<213> Artificial Sequence

<220>
<223> Antisense oligonucleotide

<400> 226
atcaggtgca gctcctcagt 20

<210> 227
<211> 20
<212> DNA
<213> Artificial Sequence

<220>
<223> Antisense oligonucleotide

<400> 227
aggtgatcag gtgcagctcc 20

<210> 228
<211> 20
<212> DNA
<213> Artificial Sequence

<220>
<223> Antisense oligonucleotide

<400> 228
ctcaaaggtg atcaggtgca 20

<210> 229
<211> 20
<212> DNA
<213> Artificial Sequence

<220>
<223> Antisense oligonucleotide

<400> 229
gaggccttgg tgatacacct 20

<210> 230
<211> 20
<212> DNA
<213> Artificial Sequence

<220>
<223> Antisense oligonucleotide

<400> 230

tcaatcttga ggccttggtg 20

<210> 231
<211> 20
<212> DNA
<213> Artificial Sequence

<220>
<223> Antisense oligonucleotide

<400> 231
ctaggtcaat cttgaggcct 20

<210> 232
<211> 20
<212> DNA
<213> Artificial Sequence

<220>
<223> Antisense oligonucleotide

<400> 232
ggtctctagg tcaatcttga 20

<210> 233
<211> 20
<212> DNA
<213> Artificial Sequence

<220>
<223> Antisense oligonucleotide

<400> 233
aaggagtggg tctctaggtc 20

<210> 234
<211> 20
<212> DNA
<213> Artificial Sequence

<220>
<223> Antisense oligonucleotide

<400> 234
ctggcaagga gtgggtctct 20

<210> 235
<211> 20
<212> DNA
<213> Artificial Sequence

<220>
<223> Antisense oligonucleotide

<400> 235
accacaactg gcaaggagtg 20

<210> 236

<211> 20
<212> DNA
<213> Artificial Sequence

<220>
<223> Antisense oligonucleotide

<400> 236
tctgacagat gttggagatc 20

<210> 237
<211> 20
<212> DNA
<213> Artificial Sequence

<220>
<223> Antisense oligonucleotide

<400> 237
tggcatctga cagatgttgg 20

<210> 238
<211> 20
<212> DNA
<213> Artificial Sequence

<220>
<223> Antisense oligonucleotide

<400> 238
gcatttggca tctgacagat 20

<210> 239
<211> 20
<212> DNA
<213> Artificial Sequence

<220>
<223> Antisense oligonucleotide

<400> 239
ttcttgggat tgttggtcag 20

<210> 240
<211> 20
<212> DNA
<213> Artificial Sequence

<220>
<223> Antisense oligonucleotide

<400> 240
gtcagctgct cgatgctcag 20

<210> 241
<211> 20
<212> DNA
<213> Artificial Sequence

<220>
<223> Antisense oligonucleotide

<400> 241
tcccaagagt ttctctgcca 20

<210> 242
<211> 20
<212> DNA
<213> Artificial Sequence

<220>
<223> Antisense oligonucleotide

<400> 242
catgtgatct gacaccctga 20

<210> 243
<211> 20
<212> DNA
<213> Artificial Sequence

<220>
<223> Antisense oligonucleotide

<400> 243
tagcccatgt gatctgacac 20

<210> 244
<211> 20
<212> DNA
<213> Artificial Sequence

<220>
<223> Antisense oligonucleotide

<400> 244
gccatgtttt ctttgcaaaa 20

<210> 245
<211> 20
<212> DNA
<213> Artificial Sequence

<220>
<223> Antisense oligonucleotide

<400> 245
ccttgccagc catgttttct 20

<210> 246
<211> 20
<212> DNA
<213> Artificial Sequence

<220>
<223> Antisense oligonucleotide

<400> 246
gaagcccttg ccagccatgt 20

<210> 247
<211> 20
<212> DNA
<213> Artificial Sequence

<220>
<223> Antisense oligonucleotide

<400> 247
aaggagaagc ccttgccagc 20

<210> 248
<211> 20
<212> DNA
<213> Artificial Sequence

<220>
<223> Antisense oligonucleotide

<400> 248
cccagaagga gaagcccttg 20

<210> 249
<211> 20
<212> DNA
<213> Artificial Sequence

<220>
<223> Antisense oligonucleotide

<400> 249
tccagccaga cccagaagga 20

<210> 250
<211> 20
<212> DNA
<213> Artificial Sequence

<220>
<223> Antisense oligonucleotide

<400> 250
tctttgctgc tttcactgaa 20

<210> 251
<211> 20
<212> DNA
<213> Artificial Sequence

<220>
<223> Antisense oligonucleotide

<400> 251
atgttgttca gctgctgctt 20

<210> 252
<211> 20
<212> DNA
<213> Artificial Sequence

<220>
<223> Antisense oligonucleotide

<400> 252
atgacatgtt gttcagctgc 20

<210> 253
<211> 20
<212> DNA
<213> Artificial Sequence

<220>
<223> Antisense oligonucleotide

<400> 253
agcaaatgac atgttgttca 20

<210> 254
<211> 20
<212> DNA
<213> Artificial Sequence

<220>
<223> Antisense oligonucleotide

<400> 254
atttcagcaa atgacatgtt 20

<210> 255
<211> 20
<212> DNA
<213> Artificial Sequence

<220>
<223> Antisense oligonucleotide

<400> 255
tgatgatttc agcaaatgac 20

<210> 256
<211> 20
<212> DNA
<213> Artificial Sequence

<220>
<223> Antisense oligonucleotide

<400> 256
ttatagccca tgatgatttc 20

<210> 257
<211> 20
<212> DNA

<213> Artificial Sequence

<220>
<223> Antisense oligonucleotide

<400> 257
tgatcttata gcccatgatg 20

<210> 258
<211> 20
<212> DNA
<213> Artificial Sequence

<220>
<223> Antisense oligonucleotide

<400> 258
atccatgatc ttatagccca 20

<210> 259
<211> 20
<212> DNA
<213> Artificial Sequence

<220>
<223> Antisense oligonucleotide

<400> 259
ggtagcatcc atgatcttat 20

<210> 260
<211> 20
<212> DNA
<213> Artificial Sequence

<220>
<223> Antisense oligonucleotide

<400> 260
aatgtcagga tagagataga 20

<210> 261
<211> 20
<212> DNA
<213> Artificial Sequence

<220>
<223> Antisense oligonucleotide

<400> 261
gcctcctcct tgggaatgtc 20

<210> 262
<211> 20
<212> DNA
<213> Artificial Sequence

<220>

<223> Antisense oligonucleotide

<400> 262
tccgaatgcc tcctccttgg 20

<210> 263
<211> 20
<212> DNA
<213> Artificial Sequence

<220>
<223> Antisense oligonucleotide

<400> 263
tactttccga atgcctcctc 20

<210> 264
<211> 20
<212> DNA
<213> Artificial Sequence

<220>
<223> Antisense oligonucleotide

<400> 264
gacaatactt tccgaatgcc 20

<210> 265
<211> 20
<212> DNA
<213> Artificial Sequence

<220>
<223> Antisense oligonucleotide

<400> 265
ctacctgggt cagcttcagg 20

<210> 266
<211> 20
<212> DNA
<213> Artificial Sequence

<220>
<223> Antisense oligonucleotide

<400> 266
ataaacttgg tcttcaggta 20

<210> 267
<211> 20
<212> DNA
<213> Artificial Sequence

<220>
<223> Antisense oligonucleotide

<400> 267

gtcgttggtg tcacacagat 20

<210> 268
<211> 20
<212> DNA
<213> Artificial Sequence

<220>
<223> Antisense oligonucleotide

<400> 268
tgcaggtcgt tggtgtcaca 20

<210> 269
<211> 20
<212> DNA
<213> Artificial Sequence

<220>
<223> Antisense oligonucleotide

<400> 269
attgctgcag gtcgttggtg 20

<210> 270
<211> 20
<212> DNA
<213> Artificial Sequence

<220>
<223> Antisense oligonucleotide

<400> 270
atggtattgc tgcaggtcgt 20

<210> 271
<211> 20
<212> DNA
<213> Artificial Sequence

<220>
<223> Antisense oligonucleotide

<400> 271
ggtcaatggt attgctgcag 20

<210> 272
<211> 20
<212> DNA
<213> Artificial Sequence

<220>
<223> Antisense oligonucleotide

<400> 272
gacatcggca ggtcaatggt 20

<210> 273

<211> 20
<212> DNA
<213> Artificial Sequence

<220>
<223> Antisense oligonucleotide

<400> 273
caatgaatct aaagtgcggg 20

<210> 274
<211> 20
<212> DNA
<213> Artificial Sequence

<220>
<223> Antisense oligonucleotide

<400> 274
tgcatcaatg aatctaaagt 20

<210> 275
<211> 20
<212> DNA
<213> Artificial Sequence

<220>
<223> Antisense oligonucleotide

<400> 275
caaactgcat caatgaatct 20

<210> 276
<211> 20
<212> DNA
<213> Artificial Sequence

<220>
<223> Antisense oligonucleotide

<400> 276
atttccaaac tgcatcaatg 20

<210> 277
<211> 20
<212> DNA
<213> Artificial Sequence

<220>
<223> Antisense oligonucleotide

<400> 277
aactgccctc ctgctgaggg 20

<210> 278
<211> 20
<212> DNA
<213> Artificial Sequence

<220>
<223> Antisense oligonucleotide

<400> 278
ggtgagggac tcaaactgcc 20

<210> 279
<211> 20
<212> DNA
<213> Artificial Sequence

<220>
<223> Antisense oligonucleotide

<400> 279
cagtcgtatc tttctgcagc 20

<210> 280
<211> 20
<212> DNA
<213> Artificial Sequence

<220>
<223> Antisense oligonucleotide

<400> 280
agatagcaga agtaggagat 20

<210> 281
<211> 20
<212> DNA
<213> Artificial Sequence

<220>
<223> Antisense oligonucleotide

<400> 281
aaagtgccca gattgctcaa 20

<210> 282
<211> 20
<212> DNA
<213> Artificial Sequence

<220>
<223> Antisense oligonucleotide

<400> 282
tttttaaaag tgcccagatt 20

<210> 283
<211> 20
<212> DNA
<213> Artificial Sequence

<220>
<223> Antisense oligonucleotide

<400> 283
cagatcaccc acattcactc 20

<210> 284
<211> 20
<212> DNA
<213> Artificial Sequence

<220>
<223> Antisense oligonucleotide

<400> 284
tgcatttaga taaaagcaga 20

<210> 285
<211> 20
<212> DNA
<213> Artificial Sequence

<220>
<223> Antisense oligonucleotide

<400> 285
gaacacatcc ttatttgcat 20

<210> 286
<211> 20
<212> DNA
<213> Artificial Sequence

<220>
<223> Antisense oligonucleotide

<400> 286
atcatgggtc tcagagaaca 20

<210> 287
<211> 20
<212> DNA
<213> Artificial Sequence

<220>
<223> Antisense oligonucleotide

<400> 287
cacatcccct gatcatgggt 20

<210> 288
<211> 20
<212> DNA
<213> Artificial Sequence

<220>
<223> Antisense oligonucleotide

<400> 288
agacatttcc tttttctccc 20

<210> 289
<211> 20
<212> DNA
<213> Artificial Sequence

<220>
<223> Antisense oligonucleotide

<400> 289
accaggaggc acttgtctaa 20

<210> 290
<211> 20
<212> DNA
<213> Artificial Sequence

<220>
<223> Antisense oligonucleotide

<400> 290
gcaggcacca ggaggcactt 20

<210> 291
<211> 20
<212> DNA
<213> Artificial Sequence

<220>
<223> Antisense oligonucleotide

<400> 291
gcttacagaa acaggcagaa 20

<210> 292
<211> 20
<212> DNA
<213> Artificial Sequence

<220>
<223> Antisense oligonucleotide

<400> 292
aggtggcctg tggcatttgc 20

<210> 293
<211> 20
<212> DNA
<213> Artificial Sequence

<220>
<223> Antisense oligonucleotide

<400> 293
gtatgtagct ataggtggcc 20

<210> 294
<211> 20
<212> DNA

<213> Artificial Sequence

<220>
<223> Antisense oligonucleotide

<400> 294
gcaatgccag gagtatgtag 20

<210> 295
<211> 20
<212> DNA
<213> Artificial Sequence

<220>
<223> Antisense oligonucleotide

<400> 295
ttaaaaagtg caatgccagg 20

<210> 296
<211> 20
<212> DNA
<213> Artificial Sequence

<220>
<223> Antisense oligonucleotide

<400> 296
ggcttagata gtcctatctt 20

<210> 297
<211> 20
<212> DNA
<213> Artificial Sequence

<220>
<223> Antisense oligonucleotide

<400> 297
taaaaagaaa cctagggctt 20

<210> 298
<211> 20
<212> DNA
<213> Artificial Sequence

<220>
<223> Antisense oligonucleotide

<400> 298
atacagaaag gctatgctga 20

<210> 299
<211> 20
<212> DNA
<213> Artificial Sequence

<220>

<223> Antisense oligonucleotide

<400> 299
ttaagtttct taaatacaga 20

<210> 300
<211> 20
<212> DNA
<213> Artificial Sequence

<220>
<223> Antisense oligonucleotide

<400> 300
gcatctgctg cttctccgtc 20

<210> 301
<211> 20
<212> DNA
<213> Artificial Sequence

<220>
<223> Antisense oligonucleotide

<400> 301
cagcatctgc tgcttctccg 20

<210> 302
<211> 20
<212> DNA
<213> Artificial Sequence

<220>
<223> Antisense oligonucleotide

<400> 302
tccagcatct gctgcttctc 20

<210> 303
<211> 20
<212> DNA
<213> Artificial Sequence

<220>
<223> Antisense oligonucleotide

<400> 303
ctccagcatc tgctgcttct 20

<210> 304
<211> 20
<212> DNA
<213> Artificial Sequence

<220>
<223> Antisense oligonucleotide

<400> 304

tgctccagca tctgctgctt 20

<210> 305
<211> 20
<212> DNA
<213> Artificial Sequence

<220>
<223> Antisense oligonucleotide

<400> 305
tgctgctcca gcatctgctg 20

<210> 306
<211> 20
<212> DNA
<213> Artificial Sequence

<220>
<223> Antisense oligonucleotide

<400> 306
ggtgctgctc cagcatctgc 20

<210> 307
<211> 20
<212> DNA
<213> Artificial Sequence

<220>
<223> Antisense oligonucleotide

<400> 307
aaggtgctgc tccagcatct 20

<210> 308
<211> 20
<212> DNA
<213> Artificial Sequence

<220>
<223> Antisense oligonucleotide

<400> 308
tgggattgtt ggtcagcatg 20

<210> 309
<211> 20
<212> DNA
<213> Artificial Sequence

<220>
<223> Antisense oligonucleotide

<400> 309
attcttggga ttgttggtca 20

<210> 310

<211> 20
<212> DNA
<213> Artificial Sequence

<220>
<223> Antisense oligonucleotide

<400> 310
acattcttgg gattgttggt 20

<210> 311
<211> 20
<212> DNA
<213> Artificial Sequence

<220>
<223> Antisense oligonucleotide

<400> 311
cacattcttg ggattgttgg 20

<210> 312
<211> 20
<212> DNA
<213> Artificial Sequence

<220>
<223> Antisense oligonucleotide

<400> 312
ttcacattct tgggattgtt 20

<210> 313
<211> 20
<212> DNA
<213> Artificial Sequence

<220>
<223> Antisense oligonucleotide

<400> 313
agttcacatt cttgggattg 20

<210> 314
<211> 20
<212> DNA
<213> Artificial Sequence

<220>
<223> Antisense oligonucleotide

<400> 314
gaagttcaca ttcttgggat 20

<210> 315
<211> 20
<212> DNA
<213> Artificial Sequence

<220>
<223> Antisense oligonucleotide

<400> 315
agattatgaa acaccaaagt 20

<210> 316
<211> 20
<212> DNA
<213> Artificial Sequence

<220>
<223> Antisense oligonucleotide

<400> 316
ggagattatg aaacaccaaa 20

<210> 317
<211> 20
<212> DNA
<213> Artificial Sequence

<220>
<223> Antisense oligonucleotide

<400> 317
caggagatta tgaaacacca 20

<210> 318
<211> 20
<212> DNA
<213> Artificial Sequence

<220>
<223> Antisense oligonucleotide

<400> 318
tcccaggaga ttatgaaaca 20

<210> 319
<211> 20
<212> DNA
<213> Artificial Sequence

<220>
<223> Antisense oligonucleotide

<400> 319
ctcccaggag attatgaaac 20

<210> 320
<211> 20
<212> DNA
<213> Artificial Sequence

<220>
<223> Antisense oligonucleotide

<400> 320
ctctcccagg agattatgaa 20

<210> 321
<211> 20
<212> DNA
<213> Artificial Sequence

<220>
<223> Antisense oligonucleotide

<400> 321
atctctccca ggagattatg 20

<210> 322
<211> 20
<212> DNA
<213> Artificial Sequence

<220>
<223> Antisense oligonucleotide

<400> 322
cttgccagcc atgttttctt 20

<210> 323
<211> 20
<212> DNA
<213> Artificial Sequence

<220>
<223> Antisense oligonucleotide

<400> 323
cccttgccag ccatgttttc 20

<210> 324
<211> 20
<212> DNA
<213> Artificial Sequence

<220>
<223> Antisense oligonucleotide

<400> 324
agcccttgcc agccatgttt 20

<210> 325
<211> 20
<212> DNA
<213> Artificial Sequence

<220>
<223> Antisense oligonucleotide

<400> 325
gagaagccct tgccagccat 20

<210> 326
<211> 20
<212> DNA
<213> Artificial Sequence

<220>
<223> Antisense oligonucleotide

<400> 326
aggagaagcc cttgccagcc 20

<210> 327
<211> 20
<212> DNA
<213> Artificial Sequence

<220>
<223> Antisense oligonucleotide

<400> 327
gaaggagaag cccttgccag 20

<210> 328
<211> 20
<212> DNA
<213> Artificial Sequence

<220>
<223> Antisense oligonucleotide

<400> 328
aagcccttgc cagccatgtt 20

<210> 329
<211> 20
<212> DNA
<213> Artificial Sequence

<220>
<223> Antisense oligonucleotide

<400> 329
agaagccctt gccagccatg 20

<210> 330
<211> 20
<212> DNA
<213> Artificial Sequence

<220>
<223> Antisense oligonucleotide

<400> 330
agaaggagaa gcccttgcca 20

<210> 331
<211> 20
<212> DNA

<213> Artificial Sequence

<220>
<223> Antisense oligonucleotide

<400> 331
ccagaaggag aagcccttgc 20

<210> 332
<211> 20
<212> DNA
<213> Artificial Sequence

<220>
<223> Antisense oligonucleotide

<400> 332
acccagaagg agaagccctt 20

<210> 333
<211> 20
<212> DNA
<213> Artificial Sequence

<220>
<223> Antisense oligonucleotide

<400> 333
tgcctcctcc ttgggaatgt 20

<210> 334
<211> 20
<212> DNA
<213> Artificial Sequence

<220>
<223> Antisense oligonucleotide

<400> 334
aatgcctcct ccttgggaat 20

<210> 335
<211> 20
<212> DNA
<213> Artificial Sequence

<220>
<223> Antisense oligonucleotide

<400> 335
cgaatgcctc ctccttggga 20

<210> 336
<211> 20
<212> DNA
<213> Artificial Sequence

<220>

<223> Antisense oligonucleotide

<400> 336
ttccgaatgc ctcctccttg 20

<210> 337
<211> 20
<212> DNA
<213> Artificial Sequence

<220>
<223> Antisense oligonucleotide

<400> 337
tttccgaatg cctcctcctt 20

<210> 338
<211> 20
<212> DNA
<213> Artificial Sequence

<220>
<223> Antisense oligonucleotide

<400> 338
actttccgaa tgcctcctcc 20

<210> 339
<211> 20
<212> DNA
<213> Artificial Sequence

<220>
<223> Antisense oligonucleotide

<400> 339
ttgcaggaag cggctatact 20

<210> 340
<211> 20
<212> DNA
<213> Artificial Sequence

<220>
<223> Antisense oligonucleotide

<400> 340
tcttgcagga agcggctata 20

<210> 341
<211> 20
<212> DNA
<213> Artificial Sequence

<220>
<223> Antisense oligonucleotide

<400> 341

actcttgcag gaagcggcta 20

<210> 342
<211> 20
<212> DNA
<213> Artificial Sequence

<220>
<223> Antisense oligonucleotide

<400> 342
gactcttgca ggaagcggct 20

<210> 343
<211> 20
<212> DNA
<213> Artificial Sequence

<220>
<223> Antisense oligonucleotide

<400> 343
tcgactcttg caggaagcgg 20

<210> 344
<211> 20
<212> DNA
<213> Artificial Sequence

<220>
<223> Antisense oligonucleotide

<400> 344
ttcgactctt gcaggaagcg 20

<210> 345
<211> 20
<212> DNA
<213> Artificial Sequence

<220>
<223> Antisense oligonucleotide

<400> 345
cattcgactc ttgcaggaag 20

<210> 346
<211> 20
<212> DNA
<213> Artificial Sequence

<220>
<223> Antisense oligonucleotide

<400> 346
tcttatagcc catgatgatt 20

<210> 347

<211> 20
<212> DNA
<213> Artificial Sequence

<220>
<223> Antisense oligonucleotide

<400> 347
gatcttatag cccatgatga 20

<210> 348
<211> 20
<212> DNA
<213> Artificial Sequence

<220>
<223> Antisense oligonucleotide

<400> 348
atgatcttat agcccatgat 20

<210> 349
<211> 20
<212> DNA
<213> Artificial Sequence

<220>
<223> Antisense oligonucleotide

<400> 349
ccatgatctt atagcccatg 20

<210> 350
<211> 20
<212> DNA
<213> Artificial Sequence

<220>
<223> Antisense oligonucleotide

<400> 350
gcatccatga tcttatagcc 20

<210> 351
<211> 20
<212> DNA
<213> Artificial Sequence

<220>
<223> Antisense oligonucleotide

<400> 351
tagcatccat gatcttatag 20

<210> 352
<211> 20
<212> DNA
<213> Artificial Sequence

<220>
<223> Antisense oligonucleotide

<400> 352
gtagcatcca tgatcttata 20

<210> 353
<211> 20
<212> DNA
<213> Artificial Sequence

<220>
<223> Antisense oligonucleotide

<400> 353
aaaggctatg ctgatacagt 20

<210> 354
<211> 20
<212> DNA
<213> Artificial Sequence

<220>
<223> Antisense oligonucleotide

<400> 354
agaaaggcta tgctgataca 20

<210> 355
<211> 20
<212> DNA
<213> Artificial Sequence

<220>
<223> Antisense oligonucleotide

<400> 355
acagaaaggc tatgctgata 20

<210> 356
<211> 20
<212> DNA
<213> Artificial Sequence

<220>
<223> Antisense oligonucleotide

<400> 356
tacagaaagg ctatgctgat 20

<210> 357
<211> 20
<212> DNA
<213> Artificial Sequence

<220>
<223> Antisense oligonucleotide

<400> 357
aatacagaaa ggctatgctg 20

<210> 358
<211> 20
<212> DNA
<213> Artificial Sequence

<220>
<223> Antisense oligonucleotide

<400> 358
aaatacagaa aggctatgct 20

<210> 359
<211> 20
<212> DNA
<213> Artificial Sequence

<220>
<223> Antisense oligonucleotide

<400> 359
ttaaatacag aaaggctatg 20

<210> 360
<211> 20
<212> DNA
<213> Artificial Sequence

<220>
<223> Antisense oligonucleotide

<400> 360
tcttaaatac agaaaggcta 20

<210> 361
<211> 20
<212> DNA
<213> Artificial Sequence

<220>
<223> Antisense oligonucleotide

<400> 361
ggtctcagag aacacatcct 20

<210> 362
<211> 20
<212> DNA
<213> Artificial Sequence

<220>
<223> Antisense oligonucleotide

<400> 362
tgggtctcag agaacacatc 20

<210> 363
<211> 20
<212> DNA
<213> Artificial Sequence

<220>
<223> Antisense oligonucleotide

<400> 363
catgggtctc agagaacaca 20

<210> 364
<211> 20
<212> DNA
<213> Artificial Sequence

<220>
<223> Antisense oligonucleotide

<400> 364
tcatgggtct cagagaacac 20

<210> 365
<211> 20
<212> DNA
<213> Artificial Sequence

<220>
<223> Antisense oligonucleotide

<400> 365
tgatcatggg tctcagagaa 20

<210> 366
<211> 20
<212> DNA
<213> Artificial Sequence

<220>
<223> Antisense oligonucleotide

<400> 366
cctgatcatg ggtctcagag 20

<210> 367
<211> 20
<212> DNA
<213> Artificial Sequence

<220>
<223> Antisense oligonucleotide

<400> 367
cccctgatca tgggtctcag 20

<210> 368
<211> 20
<212> DNA

<213> Artificial Sequence

<220>
<223> Antisense oligonucleotide

<400> 368
cagacccaga aggagaagcc 20

<210> 369
<211> 20
<212> DNA
<213> Artificial Sequence

<220>
<223> Antisense oligonucleotide

<400> 369
cagccagacc cagaaggaga 20

<210> 370
<211> 20
<212> DNA
<213> Artificial Sequence

<220>
<223> Antisense oligonucleotide

<400> 370
ccagccagac ccagaaggag 20

<210> 371
<211> 20
<212> DNA
<213> Artificial Sequence

<220>
<223> Antisense oligonucleotide

<400> 371
gtccagccag acccagaagg 20

<210> 372
<211> 20
<212> DNA
<213> Artificial Sequence

<220>
<223> Antisense oligonucleotide

<400> 372
tgtccagcca gacccagaag 20

<210> 373
<211> 20
<212> DNA
<213> Artificial Sequence

<220>

<223> Antisense oligonucleotide

<400> 373
attgtccagc cagacccaga 20

<210> 374
<211> 20
<212> DNA
<213> Artificial Sequence

<220>
<223> Antisense oligonucleotide

<400> 374
atattgtcca gccagaccca 20

<210> 375
<211> 20
<212> DNA
<213> Artificial Sequence

<220>
<223> Antisense oligonucleotide

<400> 375
catgatctta tagcccatga 20

<210> 376
<211> 20
<212> DNA
<213> Artificial Sequence

<220>
<223> Antisense oligonucleotide

<400> 376
tccatgatct tatagcccat 20

<210> 377
<211> 20
<212> DNA
<213> Artificial Sequence

<220>
<223> Antisense oligonucleotide

<400> 377
catccatgat cttatagccc 20

<210> 378
<211> 20
<212> DNA
<213> Artificial Sequence

<220>
<223> Antisense oligonucleotide

<400> 378

agcatccatg atcttatagc 20

<210> 379
<211> 20
<212> DNA
<213> Artificial Sequence

<220>
<223> Antisense oligonucleotide

<400> 379
tggtagcatc catgatctta 20

<210> 380
<211> 20
<212> DNA
<213> Artificial Sequence

<220>
<223> Antisense oligonucleotide

<400> 380
ttggtagcat ccatgatctt 20

<210> 381
<211> 20
<212> DNA
<213> Artificial Sequence

<220>
<223> Antisense oligonucleotide

<400> 381
gatattggta gcatccatga 20

<210> 382
<211> 2924
<212> DNA
<213> M. musculus

<220>
<221> CDS
<222> (173)...(2341)

<400> 382

gtcgacccac gcgtccgcgc tgaggtacaa ccccgctcgg tgtcgcctga ccgcgtcggc 60
taggagaggc caggcggccc tcgggagccc agcagctcgc gcctggagtc agcgcaggcc 120
ggccagtcgg gcctcagccc cggagacagt cgagacccct gactgcagca gg atg gct 178
Met Ala
1

cag tgg aac cag ctg cag cag ctg gac aca cgc tac ctg gag cag ctg 226
Gln Trp Asn Gln Leu Gln Gln Leu Asp Thr Arg Tyr Leu Glu Gln Leu
5 10 15

cac cag ctg tac agc gac acg ttc ccc atg gag ctg cgg cag ttc ctg 274
His Gln Leu Tyr Ser Asp Thr Phe Pro Met Glu Leu Arg Gln Phe Leu
20 25 30

gca cct tgg att gag agt caa gac tgg gca tat gca gcc agc aaa gag 322
Ala Pro Trp Ile Glu Ser Gln Asp Trp Ala Tyr Ala Ala Ser Lys Glu
35 40 45 50

tca cat gcc acg ttg gtg ttt cat aat ctc ttg ggt gaa att gac cag 370
Ser His Ala Thr Leu Val Phe His Asn Leu Leu Gly Glu Ile Asp Gln
55 60 65

caa tat agc cga ttc ctg caa gag tcc aat gtc ctc tat cag cac aac 418
Gln Tyr Ser Arg Phe Leu Gln Glu Ser Asn Val Leu Tyr Gln His Asn
70 75 80

ctt cga aga atc aag cag ttt ctg cag agc agg tat ctt gag aag cca 466
Leu Arg Arg Ile Lys Gln Phe Leu Gln Ser Arg Tyr Leu Glu Lys Pro
85 90 95

atg gaa att gcc cgg atc gtg gcc cga tgc ctg tgg gaa gag tct cgc 514
Met Glu Ile Ala Arg Ile Val Ala Arg Cys Leu Trp Glu Glu Ser Arg
100 105 110

ctc ctc cag acg gca gcc acg gca gcc cag caa ggg ggc cag gcc aac 562
Leu Leu Gln Thr Ala Ala Thr Ala Ala Gln Gln Gly Gly Gln Ala Asn
115 120 125 130

cac cca aca gcc gcc gta gtg aca gag aag cag cag atg ttg gag cag 610
His Pro Thr Ala Ala Val Val Thr Glu Lys Gln Gln Met Leu Glu Gln
135 140 145

cat ctt cag gat gtc cgg aag cga gtg cag gat cta gaa cag aaa atg 658
His Leu Gln Asp Val Arg Lys Arg Val Gln Asp Leu Glu Gln Lys Met
150 155 160

aag gtg gtg gag aac ctc cag gac gac ttt gat ttc aac tac aaa acc 706
Lys Val Val Glu Asn Leu Gln Asp Asp Phe Asp Phe Asn Tyr Lys Thr
165 170 175

ctc aag agc caa gga gac atg cag gat ctg aat gga aac aac cag tct 754
Leu Lys Ser Gln Gly Asp Met Gln Asp Leu Asn Gly Asn Asn Gln Ser
180 185 190

```
gtg acc aga cag aag atg cag cag ctg gaa cag atg ctc aca gcc ctg      802
Val Thr Arg Gln Lys Met Gln Gln Leu Glu Gln Met Leu Thr Ala Leu
195                 200                 205                 210

gac cag atg cgg aga agc att gtg agt gag ctg gcg ggg ctc ttg tca      850
Asp Gln Met Arg Arg Ser Ile Val Ser Glu Leu Ala Gly Leu Leu Ser
                215                 220                 225

gca atg gag tac gtg cag aag aca ctg act gat gaa gag ctg gct gac      898
Ala Met Glu Tyr Val Gln Lys Thr Leu Thr Asp Glu Glu Leu Ala Asp
            230                 235                 240

tgg aag agg cgg cag cag atc gcg tgc atc gga ggc cct ccc aac atc      946
Trp Lys Arg Arg Gln Gln Ile Ala Cys Ile Gly Gly Pro Pro Asn Ile
        245                 250                 255

tgc ctg gac cgt ctg gaa aac tgg ata act tca tta gca gaa tct caa      994
Cys Leu Asp Arg Leu Glu Asn Trp Ile Thr Ser Leu Ala Glu Ser Gln
    260                 265                 270

ctt cag acc cgc caa caa att aag aaa ctg gag gag ctg cag cag aaa     1042
Leu Gln Thr Arg Gln Gln Ile Lys Lys Leu Glu Glu Leu Gln Gln Lys
275                 280                 285                 290

gtg tcc tac aag ggc gac cct atc gtg cag cac cgg ccc atg ctg gag     1090
Val Ser Tyr Lys Gly Asp Pro Ile Val Gln His Arg Pro Met Leu Glu
                295                 300                 305

gag agg atc gtg gag ctg ttc aga aac tta atg aag agt gcc ttc gtg     1138
Glu Arg Ile Val Glu Leu Phe Arg Asn Leu Met Lys Ser Ala Phe Val
            310                 315                 320

gtg gag cgg cag ccc tgc atg ccc atg cac ccg gac cgg ccc tta gtc     1186
Val Glu Arg Gln Pro Cys Met Pro Met His Pro Asp Arg Pro Leu Val
        325                 330                 335

atc aag act ggt gtc cag ttt acc acg aaa gtc agg ttg ctg gtc aaa     1234
Ile Lys Thr Gly Val Gln Phe Thr Thr Lys Val Arg Leu Leu Val Lys
    340                 345                 350

ttt cct gag ttg aat tat cag ctt aaa att aaa gtg tgc att gat aaa     1282
Phe Pro Glu Leu Asn Tyr Gln Leu Lys Ile Lys Val Cys Ile Asp Lys
355                 360                 365                 370

gac tct ggg gat gtt gct gcc ctc aga ggg tct cgg aaa ttt aac att     1330
Asp Ser Gly Asp Val Ala Ala Leu Arg Gly Ser Arg Lys Phe Asn Ile
                375                 380                 385

ctg ggc acg aac aca aaa gtg atg aac atg gag gag tct aac aac ggc     1378
Leu Gly Thr Asn Thr Lys Val Met Asn Met Glu Glu Ser Asn Asn Gly
            390                 395                 400

agc ctg tct gca gag ttc aag cac ctg acc ctt agg gag cag aga tgt     1426
Ser Leu Ser Ala Glu Phe Lys His Leu Thr Leu Arg Glu Gln Arg Cys
        405                 410                 415

ggg aat gga ggc cgt gcc aat tgt gat gcc tcc ttg atc gtg act gag     1474
Gly Asn Gly Gly Arg Ala Asn Cys Asp Ala Ser Leu Ile Val Thr Glu
    420                 425                 430

gag ctg cac ctg atc acc ttc gag act gag gtg tac cac caa ggc ctc     1522
Glu Leu His Leu Ile Thr Phe Glu Thr Glu Val Tyr His Gln Gly Leu
435                 440                 445                 450
```

```
aag att gac cta gag acc cac tcc ttg cca gtt gtg gtg atc tcc aac   1570
Lys Ile Asp Leu Glu Thr His Ser Leu Pro Val Val Val Ile Ser Asn
                455                 460                 465

atc tgt cag atg cca aat gct tgg gca tca atc ctg tgg tat aac atg   1618
Ile Cys Gln Met Pro Asn Ala Trp Ala Ser Ile Leu Trp Tyr Asn Met
            470                 475                 480

ctg acc aat aac ccc aag aac gtg aac ttc ttc act aag ccg cca att   1666
Leu Thr Asn Asn Pro Lys Asn Val Asn Phe Phe Thr Lys Pro Pro Ile
        485                 490                 495

gga acc tgg gac caa gtg gcc gag gtg ctc agc tgg cag ttc tcg tcc   1714
Gly Thr Trp Asp Gln Val Ala Glu Val Leu Ser Trp Gln Phe Ser Ser
    500                 505                 510

acc acc aag cga ggg ctg agc atc gag cag ctg aca acg ctg gct gag   1762
Thr Thr Lys Arg Gly Leu Ser Ile Glu Gln Leu Thr Thr Leu Ala Glu
515                 520                 525                 530

aag ctc cta ggg cct ggt gtg aac tac tca ggg tgt cag atc aca tgg   1810
Lys Leu Leu Gly Pro Gly Val Asn Tyr Ser Gly Cys Gln Ile Thr Trp
                535                 540                 545

gct aaa ttc tgc aaa gaa aac atg gct ggc aag ggc ttc tcc ttc tgg   1858
Ala Lys Phe Cys Lys Glu Asn Met Ala Gly Lys Gly Phe Ser Phe Trp
            550                 555                 560

gtc tgg cta gac aat atc atc gac ctt gtg aaa aag tat atc ttg gcc   1906
Val Trp Leu Asp Asn Ile Ile Asp Leu Val Lys Lys Tyr Ile Leu Ala
        565                 570                 575

ctt tgg aat gaa ggg tac atc atg ggt ttc atc agc aag gag cgg gag   1954
Leu Trp Asn Glu Gly Tyr Ile Met Gly Phe Ile Ser Lys Glu Arg Glu
    580                 585                 590

cgg gcc atc cta agc aca aag ccc ccg ggc acc ttc cta ctg cgc ttc   2002
Arg Ala Ile Leu Ser Thr Lys Pro Pro Gly Thr Phe Leu Leu Arg Phe
595                 600                 605                 610

agc gag agc agc aaa gaa gga ggg gtc act ttc act tgg gtg gaa aag   2050
Ser Glu Ser Ser Lys Glu Gly Gly Val Thr Phe Thr Trp Val Glu Lys
                615                 620                 625

gac atc agt ggc aag acc cag atc cag tct gta gag cca tac acc aag   2098
Asp Ile Ser Gly Lys Thr Gln Ile Gln Ser Val Glu Pro Tyr Thr Lys
            630                 635                 640

cag cag ctg aac aac atg tca ttt gct gaa atc atc atg ggc tat aag   2146
Gln Gln Leu Asn Asn Met Ser Phe Ala Glu Ile Ile Met Gly Tyr Lys
        645                 650                 655

atc atg gat gcg acc aac atc ctg gtg tct cca ctt gtc tac ctc tac   2194
Ile Met Asp Ala Thr Asn Ile Leu Val Ser Pro Leu Val Tyr Leu Tyr
    660                 665                 670

ccc gac att ccc aag gag gag gca ttt gga aag tac tgt agg ccc gag   2242
Pro Asp Ile Pro Lys Glu Glu Ala Phe Gly Lys Tyr Cys Arg Pro Glu
675                 680                 685                 690

agc cag gag cac ccc gaa gcc gac cca ggt agt gct gcc ccg tac ctg   2290
Ser Gln Glu His Pro Glu Ala Asp Pro Gly Ser Ala Ala Pro Tyr Leu
                695                 700                 705

aag acc aag ttc atc tgt gtg aca cca ttc att gat gca gtt tgg aaa   2338
```

```
Lys Thr Lys Phe Ile Cys Val Thr Pro Phe Ile Asp Ala Val Trp Lys
            710                 715                 720

taa cggtgaaggt gctgagccct cagcaggagg gcagtttgag tcgctcacgt       2391
 *

ttgacatgga tctgacctcg gagtgtgcta cctcccccat gtgaggagct gaaaccagaa 2451
gctgcagaga cgtgacttga gacacctgcc ccgtgctcca cccctaagca gccgaacccc 2511
atatcgtctg aaactcctaa ctttgtggtt ccagattttt ttttttaatt tcctacttct 2571
gctatctttg ggcaatctgg gcacttttta aaagagagaa atgagtgagt gtgggtgata 2631
aactgttatg taaagaggag agcacctctg agtctgggga tggggctgag agcagaaggg 2691
aggcaaaggg gaacacctcc tgtcctgccc gcctgccctc ctttttcagc agctcggggg 2751
ttggttgtta gacaagtgcc tcctggtgcc catggctacc tgttgcccca ctctgtgagc 2811
tgatacccca ttctgggaac tcctggctct gcactttcaa ccttgctaat atccacatag 2871
aagctaggac taagcccagg aggttcctct ttaaattaaa aaaaaaaaaa aaa        2924
```

<210> 383
<211> 20
<212> DNA
<213> Artificial Sequence

<220>
<223> Antisense oligonucleotide

<400> 383
tggtattgct gcaggtcgtt 20

<210> 384
<211> 20
<212> DNA
<213> Artificial Sequence

<220>
<223> Antisense oligonucleotide

<400> 384
cggcaggtca atggtattgc 20

<210> 385
<211> 20
<212> DNA
<213> Artificial Sequence

<220>
<223> Antisense oligonucleotide

<400> 385
ggacatcggc aggtcaatgg 20

<210> 386
<211> 20
<212> DNA
<213> Artificial Sequence

<220>
<223> Antisense oligonucleotide

<400> 386
ttgtacctca gcgcggacgc 20

<210> 387
<211> 19
<212> DNA
<213> Artificial Sequence

<220>
<223> Antisense oligonucleotide

<400> 387
aaaagtgccc agattgccc 19

<210> 388
<211> 18
<212> DNA
<213> Artificial Sequence

<220>
<223> Antisense oligonucleotide

<400> 388
aaaagtgccc agattgcc 18

<210> 389
<211> 17
<212> DNA
<213> Artificial Sequence

<220>
<223> Antisense oligonucleotide

<400> 389
aaagtgccca gattgcc 17

<210> 390
<211> 20
<212> DNA
<213> Artificial Sequence

<220>
<223> Antisense oligonucleotide

<400> 390
gctgcaggtc gttggtgtca 20

<210> 391
<211> 20
<212> DNA
<213> Artificial Sequence

<220>
<223> Antisense oligonucleotide

<400> 391
ttctacctcg cgcgatttac 20

<210> 392
<211> 20
<212> DNA
<213> Artificial Sequence

<220>
<223> Antisense oligonucleotide

<400> 392
gtacagttat gcgcggtaga 20

<210> 393
<211> 20
<212> DNA
<213> Artificial Sequence

<220>
<223> Antisense oligonucleotide

<400> 393
ttagaatacg tcgcgttatg 20

<210> 394
<211> 20
<212> DNA
<213> Artificial Sequence

<220>
<223> Antisense oligonucleotide

<400> 394
cgttattaac ctccgttgaa 20

<210> 395
<211> 20
<212> DNA
<213> Artificial Sequence

<220>
<223> Antisense oligonucleotide

<400> 395
ctgctagcct ctggatttga 20

<210> 396
<211> 20
<212> DNA
<213> Artificial Sequence

<220>
<223> Antisense oligonucleotide

<400> 396
ctcttactgt gctgtggaca 20

<210> 397
<211> 15
<212> DNA

<213> Artificial Sequence

<220>
<223> PCR Primer

<400> 397
gaggcccgcc caaca 15

<210> 398
<211> 26
<212> DNA
<213> Artificial Sequence

<220>
<223> PCR Primer

<400> 398
ttctgctaat gacgttatcc agtttt 26

<210> 399
<211> 14
<212> DNA
<213> Artificial Sequence

<220>
<223> PCR Probe

<400> 399
ctgcctagat cggc 14

<210> 400
<211> 21
<212> DNA
<213> Artificial Sequence

<220>
<223> Antisense Oligonucleotide

<400> 400
attcttggga ttgttggtct t 21

<210> 401
<211> 21
<212> DNA
<213> Artificial Sequence

<220>
<223> Antisense Oligonucleotide

<400> 401
ctccagcatc tgctgcttct t 21

<210> 402
<211> 20
<212> DNA
<213> Artificial Sequence

<220>

<223> Antisense Oligonucleotide

<400> 402
tttgatcgag gttagccgtg 20

<210> 403
<211> 20
<212> DNA
<213> Artificial Sequence

<220>
<223> Antisense Oligonucleotide

<400> 403
actcaggcta cctttctgct 20

<210> 404
<211> 20
<212> DNA
<213> Artificial Sequence

<220>
<223> Antisense Oligonucleotide

<400> 404
gctccaatac ccgttgcttc 20

<210> 405
<211> 20
<212> DNA
<213> Artificial Sequence

<220>
<223> Antisense Oligonucleotide

<400> 405
ccgctctgct atagaggcac 20

<210> 406
<211> 20
<212> DNA
<213> Artificial Sequence

<220>
<223> Antisense Oligonucleotide

<400> 406
aaaagtatct aaatcgctca 20

<210> 407
<211> 20
<212> DNA
<213> Artificial Sequence

<220>
<223> Antisense Oligonucleotide

<400> 407

taaaagcgtc taagttgctc 20

<210> 408
<211> 20
<212> DNA
<213> Artificial Sequence

<220>
<223> Antisense Oligonucleotide

<400> 408
aggagaagcc cttgccagcc 20

<210> 409
<211> 20
<212> DNA
<213> Artificial Sequence

<220>
<223> Antisense Oligonucleotide

<400> 409
ccttccctga aggttcctcc 20

<210> 410
<211> 20
<212> DNA
<213> Artificial Sequence

<220>
<223> Antisense Oligonucleotide

<400> 410
atgcatacta cgaaaggccg 20

<210> 411
<211> 20
<212> DNA
<213> Artificial Sequence

<220>
<223> Antisense Oligonucleotide

<400> 411
ttcgcggctg gacgattcag 20

<210> 412
<211> 4978
<212> DNA
<213> H. sapiens

<220>
<221> CDS
<222> (241)...(2553)

<400> 412

```
ggtttccgga gctgcggcgg cgcagactgg gagggggagc cgggggttcc gacgtcgcag 60
ccgagggaac aagccccaac cggatcctgg acaggcaccc cggcttggcg ctgtctctcc 120
ccctcggctc ggagaggccc ttcggcctga gggagcctcg ccgcccgtcc ccggcacacg 180
cgcagccccg gcctctcggc ctctgccgga gaaacagttg ggacccctga ttttagcagg 240
atg gcc caa tgg aat cag cta cag cag ctt gac aca cgg tac ctg gag    288
Met Ala Gln Trp Asn Gln Leu Gln Gln Leu Asp Thr Arg Tyr Leu Glu
 1               5                  10                  15

cag ctc cat cag ctc tac agt gac agc ttc cca atg gag ctg cgg cag    336
Gln Leu His Gln Leu Tyr Ser Asp Ser Phe Pro Met Glu Leu Arg Gln
            20                  25                  30

ttt ctg gcc cct tgg att gag agt caa gat tgg gca tat gcg gcc agc    384
Phe Leu Ala Pro Trp Ile Glu Ser Gln Asp Trp Ala Tyr Ala Ala Ser
        35                  40                  45

aaa gaa tca cat gcc act ttg gtg ttt cat aat ctc ctg gga gag att    432
Lys Glu Ser His Ala Thr Leu Val Phe His Asn Leu Leu Gly Glu Ile
    50                  55                  60

gac cag cag tat agc cgc ttc ctg caa gag tcg aat gtt ctc tat cag    480
Asp Gln Gln Tyr Ser Arg Phe Leu Gln Glu Ser Asn Val Leu Tyr Gln
 65                  70                  75                  80

cac aat cta cga aga atc aag cag ttt ctt cag agc agg tat ctt gag    528
His Asn Leu Arg Arg Ile Lys Gln Phe Leu Gln Ser Arg Tyr Leu Glu
                85                  90                  95

aag cca atg gag att gcc cgg att gtg gcc cgg tgc ctg tgg gaa gaa    576
Lys Pro Met Glu Ile Ala Arg Ile Val Ala Arg Cys Leu Trp Glu Glu
            100                 105                 110

tca cgc ctt cta cag act gca gcc act gcg gcc cag caa ggg ggc cag    624
Ser Arg Leu Leu Gln Thr Ala Ala Thr Ala Ala Gln Gln Gly Gly Gln
        115                 120                 125

gcc aac cac ccc aca gca gcc gtg gtg acg gag aag cag cag atg ctg    672
Ala Asn His Pro Thr Ala Ala Val Val Thr Glu Lys Gln Gln Met Leu
    130                 135                 140

gag cag cac ctt cag gat gtc cgg aag aga gtg cag gat cta gaa cag    720
Glu Gln His Leu Gln Asp Val Arg Lys Arg Val Gln Asp Leu Glu Gln
145                 150                 155                 160
```

```
aaa atg aaa gtg gta gag aat ctc cag gat gac ttt gat ttc aac tat     768
Lys Met Lys Val Val Glu Asn Leu Gln Asp Asp Phe Asp Phe Asn Tyr
                165                 170                 175

aaa acc ctc aag agt caa gga gac atg caa gat ctg aat gga aac aac     816
Lys Thr Leu Lys Ser Gln Gly Asp Met Gln Asp Leu Asn Gly Asn Asn
            180                 185                 190

cag tca gtg acc agg cag aag atg cag cag ctg gaa cag atg ctc act     864
Gln Ser Val Thr Arg Gln Lys Met Gln Gln Leu Glu Gln Met Leu Thr
        195                 200                 205

gcg ctg gac cag atg cgg aga agc atc gtg agt gag ctg gcg ggg ctt     912
Ala Leu Asp Gln Met Arg Arg Ser Ile Val Ser Glu Leu Ala Gly Leu
    210                 215                 220

ttg tca gcg atg gag tac gtg cag aaa act ctc acg gac gag gag ctg     960
Leu Ser Ala Met Glu Tyr Val Gln Lys Thr Leu Thr Asp Glu Glu Leu
225                 230                 235                 240

gct gac tgg aag agg cgg caa cag att gcc tgc att gga ggc ccg ccc     1008
Ala Asp Trp Lys Arg Arg Gln Gln Ile Ala Cys Ile Gly Gly Pro Pro
                245                 250                 255

aac atc tgc cta gat cgg cta gaa aac tgg ata acg tca tta gca gaa     1056
Asn Ile Cys Leu Asp Arg Leu Glu Asn Trp Ile Thr Ser Leu Ala Glu
            260                 265                 270

tct caa ctt cag acc cgt caa caa att aag aaa ctg gag gag ttg cag     1104
Ser Gln Leu Gln Thr Arg Gln Gln Ile Lys Lys Leu Glu Glu Leu Gln
        275                 280                 285

caa aaa gtt tcc tac aaa ggg gac ccc att gta cag cac cgg ccg atg     1152
Gln Lys Val Ser Tyr Lys Gly Asp Pro Ile Val Gln His Arg Pro Met
    290                 295                 300

ctg gag gag aga atc gtg gag ctg ttt aga aac tta atg aaa agt gcc     1200
Leu Glu Glu Arg Ile Val Glu Leu Phe Arg Asn Leu Met Lys Ser Ala
305                 310                 315                 320

ttt gtg gtg gag cgg cag ccc tgc atg ccc atg cat cct gac cgg ccc     1248
Phe Val Val Glu Arg Gln Pro Cys Met Pro Met His Pro Asp Arg Pro
                325                 330                 335

ctc gtc atc aag acc ggc gtc cag ttc act act aaa gtc agg ttg ctg     1296
Leu Val Ile Lys Thr Gly Val Gln Phe Thr Thr Lys Val Arg Leu Leu
            340                 345                 350

gtc aaa ttc cct gag ttg aat tat cag ctt aaa att aaa gtg tgc att     1344
Val Lys Phe Pro Glu Leu Asn Tyr Gln Leu Lys Ile Lys Val Cys Ile
        355                 360                 365

gac aaa gac tct ggg gac gtt gca gct ctc aga gga tcc cgg aaa ttt     1392
Asp Lys Asp Ser Gly Asp Val Ala Ala Leu Arg Gly Ser Arg Lys Phe
    370                 375                 380

aac att ctg ggc aca aac aca aaa gtg atg aac atg gaa gaa tcc aac     1440
Asn Ile Leu Gly Thr Asn Thr Lys Val Met Asn Met Glu Glu Ser Asn
385                 390                 395                 400

aac ggc agc ctc tct gca gaa ttc aaa cac ttg acc ctg agg gag cag     1488
Asn Gly Ser Leu Ser Ala Glu Phe Lys His Leu Thr Leu Arg Glu Gln
                405                 410                 415
```

```
aga tgt ggg aat ggg ggc cga gcc aat tgt gat gct tcc ctg att gtg   1536
Arg Cys Gly Asn Gly Gly Arg Ala Asn Cys Asp Ala Ser Leu Ile Val
            420                 425                 430

act gag gag ctg cac ctg atc acc ttt gag acc gag gtg tat cac caa   1584
Thr Glu Glu Leu His Leu Ile Thr Phe Glu Thr Glu Val Tyr His Gln
        435                 440                 445

ggc ctc aag att gac cta gag acc cac tcc ttg cca gtt gtg gtg atc   1632
Gly Leu Lys Ile Asp Leu Glu Thr His Ser Leu Pro Val Val Val Ile
    450                 455                 460

tcc aac atc tgt cag atg cca aat gcc tgg gcg tcc atc ctg tgg tac   1680
Ser Asn Ile Cys Gln Met Pro Asn Ala Trp Ala Ser Ile Leu Trp Tyr
465                 470                 475                 480

aac atg ctg acc aac aat ccc aag aat gta aac ttt ttt acc aag ccc   1728
Asn Met Leu Thr Asn Asn Pro Lys Asn Val Asn Phe Phe Thr Lys Pro
                485                 490                 495

cca att gga acc tgg gat caa gtg gcc gag gtc ctg agc tgg cag ttc   1776
Pro Ile Gly Thr Trp Asp Gln Val Ala Glu Val Leu Ser Trp Gln Phe
            500                 505                 510

tcc tcc acc acc aag cga gga ctg agc atc gag cag ctg act aca ctg   1824
Ser Ser Thr Thr Lys Arg Gly Leu Ser Ile Glu Gln Leu Thr Thr Leu
        515                 520                 525

gca gag aaa ctc ttg gga cct ggt gtg aat tat tca ggg tgt cag atc   1872
Ala Glu Lys Leu Leu Gly Pro Gly Val Asn Tyr Ser Gly Cys Gln Ile
    530                 535                 540

aca tgg gct aaa ttt tgc aaa gaa aac atg gct ggc aag ggc ttc tcc   1920
Thr Trp Ala Lys Phe Cys Lys Glu Asn Met Ala Gly Lys Gly Phe Ser
545                 550                 555                 560

ttc tgg gtc tgg ctg gac aat atc att gac ctt gtg aaa aag tac atc   1968
Phe Trp Val Trp Leu Asp Asn Ile Ile Asp Leu Val Lys Lys Tyr Ile
                565                 570                 575

ctg gcc ctt tgg aac gaa ggg tac atc atg ggc ttt atc agt aag gag   2016
Leu Ala Leu Trp Asn Glu Gly Tyr Ile Met Gly Phe Ile Ser Lys Glu
            580                 585                 590

cgg gag cgg gcc atc ttg agc act aag cct cca ggc acc ttc ctg cta   2064
Arg Glu Arg Ala Ile Leu Ser Thr Lys Pro Pro Gly Thr Phe Leu Leu
        595                 600                 605

aga ttc agt gaa agc agc aaa gaa gga ggc gtc act ttc act tgg gtg   2112
Arg Phe Ser Glu Ser Ser Lys Glu Gly Gly Val Thr Phe Thr Trp Val
    610                 615                 620

gag aag gac atc agc ggt aag acc cag atc cag tcc gtg gaa cca tac   2160
Glu Lys Asp Ile Ser Gly Lys Thr Gln Ile Gln Ser Val Glu Pro Tyr
625                 630                 635                 640

aca aag cag cag ctg aac aac atg tca ttt gct gaa atc atc atg ggc   2208
Thr Lys Gln Gln Leu Asn Asn Met Ser Phe Ala Glu Ile Ile Met Gly
                645                 650                 655

tat aag atc atg gat gct acc aat atc ctg gtg tct cca ctg gtc tat   2256
Tyr Lys Ile Met Asp Ala Thr Asn Ile Leu Val Ser Pro Leu Val Tyr
            660                 665                 670

ctc tat cct gac att ccc aag gag gag gca ttc gga aag tat tgt cgg   2304
```

```
Leu Tyr Pro Asp Ile Pro Lys Glu Glu Ala Phe Gly Lys Tyr Cys Arg
        675                 680                 685

cca gag agc cag gag cat cct gaa gct gac cca ggt agc gct gcc cca    2352
Pro Glu Ser Gln Glu His Pro Glu Ala Asp Pro Gly Ser Ala Ala Pro
    690                 695                 700

tac ctg aag acc aag ttt atc tgt gtg aca cca acg acc tgc agc aat    2400
Tyr Leu Lys Thr Lys Phe Ile Cys Val Thr Pro Thr Thr Cys Ser Asn
705                 710                 715                 720

acc att gac ctg ccg atg tcc ccc cgc act tta gat tca ttg atg cag    2448
Thr Ile Asp Leu Pro Met Ser Pro Arg Thr Leu Asp Ser Leu Met Gln
                725                 730                 735

ttt gga aat aat ggt gaa ggt gct gaa ccc tca gca gga ggg cag ttt    2496
Phe Gly Asn Asn Gly Glu Gly Ala Glu Pro Ser Ala Gly Gly Gln Phe
            740                 745                 750

gag tcc ctc acc ttt gac atg gag ttg acc tcg gag tgc gct acc tcc    2544
Glu Ser Leu Thr Phe Asp Met Glu Leu Thr Ser Glu Cys Ala Thr Ser
        755                 760                 765

ccc atg tga ggagctgaga acggaagctg cagaaagata cgactgaggc            2593
Pro Met  *
    770

gcctacctgc attctgccac ccctcacaca gccaaacccc agatcatctg aaactactaa 2653
ctttgtggtt ccagattttt tttaatctcc tacttctgct atctttgagc aatctgggca 2713
cttttaaaaa tagagaaatg agtgaatgtg ggtgatctgc ttttatctaa atgcaaataa 2773
ggatgtgttc tctgagaccc atgatcaggg gatgtggcgg ggggtggcta gagggagaaa 2833
aaggaaatgt cttgtgttgt tttgttcccc tgccctcctt tctcagcagc tttttgttat 2893
tgttgttgtt gttcttagac aagtgcctcc tggtgcctgc ggcatccttc tgcctgtttc 2953
tgtaagcaaa tgccacaggc cacctatagc tacatactcc tggcattgca ctttttaacc 3013
ttgctgacat ccaaatagaa gataggacta tctaagccct aggtttcttt ttaaattaag 3073
aaataataac aattaaaggg caaaaaacac tgtatcagca tagcctttct gtatttaaga 3133
aacttaagca gccgggcatg gtggctcacg cctgtaatcc cagcactttg ggaggccgag 3193
gcggatcata aggtcaggag atcaagacca tcctggctaa cacggtgaaa ccccgtctct 3253
actaaaagta caaaaaatta gctgggtgtg gtggtgggcg cctgtagtcc cagctactcg 3313
ggaggctgag gcaggagaat cgcttgaacc tgagaggcgg aggttgcagt gagccaaaat 3373
tgcaccactg cacactgcac tccatcctgg gcgacagtct gagactctgt ctcaaaaaaa 3433
aaaaaaaaaa aaagaaactt cagttaacag cctccttggt gctttaagca ttcagcttcc 3493
ttcaggctgg taatttatat aatccctgaa acgggcttca ggtcaaaccc ttaagacatc 3553
tgaagctgca acctggcctt tggtgttgaa ataggaaggt ttaaggagaa tctaagcatt 3613
ttagactttt ttttataaat agacttattt tcctttgtaa tgtattggcc ttttagtgag 3673
taaggctggg cagagggtgc ttacaacctt gactcccttt ctccctggac ttgatctgct 3733
gtttcagagg ctaggttgtt tctgtgggtg ccttatcagg gctgggatac ttctgattct 3793
ggcttccttc ctgccccacc ctcccgaccc cagtccccct gatcctgcta gaggcatgtc 3853
tccttgcgtg tctaaaggtc cctcatcctg tttgttttag gaatcctggt ctcaggacct 3913
catggaagaa gaggggggaga gagttacagg ttggacatga tgcacactat ggggccccag 3973
cgacgtgtct ggttgagctc agggaatatg gttcttagcc agtttcttgg tgatatccag 4033
tggcacttgt aatggcgtct tcattcagtt catgcagggc aaaggcttac tgataaactt 4093
gagtctgccc tcgtatgagg gtgtatacct ggcctccctc tgaggctggt gactcctccc 4153
tgctgggggcc ccacaggtga ggcagaacag ctagagggcc tccccgcctg cccgccttgg 4213
ctggctagct cgcctctcct gtgcgtatgg gaacacctag cacgtgctgg atgggctgcc 4273
tctgactcag aggcatggcc ggatttggca actcaaaacc accttgcctc agctgatcag 4333
agtttctgtg gaattctgtt tgttaaatca aattagctgg tctctgaatt aaggggggaga 4393
cgaccttctc taagatgaac agggttcgcc ccagtcctcc tgcctggaga cagttgatgt 4453
gtcatgcaga gctcttactt ctccagcaac actcttcagt acataataag cttaactgat 4513
aaacagaata tttagaaagg tgagacttgg gcttaccatt gggtttaaat catagggacc 4573
tagggcgagg gttcagggct tctctggagc agatattgtc aagttcatgg ccttaggtag 4633
catgtatctg gtcttaactc tgattgtagc aaaagttctg agaggagctg agccctgttg 4693
tggcccatta aagaacaggg tcctcaggcc ctgcccgctt cctgtccact gccccctccc 4753
catccccagc ccagccgagg gaatcccgtg ggttgcttac ctacctataa ggtggtttat 4813
aagctgctgt cctggccact gcattcaaat tccaatgtgt acttcatagt gtaaaaattt 4873
```

atattattgt gaggtttttt gtcttttttt tttttttttt tttttggtat attgctgtat 4933
ctactttaac ttccagaaat aaacgttata taggaaccgt aaaaa 4978

<210> 413
<211> 20
<212> RNA
<213> Artificial Sequence

<220>
<223> Oligomeric compound

<400> 413
uuugucucug guccuuacuu 20

<210> 414
<211> 19
<212> RNA
<213> Artificial Sequence

<220>
<223> Oligomeric compound

<400> 414
uuaucgcuuc ucguugcuu 19

<210> 415
<211> 20
<212> DNA
<213> Artificial Sequence

<220>
<223> Antisense Oligonucleotide

<400> 415
tagtgcggac ctacccacga 20

<210> 416
<211> 19
<212> RNA
<213> Artificial Sequence

<220>
<223> Oligomeric compound

<400> 416
ccauuggcuu cucaagaua 19

<210> 417
<211> 19
<212> RNA
<213> Artificial Sequence

<220>
<223> Oligomeric compound

<400> 417
gccccgccag cucacucac 19

<210> 418
<211> 19
<212> RNA
<213> Artificial Sequence

<220>
<223> Oligomeric compound

<400> 418
gaguuuucug cacguacuc 19

<210> 419
<211> 19
<212> RNA
<213> Artificial Sequence

<220>
<223> Oligomeric compound

<400> 419
ccaguuucuu aauuuguug 19

<210> 420
<211> 19
<212> RNA
<213> Artificial Sequence

<220>
<223> Oligomeric compound

<400> 420
aaacuuuuug cugcaacuc 19

<210> 421
<211> 19
<212> RNA
<213> Artificial Sequence

<220>
<223> Oligomeric compound

<400> 421
uuaaguuucu aaacagcuc 19

<210> 422
<211> 19
<212> RNA
<213> Artificial Sequence

<220>
<223> Oligomeric compound

<400> 422
aauuuuaagc ugauaauuc 19

<210> 423
<211> 19
<212> RNA

<213> Artificial Sequence

<220>
<223> Oligomeric compound

<400> 423
gagucuuugu caaugcaca 19

<210> 424
<211> 19
<212> RNA
<213> Artificial Sequence

<220>
<223> Oligomeric compound

<400> 424
uuguuggauu cuuccaugu 19

<210> 425
<211> 19
<212> RNA
<213> Artificial Sequence

<220>
<223> Oligomeric compound

<400> 425
gugggucucu aggucaauc 19

<210> 426
<211> 19
<212> RNA
<213> Artificial Sequence

<220>
<223> Oligomeric compound

<400> 426
aacuggcaag gaguggguc 19

<210> 427
<211> 19
<212> RNA
<213> Artificial Sequence

<220>
<223> Oligomeric compound

<400> 427
cugaauaauu cacaccagg 19

<210> 428
<211> 19
<212> RNA
<213> Artificial Sequence

<220>

<223> Oligomeric compound

<400> 428
auuuagccca ugugaucug 19

<210> 429
<211> 19
<212> RNA
<213> Artificial Sequence

<220>
<223> Oligomeric compound

<400> 429
ucuuugcaaa auuuagccc 19

<210> 430
<211> 19
<212> RNA
<213> Artificial Sequence

<220>
<223> Oligomeric compound

<400> 430
cagccauguu uucuuugca 19

<210> 431
<211> 19
<212> RNA
<213> Artificial Sequence

<220>
<223> Oligomeric compound

<400> 431
aggucaauga uauugucca 19

<210> 432
<211> 19
<212> RNA
<213> Artificial Sequence

<220>
<223> Oligomeric compound

<400> 432
cuuuuucaca aggucaaug 19

<210> 433
<211> 19
<212> RNA
<213> Artificial Sequence

<220>
<223> Oligomeric compound

<400> 433

ccaggaugua cuuuuucac 19

<210> 434
<211> 19
<212> RNA
<213> Artificial Sequence

<220>
<223> Oligomeric compound

<400> 434
ucacugaauc uuagcagga 19

<210> 435
<211> 19
<212> RNA
<213> Artificial Sequence

<220>
<223> Oligomeric compound

<400> 435
aaagauagca gaaguagga 19

<210> 436
<211> 19
<212> RNA
<213> Artificial Sequence

<220>
<223> Oligomeric compound

<400> 436
uuuggauguc agcaagguu 19

<210> 437
<211> 19
<212> RNA
<213> Artificial Sequence

<220>
<223> Oligomeric compound

<400> 437
cccuuuaauu guuauuauu 19

<210> 438
<211> 19
<212> RNA
<213> Artificial Sequence

<220>
<223> Oligomeric compound

<400> 438
gggauuauau aaauuacca 19

<210> 439

<211> 19
<212> RNA
<213> Artificial Sequence

<220>
<223> Oligomeric compound

<400> 439
aaauaagucu auuuauaaa 19

<210> 440
<211> 19
<212> RNA
<213> Artificial Sequence

<220>
<223> Oligomeric compound

<400> 440
ggccaauaca uuacaaagg 19

<210> 441
<211> 19
<212> RNA
<213> Artificial Sequence

<220>
<223> Oligomeric compound

<400> 441
uaucaccaag aaacuggcu 19

<210> 442
<211> 19
<212> RNA
<213> Artificial Sequence

<220>
<223> Oligomeric compound

<400> 442
gacucaaguu uaucaguaa 19

<210> 443
<211> 19
<212> RNA
<213> Artificial Sequence

<220>
<223> Oligomeric compound

<400> 443
aauuccacag aaacucuga 19

<210> 444
<211> 19
<212> RNA
<213> Artificial Sequence

<220>
<223> Oligomeric compound

<400> 444
uaauuugauu uaacaaaca 19

<210> 445
<211> 19
<212> RNA
<213> Artificial Sequence

<220>
<223> Oligomeric compound

<400> 445
uauguacuga agaguguug 19

<210> 446
<211> 19
<212> RNA
<213> Artificial Sequence

<220>
<223> Oligomeric compound

<400> 446
ucucaccuuu cuaaauauu 19

<210> 447
<211> 19
<212> RNA
<213> Artificial Sequence

<220>
<223> Oligomeric compound

<400> 447
aagaccagau acaugcuac 19

<210> 448
<211> 19
<212> RNA
<213> Artificial Sequence

<220>
<223> Oligomeric compound

<400> 448
cuuuugcuac aaucagagu 19

<210> 449
<211> 19
<212> RNA
<213> Artificial Sequence

<220>
<223> Oligomeric compound

<400> 449
ccaccuuaua gguagguaa 19

<210> 450
<211> 19
<212> RNA
<213> Artificial Sequence

<220>
<223> Oligomeric compound

<400> 450
gaaguacaca uuggaauuu 19

<210> 451
<211> 19
<212> RNA
<213> Artificial Sequence

<220>
<223> Oligomeric compound

<400> 451
uggaaguuaa aguagauac 19

<210> 452
<211> 19
<212> RNA
<213> Artificial Sequence

<220>
<223> Oligomeric compound

<400> 452
uacgguuccu auauaacgu 19

<210> 453
<211> 20
<212> DNA
<213> Artificial Sequence

<220>
<223> Antisense Oligonucleotide

<221> misc_feature
<222> 1-20
<223> n = A,T,C or G

<400> 453
nnnnnnnnnn nnnnnnnnnn 20

<210> 454
<211> 20
<212> DNA
<213> Artificial Sequence

<220>
<223> Antisense Oligonucleotide

<400> 454
gccctccatg ctggcacagg 20

<210> 455
<211> 20
<212> DNA
<213> Artificial Sequence

<220>
<223> Antisense Oligonucleotide

<400> 455
ccgagctctc ttatcaacag 20

<210> 456
<211> 20
<212> DNA
<213> Artificial Sequence

<220>
<223> Antisense Oligonucleotide

<400> 456
gagtatcact atggctggcc 20

<210> 457

<220>

<400> 457
000

<210> 458

<220>

<400> 458
000

<210> 459

<220>

<400> 459
000

<210> 460

<220>

<400> 460
000

<210> 461
<211> 51981
<212> DNA
<213> Mus musculus

<220>

<400> 461

```
actcagctga gttttcagca ggacattccg ctaatgtctc ctccctcccc ccgggctttt 60
ttgtaagcta ggcctctgcg cgtcccttgg tcgcggaggg aggagcacaa agctctcaga 120
acagccaact cagggaggtg tgcccagagc tctcgacccc gccttctctc ctagaggcgt 180
ttttcattgg ttagtgggcg gggcttagga agtacagcgc tcgcccggag gcctcagggt 240
ggcctagcca gctggccatt ccttaattat gcatggaggc gtgtcttggc cagtggcggc 300
tgggtgggga ttggctggag gggctgtaat tcagcggttt ccggagctgc agtgtagaca 360
gggagggggga acctggggtt ccgacgtcgc ggcggaggga acgagcccta accggatcgc 420
tgaggtacaa ccccgctcgg tgtcgcctga ccgcgtcggc taggagaggc caggcggccc 480
tcgggagccc agcagctcgc gcctggagtc agcgcaggcc ggccagtcgg gcctcagccc 540
cggagacagg tgaagggggt gcagggtggg gccacctccc cgacggcacg gggggacggca 600
tgggggaccc cacggttcgt cagctgtggg ggtgcttaga gctgtgaatg gacacttaca 660
tggggcagga ggataggagg agacacacag ggtcgtcccg gcggcactga cgtccggatg 720
acaggaacca gcctgggaaa ggaagggacg ctgtaagttt cgagggggag atgcggagag 780
agagtgggga gttctggggg gtgtaggacc gtgggggga ggacattcta ggctgagggt 840
aacagaactg acagaagcgg ccctcctgtg actgctcccg gaggctaggg acgttcgggc 900
caccagagcc actgccactc aggtggagat cattggggtg gaggcgttag aaggagcctc 960
cttcctgccc ctgtgctggg cctggccgtt ccttgcctag ccactttctt tgccttgctc 1020
ccgttccttc tgtagtcagc tgtacccact taggaggtgg gaactgttgt gtctctcctc 1080
ctgcctctcc gcccctcccc ctccccctcc cccccccccc ccagcctctg ggactggcta 1140
gattttttttc ttaagaatca gatttcctat agaaaattga caaacttgta tcctttctaa 1200
attctctccc atctctttct gtgttaagct atgatttccc ccaccttgtt aagaacactt 1260
tttttttttcc caattcaaaa taaatccctg gccaaaccag ctctaaagag caacaaacct 1320
ctttcctctt tgacattgtt ctctcataaa cacctggtaa actcgacgag acgataaata 1380
attgagccat tgcatttact ggatcgtggt gttgcttaat tgcacaggcc agggtgagcc 1440
aatcaagagt gggagttttc tgtctcatag ccaagctctt gtgtaaaagc agaagctagg 1500
gaaacaaaga caggctggcc ttggctgagt tctgtgtgta aactttacct gttggggggt 1560
gtgtggctat aaattggtaa gttggcaaaa tccagactgt tgctcaagtc actttttttca 1620
tacctggtcc acatccctcg gttgtatcaa ttgatgtgtg tatgggtact gtcagtgctt 1680
attcagtttc cttattcaga gtgtgtgtag tttatacctg ctgtttacta cagtgcttat 1740
ttgtgtatgc attgtgaatg ttgggctgtc agtgtaggtg cagcatggac gtgaatatat 1800
taaagcaata aatcacttct atcttctttt taatgtggaa ttagactgtt aggatttgag 1860
ccagccattt tttctctttt tatattaaat aatgtctatg ttccttgagg aaacatgttt 1920
gtttcattat tgcatgtgcc ggaccagaac actaagcggg atcttttaat tttttttttag 1980
ttttagactt tatttatctt tgttctatgt gtatgagtgt tttgcctgca tgcacgtctg 2040
tgccagtgtg tgcccagtgc cctaggtggc cagaagagga catcagagct tctggaacta 2100
gtgacagatg gttgtgatcc accacctggg tcctgggaat caaacctagg gcttttgggg 2160
agaaccactg ggaagagaat ctggaataat aataatgaaa atgttggagt gtttgttctt 2220
agtgttagca cactccccac aaagaaccat tagcagattc atgaatcttt tattttcatc 2280
tgtagttcat cgggacaaat aaccacttag agataatcct cagttatgag gctctcgata 2340
ggggctgaga gatagctagg tggctcactg aatgctaacc tcattttgta gataaggaaa 2400
ttgaggccaa taggcctaga cacttgcctt aaggcatcgt gagttacaat ggtttaacgt 2460
ctgcctttta catatttctt ggtccatgct taagtattca gtttcaattt agagtgagta 2520
tgttccctgt tgagaagtat cagagaaaat ctgaataatg aaacattggc agctgaaagc 2580
caaagtaact gttctgtgtc tcaccctctc tcctccctct ccccccctctc tcctttcccc 2640
tccccccttcc ccctctctcc ctctcctgtt agcataattt actgtcaaac ttcagctatt 2700
tttttaatgg agatgtttag gcaaatttta gctaaagggc ggctgtgacc tcaatgacat 2760
ttcagccgga gagccttgtg gtcagtggtc gtttctgtct gtagaaagtt ccttttttctt 2820
ttgtcttgac atccaaatgt gagaagggcg cggaggtagt gagcaccttc cttgatgccc 2880
aagcccagaa gccttttagc tgatgttttc taagagatgg tgatggtgta cagctcagag 2940
aatacttaac ctctctgaca ttgtgaaaag tgctagggag agtgacatct tgtgtgtaga 3000
tttgcaggct gttggagcaa cttgataaat gctttattgt ctaatacaac acgttgatgt 3060
cattaaggag agcaaggcat tattacttac acctgtgcct tctgtccctc cctgctctct 3120
tctttccctc agtttccctc ccttttactc tcctttttctc cctttctggg gacgaaacct 3180
aggttgctaa caagcactct accacggaac acattttatg tcattcggat tttaaagttc 3240
tagccaaata aaaaaaccaa aaaactcatc taaagagtgc ttgaaggata gccctaaatc 3300
ttgaaggact cctggtgctg tggaccggcc tcagtgagat ggtggaggat ggcattggct 3360
tgctgtcttt gatggtgaaa agaatgtttg gcttgctctg gtgcagatag ttgggattac 3420
```

cagattgtat ttggtggcaa attcttggaa ataatttgtt taaaggagta acatttggaa 3480
gaatgaattt ctactatggt tctcattttt ttatgttatg tttgctataa ctttattcag 3540
ctaacataca cacacacata cacacacaca cacacacaca cacacacgca tgcacacatg 3600
ctagacggat gaatggtgct ggcggcacat gcccttaatt ccagcacttg gaaggcagca 3660
gcagtggatc tctaagaatt aagggccagc tggtctacaa attgagttcc aggccagcta 3720
agaccctggc ttaagatgaa caggaacaaa acactaagca gaagctggcc atggatgttt 3780
catgtctgca gttttagcat gtggctagcc aagaactgag gccaaaaaaa aaaaaaaaaa 3840
aaaaaaaaag tgagtttgaa gctagcctgg gttacctagt gaattccaag tctgctttga 3900
gtagctaata agaccctgtc tcaaaaaagg ctaggcaggt ctgctggtat atgcttgcaa 3960
ttcaaggatt tgggagacac tggctggagg ccccactacca atttaaggcc aggtattctg 4020
cgtagctagc tggagactac ctagcaaagc cccggttcaa gaaaaagtat tttcacagag 4080
acaggaggat tgctgcaaat ttgatcaagg gaagcctggg gttcatggtt tcaggccagc 4140
tggggctatg tagagaaccc cgtgttaaag aaccaaacca gaacaaaacc acagcagcag 4200
agagtagaag ctgggcagga gccaaggaga atgagtattc aaaaccccac ccaagttctc 4260
atggaactgt aggcctagtg gggaccagag tgcacactgt tgcagtattc acctaaggca 4320
gaggagtgag tggctcaagt ctgaggctac ggaggcagag agacctggga tccgatcctg 4380
gattcactgc ttcttggtca gaagctaagc ctcagtcctg cctgtcccac cacggccacc 4440
ccccagctcc cctcctgtcc caccacggcc accccccagc tcccctctgg tttttttttt 4500
ttttttgag acagagtttc tctgtgtagc cctggctgtc ctggaacact ctctgtagac 4560
caggctggcc tccaactcac agagatctgc ctgactctgc ctcccaagtg ctgagaccaa 4620
aggtgtgcac caccactgcc tggctgagcc tcagttttct tacctaactt taaaaaaaaa 4680
aaaaaaaaag acagacaaaa gggtgggggg aaggtcggtc tgttggcatg ttggcacatg 4740
cctacaactt gaggtataca gggagaccct ctctcatgca agtgaaatgt ttagagcatg 4800
cagctccaca gcaagagccc tgtatatctt aagttcttag gaaaaggtcc aaggatagag 4860
aggctcattc tatacagggg tgagaggtta caggagggct agtttccggg aacaccagag 4920
cacttacagg agggttagga attcttgcag agcttgtaca gttctttcaa ggtggtgggg 4980
gcgtggatgt gctgagctta aaacagcata gtgggtttag ggctaaaag tggaagctct 5040
taattgagga gaaggaaaca ggaaggccac agagccacat cttttatgcc ttctcttctg 5100
atcatgtcac tagaactgtg ttttaaaaag attgaattct tttcatgata gtaatatatg 5160
ctgtgttaaa aagctttacc tgttttcctg ttaatagttc agtgtgtgtt ttcccagaca 5220
ctctttaact taccagttca ttttggatct attttattga aaatcatttt aacagctcaa 5280
caaaataata tggaaggcaa atctaccccc ataagaaagt gatgtttaga ccttggcttt 5340
ccatcttgcc agctctatct gtggtaataa atgactttac tcaccactga acaattgacc 5400
tgacctttga ccttttaaat gcgtcagtca agaaactcac attctgagct ctcctaaggt 5460
aactattgct tgggtgatgt gcttgagggt tttgtggtac atggacagac gcaatggcat 5520
tctaaagcag cgctatgagg ccagacacct tgggtttgaa ttttcagata caacacctct 5580
ttgtccttaa ggctggttgc tttaacatct ctgagtttca atttctttgt ccacaaactg 5640
cctgctgggt ccactcacag ggtagctgtg aagaccgagt gagatcattt gttacatcat 5700
aagaaatttt gcgtggtgcc tagcacgtag ctaacattca aatgatatca attaacactg 5760
aaatcttcaa tctttctctc tggttaccaa gttgggcaag ttatgattca tggcaccatt 5820
agtgacctga aggaaagaaa gagtaattag gagattttga cctacagggg aagctaagtg 5880
actagggact ggggatttac atggtgacag aggagatata tctttgacat agagatatag 5940
acatggcaaa ctgtacattc ctgttacata gaggctctca tgataagcct ctttgcagct 6000
tcaaagacct atgatccact ccatgtattt taatagtcaa cacattgaaa gttatcacta 6060
ctcagtaata gtcccttgga gtttttcatg ctcttaagaa gtatagacag ctacaggcta 6120
ggaacatggc tgagttggta gagtgcttct ctagtgtgta tgaagccatg gcttccatcc 6180
ctaggactag ataaacccag atttagtggc acactcctct aaaacccctt gttgggtgga 6240
gataagaaga tcagaagttt gaggtcacca tggactacac tgggagtatg aggccagtct 6300
gggctatgtg agacactgtc ttgagacatg ggtggtggtg gtgggtgttg ctggtggcat 6360
ggctcagtgg gtaaaggtgc ttgccttcag gcctgaccac ttgagtttga tttctttctt 6420
tctttctttt tttttttttg agacagggtt tctctgtata gccctggctg tcctggaagt 6480
cactttgtag accaggctgg cctcgaactc agaaatccac ctgcctctgc ctcccgagtg 6540
ctgggattaa aggcatacgc caccacaccg ggctacgagt ttggtttctg gaacctgtgt 6600
gatggaaggt gagaattgtt ttccacaagt tgtcctctga tgtccacacc tgcagcatgg 6660
tttatgtgtg catacatatg cacaactaga gtaatagact aatgtaaaat gattttattt 6720
atttatttta aagaatatat atatatgtgt atgtatgtat atatatgtgt gtgtgtatat 6780
atgtgtgtgt gtgtatgtga gtgtgtgtgt gtgtgtgtac actctacact ctagctgtct 6840
tcagacacac cagaagaagg cattggatcc cattacagat ggttgttgct gagaattgaa 6900
ctcaggacct ctggaaggga agttggtgct cttaatcact gagccatctc tccagcccct 6960
aaaacaattt tttaaaaggg aagcagagag aatatgattg gtggtgtgtg ttcataaacc 7020
ccagtgatgc tgaagctgag gcaggaagac taggtgtttg agaccagcct ggaacttgct 7080
cagattctgt gtcagatttt tgaaaaaggg ccagagatat ataactcagt gatagaacat 7140
attcctaatg tgaacagagc tttgggttca gtctccagaa ccacacataa ttttatacac 7200
tatatatata tcacatacat acacacacac atatatatca catacataaa cacacacgca 7260
tatatatcac atacataaac acacacatat atatcacata catatacaca cacacacaca 7320

catatgtaat gtgtgtgtat atgtatatat gtatgaatga gagacagagt ctcagtatgt 7380
agctctggtg gtggcaggcc tggaactcac tctataaacc aagttggcat caaactcaga 7440
gaacagagat ctgcctgctt ctgcctccct ggtatttgta ttaaaggtgt gtgctaccat 7500
agcaaaaaga taagggacag atactcttgg acagttgaag ggtagaggag tggttagggt 7560
tgaatctttc ttaggaagga ggagacggga gaggggaaga agagatccag aaggcttgga 7620
cgttggatga catccatgaa tgacccacat gatgctggaa gttgctttgc tgtgtcctgt 7680
gcagctgata gcgtctctct tcacatctgt gtgtgtgtgt gtgtgtgtgt gtgtgtgtgt 7740
gtgtatgtgt atacaagcat gtgtgagggc gtgaatggac gtgcagatgt tcacagttac 7800
ccttcacttg atgtgtatat acagctttat cttcctgttc tgtaactttt ctttgtacct 7860
aacaagtgaa tagcctcaca gataacaacc tagctcccat gagtcaaatt tactattcac 7920
ctctttaagt ttgggttgga gaagacagga atggacctgc aaccagtcca acccagtggg 7980
cccatgtgga acagaaggaa gccagcggag ctagaaagag aaagtgaaac ttgtaactaa 8040
tggcttccgt gttgtggagg aggggcgcag tttattacgg ggatgtttgg gtagtttttg 8100
tagatgccat ttctgaaaac tgcttgggat taggaaagaa aaaccctgcc tgtctgtgta 8160
gccatctgct ttcccgacgg ctgattgatt ttattagttt gcggtgtccc gacctcttgt 8220
tctcggtatg agagtctgtg ctttgagtct ggactctgca cctgcttagg tgaaagctta 8280
ctagcaagtt tccccacatt gtatctaatg ctaaatttgt tttgctttta gagagctcgc 8340
tttttttttt tcctggcaga atctaaatgc attagactgt tcctttgtct ccccctccca 8400
cccgcacaaa aatcctaaaa acctcaaaac ccaaatgcaa gctgtgtaat tacctttccc 8460
gtaagccaaa caatgtgagt tatcattaaa atagttagcc tttgaagttt ttcctacttg 8520
tggtttggac acaataaata tttaatgtaa agctgacttc ccttggcaaa tcagtgttgt 8580
tgtggtcttt tttctgtcaa aatgtttagg aaaaacaaac ttcttaacat taatcaaagt 8640
aaactcatgc ataagagggg gtgaggtaat taaagagctc tgaagccggt gtgtatgtaa 8700
atatatgtgt gtgcatataa atgtgtgtgt gtgttttaac aggtttcact gtctagccca 8760
aagatctctc cacattcctc tgcctcagtc tctttaatac tcattcctaa ttacatgtga 8820
gaaaaaaaat ccagtatgac ttcgttcatc agggcaggct cttcgtggaa cggggagtgt 8880
tctgggcgtg ggacgctttc aagccccagt gactgtattg aagattctca gggtcacgga 8940
gtcattggga aagtcagtgt ctacagtggg ggcactgcac atgagagcag gtgggaggaa 9000
ctgggaatgc tgtaccctgc tcagttttgc tgagaaccaa agctgctctg gaagctaaat 9060
gtggtgggct acactgtagt ccttgtactt gggcagaaac aggagcgcct tggtgaatct 9120
gagggcactt gggctgcata accagtacca gaccagccag ggcctcagtg gggagcacct 9180
caaaatgcca actgcaagca aataaataat aagtagataa ataaatatgt gtaatttcct 9240
ctatattttt aaaaagctga tttcaaagtt tattaattaa aaagtgattt tttttccctg 9300
cagaaatatc tgggtgccct ggatgacaag tgttctgtgg ggtctggggt cagcatgggg 9360
ggacagtgca tgcctaagcc gtgcagagcc ctgaaaggat gacagaggag ctggggtgtg 9420
ggatgtgagc agacactgac ttaaacatga gaagagaaga gaaggtggct ttgtccaagg 9480
gggaaaagtg gtgatatttg ttgcaatttg taacatgtaa catttgaact tagaaaacga 9540
ttaaaaaaaa actgtaaaaa aattgagctt agcagtaaaa attaaaaaaa tatatacttc 9600
ccctattcag tagtttcctg ttattaatga atgtaagtaa ctcttcagtg ttatcggatg 9660
tgattttttt tttttttttt gtatgatgga gactaaaacg tgtcagcgtt taagtttgtg 9720
catcttaaaa gcaatgtttt ctaaatgact aatgtttatt acaaaggcac gttggcagac 9780
attccatgaa aggtaccagg tagagggcgt cttaatgtag ccgagggtga ccccagagtg 9840
cacacctacc tgtcctgtgg agctatcacc tggcagtgtt ttaaatggca ttaaagattc 9900
cagattactt tgaaagagtg tgtaatctgc tatttgctat tcagcatatc tggatgaagc 9960
cagctctcga tggagtaacc aaagcaatat actgcaacaa gcatatgtaa gctgttctga 10020
gactccaaca gctctttccc tccctccctc ccttcccttc cttccttcct tccttggctc 10080
tcatgcatgt tagactcagt tacttagcca cggttagccc tgaacaccga atccttctgc 10140
cttcacctct caagtgctgg gagaatacac ctgaggcatt gcacccaccc agctttaaca 10200
cttttctatt ggaccagaaa ttagattact aaaattttct ataaaaaaaa aaaaatgcag 10260
ttctgggaga gccagggcta cacagagaaa ccctgtctcg aaaaaccaaa aaaaagaaaa 10320
aagaaaaaaa aggtattatt agctgggcag tggtggccca cacctttaat ccagcacctg 10380
agagacagag gcaggcagat ctttgtgagt ttgaggccag cctggtctac agagtgagtt 10440
ccagaacagc cagggccaca cagagaaacc ctgtcttgaa aaatccaatc aaatcaacca 10500
accagacaaa aggcacattt aattttatga ttctctgtat gtgcaggcat gtgtgtggag 10560
atcagagagc aattttctgc tgtccgatct ctgtttccac ttttatatgg gttctgggga 10620
tggaactcag ggtgccaggc ttgtatggtt ggcacctttc cctcaactcc tctgagccct 10680
ctctgcagcc ctacatttgt gaaactttca gcggtgccgc ttttcccact gatttctccc 10740
ccttcggaac atgtggttac ctttcatagg ctattcctgc gacttatgtt aatgtgctgt 10800
gcacagttaa ttgcttaaat gaactttgtt tgagacagtg tcttctcgtg caatctatta 10860
ggctggcctg ggatttaggg accttccatt ggggcctctt ggccagctta tacttataac 10920
ttctattgat aggtggtatc taataggtat tttctggtgt tttgagtcaa ggtcttgtat 10980
ttccaagtac ccttgaactc acagatccta agctggcttc agttttgtgg taatccttct 11040
gcctcagcct ccattgtgct gggattacaa gtacatgcta ccactacaaa tttttaggca 11100
tctctaataa atatggtctc tgtaaatgaa gctttgttag ggtccttaaa ttttgatagc 11160
aagaccagtc ctaaaaacag tgcattttga gaactgttaa aaactattaa aacagccggg 11220

tgtggtagca cacaccttta atcccagcac ttgggaggca gtggcaggca gatttctgag 11280
gccagcctgg tctacaaagt gagttccagg acagccaggg ccatacagag aaaccctgtc 11340
ttgaaaaaca aaaaaacaaa caaacaaaaa aactattaaa actattaaaa ggttttggta 11400
taaatattct tgagctgttt tgaggagcta gggttgggga gggagatgac atctatggct 11460
ctgattaagt ggtctcggga cattgtaacc actgtgccag gtatcatggt acagatgggt 11520
ccgtaatttg agaaacactt ttacaagggc cataaattct tcagatgaat gaagtcctgt 11580
ctagaagttt ggaagttatt gtcttaaaga aaaactgctt tggagattcc tttttatctt 11640
gattttccct ggtatttatg tctctaagaa tgaatcttgg attgcgtctg attgacaaaa 11700
gagccatacc tcaccagatt ctggtagctt ctgagtgctt ggttgatttc ttttcttgtt 11760
ttgtttttct tttcttttct tttttttttt tttttttttt ttggtttttg gagacagggt 11820
ttctctgtat agccttggct gtcctggaac tcactttgta gaccaggctg gcctcgaact 11880
cagaaatctg cctgcctctg cctctgctgg gatcaaaggc gtgcataacc acacccggct 11940
tgttttgttt ttctaagaaa gggtctggat aaccctagct gtcctggaac tcaactttat 12000
agaccaggct atcctcaaac tcagagatct atctgcctct gcctcctgag tgctggaatt 12060
aaggctttca acaccacacg cagctataat ttttaaagat aattctgatg cgaattacag 12120
tttaatttgg aaagaaatgt tgacttaaat cagatataaa aggaagtttt tttcttgctg 12180
tagtgactgt ccttggatgt tatctgccat gcactcactt ccctttatca gctgtagtca 12240
cttctctctc ctgtccctct ctataaaatg ggaaagactt ttactgggag cctgctgata 12300
tcatcagtac cctcagggtt ccctctggtt gagagagtga ctggagagat tccttgtgaa 12360
ttcttgcttc cggcacaggt tacagctctc cctgaaacct gaataggggtt gttgttgttg 12420
tttccctcct cctcctcttt ttttttagc agagtttctc tgtgtagcca tgactgttct 12480
ggaacttact ctgtagaaca gactagcttt gaactcagat ccacctgtct ctgcctctgc 12540
tggaattaat ggcttgttcc acctctgccc agcttgttcc tttcttaaaa aaaaaaaatt 12600
aaaattaact ttatttagtg tatatgtatc cccacatata cactaggtca gaggacaagc 12660
ttcaggagtc agatcttgct ttctatcatg tggaccctgg ggattgaatt ttggtcttca 12720
ggtttggcag cagcccctgc tgagccattt tctgacccta atattacatt gttgttgcct 12780
tctgtgtggg agccaatcat gagagcagtc gttcttattt attcttctca gtatgttatt 12840
aattttgtga agacttcata gaaaagcttg tgtagttaat ctttctgggg tcagactgct 12900
tgttttgttt tgtttttgct tcgtttgttt gagacacggt ctttatccca ggctggcctc 12960
attgtatagc tgaacatgac cttgattttt gtttttgttt tttgtttttt tgttttttg 13020
tttttcaaga caaacccttg ctgggttgca actggcttgt agaccaggct ggccttgaac 13080
tcacatagct gcccatctct gcctcctgaa tgctgggatt atagcatatg ctaccatagc 13140
tggtttatgt ggtgccaggt atgctaggta aacactctac caactgagct acatctccag 13200
ctctaggctt gtcttttttta aagctttcca taaagttaaa ttcatttcaa acacacacac 13260
acacacacac acacacacac acacacacac acacacacgt ttgagtcatt gttataaagc 13320
tctgtgtggg cctgttgctt gatatatagt cttcattggc cttagaccta tggcaatcct 13380
cctgtctctg cttccagagt gctgggatac aggtaggttg acaaagtaga aaatacctct 13440
tactttccct gtccagtttc tccattgcta gcatcttgca ttagtgtgat acatattatg 13500
cagttgataa tagcagctat atgttgttat taattaaaga atacacagtt tctatcacac 13560
tttactgtta catattcttt gctgtttgat agtgttggag acatgttgtc tgttgtagct 13620
caggatgcaa gtaaacttgg aatctgccca tctcaggttc tggagtcctg gcattccagg 13680
tgtgcccctc tgtgcttggg ccattttgtg gatttccaca gacatgctgt gtggccacca 13740
gcacagtacc acttgaagta cactctgcct ccatagcccg gtgctctacc cactcacccc 13800
tctgcagccc ctgccagtcg ctcatcccct ttttctatct ccatagtcct gtttttcaga 13860
atattcatct gacatcatgt aagcagtagc cttctaggca gcagtggccc agtgcctctc 13920
tggttcgcct tctttcacgg agcagtatgc gttcagcttc ctccttgcct ttggtagctt 13980
tgcgaggaac actgccttgt atgacccttc ccttagtgat gttcccttca tctgaagggc 14040
atcttggctt tgtcgtttgg gctgatagga ataaagctac ccattattgt ttgggtttgc 14100
actcttctaa tgaccaggct ggcctcgaac tcagaaatct gccgcctctg cctcctaagt 14160
actggctttc atatgccgtc tgtgtactct ctttggtgtg atatcggatc agactttctc 14220
ccaattttgt tccatatttc cgtacaatgg aaattgaatt tagggttcca cagctgggtg 14280
tagtggcatg ttcctttaat ctcagtgctc aggagacaga ggcgggcaaa actctgttga 14340
gtttgaggcc agactgatct acagagggag ttacaggaca gtcagggcta cacagagaaa 14400
ccctgcctca aaacaaaaaa gcaagcaaga gtttagggtc ctacttactg gcaagtgttc 14460
tttaccactg agctacaccc cgatccttat tttaaaaaat ttattttgag accagggtct 14520
cataactttg atcaggccag tcttgaagtt gtgttgtagc ccatgcagga cctgaccttg 14580
ggaagcacct gtcttggttt tcccagtagc tgggcttaca ggcctgctct gccaggccca 14640
gctctcaccc agatttacac ggagattttt ttttttaaat tgttgagagt tttaaaagtt 14700
tctcagtctg tattgtatat acaagtgtac atgtacattt gggggaggag accagggcca 14760
taacacacaa ggcaagctcc aacactgatc tgtagtcata gttttctttc gatgtattgg 14820
gtttttttgt ttttttgttt ttttgttttt tttgttttgt ttttgttttt ttcatgaaac 14880
tcaatgtctt aacacttgtt agactgggct atgcctctag ccccgcctcct ttttaggtgg 14940
catatagttt taagtttttc ttgttggaga attcagtctc atgcagacac taatgctgtg 15000
agcttgaagt cagagacaag gaagtgaagt acttgtatct caccagccca aatggataat 15060
tatgatctta acctttagct ccactgcctt tagataaggc tgggcatggg gctgcagccc 15120

caactacata ccaaggtgct gacacttctg ttctgactgc tgtctctgct cttagacctg 15180
tcccctggat tatttctgac tcctcctttg gaactgagcc ttgctgactt ggatcttgcc 15240
cagcttctac tctctgttgc tgctgttttg tcctggtttg gttccttaac acagggttct 15300
ctatgtagcc ctggctggcc tggaactcaa tatgtagacc aggctggcct caaatttgca 15360
gcaatcctcc tgcctctgtg gaagtggttt ttttcttgaa acaggatcca acaagctacg 15420
tacaaaaaat ggtcttaagt tactgctagg tatgtaacct tgggcaagca ttgacattaa 15480
ggcacacccc caacctgaaa aataattgtt tgagaagaat cagcatttct agaattattc 15540
ttcttggttt ttaatttaat tttatttttt aaaacttaaa atgtgtgcag atgcatggtg 15600
tatgtgttgt ggggggatgc aggtgcttgg tgtttgtata gaggtcagag atcaattttg 15660
tggagttatt ttttcttcta tctttatgtg agtcccaggt taaattcagg tcaccaggct 15720
tatacaacat gcccttacct actgggccat cttattggcc ttcttggggc ccattttaat 15780
ctctgtcttt tgggggggtat aaattaaagg aaaaaagtaa aacatagcct tccagggggc 15840
tctgggcttt atttaaatta aaattttcaa aatgatttta ttttatctgt atgggtattt 15900
tgcctgcatg aatacctgca gttcacggag gccagaaaag gacatcagag tccctggaac 15960
tggagttaca gaggttggga gcacctgtgt gggtgcttgg aatcaaaccc aggttctctg 16020
gaagagcagc cagtactctt cttaaccagt gagccatctc tccagccctt tttcttgagt 16080
cttacagacc tttccttatg cccttccttt cctggtcatc cttcttctga actcaaaggt 16140
tgaagagcct ttgccctctc tgggtggggt tctcttctct tcactttctg tgttctctat 16200
gtaggttatt tcagccactg ctttgatgta tgtatgtatg ttacatggct ggggagtaaa 16260
cccagggctt tgtgcatgga aggcaagcga gcaccctacc gactgagcta ggtccccagc 16320
tcacttagcc attactttgg ccataattct gataatgacc accaggtgtt catctccacc 16380
cctgtaggct tctagcaccc acctcgtgct gctttccgac atttctccat gggtaccttg 16440
tgtgtacttg cctcttggtc ctactgttga aaacttaacc ccatagccac ctctctgaaa 16500
gtctgtgctt ctctcttcgt tcaacaatga aataaatgat agttcttggt ctgctgtctt 16560
agttactgct ctattgctgt gataaaacac catgaccaag ggaacttgaa aacatttagt 16620
ttggagttta tggtttcaga gagttagaga ccataatggc agggaacctg gcagcagaca 16680
ggcagacata gaactgaagt aactgatagc tcacatcttg agatgtaatc acaaggcaga 16740
gagagagaga cagacagaca gacacagaca catggggcag ggagactgag acacagatac 16800
agagagaatg ggagacacac tgggaatcct gttagtcttt tgcaacctcc aggcctgtac 16860
cctatgatat acttcttcca atgaggccat acttcctaat ccttcccaga cagttccacc 16920
aactggggac caagtattta aacacacgag tgtgtgaggg ccactcctat tcaatcatca 16980
tactgtgcaa gccagagccc cccagccaac cttgactcct tccctccctc atcctcctgc 17040
ttctgttcag atcggcagcg agtcttgatc tcacatctcg agtttcctca actccccttg 17100
actgtcatgt cccttctgtg gtggtgatgc taaaattgct cctgtacaag actcgaaatg 17160
ctggataaat ggagctctcc agtaaaaaga aaacttaaga tgcaaaagca gatagtctca 17220
tcaaaagaaa gaacagatgc aggcctggag tgtttcatcc cacaccactt ctaagtattg 17280
atccccagtc acagccctaa aatgcctcag gcttttgagt gctgcattat aggcatagac 17340
catcatgcta ggcttcagga gttctcactc ccatcccttc acaccttgac ctggcttcac 17400
tgtgcagccc atagcccagg ctggccttga actcaccatt ctcctactct agcctcccaa 17460
gtcctgggct tataggcttg tgttaccaca tccagcttat aggatcatca cctattctcc 17520
tgctatccgc tgaggcttct gacctgccat ctactgtacc ttccacctcc agctttgggc 17580
tgtttgaggt tgtaggccat actgcacatc caacagcact ttggtgctat tttcttacct 17640
gtgctagctc aggtaccatg ctgcactctc tgggttctag agttctatga gctccccccc 17700
ccaatccctt ccatcttgct tttcattctt tgtccatgtg cttaggttct gatcactcag 17760
aagattcgtc acattccttg ttttcacacg ctgttcccct tgcttagact ggctgcccct 17820
tcctctccag aatggccatg tctcctgtag ccctgctatc tttgtgaaac tttccagctc 17880
tcattcttag ggctgccatt ccatcaaaac agtgctcggt gccttagaca gtaggaacca 17940
agctgcgtag agcttgcggg gactctttgc cttaccagat gctaccccaa gctgcctctc 18000
aggctttgtc tttgtcttag aggaggctct gactctacct gtgtttctct gggcttagca 18060
gagcacctgg caaagagttg ctgaaatatt cagcaagcca gtggagccct gtacggctgg 18120
cctgaatcca tagatcggtc agaaaaccac agagaaaaga aaagcatgca ttttgcatgt 18180
tgctctacca aggccacaat tatactcttt ctttttctga gatagcctct gagcagtctc 18240
aactattctc agacccacaa agtcttagtc atctaagtgg tggtgttgag aggcagacac 18300
cagactctgg tgctcatggg cgaggatggg gctagaggtg agatctaggg actgccggag 18360
tgtagagtgt gctttgaagg agtgagactg catgaatcat ttagaaagtg agttgtagag 18420
aaagcaggag gtttatatta agagagtttt acagcaaagc ctgtccctgt aaaattgtga 18480
aatattttct catgaatgtt agactctgta aagattgcat tcagtcattc atttgggaaa 18540
aatatctttg gaatcaacaa acacagtgaa ccaccttgaa cttcatgctt aatagacaaa 18600
cattgtgtta actctttctg gggcagggat tctggagaca tggcaggggg tgggggggcgc 18660
ttgtcttcac tgtgtgatat ctgggtatca gctgggaagc cttgaacatc tgggcttttc 18720
tttagttccc agcccaggcc tggcctagca tgattgcagc tgtgatggta gatcaactgc 18780
atgtggcctc tttgtctgtc ttgtgtttct tgtagaatgg tggttgggtt ctggagcgcc 18840
tttgtccttt caggagaatc ccaacagagg tgttggagat ttcatatcaa gcttggtctc 18900
atggcacagg tctgaaatcc tagttacttg ggaggctgag gcaggaggat ctcaaattta 18960
aggcttgctt atactactga gtgatttcaa gaccagactg agcaatttag taagactttg 19020

```
tttaaaaaca acagaccccc cccccaaacc ctcgaaagtc aggtttaatt cctcagcacc 19080
acaaatgaga cagtgcattt cacactttca gcactgtgat gctgggctag ggatggcttt 19140
agccaggtcc aggggaaggg gagcaaagga tgaggaaggg gacaggttct ccatccaccg 19200
ctggacgctt gggtgtgagg ctctcctaca aatccactat gagatgacgt caagggaaag 19260
gagcaggtgt gtgccaccat acaccctgga gagaagaatt tcctccctcc tcttccctcc 19320
tgagcccctc cctccctcct ctcctccctc cttccctttc tttttacctc ctttttgagg 19380
cagagtctta ccttgtaatc caggctgttc ctgaactcat ggccattttc ctgcctcagc 19440
cttctgagtg ctaatattac agatataaac atataggttc ttagcccact ttagcctctg 19500
ctgaataaca aagggcttcc ttggggtcac ttaaaggaaa agtgacctga ataaatttgg 19560
ggaatttctt tcttttctcc agtaatcatt gagctacatc gtaggcttgt gactttgaaa 19620
ttccaaactt catttccttt atcagatcct gtatctctac ctccctagca ctggctccca 19680
agcccacgct gcaggtgctg ggacccaaac ataggtccta tgcttatgtg gcaggcccat 19740
acagggctat ctcctcagcc ctgaaatgtt tgtttttgat ttgagtaaca tttctcttgt 19800
taaaagatcc ctaaaaagtc gttgagtttt ccaaacagca aaacagaaga gctggactcc 19860
tcaatgaata cttttttgct gggagtgtgt cttcactttg gaaaatgtgg tccccttagc 19920
caggagtggt ggcacacgcc tttaatccca gcacttggga ggcagaggca gaaggatttt 19980
tgagttcgag actagcctgg tctacaaagt gagttccagg acaaaacaaa acaaaaaaat 20040
gtagtcccct taatgccaga taattccctc cagcagttca tcagttctac tacacacaca 20100
cacacacaca cacacacaca cacacacaca catgctttct gttcatctgt taggaactgg 20160
acactgtgct ggatgctgga gagggggggca cattagtgag ccgaagccaa ggttcttcct 20220
gtcatcatgg accttagaat gaaggagttg aagcaaggca taggtcccct gatactgaag 20280
gcaagcacac atgtgaaggc ccgtgaacgg aggagtagag tagccaagtg agtgtaggag 20340
tgtagctagc aggcaagtgg cagccagaca gacacagggc tatcttgtca tgcatgtttg 20400
ctctgtcctg ctagctttgt gtgctttaag tccggtctga cactgaccct gtgaagtgag 20460
tacagttgtc ttctattttt ctgcataagg aaggctagac acctggcacg catctcggag 20520
gggagtcagg gtccacaggc tgttgctttc atagttgatg atttttagtc tcagagcaaa 20580
aagtggtgag cctttatgga cgtggatggc agctcttcag aaagcctgga gggatgcagg 20640
cagatgtgca gatacaggta aaagctccta gctgtgggtt tgtagaagcc tgggagtgtt 20700
ttagaaacag ctagagtaca gactctggac atctgaccct tttggttaaa tgtcaacctg 20760
ctttatctct gcgcatgttt attattggga tgcagcggtc tcagtgtccc aagtgagcgg 20820
agctgaaatg gcttcagagg ccgtgcagcc ccttgccctc ctggagtctg cattatggct 20880
gtgatacctc aaggaagagc atctccatgt aagtgtcaga tgcaacagga acgaggcagc 20940
tgccccgtac ataactctct tcatgccatt gcccagtgaa gttatatctg agaggtctgt 21000
tttttggaga cggcttctca gcactgactg tcctggatct cactctgtag accaggctgg 21060
ccttgaactc acagagatcc tccatgcctc tgcctcccaa gtactgggat taaaagcatt 21120
tgacaccacc acccagttta gaattccatt taaattgtca tctacacaat gactgtgacc 21180
tcccagatgt ggccaggcac agcaagacta acagaccctt tggctcaggc gccatacatc 21240
tgttggaact ctggaaaagt tctttgactt agcatcgttc ttgactcatg acaaaattcc 21300
cagatcagaa tcccccaggag ctctgacagc aggctgcatc aggatgctgc tccatgccat 21360
tactgcctgt gagatcctga ataaatcatg tcaaattctg ggccttcatt tctcctcggt 21420
aagatgaaga taaaatattg tcttgaccaa tgagatggct caacaggtaa aggtgcttgc 21480
agctgagccc gacagcttac gtttgatccc taccacccac acagtgggag gagagaaatg 21540
actccttgaa gattgtcttc tggatgcacc acatcatcat tatcatcaat ggttagcaca 21600
aagatgagat gaaagttcca cacggtattc agctatagat actggttttc ttcttttctt 21660
tcttccttct tttttttttt tttttttttt tttttttgaa gacagggtct catgtagccc 21720
aggctatctt caaattcact gtataataag gatgaccttg aacttctgat gcttttgcct 21780
ccacctcccg agtgcttgcg tgtgcacatc tccatgccat gcctggtttt atgtggtgtt 21840
ggcagtggaa cccagggcct cgtacgtgct agggaatctc ttcactaact cagctacacc 21900
tctagctttt ggattgttct tcaaccaagg ctcttgttga cattcatctt tttattttga 21960
atagtactta gtgggttttt cattgtatgc tgctggtggt ggttttaaga cagggtctag 22020
catagtccag gttggcttca aactcaccat ttagtggaag gatgtcctta aactgatctt 22080
cccatatcta agctctaagg ataggaattg aaagcatgca ttttcatctt tagtgttatt 22140
aggcctagag ctatcctgtg caaaatatgt gtggagagat ttgatcatct tcagacatct 22200
gtcctgaggt agggtcacca gatgctaatt cccccccaccc caccccccagg atatgacagc 22260
agggtccatt ataatgcacg tgcttctcca tatcagggaa agctctgtgg ttgtctctga 22320
taacacctag ggaattggat gaactgtttg ctctttggct tcctgaggca ctgagaaatg 22380
gaattcctgt taaatgagag ggtcaaagac tcagaaggag caactactgt tcacctgtga 22440
cgtcacttaa gtttagtacc atccttccga ggcggggtag tccctgttgt gctgaagagg 22500
aggcctattc agcagtcaag ccttgtagag agagtggaag ggtcacagcc tggggaggtg 22560
accttacgca caaactgagt ccttttcctt ttttctagag ctagaggcag ctctgaccgt 22620
cgacttagtg gttgacaaag cacgtctggc tgtgtcaatc taaaggtttg ctgtcttctg 22680
gctcctttta tctacaatat ttgaaatcaa atatggtgca tatcccatat cagttatgga 22740
tttttgtagt aaatatttaa tctcaattgg gggtttctac ccccaccttag atcatttagt 22800
tcccagataa aagacccaaa acttttatat ttataataag ccttaaacaa cactaaagct 22860
gagcagatat catctctctg tgtctatttt gtctactttc aggcaaataa ccctgagcta 22920
```

tgacttgccg tgttccactt gagccgctct tactccactg gccagccctc atggccagat 22980
ctcacaatct cttaccccat ggtgtcttcc tctctccacc ttctctctct tccctttcat 23040
ggtcctttct ctgaccccaa gcccagaaac caaaattcta cccacctctc ttctgcccag 23100
ctataggctg taggtgtctt agtcaaccaa tagttttaaa ctaaggagca aagttgcata 23160
acatgacttg gtgtgtttga ggatttcctc atccctgggg caaccgaccc tggggccagt 23220
attccgcatc acagtacata ggaataggtc agtgcattat ttgttttgtg ttctctctgt 23280
ctccttcccc tacccccctt tttcctgact ttcatgctgg aagtgaagag gaagtggtga 23340
atcacacctt taatcctagt acccaggagg ccaggcaggc agatctcttg atgttgaggc 23400
cagtctgctc tacatatcca atttcaggtc agcccgagct acatatatgg ccccaaagaa 23460
ggctttttga cacactgtga aattgaacag gaattgtcct cttttaaaga tagagtccca 23520
cttagctcag gtgggcactg aacttattat gtagctgagg cagactttaa actcctgagc 23580
ctgctgtttc ttccttccga atgctgggat taagggtgag tcccattaca ctcgcttcag 23640
aaatgtctct gtatcacttt ctgttgtgga tttttatttg catttgcatt ttgtccctct 23700
cttcatcctc cattttaggt ttactggttc atttatgttc atacatgtct tagagagttt 23760
aggaagttgt gtgattaaat cccttctatc tgtggtctcc tgtggttccc ctagtttctt 23820
tagactgaac tatacaaatg aggactgtta ctttaatgat aatttttcta ttaagaagtt 23880
ccttattaga ctgaaattat tttccttatg acttactgtt ggtaggttat tccccacagg 23940
gctaagaaat tgctgtttgg aaaagtctag aaggatttgg tgacatgctg tttcattttc 24000
cttttctctt gagctataga aactaggatc atcagggact agagagatag cttgttagtt 24060
gagagcactg actgctctta caaagaacac aagttcagtt tctagcaccc tcatggccat 24120
tcatcatcca actgtaactc tactttcagg aaattcagcg ctcgtgtctg gcctctgctg 24180
gtacacacac aaacacacac actgcacata tataatatgt aagcaaaact tttatataca 24240
taaaataata aatctcttaa aacagaagaa aatagggtca cagcatctta gttactgttc 24300
tgtggctgtg aagagaccca tgaccaaaac agctcttact aaagaaagca tttaatttgg 24360
ggcttgcgta cagttttaga cggttaagca ctgctcatca tgacaggaag cagtctggca 24420
agggacagta gctgagaact ttatatcctc acagtagcag gcagagaaag ataaggcctg 24480
atatgggctt acagtgacaa acctccagca ggccacacct cctaatcctt cccaaacgct 24540
tccactaact gatgggcaag cattcaaaca tatgagcagc attctcattc aaaggttcac 24600
acacacttta tctttcctct gaacatttgc aatgatgttc tgcccacctc agcctcatga 24660
aagccagcag agcagcaagc ttgtgtcata ctctaaccca tccactggag caatgacttg 24720
acttctactc tggtgcccag gatggtactt ggcattcaaa ctgttggtag atcacttttt 24780
gtgttctggc ttctgagagc tggttattaa agcacaggct tcaagtgatt tttgaagaca 24840
aaagttagat cttaacagaa gtcgggcatg atgtgtgccc ttccagtctt ggtactcaga 24900
ctgaggcagg gagatcagcc agcctacact acccagccat agcacgtaga gtggatcaga 24960
ttaagttgtt tatgttggtg tttgtgatgc tgaggatgca actgagcatg ctgggtaagc 25020
actgtaccac taagcaatgt ccccagcctc ttaaatcagt ttctaacttg aaaaacagac 25080
ttgaggtggt aaagtgtctt ctagatgttt tccaggaaaa caaatgcagc atacggactg 25140
gcagaggttt cctggacagc ttggctgacg atctctaaac gcccagctgt cagtgtgcag 25200
aatgcttagt ctagttgctc ttgtgttgat tagtatggct tgcagctaag cagcatctga 25260
ggacatggct gttggacact cagggctaaa ccatggcacc cacacgccca tgttctcctg 25320
aaaataacat ctgcgctttg cttttagctt aggataattt tcttcatgta gaatagtttg 25380
gcagtattac ctatcttggt tagatcaaaa caaaacacaa aattagtcgt tcttgatggt 25440
ccagttaatt ggaaaaaggc acaagctggt ctctggttaa gaaaaagcca ttctaccagc 25500
gaaatacacc aaacatcaac actgaaacaa gcctcttgag ttacagacag aaataagtcg 25560
gctatgggta acttagcttt tttcttatgt tagattgaaa atgttccccc gactggagtg 25620
gtcatctgtg atggagtagg tagagtgggt gaggttttat ggtttttatc ttgggactgg 25680
ctctgcattt gtgtccaaga taaatgatga aatcatctgg ttttgtaact tatagcattg 25740
gaatttacta tttcaagcag atgatttaat taggatcatg gcattttttt tacagtgtgg 25800
cccagaacga cagtgtcttt gattttcata aatagaaagc gggtcacata tgtcatacta 25860
cattttcttt tcttaaggtt ttgtttgttt tgtttttgtt ttttagatgg gggtctctgt 25920
gtagttctgg ctttcctgga actcactatg tagactaggc tggcctcaaa ctcacagata 25980
tctgactacc tctgtctccc aagtactagg attaaagata tgcaccatca cacctggcat 26040
gttaaatttt ctagtaactg tattaaaaaa aaaaaagaag tgggaagaat aattttgata 26100
atgtataaaa cctaacattt ctaaaataat atttcaacat tgagatttgt acatttattt 26160
ctttcactgt tttcaaatgc tgcatatatg ggtgtgagcg tccacacttc cttggcccag 26220
tggccacagg tgggtggtgt ctcccatgct caacaacgca atcttttggt tcaatggagt 26280
cctgtgcagt gatgtttaca taaactgtca tatagatttt cttttgtgtt tctttgtgca 26340
catgttcatg catgtgtagg tacatgtatg tgcaggtcaa agggcaaccg tgggtgtcat 26400
tccttcgact tttgtttgac acagcatctc attagcctga aactttgcca agtaggctag 26460
accaactggc cattgaactt ccaggggtcc acatgtctcc acctcctatc ttgccctcat 26520
tggcattatg ggcacaccac cacacccagc tttttattaa tatgtgagtt ctaggaatcc 26580
aatctcaggt ccccagcaag catggtacca actgaacctt cttcccagct tcaaattggt 26640
atggactgct atgtaagtca ggtcctggga accacaggac aacaggaagc ttttcatcta 26700
ggagaggctg attctgactt ggcctccatg taagagggcc ctgatagcct ttggtgcctt 26760
actggggcac tgcacggaga ggcacagcgc ttcaggctgg agcatgtgtt cctgcaggcg 26820

gcttttattc cacctagggt ttgctgtaca cataccactc cctaatcttg ggctcttact 26880
catggttatt tttagacctc cagtgttctg gagagacatt gtcatgagtg gttagctggg 26940
tcgggttctc gtagccgagc gttggagtgg ggcagtcccc atctcaggat taagttgttt 27000
acgtggtatt cagctctcct tcctttctta ccccagattt agaaaaaata tattaaaatc 27060
tatttatata aatgtatatt tatataaata attccttttg ttgagtgggc agagtccagg 27120
cacatggctc ttgtccttgt gagcctgccg ctctgctctc tgtcctccct aagcaccggt 27180
ctgagccagt gcaggtttcc tgtctatagc cgcacggatg gcctggatgc agcaggtgtg 27240
tgtgggaggg aaggaaccga accgacgtgt gctggacagc tgctgggcgc tgtattctta 27300
gatgaggctt taggaatatt ccttcagcgg ccccaagaag tgatcatctg tattctagag 27360
accaggaaac agaaatccag aagaaggaaa ccagccagct tcacgtgaca tttgcttaac 27420
cccgcttgac ctctggtaga gaataggctt gttgttgtca tttggtcagg tttttagatt 27480
tattttattt atatgagtac actgtagttg tctttacata cactaaaaga tggcatcaga 27540
tcccattaca gatggttgtg agccaccatg tggttgctag gatttgaact caggacctct 27600
ggaagagcag ccagtgctct taacctctga gtcatctctc cagccctggt cagttgtttt 27660
gttgttgaag atgatgatgt tgtttctttg tttgtttttt aaatttttta ttttgtttta 27720
ttctaatgag catttttccc aaatgtaccc atgtgtacta tgtgacttgt ccagtgcctt 27780
cagcgatcag aagactctgt cagatagatc ctctggagct ggggataagt atactgtgag 27840
ccaccatatg ggtgctggga attgaacttg gtcctctgca agagcaacaa gtgcattaaa 27900
ctactgaacc agctctccag cacatgtgct ctctctcccc ctgtgtatgt gtgggaatgg 27960
aggtggtggc gagggtttag ggctcaggct ggccttgacc tgtctgtcat gatatcatag 28020
gagttctggg attacaggca tgagccgctg tgcctggctt caaagtagat tctagaactt 28080
aaaatcacac ctaattgtaa cagaagaaat tgcgtggcag ccttgaatgg cagctcatgc 28140
ctgaaattca agcacttagg aggcagaggt aggaaataac aaggctagcc tgagaccctg 28200
ttttagaaaa tcaaggacct ggagactagc tcagtggtgg agaacttgta tagtgtttga 28260
tccccagcat caggagtggg cagagctgtg gtggtaggat ttgtgaatgc aaacacgtat 28320
tattcaaagg actacaaaaa acatacaagc tccttttcag agtcaccagg gtatccccta 28380
tgagtacttg tggtgaccac atatatattt tggcttacag tcgagacccc tgactgcagc 28440
aggatggctc agtggaacca gctgcagcag ctggacacac gctacctgga gcagctgcac 28500
cagctgtaca gcgacagctt ccccatggag ctgcggcagt tcctggcacc ttggattgag 28560
agtcaagact ggtgagtcct tcttgaaaac tccctgactt gggggtttta gcattgagtc 28620
aggacacaac ctgttctagg ttcacacctt actaaccctt tgatcagaca tgtgagggca 28680
ggtgcggggg aaggattggg agaacagagg taacttagtc tcttaacgca tctgttcaca 28740
gtgcatatga cagcggcctc tttgcacttt gaaacagctt gctggtagat gttttggagt 28800
caggtttgag tctcacttac ttggtgagat ctctgcacat tttgagtcta gaacagtgag 28860
atacagatgt ttttcctttt taaatctaag aattctatgt caggtagaaa ttggggtcta 28920
ccacacacgt ggcttcccct ggtaaagagg aggcatggga tgaggaggga actagaggca 28980
tagaggaaat gccagctgtt tacctcactt tagtgtattc aggcccgtgt gctagttctg 29040
gcctaagtca gccctatctg cgtgtgacct actttttcca gaagacatac ctgatttggt 29100
gatgggccaa agaggtgtgg atcacacctc agcctggcat tgctcattaa gctaagaagc 29160
acttatagac actagtctga ctgaatacag ggttggccaa gaatttaagg caaacaacct 29220
tatctgtcct ttaaaaaaac aaaaacaaaa catttaccag tatgtgtgca cgtttgtata 29280
gattcaggcg tgtgtgcatt cgtgtatgcg tgtaggccag aagacaacct caagtgtcat 29340
tcttaagaac gccttttggg ttcgttgaga ctgagtcatt cattggtcca gagttaacca 29400
attagggcag accttcagga tctatctctg atgcccagcg ctgggcttac acgcatgtgc 29460
tactgtgccc agcattttct ttttttaatt taaaatagat ttttttaaag atatatttct 29520
ggctatgttt tctcttcccc catctcctct aagatcttcc ctaactcccc tcctacccaa 29580
atccacaccc tttctctctc tctctcacta gaacacaaac aggcactaag aaacagtaat 29640
aagataaggc aaacaaactg gaataaggtg aaatagacca acagaaaaaa aaaaagagcc 29700
aaagagaaag tataagaatg ataatatatt tgccagcatt tttatgtgag ttctgtggat 29760
gggagctcag gtcctcatcg ctctcacggt tgaaccaact gcccagactg catctgtcct 29820
ttcattgtca tcattgttgt tgttgtttgg ttattgttgt gtgtatatgg tattggggca 29880
gcgcgtggac atgccgccgt gtgcacgcgg aggtcagagg gcagcttcat gaagtcagtt 29940
ctctgctctc taaggattaa actaatgttg ccaggtttcc caaccaagaa cttttatctg 30000
tggcgccatc tcaccatccc ctgtttttgt tttgttttttg ttttaagaca aggtcttgta 30060
gtctaggcta gcctgctact tgctgcagtc agctgagggt cccttgaact acttaccttc 30120
ctgcttctac ctccaagtat taggactgta ggtgtgtgct gccatactaa ggtctgggtt 30180
tgttttttgt ttgtttatga gtcaaaaagt cgggctggtg agatggctca gtgagtaaga 30240
gcacccgact gctcttccga aggtccggag ttcaaatccc agcaaccaca tggtggctca 30300
caaccatcgg taacaagatc tgactccctc ttctggtgtg tctgaagata gctacagtgt 30360
acttacatat aataataaat aaatctttaa aaaaaaaaaa aaaaaaaaaa gagtcaaaaa 30420
gtcttactat atagcctaaa actggccttg aacttactat atagcccagg ctggtcttca 30480
actttcaatc ctcttgcctc agcttctcaa gtagttctca gattacatac gttgcaccac 30540
tagacttgta tctgtgcatc ttgctcaaat attttagtcc tatagattat aagaatgaat 30600
aacagtatct ggaaacattc tcatattaac ctttaacctt tttttttcca tgctggggac 30660
caaagccaga gccttgagca cactaggcag gtgtgtgtca ctgagctaca ccacctattg 30720

atggtactgt tgatctgaga atgagaaatg gaaaatgatg gatttgttat gacatcagat 30780
gttataacaa ccccttgatg gtggattctg gggtatacg ttttagagtc ctattattac 30840
tgcctttgag agaatgtcat ccaaactggc ttggaactct ctaggtaggg aaagctggcc 30900
ttgaactctt gatcctcctg tctccaccac cccgtataca gatgtgtgct ctcacaaaac 30960
gctctccaaa ttactcatgc agcttacctc ctcttcctag ggcatatgca gccagcaaag 31020
agtcacatgc cacgttggtg tttcataatc tcttgggtga aattgaccag caatatagcc 31080
gattcctgca agagtccaat gtcctctatc agcacaacct tcgaagaatc aagcagtttc 31140
tgcaggtgcg gtgggaaatg gatgtgagac ggcagcccca cagagtggag tgctgatgtt 31200
cctgcccgga agttttcctt acttggctaa gtgacgcctg tcttgtactc taaatcaaag 31260
gagaagtaat tagctccttc tcaattccct gtacactggc cctgggcacc attaccagaa 31320
attcacttct tatgttacag ttgcagcaat aagcaagggc ttaatgttat tctgttataa 31380
aagcacacta ttaacaccta cttgtcatag gcacatgact ctcaggtcta aatgttaaaa 31440
gtttagccat taactgggta ctcctgggta aagtcagtca tttccctata aagtcagatg 31500
acccacggtg ccaggatgac ctcacacttc tggcttctgc catcacagag gccacatgct 31560
cctgccttcc cttccctccc catggatttc agtcagatgc tgaagccttt gatctgtggt 31620
ttcatggagg ctctgaggaa gtgacttaag ttagccaagt tcactccaca cctccttagc 31680
cggctatagc ttcctactct ccatcttgct tacagagcag gtatcttgag aagccaatgg 31740
aaattgcccg gatcgtggcc cgatgcctgt gggaagagtc tcgcctcctc cagacggcag 31800
ccacggcagc ccaggtgaga gcaagaagaa gcaaagtggc gagcagggga ctgggaagca 31860
agcgattgta taaataagtg gaacagtaat ggtttagtgc ctctgaagag gagatcacta 31920
gactacatta aagccctaaa catacattga aaatgacaaa aaatttcata cagtctctat 31980
tccactccca accaacagtg ttgctgaagg gtgaacttga gctcagattc ctgagggctg 32040
ggagttgagg tgctcacacc acatcctata agtgaggtgc taggggcaga accagggctg 32100
cctgcgtggt ggacacacac tcggagacat attcccaacc ccggattgtt gatgttgttg 32160
atcgggttct tgctatgcat agctgacttc cagtttggga taatcttcct gcctctgcca 32220
cccaagtgca ggggtttcgg tgatgccacc aaacttccca cacgtccatt tcttgttttc 32280
attttcagct tttgagatgg agtcacagtc tgatacccag ctgcctagaa ccctgtgcgt 32340
gggctgagcc ctttcaaacc cacaccagtc ccttgcttca gtctctagtc attgagattt 32400
taggcatggg ccaccaggct cagcttcctt ttgagacaga gtctcaatac atacccccat 32460
atggtaaact ctagatcctc ctgcctcagc ctccctgtga gccaccatgc ctgatccaca 32520
gggacttccc agtgcctgac ccctgagtta gaacttggga gttggcaaag cacacctccc 32580
tagcaatttg tacggagctt gagagactct agaccactgc ttggggaaca tagaggtggt 32640
ttttagggac aaagccttca ttcttgccat aggcaagttt aggagatagg agataggaga 32700
gggagggacc cagctttggg gctgggcact gtgaaggtct gtctgtagta cttctttatg 32760
tcttatcagt cagggtcgat cccacagggg aaaaatgtag tggctgggtt gtttttccaa 32820
gtcttctgag aggctgttat actatagttt gagcatccat gagaagtggg aagagcgcct 32880
gtctctaaaa tgcctgttca gctctacttt ggagactcgt ggtcgtagta gagagaacag 32940
accagaagtg agaggttgta gaagcaaagg tctgggctgg aggacagacc tggcttcagt 33000
tcctagcatt caagaggtgg ctcacaacca tctgcaattc cagttccagg gaatccaaca 33060
cccatttggg caccaggcac acatgctggc aaaaacagcc acacacataa aatgaagtaa 33120
ataagtctaa agaatagaaa acaaaacaaa gtaaaaagaa gtaaaaagcc ggcagttgga 33180
acctgactgg agcccttgac cagtgcgatg ggtgtgaggg agtggttagc tgtaggccca 33240
gggtgtgagc atttctagct ttgaaaactg gatggataac atctctcctt atcatagtct 33300
tcaagatcct caggctgctg tcaggctatc tggttggttc ttaaagcatc tacttgttag 33360
tttctgctcc cagcaaagcc ccatcatttt acctatgacc ccttaaggca ttagcctctc 33420
cgggctgggc agaagccttg gaagcacaat tttaaaagtc cttgggaaat gttttttcat 33480
ggaaatatgt tacactttgt tgtcatcagt tcttaattta tttattaata ttctctgagc 33540
actgggatca agcctgggct tcactcattt aggcaggatc tccagtactg agctgtaccc 33600
cagctctttc tagatttttt tttttttttt tgacagttta cctttaggct gcttgaagga 33660
tgaggcatct tagttcaaca tgtgggctcc tggtttaatg tggagaatgg gtttgcagtg 33720
tttcctgttg ataaaaagcc agtggcacag agagaacagt gcaagcaccc tcccagcacc 33780
ctccccttg gatgggatgg agctcagacg agagggttcc acaaatccat gtgtgtgcta 33840
atcttagtta tttattattt atttatttat tggcagcaag ggggccaggc caaccaccca 33900
acagccgccg tagtgacaga gaagcagcag atgttggagc agcatcttca ggatgtccgg 33960
aagcgagtgc aggtaatcgg gcctcaccca gggagccgcg ctgtgctgtg cggctgcaga 34020
gtgtggtcct gcgtgcacgc tttcattatc ttcaaaactt tcttttccct ttttcccaaa 34080
ggtaacctgt gttttacttc tgtttttggt tggtttgttt gtttaaacag gatctagaac 34140
agaaaatgaa ggtggtggag aacctccagg acgactttga tttcaactac aaaaccctca 34200
agagccaagg aggcaagcga gctgtgggga cggggcagtc tgtgcaaggc tacgccgtgt 34260
cactgagtct cagggcctgc cttccatggt aggaggagga gctgctggcc cttcacggga 34320
caatttttgc attgtttgtt tttgagatgg gatttgacca tgtagctctg gctgaccgtg 34380
agcctacaac catcttactt cagctctctg agtagccggg actgcaggcg cacagatggt 34440
gtagagcctt gttaagaagg ctgtggtgag tccctctgct caaggagtct ttcctccttc 34500
ccttagacat gcaggatctg aatggaaaca accagtctgt gaccagacag aagatgcagc 34560
agctggaaca gatgctcaca gccctggacc agatgcggag agtaagcgat ctggatgctt 34620

cctgagcatc gcacgcctag cgtggaagac agtttctgat ggcttctttc tctagaactg 34680
actagagact gcggttagac atgggctggg gtaatgtgtt ctgacttggt tttggtttcc 34740
aaacagagca ttgtgagtga gctggcgggg ctcttgtcag caatggagta cgtgcagaag 34800
acactgactg atgaagagct ggctgactgg aagaggcggc agcagatcgc gtgcatcgga 34860
ggccctccca acatctgcct ggaccgtctg gaaaactggt aagagttgga agaaaggctt 34920
ctttttcttt agtcacgggc ctgcaagggg gggggggggg ggcggggcgg agcggggcgg 34980
aggcggggca tgctgctccc tcgccatgct tttgcttttg cttttgcttt tgctttttca 35040
tgctggggtg gatgtgtggt agacaagtgt gccagagcca ctggactaca ctcctagtgc 35100
tggacactat tgctaagatg gcaccttttt ttttctttga gaaagaatct cactgagctg 35160
cataggctgg ccctcatacc cactccatag cccaggctgg ccctcatacc cactccatag 35220
cccaggctgg ccctcatacc tactctatag cccaggctga ccttgaattt gcaatcctcc 35280
tgcttcatct tcccaaatgg aattatctgg gattataggc atgcatcact ctgcctgcct 35340
taaggtggcc ttttttacac agtcaagttt agtccaagaa ttaaaaccat tacctaattt 35400
taactttttt gttttttttct gagacagagt ttctctattt agccttagct gtccttgaac 35460
ttgctctgta gactacactg gctcaaactc atagaggtct gcctgcctct gcttcccaag 35520
tactgagatt aaagacatgc accaccattg cccaaccaac aaatttttaa ttaaaaagaa 35580
taaattaata aagcttagat cactaattta agatatttaa ataatacttt cagttgtatt 35640
taacactaaa ttctccattt tagttatttt aataatctaa aacaatatat gttatttgca 35700
aagggggggaa ataactgaat atatataaaa ctaataaaat gaaataaaaa aaatgaaaat 35760
ctagtctttg agtagtacca gcaagtcacc agactttgtg atagtcattt tgtaggcatc 35820
tgtcgtgatt aaacatgtga tttcatgact atacatgtga tgtcataact atacatgtga 35880
agctagaaat cgagttgtcc gtctccttcc tcccttcctt ccttccttcc ttcctttgtt 35940
gtggtgggat ggatgcctca gggtcccttt gcttcagtct cctgagtgct ggggtcacca 36000
ctaggggaca gaggtgactg gaacttcctt ccattatggc aggataactt cattagcaga 36060
atctcaactt cagacccgcc aacaaattaa gaaactggag gagctgcagc agaaagtgtc 36120
ctacaagggc gaccctatcg tgcagcaccg gcccatgctg gaggagagga tcgtggagct 36180
gttcagaaac ttaatgaaga ggtaacttgt aagagatgac gttccgaagg gaggaaccgg 36240
cagaagactg gtcaccacag cgtctgagat gccatccttt tcccttccag tgccttcgtg 36300
gtggagcggc agccctgcat gcccatgcac ccggaccggc ccttagtcat caagactggt 36360
gtccagttta ccacgaaagt caggtgggcc tgtccttaca ctttgggtgg agttttgatt 36420
gatgtccagt cctcttccct gaggatttta agggaaggaa ggttctgtgg tgccatagct 36480
gaggggcccc attgggtgag agatgaagcc cactcctcac ctggctcaga acacgtgcag 36540
tatttctcat tgaatgcttt ttattgcctt cccgtgagaa gtctcttaca acttgcttca 36600
catgcttagg catctcagta ctttctagct gaagggttag tgttcacttt taatgtcaga 36660
gatgactctg cggaagtaga atttgggttt gtaaaaataa tatatatata tatatatata 36720
tatatgcgtg cgtgcgtgtg tgtgtgtgtg tgtgtgtgtg tgtgtgtgta tttttcatct 36780
agacaggtct ttgtagccct ggctggcctg gaacactctc tgtaaactag gctggcttga 36840
acccagagat tcacctccct ttgcctccca agtgctggga ttaaaggcat gcaccaccat 36900
gctcagcaat atttttacat cttaggaatt gaagtattag gggggtttata ttttaacgag 36960
atcaaggtag ggagcctact aactttcctt gactatattt tttaataggt tgctggtcaa 37020
atttcctgag ttgaattatc agcttaaaat taaagtgtgc attgataagt aagtattctt 37080
tatgtgtgtg tgtgtgtaag tgtataattt ttattattta aaagcaactg ttaaattcat 37140
gctttgctca tattcttccc ttctcccaag tacattcaga tctcctcctc ctgtactcac 37200
ccaagtttct caaaaaaact aaacaaccaa aaccccaata caacaaaatc cccaaaagac 37260
tgagaacaaa atacaacacc caaaaagaaa aaagatggga ctaaaattgt gattaaataa 37320
gagcatgcac aacaaactat ttgttggtca actgtcctgc tcacgagacc cgcactgagt 37380
ggttgacaaa gccagtgctg ctcccagcag ctccattgct aatctttcac cctagtggtg 37440
ggtttacatt cattcattca ttcattcatt ttttttaatg taacaaaata aaatataaca 37500
aagcaaaaca aaggctatga catccaagtt ggacgagaca aaccaacagg aggaaaagaa 37560
cccaagagaa ggcacaggag tcggagtccc actcgtttgc accctgggaa tcccatgaga 37620
acactacatg ggaagccaca atattagcac agaggccctg gtgcaggccc ctgcaggccc 37680
agggcatgct gctccagtct ctgcgagttc atatgagctt tgctcgtggt gattcagagg 37740
gccttgtggt tttggtgacc tctaacccct ctggttctta atgtttttttc tgctgcttct 37800
tccttggtat tccctgagct ctgaggggag ggatttgctg gagacatccc atttagggaa 37860
tgagtgtttc aaggtctttc tctgcatgat gtctggctgt gggtctctat atttcttccc 37920
atctttggac aggaggactc ttctctgatg atggctgagc aaggcactga tatctgagtc 37980
tagcagaatg tcacctggag tcattttatt gtttttttttt cctttctctt ttttagatca 38040
gtattattta gtttttccct aggtccttgg gctatctagt ctctggttct tggccaccca 38100
agccttgttg ggtatgagtt tcatcttgta tagtgagcct taagactctt gttggttggt 38160
tacttccaca ggtttgtgcc accattgccc tagcatatcc tgcaggcagt acactattac 38220
agatagaggt ttgaggctgg cttgctgttc acacttctct tttgatagca tgcagagtac 38280
cttcctataa caaagactct ggcatacagt ggggggctct atggaggcac cagcttcacg 38340
tgcccatgtt cagtgagttg tgtaggtgtt atcctcaaca gtggggcctt gctgtcagtt 38400
gtggaaagca accgatggtc ttgataacag cgtggattgt ttgaggattc cctttggcca 38460
acaattcaat tagacttaac cctaacccag tactggaagc tttatttggt ggcaagagat 38520

cgccagttgg gaccccaact ctcccccatt acttggcgat tgaatctaga tcaccttcac 38580
atatgtgaat gttttaggag gttcctactg tacgagatgt cgatactact ccacggatgc 38640
tccataagtg ttgccgtctc ccattgttcc ctcccaccac cagctctcct ctcccctcgg 38700
cttccccatt tgctcctcca agttcagacc tcgtctatcc agaatccctc atctatccag 38760
aatccctcat ctatccagaa tccctcatct atccagaacc ccttctattt ctttttttcct 38820
aattagatct atttgttctc tgtagttcct tagtctgtat tcaccctctg tggttcttaa 38880
ggattgcagc ttggttatca ttgacttaat ggctaatatc catacataag tgagtacaca 38940
ccacatttgt ctttctgggt ctaggttacc tcactcagga agattttttt tttcctagtt 39000
ccatctattt gcctgcaagt ttcttttttt ttttttttaat ggctgagtaa tactccgtat 39060
gtaaatgtac cacattttct ttattcattc ttctgttgag ggacatctag gttgtttcca 39120
gcttctggtt agtatggaga gaacagcagt gaacgtggtt gagcaagtgt ctgtggtagg 39180
atgaagcatc ctttgggtat gtgcccaaga gtggtctaac tggatcttga ggtagatgga 39240
ttcccgtctt cctgaggagc ggccatactg atttccatag ttgctgcatg agcttgcatt 39300
cccaccagca atatgtgatg gtggccctta ctccacatcc tcgacagcat gagctgtcac 39360
ttgtgttgat cttaactatc ctgttaggca tatgatagac tctcacagca gtttgatttg 39420
cattccctga tcctaaggat gttggacatt tctctgtttc tcagccactt gggtttcctc 39480
tattaagaat tttctgttta gatctgtacc ccatttctaa ctggtctgta ttttcttgat 39540
atttattttc ttaaaattct tttcgggcat ttgtcctcta ttggatatgt agttggttta 39600
aaagaaaaat cccgttctgt aggctaccaa tccgtattct taagcctaac tcttgttttc 39660
aaatgaggga tggagaagca agaacttgga cagacatttg actttagaga tgtctccatt 39720
cagggatctt ctttttgtga acagctgtgt tcaaaaccct tgcaaaccct gcccggtttg 39780
cctgaagcat tgaaaagaaa agtttgagtg tgtgcagctc agtattgcag gctcacctag 39840
cacgcacgca tgcacgaggc ccgggttcaa cccccatact gaaaaataaa cgtgtaaata 39900
agtaatgctt gaaaatagag tatatggaaa gagaactggt ttgaagttaa taatcttctt 39960
ccacaaaccc accaacacat gctatttgta ggtaaaaata tagcaatgga aaagaaattt 40020
tacttagttt tattttgaga gagcgtctga ctctacaacc ctggctggcc ttgaattcac 40080
agtgactcac ctgcctgtgc tccccagtgc taggattaaa ggtatctgac accaagtctg 40140
gctaagaaga acttgtaaaa tagtgttctg aaataagata aagacagaaa tgtcataata 40200
taaaaagatg gcctgtcaga gacaggcagc agtggtcacg tgaggatagc atttccagct 40260
gccttgtggg aggtggtgtc agtttttggt aacaacatac ctttctggaa aacccagcgt 40320
ggctccgata ataaggaaat tcaccacccg tgtaactgga aatgcagttg gggcaagtac 40380
ctaaggatga ggctggtgac tcacaggtgt cttcaggaac cctgggtctt ccatgccatc 40440
cttaccgtgc catccttacc gtgccagctg tagacaggtt cccttccagg tggtctcatg 40500
gctgtggaca gaagatcagc ctaggctatt gcctccttgg tctggctgag aacaggcctt 40560
taatcccagc acccaggagg cagaagcagg tgatctctga gttcaggtct acagagcaag 40620
ttccacgaca gccaggaggg ctacacagag cgctgtctcg gaaagccgac aaaacagaca 40680
aggcagaccc cgtcttcacc tgctctgtgg cttcctctgt tctgatgaat gcagtgatcg 40740
gggttccctg gagatagatg gctctgccat ccccttactga gagaatggag ctttccttag 40800
gaagggaaag aagctgtggt taggccccca gctaaggcta ctgtttaagt gcatacagag 40860
ttgtgtttca caccgaaaat gtaactgagc atcgaaaggc atgttccatc agagtaggtt 40920
ctggaaagaa agaggggctc taatctcagc ttttattaac ccttgtctgt gttctttaca 40980
gagactctgg ggatgttgct gccctcagag ggtaagttgg gcctcctttt gttctttctc 41040
atccaacttt accctctggc tactcagcgc tcactgtgtg ttggtttttc tcccatgatt 41100
taggtctcgg aaatttaaca ttctgggcac gaacacaaaa gtgatgaaca tggaggagtc 41160
taacaacggc agcctgtctg cagagttcaa gcacctggtc ggtgtccgag gggagaggag 41220
gcggcacagg ccaggtggca gaggaggggg gagagactgg cacagcctca agtgacactt 41280
gcctccttct tcttcctttc ttccagaccc ttagggagca gagatgtggg aatggaggcc 41340
gtgccaattg tgatgtaagt aagttttgtt cgggctaaaa gcaattgttt tctttctttg 41400
aggaagaaag agaaagctct ctcgctctct ctctctctct ctctctctct ctctctctct 41460
ctctctctct ctctccctct ctccctctct ccctctctcc aacccagaat taaaacttta 41520
acccactact tcagatgttt gtcctttaga aaagttggtc gctctgggcc agggtgacat 41580
gctcagaagt cacaggtgct gtggagtttt ctagcgagaa acatttccct gagtgttggg 41640
gagttggctc cctttcagca gtgggaagtc cctcgccaag ctgttaccca cacccatctg 41700
gtagaaaaga agtttccaca ttgcccgggg cttcccagag tcccttgcta ctacctgcca 41760
gctggctccc ttttcagtgc ctggtgagac aaaaggaaga catcaagtca gccagtcaga 41820
gctaaacaga aaaaaaaaca acaacaaaac aaaacaaaca aaaaaaaacc accaaccaaa 41880
ctgtgctcag gagctgaggc aggggaattg tgagtttgag atcattctgt cattcagcac 41940
atacatgtaa aagagctgga aattcattcc tgagaattca tcagtaattt gtgtactaat 42000
tgatgagggt ctgtggtccc gtgcaggtcc cagttcctct ttcagagctg tagagtattc 42060
tgctgcctac accacagctc atgaccacag gtagccacat tgcttgactg cttgtgtggc 42120
actgaaggtt atcacagtca ctttagaaag tgacttggca taaaaatgag cacaccctgt 42180
aaaccggcag ttccagtctt aggaattaac agtaaagaaa gctttgcata ggtgaactaa 42240
ggacagataa cacagtcaca gccattggtc attgacacag ctcagatctc tcagcactgg 42300
ggtcatgagc tgtggtgtag gcagtggagt actctacagc tctgaaagag gaactgggac 42360
ctgcacggga ccacagaccc tcatcaaggt gggggtgggg tgtcaggagc aggaacacac 42420

tgagagtgca gttcactgtg tgaagtacac atgcttggag aaacaaccat gaacacggtt 42480
gggaggagct gggagacagc agagacaaag acacagtaaa gtgcagagaa gggggattca 42540
cctggggaat ctcggtgata atggcaactt atgcgggatg ataatgctag gctatcgctc 42600
agtgttgggg tgcacttaga tgcatgagac cctgagtttg ctccctagtg catataaaag 42660
gaaggaggga gagagagaag aaacctaaga agccttcctt tattccttcc tattctcccc 42720
tccttccttc cctttctccc tccctccttt cctctctcct ccctctccct ccctcctccc 42780
tctccctccc tctccctctc tcctccctct ccctcccttc ctctctctcc cctccctccc 42840
ctcccttccc tcttcctccc tcccttcctt cctccttcct ctttctctcc cctccctcct 42900
tctccctccc tcctccctcc catcctccct tcctccctcc ctccctccct ccctccctcc 42960
ctccctccct ccctccctcc ctccctccct cccttccttc cttccttcct tccttccttc 43020
cttccttcct tcctgtgtgt ctgtgtttgt gtatggtcgc acatgtgcga tggtgtgaat 43080
gtgaaggtca agttgccact tgaaggactt gtttcttctt ccaccaagtg ggtcccaggg 43140
atgaggctcg ggttggcagg cagatgccct tgcccactga gccatcttgc tggccctcaa 43200
ataaggacgt aagctgggtg gcttatcctt ggatgctcat tgctttgtta ttcttcgtgc 43260
cttgaatgtg tattgtaaga tatggttatg tactatgtag tgcatatgtg aggatcatgt 43320
ccttgggtct ttgggtggaa gaaagagggc agagttgtgt ccctgccagt gtctctgaga 43380
gaccttatgc tgttgcaggc ctccttgatc gtgactgagg agctgcacct gatcaccttc 43440
gagactgagg tgtaccacca aggcctcaag attgacctag aggtgaggcc ttcccagaac 43500
caggcggctc gtgtgcgggg gtgcgtgtgc agggaggcca ggacagaacg cctgccggga 43560
tgatggctgt ctgcccagcc tggatttgtt tctcattatc tatctcagaa acgtccctga 43620
gagccaccct gtccccaaga gtaaagtctt gcttgagtac ctgttatgaa aatctatatt 43680
agtgattgtt tagaatcgat ttgatgaagt ttgggcaagt gggtcttagt agccgccatt 43740
tagattttat tttcctgctt gcttgcttgc ttgtttcctt ccttccttcc ttaaagattt 43800
atttatttta tgtatgtgag tacaccatta ctctcttcag acacaccaga agagggaatt 43860
agattccatc acagatggtt gtaaagccac catgtggttg ctgggaattg aactcaggac 43920
ctcaggaaga atagtcagtg ctcttaactg ataggccatc tctccagccc ctgttttcca 43980
tctttcttga tcttattaag tttagacctg gaaaagcaga catccttagg tttgatttct 44040
aacacctcag cttcatggaa agatgcaggt tgtttggagg ttgccttcca ggtctgtctc 44100
tggagagtcg ggctgagggc ctgggttagc cttagtgttt gggttagtgt tagttgtttg 44160
tttgtttgtt tttaaaggtt tgtttgttta tttatttatt atgagtgcac tgtggctgtc 44220
ttcagacact ccagaagagg gagtcatatc ttttttatgg atggttgtga gccaccgtgt 44280
ggttgctggg atttgaactc gggaccttcg gaagagcagt cagtgctcat aactgctgag 44340
ccatctctcc agccctagtg ttagttttta gggcctgact ttgaacaagt cactgatgat 44400
gttcagacat cagtgctgat gcaggctggt cctgatgcag gctggtcctg atgcaggctg 44460
gtcctgatgc aggctggtct ttctcctctt ggtgttcttg ctccagcctg cacttgccgt 44520
gaggggtggc catggtgtga tttttaattc taacttattg agatgcgggt gacaagggtt 44580
gggagctgtg agagcccagc tgtcccacta agtcattcac cgcctgtccc catactctac 44640
agacccactc cttgccagtt gtggtgatct ccaacatctg tcagatgcca aatgcttggg 44700
catcaatcct gtggtataac atgctgacca ataaccccaa ggtgagtttc ccaccccccaa 44760
ccctctctga aatccctgaa ccttgctgct tctccacccc cggtgcaggc tgtcagtgtc 44820
actctctcat ccaagtgggt gcaggctgtc agtgtcactc tctcatccaa gtgggtgcag 44880
gctgtcagtg tcactctctc atccaagtgg gtgcaggctg tcagtgtcac tctctcatcc 44940
aagtgaacaa atgtgttctt ttcaatagaa cgtgaacttc ttcactaagc cgccaattgg 45000
aacctgggac caagtggccg aggtgctcag ctggcagttc tcgtccacca ccaagcgggg 45060
gctgagcatc gagcagctga caacgctggc tgagaagctc ctaggttgac agcttcagac 45120
atgtctggcc tggggggtgg tcattcagtc ggggtcccca tgtcagccta gggcaaggca 45180
ccatgccagg accaggttgc taagtgtgtt tgaagagcat gtttctttct ttaaaaagcc 45240
aaagacaata gttttaaatt ttttctcccc tttaaaaaaa tatctggact gaaagtaact 45300
ttgacctgtg ctcctacaaa aaaaaaaaaa aaaaggctcc tgaccccagc tgccagcttt 45360
gtcactgtgt gttgccagtt ctcagaccac acaaggcaag gacaagcagg ggtccaacag 45420
gacatctgtg ctggcctaat ctagggacag ctgttgggct tgggtgtgtg tgggcatgtg 45480
tggcacacac cctgtaagta catgtacaaa tgcatggcct ttttgtaaat tctgcttaag 45540
aaatagcttt ggccaaggct gaaatccggt tgttgttttc tcttccaggg cctggtgtga 45600
actactcagg gtgtcagatc acatgggcta aattctgcaa agtaagtgat cctgttgatt 45660
ccctcagtta ggaggtaggt gtggggtgcc caggacccct ttctccagca gctgtcagga 45720
gctgcctgag gatagaggaa cctgagggtg taagaaggct ctggcaggaa agagctgggg 45780
tgagtggtgg tggacttgct ttcccattct tttctccagg aaaacatggc tggcaagggc 45840
ttctccttct gggtctggct agacaatatc atcgaccttg tgaaaaagta tatcttggcc 45900
ctttggaatg aagggtaagg ttaaactctg cagtaaggct gccctgtgta acgagtcacc 45960
tactcccagc aagcccgtga ctggttgtcc ttggctgcag gtacatcatg ggtttcatca 46020
gcaaggagcg ggagcgggcc atcctaagca caaagccccc gggcaccttc ctactgcgct 46080
tcagcgagag cagcaaagaa ggaggggtca ctttcacttg ggtggaaaag gacatcagtg 46140
gtaagaacgc ttgcctcctg tatcacttgc tccacctgct ggccactggc agcacccggc 46200
tacttcatgt tctgggtact actgccccct gaagggcaga gagggcctgt gcctcctgaa 46260
gaggtggcct gccagtttgt cctgttcact catttggcta acatttgtgt agaaaagtgt 46320

gattgaaatc ttacttctgt actccctttc ctgcctaccc cgttaataca tttgcggcag 46380
gtgtttttgc ctatgagcca gctggatctc tacaagcccg cctggatatt gcctcagttc 46440
caagtcactg ggcagggcat tcagagtctc cagcactcac tccactggca taatctctcc 46500
ttccatggtc cgtgacaggc aagacccaga tccagtctgt agagccatac accaagcagc 46560
agctgaacaa catgtcattt gctgaaatca tcatgggcta taagatcatg gatgcgacca 46620
acatcctggt gtctccactt gtctacctct accccgacat tcccaaggag gaggcatttg 46680
gaaagtactg taggcccgag agccaggagc accccgaagc cgacccaggt agttgttgac 46740
tttccatggc tttgccttcc ttcctagtga gaaagtacgc atccttggag agacaaggac 46800
atggctgagt atcttgtggg agcagggctt ggtgattcct tcttttggga aagagtggtt 46860
tattgggttc cttgacaggg tgactaactc aggattcaag aggaggacag aatcagccct 46920
cagggagctt ggaagtctga agatgaaaat agtatctctc cctcttcctc tcttctccct 46980
ttccatctcc ccctccctcc caccccgcca ctttaaaaca ctagaaagag aaatgaaggc 47040
aggtctctct ggtgcttctc cagtcagtgg gttccagaag acccgagaca gatttgagtc 47100
cagcatgctt tgctgtcctg agtgagcagt gccaggagtc tgaagaaagg ctttctgggg 47160
cagtggctgt tggcagcctg ggcagagct gacatcaagc tggaggtgtt gatgttccag 47220
agatgatgac tggagggaat gtggacttgg catttagaaa caagatgcat ctactctgtg 47280
ggagtgcgtg tggctgtaaa tcagcatttc tcagcctttc acggagcgct taagactata 47340
ggaaaacaga gatttccatt atgattcata actgtagcaa aattacagct atgaagtagt 47400
aagaaaaagt aatgtatggt tggggtcacc acaacatgag gagctgtatt aaagggtcat 47460
agcattcaga aggttgagaa ctgctggtat aaattatatg aaaaatagaa attatgttgt 47520
gttaagtctg tagtgtctgg ggcggtccca gtgtaaagag tcagaggtgt ctggtctgaa 47580
gccatttcaa acttgttgca ttgacgcttg cctctgtcct tatgctggtg aggagtctgg 47640
ttttctaaga ctgttgttga tcattgcaca tgctgagctc cgaggtgccc tgtgtgagcc 47700
ggcctccttg ctctgaggtg ccctgtgtga gccggcctcc ttgctctgag gtgccctgtg 47760
tgagccggcc tccttgctct gaggtgccct gtgtgagccg gcctccttgc tctgaggtgc 47820
cttgtgtgag ccggcctcct tgctctggtg ttttttcacc aaccgcttct gttcacatag 47880
cacctgcaca cagcttcccc ggctctgccc accattcttg gaggagtcgc ttgttgacgg 47940
ttactttcct ttccctttgt tctgctgttg agttctcttc attctgtgga ggagttcagc 48000
tctcacttcc tgtctgtccc ttatctctgc ttccaccttg ccttcagttg gaagatctgt 48060
ttatcagtcc ctctcacact ggtctttgtt cttttcacag aggactttga agtccgagca 48120
ctgatccttt tgactaattt tgctttcaag atccaggccc ctggtcaggc atccacctcc 48180
atcaccattc ctgtgtaggc tgcccagaga cattcctcag gggcaaggcc tgggcatgag 48240
aaaggagccc actgcctgat gggcttgcct gtgagcctag ggagagtgca ctgggggctc 48300
cggagcccga tgatggaagg gtaggggggtt cagatgatcc ccttagggag gtgagcctgt 48360
atgtccctgt cctctgcccc tgctacagga gtattgcagt agagacctcc ccggagcctc 48420
gtcctgggtc ttccttgtct gtcaggaggc tgtaatatac cctgtgctcc tctggaggga 48480
ttttgagcaa gttcaccagg cagcaacagg atgttccttc tctcttccat ctccagtctg 48540
tgtcccgcac tcctacctcg ggccgggtca aagaatattt ctctcttgac cactggagaa 48600
tgaaagtctt ctcttctccg ctctaactta ttgtgtatgc atgtgtgttg ggggtggtcc 48660
ttgttggagg accttgaggg tgtcagctct ctccttctac catgtagccc ctgaggacag 48720
agctcaggct tggtgacaga tgacttaagc actaagccat ctccctgatc ccctctgtct 48780
ctttaaggtg acagttctca ctctagagac cttttctaga actgtaggca gcacagttgc 48840
ctcaataatt cctttgcttc tgtgctgagt taacctgttt cttggccaca aatgagcttg 48900
catattcatc taagagcact ttctaatggc ttctcccagg ggtttgtctt gctggatgct 48960
tgtaagcccc agtgttctgt ctgtgctttt cttagcctac ccacacagcc ccaggcccgc 49020
tgcgagcatc attaactgaa cccccagggc ttcatgccag tgtccccagg gcttccagat 49080
ttcttggcct aacatttcag tcctcacatt tccaacataa acggcagaga tggaagacag 49140
ccagtcctgt ctctttctct gtcccacaca tgaaagagat ggcgccttgg ccaggtttcc 49200
tggctgttgc acatgttctc ggacctggtg tcctgtctcc cagtggctgg ggtatgtttt 49260
cctgattgga agtggctttg gagcctccag cacgtaagca tttagtcctg ttggtgtctg 49320
ggctggtcac caaacaggga agtcaattca caagtgtgac caggtcacag gagaagccta 49380
acgctacaca aagatggttg gtcgctcagg cagggtgggg aaggatgcac aagagttggc 49440
ggtaggccgg gcagtggtgg tgcacacctt taatcccagc acttgggaag cagaggcagg 49500
cggatttctg agttcaagcc cagcctggtc tacagagtaa gttccaggac agccagggct 49560
acacagagaa accctgtctc aaaaaaacaa aaaacaaaaa caaaagagtt gacggtaggc 49620
tgggtagagg tggggcaggt gtctctgcag aagtctttgc agctcaggaa attctatgga 49680
agagatagaa cttcaaactg caagactgga tggcaagcag catgtgatga gggaggacag 49740
ccctaagagc tggactgcgg tgtctgggca gtttctgtaa ggttggctca cgccgtctgg 49800
gagaggcagg aggattcctc ttgcatgttc tcaaagcaag tcacaatctg ttaaagcccg 49860
ggtttcctct ctgcaggtgc tgccccgtac ctgaagacca agttcatctg tgtgacaccg 49920
taagtggccg cccttcccag tttcgcattc atttctagtg tcctagtgtg ctcagttgtc 49980
cccaggaata gggaggacat ggggtgagag ttaccgtgaa gggctggtag ctgctgggct 50040
tgtttagtgc tcagagtgac cttgtgtgtt taaaaagctt gagtttttat tttttctgtt 50100
ggagaccaga gtttgatggc ttgtgtgtgt gtgtttgttc ttttttttt tccccctcca 50160
ttgtgtcttg tcaacctggc ccttcccact cctgccgtcc cccctttcct acagaacgac 50220

```
ctgcagcaat accattgacc tgccgatgtc cccccgcact ttagattcat tgatgcagtt 50280
tggaaataac ggtgaaggtg ctgagccctc agcaggaggg cagtttggtg agtgtttgga 50340
gggtcaggcc ccattgatgc ttgcctgtac agagccaggg cctgccatcc acactggtag 50400
aaatcggctc tagagctggg tgtggtggcc caagcctgag gcagaatgat ccctccctgt 50460
gagttctgag gccagcctgg tctataaagt gagtccagga tagccggggc tctattacac 50520
agagaaaccc agtcttgaaa aaacaaaaca aaacaaaaaa tggctctagg gccatgttct 50580
gctgtggaaa tgatactcac acacatgcag tttcccagta gccacagtaa aagcagtaag 50640
agactttgac agcttgggta agcagcagcg gcacttcagc atctgagaaa tcatggagct 50700
gtcctcccct gtcccgcctc ctctactgag tctttgaaat ggagtggctt tgatgccttt 50760
gatttaggct agtctcaggt ggcagtgtgt ggctaaggct accacaccca gggcagcctc 50820
agaccccctga aagagagagt gagggctgag gtggagactg tgctcttctg ggagggcaag 50880
gccctcctgt gaggggcagg aagtctggct tttctgacct tgtccatgtc tgaaggaaca 50940
ggagactggt ggttcctgag ccctgtcctc gtacagaggg atgagggttt gtgagtcggc 51000
tttagtcagg tggctgaact gacactctct gtgtgtctct gcagagtcgc tcacgtttga 51060
catggatctg acctcggagt gtgctacctc ccccatgtga ggagctgaaa ccagaagctg 51120
cagagacgtg acttgagaca cctgccccgt gctccacccc taagcagccg aaccccatat 51180
cgtctgaaac tcctaacttt gtggttccag attttttttt ttaatttcct acttctgcta 51240
tctttgggca atctgggcac tttttaaaat agagaaatga gtgagtgtgg gtgataaact 51300
gttatgtaaa gaggagagca cctctgagtc tggggatggg gctgagagca gaagggagca 51360
aggggaacac ctcctgtcct gcccgcctgc cctccttttt cagcagctcg gggttggttg 51420
ttagacaagt gcctcctggt gcccatggca tcctgttgcc ccactctgtg agctgatacc 51480
ccaggctggg aactcctggc tctgcacttt caaccttgct aatatccaca tagaagctag 51540
gactaagccc agaggttcct ctttaaatta aaaaaaaaaa aaataagaat taaagggcaa 51600
aacacactga cacagcatag cctttccata tcaaggaata ctcagttaac agcctctcca 51660
gcgctgtctt caggctgatc atctatataa accctggaat ggttgcagat caaatctgta 51720
aaagagatcc gagagctgtg gcttggcctc tggttcaaac acaaaggcta gagagaacct 51780
agatatccct gggttttgtt tacccagtat gcttgtcggt tggaggtgtg aggtaggcca 51840
agggcactgg aaagcctttg tcatcaccct actccctccc caacccagac tccagaccct 51900
gtttcagggt cagcctgccc tgtgggtgcc ttactgggcc tagggtcaac ctgccttcct 51960
ttcccacttg accttgctgg t                                           51981
```

<210> 462
<211> 2951
<212> DNA
<213> Mus musculus

<220>
<221> CDS
<222> (156)...(2468)

<400> 462

```
cgctgaggta caacccccgct cggtgtcgcc tgaccgcgtc ggctaggaga ggccaggcgg 60
ccctcgggag cccagcagct cgcgcctgga gtcagcgcag gccggccagt cgggcctcag 120
ccccggagac agtcgagacc cctgactgca gcagg atg gct cag tgg aac cag    173
                                     Met Ala Gln Trp Asn Gln
                                      1               5

ctg cag cag ctg gac aca cgc tac ctg gag cag ctg cac cag ctg tac   221
Leu Gln Gln Leu Asp Thr Arg Tyr Leu Glu Gln Leu His Gln Leu Tyr
            10                  15                  20

agc gac agc ttc ccc atg gag ctg cgg cag ttc ctg gca cct tgg att   269
Ser Asp Ser Phe Pro Met Glu Leu Arg Gln Phe Leu Ala Pro Trp Ile
        25                  30                  35

gag agt caa gac tgg gca tat gca gcc agc aaa gag tca cat gcc acg   317
Glu Ser Gln Asp Trp Ala Tyr Ala Ala Ser Lys Glu Ser His Ala Thr
    40                  45                  50

ttg gtg ttt cat aat ctc ttg ggt gaa att gac cag caa tat agc cga   365
Leu Val Phe His Asn Leu Leu Gly Glu Ile Asp Gln Gln Tyr Ser Arg
 55                  60                  65                  70
```

```
ttc ctg caa gag tcc aat gtc ctc tat cag cac aac ctt cga aga atc   413
Phe Leu Gln Glu Ser Asn Val Leu Tyr Gln His Asn Leu Arg Arg Ile
                75                  80                  85

aag cag ttt ctg cag agc agg tat ctt gag aag cca atg gaa att gcc   461
Lys Gln Phe Leu Gln Ser Arg Tyr Leu Glu Lys Pro Met Glu Ile Ala
            90                  95                  100

cgg atc gtg gcc cga tgc ctg tgg gaa gag tct cgc ctc ctc cag acg   509
Arg Ile Val Ala Arg Cys Leu Trp Glu Glu Ser Arg Leu Leu Gln Thr
        105                 110                 115

gca gcc acg gca gcc cag caa ggg ggc cag gcc aac cac cca aca gcc   557
Ala Ala Thr Ala Ala Gln Gln Gly Gly Gln Ala Asn His Pro Thr Ala
    120                 125                 130

gcc gta gtg aca gag aag cag cag atg ttg gag cag cat ctt cag gat   605
Ala Val Val Thr Glu Lys Gln Gln Met Leu Glu Gln His Leu Gln Asp
135                 140                 145                 150

gtc cgg aag cga gtg cag gat cta gaa cag aaa atg aag gtg gtg gag   653
Val Arg Lys Arg Val Gln Asp Leu Glu Gln Lys Met Lys Val Val Glu
                155                 160                 165

aac ctc cag gac gac ttt gat ttc aac tac aaa acc ctc aag agc caa   701
Asn Leu Gln Asp Asp Phe Asp Phe Asn Tyr Lys Thr Leu Lys Ser Gln
            170                 175                 180

gga gac atg cag gat ctg aat gga aac aac cag tct gtg acc aga cag   749
Gly Asp Met Gln Asp Leu Asn Gly Asn Asn Gln Ser Val Thr Arg Gln
        185                 190                 195

aag atg cag cag ctg gaa cag atg ctc aca gcc ctg gac cag atg cgg   797
Lys Met Gln Gln Leu Glu Gln Met Leu Thr Ala Leu Asp Gln Met Arg
    200                 205                 210

aga agc att gtg agt gag ctg gcg ggg ctc ttg tca gca atg gag tac   845
Arg Ser Ile Val Ser Glu Leu Ala Gly Leu Leu Ser Ala Met Glu Tyr
215                 220                 225                 230

gtg cag aag aca ctg act gat gaa gag ctg gct gac tgg aag agg cgg   893
Val Gln Lys Thr Leu Thr Asp Glu Glu Leu Ala Asp Trp Lys Arg Arg
                235                 240                 245

cag cag atc gcg tgc atc gga ggc cct ccc aac atc tgc ctg gac cgt   941
Gln Gln Ile Ala Cys Ile Gly Gly Pro Pro Asn Ile Cys Leu Asp Arg
            250                 255                 260

ctg gaa aac tgg ata act tca tta gca gaa tct caa ctt cag acc cgc   989
Leu Glu Asn Trp Ile Thr Ser Leu Ala Glu Ser Gln Leu Gln Thr Arg
        265                 270                 275

caa caa att aag aaa ctg gag gag ctg cag cag aaa gtg tcc tac aag   1037
Gln Gln Ile Lys Lys Leu Glu Glu Leu Gln Gln Lys Val Ser Tyr Lys
    280                 285                 290

ggc gac cct atc gtg cag cac cgg ccc atg ctg gag gag agg atc gtg   1085
Gly Asp Pro Ile Val Gln His Arg Pro Met Leu Glu Glu Arg Ile Val
295                 300                 305                 310

gag ctg ttc aga aac tta atg aag agt gcc ttc gtg gtg gag cgg cag   1133
Glu Leu Phe Arg Asn Leu Met Lys Ser Ala Phe Val Val Glu Arg Gln
                315                 320                 325

ccc tgc atg ccc atg cac ccg gac cgg ccc tta gtc atc aag act ggt   1181
```

```
Pro Cys Met Pro Met His Pro Asp Arg Pro Leu Val Ile Lys Thr Gly
            330                 335                 340

gtc cag ttt acc acg aaa gtc agg ttg ctg gtc aaa ttt cct gag ttg    1229
Val Gln Phe Thr Thr Lys Val Arg Leu Leu Val Lys Phe Pro Glu Leu
        345                 350                 355

aat tat cag ctt aaa att aaa gtg tgc att gat aaa gac tct ggg gat    1277
Asn Tyr Gln Leu Lys Ile Lys Val Cys Ile Asp Lys Asp Ser Gly Asp
    360                 365                 370

gtt gct gcc ctc aga ggg tct cgg aaa ttt aac att ctg ggc acg aac    1325
Val Ala Ala Leu Arg Gly Ser Arg Lys Phe Asn Ile Leu Gly Thr Asn
375                 380                 385                 390

aca aaa gtg atg aac atg gag gag tct aac aac ggc agc ctg tct gca    1373
Thr Lys Val Met Asn Met Glu Glu Ser Asn Asn Gly Ser Leu Ser Ala
                395                 400                 405

gag ttc aag cac ctg acc ctt agg gag cag aga tgt ggg aat gga ggc    1421
Glu Phe Lys His Leu Thr Leu Arg Glu Gln Arg Cys Gly Asn Gly Gly
            410                 415                 420

cgt gcc aat tgt gat gcc tcc ttg atc gtg act gag gag ctg cac ctg    1469
Arg Ala Asn Cys Asp Ala Ser Leu Ile Val Thr Glu Glu Leu His Leu
        425                 430                 435

atc acc ttc gag act gag gtg tac cac caa ggc ctc aag att gac cta    1517
Ile Thr Phe Glu Thr Glu Val Tyr His Gln Gly Leu Lys Ile Asp Leu
    440                 445                 450

gag acc cac tcc ttg cca gtt gtg gtg atc tcc aac atc tgt cag atg    1565
Glu Thr His Ser Leu Pro Val Val Val Ile Ser Asn Ile Cys Gln Met
455                 460                 465                 470

cca aat gct tgg gca tca atc ctg tgg tat aac atg ctg acc aat aac    1613
Pro Asn Ala Trp Ala Ser Ile Leu Trp Tyr Asn Met Leu Thr Asn Asn
                475                 480                 485

ccc aag aac gtg aac ttc ttc act aag ccg cca att gga acc tgg gac    1661
Pro Lys Asn Val Asn Phe Phe Thr Lys Pro Pro Ile Gly Thr Trp Asp
            490                 495                 500

caa gtg gcc gag gtg ctc agc tgg cag ttc tcg tcc acc acc aag cgg    1709
Gln Val Ala Glu Val Leu Ser Trp Gln Phe Ser Ser Thr Thr Lys Arg
        505                 510                 515

ggg ctg agc atc gag cag ctg aca acg ctg gct gag aag ctc cta ggg    1757
Gly Leu Ser Ile Glu Gln Leu Thr Thr Leu Ala Glu Lys Leu Leu Gly
    520                 525                 530

cct ggt gtg aac tac tca ggg tgt cag atc aca tgg gct aaa ttc tgc    1805
Pro Gly Val Asn Tyr Ser Gly Cys Gln Ile Thr Trp Ala Lys Phe Cys
535                 540                 545                 550

aaa gaa aac atg gct ggc aag ggc ttc tcc ttc tgg gtc tgg cta gac    1853
Lys Glu Asn Met Ala Gly Lys Gly Phe Ser Phe Trp Val Trp Leu Asp
                555                 560                 565

aat atc atc gac ctt gtg aaa aag tat atc ttg gcc ctt tgg aat gaa    1901
Asn Ile Ile Asp Leu Val Lys Lys Tyr Ile Leu Ala Leu Trp Asn Glu
            570                 575                 580

ggg tac atc atg ggt ttc atc agc aag gag cgg gag cgg gcc atc cta    1949
Gly Tyr Ile Met Gly Phe Ile Ser Lys Glu Arg Glu Arg Ala Ile Leu
```

```
        585                 590                 595

agc aca aag ccc ccg ggc acc ttc cta ctg cgc ttc agc gag agc agc     1997
Ser Thr Lys Pro Pro Gly Thr Phe Leu Leu Arg Phe Ser Glu Ser Ser
    600                 605                 610

aaa gaa gga ggg gtc act ttc act tgg gtg gaa aag gac atc agt ggc     2045
Lys Glu Gly Gly Val Thr Phe Thr Trp Val Glu Lys Asp Ile Ser Gly
615                 620                 625                 630

aag acc cag atc cag tct gta gag cca tac acc aag cag cag ctg aac     2093
Lys Thr Gln Ile Gln Ser Val Glu Pro Tyr Thr Lys Gln Gln Leu Asn
                635                 640                 645

aac atg tca ttt gct gaa atc atc atg ggc tat aag atc atg gat gcg     2141
Asn Met Ser Phe Ala Glu Ile Ile Met Gly Tyr Lys Ile Met Asp Ala
            650                 655                 660

acc aac atc ctg gtg tct cca ctt gtc tac ctc tac ccc gac att ccc     2189
Thr Asn Ile Leu Val Ser Pro Leu Val Tyr Leu Tyr Pro Asp Ile Pro
        665                 670                 675

aag gag gag gca ttt gga aag tac tgt agg ccc gag agc cag gag cac     2237
Lys Glu Glu Ala Phe Gly Lys Tyr Cys Arg Pro Glu Ser Gln Glu His
    680                 685                 690

ccc gaa gcc gac cca ggt agt gct gcc ccg tac ctg aag acc aag ttc     2285
Pro Glu Ala Asp Pro Gly Ser Ala Ala Pro Tyr Leu Lys Thr Lys Phe
695                 700                 705                 710

atc tgt gtg aca cca acg acc tgc agc aat acc att gac ctg ccg atg     2333
Ile Cys Val Thr Pro Thr Thr Cys Ser Asn Thr Ile Asp Leu Pro Met
                715                 720                 725

tcc ccc cgc act tta gat tca ttg atg cag ttt gga aat aac ggt gaa     2381
Ser Pro Arg Thr Leu Asp Ser Leu Met Gln Phe Gly Asn Asn Gly Glu
            730                 735                 740

ggt gct gag ccc tca gca gga ggg cag ttt gag tcg ctc acg ttt gac     2429
Gly Ala Glu Pro Ser Ala Gly Gly Gln Phe Glu Ser Leu Thr Phe Asp
        745                 750                 755

atg gat ctg acc tcg gag tgt gct acc tcc ccc atg tga ggagctgaaa      2478
Met Asp Leu Thr Ser Glu Cys Ala Thr Ser Pro Met  *
    760                 765                 770

ccagaagctg cagagacgtg acttgagaca cctgccccgt gctccacccc taagcagccg 2538
aaccccatat cgtctgaaac tcctaacttt gtggttccag attttttttt ttaatttcct 2598
acttctgcta tctttgggca atctgggcac tttttaaaat agagaaatga gtgagtgtgg 2658
gtgataaact gttatgtaaa gaggagagca cctctgagtc tggggatggg gctgagagca 2718
gaagggagca aggggaacac ctcctgtcct gcccgcctgc cctccttttt cagcagctcg 2778
gggttggttg ttagacaagt gcctcctggt gcccatggca tcctgttgcc ccactctgtg 2838
agctgatacc ccaggctggg aactcctggc tctgcacttt caaccttgct aatatccaca 2898
tagaagctag gactaagccc agaggttcct ctttaaatta aaaaaaaaaa aaa        2951
```

<210> 463
<211> 2918
<212> DNA
<213> Mus musculus

<220>

<221> CDS
<222> (173)...(2341)

<400> 463

```
acgagcccta accggatcgc tgaggtacaa ccccgctcgg tgtcgcctga ccgcgtcggc 60
taggagaggc caggcggccc tcgggagccc agcagctcgc gcctggagtc agcgcaggcc 120
ggccagtcgg gcctcagccc cggagacagt cgagacccct gactgcagca gg atg gct 178
                                                          Met Ala
                                                          1

cag tgg aac cag ctg cag cag ctg gac aca cgc tac ctg gag cag ctg   226
Gln Trp Asn Gln Leu Gln Gln Leu Asp Thr Arg Tyr Leu Glu Gln Leu
        5                   10                  15

cac cag ctg tac agc gac agc ttc ccc atg gag ctg cgg cag ttc ctg   274
His Gln Leu Tyr Ser Asp Ser Phe Pro Met Glu Leu Arg Gln Phe Leu
    20                  25                  30

gca cct tgg att gag agt caa gac tgg gca tat gca gcc agc aaa gag   322
Ala Pro Trp Ile Glu Ser Gln Asp Trp Ala Tyr Ala Ala Ser Lys Glu
35                  40                  45                  50

tca cat gcc acg ttg gtg ttt cat aat ctc ttg ggt gaa att gac cag   370
Ser His Ala Thr Leu Val Phe His Asn Leu Leu Gly Glu Ile Asp Gln
                55                  60                  65

caa tat agc cga ttc ctg caa gag tcc aat gtc ctc tat cag cac aac   418
Gln Tyr Ser Arg Phe Leu Gln Glu Ser Asn Val Leu Tyr Gln His Asn
            70                  75                  80

ctt cga aga atc aag cag ttt ctg cag agc agg tat ctt gag aag cca   466
Leu Arg Arg Ile Lys Gln Phe Leu Gln Ser Arg Tyr Leu Glu Lys Pro
        85                  90                  95

atg gaa att gcc cgg atc gtg gcc cga tgc ctg tgg gaa gag tct cgc   514
Met Glu Ile Ala Arg Ile Val Ala Arg Cys Leu Trp Glu Glu Ser Arg
    100                 105                 110

ctc ctc cag acg gca gcc acg gca gcc cag caa ggg ggc cag gcc aac   562
Leu Leu Gln Thr Ala Ala Thr Ala Ala Gln Gln Gly Gly Gln Ala Asn
115                 120                 125                 130

cac cca aca gcc gcc gta gtg aca gag aag cag cag atg ttg gag cag   610
His Pro Thr Ala Ala Val Val Thr Glu Lys Gln Gln Met Leu Glu Gln
                135                 140                 145

cat ctt cag gat gtc cgg aag cga gtg cag gat cta gaa cag aaa atg   658
His Leu Gln Asp Val Arg Lys Arg Val Gln Asp Leu Glu Gln Lys Met
            150                 155                 160

aag gtg gtg gag aac ctc cag gac gac ttt gat ttc aac tac aaa acc   706
Lys Val Val Glu Asn Leu Gln Asp Asp Phe Asp Phe Asn Tyr Lys Thr
        165                 170                 175

ctc aag agc caa gga gac atg cag gat ctg aat gga aac aac cag tct   754
Leu Lys Ser Gln Gly Asp Met Gln Asp Leu Asn Gly Asn Asn Gln Ser
    180                 185                 190

gtg acc aga cag aag atg cag cag ctg gaa cag atg ctc aca gcc ctg   802
Val Thr Arg Gln Lys Met Gln Gln Leu Glu Gln Met Leu Thr Ala Leu
195                 200                 205                 210

gac cag atg cgg aga agc att gtg agt gag ctg gcg ggg ctc ttg tca   850
Asp Gln Met Arg Arg Ser Ile Val Ser Glu Leu Ala Gly Leu Leu Ser
                215                 220                 225

gca atg gag tac gtg cag aag aca ctg act gat gaa gag ctg gct gac   898
Ala Met Glu Tyr Val Gln Lys Thr Leu Thr Asp Glu Glu Leu Ala Asp
            230                 235                 240
```

```
tgg aag agg cgg cag cag atc gcg tgc atc gga ggc cct ccc aac atc     946
Trp Lys Arg Arg Gln Gln Ile Ala Cys Ile Gly Gly Pro Pro Asn Ile
        245                 250                 255

tgc ctg gac cgt ctg gaa aac tgg ata act tca tta gca gaa tct caa     994
Cys Leu Asp Arg Leu Glu Asn Trp Ile Thr Ser Leu Ala Glu Ser Gln
    260                 265                 270

ctt cag acc cgc caa caa att aag aaa ctg gag gag ctg cag cag aaa    1042
Leu Gln Thr Arg Gln Gln Ile Lys Lys Leu Glu Glu Leu Gln Gln Lys
275                 280                 285                 290

gtg tcc tac aag ggc gac cct atc gtg cag cac cgg ccc atg ctg gag    1090
Val Ser Tyr Lys Gly Asp Pro Ile Val Gln His Arg Pro Met Leu Glu
                295                 300                 305

gag agg atc gtg gag ctg ttc aga aac tta atg aag agt gcc ttc gtg    1138
Glu Arg Ile Val Glu Leu Phe Arg Asn Leu Met Lys Ser Ala Phe Val
            310                 315                 320

gtg gag cgg cag ccc tgc atg ccc atg cac ccg gac cgg ccc tta gtc    1186
Val Glu Arg Gln Pro Cys Met Pro Met His Pro Asp Arg Pro Leu Val
        325                 330                 335

atc aag act ggt gtc cag ttt acc acg aaa gtc agg ttg ctg gtc aaa    1234
Ile Lys Thr Gly Val Gln Phe Thr Thr Lys Val Arg Leu Leu Val Lys
    340                 345                 350

ttt cct gag ttg aat tat cag ctt aaa att aaa gtg tgc att gat aaa    1282
Phe Pro Glu Leu Asn Tyr Gln Leu Lys Ile Lys Val Cys Ile Asp Lys
355                 360                 365                 370

gac tct ggg gat gtt gct gcc ctc aga ggg tct cgg aaa ttt aac att    1330
Asp Ser Gly Asp Val Ala Ala Leu Arg Gly Ser Arg Lys Phe Asn Ile
                375                 380                 385

ctg ggc acg aac aca aaa gtg atg aac atg gag gag tct aac aac ggc    1378
Leu Gly Thr Asn Thr Lys Val Met Asn Met Glu Glu Ser Asn Asn Gly
            390                 395                 400

agc ctg tct gca gag ttc aag cac ctg acc ctt agg gag cag aga tgt    1426
Ser Leu Ser Ala Glu Phe Lys His Leu Thr Leu Arg Glu Gln Arg Cys
        405                 410                 415

ggg aat gga ggc cgt gcc aat tgt gat gcc tcc ttg atc gtg act gag    1474
Gly Asn Gly Gly Arg Ala Asn Cys Asp Ala Ser Leu Ile Val Thr Glu
    420                 425                 430

gag ctg cac ctg atc acc ttc gag act gag gtg tac cac caa ggc ctc    1522
Glu Leu His Leu Ile Thr Phe Glu Thr Glu Val Tyr His Gln Gly Leu
435                 440                 445                 450

aag att gac cta gag acc cac tcc ttg cca gtt gtg gtg atc tcc aac    1570
Lys Ile Asp Leu Glu Thr His Ser Leu Pro Val Val Val Ile Ser Asn
                455                 460                 465

atc tgt cag atg cca aat gct tgg gca tca atc ctg tgg tat aac atg    1618
Ile Cys Gln Met Pro Asn Ala Trp Ala Ser Ile Leu Trp Tyr Asn Met
            470                 475                 480

ctg acc aat aac ccc aag aac gtg aac ttc ttc act aag ccg cca att    1666
Leu Thr Asn Asn Pro Lys Asn Val Asn Phe Phe Thr Lys Pro Pro Ile
        485                 490                 495
```

```
gga acc tgg gac caa gtg gcc gag gtg ctc agc tgg cag ttc tcg tcc    1714
Gly Thr Trp Asp Gln Val Ala Glu Val Leu Ser Trp Gln Phe Ser Ser
    500                 505                 510

acc acc aag cgg ggg ctg agc atc gag cag ctg aca acg ctg gct gag    1762
Thr Thr Lys Arg Gly Leu Ser Ile Glu Gln Leu Thr Thr Leu Ala Glu
515                 520                 525                 530

aag ctc cta ggg cct ggt gtg aac tac tca ggg tgt cag atc aca tgg    1810
Lys Leu Leu Gly Pro Gly Val Asn Tyr Ser Gly Cys Gln Ile Thr Trp
                535                 540                 545

gct aaa ttc tgc aaa gaa aac atg gct ggc aag ggc ttc tcc ttc tgg    1858
Ala Lys Phe Cys Lys Glu Asn Met Ala Gly Lys Gly Phe Ser Phe Trp
            550                 555                 560

gtc tgg cta gac aat atc atc gac ctt gtg aaa aag tat atc ttg gcc    1906
Val Trp Leu Asp Asn Ile Ile Asp Leu Val Lys Lys Tyr Ile Leu Ala
        565                 570                 575

ctt tgg aat gaa ggg tac atc atg ggt ttc atc agc aag gag cgg gag    1954
Leu Trp Asn Glu Gly Tyr Ile Met Gly Phe Ile Ser Lys Glu Arg Glu
    580                 585                 590

cgg gcc atc cta agc aca aag ccc ccg ggc acc ttc cta ctg cgc ttc    2002
Arg Ala Ile Leu Ser Thr Lys Pro Pro Gly Thr Phe Leu Leu Arg Phe
595                 600                 605                 610

agc gag agc agc aaa gaa gga ggg gtc act ttc act tgg gtg gaa aag    2050
Ser Glu Ser Ser Lys Glu Gly Gly Val Thr Phe Thr Trp Val Glu Lys
                615                 620                 625

gac atc agt ggc aag acc cag atc cag tct gta gag cca tac acc aag    2098
Asp Ile Ser Gly Lys Thr Gln Ile Gln Ser Val Glu Pro Tyr Thr Lys
            630                 635                 640

cag cag ctg aac aac atg tca ttt gct gaa atc atc atg ggc tat aag    2146
Gln Gln Leu Asn Asn Met Ser Phe Ala Glu Ile Ile Met Gly Tyr Lys
        645                 650                 655

atc atg gat gcg acc aac atc ctg gtg tct cca ctt gtc tac ctc tac    2194
Ile Met Asp Ala Thr Asn Ile Leu Val Ser Pro Leu Val Tyr Leu Tyr
    660                 665                 670

ccc gac att ccc aag gag gag gca ttt gga aag tac tgt agg ccc gag    2242
Pro Asp Ile Pro Lys Glu Glu Ala Phe Gly Lys Tyr Cys Arg Pro Glu
675                 680                 685                 690

agc cag gag cac ccc gaa gcc gac cca ggt agt gct gcc ccg tac ctg    2290
Ser Gln Glu His Pro Glu Ala Asp Pro Gly Ser Ala Ala Pro Tyr Leu
                695                 700                 705

aag acc aag ttc atc tgt gtg aca cca ttc att gat gca gtt tgg aaa    2338
Lys Thr Lys Phe Ile Cys Val Thr Pro Phe Ile Asp Ala Val Trp Lys
            710                 715                 720

taa cggtgaaggt gctgagccct cagcaggagg gcagtttgag tcgctcacgt         2391
 *

ttgacatgga tctgacctcg gagtgtgcta cctcccccat gtgaggagct gaaaccagaa 2451
gctgcagaga cgtgacttga gacacctgcc ccgtgctcca cccctaagca gccgaacccc 2511
atatcgtctg aaactcctaa ctttgtggtt ccagattttt tttttaatt tcctacttct 2571
gctatctttg ggcaatctgg gcacttttta aaatagagaa atgagtgagt gtgggtgata 2631
aactgttatg taaagaggag agcacctctg agtctgggga tggggctgag agcagaaggg 2691
```

```
agcaagggga acacctcctg tcctgcccgc ctgccctcct ttttcagcag ctcggggttg 2751
gttgttagac aagtgcctcc tggtgcccat ggcatcctgt tgccccactc tgtgagctga 2811
taccccaggc tgggaactcc tggctctgca ctttcaacct tgctaatatc cacatagaag 2871
ctaggactaa gcccagaggt tcctctttaa attaaaaaaa aaaaaaa              2918
```

<210> 464
<211> 23
<212> DNA
<213> Artificial Sequence

<220>
<223> PCR Primer

<400> 464
gccacgttgg tgtttcataa tct 23

<210> 465
<211> 24
<212> DNA
<213> Artificial Sequence

<220>
<223> PCR Primer

<400> 465
gatagaggac attggactct tgca 24

<210> 466
<211> 29
<212> DNA
<213> Artificial Sequence

<220>
<223> PCR probe

<400> 466
ttgggtgaaa ttgaccagca atatagccg 29

<210> 467
<211> 20
<212> DNA
<213> Artificial Sequence

<220>
<223> Antisense Oligonucleotide

<400> 467
cttaattctt agagatccac 20

<210> 468
<211> 20
<212> DNA
<213> Artificial Sequence

<220>
<223> Antisense Oligonucleotide

<400> 468

cacactaatg caagatgcta 20

<210> 469
<211> 20
<212> DNA
<213> Artificial Sequence

<220>
<223> Antisense Oligonucleotide

<400> 469
aaaaagtgat ctaccaacag 20

<210> 470
<211> 20
<212> DNA
<213> Artificial Sequence

<220>
<223> Antisense Oligonucleotide

<400> 470
caactcttac cagttttcca 20

<210> 471
<211> 20
<212> DNA
<213> Artificial Sequence

<220>
<223> Antisense Oligonucleotide

<400> 471
atcaatgaat ctaaagtgcg 20

<210> 472
<211> 20
<212> DNA
<213> Artificial Sequence

<220>
<223> Antisense Oligonucleotide

<400> 472
cactgagcca tcctgctgca 20

<210> 473
<211> 20
<212> DNA
<213> Artificial Sequence

<220>
<223> Antisense Oligonucleotide

<400> 473
agctggttcc actgagccat 20

<210> 474

<211> 20
<212> DNA
<213> Artificial Sequence

<220>
<223> Antisense Oligonucleotide

<400> 474
tgtgctgata gaggacattg 20

<210> 475
<211> 20
<212> DNA
<213> Artificial Sequence

<220>
<223> Antisense Oligonucleotide

<400> 475
agactcttcc cacaggcatc 20

<210> 476
<211> 20
<212> DNA
<213> Artificial Sequence

<220>
<223> Antisense Oligonucleotide

<400> 476
cagtcagcca gctcttcatc 20

<210> 477
<211> 20
<212> DNA
<213> Artificial Sequence

<220>
<223> Antisense Oligonucleotide

<400> 477
agttatccag ttttccagac 20

<210> 478
<211> 20
<212> DNA
<213> Artificial Sequence

<220>
<223> Antisense Oligonucleotide

<400> 478
tccagtttct taatttgttg 20

<210> 479
<211> 20
<212> DNA
<213> Artificial Sequence

<220>
<223> Antisense Oligonucleotide

<400> 479
tgtaggacac tttctgctgc 20

<210> 480
<211> 20
<212> DNA
<213> Artificial Sequence

<220>
<223> Antisense Oligonucleotide

<400> 480
actggacacc agtcttgatg 20

<210> 481
<211> 20
<212> DNA
<213> Artificial Sequence

<220>
<223> Antisense Oligonucleotide

<400> 481
tttaatttta agctgataat 20

<210> 482
<211> 20
<212> DNA
<213> Artificial Sequence

<220>
<223> Antisense Oligonucleotide

<400> 482
cacactttaa ttttaagctg 20

<210> 483
<211> 20
<212> DNA
<213> Artificial Sequence

<220>
<223> Antisense Oligonucleotide

<400> 483
caatgcacac tttaatttta 20

<210> 484
<211> 20
<212> DNA
<213> Artificial Sequence

<220>
<223> Antisense Oligonucleotide

<400> 484
gcccagaatg ttaaatttcc 20

<210> 485
<211> 20
<212> DNA
<213> Artificial Sequence

<220>
<223> Antisense Oligonucleotide

<400> 485
ccctaagggt caggtgcttg 20

<210> 486
<211> 20
<212> DNA
<213> Artificial Sequence

<220>
<223> Antisense Oligonucleotide

<400> 486
ccattcccac atctctgctc 20

<210> 487
<211> 20
<212> DNA
<213> Artificial Sequence

<220>
<223> Antisense Oligonucleotide

<400> 487
aaggtgatca ggtgcagctc 20

<210> 488
<211> 20
<212> DNA
<213> Artificial Sequence

<220>
<223> Antisense Oligonucleotide

<400> 488
gggtctctag gtcaatcttg 20

<210> 489
<211> 20
<212> DNA
<213> Artificial Sequence

<220>
<223> Antisense Oligonucleotide

<400> 489
caccacaact ggcaaggagt 20

<210> 490
<211> 20
<212> DNA
<213> Artificial Sequence

<220>
<223> Antisense Oligonucleotide

<400> 490
ggtcagcatg ttataccaca 20

<210> 491
<211> 20
<212> DNA
<213> Artificial Sequence

<220>
<223> Antisense Oligonucleotide

<400> 491
acttggtccc aggttccaat 20

<210> 492
<211> 20
<212> DNA
<213> Artificial Sequence

<220>
<223> Antisense Oligonucleotide

<400> 492
gacaccctga gtagttcaca 20

<210> 493
<211> 20
<212> DNA
<213> Artificial Sequence

<220>
<223> Antisense Oligonucleotide

<400> 493
ccagccatgt tttctttgca 20

<210> 494
<211> 20
<212> DNA
<213> Artificial Sequence

<220>
<223> Antisense Oligonucleotide

<400> 494
atgatattgt ctagccagac 20

<210> 495
<211> 20
<212> DNA

<213> Artificial Sequence

<220>
<223> Antisense Oligonucleotide

<400> 495
ccaagatata ctttttcaca 20

<210> 496
<211> 20
<212> DNA
<213> Artificial Sequence

<220>
<223> Antisense Oligonucleotide

<400> 496
cttcattcca aagggccaag 20

<210> 497
<211> 20
<212> DNA
<213> Artificial Sequence

<220>
<223> Antisense Oligonucleotide

<400> 497
ccaagtgaaa gtgacccctc 20

<210> 498
<211> 20
<212> DNA
<213> Artificial Sequence

<220>
<223> Antisense Oligonucleotide

<400> 498
actgatgtcc ttttccaccc 20

<210> 499
<211> 20
<212> DNA
<213> Artificial Sequence

<220>
<223> Antisense Oligonucleotide

<400> 499
gggtcttgcc actgatgtcc 20

<210> 500
<211> 20
<212> DNA
<213> Artificial Sequence

<220>

<223> Antisense Oligonucleotide

<400> 500
gactggatct gggtcttgcc 20

<210> 501
<211> 20
<212> DNA
<213> Artificial Sequence

<220>
<223> Antisense Oligonucleotide

<400> 501
cacagatgaa cttggtcttc 20

<210> 502
<211> 20
<212> DNA
<213> Artificial Sequence

<220>
<223> Antisense Oligonucleotide

<400> 502
gttggtgtca cacagatgaa 20

<210> 503
<211> 20
<212> DNA
<213> Artificial Sequence

<220>
<223> Antisense Oligonucleotide

<400> 503
caatggtatt gctgcaggtc 20

<210> 504
<211> 20
<212> DNA
<213> Artificial Sequence

<220>
<223> Antisense Oligonucleotide

<400> 504
caggtcaatg gtattgctgc 20

<210> 505
<211> 20
<212> DNA
<213> Artificial Sequence

<220>
<223> Antisense Oligonucleotide

<400> 505

catcaatgaa tctaaagtgc 20

<210> 506
<211> 20
<212> DNA
<213> Artificial Sequence

<220>
<223> Antisense Oligonucleotide

<400> 506
aactgcatca atgaatctaa 20

<210> 507
<211> 20
<212> DNA
<213> Artificial Sequence

<220>
<223> Antisense Oligonucleotide

<400> 507
ttccaaactg catcaatgaa 20

<210> 508
<211> 20
<212> DNA
<213> Artificial Sequence

<220>
<223> Antisense Oligonucleotide

<400> 508
gttatttcca aactgcatca 20

<210> 509
<211> 20
<212> DNA
<213> Artificial Sequence

<220>
<223> Antisense Oligonucleotide

<400> 509
aggtcagatc catgtcaaac 20

<210> 510
<211> 20
<212> DNA
<213> Artificial Sequence

<220>
<223> Antisense Oligonucleotide

<400> 510
agtcctagct tctatgtgga 20

<210> 511

<211> 20
<212> DNA
<213> Artificial Sequence

<220>
<223> Antisense Oligonucleotide

<400> 511
atcaatgaat ggtgtcacac 20

**Description**

## FIELD OF THE INVENTION

This invention relates to compositions and methods for modulating expression of the human STAT3 gene, which encodes a naturally present DNA-binding protein involved in signal transduction and transcriptional activation, and is implicated in disease. This invention also relates to methods for inhibiting STAT3-mediated signal transduction and transcriptional activation; these methods can be used diagnostically or therapeutically. Furthermore, this invention relates to treatment of conditions associated with expression of the human STAT3 gene.

## BACKGROUND OF THE INVENTION

The STAT (signal transducers and activators of transcription) proteins are DNA-binding proteins that play a dual role in signal transduction and activation of transcription. Presently, there are six distinct members of the STAT family (STAT1, STAT2, STAT3, STAT4, STAT5, and STAT6) and several isoforms (STAT1-alpha, STAT1-beta, STAT3-alpha and STAT3-beta). The activities of the STATs are modulated by various cytokines and mitogenic stimuli.

Binding of a cytokine to its receptor results in the activation of Janus protein tyrosine kinases (JAKs) associated with these receptors. This in turn, phosphorylates STAT, resulting in translocation to the nucleus and transcriptional activation of STAT responsive genes. Phosphorylation on a specific tyrosine residue on the STATs results in their activation, resulting in the formation of homodimers and/or heterodimers of STAT which bind to specific gene promoter sequences. Events mediated by cytokines through STAT activation include cell proliferation and differentiation and prevention of apoptosis.

The specificity of STAT activation is due to specific cytokines, i.e. each STAT is responsive to a small number of specific cytokines. Other non-cytokine signaling molecules, such as growth factors, have also been found to activate STATs. Binding of these factors to a cell surface receptor associated with protein tyrosine kinase also results in phosphorylation of STAT.

STAT3 [also acute phase response factor (APRF), STAT3-alpha], in particular, has been found to be responsive to interleukin-6 (IL-6) as well as epidermal growth factor (EGF) (Darnell, Jr., J.E., et al., Science, 1994, 264, 1415-1421). In addition, STAT3 has been found to have an important role in signal transduction by interferons (Yang, C.-H., et al., Proc. Natl. Acad. Sci. USA, 1998, 95, 5568-5572). Evidence exists suggesting that STAT3 may be regulated by the MAPK pathway. ERK2 induces serine phosphorylation and also associates with STAT3 (Jain, N., et al. , Oncogene, 1998, 17, 3157-3167).

STAT3 is expressed in most cell types (Zhong, Z., et al., Proc. Natl. Acad. Sci . USA, 1994, 91, 4806-4810). It induces the expression of genes involved in response to tissue injury and inflammation. STAT3 has also been shown to prevent apoptosis through the expression of bcl-2 (Fukada, T., et al., Immunity, 1996, 5, 449-460).

Aberrant expression of or constitutive expression of STAT3 is associated with a number of disease processes. STAT3 has been shown to be involved in cell transformation. It is constitutively activated in v-src-transformed cells (Yu, C.-L., et al., Science, 1995, 269, 81-83). Constitutively active STAT3 also induces STAT3 mediated gene expression and is required for cell transformation by src (Turkson, J., et al., Mol. Cell. Biol., 1998, 18, 2545-2552). STAT3 is also constitutively active in human T cell lymphotropic virus I (HTLV-I) transformed cells (Migone, T.-S. et al., Science, 1995, 269, 79-83).

Constitutive activation and/or overexpression of STAT3 appears to be involved in several forms of cancer, including myeloma, breast carcinomas, prostate cancer, brain tumors, head and neck carcinomas, melanoma, leukemias and lymphomas, particularly chronic myelogenous leukemia and multiple myeloma (Niu et al., Cancer Res., 1999, 59, 5059-5063). Breast cancer cell lines that overexpress EGFR constitutively express phosphorylated STAT3 (Sartor, C.I., et al., Cancer Res., 1997, 57, 978-987; Garcia, R., et al., Cell Growth and Differentiation, 1997, 8, 1267-1276). Activated STAT3 levels were also found to be elevated in low grade glioblastomas and medulloblastomas (Cattaneo, E., et al., Anticancer Res., 1998, 18, 2381-2387).

Cells derived from both rat and human prostate cancers have been shown to have constitutively activated STAT3, with STAT3 activation being correlated with malignant potential. Expression of a dominant-negative STAT3 was found to significantly inhibit the growth of human prostate cells (Ni et al., Cancer Res., 2000, 60, 1225-1228).

STAT3 has also been found to be constitutively activated in some acute leukemias (Gouilleux-Gruart, V., et al., Leuk. Lymphoma, 1997, 28, 83-88) and T cell lymphoma (Yu, C.-L., et al., J. Immunol., 1997, 159, 5206-5210). Interestingly, STAT3 has been found to be constitutively phosphorylated on a serine residue in chronic lymphocytic leukemia (Frank, D. A., et al., J. Clin. Invest., 1997, 100, 3140-3148). In addition, antisense oligonucleotides to STAT3 have been shown to promote apoptosis in non-small cell lung cancer cells (Song et al., Oncogene 22:4150, 2003) and prostate cancer cells (Mora et al., Cancer Res. 62:6659, 2002).

STAT3 has been found to be constitutively active in myeloma tumor cells, both in culture and in bone marrow

mononuclear cells from patients with multiple myeloma. These cells are resistant to Fas-mediated apoptosis and express high levels of Bcl-xL. STAT3 signaling was shown to be essential for survival of myeloma tumor cells by conferring resistance to apoptosis (Catlett-Falcone, R., et al., Immunity, 1999, 10, 105-115). Constitutively activated STAT3 is also associated with chronic myelogenous leukemia. Thus STAT3 is a potential target for therapeutic intervention in multiple myeloma and other cancers with activated STAT3 signaling pathways. Options for treatment of patients with multiple myeloma are not completely satisfactory. Velcade was approved in May 2003 with a 188 evaluable patient pivotal trial based on tumor shrinkage, not survival. Only 28% of patients showed a partial response. The data are currently under FDA review. There is a distinct medical need for novel therapies for chemoresistant myeloma.

A gene therapy approach in a syngeneic mouse tumor model system has been used to inhibit activated STAT3 in vivo using a dominant-negative STAT3 variant. This inhibition of activated STAT3 signaling was found to suppress B16 melanoma tumor growth and induce apoptosis of B16 tumor cells in vivo. Interestingly, the number of apoptotic cells (95%) exceeded the number of transfected cells, indicating a possible antitumor "bystander effect" in which an inflammatory response (tumor infiltration by acute and chronic inflammatory cells) may participate in killing of residual tumor cells (Niu et al., Cancer Res., 1999, 59, 5059-5063).

STAT3 may also play a role in inflammatory diseases including rheumatoid arthritis. Activated STAT3 has been found in the synovial fluid of rheumatoid arthritis patients (Sengupta, T.K., et al., J. Exp. Med., 1995, 181, 1015-1025) and cells from inflamed joints (Wang, F., et al., J. Exp. Med. , 1995, 182, 1825-1831).

Alternative splicing results in the formation of STAT3 , which is characterized by the absence of 55 amino acid residues found at the C-terminus of STAT3. This domain contains the transactivation domain, and thus, STAT3 may act as a negative regulator of STAT3 function.

STAT3 has been found to be more stable and have greater DNA-binding activity than STAT3, whereas STAT3 is more transcriptionally active. STAT3 , as well as STAT3, forms homodimers, and the two can also bind together as a heterodimer (Caldenhoven, E., et al., J. Biol. Chem., 1996, 271, 13221-13227).

There are currently several approaches for inhibiting STAT3 expression. US Patent Nos. 5,719,042 and 5,844,082, both issued jointly to Akira, S. and Kishimoto, T. teach the use of inhibitors of APRF, including antibodies, antisense nucleic acids and ribozymes for the treatment of IL-6 associated diseases, such as inflammatory diseases, leukemia, and cancer. Schreiber, R.D., et al., in US Patent Nos. 5,731,155; 5,582,999; and 5,463,023, disclose methods of inhibiting transcriptional activation using short peptides that bind p91. Darnell, J.E., et al., in US Patent No. 5,716,622, disclose peptides containing the DNA binding domain of STATs, chimeric proteins containing the DNA binding domain, and antibodies to STATs for inhibiting STAT transcriptional activation.

The use of an antisense oligonucleotide targeted to the translation start region of human STAT3 has been disclosed (Grandis, J. R., et al., J. Clin. Invest., 1998, 102, 1385-1392). In this report, a phosphorothioate oligodeoxynucleotide complementary to the translation start region of STAT3 inhibited THF-β stimulated cell growth mediated by the epidermal growth factor receptor (EGFR), suggesting that STAT3 is a component of an EGFR signaling pathway.

WO 00/61102 discloses compositions, compounds and methods for inhibiting STAT3 expression. The compositions comprise antisense oligonucleotides targeted to nucleic acids encoding STAT3.

There remains an unmet need for therapeutic compositions and methods targeting expression of STAT3, and disease processes associated therewith.

## SUMMARY OF THE INVENTION

The invention provides an antisense oligonucleotide 12 to 50 nucleobases in length, which specifically hybridises with a nucleic acid molecule encoding STAT3 and is capable of inhibiting STAT3 expression, and wherein the oligonucleotide is targeted to nucleotides 2983 to 3127 of SEQ ID NO:154.

The invention also provides a pharmaceutical composition comprising an antisense oligonucleotide according to the invention and a pharmaceutically acceptable carrier or diluent.

The invention also provides an antisense oligonucleotide according to the invention for use in therapy or prophylaxis.

## SUMMARY OF THE DISCLOSURE

The present disclosure provides oligonucleotides which are targeted to nucleic acids encoding STAT3 and are capable of modulating STAT3 expression. The present disclosure also provides chimeric oligonucleotides targeted to nucleic acids encoding human STAT3.

The present disclosure also comprises methods of modulating the expression of human STAT3, in cell and tissues, using the oligonucleotides of the disclosure.

The present disclosure further comprises methods for diagnosing and treating inflammatory diseases, particularly rheumatoid arthritis, and cancers, including those of the breast, prostate, head and neck, and brain, myelomas,

melanomas, leukemias, and lymphomas. The oligonucleotides and methods of the present disclosure are believed to be useful therapeutically and prophylactically. The oligonucleotides of the present disclosure are further believed to be useful as clinical research and diagnostic tools, as well as for detecting and determining the role of STAT3 in various cell functions and physiological processes and conditions and for diagnosing conditions associated with expression of STAT3.

**DETAILED DESCRIPTION**

STAT3 plays an important role in cytokine signal transduction. Overexpression and/or constitutive activation of STAT3 is associated with a number of inflammatory diseases and cancers. As such, this DNA-binding protein represents an attractive target for treatment of such diseases. In particular, modulation of the expression of STAT3 may be useful for the treatment of diseases such as rheumatoid arthritis, breast cancer, prostate cancer, brain cancer, head and neck cancer, myelomas, melanomas, leukemias and lymphomas.

The present invention employs antisense compounds, particularly oligonucleotides, for use in modulating the function of nucleic acid molecules encoding STAT3, ultimately modulating the amount of STAT3 produced. This is accomplished by providing oligonucleotides which specifically hybridize with nucleic acids, preferably mRNA, encoding STAT3.

This relationship between an antisense compound such as an oligonucleotide and its reverse complementary nucleic acid target, to which it hybridizes, is commonly referred to as "antisense". "Targeting" an oligonucleotide to a chosen nucleic acid target, in the context of this invention, is a multistep process. The process usually begins with identifying a nucleic acid sequence whose function is to be modulated. This may be, as examples, a cellular gene (or mRNA made from the gene) whose expression is associated with a particular disease state, or a foreign nucleic acid from an infectious agent.

In the present invention, the targets are nucleic acids encoding STAT3; in other words, a gene encoding STAT3, or mRNA expressed from the STAT3 gene. mRNA which encodes STAT3 is presently the preferred target. The targeting process also includes determination of a site or sites within the nucleic acid sequence for the antisense interaction to occur such that modulation of gene expression will result.

In accordance with this invention, persons of ordinary skill in the art will understand that messenger RNA includes not only the information to encode a protein using the three letter genetic code, but also associated ribonucleotides which form a region known to such persons as the 5'-untranslated region, the 3'-untranslated region, the 5' cap region and intron/exon junction ribonucleotides. Thus, oligonucleotides may be formulated in accordance with this invention which are targeted wholly or in part to these associated ribonucleotides as well as to the informational ribonucleotides. The oligonucleotide may therefore be specifically hybridizable with a transcription initiation site region, a translation initiation codon region, a 5' cap region, an intron/exon junction, coding sequences, a translation termination codon region or sequences in the 5'- or 3'-untranslated region. Since, as is known in the art, the translation initiation codon is typically 5'-AUG (in transcribed mRNA molecules; 5'-ATG in the corresponding DNA molecule), the translation initiation codon is also referred to as the "AUG codon," the "start codon" or the "AUG start codon." A minority of genes have a translation initiation codon having the RNA sequence 5'-GUG, 5'-UUG or 5'-CUG, and 5'-AUA, 5'-ACG and 5'-CUG have been shown to function in *vivo.* Thus, the terms "translation initiation codon" and "start codon" can encompass many codon sequences, even though the initiator amino acid in each instance is typically methionine (in eukaryotes) or formylmethionine (prokaryotes). It is also known in the art that eukaryotic and prokaryotic genes may have two or more alternative start codons, any one of which may be preferentially utilized for translation initiation in a particular cell type or tissue, or under a particular set of conditions. In the context of the invention, "start codon" and "translation initiation codon" refer to the codon or codons that are used *in vivo* to initiate translation of an mRNA molecule transcribed from a gene encoding STAT3, regardless of the sequence(s) of such codons. It is also known in the art that a translation termination codon (or "stop codon") of a gene may have one of three sequences, i.e., 5'-UAA, 5'-UAG and 5'-UGA (the corresponding DNA sequences are 5'-TAA, 5'-TAG and 5'-TGA, respectively). The terms "start codon region," "AUG region" and "translation initiation codon region" refer to a portion of such an mRNA or gene that encompasses from about 25 to about 50 contiguous nucleotides in either direction (i.e., 5' or 3') from a translation initiation codon. This region is a preferred target region. Similarly, the terms "stop codon region" and "translation termination codon region" refer to a portion of such an mRNA or gene that encompasses from about 25 to about 50 contiguous nucleotides in either direction (i.e., 5' or 3') from a translation termination codon. This region is a preferred target region. The open reading frame (ORF) or "coding region," which is known in the art to refer to the region between the translation initiation codon and the translation termination codon, is also a region which may be targeted effectively. Other preferred target regions include the 5' untranslated region (5'UTR), known in the art to refer to the portion of an mRNA in the 5' direction from the translation initiation codon, and thus including nucleotides between the 5' cap site and the translation initiation codon of an mRNA or corresponding nucleotides on the gene and the 3' untranslated region (3'UTR), known in the art to refer to the portion of an mRNA in the 3' direction from the translation termination codon, and thus including nucleotides

between the translation termination codon and 3' end of an mRNA or corresponding nucleotides on the gene. The 5' cap of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap. The 5' cap region may also be a preferred target region.

Although some eukaryotic mRNA transcripts are directly translated, many contain one or more regions, known as "introns", which are excised from a pre-mRNA transcript to yield one or more mature mRNA. The remaining (and therefore translated) regions are known as "exons" and are spliced together to form a continuous mRNA sequence. mRNA splice sites, i.e., exon-exon or intron-exon junctions, may also be preferred target regions, and are particularly useful in situations where aberrant splicing is implicated in disease, or where an overproduction of a particular mRNA splice product is implicated in disease. Aberrant fusion junctions due to rearrangements or deletions are also preferred targets. Targeting particular exons in alternatively spliced mRNAs may also be preferred. It has also been found that introns can also be effective, and therefore preferred, target regions for antisense compounds targeted, for example, to DNA or pre-mRNA.

Once the target site or sites have been identified, oligonucleotides are chosen which are sufficiently complementary to the target, i.e., hybridize sufficiently well and with sufficient specificity, to give the desired modulation.

"Hybridization", in the context of this invention, means hydrogen bonding, also known as Watson-Crick base pairing, between complementary bases, usually on opposite nucleic acid strands or two regions of a nucleic acid strand. Guanine and cytosine are examples of complementary bases which are known to form three hydrogen bonds between them. Adenine and thymine are examples of complementary bases which form two hydrogen bonds between them.

"Specifically hybridizable" and "complementary" are terms which are used to indicate a sufficient degree of complementarity such that stable and specific binding occurs between the DNA or RNA target and the oligonucleotide.

It is understood that an oligonucleotide need not be 100% complementary to its target nucleic acid sequence to be specifically hybridizable. An oligonucleotide is specifically hybridizable when binding of the oligonucleotide to the target interferes with the normal role of the target molecule to cause a loss of function or activity, and there is a sufficient degree of complementarity to avoid non-specific binding of the oligonucleotide to non-target sequences under conditions in which specific binding is desired, i.e., under physiological conditions in the case of *in vivo* assays or therapeutic treatment or, in the case of *in vitro* assays, under conditions in which the assays are conducted.

Hybridization of antisense oligonucleotides with mRNA interferes with one or more of the normal functions of mRNA. The functions of mRNA to be interfered with include any vital functions such as, for example, translocation of the RNA to the site of protein translation, translation of protein from the RNA, splicing of the RNA to yield one or more mRNA species, and catalytic activity which may be engaged in by the RNA. Binding of specific protein(s) to the RNA may also be interfered with by antisense oligonucleotide hybridization to the RNA.

The locations on the target nucleic acid to which the preferred antisense compounds hybridize are hereinbelow referred to as "target segments." As used herein the term "target segment" is defined as at least an 8-nucleobase portion of a target region to which an active antisense compound is targeted. While not wishing to be bound by theory, it is presently believed that these target segments represent portions of the target nucleic acid which are accessible for hybridization.

While the specific sequences of certain target segments are set forth herein, one of skill in the art will recognize that these serve to illustrate and describe particular embodiments within the scope of the present invention. Additional target segments may be identified by one having ordinary skill.

Target segments 8-80 nucleobases in length comprising a stretch of at least eight (8) consecutive nucleobases selected from within the illustrative preferred target segments are considered to be suitable for targeting as well.

Target segments can include DNA or RNA sequences that comprise at least the 8 consecutive nucleobases from the 5'-terminus of one of the illustrative target segments (the remaining nucleobases being a consecutive stretch of the same DNA or RNA beginning immediately upstream of the 5'-terminus of the target segment and continuing until the DNA or RNA contains about 8 to about 80 nucleobases). Similarly target segments are represented by DNA or RNA sequences that comprise at least the 8 consecutive nucleobases from the 3'-terminus of one of the illustrative preferred target segments (the remaining nucleobases being a consecutive stretch of the same DNA or RNA beginning immediately downstream of the 3'-terminus of the target segment and continuing until the DNA or RNA contains about 8 to about 80 nucleobases). It is also understood that antisense target segments may be represented by DNA or RNA sequences that comprise at least 8 consecutive nucleobases from an internal portion of the sequence of an illustrative preferred target segment, and may extend in either or both directions until the oligonucleotide contains about 8 to about 80 nucleobases. One having skill in the art armed with the target segments illustrated herein will be able, without undue experimentation, to identify further preferred target segments.

Once one or more target regions, segments or sites have been identified, antisense compounds are chosen which are sufficiently complementary to the target, i.e., hybridize sufficiently well and with sufficient specificity, to give the desired effect.

The oligomeric antisense compounds can also be targeted to regions of a target nucleobase sequence, such

as those disclosed herein. All regions of a nucleobase sequence to which an oligomeric antisense compound can be targeted, wherein the regions are greater than or equal to 8 and less than or equal to 80 nucleobases, are described as follows:

Let R(n, n+m-1) be a region from a target nucleobase sequence, where "n" is the 5'-most nucleobase position of the region, where "n+m-1" is the 3'-most nucleobase position of the region and where "m" is the length of the region. A set "S(m)", of regions of length "m" is defined as the regions where n ranges from 1 to L-m+1, where L is the length of the target nucleobase sequence and L>m. A set, "A", of all regions can be constructed as a union of the sets of regions for each length from where m is greater than or equal to 8 and is less than or equal to 80.

This set of regions can be represented using the following mathematical notation:

$$A = \bigcup_{m} S(m) \text{ where } m \in N | 8 \leq m \leq 80$$

and

$$S(m) = \{R_{n,n+m-1} | n \in \{1,2,3,\ldots, L-m+1\}\}$$

where the mathematical operator | indicates "such that",
where the mathematical operator ∈ indicates "a member of a set" (e.g. y ∈ Z indicates that element y is a member of set Z),
where x is a variable,
where N indicates all natural numbers, defined as positive integers,
and where the mathematical operator U indicates "the union of sets".

For example, the set of regions for m equal to 8, 20 and 80 can be constructed in the following manner. The set of regions, each 8 nucleobases in length, S(m=8), in a target nucleobase sequence 100 nucleobases in length (L=100), beginning at position 1 (n=1) of the target nucleobase sequence, can be created using the following expression:

$$S(8) = \{R_{1,8} | n \in \{1,2,3,\ldots,93\}\}$$

and describes the set of regions comprising nucleobases 1-8, 2-9, 3-10, 4-11, 5-12, 6-13, 7-14, 8-15, 9-16, 10-17, 11-18, 12-19, 13-20, 14-21, 15-22, 16-23, 17-24, 18-25, 19-26, 20-27, 21-28, 22-29, 23-30, 24-31, 25-32, 26-33, 27-34, 28-35, 29-36, 30-37, 31-38, 32-39, 33-40, 34-41, 35-42, 36-43, 37-44, 38-45, 39-46, 40-47, 41-48, 42-49, 43-50, 44-51, 45-52, 46-53, 47-54, 48-55, 49-56, 50-57, 51-58, 52-59, 53-60, 54-61, 55-62, 56-63, 57-64, 58-65, 59-66, 60-67, 61-68, 62-69, 63-70, 64-71, 65-72, 66-73, 67-74, 68-75, 69-76, 70-77, 71-78, 72-79, 73-80, 74-81, 75-82, 76-83, 77-84, 78-85, 79-86, 80-87, 81-88, 82-89, 83-90, 84-91, 85-92, 86-93, 87-94, 88-95, 89-96, 90-97, 91-98, 92-99, 93-100.

An additional set for regions 20 nucleobases in length, in a target sequence 100 nucleobases in length, beginning at position 1 of the target nucleobase sequence, can be described using the following expression:

$$S(20) = \{R_{1,20} | n \in \{1,2,3,\ldots,81\}\}$$

and describes the set of regions comprising nucleobases 1-20, 2-21, 3-22, 4-23, 5-24, 6-25, 7-26, 8-27, 9-28, 10-29, 11-30, 12-31, 13-32, 14-33, 15-34, 16-35, 17-36, 18-37, 19-38, 20-39, 21-40, 22-41, 23-42, 24-43, 25-44, 26-45, 27-46, 28-47, 29-48, 30-49, 31-50, 32-51, 33-52, 34-53, 35-54, 36-55, 37-56, 38-57, 39-58, 40-59, 41-60, 42-61, 43-62, 44-63, 45-64, 46-65, 47-66, 48-67, 49-68, 50-69, 51-70, 52-71, 53-72, 54-73, 55-74, 56-75, 57-76, 58-77, 59-78, 60-79, 61-80, 62-81, 63-82, 64-83, 65-84, 66-85, 67-86, 68-87, 69-88, 70-89, 71-90, 72-91, 73-92, 74-93, 75-94, 76-95, 77-96, 78-97, 79-98, 80-99, 81-100.

An additional set for regions 80 nucleobases in length, in a target sequence 100 nucleobases in length, beginning at position 1 of the target nucleobase sequence, can be described using the following expression:

$$S(80) = \{R_{1,80} | n \in \{1,2,3,...,21\}\}$$

and describes the set of regions comprising nucleobases 1-80, 2-81, 3-82, 4-83, 5-84, 6-85, 7-86, 8-87, 9-88, 10-89, 11-90, 12-91, 13-92, 14-93, 15-94, 16-95, 17-96, 18-97, 19-98, 20-99, 21-100.

Thus, in this example, *A* would include regions 1-8, 2-9, 3-10...93-100, 1-20, 2-21, 3-22...81-100, 1-80, 2-81, 3-82...21-100.

The union of these aforementioned example sets and other sets for lengths from 10 to 19 and 21 to 79 can be described using the mathematical expression

$$A = \bigcup_{m} S(m)$$

where U represents the union of the sets obtained by combining all members of all sets.

The mathematical expressions described herein defines all possible target regions in a target nucleobase sequence of any length L, where the region is of length m, and where m is greater than or equal to 8 and less than or equal to 80 nucleobases and, and where m is less than L, and where n is less than L-m+1.

The overall effect of interference with mRNA function is modulation of expression of STAT3. In the context of this invention "modulation" means either inhibition or stimulation; i.e., either a decrease or increase in expression. This modulation can be measured in ways which are routine in the art, for example by Northern blot assay of mRNA expression, or reverse transcriptase PCR, as taught in the examples of the instant application or by Western blot or ELISA assay of protein expression, or by an immunoprecipitation assay of protein expression. Effects on cell proliferation or tumor cell growth can also be measured, as taught in the examples of the instant application. Inhibition is presently preferred.

In addition to the well known antisense effects of oligonucleotides, it has also been found that oligonucleotide analogs having at least one phosphorothioate bond can induce stimulation of a local immune response. This is described in U.S. Patent 5,663,153. This immunostimulatory effect does not appear to be related to any antisense effect which these oligonucleotide analogs may or may not possess. These oligonucleotide analogs are useful as immunopotentiators, either alone or in combination with other therapeutic modalities, such as drugs, particularly antiinfective and anticancer drugs, and surgical procedures to increase efficacy. In addition, the antiinfective and anticancer effects already possessed by certain antisense oligonucleotide analogs are enhanced through such immune stimulation.

It has also been found that oligonucleotide analogs having at least one phosphorothioate bond can be used to induce stimulation of a systemic or humoral immune response. Thus, these oligonucleotides are also useful as immunopotentiators of an antibody response, either alone or in combination with other therapeutic modalities. This is described in U.S. Patent 5,663,153.

It is presently believed, therefore, that, in addition to the antisense effects of oligonucleotides targeted to STAT3, oligonucleotides containing at least one phosphorothioate backbone linkage may be useful in eliciting an immune response which may add to the antitumor "bystander effect" already observed with dominant negative inhibitors of STAT3 signaling (Niu et al., Cancer Res., 1999, 59, 5059-5063). This effect is believed to be related to tumor infiltration by acute and chronic inflammatory cells which may participate in killing of residual tumor cells. Thus the therapeutic effects of antisense oligonucleotides targeted to STAT3 may be potentiated by the immunostimulatory properties of the oligonucleotides themselves. Alternatively, oligonucleotides which may not be targeted to STAT3 but which contain at least one phosphorothioate backbone linkage may be used as adjuvants in combination with antisense or other inhibitors of STAT3.

The oligonucleotides of this invention can be used in diagnostics, therapeutics, prophylaxis, and as research reagents and in kits. Since the oligonucleotides of this invention hybridize to nucleic acids encoding STAT3, sandwich, colorimetric and other assays can easily be constructed to exploit this fact. Provision of means for detecting hybridization of oligonucleotide with the STAT3 gene or mRNA can routinely be accomplished. Such provision may include enzyme conjugation, radiolabelling or any other suitable detection systems. Kits for detecting the presence or absence of STAT3 may also be prepared.

The present invention is also suitable for diagnosing abnormal inflammatory states or certain cancers in tissue or other samples from patients suspected of having an inflammatory disease such as rheumatoid arthritis or cancers such as breast, brain, or head and neck cancer, melanomas, myelomas, leukemias and lymphomas.

A number of assays may be formulated employing the present invention, which assays will commonly comprise contacting a tissue sample with an oligonucleotide of the invention under conditions selected to permit detection and, usually, quantitation of such inhibition. In the context of this invention, to "contact" tissues or cells with an oligonucleotide or oligonucleotides means to add the oligonucleotide(s), usually in a liquid carrier, to a cell suspension or tissue sample, either *in vitro* or *ex vivo,* or to administer the oligonucleotide(s) to cells or tissues within an animal.

The oligonucleotides of this invention may also be used for research purposes. Thus, the specific hybridization exhibited by the oligonucleotides may be used for assays, purifications, cellular product preparations and in other methodologies which may be appreciated by persons of ordinary skill in the art.

In the context of this invention, the term "oligonucleotide" refers to an oligomer or polymer of ribonucleic acid or deoxyribonucleic acid. This term includes oligonucleotides composed of naturally-occurring nucleobases, sugars and covalent intersugar (backbone) linkages as well as oligonucleotides having non-naturally-occurring portions which function similarly. Such modified or substituted oligonucleotides are often preferred over native forms because of desirable properties such as, for example, enhanced cellular uptake, enhanced binding to target and increased stability in the presence of nucleases.

The antisense compounds in accordance with this disclosure preferably comprise from about 8 to about 80 nucleobases (i.e. from about 8 to about 80 linked nucleosides). One of ordinary skill in the art will appreciate that the disclosure embodies compounds of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleobases in length.

In one embodiment, the antisense compounds of the invention are 12 to 50 nucleobases in length. One having ordinary skill in the art will appreciate that this embodies compounds of 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleobases in length.

In one embodiment, the antisense compounds of the invention are 13 to 40 nucleobases in length. One having ordinary skill in the art will appreciate that this embodies compounds of 13, 14, 15, 16, 17, 18, 19, 20, 21, 22, 23, 24, 25, 26, 27, 28, 29, 30, 31, 32, 33, 34, 35, 36, 37, 38, 39 or 40 nucleobases in length.

In another embodiment, the antisense compounds of the invention are 15 to 30 nucleobases in length. One having ordinary skill in the art will appreciate that this embodies compounds of 15, 16, 17, 18, 19, 20, 21, 22, 23, 24, 25, 26, 27, 28, 29, or 30 nucleobases in length.

In one embodiment, the antisense compounds of the invention are 19 to 25 nucleobases in length. One having ordinary skill in the art will appreciate that this embodies compounds of 19, 20, 21, 22, 23, 24, or 25 nucleobases in length.

In another embodiment, the antisense compounds of the invention are 19 to 21 nucleobases in length. One having ordinary skill in the art will appreciate that this embodies compounds of 19, 20 or 21 nucleobases in length.

As used herein the term "oligomeric compound" is defined as a polymeric compound substantially comprising nucleic acid based monomer subunits. Oligomeric compounds include oligonucleotides and their analogs, mimics or mimetics.

While the preferred form of antisense compound is a single-stranded antisense oligonucleotide, in many species the introduction of double-stranded structures, such as double-stranded RNA (dsRNA) molecules, has been shown to induce potent and specific antisense-mediated reduction of the function of a gene or its associated gene products. This phenomenon occurs in both plants and animals and is believed to have an evolutionary connection to viral defense and transposon silencing.

The first evidence that dsRNA could lead to gene silencing in animals came in 1995 from work in the nematode, *Caenorhabditis elegans* (Guo and Kempheus, Cell, 1995, 81, 611-620). Montgomery et al. have shown that the primary interference effects of dsRNA are posttranscriptional (Montgomery et al., Proc. Natl. Acad. Sci. USA, 1998, 95, 15502-15507). The posttranscriptional antisense mechanism defined in *Caenorhabditis elegans* resulting from exposure to double-stranded RNA (dsRNA) has since been designated RNA interference (RNAi). This term has been generalized to mean antisense-mediated gene silencing involving the introduction of dsRNA leading to the sequence-specific reduction of endogenous targeted mRNA levels (Fire et al., Nature, 1998, 391, 806-811). Recently, it has been shown that it is, in fact, the single-stranded RNA oligomers of antisense polarity of the dsRNAs which are the potent inducers of RNAi (Tijsterman et al., Science, 2002, 295, 694-697). The use of these double-stranded RNA molecules (short interfering RNA or siRNA) for targeting and inhibiting the expression of STAT3 mRNA is also contemplated. These double stranded RNA molecules target regions similar to those targeted by antisense oligocleotides and have similar effects. These double stranded RNA molecules are generally 19-21 base pairs in length, but may range between 8 and 50 nucleobases. The production of siRNA molecules is described in a general sense in the examples provided below, but it will be appreciated that any desired siRNA targeted to STAT3 may be synthesized by conventional oligonucleotide synthesis techniques. Once the sequence of the antisense strand is known, the complementary sense strand is synthesized. The sense and antisense strands are then combined to form the siRNA.

**Oligomer and Monomer Modifications**

As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5'

hydroxyl moiety of the sugar. In forming oligonucleotides, the phosphate groups covalently link adjacent nucleosides to one another to form a linear polymeric compound. In turn, the respective ends of this linear polymeric compound can be further joined to form a circular compound, however, linear compounds are generally preferred. In addition, linear compounds may have internal nucleobase complementarity and may therefore fold in a manner as to produce a fully or partially double-stranded compound. Within oligonucleotides, the phosphate groups are commonly referred to as forming the internucleoside linkage or in conjunction with the sugar ring the backbone of the oligonucleotide. The normal internucleoside linkage that makes up the backbone of RNA and DNA is a 3' to 5' phosphodiester linkage.

Modified Internucleoside Linkages

Specific examples of preferred antisense oligomeric compounds useful in this invention include oligonucleotides containing modified e.g. non-naturally occurring internucleoside linkages. As defined in this specification, oligonucleotides having modified internucleoside linkages include internucleoside linkages that retain a phosphorus atom and internucleoside linkages that do not have a phosphorus atom. For the purposes of this specification, and as sometimes referenced in the art, modified oligonucleotides that do not have a phosphorus atom in their internucleoside backbone can also be considered to be oligonucleosides.

In the *C. elegans* system, modification of the internucleotide linkage (phosphorothioate) did not significantly interfere with RNAi activity. Based on this observation, it is suggested that certain preferred oligomeric compounds of the invention can also have one or more modified internucleoside linkages. A preferred phosphorus containing modified internucleoside linkage is the phosphorothioate internucleoside linkage.

Preferred modified oligonucleotide backbones containing a phosphorus atom therein include, for example, phosphorothioates, chiral phosphorothioates, phosphoro-dithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphorainidates, thionophosphoramidates, thionoalkylphosphonates, thionoalkylphosphotriesters, selenophosphates and borano-phosphates having normal 3'-5' linkages, 2'-5' linked analogs of these, and those having inverted polarity wherein one or more internucleotide linkages is a 3' to 3', 5' to 5' or 2' to 2' linkage. Preferred oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included.

Representative United States patents that teach the preparation of the above phosphorus-containing linkages include, but are not limited to, U.S.: 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,194,599; 5,565,555; 5,527,899; 5,721,218; 5,672,697 and 5,625,050.

In more preferred embodiments of the invention, oligomeric compounds have one or more phosphorothioate and/or heteroatom internucleoside linkages, in particular $-CH_2-NH-O-CH_2-$, $-CH_2-N(CH_3)-O-CH_2-$ [known as a methylene (methylimino) or MMI backbone], $-CH_2-O-N(CH_3)-CH_2-$, $-CH_2-N(CH_3)-N(CH_3)-CH_2-$ and $-O-N(CH_3)-CH_2-CH_2-$ [wherein the native phosphodiester internucleotide linkage is represented as $-O-P(=O)(OH)-O-CH_2-$]. The MMI type internucleoside linkages are disclosed in the above referenced U.S. patent 5,489,677. Preferred amide internucleoside linkages are disclosed in the above referenced U.S. patent 5,602,240.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and $CH_2$ component parts.

Representative United States patents that teach the preparation of the above oligonucleosides include, but are not limited to, U.S.: 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439.

**Oligonucleotide Mimetics**

Another group of compounds of the present invention includes oligonucleotide mimetics. The term mimetic as it is applied to oligonucleotides is intended to include oligomeric compounds wherein only the furanose ring or both the

furanose ring and the internucleotide linkage are replaced with novel groups, replacement of only the furanose ring is also referred to in the art as being a sugar surrogate. The heterocyclic base moiety or a modified heterocyclic base moiety is maintained for hybridization with an appropriate target nucleic acid. One such oligomeric compound, an oligonucleotide mimetic that has been shown to have excellent hybridization properties, is referred to as a peptide nucleic acid (PNA). In PNA oligomeric compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA oligomeric compounds include, but are not limited to, U.S.: 5,539,082; 5,714,331; and 5,719,262.

Further teaching of PNA oligomeric compounds can be found in Nielsen et al., Science, 1991, 254, 1497-1500.

One oligonucleotide mimetic that has been reported to have excellent hybridization properties is peptide nucleic acids (PNA). The backbone in PNA compounds is two or more linked aminoethylglycine units which gives PNA an amide containing backbone. The heterocyclic base moieties are bound directly or indirectly to aza nitrogen atoms of the amide portion of the backbone. Representative United States patents that teach the preparation of PNA compounds include, but are not limited to, U.S.: 5,539,082; 5,714,331; and 5,719,262. Further teaching of PNA compounds can be found in Nielsen et al., Science, 1991, 254, 1497-1500.

PNA has been modified to incorporate numerous modifications since the basic PNA structure was first prepared. The basic structure is shown below:

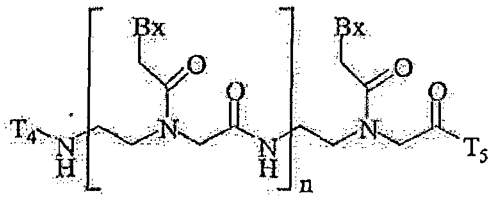

wherein

Bx is a heterocyclic base moiety;

$T_4$ is hydrogen, an amino protecting group, $-C(O)R_5$, substituted or unsubstituted $C_1$-$C_{10}$ alkyl, substituted or unsubstituted $C_2$-$C_{10}$ alkenyl, substituted or unsubstituted $C_2$-$C_{10}$ alkynyl, alkylsulfonyl, arylsulfonyl, a chemical functional group, a reporter group, a conjugate group, a D or L $\alpha$-amino acid linked via the $\alpha$-carboxyl group or optionally through the ($\omega$-carboxyl group when the amino acid is aspartic acid or glutamic acid or a peptide derived from D, L or mixed D and L amino acids linked through a carboxyl group, wherein the s-ubstituent groups are selected from hydroxyl, amino, alkoxy, carboxy, benzyl, phenyl, nitro, thiol, thioalkoxy, halogen, alkyl, aryl, alkenyl and alkynyl;

$T_5$ is -OH, $-N(Z_1)Z_2$, $R_5$, D or L $\alpha$-amino acid linked via the $\alpha$-amino group or optionally through the $\omega$-amino group when the amino acid is lysine or ornithine or a peptide derived from D, L or mixed D and L amino acids linked through an amino group, a chemical functional group, a reporter group or a conjugate group;

$Z_1$ is hydrogen, $C_1$-$C_6$ alkyl, or an amino protecting group;

$Z_2$ is hydrogen, $C_1$-$C_6$ alkyl, an amino protecting group, $-C(=O)-(CH_2)_n-J-Z_3$, a D or L $\alpha$-amino acid linked via the $\alpha$-carboxyl group or optionally through the $\omega$-carboxyl group when the amino acid is aspartic acid or glutamic acid or a peptide derived from D, L or mixed D and L amino acids linked through a carboxyl group;

$Z_3$ is hydrogen, an amino protecting group, $-C_1$-$C_6$ alkyl, $-C(=O)-CH_3$, benzyl, benzoyl, or - $(CH_2)_n-N(H)\ Z_1$;

each J is O, S or NH;

$R_5$ is a carbonyl protecting group; and

n is from 2 to about 50.

Another class of oligonucleotide mimetic that has been studied is based on linked morpholino units (morpholino nucleic acid) having heterocyclic bases attached to the morpholino ring. A number of linking groups have been reported that link the morpholino monomeric units in a morpholino nucleic acid. A preferred class of linking groups has been selected to give a non-ionic oligomeric compound. The non-ionic morpholino-based oligomeric compounds are less likely to have undesired interactions with cellular proteins. Morpholino-based oligomeric compounds are non-ionic mimics of oligonucleotides which are less likely to form undesired interactions with cellular proteins (Dwaine A. Braasch and David R. Corey, Biochemistry, 2002, 41(14), 4503-4510). Morpholino-based oligomeric compounds are disclosed in United States Patent 5,034,506, issued July 23, 1991. The morpholino class of oligomeric compounds has been prepared having a variety of different linking groups joining the monomeric subunits.

Morpholino nucleic acids have been prepared having a variety of different linking groups ($L_2$) joining the monomeric subunits. The basic formula is shown below:

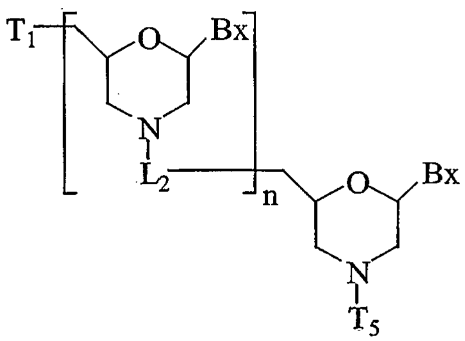

wherein

$T_1$ is hydroxyl or a protected hydroxyl;
$T_5$ is hydrogen or a phosphate or phosphate derivative;
$L_2$ is a linking group; and
n is from 2 to about 50.

A further class of oligonucleotide mimetic is referred to as cyclohexenyl nucleic acids (CeNA). The furanose ring normally present in an DNA/RNA molecule is replaced with a cyclohenyl ring. CeNA DMT protected phosphoramidite monomers have been prepared and used for oligomeric compound synthesis following classical phosphoramidite chemistry. Fully modified CeNA oligomeric compounds and oligonucleotides having specific positions modified with CeNA have been prepared and studied (see Wang et al., J. Am. Chem. Soc., 2000, 122, 8595-8602). In general the incorporation of CeNA monomers into a DNA chain increases its stability of a DNA/RNA hybrid. CeNA oligoadenylates formed complexes with RNA and DNA complements with similar stability to the native complexes. The study of incorporating CeNA structures into natural nucleic acid structures was shown by NMR and circular dichroism to proceed with easy conformational adaptation. Furthermore the incorporation of CeNA into a sequence targeting RNA was stable to serum and able to activate E. Coli RNase resulting in cleavage of the target RNA strand.

The general formula of CeNA is shown below:

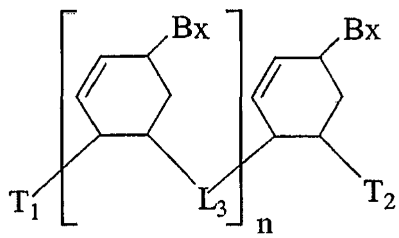

wherein

each Bx is a heterocyclic base moiety;
$T_1$ is hydroxyl or a protected hydroxyl; and
T2 is hydroxyl or a protected hydroxyl.

Another class of oligonucleotide mimetic (anhydrohexitol nucleic acid) can be prepared from one or more anhydrohexitol nucleosides (see, Wouters and Herdewijn, Bioorg. Med. Chem. Lett., 1999, 9, 1563-1566) and would have the general formula:

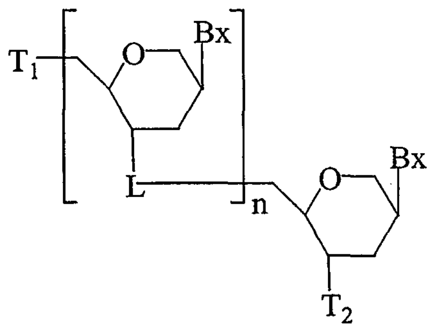

A further preferred modification includes Locked Nucleic Acids (LNAs) in which the 2'-hydroxyl group is linked to the 4' carbon atom of the sugar ring thereby forming a 2'-C,4'-C-oxymethylene linkage thereby forming a bicyclic sugar moiety. The linkage is preferably a methylene $(-CH_2-)_n$ group bridging the 2' oxygen atom and the 4' carbon atom wherein n is 1 or 2 (Singh et al., Chem. Commun., 1998, 4, 455-456). LNA and LNA analogs display very high duplex thermal stabilities with complementary DNA and RNA (Tm = +3 to +10 C), stability towards 3'-exonucleolytic degradation and good solubility properties. The basic structure of LNA showing the bicyclic ring system is shown below:

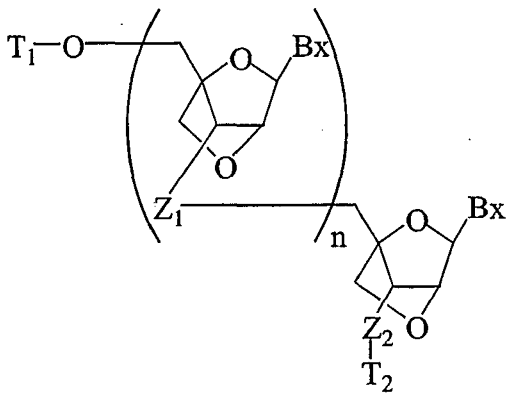

The conformations of LNAs determined by 2D NMR spectroscopy have shown that the locked orientation of the LNA nucleotides, both in single-stranded LNA and in duplexes, constrains the phosphate backbone in such a way as to introduce a higher population of the N-type conformation (Petersen et al., J. Mol. Recognit., 2000, 13, 44-53). These conformations are associated with improved stacking of the nucleobases (Wengel et al., Nucleosides Nucleotides, 1999, 18, 1365-1370).

LNA has been shown to form exceedingly stable LNA:LNA duplexes (Koshkin et al., J. Am. Chem. Soc., 1998, 120, 13252-13253). LNA:LNA hybridization was shown to be the most thermally stable nucleic acid type duplex system, and the RNA-mimicking character of LNA was established at the duplex level. Introduction of 3 LNA monomers (T or A) significantly increased melting points (Tm = +15/+11) toward DNA complements. The universality of LNA-mediated hybridization has been stressed by the formation of exceedingly stable LNA:LNA duplexes. The RNA-mimicking of LNA was reflected with regard to the N-type conformational restriction of the monomers and to the secondary structure of the LNA:RNA duplex.

LNAs also form duplexes with complementary DNA, RNA or LNA with high thermal affinities. Circular dichroism (CD) spectra show that duplexes involving fully modified LNA (esp. LNA:RNA) structurally resemble an A-form RNA:RNA duplex. Nuclear magnetic resonance (NMR) examination of an LNA:DNA duplex confirmed the 3'-endo conformation of an LNA monomer. Recognition of double-stranded DNA has also been demonstrated suggesting strand invasion by LNA. Studies of mismatched sequences show that LNAs obey the Watson-Crick base pairing rules with generally improved selectivity compared to the corresponding unmodified reference strands.

Novel types of LNA-oligomeric compounds, as well as the LNAs, are useful in a wide range of diagnostic and therapeutic applications. Among these are antisense applications, PCR applications, strand-displacement oligomers, substrates for nucleic acid polymerases and generally as nucleotide based drugs. Potent and nontoxic antisense oligonucleotides containing LNAs have been described (Wahlestedt et al., Proc. Natl. Acad. Sci. U. S. A., 2000, 97, 5633-5638.) The authors have demonstrated that LNAs confer several desired properties to antisense agents. LNA/DNA copolymers were not degraded readily in blood serum and cell extracts. LNA/DNA copolymers exhibited potent antisense activity in assay systems as disparate as G-protein-coupled receptor signaling in living rat brain and detection of reporter genes

in Escherichia coli. LIPOFECTIN™ -mediated efficient delivery of LNA into living human breast cancer cells has also been accomplished.

The synthesis and preparation of the LNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). LNAs and preparation thereof are also described in WO 98/39352 and WO 99/14226.

The first analogs of LNA, phosphorothioate-LNA and 2'-thio-LNAs, have also been prepared (Kumar et al., Bioorg. Med. Chem. Lett., 1998, 8, 2219-2222). Preparation of locked nucleoside analogs containing oligodeoxyribonucleotide duplexes as substrates for nucleic acid polymerases has also been described (Wengel et al., PCT International Application WO 98-DK393 19980914). Furthermore, synthesis of 2'-amino-LNA, a novel conformationally restricted high-affinity oligonucleotide analog with a handle has been described in the art (Singh et al., J. Org. Chem., 1998, 63, 10035-10039). In addition, 2'-Amino- and 2'-methylamino-LNA's have been prepared and the thermal stability of their duplexes with complementary RNA and DNA strands has been previously reported.

Further oligonucleotide mimetics have been prepared to include bicyclic and tricyclic nucleoside analogs having the formulas (amidite monomers shown):

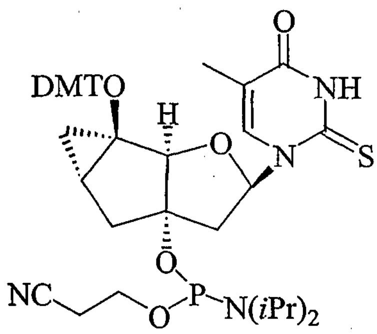
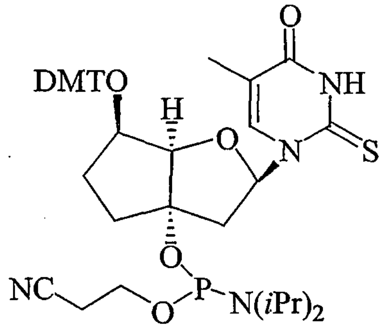

(see Steffens et al., Helv. Chim. Acta, 1997, 80, 2426-2439; Steffens et al., J. Am. Chem. Soc., 1999, 121, 3249-3255; and Renneberg et al., J. Am. Chem. Soc., 2002, 124, 5993-6002). These modified nucleoside analogs have been oligomerized using the phosphoramidite approach and the resulting oligomeric compounds containing tricyclic nucleoside analogs have shown increased thermal stabilities (Tm's) when hybridized to DNA, RNA and itself. Oligomeric compounds containing bicyclic nucleoside analogs have shown thermal stabilities approaching that of DNA duplexes.

Another class of oligonucleotide mimetic is referred to as phosphonomonoester nucleic acids incorporate a phosphorus group in a backbone the backbone. This class of olignucleotide mimetic is reported to have useful physical and biological and pharmacological properties in the areas of inhibiting gene expression (antisense oligonucleotides, ribozymes, sense oligonucleotides and triplex-forming oligonucleotides), as probes for the detection of nucleic acids and as auxiliaries for use in molecular biology.

The general formula (for definitions of Markush variables see: United States Patents 5,874,553 and 6,127,346) is shown below.

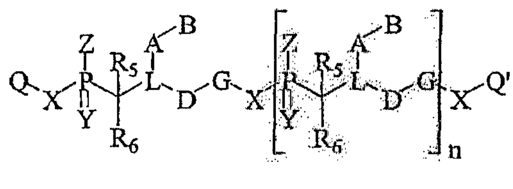

Another oligonucleotide mimetic has been reported wherein the furanosyl ring has been replaced by a cyclobutyl moiety.

Modified sugars

Oligomeric compounds of the invention may also contain one or more substituted sugar moieties. Preferred oligomeric compounds comprise a sugar substituent group selected from: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted $C_1$ to $C_{10}$ alkyl or $C_2$ to $C_{10}$ alkenyl and alkynyl. Particularly preferred are $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3]_2$, where n and m are from 1 to about 10. Other preferred oligonucleotides comprise a sugar substituent group selected from: $C_1$ to $C_{10}$ lower alkyl, substituted lower alkyl, alkenyl,

alkynyl, alkaryl, aralkyl, O-alkaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O-$CH_2CH_2OCH_3$, also known as 2'-O-(2-methoxyethyl) or 2'-MOE) (Martin et al., Helv. Chim. Acta, 1995, 78, 486-504) i.e., an alkoxyalkoxy group. A further preferred modification includes 2'-dimethylaminooxyethoxy, i.e., a $O(CH_2)_2ON(CH_3)_2$ group, also known as 2'-DMAOE, as described in examples hereinbelow, and 2'-dimethylamino-ethoxyethoxy (also known in the art as 2'-O-dimethyl-amino-ethoxy-ethyl or 2'-DMAEOE), i.e., 2'-O-$CH_2$-O-$CH_2$-$N(CH_3)_2$.

Other preferred sugar substituent groups include methoxy (-O-$CH_3$), aminopropoxy (-$OCH_2CH_2CH_2NH_2$), allyl (-$CH_2$-CH=$CH_2$, -O-allyl (-O-$CH_2$-CH=$CH_2$) and fluoro (F). 2'-Sugar substituent groups may be in the arabino (up) position or ribo (down) position. A preferred 2'-arabino modification is 2'-F. Similar modifications may also be made at other positions on the oligomeric compound, particularly the 3' position of the sugar on the 3' terminal nucleoside or in 2'-5' linked oligonucleotides and the 5' position of 5' terminal nucleotide. Oligomeric compounds may also have sugar mimetics such as cyclobutyl moieties in place of the pentofuranosyl sugar. Representative United States patents that teach the preparation of such modified sugar structures include, but are not limited to, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920.

Further representative sugar substituent groups include groups of formula $I_a$ or $II_a$:

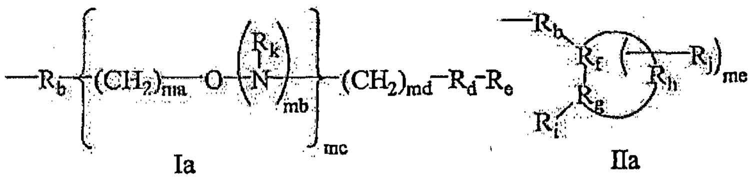

Ia IIa

wherein:

$R_b$ is O, S or NH;

$R_d$ is a single bond, O, S or C(=O);

$R_e$ is $C_1$-$C_{10}$ alkyl, $N(R_k)$ $(R_m)$, N(Rk)$(R_n)$, N=$C(R_p)(R_q)$, N=$C(R_p)(R_r)$ or has formula $III_a$;

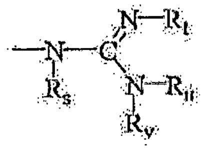

IIIa

$R_p$ and $R_q$ are each independently hydrogen or $C_1$-$C_{10}$ alkyl;

$R_r$ is -$R_x$-$R_y$;

each $R_s$, $R_c$, $R_u$ and $R_v$ is, independently, hydrogen, $C(O)R_w$, substituted or unsubstituted $C_1$-$C_{10}$ alkyl, substituted or unsubstituted $C_2$-$C_{10}$ alkenyl, substituted or unsubstituted $C_2$-$C_{10}$ alkynyl, alkylsulfonyl, arylsulfonyl, a chemical functional group or a conjugate group, wherein the substituent groups are selected from hydroxyl, amino, alkoxy, carboxy, benzyl, phenyl, nitro, thiol, thioalkoxy, halogen, alkyl, aryl, alkenyl and alkynyl;

or optionally, $R_u$ and $R_v$, together form a phthalimido moiety with the nitrogen atom to which they are attached;

each $R_w$ is, independently, substituted or unsubstituted $C_1$-$C_{10}$ alkyl, trifluoromethyl, cyanoethyloxy, methoxy, ethoxy, t-butoxy, allyloxy, 9-fluorenylmethoxy, 2-(trimethylsilyl)-ethoxy, 2,2,2-trichloroethoxy, benzyloxy, butyryl, iso-butyryl, phenyl or aryl;

$R_k$ is hydrogen, a nitrogen protecting group or -$R_x$-$R_y$;

$R_p$ is hydrogen, a nitrogen protecting group or -$R_x$-$R_y$;

$R_x$ is a bond or a linking moiety;

$R_y$ is a chemical functional group, a conjugate group or a solid support medium;

each $R_m$ and $R_n$ is, independently, H, a nitrogen protecting group, substituted or unsubstituted $C_1$-$C_{10}$ alkyl, substituted or unsubstituted $C_2$-$C_{10}$ alkenyl, substituted or unsubstituted $C_2$-$C_{10}$ alkynyl, wherein the substituent groups are selected from hydroxyl, amino, alkoxy, carboxy, benzyl, phenyl, nitro, thiol, thioalkoxy, halogen, alkyl, aryl, alkenyl, alkynyl; $NH_3^+$, $N(R_u)$ $(R_v)$, guanidino and acyl where said acyl is an acid amide or an ester;

or $R_m$ and $R_n$, together, are a nitrogen protecting group, are joined in a ring structure that optionally includes an additional heteroatom selected from N and O or are a chemical functional group;
$R_i$ is $OR_z$, $SR_z$, or $N(R_z)_2$;
each $R_z$ is, independently, H, $C_1$-$C_8$ alkyl, $C_1$-$C_8$ haloalkyl, $C(=NH)N(H)R_u$, $C(=O)N(H)R_u$ or $OC(=O)N(H)R_u$;
$R_f$, $R_g$ and $R_h$ comprise a ring system having from about 4 to about 7 carbon atoms or having from about 3 to about 6 carbon atoms and 1 or 2 heteroatoms wherein said heteroatoms are selected from oxygen, nitrogen and sulfur and wherein said ring system is aliphatic, unsaturated aliphatic, aromatic, or saturated or unsaturated heterocyclic;
$R_j$ is alkyl or haloalkyl having 1 to about 10 carbon atoms, alkenyl having 2 to about 10 carbon atoms, alkynyl having 2 to about 10 carbon atoms, aryl having 6 to about 14 carbon atoms, $N(R_k)$ $(R_m)$ $OR_k$, halo, $SR_k$ or CN;
$m_a$ is 1 to about 10;
each mb is, independently, 0 or 1;
mc is 0 or an integer from 1 to 10;
md is an integer from 1 to 10;
me is from 0, 1 or 2; and
provided that when mc is 0, md is greater than 1.
Representative substituents groups of Formula I are disclosed in United States Patent Application Serial No. 09/130,973, filed August 7, 1998, entitled "Capped 2'-Oxyethoxy Oligonucleotides".
Representative cyclic substituent groups of Formula II are disclosed in United States Patent Application Serial No. 09/123,108, filed July 27, 1998, entitled "RNA Targeted 2'-Oligomeric compounds that are Conformationally Preorganized".
Particularly preferred sugar substituent groups include $O[(CH_2)nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10.
Representative guanidino substituent groups that are shown in formula III and IV are disclosed in co-owned United States Patent Application 09/349,040, entitled "Functionalized Oligomers", filed July 7, 1999.
Representative acetamido substituent groups are disclosed in United States Patent 6,147,200 which is hereby incorporated by reference in its entirety.
Representative dimethylaminoethyloxyethyl substituent groups are disclosed in International Patent Application PCT/US99/17895, entitled "2'-O-Dimethylaminoethyloxyethyl-Oligomeric compounds", filed August 6, 1999.

Modified Nualeobases/Naturally occurring nucleobases

Oligomeric compounds may also include nucleobase (often referred to in the art simply as "base" or "heterocyclic base moiety") modifications or substitutions. As used herein, "unmodified" or "natural" nucleobases include the purine bases adenine (A) and guanine (G), and the pyrimidine bases thymine (T), cytosine (C) and uracil (U). Modified nucleobases also referred herein as heterocyclic base moieties include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl ($-C{\equiv}C-CH_3$) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine.

Heterocyclic base moieties may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, pages 858-859, Kroschwitz, J.I., ed. John Wiley & Sons, 1990, those disclosed by Englisch et al., Angewandte Chemie, International Edition, 1991, 30, 613, and those disclosed by Sanghvi, Y.S., Chapter 15, Antisense Research and Applications, pages 289-302, Crooke, S.T. and Lebleu, B., ed., CRC Press, 1993. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyl-adenine, 5-propynyluracil and 5-propynylcytosine. 5-methylcytosine substitutions have been shown to increase nucleic acid duplex stability by 0.6-1.2°C (Sanghvi, Y.S., Crooke, S.T. and Lebleu, B., eds., Antisense Research and Applications, CRC Press, Boca Raton, 1993, pp. 276-278) and are presently preferred base substitutions, even more particularly when combined with 2'-O-methoxyethyl sugar modifications.

In one aspect of the present invention oligomeric compounds are prepared having polycyclic heterocyclic compounds in place of one or more heterocyclic base moieties. A number of tricyclic heterocyclic compounds have been previously reported. These compounds are routinely used in antisense applications to increase the binding properties of the modified strand to a target strand. The most studied modifications are targeted to guanosines hence they have

been termed G-clamps or cytidine analogs. Many of these polycyclic heterocyclic compounds have the general formula:

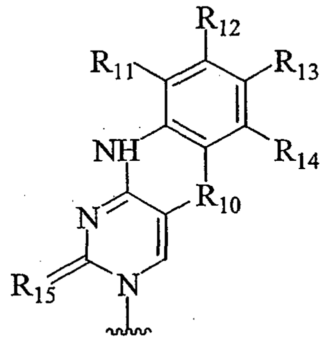

Representative cytosine analogs that make 3 hydrogen bonds with a guanosine in a second strand include 1,3-diazaphenoxazine-2-one ($R_{10}$ = O, $R_{11}$ - $R_{14}$= H) [Kurchavov, et al., Nucleosides and Nucleotides, 1997, 16, 1837-1846], 1,3-diazaphenothiazine-2-one ($R_{10}$= S, $R_{11}$ - $R_{14}$= H), [Lin, K.-Y.; Jones, R. J.; Matteucci, M. J. Am. Chem. Soc. 1995, 117, 3873-3874] and 6,7,8,9-tetrafluoro-1,3-diazaphenoxazine-2-one ($R_{10}$ = O, $R_{11}$ - $R_{14}$ = F) [Wang, J.; Lin, K.-Y., Matteucci, M. Tetrahedron Lett. 1998, 39, 8385-8388]. Incorporated into oligonucleotides these base modifications were shown to hybridize with complementary guanine and the latter was also shown to hybridize with adenine and to enhance helical thermal stability by extended stacking interactions(also see U.S. Patent Application entitled "Modified Peptide Nucleic Acids" filed May 24, 2002, Serial number 10/155,920; and U.S. Patent Application entitled "Nuclease Resistant Chimeric Oligonucleotides" filed May 24, 2002, Serial number 10/013,295).

Further helix-stabilizing properties have been observed when a cytosine analog/substitute has an aminoethoxy moiety attached to the rigid 1,3-diazaphenox-azine-2-one scaffold ($R_{10}$ = O, $R_{11}$ = $-O-(CH_2)_2-NH_2$, $R_{12-14}$=H) [Lin, K.-Y.; Matteucci, M. J. Am. Chem. Soc. 1998, 120, 8531-8532]. Binding studies demonstrated that a single incorporation could enhance the binding affinity of a model oligonucleotide to its complementary target DNA or RNA with a $\Delta T_m$ of up to 18° relative to 5-methyl cytosine ($dC5^{me}$), which is the highest known affinity enhancement for a single modification, yet. On the other hand, the gain in helical stability does not compromise the specificity of the oligonucleotides. The $T_m$ data indicate an even greater discrimination between the perfect match and mismatched sequences compared to $dC5^{ma}$. It was suggested that the tethered amino group serves as an additional hydrogen bond donor to interact with the Hoogsteen face, namely the 06, of a complementary guanine thereby forming 4 hydrogen bonds. This means that the increased affinity of G-clamp is mediated by the combination of extended base stacking and additional specific hydrogen bonding.

Further tricyclic heterocyclic compounds and methods ot using them that are amenable to the present invention are disclosed in United States Patent Serial Number 6,028,183, which issued on May 22, 2000, and United States Patent Serial Number 6,007,992.

The enhanced binding affinity of the phenoxazine derivatives together with their uncompromised sequence specificity makes them valuable nucleobase analogs for the development of more potent antisense-based drugs. In fact, promising data have been derived from in vitro experiments demonstrating that heptanucleotides containing phenoxazine substitutions are capable to activate RNaseH, enhance cellular uptake and exhibit an increased antisense activity [Lin, K-Y; Matteucci, M. J. Am. Chem. Soc. 1998, 120, 8531-8532]. The activity enhancement was even more pronounced in case of G-clamp, as a single substitution was shown to significantly improve the in vitro potency of a 20mer 2'-deoxyphosphorothioate oligonucleotides [Flanagan, W. M.; Wolf, J.J.; Olson, P.; Grant, D.; Lin, K.-Y.; Wagner, R. W.; Matteucci, M. Proc. Natl. Acad. Sci. USA, 1999, 96, 3513-3518]. Nevertheless, to optimize oligonucleotide design and to better understand the impact of these heterocyclic modifications on the biological activity, it is important to evaluate their effect on the nuclease stability of the oligomers.

Further modified polycyclic heterocyclic compounds useful as heterocyclcic bases are disclosed in but not limited to, the above noted U.S. 3,687,808, as well as U.S.: 4,845,205; 5,130,302; 5,134,066; 5,175,273; 5,367,066; 5,432,272; 5,434,257; 5,457,187; 5,459,255; 5,484,908; 5,502,177; 5,525,711; 5,552,540; 5,587,469; 5,594,121, 5,596,091; 5,614,617; 5,645,985; 5,646,269; 5,750,692; 5,830,653; 5,763,588; 6,005,096; and 5,681,941 and Unites States Patent Application Serial number 09/996,292 filed November 28, 2001.

The oligonucleotides of the present invention also include variants in which a different base is present at one or more of the nucleotide positions in the oligonucleotide. For example, if the first nucleotide is an adenosine, variants may be produced which contain thymidine, guanosine or cytidine at this position. This may be done at any of the positions of the oligonucleotide. These oligonucleotides are then tested using the methods described herein to determine their ability to inhibit expression of STAT3.

**Conjugates**

A further preferred substitution that can be appended to the oligomeric compounds of the invention involves the linkage of one or more moieties or conjugates which enhance the activity, cellular distribution or cellular uptake of the resulting oligomeric compounds. In one embodiment such modified oligomeric compounds are prepared by covalently attaching conjugate groups to functional groups such as hydroxyl or amino groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugates groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve oligomer uptake, enhance oligomer resistance to degradation, and/or strengthen sequence-specific hybridization with RNA. Groups that enhance the pharmacokinetic properties, in the context of this invention, include groups that improve oligomer uptake, distribution, metabolism or excretion. Representative conjugate groups are disclosed in International Patent Application PCT/US92/09196, filed October 23, 1992. Conjugate moieties include but are not limited to lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., dodecandiol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937.

The oligomeric compounds of the invention may also be conjugated to active drug substances, for example, aspirin, warfarin, phenylbutazone, ibuprofen, suprofen, fenbufen, naproxen, ketoprofen, (*S*)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic. Oligonucleotide-drug conjugates and their preparation are described in United States Patent Application 09/334,130 (filed June 15, 1999).

Representative United States patents that teach the preparation of such oligonucleotide conjugates include, but are not limited to, U.S.: 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941, each of which is herein incorporated by reference.

Oligomeric compounds used in the compositions of the present invention can also be modified to have one or more stabilizing groups that are generally attached to one or both termini of oligomeric compounds to enhance properties such as for example nuclease stability. Included in stabilizing groups are cap structures. By "cap structure or terminal cap moiety"-is meant chemical modifications, which have been incorporated at either terminus of oligonucleotides (see for example Wincott et al., WO 97/26270). These terminal modifications protect the oligomeric compounds having terminal nucleic acid molecules from exonuclease degradation, and can help in delivery and/or localization within a cell. The cap can be present at the 5'-terminus (5'-cap) or at the 3'-terminus (3'-cap) or can be present on both termini. In non-limiting examples, the 5'-cap includes inverted abasic residue (moiety), 4',5'-methylene nucleotide; 1-(beta-D-erythrofuranosyl) nucleotide, 4'-thio nucleotide, carbocyclic nucleotide; 1,5-anhydrohexitol nucleotide; L-nucleotides; alpha-nucleotides; modified base nucleotide; phosphorodithioate linkage; threo-pentofuranosyl nucleotide; acyclic 3',4'-seco nucleotide; acyclic 3,4-dihydroxybutyl nucleotide; acyclic 3,5-dihydroxypentyl riucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety (for more details see Wincott et al., International PCT publication No. WO 97/26270).

Particularly preferred 3'-cap structures of the present invention include, for example 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate;

threo-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inverted abasic moiety; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moieties (for more details see Beaucage and Tyer, 1993, Tetrahedron 49, 1925).

Further 3' and 5'-stabilizing groups that can be used to cap one or both ends of an oligomeric compound to impart nuclease stability include those disclosed in WO 03/004602 published on January 16, 2003.

Chimeric oligomeric compounds

It is not necessary for all positions in an oligomeric compound to be uniformly modified, and in fact more than one of the aforementioned modifications may be incorporated in a single oligomeric compound or even at a single monomeric subunit such as a nucleoside within a oligomeric compound. The present invention also includes oligomeric compounds which are chimeric oligomeric compounds. "Chimeric" oligomeric compounds or "chimeras," in the context of this invention, are oligomeric compounds that contain two or more chemically distinct regions, each made up of at least one monomer unit, i.e., a nucleotide in the case of a nucleic acid based oligomer.

Chimeric oligomeric compounds typically contain at least one region modified so as to confer increased resistance to nuclease degradation, increased cellular uptake, and/or increased binding affinity for the target nucleic acid. An additional region of the oligomeric compound may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of inhibition of gene expression. Consequently, comparable results can often be obtained with shorter oligomeric compounds when chimeras are used, compared to for example phosphorothioate deoxyoligonucleotides hybridizing to the same target region. Cleavage of the RNA target can be routinely detected by gel electrophoresis and, if necessary, associated nucleic acid hybridization techniques known in the art.

Chimeric oligomeric compounds of the invention may be formed as composite structures of two or more oligonucleotides, oligonucleotide analogs, oligonucleosides and/or oligonucleotide mimetics as described above. Such oligomeric compounds have also been referred to in the art as hybrids hemimers, gapmers or inverted gapmers. Representative United States patents that teach the preparation of such hybrid structures include, but are not limited to, U.S.: 5,013,830; 5,149,797; 5,220,007; 5,256,775; 5,366,878; 5,403,711; 5,491,133; 5,565,350; 5,623,065; 5,652,355; 5,652,356; and 5,700,922.

*3'-endo modifications*

In one aspect of the present invention oligomeric compounds include nucleosides synthetically modified to induce a 3'-endo sugar conformation. A nucleoside can incorporate synthetic modifications of the heterocyclic base, the sugar moiety or both to induce a desired 3'-endo sugar conformation. These modified nucleosides are used to mimic RNA like nucleosides so that particular properties of an oligomeric compound can be enhanced while maintaining the desirable 3'-endo conformational geometry.

There is an apparent preference for an RNA type duplex (A form helix, predominantly 3'-endo) as a requirement (e.g. trigger) of RNA interference which is supported in part by the fact that duplexes composed of 2'-deoxy-2'-F-nucleosides appears efficient in triggering RNAi response in the *C. elegans* system. Properties that are enhanced by using more stable 3'-endo nucleosides include but aren't limited to modulation of pharmacokinetic properties through modification of protein binding, protein off-rate, absorption and clearance; modulation of nuclease stability as well as chemical stability; modulation of the binding affinity and specificity of the oligomer (affinity and specificity for enzymes as well as for complementary sequences); and increasing efficacy of RNA cleavage. The present invention provides oligomeric triggers of RNA having one or more nucleosides modified in such a way as to favor a C3'-endo type conformation.

Scheme 1

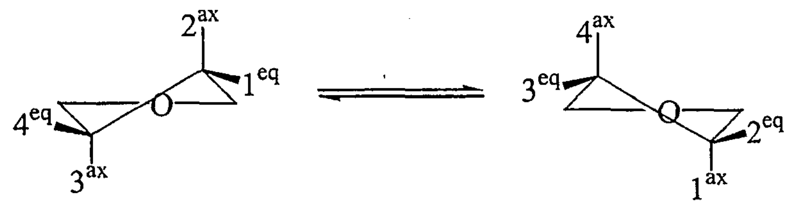

C2'-endo/Southern C3'-endo/Northern

Nucleoside conformation is influenced by various factors including substitution at the 2', 3' or 4'-positions of the pentofuranosyl sugar. Electronegative substituents generally prefer the axial positions, while sterically demanding substituents generally prefer the equatorial positions (Principles of Nucleic Acid Structure, Wolfgang Sanger, 1984, Springer-Verlag.) Modification of the 2' position to favor the 3'-endo conformation can be achieved while maintaining the 2'-OH as a recognition element, as illustrated in Figure 2, below (Gallo et al., Tetrahedron (2001), 57, 5707-5713. Harry-O'kuru et al., J. Org. Chem., (1997), 62(6), 1754-1759 and Tang et al., J. Org. Chem. (1999), 64, 747-754.) Alternatively, preference for the 3'-endo conformation can be achieved by deletion of the 2'-OH as exemplified by 2'deoxy-2'F-nucleosides (Kawasaki et al., J. Med. Chem. (1993), 36, 831-841), which adopts the 3'-endo conformation positioning the electronegative fluorine atom in the axial position. Other modifications of the ribose ring, for example substitution at the 4'-position to give 4'-F modified nucleosides (Guillerm et al., Bioorganic and Medicinal Chemistry Letters (1995), 5, 1455-1460 and Owen et al., J. Org. Chem. (1976), 41, 3010-3017), or for example modification to yield methanocarba nucleoside analogs (Jacobson et al., J. Med. Chem. Lett. (2000), 43, 2196-2203 and Lee et al., Bioorganic and Medicinal Chemistry Letters (2001), 11, 1333-1337) also induce preference for the 3'-endo conformation. Along similar lines, oligomeric triggers of RNAi response might be composed of one or more nucleosides modified in such a way that conformation is locked into a C3'-endo type conformation, i.e. Locked Nucleic Acid (LNA, Singh et al, Chem. Commun. (1998), 4, 455-456), and ethylene bridged Nucleic Acids (ENA, Morita et al, Bioorganic & Medicinal Chemistry Letters (2002), 12, 73-76.) Examples of modified nucleosides amenable to the present invention are shown below in Table I. These examples are meant to be representative and not exhaustive.

**Table I**

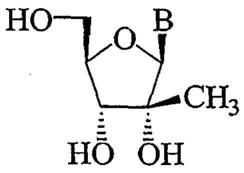
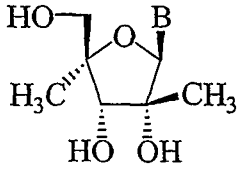
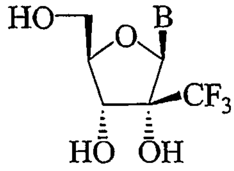
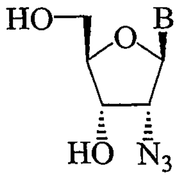
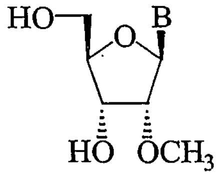
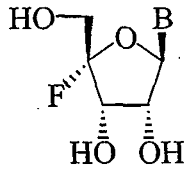
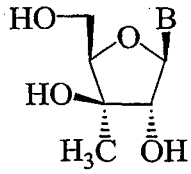
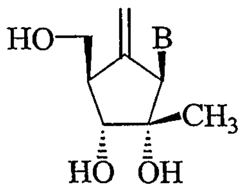
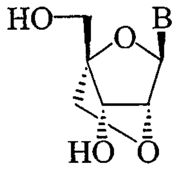
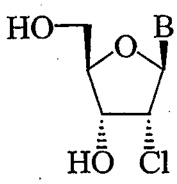
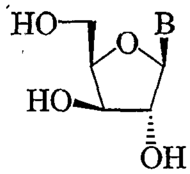
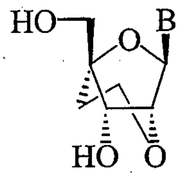
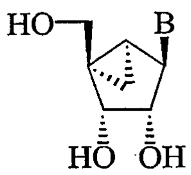
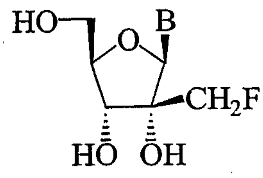
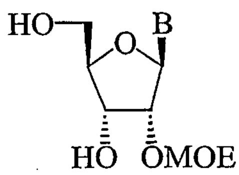
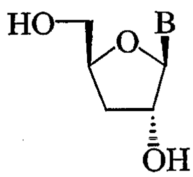
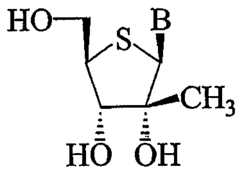
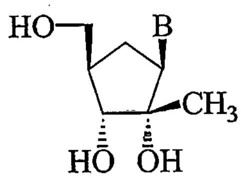
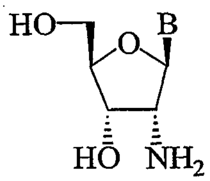

The preferred conformation of modified nucleosides and their oligomers can be estimated by various methods such as molecular dynamics calculations, nuclear magnetic resonance spectroscopy and CD measurements. Hence, modifications predicted to induce RNA like conformations, A-form duplex geometry in an oligomeric context, are selected for use in the modified oligoncleotides of the present invention. The synthesis of numerous of the modified nucleosides amenable to the present invention are known in the art (see for example, Chemistry of Nucleosides and Nucleotides Vol 1-3, ed. Leroy B. Townsend, 1988, Plenum press., and the examples section below.) Nucleosides known to be inhibitors/substrates for RNA dependent RNA polymerases (for example HCV NS5B

In one aspect, the present invention is directed to oligonucleotides that are prepared having enhanced properties compared to native RNA against nucleic acid targets. A target is identified and an oligonucleotide is selected having an effective length and sequence that is complementary to a portion of the target sequence. Each nucleoside of the selected

sequence is scrutinized for possible enhancing modifications. A preferred modification would be the replacement of one or more RNA nucleosides with nucleosides that have the same 3'-endo conformational geometry. Such modifications can enhance chemical and nuclease stability relative to native RNA while at the same time being much cheaper and easier to synthesize and/or incorporate into an oligonulceotide. The selected sequence can be further divided into regions and the nucleosides of each region evaluated for enhancing modifications that can be the result of a chimeric configuration. Consideration is also given to the 5' and 3'-termini as there are often advantageous modifications that can be made to one or more of the terminal nucleosides. The oligomeric compounds of the present invention may include at least one 5'-modified phosphate group on a single strand or on at least one 5'-position of a double stranded sequence or sequences. Further modifications are also considered such as internucleoside linkages, conjugate groups, substitute sugars or bases, substitution of one or more nucleosides with nucleoside mimetics and any other modification that can enhance the selected sequence for its intended target. The terms used to describe the conformational geometry of homoduplex nucleic acids are "A Form" for RNA and "B Form" for DNA. The respective conformational geometry for RNA and DNA duplexes was determined from X-ray diffraction analysis of nucleic acid fibers (Arnott and Hukins, Biochem. Biophys. Res. Comm., 1970, 47, 1504.) In general, RNA:RNA duplexes are more stable and have higher melting temperatures (Tm's) than DNA:DNA duplexes (Sanger et al., Principles of Nucleic Acid Structure, 1984, Springer-Verlag; New York, NY.; Lesnik et al., Biochemistry, 1995, 34, 10807-10815; Conte et al., Nucleic Acids Res., 1997, 25, 2627-2634). The increased stability of RNA has been attributed to several structural features, most notably the improved base stacking interactions that result from an A-form geometry (Searle et al., Nucleic Acids Res., 1993, 21, 2051-2056). The presence of the 2' hydroxyl in RNA biases the sugar toward a C3' endo pucker, i.e., also designated as Northern pucker, which causes the duplex to favor the A-form geometry. In addition, the 2' hydroxyl groups of RNA can form a network of water mediated hydrogen bonds that help stabilize the RNA duplex (Egli et al., Biochemistry, 1996, 35, 8489-8494). On the other hand, deoxy nucleic acids prefer a C2' endo sugar pucker, i.e., also known as Southern pucker, which is thought to impart a less stable B-form geometry (Sanger, W. (1984) Principles of Nucleic Acid Structure, Springer-Verlag, New York, NY). As used herein, B-form geometry is inclusive of both C2'-endo pucker and O4'-endo pucker. This is consistent with Berger, et. al., Nucleic Acids Research, 1998, 26, 2473-2480, who pointed out that in considering the furanose conformations which give rise to B-form duplexes consideration should also be given to a O4'-endo pucker contribution.

DNA:RNA hybrid duplexes, however, are usually less stable than pure RNA:RNA duplexes, and depending on their sequence may be either more or less stable than DNA:DNA duplexes (Searle et al., Nucleic Acids Res., 1993, 21, 2051-2056). The structure of a hybrid duplex is intermediate between A- and B-form geometries, which may result in poor stacking interactions (Lane et al., Eur. J. Biochem., 1993, 215, 297-306; Fedoroff et al., J. Mol. Biol., 1993, 233, 509-523; Gonzalez et al., Biochemistry, 1995, 34, 4969-4982; Horton et al., J. Mol. Biol., 1996, 264, 521-533). The stability of the duplex formed between a target RNA and a synthetic sequence is central to therapies such as but not limited to antisense and RNA interference as these mechanisms require the binding of a synthetic oligonucleotide strand to an RNA target strand.

In the case of antisense, effective inhibition of the mRNA requires that the antisense DNA have a very high binding affinity with the mRNA. Otherwise the desired interaction between the synthetic oligonucleotide strand and target mRNA strand will occur infrequently, resulting in decreased efficacy.

One routinely used method of modifying the sugar puckering is the substitution of the sugar at the 2'-position with a substituent group that influences the sugar geometry. The influence on ring conformation is dependant on the nature of the substituent at the 2'-position. A number of different substituents have been studied to determine their sugar puckering effect. For example, 2'-halogens have been studied showing that the 2'-fluoro derivative exhibits the largest population (65%) of the C3'-endo form, and the 2'-iodo exhibits the lowest population (7%). The populations of adenosine (2'-OH) versus deoxyadenosine (2'-H) are 36% and 19%, respectively. Furthermore, the effect of the 2'-fluoro group of adenosine dimers (2'-deoxy-2'-fluoroadenosine - 2'-deoxy-2'-fluoro-adenosine) is further correlated to the stabilization of the stacked conformation.

As expected, the relative duplex stability can be enhanced by replacement of 2'-OH groups with 2'-F groups thereby increasing the C3'-endo population. It is assumed that the highly polar nature of the 2'-F bond and the extreme preference for C3'-endo puckering may stabilize the stacked conformation in an A-form duplex. Data from UV hypochromicity, circular dichroism, and $^{1}$H NMR also indicate that the degree of stacking decreases as the electronegativity of the halo substituent decreases. Furthermore, steric bulk at the $2^{1}$-position of the sugar moiety is better accommodated in an A-form duplex than a B-form duplex. Thus, a 2'-substituent on the 3'-terminus of a dinucleoside monophosphate is thought to exert a number of effects on the stacking conformation: steric repulsion, furanose puckering preference, electrostatic repulsion, hydrophobic attraction, and hydrogen bonding capabilities. These substituent effects are thought to be determined by the molecular size, electronegativity, and hydrophobicity of the substituent. Melting temperatures of complementary strands is also increased with the 2'-substituted adenosine diphosphates. It is not clear whether the 3'-endo preference of the conformation or the presence of the substituent is responsible for the increased binding. However, greater overlap of adjacent bases (stacking) can be achieved with the 3'-endo conformation.

One synthetic 2'-modification that imparts increased nuclease resistance and a very high binding affinity to

nucleotides is the 2-methoxyethoxy (2'-MOE, 2'-$OCH_2CH_2OCH_3$) side chain (Baker et al., J. Biol. Chem., 1997, 272, 11944-12000). One of the immediate advantages of the 2'-MOE substitution is the improvement in binding affinity, which is greater than many similar 2' modifications such as O-methyl, O-propyl, and O-aminopropyl. Oligonucleotides having the 2'-O-methoxyethyl substituent also have been shown to be antisense inhibitors of gene expression with promising features for *in vivo* use (Martin, P., Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926). Relative to DNA, the oligonucleotides having the 2'-MOE modification displayed improved RNA affinity and higher nuclease resistance. Chimeric oligonucleotides having 2'-MOE substituents in the wing nucleosides and an internal region of deoxy-phosphorothioate nucleotides (also termed a gapped oligonucleotide or gapmer) have shown effective reduction in the growth of tumors in animal models at low doses. 2'-MOE substituted oligonucleotides have also shown outstanding promise as antisense agents in several disease states. One such MOE substituted oligonucleotide is presently being investigated in clinical trials for the treatment of CMV retinitis.

**Chemistries Defined**

Unless otherwise defined herein, alkyl means $C_1$-$C_{12}$, preferably $C_1$-$C_8$, and more preferably $C_1$-$C_6$, straight or (where possible) branched chain aliphatic hydrocarbyl.

Unless otherwise defined herein, heteroalkyl means $C_1$-$C_{12}$, preferably $C_1$-$C_8$, and more preferably $C_1$-$C_6$, straight or (where possible) branched chain aliphatic hydrocarbyl containing at least one, and preferably about 1 to about 3, hetero atoms in the chain, including the terminal portion of the chain. Preferred heteroatoms include N, O and S. Unless otherwise defined herein, cycloalkyl means $C_3$-$C_{12}$, preferably $C_3$-$C_8$, and more preferably $C_3$-$C_6$, aliphatic hydrocarbyl ring.

Unless otherwise defined herein, alkenyl means $C_2$-$C_{12}$, preferably $C_2$-$C_8$, and more preferably $C_2$-$C_6$ alkenyl, which may be straight or (where possible) branched hydrocarbyl moiety, which contains at least one carbon-carbon double bond.

Unless otherwise defined herein, alkynyl means $C_2$-$C_{12}$, preferably $C_2$-$C_8$, and more preferably $C_2$-$C_6$ alkynyl, which may be straight or (where possible) branched hydrocarbyl moiety, which contains at least one carbon-carbon triple bond.

Unless otherwise defined herein, heterocycloalkyl means a ring moiety containing at least three ring members, at least one of which is carbon, and of which 1, 2 or three ring members are other than carbon. Preferably the number of carbon atoms varies from 1 to about 12, preferably 1 to about 6, and the total number of ring members varies from three to about 15, preferably from about 3 to about 8. Preferred ring heteroatoms are N, O and S. Preferred heterocycloalkyl groups include morpholino, thiomorpholino, piperidinyl, piperazinyl, homopiperidinyl, homopiperazinyl, homomorpholino, homothiomorpholino, pyrrolodinyl, tetrahydrooxazolyl, tetrahydroimidazolyl, tetrahydrothiazolyl, tetrahydroisoxazolyl, tetrahydropyrrazolyl, furanyl, pyranyl, and tetrahydroisothiazolyl.

Unless otherwise defined herein, aryl means any hydrocarbon ring structure containing at least one aryl ring. Preferred aryl rings have about 6 to about 20 ring carbons. Especially preferred aryl rings include phenyl, napthyl, anthracenyl, and phenanthrenyl.

Unless otherwise defined herein, hetaryl means a ring moiety containing at least one fully unsaturated ring, the ring consisting of carbon and non-carbon atoms. Preferably the ring system contains about 1 to about 4 rings. Preferably the number of carbon atoms varies from 1 to about 12, preferably 1 to about 6, and the total number of ring members varies from three to about 15, preferably from about 3 to about 8. Preferred ring heteroatoms are N, O and S. Preferred hetaryl moieties include pyrazolyl, thiophenyl, pyridyl, imidazolyl, tetrazolyl, pyridyl, pyrimidinyl, purinyl, quinazolinyl, quinoxalinyl, benzimidazolyl, benzothiophenyl, etc.

Unless otherwise defined herein, where a moiety is defined as a compound moiety, such as hetarylalkyl (hetaryl and alkyl), aralkyl (aryl and alkyl), etc., each of the sub-moieties is as defined herein.

Unless otherwise defined herein, an electron withdrawing group is a group, such as the cyano or isocyanato group that draws electronic charge away from the carbon to which it is attached. Other electron withdrawing groups of note include those whose electronegativities exceed that of carbon, for example halogen, nitro, or phenyl substituted in the ortho- or para-position with one or more cyano, isothiocyanato, nitro or halo groups.

Unless otherwise defined herein, the terms halogen and halo have their ordinary meanings. Preferred halo (halogen) substituents are Cl, Br, and I.

The aforementioned optional substituents are, unless otherwise herein defined, suitable substituents depending upon desired properties. Included are halogens (Cl, Br, I), alkyl, alkenyl, and alkynyl moieties, $NO_2$, $NH_3$ (substituted and unsubstituted), acid moieties (e.g. - $CO_2H$, -$OSO_3H_2$, etc.), heterocycloalkyl moieties, hetaryl moieties, aryl moieties, etc. In all the preceding formulae, the squiggle (~) indicates a bond to an oxygen or sulfur of the 5'-phosphate.

Phosphate protecting groups include those described in US Patents No. US 5,760,209, US 5,614,621, US 6,051,699, US 6,020,475, US 6,326,478, US 6,169,177, US 6,121,437, US 6,465,628.

The oligonucleotides used in accordance with this invention may be conveniently and routinely made through the well-known technique of solid phase synthesis. Equipment for such synthesis is sold by several vendors including Applied Biosystems. Any other means for such synthesis may also be employed; the actual synthesis of the oligonucleotides is well within the talents of the routineer. It is well known to use similar techniques to prepare oligonucleotides such as the phosphorothioates and 2'-alkoxy or 2'-alkoxyalkoxy derivatives, including 2'-O-methoxyethyl oligonucleotides (Martin, P., Helv. Chim. Acta 1995, 78, 486-504). It is also well known to use similar techniques and commercially available modified amidites and controlled-pore glass (CPG) products such as biotin, fluorescein, acridine or psoralen-modified amidites and/or CPG (available from Glen Research, Sterling, VA) to synthesize fluorescently labeled, biotinylated or other conjugated oligonucleotides.

## Antisense Compositions and Formulations

The compounds of the invention may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption. Representative United States patents that teach the preparation of such uptake, distribution and/or absorption assisting formulations include, but are not limited to, U.S.: 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

The antisense compounds of the invention encompass any pharmaceutically acceptable salts, esters, or salts of such esters, or any other compound which, upon administration to an animal including a human, is capable of providing (directly or indirectly) the biologically active metabolite or residue thereof. Accordingly, for example, the disclosure is also drawn to prodrugs and pharmaceutically acceptable salts of the compounds of the invention, pharmaceutically acceptable salts of such prodrugs, and other bioequivalents.

The term "prodrug" indicates a therapeutic agent that is prepared in an inactive form that is converted to an active form (i.e., drug) within the body or cells thereof by the action of endogenous enzymes or other chemicals and/or conditions. In particular, prodrug versions of the oligonucleotides of the invention are prepared as SATE [(S-acetyl-2-thioethyl) phosphate] derivatives according to the methods disclosed in WO 93/24510 to Gosselin et al., published December 9, 1993 or in WO 94/26764 and U.S. 5,770,713 to Imbach et al.

The term "pharmaceutically acceptable salts" refers to physiologically and pharmaceutically acceptable salts of the compounds of the invention: i.e., salts that retain the desired biological activity of the parent compound and do not impart undesired toxicological effects thereto.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine (see, for example, Berge et al., "Pharmaceutical Salts," J. of Pharma Sci., 1977, 66, 1-19). As used herein, a "pharmaceutical addition salt" includes a pharmaceutically acceptable salt of an acid form of one of the components of the compositions of the invention. These include organic or inorganic acid salts of the amines. Preferred acid salts are the hydrochlorides, acetates, salicylates, nitrates and phosphates. Other suitable pharmaceutically acceptable salts are well known to those skilled in the art and include basic salts of a variety of inorganic and organic acids.

For oligonucleotides, preferred examples of pharmaceutically acceptable salts include but are not limited to (a) salts formed with cations such as sodium, potassium, ammonium, magnesium, calcium, polyamines such as spermine and spermidine, etc.; (b) acid addition salts formed with inorganic acids, for example hydrochloric acid, hydrobromic acid, sulfuric acid, phosphoric acid, nitric acid and the like; (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (d) salts formed from elemental anions such as chlorine, bromine, and iodine.

## Pharmaceutical Compositions and Routes of Administration

The pharmaceutical compositions of the present invention may be administered in a number of ways depending upon whether local or systemic treatment is desired and upon the area to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular

injection or infusion; or intracranial, e.g., intrathecal or intraventricular, administration. Oligonucleotides with at least one 2'-O-methoxyethyl modification are believed to be particularly useful for oral administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful. Preferred topical formulations include those in which the oligonucleotides of the invention are in admixture with a topical delivery agent such as lipids, liposomes, fatty acids, fatty acid esters, steroids, chelating agents and surfactants. Preferred lipids and liposomes include neutral (e.g. dioleoylphosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoylphosphatidyl ethanolamine DOTMA). Oligonucleotides of the invention may be encapsulated within liposomes or may form complexes thereto, in particular to cationic liposomes. Alternatively, oligonucleotides may be complexed to lipids, in particular to cationic lipids. Preferred fatty acids and esters include but are not limited arachidonic acid, oleic acid, eicosanoic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a $C_{1-10}$ alkyl ester (e.g. isopropylmyristate IPM), monoglycericle, diglyceride or pharmaceutically acceptable salt thereof.

In some embodiments, an oligonucleotide may be administered to a subject via an oral route of administration. The subjects of the present invention comprise animals. An animal subject may be a mammal, such as a mouse, a rat, a dog, a guinea pig, a monkey, a human, a non-human primate, a cat or a pig. Non-human primates include monkeys and chimpanzees. A suitable animal subject may be an experimental animal, such as a mouse, a rat, a dog, a non-human primate, a cat or a pig.

In some embodiments, the subject may be a human. In certain embodiments, the subject may be a human patient as discussed in more detail herein. In certain embodiments, it may be necessary to modulate the expression of one or more genes of the human patient. In some particular embodiments, it may be necessary to inhibit expression of one or more genes of the human patient. In particular embodiments, it may be necessary to modulate, i.e. inhibit or enhance, STAT3 in order to obtain therapeutic outcomes discussed herein.

In some embodiments, non-parenteral (e.g. oral) oligonucleotide formulations according to the present invention result in enhanced bioavailability of the oligonucleotide. In this context, the term "bioavailability" refers to a measurement of that portion of an administered drug which reaches the circulatory system (e.g. blood, especially blood plasma) when a particular mode of administration is used to deliver the drug. Enhanced bioavailability refers to a particular mode of administration's ability to deliver oligonucleotide to the peripheral blood plasma of a subject relative to another mode of administration. For example, when a non-parenteral mode of administration (e.g. an oral mode) is used to introduce the drug into a subject, the bioavailability for that mode of administration may be compared to a different mode of administration, e.g. an IV mode of administration. In some embodiments, the area under a compound's blood plasma concentration curve ($AUC_0$) after non-parenteral administration may be divided by the area under the drug's plasma concentration curve after intravenous (i.v.) administration ($AUC_{iv}$) to provide a dimensionless quotient (relative bioavailability, RB) that represents fraction of compound absorbed via the non-parenteral route as compared to the IV route. A composition's bioavailability is said to be enhanced in comparison to another composition's bioavailability when the first composition's relative bioavailability ($RB_1$) is greater than the second composition's relative bioavailability ($RB_2$).

In general, bioavailability correlates with therapeutic efficacy when a compound's therapeutic efficacy is related to the blood concentration achieved, even if the drug's ultimate site of action is intracellular (van Berge-Henegouwen et al., Gastroenterol., 1977, 73, 300). Bioavailability studies have been used to determine the degree of intestinal absorption of a drug by measuring the change in peripheral blood levels of the drug after an oral dose (DiSanto, Chapter 76 In: Remington's Pharmaceutical Sciences, 18th Ed., Gennaro, ed., Mack Publishing Co., Easton, PA, 1990, pages 1451-1458).

In general, an oral composition (comprising an oligonucleotide) bioavailability is said to be "enhanced" when its relative bioavailability is greater than the bioavailability of a composition substantially consisting of pure oligonucleotide, i.e. oligonucleotide in the absence of a penetration enhancer.

Organ bioavailability refers to the concentration of compound in an organ. Organ bioavailability may be measured in test subjects by a number of means, such as by whole-body radiography. Organ bioavailability may be modified, e.g. enhanced, by one or more modifications to the oligonucleotide, by use of one or more carrier compounds or excipients, etc. as discussed in more detail herein. In general, an increase in bioavailability will result in an increase in organ bioavailability.

Oral oligonucleotide compositions according to the present invention may comprise one or more "mucosal penetration enhancers," also known as "absorption enhancers" or simply as "penetration enhancers." Accordingly, some embodiments of the invention comprise at least one oligonucleotide in combination with at least one penetration enhancer. In general, a penetration enhancer is a substance that facilitates the transport of a drug across mucous membrane(s) associated with the desired mode of administration, e.g. intestinal epithelial membranes. Accordingly it is desirable to

select one or more penetration enhancers that facilitate the uptake of an oligonucleotide, without interfering with the activity of the oligonucleotide, and in such a manner the oligonucleotide can be introduced into the body of an animal without unacceptable degrees of side-effects such as toxicity, irritation or allergic response.

Embodiments of the present invention provide compositions comprising one or more pharmaceutically acceptable penetration enhancers, and methods of using such compositions, which result in the improved bioavailability of oligonucleotides administered via non-parenteral modes of administration. Heretofore, certain penetration enhancers have been used to improve the bioavailability of certain drugs. See Muranishi, Crit. Rev. Ther. Drug Carrier Systems, 1990, 7, 1 and Lee et al., Crit. Rev. Ther. Drug Carrier Systems, 1991, 8, 91. It has been found that the uptake and delivery of oligonucleotides can be greatly improved even when administered by non-parenteral means through the use of a number of different classes of penetration enhancers.

In some embodiments, compositions for non-parenteral administration include one or more modifications to naturally-occurring oligonucleotides (i.e. full-phosphodiester deoxyribosyl or full-phosphodiester ribosyl oligonucleotides). Such modifications may increase binding affinity, nuclease stability, cell or tissue permeability, tissue distribution, or other biological or pharmacokinetic property. Modifications may be made to the base, the linker, or the sugar, in general, as discussed in more detail herein with regards to oligonucleotide chemistry. In some embodiments of the invention, compositions for administration to a subject, and in particular oral compositions for administration to an animal (human or non-human) subject, will comprise modified oligonucleotides having one or more modifications for enhancing affinity, stability, tissue distribution, or other biological property.

Suitable modified linkers include phosphorothioate linkers. In some embodiments according to the invention, the oligonucleotide has at least one phosphorothioate linker. Phosphorothioate linkers provide nuclease stability as well as plasma protein binding characteristics to the oligonucleotide. Nuclease stability is useful for increasing the *in vivo* lifetime of oligonucleotides, while plasma protein binding decreases the rate of first pass clearance of oligonucleotide via renal excretion. In some embodiments according to the present invention, the oligonucleotide has at least two phosphorothioate linkers. In some embodiments, wherein the oligonucleotide has exactly n nucleosides, the oligonucleotide has from one to n-1 phosphorothioate linkages. In some embodiments, wherein the oligonucleotide has exactly n nucleosides, the oligonucleotide has n-1 phosphorothioate linkages. In other embodiments wherein the oligonucleotide has exactly n nucleoside, and n is even, the oligonucleotide has from 1 to n/2 phosphorothioate linkages, or, when n is odd, from 1 to (n-1)/2 phosphorothioate linkages. In some embodiments, the oligonucleotide has alternating phosphodiester (PO) and phosphorothioate (PS) linkages. In.other embodiments, the oligonucleotide has at least one stretch of two or more consecutive PO linkages and at least one stretch of two or more PS linkages. In other embodiments, the oligonucleotide has at least two stretches of PO linkages interrupted by at least on PS linkage.

In some embodiments, at least one of the nucleosides is modified on the ribosyl sugar unit by a modification that imparts nuclease stability, binding affinity or some other beneficial biological property to the sugar. In some cases, the sugar modification includes a 2'-modification, e.g. the 2'-OH of the ribosyl sugar is replaced or substituted. Suitable replacements for 2'-OH include 2'-F and 2'-arabino-F. Suitable substitutions for OH include 2'-*O*-alkyl, e.g. 2-*O*-methyl, and 2'-*O*-substituted alkyl, e.g. 2'-*O*-methoxyethyl, 2'-$NH_2$, 2'-*O*-aminopropyl, etc. In some embodiments, the oligonucleotide contains at least one 2'-modification. In some embodiments, the oligonucleotide contains at least two 2'-modifications. In some embodiments, the oligonucleotide has at least one 2'-modification at each of the termini (i.e. the 3'- and 5'-terminal nucleosides each have the same or different 2'-modifications). In some embodiments, the oligonucleotide has at least two sequential 2'-modifications at each end of the oligonucleotide. In some embodiments, oligonucleotides further comprise at least one deoxynucleoside. In particular embodiments, oligonucleotides comprise a stretch of deoxynucleosides such that the stretch is capable of activating RNase (e.g. RNase H) cleavage of an RNA to which the oligonucleotide is capable of hybridizing. In some embodiments, a stretch of deoxynucleosides capable of activating RNase-mediated cleavage of RNA comprises about 6 to about 16, e.g. about 8 to about 16 consecutive deoxynucleosides. In further embodiments, oligonucleotides are capable of eliciting cleaveage by dsRNAse enzymes which act on RNA:RNA hybrids.

Oligonucleotide compositions of the present invention may be formulated in various dosage forms such as, but not limited to, tablets, capsules, liquid syrups, soft gels, suppositories, and enemas. The term "alimentary delivery" encompasses e.g. oral, rectal, endoscopic and sublingual/buccal administration. A common requirement for these modes of administration is absorption over some portion or all of the alimentary tract and a need for efficient mucosal penetration of the oligonucleotides or mimetics thereof so administered.

Delivery of a drug via the oral mucosa, as in the case of buccal and sublingual administration, has several desirable features, including, in many instances, a more rapid rise in plasma concentration of the drug (Harvey, Chapter 35 In: Remington's Pharmaceutical Sciences, 18th Ed., Gennaro, ed., Mack Publishing Co., Easton, PA, 1990, page 711).

Endoscopy may be used for drug delivery directly to an interior portion of the alimentary tract. For example, endoscopic retrograde cystopancreatography (ERCP) takes advantage of extended gastroscopy and permits selective access to the biliary tract and the pancreatic duct (Hirahata et al., Gan To Kagaku Ryoho, 1992, 19(10 Suppl.), 1591). Pharmaceutical compositions, including liposomal formulations, can be delivered directly into portions of the alimentary

canal, such as, *e.g.*, the duodenum (Somogyi et al., Pharm. Res., 1995, 12, 149) or the gastric submucosa (Akamo et al., Japanese J. Cancer Res., 1994, 85, 652) via endoscopic means. Gastric lavage devices (Inoue et al., Artif. Organs, 1997, 21, 28) and percutaneous endoscopic feeding devices (Pennington et al., Ailment Pharmacol. Ther., 1995, 9, 471) can also be used for direct alimentary delivery of pharmaceutical compositions.

In some embodiments, oligonucleotide formulations may be administered through the anus into the rectum or lower intestine. Rectal suppositories, retention enemas or rectal catheters can be used for this purpose and may be preferred when patient compliance might otherwise be difficult to achieve (*e.g.,* in pediatric and geriatric applications, or when the patient is vomiting or unconscious). Rectal administration can result in more prompt and higher blood levels than the oral route. (Harvey, Chapter 35 In: Remington's Pharmaceutical Sciences, 18th Ed., Gennaro, ed., Mack Publishing Co., Easton, PA, 1990, page 711). Because about 50% of the drug that is absorbed from the rectum will likely bypass the liver, administration by this route significantly reduces the potential for first-pass metabolism (Benet et al., Chapter 1 In: Goodman & Gilman's The Pharmacological Basis of Therapeutics, 9th Ed., Hardman et al., eds., McGraw-Hill, New York, NY, 1996).

Other excipients that may be added to oral oligonucleotide compositions include surfactants (or "surface-active agents"). These are chemical entities which, when dissolved in an aqueous solution, reduce the surface tension of the solution or the interfacial tension between the aqueous solution and another liquid, with the result that absorption of oligonucleotides through the alimentary mucosa and other epithelial membranes is enhanced. In addition to bile salts and fatty acids, surfactants include, for example, sodium lauryl sulfate, polyoxyethylene-9-lauryl ether and polyoxyethylene-20-cetyl ether (Lee et al., Critical Reviews in Therapeutic Drug Carrier Systems, 1991, page 92); and perfluorohemical emulsions, such as FC-43 (Takahashi et al., J. Pharm. Phamacol., 1988, 40, 252).

Fatty acids and their derivatives which act as penetration enhancers and may be used in compositions of the present invention include, for example, oleic acid, lauric acid, capric acid (n-decanoic acid), myristic acid, palmitic acid, stearic acid, linoleic acid, linolenic acid, dicaprate, tricaprate, monoolein (1-monooleoyl-rac-glycerol), dilaurin, caprylic acid, arachidonic acid, glyceryl 1-monocaprate, 1-dodecylazacycloheptan-2-one, acylcarnitines, acylcholines and mono- and di-glycerides thereof and/or physiologically acceptable salts thereof (*i.e.*, oleate, laurate, caprate, myristate, palmitate, stearate, linoleate, *etc.*) (Lee et al., Critical Reviews in Therapeutic Drug Carrier Systems, 1991, page 92; Muranishi, Critical Reviews in Therapeutic Drug Carrier Systems, 1990, 7, 1; El-Hariri et al., J. Pharm. Pharmacol., 1992, 44, 651).

In some embodiments, oligonucleotide compositions for oral delivery comprise at least two discrete phases, which phases may comprise particles, capsules, gel-capsules, microspheres, etc. Each phase may contain one or more oligonucleotides, penetration enhancers, surfactants, bioadhesives, effervescent agents, or other adjuvant, excipient or diluent. In some embodiments, one phase comprises at least one oligonucleotide and at lease one penetration enhancer. In some embodiments, a first phase comprises at least one oligonucleotide and at least one penetration enhancer, while a second phase comprises at least one penetration enhancer. In some embodiments, a first phase comprises at least one oligonucleotide and at least one penetration enhancer, while a second phase comprises at least one penetration enhancer and substantially no oligonucleotide. In some embodiments, at least one phase is compounded with at least one degradation retardant, such as a coating or a matrix, which delays release of the contents of that phase. In some embodiments, at least one phase In some embodiments, a first phase comprises at least one oligonucleotide, at least one penetration enhancer, while a second phase comprises at least one penetration enhancer and a release-retardant. In particular embodiments, an oral oligonucleotide composition comprises a first phase comprising particles containing an oligonucleotide and a penetration enhancer, and a second phase comprising particles coated with a release-retarding agent and containing penetration enhancer.

A variety of bile salts also function as penetration enhancers to facilitate the uptake and bioavailability of drugs. The physiological roles of bile include the facilitation of dispersion and absorption of lipids and fat-soluble vitamins (Brunton, Chapter 38 In: Goodman & Gilman's The Pharmacological Basis of Therapeutics, 9th Ed., Hardman et al., eds., McGraw-Hill, New York, NY, 1996, pages 934-935). Various natural bile salts, and their synthetic derivatives, act as penetration enhancers. Thus, the term "bile salt" includes any of the naturally occurring components of bile as well as any of their synthetic derivatives. The bile salts of the invention include, for example, cholic acid (or its pharmaceutically acceptable sodium salt, sodium cholate), dehydrocholic acid (sodium dehydrocholate), deoxycholic acid (sodium deoxycholate), glucholic acid (sodium glucholate), glycholic acid (sodium glycocholate), glycodeoxycholic acid (sodium glycodeoxycholate), taurocholic acid (sodium taurocholate), taurodeoxycholic acid (sodium taurodeoxycholate), chenodeoxycholic acid (CDCA, sodium chenodeoxycholate), ursodeoxycholic acid (UDCA), sodium tauro-24,25-dihydro-fusidate (STDHF), sodium glycodihydrofusidate and polyoxyethylene-9-lauryl ether (POE) (Lee et al., Critical Reviews in Therapeutic Drug Carrier Systems, 1991, page 92; Swinyard, Chapter 39 In: Remington's Pharmaceutical Sciences, 18th Ed., Gennaro, ed., Mack Publishing Co., Easton, PA, 1990, pages 782-783; Muranishi, Critical Reviews in Therapeutic Drug Carrier Systems, 1990, 7, 1; Yamamoto et al., J. Pharm. Exp. Ther., 1992, 263, 25; Yamashita et al., J. Pharm. Sci., 1990, 79, 579).

In some embodiments, penetration enhancers of the present invention are mixtures of penetration enhancing compounds. One such penetration mixture is UDCA (and/or CDCA) with capric and/or lauric acids or salts thereof e.g.

sodium. Such mixtures are useful for enhancing the delivery of biologically active substances across mucosal membranes, in particular intestinal mucosa. Other penetration enhancer mixtures comprise about 5-95% of bile acid or salt(s) UDCA and/or CDCA with 5-95% capric and/or lauric acid. Particular penetration enhancers are mixtures of the sodium salts of UDCA, capric acid and lauric acid in a ratio of about 1:2:2 respectively. Anther such penetration enhancer is a mixture of capric and lauric acid (or salts thereof) in a 0.01:1 to 1:0.01 ratio (mole basis). In particular embodiments capric acid and lauric acid are present in molar ratios of e.g. about 0.1:1 to about 1:0.1, in particular about 0.5:1 to about 1:0.5.

Other excipients include chelating agents, i.e. compounds that remove metallic ions from solution by forming complexes therewith, with the result that absorption of oligonucleotides through the alimentary and other mucosa is enhanced. With regards to their use as penetration enhancers in compositions containing DNA-like oligonucleotides in the present invention, chelating agents have the added advantage of also serving as DNase inhibitors, as most characterized DNA nucleases require a divalent metal ion for catalysis and are thus inhibited by chelating agents (Jarrett, J. Chromatogr., 1993, 618, 315). Chelating agents of the invention include, but are not limited to, disodium ethylenediaminetetraacetate (EDTA), citric acid, salicylates (*e.g.*, sodium salicylate, 5-methoxysalicylate and homovanilate), *N*-acyl derivatives of collagen, laureth-9 and *N*-amino acyl derivatives of beta-diketones (enamines)(Lee et al., Critical Reviews in Therapeutic Drug Carrier Systems, 1991, page 92; Muranishi, Critical Reviews in Therapeutic Drug Carrier Systems, 1990, 7, 1; Buur et al., J. Control Rel. , 1990, 14, 43).

As used herein, non-chelating non-surfactant penetration enhancers may be defined as compounds that demonstrate insignificant activity as chelating agents or as surfactants but that nonetheless enhance absorption of oligonucleotides through the alimentary and other mucosal membranes (Muranishi, Critical Reviews in Therapeutic Drug Carrier Systems, 1990, 7, 1). This class of penetration enhancers includes, but is not limited to, unsaturated cyclic ureas, 1-alkyl- and 1-alkenylazacyclo-alkanone derivatives (Lee et al., Critical Reviews in Therapeutic Drug Carrier Systems, 1991, page 92); and non-steroidal anti-inflammatory agents such as diclofenac sodium, indomethacin and phenylbutazone (Yamashita et al., J. Pharm. Pharmacol., 1987, 39, 621).

Agents that enhance uptake of oligonucleotides at the cellular level may also be added to the pharmaceutical, therapeutic and other compositions of the present invention. For example, cationic lipids, such as LIPOFECTIN™ (Junichi et al, U.S. Patent No. 5,705,188), cationic glycerol derivatives, and polycationic molecules, such as polylysine (Lollo et al., PCT Application WO 97/30731), can be used.

A "pharmaceutical carrier" or "excipient" may be a pharmaceutically acceptable solvent, suspending agent or any other pharmacologically inert vehicle for delivering one or more nucleic acids to an animal. The excipient may be liquid or solid and is selected, with the planned manner of administration in mind, so as to provide for the desired bulk, consistency, *etc*., when combined with a an oligonucleotide and the other components of a given pharmaceutical composition. Typical pharmaceutical carriers include, but are not limited to, binding agents (*e.g.,* pregelatinised maize starch, polyvinylpyrrolidone or hydroxypropyl methylcellulose, *etc*.); fillers (*e.g.,* lactose and other sugars, microcrystalline cellulose, pectin, gelatin, calcium sulfate, ethyl cellulose, polyacrylates or calcium hydrogen phosphate, *etc*.); lubricants (*e.g*., magnesium stearate, talc, silica, colloidal silicon dioxide, stearic acid, metallic stearates, hydrogenated vegetable oils, corn starch, polyethylene glycols, sodium benzoate, sodium acetate, *etc*.); disintegrants (*e.g*., starch, sodium starch glycolate, EXPLOTAB); and wetting agents (*e.g*., sodium lauryl sulphate, *etc*.).

Oral oligonucleotide compositions may additionally contain other adjunct components conventionally found in pharmaceutical compositions, at their art-established usage levels. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the composition of present invention, such as dyes, flavoring agents, preservatives, antioxidants, opacifiers, thickening agents and stabilizers. However, such materials, when added, should not unduly interfere with the biological activities of the components of the compositions of the present invention.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product.

The compositions of the present invention may be formulated into any of many possible dosage forms such as, but not limited to, tablets, capsules, gel capsules, liquid syrups, soft gels, suppositories, and enemas. The compositions of the present invention may also be formulated as suspensions in aqueous, non-aqueous or mixed media. Aqueous

suspensions may further contain substances which increase the viscosity of the suspension including, for example, sodium carboxymethylcellulose, sorbitol and/or dextran. The suspension may also contain stabilizers.

In one embodiment of the present invention the pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product. The preparation of such compositions and formulations is generally known to those skilled in the pharmaceutical and formulation arts and may be applied to the formulation of the compositions of the present invention.

**Emulsions**

The compositions of the present invention may be prepared and formulated as emulsions. Emulsions are typically heterogenous systems of one liquid dispersed in another in the form of droplets usually exceeding 0.1 $\mu$m in diameter (Idson, in Pharmaceutical Dosage Forms, Lieberman, Rieger and Banker (Eds.), 1988, Marcel Dekker, Inc., New York, N.Y., volume 1, p. 199; Rosoff, in Pharmaceutical Dosage Forms, Lieberman, Rieger and Banker (Eds.), 1988, Marcel Dekker, Inc., New York, N.Y., Volume 1, p. 245; Block in Pharmaceutical Dosage Forms, Lieberman, Rieger and Banker (Eds.), 1988, Marcel Dekker, Inc., New York, N.Y., volume 2, p. 335; Higuchi et al., in Remington's Pharmaceutical Sciences, Mack Publishing Co., Easton, PA, 1985, p. 301). Emulsions are often biphasic systems comprising of two immiscible liquid phases intimately mixed and dispersed with each other. In general, emulsions may be either water-in-oil (w/o) or of the oil-in-water (o/w) variety. When an aqueous phase is finely divided into and dispersed as minute droplets into a bulk oily phase the resulting composition is called a water-in-oil (w/o) emulsion. Alternatively, when an oily phase is finely divided into and dispersed as minute droplets into a bulk aqueous phase the resulting composition is called an oil-in-water (o/w) emulsion. Emulsions may contain additional components in addition to the dispersed phases and the active drug which may be present as a solution in either the aqueous phase, oily phase or itself as a separate phase. Pharmaceutical excipients such as emulsifiers, stabilizers, dyes, and anti-oxidants may also be present in emulsions as needed. Pharmaceutical emulsions may also be multiple emulsions that are comprised of more than two phases such as, for example, in the case of oil-in-water-in-oil (o/w/o) and water-in-oil-in-water (w/o/w) emulsions. Such complex formulations often provide certain advantages that simple binary emulsions do not. Multiple emulsions in which individual oil droplets of an o/w emulsion enclose small water droplets constitute a w/o/w emulsion. Likewise a system of oil droplets enclosed in globules of water stabilized in an oily continuous provides an o/w/o emulsion.

Often, the dispersed or discontinuous phase of the emulsion is well dispersed into the external or continuous phase and maintained in this form through the means of emulsifiers or the viscosity of the formulation. Either of the phases of the emulsion may be a semisolid or a solid, as is the case of emulsion-style ointment bases and creams. Other means of stabilizing emulsions entail the use of emulsifiers that may be incorporated into either phase of the emulsion. Emulsifiers may broadly be classified into four categories: synthetic surfactants, naturally occurring emulsifiers, absorption bases, and finely dispersed solids (Idson, in Pharmaceutical Dosage Forms, Lieberman, Rieger and Banker (Eds.), 1988, Marcel Dekker, Inc., New York, N.Y., volume 1, p. 199).

Synthetic surfactants, also known as surface active agents, have found wide applicability in the formulation of emulsions and have been reviewed in the literature (Rieger, in Pharmaceutical Dosage Forms, Lieberman, Rieger and Banker (Eds.), 1988, Marcel Dekker, Inc., New York, N.Y., volume 1, p. 285; Idson, in Pharmaceutical Dosage Forms, Lieberman, Rieger and Banker (Eds.), Marcel Dekker, Inc., New York, N.Y., 1988, volume 1, p. 199). Surfactants are typically amphiphilic and comprise a hydrophilic and a hydrophobic portion. The ratio of the hydrophilic to the hydrophobic nature of the surfactant has been termed the hydrophile/lipophile balance (HLB) and is a valuable tool in categorizing and selecting surfactants in the preparation of formulations. Surfactants may be classified into different classes based on the nature of the hydrophilic group: nonionic, anionic, cationic and amphoteric (Rieger, in Pharmaceutical Dosage Forms, Lieberman, Rieger and Banker (Eds.), 1988, Marcel Dekker, Inc., New York, N.Y., volume 1, p. 285).

Naturally occurring emulsifiers used in emulsion formulations include lanolin, beeswax, phosphatides, lecithin and acacia. Absorption bases possess hydrophilic properties such that they can soak up water to form w/o emulsions yet retain their semisolid consistencies, such as anhydrous lanolin and hydrophilic petrolatum. Finely divided solids have also been used as good emulsifiers especially in combination with surfactants and in viscous preparations. These include polar inorganic solids, such as heavy metal hydroxides, nonswelling clays such as bentonite, attapulgite, hectorite, kaolin, montmorillonite, colloidal aluminum silicate and colloidal magnesium aluminum silicate, pigments and nonpolar solids such as carbon or glyceryl tristearate.

A large variety of non-emulsifying materials are also included in emulsion formulations and contribute to the properties of emulsions. These include fats, oils, waxes, fatty acids, fatty alcohols, fatty esters, humectants, hydrophilic colloids, preservatives and antioxidants (Block, in Pharmaceutical Dosage Forms, Lieberman, Rieger and Banker (Eds.), 1988, Marcel Dekker, Inc., New York, N.Y., volume 1, p. 335; Idson, in Pharmaceutical Dosage Forms, Lieberman, Rieger and Banker (Eds.), 1988, Marcel Dekker, Inc., New York, N.Y., volume 1, p. 199).

Hydrophilic colloids or hydrocolloids include naturally occurring gums and synthetic polymers such as polysaccharides (for example, acacia, agar, alginic acid, carrageenan, guar gum, karaya gum, and tragacanth), cellulose derivatives (for example, carboxymethylcellulose and carboxypropylcellulose), and synthetic polymers (for example, carbomers, cellulose ethers, and carboxyvinyl polymers). These disperse or swell in water to form colloidal solutions that stabilize emulsions by forming strong interfacial films around the dispersed-phase droplets and by increasing the viscosity of the external phase.

Since emulsions often contain a number of ingredients such as carbohydrates, proteins, sterols and phosphatides that may readily support the growth of microbes, these formulations often incorporate preservatives. Commonly used preservatives included in emulsion formulations include methyl paraben, propyl paraben, quaternary ammonium salts, benzalkonium chloride, esters of p-hydroxybenzoic acid, and boric acid. Antioxidants are also commonly added to emulsion formulations to prevent deterioration of the formulation. Antioxidants used may be free radical scavengers such as tocopherols, alkyl gallates, butylated hydroxyanisole, butylated hydroxytoluene, or reducing agents such as ascorbic acid and sodium metabisulfite, and antioxidant synergists such as citric acid, tartaric acid, and lecithin.

The application of emulsion formulations via dermatological, oral and parenteral routes and methods for their manufacture have been reviewed in the literature (Idson, in Pharmaceutical Dosage Forms, Lieberman, Rieger and Banker (Eds.), 1988, Marcel Dekker, Inc., New York, N.Y., volume 1, p. 199). Emulsion formulations for oral delivery have been very widely used because of reasons of ease of formulation, efficacy from an absorption and bioavailability standpoint. (Rosoff, in Pharmaceutical Dosage Forms, Lieberman, Rieger and Banker (Eds.), 1988, Marcel Dekker, Inc., New York, N.Y., volume 1, p. 245; Idson, in Pharmaceutical Dosage Forms, Lieberman, Rieger and Banker (Eds.), 1988, Marcel Dekker, Inc., New York, N.Y., volume 1, p. 199). Mineral-oil base laxatives, oil-soluble vitamins and high fat nutritive preparations are among the materials that have commonly been administered orally as o/w emulsions.

In one embodiment of the present invention, the compositions of oligonucleotides are formulated as microemulsions. A microemulsion may be defined as a system of water, oil and amphiphile which is a single optically isotropic and thermodynamically stable liquid solution (Rosoff, in Pharmaceutical Dosage Forms, Lieberman, Rieger and Banker (Eds.), 1988, Marcel Dekker, Inc., New York, N.Y., volume 1, p. 245). Typically microemulsions are systems that are prepared by first dispersing an oil in an aqueous surfactant solution and then adding a sufficient amount of a fourth component, generally an intermediate chain-length alcohol to form a transparent system. Therefore, microemulsions have also been described as thermodynamically stable, isotropically clear dispersions of two immiscible liquids that are stabilized by interfacial films of surface-active molecules (Leung and Shah, in: Controlled Release of Drugs: Polymers and Aggregate Systems, Rosoff, M., Ed., 1989, VCH Publishers, New York, pages 185-215). Microemulsions commonly are prepared via a combination of three to five components that include oil, water, surfactant, cosurfactant and electrolyte. Whether the microemulsion is of the water-in-oil (w/o) or an oil-in-water (o/w) type is dependent on the properties of the oil and surfactant used and on the structure and geometric packing of the polar heads and hydrocarbon tails of the surfactant molecules (Schott, in Remington's Pharmaceutical Sciences, Mack Publishing Co., Easton, PA, 1985, p. 271).

The phenomenological approach utilizing phase diagrams has been extensively studied and has yielded a comprehensive knowledge, to one skilled in the art, of how to formulate microemulsions (Rosoff, in Pharmaceutical Dosage Forms, Lieberman, Rieger and Banker (Eds.), 1988, Marcel Dekker, Inc., New York, N.Y., volume 1, p. 245; Block, in Pharmaceutical Dosage Forms, Lieberman, Rieger and Banker (Eds.), 1988, Marcel Dekker, Inc., New York, N.Y., volume 1, p. 335). Compared to conventional emulsions, microemulsions offer the advantage of solubilizing water-insoluble drugs in a formulation of thermodynamically stable droplets that are formed spontaneously.

Surfactants used in the preparation of microemulsions include, but are not limited to, ionic surfactants, non-ionic surfactants, Brij 96, polyoxyethylene oleyl ethers, polyglycerol fatty acid esters, tetraglycerol monolaurate (ML310), tetraglycerol monooleate (MO310), hexaglycerol monooleate (PO310), hexaglycerol pentaoleate (PO500), decaglycerol monocaprate (MCA750), decaglycerol monooleate (MO750), decaglycerol sequioleate (SO750), decaglycerol decaoleate (DAO750), alone or in combination with cosurfactants. The cosurfactant, usually a short-chain alcohol such as ethanol, 1-propanol, and 1-butanol, serves to increase the interfacial fluidity by penetrating into the surfactant film and consequently creating a disordered film because of the void space generated among surfactant molecules. Microemulsions may, however, be prepared without the use of cosurfactants and alcohol-free self-emulsifying microemulsion systems are known in the art. The aqueous phase may typically be, but is not limited to, water, an aqueous solution of the drug, glycerol, PEG300, PEG400, polyglycerols, propylene glycols, and derivatives of ethylene glycol. The oil phase may include, but is not limited to, materials such as Captex 300, Captex 355, Capmul MCM, fatty acid esters, medium chain (C8-C12) mono, di, and tri-glycerides, polyoxyethylated glyceryl fatty acid esters, fatty alcohols, polyglycolized glycerides, saturated polyglycolized C8-C10 glycerides, vegetable oils and silicone oil.

Microemulsions are particularly of interest from the standpoint of drug solubilization and the enhanced absorption of drugs. Lipid based microemulsions (both o/w and w/o) have been proposed to enhance the oral bioavailability of drugs, including peptides (Constantinides et al., Pharmaceutical Research, 1994, 11, 1385-1390; Ritschel, Meth. Find. Exp. Clin. Pharmacol., 1993, 13, 205). Microemulsions afford advantages of improved drug solubilization, protection of drug from enzymatic hydrolysis, possible enhancement of drug absorption due to surfactant-induced alterations in membrane fluidity and permeability, ease of preparation, ease of oral administration over solid dosage forms, improved clinical

potency, and decreased toxicity (Constantinides et al., Pharmaceutical Research, 1994, 11, 1385; Ho et al., J. Pharm. Sci., 1996, 85, 138-143). Often microemulsions may form spontaneously when their components are brought together at ambient temperature. This may be particularly advantageous when formulating thermolabile drugs, peptides or oligonucleotides. Microemulsions have also been effective in the transdermal delivery of active components in both cosmetic and pharmaceutical applications. It is expected that the microemulsion compositions and formulations of the present invention will facilitate the increased systemic absorption of oligonucleotides from the gastrointestinal tract, as well as improve the local cellular uptake of oligonucleotides within the gastrointestinal tract, vagina, buccal cavity and other areas of administration.

Microemulsions of the present invention may also contain additional components and additives such as sorbitan monostearate (Grill 3), Labrasol, and penetration enhancers to improve the properties of the formulation and to enhance the absorption of the oligonucleotides and nucleic acids of the present invention. Penetration enhancers used in the microemulsions of the present invention may be classified as belonging to one of five broad categories - surfactants, fatty acids, bile salts, chelating agents, and non-chelating non-surfactants (Lee et al., Critical Reviews in Therapeutic Drug Carrier Systems, 1991, p. 92). Each of these classes has been discussed above.

**Liposomes**

There are many organized surfactant structures besides microemulsions that have been studied and used in the formulation of drugs. These include monolayers, micelles, bilayers and vesicles. Vesicles, such as liposomes, have attracted great interest because of their specificity and the duration of action they offer from the standpoint of drug delivery. As used in the present invention, the term "liposome" means a vesicle composed of amphiphilic lipids arranged in a spherical bilayer or bilayers.

Liposomes are unilamellar or multilamellar vesicles which have a membrane formed from a lipophilic material and an aqueous interior. The aqueous portion contains the composition to be delivered. Cationic liposomes possess the advantage of being able to fuse to the cell wall. Non-cationic liposomes, although not able to fuse as efficiently with the cell wall, are taken up by macrophages *in vivo.*

In order to cross intact mammalian skin, lipid vesicles must pass through a series of fine pores, each with a diameter less than 50 nm, under the influence of a suitable transdermal gradient. Therefore, it is desirable to use a liposome which is highly deformable and able to pass through such fine pores.

Further advantages of liposomes include; liposomes obtained from natural phospholipids are biocompatible and biodegradable; liposomes can incorporate a wide range of water and lipid soluble drugs; liposomes can protect encapsulated drugs in their internal compartments from metabolism and degradation (Rosoff, in Pharmaceutical Dosage Forms, Lieberman, Rieger and Banker (Eds.), 1988, Marcel Dekker, Inc., New York, N.Y., volume 1, p. 245). Important considerations in the preparation of liposome formulations are the lipid surface charge, vesicle size and the aqueous volume of the liposomes.

Liposomes are useful for the transfer and delivery of active ingredients to the site of action. Because the liposomal membrane is structurally similar to biological membranes, when liposomes are applied to a tissue, the liposomes start to merge with the cellular membranes. As the merging of the liposome and cell progresses, the liposomal contents are emptied into the cell where the active agent may act.

Liposomal formulations have been the focus of extensive investigation as the mode of delivery for many drugs. There is growing evidence that for topical administration, liposomes present several advantages over other formulations. Such advantages include reduced side-effects related to high systemic absorption of the administered drug, increased accumulation of the administered drug at the desired target, and the ability to administer a wide variety of drugs, both hydrophilic and hydrophobic, into the skin.

Several reports have detailed the ability of liposomes to deliver agents including high-molecular weight DNA into the skin. Compounds including analgesics, antibodies, hormones and high-molecular weight DNAs have been administered to the skin. The majority of applications resulted in the targeting of the upper epidermis.

Liposomes fall into two broad classes and are useful for the delivery of DNA, RNA or any nucleic acid-based construct. Cationic liposomes are positively charged liposomes which interact with negatively charged DNA molecules to form a stable complex. The positively charged DNA/liposome complex binds to the negatively charged cell surface and is internalized in an endosome. Due to the acidic pH within the endosome, the liposomes are ruptured, releasing their contents into the cell cytoplasm (Wang et al., Biochem. Biophys. Res. Commun., 1987, 147, 980-985).

Liposomes which are pH-sensitive or negatively-charged, entrap DNA rather than complex with it. Since both the DNA and the lipid are similarly charged, repulsion rather than complex formation occurs. Nevertheless, some DNA is entrapped within the aqueous interior of these liposomes. pH-sensitive liposomes have been used to deliver DNA encoding the thymidine kinase gene to cell monolayers in culture. Expression of the exogenous gene was detected in the target cells (Zhou et al., Journal of Controlled Release, 1992, 19, 269-274).

One major type of liposomal composition includes phospholipids other than naturally-derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Several studies have assessed the topical delivery of liposomal drug formulations to the skin. Application of liposomes containing interferon to guinea pig skin resulted in a reduction of skin herpes sores while delivery of interferon via other means (*e.g.* as a solution or as an emulsion) were ineffective (Weiner et al., Journal of Drug Targeting, 1992, 2, 405-410). Further, an additional study tested the efficacy of interferon administered as part of a liposomal formulation to the administration of interferon using an aqueous system, and concluded that the liposomal formulation was superior to aqueous administration (du Plessis et al., Antiviral Research, 1992, 18, 259-265).

Non-ionic liposomal systems have also been examined to determine their utility in the delivery of drugs to the skin, in particular systems comprising non-ionic surfactant and cholesterol. Non-ionic liposomal formulations comprising Novasome™ I (glyceryl dilaurate/cholesterol/polyoxyethylene-10-stearyl ether) and Novasome™ II (glyceryl distearate/cholesterol/polyoxyethylene-10-stearyl ether) were used to deliver cyclosporin-A into the dermis of mouse skin. Results indicated that such non-ionic liposomal systems were effective in facilitating the deposition of cyclosporin-A into different layers of the skin (Hu et al. S.T.P.Pharma. Sci., 1994, 4, 6, 466).

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome (A) comprises one or more glycolipids, such as monosialoganglioside $G_{M1}$, or (B) is derivatized with one or more hydrophilic polymers, such as a polyethylene glycol (PEG) moiety. While not wishing to be bound by any particular theory, it is thought in the art that, at least for sterically stabilized liposomes containing gangliosides, sphingomyelin, or PEG-derivatized lipids, the enhanced circulation half-life of these sterically stabilized liposomes derives from a reduced uptake into cells of the reticuloendothelial system (RES) (Allen et al., FEBS Letters, 1987, 223, 42; Wu et al., Cancer Research, 1993, 53, 3765).

Various liposomes comprising one or more glycolipids are known in the art. Papahadjopoulos et al. (Ann. N.Y. Acad. Sci., 1987, 507, 64) reported the ability of monosialoganglioside $G_{M1}$, galactocerebroside sulfate and phosphatidylinositol to improve blood half-lives of liposomes. These findings were expounded upon by Gabizon et al. (Proc. Natl. Acad. Sci . U. S.A., 1988, 85, 6949). U.S. Patent No. 4,837,028 and WO 88/04924, both to Allen *et al.*, disclose liposomes comprising (1) sphingomyelin and (2) the ganglioside $G_{M1}$ or a galactocerebroside sulfate ester. U.S. Patent No. 5,543,152 (Webb et al.) discloses liposomes comprising sphingomyelin. Liposomes comprising 1,2-*sn*-dimyristoylphosphatidylcholine are disclosed in WO 97/13499 (Lim et al.).

Many liposomes comprising lipids derivatized with one or more hydrophilic polymers, and methods of preparation thereof, are known in the art. Sunamoto et al. (Bull. Chem. Soc. Jpn., 1980, 53, 2778) described liposomes comprising a nonionic detergent, $2C_{12}15G$, that contains a PEG moiety. Illum et al. (FEBS Lett., 1984, 167, 79) noted that hydrophilic coating of polystyrene particles with polymeric glycols results in significantly enhanced blood half-lives. Synthetic phospholipids modified by the attachment of carboxylic groups of polyalkylene glycols (*e.g.,* PEG) are described by Sears (U.S. Patent Nos. 4,426,330 and 4,534,899). Klibanov et al. (FEBS Lett., 1990, 268, 235) described experiments demonstrating that liposomes comprising phosphatidylethanolamine (PE) derivatized with PEG or PEG stearate have significant increases in blood circulation half-lives. Blume et al. (Biochimica et Biophysica Acta, 1990, 1029, 91) extended such observations to other PEG-derivatized phospholipids, *e.g.*, DSPE-PEG, formed from the combination of distearoylphosphatidylethanolamine (DSPE) and PEG. Liposomes having covalently bound PEG moieties on their external surface are described in European Patent No. EP 0 445 131 B1 and WO 90/04384 to Fisher. Liposome compositions containing 1-20 mole percent of PE derivatized with PEG, and methods of use thereof, are described by Woodle et al. (U.S. Patent Nos. 5,013,556 and 5,356,633) and Martin et al. (U.S. Patent No. 5,213,804 and European Patent No. EP 0 496 813 B1). Liposomes comprising a number of other lipid-polymer conjugates are disclosed in WO 91/05545 and U.S. Patent No. 5,225,212 (both to Martin *et al.*) and in WO 94/20073 (Zalipsky et al.) Liposomes comprising PEG-modified ceramide lipids are described in WO 96/10391 (Choi et al.). U.S. Patent Nos. 5,540,935 (Miyazaki et al.) and 5,556,948 (Tagawa et al.) describe PEG-containing liposomes that can be further derivatized with functional moieties on their surfaces.

WO 96/40062 to Thierry et al. discloses methods for encapsulating high molecular weight nucleic acids in liposomes. U.S. Patent No. 5,264,221 to Tagawa et al. discloses protein-bonded liposomes and asserts that the contents of such liposomes may include an antisense RNA. U.S. Patent No. 5,665,710 to Rahman et al. describes certain methods of encapsulating oligodeoxynucleotides in liposomes. WO 97/04787 to Love et al. discloses liposomes comprising antisense oligonucleotides targeted to the raf gene.

Transfersomes are yet another type of liposomes, and are highly deformable lipid aggregates which are attractive candidates for drug delivery vehicles. Transfersomes may be described as lipid droplets which are so highly deformable that they are easily able to penetrate through pores which are smaller than the droplet. Transfersomes are adaptable

to the environment in which they are used, *e.g.* they are self-optimizing (adaptive to the shape of pores in the skin), self-repairing, frequently reach their targets without fragmenting, and often self-loading. To make transfersomes it is possible to add surface edge-activators, usually surfactants, to a standard liposomal composition. Transfersomes have been used to deliver serum albumin to the skin. The transfersome-mediated delivery of serum albumin has been shown to be as effective as subcutaneous injection of a solution containing serum albumin.

Surfactants find wide application in formulations such as emulsions (including microemulsions) and liposomes. The most common way of classifying and ranking the properties of the many different types of surfactants, both natural and synthetic, is by the use of the hydrophile/lipophile balance (HLB). The nature of the hydrophilic group (also known as the "head") provides the most useful means for categorizing the different surfactants used in formulations (Rieger, in Pharmaceutical Dosage Forms, Marcel Decker, Inc., New York, NY, 1988, p. 285).

If the surfactant molecule is not ionized, it is classified as a nonionic surfactant. Nonionic surfactants find wide application in pharmaceutical and cosmetic products and are usable over a wide range of pH values. In general their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic. surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides.

The use of surfactants in drug products, formulations and in emulsions has been reviewed (Rieger, in Pharmaceutical Dosage Forms, Marcel Dekker, Inc., New York, NY, 1988, p. 285).

Pharmaceutically acceptable organic or inorganic excipient suitable for non-parenteral administration which do not deleteriously react with nucleic acids can also be used to formulate the compositions of the present invention. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like.

Formulations for topical administration of nucleic acids may include sterile and non-sterile aqueous solutions, non-aqueous solutions in common solvents such as alcohols, or solutions of the nucleic acids in liquid or solid oil bases. The solutions may also contain buffers, diluents and other suitable additives. Pharmaceutically acceptable organic or inorganic excipients suitable for non-parenteral administration which do not deleteriously react with nucleic acids can be used.

Suitable pharmaceutically acceptable excipients include, but are not limited to, water, salt solutions, alcohol, polyethylene glycols, gelatin, lactose, amylose, magnesium stearate, talc, silicic acid, viscous paraffin, hydroxymethylcellulose, polyvinylpyrrolidone and the like. Pulsatile Delivery

The compounds of the present invention may also be administered by pulsatile delivery. "Pulsatile delivery" refers to a pharmaceutical formulation that delivers a first pulse of drug (e.g. an antisense compound) combined with a penetration enhancer and a second pulse of penetration enhancer to promote absorption of drug which is not absorbed upon release with the first pulse of penetration enhancer.

One embodiment of the present invention is a delayed release oral formulation for enhanced intestinal drug absorption, comprising:

(a) a first population of carrier particles comprising said drug and a penetration enhancer, wherein said drug and said penetration enhancer are released at a first location in the intestine; and

(b) a second population of carrier particles comprising a penetration enhancer and a (delayed release coating or matrix, wherein the penetration enhancer is released at a second location in the intestine downstream from the first location, whereby absorption of the drug is enhanced when the drug reaches the second location.

Alternatively, the penetration enhancer in (a) and (b) is different.

This enhancement is obtained by encapsulating at least two populations of carrier particles. The first population

of carrier particles comprises a biologically active substance and a penetration enhancer, and the second (and optionally additional) population of carrier particles comprises a penetration enhancer and a delayed release coating or matrix.

A "first pass effect" that applies to orally administered drugs is degradation due to the action of gastric acid and various digestive enzymes. One means of ameliorating first pass clearance effects is to increase the dose of administered drug, thereby compensating for proportion of drug lost to first pass clearance. Although this may be readily achieved with i.v. administration by, for example, simply providing more of the drug to an animal, other factors influence the bioavailability of drugs administered via non-parenteral means. For example, a drug may be enzymatically or chemically degraded in the alimentary canal or blood stream and/or may be impermeable or semipermeable to various mucosal membranes.

It is also contemplated that these pharmacutical compositons are capable of enhancing absorption of biologically active substances when administered via the rectal, vaginal, nasal or pulmonary routes. It is also contemplated that release of the biologically active substance can be achieved in any part of the gastrointestinal tract.

Liquid pharmaceutical compositions of oligonucleotide can be prepared by combining the oligonucleotide with a suitable vehicle, for example sterile pyrogen free water, or saline solution. Other therapeutic compounds may optionally be included.

The present invention also contemplates the use of solid particulate compositions. Such compositions preferably comprise particles of oligonucleotide that are of respirable size. Such particles can be prepared by, for example, grinding dry oligonucleotide by conventional means, fore example with a mortar and pestle, and then passing the resulting powder composition through a 400 mesh screen to segregate large particles and agglomerates. A solid particulate composition comprised of an active oligonucleotide can optionally contain a dispersant which serves to facilitate the formation of an aerosol, for example lactose.

In accordance with the present invention, oligonucleotide compositions can be aerosolized. Aerosolization of liquid particles can be produced by any suitable means, such as with a nebulizer. See, for example, U.S. Patent No. 4,501,729. Nebulizers are commercially available devices which transform solutions or suspensions into a therapeutic aerosol mist either by means of acceleration of a compressed gas, typically air or oxygen, through a narrow venturi orifice or by means of ultrasonic agitation. Suitable nebulizers include those sold by Blairex® under the name PARI LC PLUS, PARI DURA-NEB 2000, PARI-BABY Size, PARI PRONEB Compressor with LC PLUS, PARI WALKHALER Compressor/Nebulizer System, PARI LC PLUS Reusable Nebulizer, and PARI LC Jet+ ®Nebulizer.

Formulations for use in nebulizers may consist of an oligonucleotide in a liquid, such as sterile, pyragen free water, or saline solution, wherein the oligonucleotide comprises up to about 40% w/w of the formulation. Preferably, the oligonucleotide comprises less than 20% w/w. If desired, further additives such as preservatives (for example, methyl hydroxybenzoate) antioxidants, and flavoring agents can be added to the composition. Solid particles comprising an oligonucleotide can also be aerosolized using any solid particulate medicament 5 aerosol generator known in the art. Such aerosol generators produce respirable particles, as described above, and further produce reproducible metered dose per unit volume of aerosol. Suitable solid particulate aerosol generators include insufflators and metered dose 10 inhalers. Metered dose inhalers are used in the art and are useful in the present invention.

Preferably, liquid or solid aerosols are produced at a rate of from about 10 to 150 liters per minute, more preferably from about 30 to 150 liters per minute, and 15 most preferably about 60 liters per minute.

Enhanced bioavailability of biologically active substances is also achieved via the oral administration of the compositions of the present invention. Other Components

20 The compositions of the present invention may additionally contain other adjunct components conventionally found in pharmaceutical compositions, at their art-established usage levels. Thus, for example, the compositions may contain additional, compatible, 25 pharmaceutically-active materials such as, for example, antipruritics, astringents, local anesthetics or anti-inflammatory agents, or may contain additional materials useful in physically formulating various dosage forms of the compositions of the present invention, such as dyes, 30 flavoring agents, preservatives, antioxidants, opacifiers, thickening agents and stabilizers. However, such materials, when added, should not unduly interfere with the biological activities of the components of the compositions of the present invention. The formulations can be sterilized and, if desired, mixed with auxiliary agents, *e.g.,* lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Aqueous suspensions may contain substances which increase the viscosity of the suspension including, for example, sodium carboxymethylcellulose, sorbitol and/or dextran. The suspension may also contain stabilizers.

Certain embodiments of the invention provide pharmaceutical compositions containing (a) one or more antisense compounds and (b) one or more other chemotherapeutic agents which function by a non-antisense mechanism. Examples of such chemotherapeutic agents include but are not limited to daunorubicin, daunomycin, dactinomycin, doxorubicin, epirubicin, idarubicin, esorubicin, bleomycin, mafosfamide, ifosfamide, cytosine arabinoside, bis-chloroethylnitrosurea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitoxantrone, amsacrine, chlorambucil, methylcyclohexylnitrosurea, nitrogen mustards, melphalan, cyclophosphamide, 6-mercapt opurine, 6-thioguanine, cytarabine, 5-azacytidine, hydroxyurea, deoxycoformycin, 4-hydroxyperoxycyclophosphoramide, 5-fluorouracil (5-FU), 5-fluorodeoxyuridine (5-FUdR), methotrexate (MTX), colchicine, taxol, vincristine, vinblastine, etoposide (VP-16), trimetrexate, irinotecan, topotecan, gemcitabine, teniposide, cisplatin and diethylstilbestrol (DES). See, generally, The Merck Manual of Diagnosis and Therapy, 15th Ed. 1987, pp. 1206-1228, Berkow et al., eds., Rahway, N.J.

When used with the compounds of the invention, such chemotherapeutic agents may be used individually (e.g., 5-FU and oligonucleotide), sequentially (e.g., 5-FU and oligonucleotide for a period of time followed by MTX and oligonucleotide), or in combination with one or more other such chemotherapeutic agents (*e.g.*, 5-FU, MTX and oligonucleotide, or 5-FU, radiotherapy and oligonucleotide). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the invention. See, generally, The Merck Manual of Diagnosis and Therapy, 15th Ed., Berkow et al., eds., 1987, Rahway, N.J., pages 2499-2506 and 46-49, respectively). Other non-antisense chemotherapeutic agents are also within the scope of this invention. Two or more combined compounds may be used together or sequentially.

In another related embodiment, compositions of the invention may contain one or more antisense compounds, particularly oligonucleotides, targeted to a first nucleic acid and one or more additional antisense compounds targeted to a second nucleic acid target. Two or more antisense compounds may be used together or sequentially.

**Dosing**

Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skill in the art can easily determine optimum dosages, dosing methodologies and repetition rates.

The following examples illustrate the present invention and are not intended to limit the same.

**EXAMPLES**

**EXAMPLE 1: Synthesis of oligonucleotides**

Unmodified oligodeoxynucleotides are synthesized on an automated DNA synthesizer (Applied Biosystems model 380B) using standard phosphoramidite chemistry with oxidation by iodine. ß-cyanoethyldiisopropyl-phosphoramidites are purchased from Applied Biosystems (Foster City, CA). For phosphorothioate oligonucleotides, the standard oxidation bottle was replaced by a 0.2 M solution of $^{3}$H-1,2-benzodithiole-3-one 1,1-dioxide in acetonitrile for the stepwise thiation of the phosphite linkages. The thiation cycle wait step was increased to 68 seconds and was followed by the capping step. Cytosines may be 5-methyl cytosines. (5-methyl deoxycytidine phosphoramidites available from Glen Research, Sterling, VA or Amersham Pharmacia Biotech, Piscataway, NJ)

2'-methoxy oligonucleotides are synthesized using 2'-methoxy ß-cyanoethyldiisopropyl-phosphoramidites (Chemgenes, Needham, MA) and the standard cycle for unmodified oligonucleotides, except the wait step after pulse delivery of tetrazole and base is increased to 360 seconds. Other 2'-alkoxy oligonucleotides are synthesized by a modification of this method, using appropriate 2'-modified amidites such as those available from Glen Research, Inc., Sterling, VA.

2'-fluoro oligonucleotides are synthesized as described in Kawasaki et al. (J. Med. Chem. 1993, 36, 831-841). Briefly, the protected nucleoside $N^6$-benzoyl-2'-deoxy-2'-fluoroadenosine is synthesized utilizing commercially available 9-ß-D-arabinofuranosyladenine as starting material and by modifying literature procedures whereby the 2'-a-fluoro atom is introduced by a $S_N2$-displacement of a 2'-ß-O-trifyl group. Thus $N^6$-benzoyl-9-ß-D-arabinofuranosyladenine is selectively protected in moderate yield as the 3',5'-ditetrahydropyranyl (THP) intermediate. Deprotection of the THP and $N^6$-benzoyl groups is accomplished using standard methodologies and standard methods are used to obtain the 5'-dimethoxytrityl- (DMT) and 5'-DMT-3'-phosphoramidite intermediates.

The synthesis of 2'-deoxy-2'-fluoroguanosine is accomplished using tetraisopropyldisiloxanyl (TPDS) protected 9-ß-D-arabinofuranosylguanine as starting material, and conversion to the intermediate diisobutyryl-arabinofuranosylguanosine. Deprotection of the TPDS group is followed by protection of the hydroxyl group with THP to give diisobutyryl di-THP protected arabinofuranosylguanine. Selective O-deacylation and triflation is followed by treatment of the crude product with fluoride, then deprotection of the THP groups. Standard methodologies are used to obtain the 5'-DMT- and 5'-DMT-3'-phosphoramidites.

Synthesis of 2'-deoxy-2'-fluorouridine is accomplished by the modification of a known procedure in which 2, $2^1$-anhydro-1-ß-D-arabinofuranosyluracil is treated with 70% hydrogen fluoride-pyridine. Standard procedures are used

to obtain.the 5'-DMT and 5'-DMT-3'phosphoramidites.

2'-deoxy-2'-fluorocytidine is synthesized via amination of 2'-deoxy-2'-fluorouridine, followed by selective protection to give $N^4$-benzoyl-2'-deoxy-2'-fluorocytidine. Standard procedures are used to obtain the 5'-DMT and 5'-DMT-3'phosphoramidites.

2'-(2-methoxyethyl)-modified amidites were synthesized according to Martin, P. (Helv. Chim. Acta 1995, 78, 486-506). For ease of synthesis, the last nucleotide may be a deoxynucleotide. 2 '-O-$CH_2CH_2OCH_{3-}$ cytosines may be 5-methyl cytosines.

*Synthesis of 5-Methyl cytosine monomers:*

2,2'-Anhydro[1-(ß-D-arabinofuranosyl)-5-methyluridine] :

5-Methyluridine (ribosylthymine, commercially available through Yamasa, Choshi, Japan) (72.0 g, 0.279 M), diphenylcarbonate (90.0 g, 0.420 M) and sodium bicarbonate (2.0 g, 0.024 M) were added to DMF (300 mL). The mixture was heated to reflux, with stirring, allowing the evolved carbon dioxide gas to be released in a controlled manner. After 1 hour, the slightly darkened solution was concentrated under reduced pressure. The resulting syrup was poured into diethylether (2.5 L), with stirring. The product formed a gum. The ether was decanted and the residue was dissolved in a minimum amount of methanol (ca. 400 mL). The solution was poured into fresh ether (2.5 L) to yield a stiff gum. The ether was decanted and the gum was dried in a vacuum oven (60°C at 1 mm Hg for 24 h) to give a solid which was crushed to a light tan powder (57 g, 85% crude yield). The material was used as is for further reactions.

2'-O-Methoxyethyl-5-methyluridine:

2,2'-Anhydro-5-methyluridine (195 g, 0.81 M), tris(2-methoxyethyl)borate (231 g, 0.98 M) and 2-methoxyethanol (1.2 L) were added to a 2 L stainless steel pressure vessel and placed in a pre-heated oil bath at 160°C. After heating for 48 hours at 155-160°C, the vessel was opened and the solution evaporated to dryness and triturated with MeOH (200 mL). The residue was suspended in hot acetone (1 L). The insoluble salts were filtered, washed with acetone (150 mL) and the filtrate evaporated. The residue (280 g) was dissolved in $CH_3CN$ (600 mL) and evaporated. A silica gel column (3 kg) was packed in $CH_2Cl_2$/acetone/MeOH (20:5:3) containing 0.5% $Et_3NH$. The residue was dissolved in $CH_2Cl_2$ (250 mL) and adsorbed onto silica (150 g) prior to loading onto the column. The product was eluted with the packing solvent to give 160 g (63%) of product.

2'-O-Methoxyethyl-5'-O-dimethoxytrityl-5-methyluridine:

2'-O-Methoxyethyl-5-methyluridine (160 g, 0.506 M) was co-evaporated with pyridine (250 mL) and the dried residue dissolved in pyridine (1.3 L). A first aliquot of dimethoxytrityl chloride (94.3 g, 0.278 M) was added and the mixture stirred at room temperature for one hour. A second aliquot of dimethoxytrityl chloride (94.3 g, 0.278 M) was added and the reaction stirred for an additional one hour. Methanol (170 mL) was then added to stop the reaction. HPLC showed the presence of approximately 70% product. The solvent was evaporated and triturated with $CH_3CN$ (200 mL). The residue was dissolved in $CHCl_3$ (1.5 L) and extracted with 2x500 mL of saturated $NaHCO_3$ and 2x500 mL of saturated NaCl. The organic phase was dried over $Na_2SO_4$, filtered and evaporated. 275 g of residue was obtained. The residue was purified on a 3.5 kg silica gel column, packed and eluted with EtOAc/Hexane/Acetone (5:5:1) containing 0.5% $Et_3NH$. The pure fractions were evaporated to give 164 g of product. Approximately 20 g additional was obtained from the impure fractions to give a total yield of 183 g (57%).

3'-O-Acetyl-2'-O-methoxyethyl-5'-O-dimethoxytrityl-5-methyluridine:

2'-O-Methoxyethyl-5'-O-dimethoxytrityl-5-methyl-uridine (106 g, 0.167 M), DMF/pyridine (750 mL of a 3:1 mixture prepared from 562 mL of DMF and 188 mL of pyridine) and acetic anhydride (24.38 mL, 0.258 M) were combined and stirred at room temperature for 24 hours. The reaction was monitored by tlc by first quenching the tlc sample with the addition of MeOH. Upon completion of the reaction, as judged by tlc, MeOH (50 mL) was added and the mixture evaporated at 35°C. The residue was dissolved in $CHCl_3$ (800 mL) and extracted with 2x200 mL of saturated sodium bicarbonate and 2x200 mL of saturated NaCl. The water layers were back extracted with 200 mL of $CHCl_3$. The combined organics were dried with sodium sulfate and evaporated to give 122 g of residue (approx. 90% product). The residue was purified on a 3.5 kg silica gel column and eluted using EtOAc/Hexane(4:1). Pure product fractions were evaporated to yield 96 g (84%).

3'-O-Acetyl-2'-O-methoxyethyl-5'-O-dimethoxytrityl-5-methyl-4-triazoleuridine:

A first solution was prepared by dissolving 3'-O-acetyl-2'-O-methoxyethyl-5'-O-dimethoxytrityl-5-methyluridine (96 g, 0.144 M) in $CH_3CN$ (700 mL) and set aside. Triethylamine (189 mL, 1.44 M) was added to a solution of triazole (90 g, 1.3 M) in $CH_3CN$ (1 L), cooled to -5°C and stirred for 0.5 h using an overhead stirrer. $POCl_3$ was added dropwise, over a 30 minute period, to the stirred solution maintained at 0-10°C, and the resulting mixture stirred for an additional 2 hours. The first solution was added dropwise, over a 45 minute period, to the later solution. The resulting reaction mixture was stored overnight in a cold room. Salts were filtered from the reaction mixture and the solution was evaporated. The residue was dissolved in EtOAc (1 L) and the insoluble solids were removed by filtration. The filtrate was washed with 1x300 mL of $NaHCO_3$ and 2x300 mL of saturated NaCl, dried over sodium sulfate and evaporated. The residue was triturated with EtOAc to give the title compound.

2'-O-Methoxyethyl-5'-O-dimethoxytrityl-5-methylcytidine:

A solution of 3'-O-acetyl-2'-O-methoxyethyl-5'-O-dimethoxytrityl-5-methyl-4-triazoleuridine (103 g, 0.141 M) in dioxane (500 mL) and $NH_4OH$ (30 mL) was stirred at room temperature for 2 hours. The dioxane solution was evaporated and the residue azeotroped with MeOH (2x200 mL). The residue was dissolved in MeOH (300 mL) and transferred to a 2 liter stainless steel pressure vessel. MeOH (400 mL) saturated with $NH_3$ gas was added and the vessel heated to 100°C for 2 hours (tlc showed complete conversion). The vessel contents were evaporated to dryness and the residue was dissolved in EtOAc (500 mL) and washed once with saturated NaCl (200 mL). The organics were dried over sodium sulfate and the solvent was evaporated to give 85 g (95%) of the title compound.

$N^4$-Benzoyl-2'-O-methoxyethyl-5'-O-dimethoxytrityl-5-methylcytidine:

2'-O-Methoxyethyl-5'-O-dimethoxytrityl-5-methyl-cytidine (85 g, 0.134 M) was dissolved in DMF (800 mL) and benzoic anhydride (37.2 g, 0.165 M) was added with stirring. After stirring for 3 hours, tlc showed the reaction to be approximately 95% complete. The solvent was evaporated and the residue azeotroped with MeOH (200 mL). The residue was dissolved in $CHCl_3$ (700 mL) and extracted with saturated $NaHCO_3$ (2x300 mL) and saturated NaCl (2x300 mL), dried over $MgSO_4$ and evaporated to give a residue
(96 g). The residue was chromatographed on a 1.5 kg silica column using EtOAc/Hexane (1:1) containing 0.5% $Et_3NH$ as the eluting solvent. The pure product fractions were evaporated to give 90 g (90%) of the title compound.

$N^4$-Benzoyl-2'-O-methoxyethyl-5'-O-dimethoxytrityl-5-methylcytidine-3'-amidite:

$N^4$-Benzoyl-2'-O-methoxyethyl-5'-O-dimethoxytrityl-5-methylcytidine (74 g, 0.10 M) was dissolved in $CH_2Cl_2$ (1 L). Tetrazole diisopropylamine (7.1 g) and 2-cyanoethoxy-tetra(isopropyl)phosphite (40.5 mL, 0.123 M) were added with stirring, under a nitrogen atmosphere. The resulting mixture was stirred for 20 hours at room temperature (tlc showed the reaction to be 95% complete). The reaction mixture was extracted with saturated $NaHCO_3$ (1x300 mL) and saturated NaCl (3x300 mL). The aqueous washes were back-extracted with $CH_2Cl_2$ (300 mL), and the extracts were combined, dried over $MgSO_4$ and concentrated. The residue obtained was chromatographed on a 1.5 kg silica column using EtOAc\Hexane (3:1) as the eluting solvent. The pure fractions were combined to give 90.6 g (87%) of the title compound.

5-methyl-2'-deoxycytidine (5-me-C) containing oligonucleotides were synthesized according to published methods (Sanghvi et al., Nucl. Acids Res. 1993, 21, 3197-3203) using commercially available phosphoramidites (Glen Research, Sterling VA or ChemGenes, Needham MA). 2'-O-(dimethylaminooxyethyl) nucleoside amidites

2'-(Dimethylaminooxyethoxy) nucleoside amidites [also known in the art as 2'-O-(dimethylaminooxyethyl) nucleoside amidites] are prepared as described in the following paragraphs. Adenosine, cytidine and guanosine nucleoside amidites are prepared similarly to the thymidine (5-methyluridine) except the exocyclic amines are protected with a benzoyl moiety in the case of adenosine and cytidine and with isobutyryl in the case of guanosine.

5'-O-tert-Butyldiphenylsilyl-$O^2$-2'-anhydro-5-methyluridine

$O^2$-2'-anhydro-5-methyluridine (Pro. Bio. Sint., Varese, Italy, 100.0g, 0.416 mmol), dimethylaminopyridine (0.66g, 0.013eq, 0.0054mmol) were dissolved in dry pyridine (500 ml) at ambient temperature under an argon atmosphere and with mechanical stirring. *tert*-Butyldiphenyl chlorosilane (125.8g, 119.0mL, 1.1eq, 0.458mmol) was added in one portion. The reaction was stirred for 16 h at ambient temperature. TLC (Rf 0.22, ethyl acetate) indicated a complete reaction. The solution was concentrated under reduced pressure to a thick oil. This was partitioned between dichloromethane (1 L) and saturated sodium bicarbonate (2x1 L) and brine (1 L). The organic layer was dried over sodium sulfate and concentrated under reduced pressure to a thick oil. The oil was dissolved in a 1:1 mixture of ethyl acetate

and ethyl ether (600mL) and the solution was cooled to-10°C. The resulting crystalline product was collected by filtration, washed with ethyl ether (3x200 mL) and dried (40°C, 1mm Hg, 24 h) to 149g (74.8%) of white solid. TLC and NMR were consistent with pure product.

5'-O-tert-Butyldiphenylsilyl-2'-O-(2-hydroxyethyl)-5-methyluridine

In a 2 L stainless steel, unstirred pressure reactor was added borane in tetrahydrofuran (1.0 M, 2.0 eq, 622 mL). In the fume hood and with manual stirring, ethylene glycol (350 mL, excess) was added cautiously at first until the evolution of hydrogen gas subsided. 5'-O-tert-Butyldiphenylsilyl-$O^2$-2'-anhydro-5-methyluridine (149 g, 0.311 mol) and sodium bicarbonate (0.074 g, 0.003 eq) were added with manual stirring. The reactor was sealed and heated in an oil bath until an internal temperature of 160 °C was reached and then maintained for 16 h (pressure < 100 psig). The reaction vessel was cooled to ambient and opened. TLC (Rf 0.67 for desired product and Rf 0.82 for ara-T side product, ethyl acetate) indicated about 70% conversion to the product. In order to avoid additional side product formation, the reaction was stopped, concentrated under reduced pressure (10 to 1mm Hg) in a warm water bath (40-100°C) with the more extreme conditions used to remove the ethylene glycol.

[Alternatively, once the low boiling solvent is gone, the remaining solution can be partitioned between ethyl acetate and water. The product will be in the organic phase.] The residue was purified by column chromatography (2kg silica gel, ethyl acetate-hexanes gradient 1:1 to 4:1). The appropriate fractions were combined, stripped and dried to product as a white crisp foam (84g, 50%), contaminated starting material (17.4g) and pure reusable starting material 20 g. The yield based on starting material less pure recovered starting material was 58%. TLC and NMR were consistent with 99% pure product.

2'-O-([2-phthalimidoxy)ethyl]-5'-t-butyldiphenylsilyl-5-methyluridine

5'-O-tert-Butyldiphenylsilyl-2'-O-(2-hydroxyethyl)-5-methyluridine (20g, 36.98mmol) was mixed with triphenylphosphine (11.63g, 44.36mmol) and N-hydroxyphthalimide (7.24g, 44.36mmol). It was then dried over $P_2O_5$ under high vacuum for two days at 40°C. The reaction mixture was flushed with argon and dry THF (369 mL, Aldrich, sure seal bottle) was added to get a clear solution. Diethyl-azodicarboxylate (6.98mL, 44.36mmol) was added dropwise to the reaction mixture. The rate of addition is maintained such that resulting deep red coloration is just discharged before adding the next drop. After the addition was complete, the reaction was stirred for 4 hrs. By that time TLC showed the completion of the reaction (ethylacetate:hexane, 60:40). The solvent was evaporated in vacuum. Residue obtained was placed on a flash column and eluted with ethyl acetate:hexane (60:40), to get 2'-O-([2-phthalimidoxy)ethyl]-5'-*t*-butyldiphenylsilyl-5-methyluridine as white foam (21.819, 86%).

5'-O-*tert*-butyldiphenylsilyl-2'-O-[(2-formadoximinooxy)ethyl]-5-methyluridine

2'-O-([2-phthalimidoxy)ethyl]-5'-*t*-butyldiphenylsilyl-5-methyluridine (3.1g, 4.5mmol) was dissolved in dry $CH_2Cl_2$ (4.5mL) and methylhydrazine (300mL, 4.64mmol) was added dropwise at -10°C to 0°C. After 1 hr the mixture was filtered, the filtrate was washed with ice cold $CH_2Cl_2$ and the combined organic phase was washed with water, brine and dried over anhydrous $Na_2SO_4$. The solution was concentrated to get 2'-*O*-(aminooxyethyl) thymidine, which was then dissolved in MeOH (67.5mL). To this formaldehyde (20% aqueous solution, w/w, 1.1eg.) was added and the mixture for 1 hr. Solvent was removed under vacuum; residue chromatographed to get 5'-O-*tert*-butyldiphenylsilyl-2'-O-[(2-formadoximinooxy) ethyl]-5-methyluridine as white foam (1.95, 78%).

5'-O-*tert*-Butyldiphenylsilyl-2'-O-[N,N-dimethylaminooxyethyl]-5-methyluridine

5'-O-*tert*-butyldiphenylsilyl-2'-O-[(2-formadoximinooxy) ethyl] -5-methyluridine (1.77g, 3.12mmol) was dissolved in a solution of 1M pyridinium p-toluenesulfonate (PPTS) in dry MeOH (30.6mL). Sodium cyanoborohydride (0.39g, 6.13mmol) was added to this solution at 10°C under inert atmosphere. The reaction mixture was stirred for 10 minutes at 10°C. After that the reaction vessel was removed from the ice bath and stirred at room temperature for 2 hr, the reaction monitored by TLC (5% MeOH in $CH_2Cl_2$). Aqueous $NaHCO_3$ solution (5%, 10mL) was added and extracted with ethyl acetate (2x20mL). Ethyl acetate phase was dried over anhydrous $Na_2SO4$, evaporated to dryness. Residue was dissolved in a solution of 1M PPTS in MeOH (30.6mL). Formaldehyde (20% w/w, 30mL, 3.37mmol) was added and the reaction mixture was stirred at room temperature for 10 minutes. Reaction mixture cooled to 10°C in an ice bath, sodium cyanoborohydride (0.39g, 6.13mmol) was added and reaction mixture stirred at 10°C for 10 minutes. After 10 minutes, the reaction mixture was removed from the ice bath and stirred at room temperature for 2 hrs. To the reaction mixture 5% $NaHCO_3$ (25mL) solution was added and extracted with ethyl acetate (2x25mL). Ethyl acetate layer was dried over anhydrous $Na_2SO_4$ and evaporated to dryness. The residue obtained was purified by flash column chromatography and eluted with 5% MeOH in $CH_2Cl_2$ to get 5'-O-*tert*-butyldiphenylsilyl-2'-O-[N,N-dimethylaminooxyethyl]-5-methyluridine as a white foam (14.6g, 80%).

2'-O-(dimethylaminooxyethyl)-5-methyluridine

Triethylamine trihydrofluoride (3.91mL, 24.0mmol) was dissolved in dry THF and triethylamine (1.67mL, 12mmol, dry, kept over KOH). This mixture of triethylamine-2HF was then added to 5'-O-*tert*-butyldiphenylsilyl-2'-O-[N,N-dimethylaminooxyethyl]-5-methyluridine (1.40g, 2.4mmol) and stirred at room temperature for 24 hrs. Reaction was monitored by TLC (5% MeOH in $CH_2Cl_2$). Solvent was removed under vacuum and the residue placed on a flash column and eluted with 10% MeOH in $CH_2Cl_2$ to get 2'-O-(dimethylaminooxyethyl)-5-methyluridine (766mg, 92.5%).

5'-O-DMT-2'-O-(dimethylaminooxyethyl)-5-methyluridine

2'-O-(dimethylaminooxyethyl)-5-methyluridine (750mg, 2.17mmol) was dried over $P_2O_5$ under high vacuum overnight at 40°C. It was then co-evaporated with anhydrous pyridine (20mL). The residue obtained was dissolved in pyridine (11mL) under argon atmosphere. 4-dimethylaminopyridine (26.5mg, 2.60mmol), 4,4'-dimethoxytrityl chloride (880mg, 2.60mmol) was added to the mixture and the reaction mixture was stirred at room temperature until all of the starting material disappeared. Pyridine was removed under vacuum and the residue chromatographed and eluted with 10% MeOH in $CH_2CL_2$ (containing a few drops of pyridine) to get 5'-O-DMT-2'-O-(dimethylamino-oxyethyl)-5-methyluridine (1.13g, 80%).

5'-O-DMT-2'-O-(2-N,N-dimethylaminooxyethyl)-5-methyluridine-3'-[(2-cyanoethyl)-N,N-diisopropylphosphoramidite]

5'-O-DMT-2'-O-(dimethylaminooxyethyl)-5-methyluridine (1.08g, 1.67mmol) was co-evaporated with toluene (20mL). To the residue N,N-diisopropylamine tetrazonide (0.29g, 1.67mmol) was added and dried over $P_2O_5$ under high vacuum overnight at 40°C. Then the reaction mixture was dissolved in anhydrous acetonitrile (8.4mL) and 2-cyanoethyl-N,N,$N^1$,$N^1$-tetraisopropylphosphoramidite (2.12mL, 6.08mmol) was added. The reaction mixture was stirred at ambient temperature for 4 hrs under inert atmosphere. The progress of the reaction was monitored by TLC (hexane:ethyl acetate 1:1). The solvent was evaporated, then the residue was dissolved in ethyl acetate (70mL) and washed with 5% aqueous $NaHCO_3$ (40mL). Ethyl acetate layer was dried over anhydrous $Na_2SO_4$ and concentrated. Residue obtained was chromatographed (ethyl acetate as eluent) to get 5'-O-DMT-2'-O-(2-N,N-dimethylaminooxyethyl)-5-methyluridine-3'-[(2-cyanoethyl)-N,N-diisopropylphosphoramidite] as a foam (1.04g, 74.9%).

Oligonucleotides having methylene (methylimino) (MMI) backbones are synthesized according to U.S. Patent 5, 378, 825. For ease of synthesis, various nucleoside dimers containing MMI linkages are synthesized and incorporated into oligonucleotides. Other nitrogen-containing backbones are synthesized according to WO 92/20823.

Oligonucleotides having amide backbones are synthesized according to De Mesmaeker et al. (Acc. Chem. Res. 1995, 28, 366-374). The amide moiety is readily accessible by simple and well-known synthetic methods and is compatible with the conditions required for solid phase synthesis of oligonucleotides.

Oligonucleotides with morpholino backbones are synthesized according to U.S. Patent 5,034,506 (Summerton and Weller).

Peptide-nucleic acid (PNA) oligomers are synthesized according to P.E. Nielsen et al. (Science 1991, 254, 1497-1500).

After cleavage from the controlled pore glass column (Applied Biosystems) and deblocking in concentrated ammonium hydroxide at 55°C for 18 hours, the oligonucleotides are purified by precipitation twice out of 0.5 M NaCl with 2.5 volumes ethanol. Synthesized oligonucleotides were analyzed by polyacrylamide gel electrophoresis on denaturing gels or capillary gel electrophoresis and judged to be at least 85% full length material.. The relative amounts of phosphorothioate and phosphodiester linkages obtained in synthesis were periodically checked by $^{31}P$ nuclear magnetic resonance spectroscopy, and for some studies oligonucleotides were purified by HPLC, as described by Chiang et al. (J. Biol. Chem. 1991, 266, 18162). Results obtained with HPLC-purified material were similar to those obtained with non-HPLC purified material.

Alternatively, oligonucleotides were synthesized in 96 well plate format via solid phase P(III) phosphoramidite chemistry on an automated synthesizer capable of assembling 96 sequences simultaneously in a standard 96 well format. Phosphodiester internucleotide linkages were afforded by oxidation with aqueous iodine. Phosphorothioate internucleotide linkages were generated by sulfurization utilizing 3,H-1,2 benzodithiole-3-one 1,1 dioxide (Beaucage Reagent) in anhydrous acetonitrile. Standard base-protected beta-cyanoethyl-di-isopropyl phosphoramidites were purchased from commercial vendors (e.g. PE-Applied Biosystems, Foster City, CA, or Pharmacia, Piscataway, NJ). Non-standard nucleosides are synthesized as per published methods. They are utilized as base protected beta-cyanoethyldiisopropyl phosphoramidites.

Oligonucleotides were cleaved from support and deprotected with concentrated $NH_4OH$ at elevated temperature (55-60°C) for 12-16 hours and the released product then dried in vacuo. The dried product was then re-suspended in sterile water to afford a master plate from which all analytical and test plate samples are then diluted utilizing robotic pipettors:

**EXAMPLE 2: Human STAT3 oligodeoxynucleotide sequences**

Antisense oligonucleotides were designed to target human STAT3. Target sequence data are from the APRF cDNA sequence published by Akira, S. et al. (Cell, 1994, 77, 63-71); GenBank® accession number L29277, provided herein as SEQ ID NO: 1. A series of oligodeoxynucleotides was synthesized with phosphorothioate linkages. 2'-deoxy cytosines were 5-methyl cytosines. These oligonucleotide sequences are shown in Table 1. An additional set of oligonucleotides was synthesized as chimeric oligonucleotides ("gapmers") 20 nucleotides in length, composed of a central "gap" region consisting of ten 2'-deoxynucleotides, which is flanked on both sides (5' and 3' directions) by five-nucleotide "wings." The wings are composed of 2'-O-methoxyethyl (2'-MOE)nucleotides. The internucleoside (backbone) linkages are phosphorothioate (P=S) throughout the oligonucleotide. All 2'-MOE cytosines and 2'-deoxy cytosines were 5-methylcytosines.

These oligonucleotide sequences are shown in Table 2.

An appropriate cell line, typically expressing high levels of STAT3, is chosen for *in vitro* studies. Cell culture conditions are those standard for that particular cell line. Oligonucleotide treatment is for four hours and mRNA usually isolated 24 to 48 hours following initial treatment. mRNA is isolated using the RNAEASY7 kit (Qiagen, Santa Clarita, CA).

**TABLE 1:**

| Nucleotide Sequences of Human STAT3 Phosphorothioate Oligodeoxynucleotides | | | | |
|---|---|---|---|---|
| ISIS NO. | NUCLEOTIDE SEQUENCE[1] (5' -> 3') | SEQ ID NO: | TARGET GENE NUCLEOTIDE CO-ORDINATES[2] | GENE TARGET REGION |
| 106691 | GTCTGCGCCGCCGCCCCGAA | 2 | 0010-0029 | 5'-UTR |
| 106692 | GGCCGAAGGGCCTCTCCGAG | 3 | 0130-0149 | 5'-UTR |
| 106693 | TCCTGTTTCTCCGGCAGAGG | 4 | 0202-0221 | AUG |
| 106694 | CATCCTGTTTCTCCGGCAGA | 5 | 0204-0223 | AUG |
| 106695 | GCCATCCTGTTTCTCCGGCA | 6 | 0206-0225 | AUG |
| 106696 | GGGCCATCCTGTTTCTCCGG | 7 | 0208-0227 | AUG |
| 106697 | TTGGGCCATCCTGTTTCTCC | 8 | 0210-0229 | AUG |
| 106698 | CATTGGGCCATCCTGTTTCT | 9 | 0212-0231 | AUG |
| 106699 | TCCATTGGGCCATCCTGTTT | 10 | 0214-0233 | AUG |
| 106700 | ATTCCATTGGGCCATCCTGT | 11 | 0216-0235 | AUG |
| 106701 | TGATTCCATTGGGCCATCCT | 12 | 0218-0237 | AUG |
| 106702 | GCTGATTCCATTGGGCCATC | 13 | 0220-0239 | AUG |
| 106703 | TAGCTGATTCCATTGGGCCA | 14 | 0222-0241 | AUG |
| 106704 | TGTAGCTGATTCCATTGGGC | 15 | 0224-0243 | coding |
| 106705 | CTGTAGAGCTGATGGAGCTG | 16 | 0269-0288 | coding |
| 106706 | CCCAATCTTGACTCTCAATC | 17 | 0331-0350 | coding |
| 106707 | CCCAGGAGATTATGAAACAC | 18 | 0386-0405 | coding |
| 106708 | ACATTCGACTCTTGCAGGAA | 19 | 0431-0450 | coding |
| 106709 | TCTGAAGAAACTGCTTGATT | 20 | 0475-0494 | coding |
| 106710 | GGCCACAATCCGGGCAATCT | 21 | 0519-0538 | coding |
| 106711 | TGGCTGCAGTCTGTAGAAGG | 22 | 0562-0581 | coding |

(continued)

| Nucleotide Sequences of Human STAT3 Phosphorothioate Oligodeoxynucleotides | | | | |
|---|---|---|---|---|
| ISIS NO. | NUCLEOTIDE SEQUENCE[1] (5' -> 3') | SEQ ID NO: | TARGET GENE NUCLEOTIDE CO-ORDINATES[2] | GENE TARGET REGION |
| 106712 | CTGCTCCAGCATCTGCTGCT | 23 | 0639-0658 | coding |
| 106713 | TTTCTGTTCTAGATCCTGCA | 24 | 0684-0703 | coding |
| 106714 | TAGTTGAAATCAAAGTCATC | 25 | 0728-0747 | coding |
| 106715 | TTCCATTCAGATCTTGCATG | 26 | 0772-0791 | coding |
| 106716 | TCTGTTCCAGCTGCTGCATC | 27 | 0817-0836 | coding |
| 106717 | TCACTCACGATGCTTCTCCG | 28 | 0860-0879 | coding |
| 106718 | GAGTTTTCTGCACGTACTCC | 29 | 0904-0923 | coding |
| 106719 | ATCTGTTGCCGCCTCTTCCA | 30 | 0947-0968 | coding |
| 106720 | CTAGCCGATCTAGGCAGATG | 31 | 0991-1010 | coding |
| 106721 | CGGGTCTGAAGTTGAGATTC | 32 | 1034-1053 | coding |
| 106722 | CGGCCGGTGCTGTACAATGG | 33 | 1110-1129 | coding |
| 106723 | TTTCATTAAGTTTCTGAACA | 34 | 1155-1174 | coding |
| 106724 | AGGATGCATGGGCATGCAGG | 35 | 1200-1219 | coding |
| 106725 | GACCAGCAACCTGACTTTAG | 36 | 1260-1279 | coding |
| 106726 | ATGCACACTTTAATTTTAAG | 37 | 1304-1323 | coding |
| 106727 | TTCCGGGATCCTCTGAGAGC | 38 | 1349-1368 | coding |
| 106728 | TTCCATGTTCATCACTTTTG | 39 | 1392-1411 | coding |
| 106729 | GTCAAGTGTTTGAATTCTGC | 40 | 1436-1455 | coding |
| 106730 | CAATCAGGGAAGCATCACAA | 41 | 1495-1514 | coding |
| 106731 | TACACCTCGGTCTCAAAGGT | 42 | 1538-1557 | coding |
| 106732 | TGACAAGGAGTGGGTCTCTA | 43 | 1581-1600 | coding |
| 106733 | CGCCCAGGCATTTGGCATCT | 44 | 1626-1645 | coding |
| 106734 | CATTCTTGGGATTGTTGGTC | 45 | 1669-1688 | coding |
| 106735 | CACTTGGTCCCAGGTTCCAA | 46 | 1713-1732 | coding |
| 106736 | CCCGCTTGGTGGTGGACGAG | 47 | 1756-1775 | coding |
| 106737 | AGTTCACACCAGGCCCTAGG | 48 | 1816-1835 | coding |
| 106738 | GTTTTCTTTGCAGAAGTTAG | 49 | 1860-1879 | coding |
| 106739 | ATATTGTCTAGCCAGACCCA | 50 | 1904-1923 | coding |
| 106740 | AACCCATGATGTACCCTTCA | 51 | 1963-1982 | coding |
| 106741 | GCTTAGTGCTCAAGATGGCC | 52 | 2005-2024 | coding |
| 106742 | GCTGCTTTCACTGAAGCGCA | 53 | 2043-2062 | coding |
| 106743 | GTGAAAGTGACGCCTCCTTC | 54 | 2066-2085 | coding |
| 106744 | CTGATGTCCTTCTCCACCCA | 55 | 2087-2106 | coding |
| 106745 | ACTGGATCTGGGTCTTACCG | 56 | 2107-2126 | coding |
| 106746 | AAATGACATGTTGTTCAGCT | 57 | 2151-2170 | coding |

(continued)

| Nucleotide Sequences of Human STAT3 Phosphorothioate Oligodeoxynucleotides | | | | |
|---|---|---|---|---|
| ISIS NO. | NUCLEOTIDE SEQUENCE[1] (5' -> 3') | SEQ ID NO: | TARGET GENE NUCLEOTIDE CO-ORDINATES[2] | GENE TARGET REGION |
| 106747 | GCCCATGATGATTTCAGCAA | 58 | 2169-2188 | coding |
| 106748 | TATTGGTAGCATCCATGATC | 59 | 2194-2213 | coding |
| 106749 | ATAGACAAGTGGAGACAACA | 60 | 2217-2236 | coding |
| 106750 | TTGGGAATGTCAGGATAGAG | 61 | 2237-2256 | coding |
| 106751 | CTCCTGGCTCTCTGGCCGAC | 62 | 2280-2299 | coding |
| 106752 | ACCTGGGTCAGCTTCAGGAT | 63 | 2301-2320 | coding |
| 106753 | CACAGATAAACTTGGTCTTC | 64 | 2338-2357 | coding |
| 106754 | ATCGGCAGGTCAATGGTATT | 65 | 2378-2397 | coding |
| 106755 | CCAAACTGCATCAATGAATC | 66 | 2414-2433 | coding |
| 106756 | GGTTCAGCACCTTCACCATT | 67 | 2438-2457 | coding |
| 106757 | GAGGGACTCAAACTGCCCTC | 68 | 2466-2485 | coding |
| 106758 | CAACTCCATGTCAAAGGTGA | 69 | 2484-2503 | coding |
| 106759 | TTCTCAGCTCCTCACATGGG | 70 | 2525-2544 | STOP |
| 106760 | CGTTCTCAGCTCCTCACATG | 71 | 2527-2546 | STOP |
| 106761 | TCCGTTCTCAGCTCCTCACA | 72 | 2529-2548 | STOP |
| 106762 | CTTCCGTTCTCAGCTCCTCA | 73 | 2531-2550 | STOP |
| 106763 | AGCTTCCGTTCTCAGCTCCT | 74 | 2533-2552 | STOP |
| 106764 | AGAATGCAGGTAGGCGCCTC | 75 | 2569-2588 | 3'-UTR |
| 106765 | ACCACAAAGTTAGTAGTTTC | 76 | 2623-2642 | 3'-UTR |
| 106766 | TGCTCAAAGATAGCAGAAGT | 77 | 2665-2684 | 3'-UTR |
| 106767 | ATTCACTCATTTCTCTATTT | 78 | 2701-2720 | 3'-UTR |
| 106768 | CATTTAGATAAAAGCAGATC | 79 | 2727-2746 | 3'-UTR |
| 106769 | ACATCCTTATTTGCATTTAG | 80 | 2740-2759 | 3'-UTR |
| 106770 | GATCATGGGTCTCAGAGAAC | 81 | 2760-2779 | 3'-UTR |
| [1] "C" resdues are 5-methyl-cytosines; all linkages are phosphorothioate linkages.<br>[2] Coordinates from GenBank® Accession No. L29277, locus name "HUMAPRF", SEQ ID NO: 1. | | | | |

**TABLE 2:**

| Nucleotide Sequences of Human STAT3 Chimeric (deoxy gapped) Phosphorothioate Oligonucleotides | | | | |
|---|---|---|---|---|
| ISIS NO. | NUCLEOTIDE SEQUENCE[1] (5' -> 3') | SEQ ID NO: | TARGET GENE NUCLEOTIDE CO-ORDINATES[2] | GENE TARGET REGION |
| 106771 | GTCTGCGCCGCCGCCCCGAA | 2 | 0010-0029 | 5'-UTR |
| 106772 | GGCCGAAGGGCCTCTCCGAG | 3 | 0130-0149 | 5'-UTR |
| 106773 | TCCTGTTTCTCCGGCAGAGG | 4 | 0202-0221 | AUG |
| 106774 | CATCCTGTTTCTCCGGCAGA | 5 | 0204-0223 | AUG |

(continued)

| Nucleotide Sequences of Human STAT3 Chimeric (deoxy gapped) Phosphorothioate Oligonucleotides | | | | |
|---|---|---|---|---|
| ISIS NO. | NUCLEOTIDE SEQUENCE[1] (5' -> 3') | SEQ ID NO: | TARGET GENE NUCLEOTIDE CO-ORDINATES[2] | GENE TARGET REGION |
| 106775 | GCCATCCTGTTTCTCCGGCA | 6 | 0206-0225 | AUG |
| 106776 | GGGCCATCCTGTTTCTCCGG | 7 | 0208-0227 | AUG |
| 106777 | TTGGGCCATCCTGTTTCTCC | 8 | 0210-0229 | AUG |
| 106778 | CATTGGGCCATCCTGTTTCT | 9 | 0212-0231 | AUG |
| 106779 | TCCATTGGGCCATCCTGTTT | 10 | 0214-0233 | AUG |
| 106780 | ATTCCATTGGGCCATCCTGT | 11 | 0216-0235 | AUG |
| 106781 | TGATTCCATTGGGCCATCCT | 12 | 0218-0237 | AUG |
| 106782 | GCTGATTCCATTGGGCCATC | 13 | 0220-0239 | AUG |
| 106783 | TAGCTGATTCCATTGGGCCA | 14 | 0222-0241 | AUG |
| 106784 | TGTAGCTGATTCCATTGGGC | 15 | 0224-0243 | coding |
| 106785 | CTGTAGAGCTGATGGAGCTG | 16 | 0269-0288 | coding |
| 106786 | CCCAATCTTGACTCTCAATC | 17 | 0331-0350 | coding |
| 106787 | CCCAGGAGATTATGAAACAC | 18 | 0386-0405 | coding |
| 106788 | ACATTCGACTCTTGCAGGAA | 19 | 0431-0450 | coding |
| 106789 | TCTGAAGAAACTGCTTGATT | 20 | 0475-0494 | coding |
| 106790 | GGCCACAATCCGGGCAATCT | 21 | 0519-0538 | coding |
| 106791 | TGGCTGCAGTCTGTAGAAGG | 22 | 0562-0581 | coding |
| 106792 | CTGCTCCAGCATCTGCTGCT | 23 | 0639-0658 | coding |
| 106793 | TTTCTGTTCTAGATCCTGCA | 24 | 0684-0703 | coding |
| 106794 | TAGTTGAAATCAAAGTCATC | 25 | 0728-0747 | coding |
| 106795 | TTCCATTCAGATCTTGCATG | 26 | 0772-0791 | coding |
| 106796 | TCTGTTCCAGCTGCTGCATC | 27 | 0817-0836 | coding |
| 106797 | TCACTCACGATGCTTCTCCG | 28 | 0860-0879 | coding |
| 106798 | GAGTTTTCTGCACGTACTCC | 29 | 0904-0923 | coding |
| 106799 | ATCTGTTGCCGCCTCTTCCA | 30 | 0947-0968 | coding |
| 106800 | CTAGCCGATCTAGGCAGATG | 31 | 0991-1010 | coding |
| 106801 | CGGGTCTGAAGTTGAGATTC | 32 | 1034-1053 | coding |
| 106802 | CGGCCGGTGCTGTACAATGG | 33 | 1110-1129 | coding |
| 106803 | TTTCATTAAGTTTCTGAACA | 34 | 1155-1174 | coding |
| 106804 | AGGATGCATGGGCATGCAGG | 35 | 1200-1219 | coding |
| 106805 | GACCAGCAACCTGACTTTAG | 36 | 1260-1279 | coding |
| 106806 | ATGCACACTTTAATTTTAAG | 37 | 1304-1323 | coding |
| 106807 | TTCCGGGATCCTCTGAGAGC | 38 | 1349-1368 | coding |
| 106808 | TTCCATGTTCATCACTTTTG | 39 | 1392-1411 | coding |
| 106809 | GTCAAGTGTTTGAATTCTGC | 40 | 1436-1455 | coding |

(continued)

| Nucleotide Sequences of Human STAT3 Chimeric (deoxy gapped) Phosphorothioate Oligonucleotides | | | | |
|---|---|---|---|---|
| ISIS NO. | NUCLEOTIDE SEQUENCE[1] (5' -> 3') | SEQ ID NO: | TARGET GENE NUCLEOTIDE CO-ORDINATES[2] | GENE TARGET REGION |
| 106810 | CAATCAGGGAAGCATCACAA | 41 | 1495-1514 | coding |
| 106811 | TACACCTCGGTCTCAAAGGT | 42 | 1538-1557 | coding |
| 106812 | TGACAAGGAGTGGGTCTCTA | 43 | 1581-1600 | coding |
| 106813 | CGCCCAGGCATTTGGCATCT | 44 | 1626-1645 | coding |
| 106814 | CATTCTTGGGATTGTTGGTC | 45 | 1669-1688 | coding |
| 106815 | CACTTGGTCCCAGGTTCCAA | 46 | 1713-1732 | coding |
| 106816 | CCCGCTTGGTGGTGGACGAG | 47 | 1756-1775 | coding |
| 106817 | AGTTCACACCAGGCCCTAGG | 48 | 1816-1835 | coding |
| 106818 | GTTTTCTTTGCAGAAGTTAG | 49 | 1860-1879 | coding |
| 106819 | ATATTGTCTAGCCAGACCCA | 50 | 1904-1923 | coding |
| 106820 | AACCCATGATGTACCCTTCA | 51 | 1963-1982 | coding |
| 106821 | GCTTAGTGCTCAAGATGGCC | 52 | 2005-2024 | coding |
| 106822 | GCTGCTTTCACTGAAGCGCA | 53 | 2043-2062 | coding |
| 106823 | GTGAAAGTGACGCCTCCTTC | 54 | 2066-2085 | coding |
| 106824 | CTGATGTCCTTCTCCACCCA | 55 | 2087-2106 | coding |
| 106825 | ACTGGATCTGGGTCTTACCG | 56 | 2107-2126 | coding |
| 106826 | AAATGACATGTTGTTCAGCT | 57 | 2151-2170 | coding |
| 106827 | GCCCATGATGATTTCAGCAA | 58 | 2169-2188 | coding |
| 106828 | TATTGGTAGCATCCATGATC | 59 | 2194-2213 | coding |
| 106829 | ATAGACAAGTGGAGACAACA | 60 | 2217-2236 | coding |
| 106830 | TTGGGAATGTCAGGATAGAG | 61 | 2237-2256 | coding |
| 106831 | CTCCTGGCTCTCTGGCCGAC | 62 | 2280-2299 | coding |
| 106832 | ACCTGGGTCAGCTTCAGGAT | 63 | 2301-2320 | coding |
| 106833 | CACAGATAAACTTGGTCTTC | 64 | 2338-2357 | coding |
| 106834 | ATCGGCAGGTCAATGGTATT | 65 | 2378-2397 | coding |
| 106835 | CCAAACTGCATCAATGAATC | 66 | 2414-2433 | coding |
| 106836 | GGTTCAGCACCTTCACCATT | 67 | 2438-2457 | coding |
| 106837 | GAGGGACTCAAACTGCCCTC | 68 | 2466-2485 | coding |
| 106838 | CAACTCCATGTCAAAGGTGA | 69 | 2484-2503 | coding |
| 106839 | TTCTCAGCTCCTCACATGGG | 70 | 2525-2544 | STOP |
| 106840 | CGTTCTCAGCTCCTCACATG | 71 | 2527-2546 | STOP |
| 106841 | TCCGTTCTCAGCTCCTCACA | 72 | 2529-2548 | STOP |
| 106842 | CTTCCGTTCTCAGCTCCTCA | 73 | 2531-2550 | STOP |
| 106843 | AGCTTCCGTTCTCAGCTCCT | 74 | 2533-2552 | STOP |
| 106844 | AGAATGCAGGTAGGCGCCTC | 75 | 2569-2588 | 3'-UTR |

(continued)

| Nucleotide Sequences of Human STAT3 Chimeric (deoxy gapped) Phosphorothioate Oligonucleotides | | | | |
|---|---|---|---|---|
| ISIS NO. | NUCLEOTIDE SEQUENCE[1] (5' -> 3') | SEQ ID NO: | TARGET GENE NUCLEOTIDE CO-ORDINATES[2] | GENE TARGET REGION |
| 106845 | ACCACAAAGTTAGTAGTTTC | 76 | 2623-2642 | 3'-UTR |
| 106846 | TGCTCAAAGATAGCAGAAGT | 77 | 2665-2684 | 3'-UTR |
| 106847 | ATTCACTCATTTCTCTATTT | 78 | 2701-2720 | 3'-UTR |
| 106848 | CATTTAGATAAAAGCAGATC | 79 | 2727-2746 | 3'-UTR |
| 106849 | ACATCCTTATTTGCATTTAG | 80 | 2740-2759 | 3'-UTR |
| 106850 | GATCATGGGTCTCAGAGAAC | 81 | 2760-2779 | 3'-UTR |
| [1] Emboldened residues are 2'- methoxyethoxy residues, 2'-methoxyethoxy cytosine residues and 2'-OH cytosine residues are 5-methyl-cytosines; all linkages are phosphorothioate linkages. [2] Coordinates from GenBank® Accession No. L29277, locus name "HUMAPRF", SEQ ID NO: 1. | | | | |

Oligonucleotide activity is assayed by quantitation of STAT3 mRNA levels by real-time PCR using the ABI PRISM™ 7700 Sequence Detection System (PE-Applied Biosystems, Foster City, CA) according to manufacturer's instructions. This is a closed-tube, non-gel-based, fluorescence detection system which allows high-throughput quantitation of polymerase chain reaction (PCR) products in real-time. As opposed to standard PCR, in which amplification products are quantitated after the PCR is completed, products in real-time PCR are quantitated as they accumulate. This is accomplished by including in the PCR reaction an oligonucleotide probe that anneals specifically between the forward and reverse PCR primers, and contains two fluorescent dyes. A reporter dye (e.g., JOE or FAM, PE-Applied Biosystems, Foster City, CA) is attached to the 5' end of the probe and a quencher dye (e.g., TAMRA or MGB, PE-Applied Biosystems, Foster City, CA) is attached to the 3' end of the probe. When the probe and dyes are intact, reporter dye emission is quenched by the proximity of the 3' quencher dye. During amplification, annealing of the probe to the target sequence creates a substrate that can be cleaved by the 5'-exonuclease activity of Taq polymerase. During the extension phase of the PCR amplification cycle, cleavage of the probe by Taq polymerase releases the reporter dye from the remainder of the probe (and hence from the quencher moiety) and a sequence-specific fluorescent signal is generated. With each cycle, additional reporter dye molecules are cleaved from their respective probes, and the fluorescence intensity is monitored at regular (six-second) intervals by laser optics built into the ABI PRISM™ 7700 Sequence Detection System. In each assay, a series of parallel reactions containing serial dilutions of mRNA from untreated control samples generates a standard curve that is used to quantitate the percent inhibition after antisense oligonucleotide treatment of test samples.

Isolated RNA is first subjected to a reverse transcriptase reaction to prepare complementary DNA (cDNA) from RNA. The resultant cDNA is the substrate for real-time PCR. Reverse transcriptase and real-time PCR reagents are obtained from PE-Applied Biosystems, Foster City, CA. Reactions are carried out by adding 25 1 PCR cocktail (1x TAQMAN7 buffer A, 5.5 mM $MgCl_2$, 300 M each of dATP, dCTP and dGTP, 600 M of dUTP, 100 nM each of forward primer, reverse primer, and probe, 20 U RNAse inhibitor, 1.25 units AMPLITAQ GOLD7, and 12.5 U MuLV reverse transcriptase) to 96 well plates containing 25 1 poly (A) mRNA solution. The reverse transcriptase reaction is carried out by incubation for 30 minutes at 48°C following a 10 minute incubation at 95°C to activate the AMPLITAQ GOLD7, 40 cycles of a two-step PCR protocol are carried out: 95°C for 15 seconds (denaturation) followed by 60°C for 1.5 minutes (annealing/extension).

STAT3 PCR primers and a probe can be designed using commercial software (e.g. Oligo 5.0 or Primer Express®).

**EXAMPLE 3: Mouse STAT3 oligonucleotide sequences**

Antisense oligonucleotides were designed to target mouse STAT3. Target sequence data are from the STAT3 cDNA sequence submitted by Zhong, Z.; GenDank® accession number U06922, provided herein as SEQ ID NO: 82. Oligonucleotides were synthesized as chimeric oligonucleotides ("gapmers") 20 nucleotides in length, composed of a central "gap" region consisting of ten 2'-deoxynucleotides, which is flanked on both sides (5' and 3' directions) by five-nucleotide "wings." The wings are composed of 2'-O-methoxyethyl (2'-MOE)nucleotides. The internucleoside (backbone) linkages are phosphorothioate (P=S) throughout the oligonucleotide. All 2'-MOE cytosines were 5-methyl-cytosines.

Oligonucleotide sequences are shown in Table 3. The B lymphoma cell line, BCL1 was obtained from ATCC (Manassas, VA). BCL1 cells were cultured under standard conditions in RPMI 1640 medium.

BCL1 cells (5 X $10^6$ cells in PBS) were transfected with oligonucleotides by electroporation, at 200V, 1000 F using a BTX Electro Cell Manipulator™ 600 (Genetronics, San Diego, CA). For an initial screen, BCL1 cells were electroporated with 10 M oligonucleotide, and RNA was collected 24 hours later. Control samples without oligonucleotide were subjected to the same electroporation conditions.

Total cellular RNA was isolated using the RNEASY7® kit (Qiagen, Santa Clarita, CA). RNAse protection experiments were conducted using RIBOQUANT™ kits and template sets according to the manufacturer's instructions (Pharmingen, San Diego, CA). Northern blotting was performed as described in Chiang, M-Y. et al. (J. Biol. Chem., 1991, 266, 18162-18171), using a rat cDNA probe prepared by Xho I/Sal I restriction digest of the psvsport-1 plasmid (ATCC, Rockville, MD). mRNA levels were quantitated using a PhosphorImager™ (Molecular Dynamics, Sunnyvale, CA).

**TABLE 3:**

| Nucleotide Sequences of Mouse STAT3 Chimeric (deoxy gapped) Phosphorothioate Oligodeoxynucleotides | | | | |
|---|---|---|---|---|
| ISIS NO. | NUCLEOTIDE SEQUENCE[1] (5' -> 3') | SEQ ID NO: | TARGET GENE NUCLEOTIDE COORDINATES[2] | GENE TARGET REGION |
| 17136 | GTTCCACTGAGCCATCCTGC | 83 | 0064-0083 | AUG |
| 17137 | TTCAGGTAGCGTGTGTCCAG | 84 | 0096-0115 | coding |
| 17138 | ATGTGACTCTTTGCTGGCTG | 85 | 0205-0224 | coding |
| 17139 | CCAAGAGATTATGAAACACC | 86 | 0233-0252 | coding |
| 17140 | GCTCCAACATCTGCTGCTTC | 87 | 0485-0504 | coding |
| 17141 | GCTCTTCATCAGTCAGTGTC | 88 | 0767-0786 | coding |
| 17142 | ATCTGACACCCTGAGTAGTT | 89 | 1680-1699 | coding |
| 17143 | GCCAGACCCAGAAGGAGAAG | 90 | 1742-1761 | coding |
| 17144 | CGCTCCTTGCTGATGAAACC | 91 | 1827-1846 | coding |
| 17145 | AACTTGGTCTTCAGGTACGG | 92 | 2178-2197 | coding |
| 17146 | ATCAATGAATCTAAAGTGCG | 93 | 2253-2272 | coding |
| 17147 | TCAGCACCTTCACCGTTATT | 94 | 2283-2302 | coding |
| 17148 | ACTCAAACTGCCCTCCTGCT | 95 | 2309-2328 | coding |
| 17149 | GGTTTCAGCTCCTCACATGG | 96 | 2374-2393 | STOP |
| 17150 | TAAAAAAAAAAATCTGGAAC | 97 | 2485-2504 | 3'-UTR |
| 17151 | AAGATAGCAGAAGTAGGAAA | 98 | 2506-2525 | 3'-UTR |
| 17152 | AAAAAGTGCCCAGATTGCCC | 99 | 2527-2546 | 3'-UTR |
| 17153 | ATCACCCACACTCACTCATT | 100 | 2557-2645 | 3'-UTR |
| 17154 | CCTTTGCCTCCCTTCTGCTC | 101 | 2626-2645 | 3'-UTR |
| 17155 | TGAAAAAGGAGGGCAGGCGG | 102 | 2665-2684 | 3'-UTR |
| 17156 | CACCAGGAGGCACTTGTCTA | 103 | 2705-2724 | 3'-UTR |
| 17157 | AACCTCCTGGGCTTAGTCCT | 104 | 2822-2841 | 3'-UTR |
| 23176 | AAAAAGTGCGCAGATTGCCC | 105 | 1 base mismatch control | |
| 23177 | AAAAAGTCCGCTGATTGCCC | 106 | 3 base mismatch control | |
| 23178 | AAAAACTCCGCTGAATGCCC | 107 | 5 base mismatch control | |
| [1] All 2'-MOE cytosine residues are 5-methyl-cytosines; all linkages are phosphorothioate linkages. [2]Co-ordinates from GenBank® Accession No. U06922, locus name "MMU06922", SEQ ID NO: 82. | | | | |

Results are shown in Table 4. Oligonucleotides 17138 (SEQ ID NO: 85), 17139 (SEQ ID NO: 86), 17140 (SEQ ID NO: 87), 17143 (SEQ ID NO: 90), 17144 (SEQ ID NO: 91), 17152 (SEQ ID NO: 99), 17153 (SEQ ID NO: 100), 17156 (SEQ ID NO: 103), and 17157 (SEQ ID NO: 104) gave better than 45% inhibition in this assay.

**TABLE 4**

| Inhibition of Mouse STAT3 mRNA expression in BCL1 Cells by Chimeric (deoxy gapped) Phosphorothioate Oligonucleotides | | | | |
|---|---|---|---|---|
| ISIS No: | SEQ ID NO: | GENE TARGET REGION | % mRNA EXPRESSION | % mRNA INHIBITION |
| control | --- | --- | 100% | 0% |
| 17136 | 83 | AUG | 75% | 25% |
| 17137 | 84 | coding | 75% | 25% |
| 17138 | 85 | coding | 37% | 63% |
| 17139 | 86 | coding | 41% | 59% |
| 17140 | 87 | coding | 40% | 60% |
| 17141 | 88 | coding | 62% | 38% |
| 17142 | 89 | coding | 70% | 30% |
| 17143 | 90 | coding | 42% | 58% |
| 17144 | 91 | coding | 55% | 45% |
| 17145 | 92 | coding | 89% | 11% |
| 17146 | 93 | coding | 91% | 9% |
| 17147 | 94 | coding | 70% | 30% |
| 17148 | 95 | coding | 69% | 31% |
| 17149 | 96 | STOP | 70% | 30% |
| 17150 | 97 | 3'-UTR | 95% | 5% |
| 17151 | 98 | 3'-UTR | 92% | 8% |
| 17152 | 99 | 3'-UTR | 25% | 75% |
| 17153 | 100 | 3'-UTR | 44% | 56% |
| 17154 | 101 | 3'-UTR | 80% | 20% |
| 17155 | 102 | 3'-UTR | 78% | 22% |
| 17156 | 103 | 3'-UTR | 40% | 60% |
| 17157 | 104 | 3'-UTR | 53% | 47% |

**EXAMPLE 4: Dose response of antisense chimeric (deoxy gapped) phosphorothioate oligonucleotide effects on mouse STAT3 protein levels in BCL1 cells**

ISIS 17152 (SEQ ID. NO. 99) was chosen for further study. The effect of this oligonucleotide on protein levels was determined by Western blot. ISIS 23177 (SEQ ID NO: 106), a 3 base mismatch, was used as a control. BCL1 cells were grown, treated and processed essentially as described in Example 3.

Nuclear extracts were prepared as from primary B cells and B lymphoma cell lines as described in Karras, J.G., et al. (J. Exp. Med. , 1997, 185, 1035-1042).

Western blotting was performed as described in Karras, J.G. et al. (J. Immunol., 1996, 157, 2299). STAT1 and STAT3 antibodies were obtained from UBI (Lake Placid, NY).

Results are shown in Table 5. ISIS 17152 (SEQ ID NO: 99) significantly reduced STAT3 protein levels as compared to a mismatch control.

**TABLE 5**

| Dose Response of BCL1 cells to STAT3 Chimeric (deoxy gapped) Phosphorothioate Oligonucleotides | | | | | |
|---|---|---|---|---|---|
| ISIS # | SEQ ID NO: | ASO Gene Target | Dose | % protein Expression | % protein Inhibition |
| control | --- | --- | --- | 100% | --- |
| 17152 | 99 | 3'-UTR | 10 nM | 41.7% | 58.3% |
| " | " | " | 15 nM | 42.5% | 57.5% |
| " | " | " | 20 nM | 26.5% | 73.5% |
| 23177 | 106 | control | 10 nM | 75.1% | 24.9% |
| " | " | " | 15 nM | 67.6% | 32.4% |
| " | " | " | 20 nM | 62.6% | 37.4% |

**EXAMPLE 5: Inhibition of BCL1 proliferation by STAT3 antisense chimeric (deoxy gapped) phosphorothioate oligonucleotide**

The effect of ISIS 17152 (SEQ ID NO: 99) on BCL1 proliferation was determined. BCL1 cells contain constitutively active STAT3 which is thought to be responsible for their proliferation. BCL1 cells (1 x $10^5$) were electroporated essentially as described in Example 3 with 10 nm, 15 nm or 20 nm of a STAT3 (ISIS 17152) or mismatch (ISIS 23177) oligonucleotide. Cells were incubated in 96-well plates in 200 L complete RPMI for 48 hours following electroporation. Cultures were pulsed with 1 Ci of [$^3$H]-thymidine for the final 8 hours in culture. Cells were harvested and analyzed for thymidine incorporation as described in Francis, D.A. et al. (Int. Immunol., 1995, 7, 151-161).

Results are shown in Table 6. ISIS 17152 (SEQ ID NO: 99) reduced BCL1 cell proliferation by approximately 50%, whereas the mismatch control had no effect on cell proliferation.

**TABLE 6**

| Inhibition of BCL1 Cell Proliferation with STAT3 Chimeric (deoxy gapped) Phosphorothioate Oligonucleotides | | | | | |
|---|---|---|---|---|---|
| ISIS # | SEQ ID NO: | ASO Gene Target | Dose | % Cell Proliferation | % Cell Inhibition |
| control | --- | --- | --- | 100% | --- |
| 17152 | 99 | 3'-UTR | 10 nM | 78.5% | 21.5% |
| " | " | " | 15 nM | 54.4% | 45.6% |
| " | " | " | 20 nM | 50.2% | 49.8% |
| 23177 | 106 | control | 10 nM | 117.0% | --- |
| " | " | " | 15 nM | 99.7% | 0.3% |
| " | " | " | 20 nM | 107.0% | --- |

**EXAMPLE 6: Inhibition of BCL1 IgM Secretion by STAT3 antisense chimeric (deoxy gapped) phosphorothioate oligonucleotides**

The effect of ISIS 17152 (SEQ ID. NO. 99) on IgM secretion levels in BCL1 cells was determined. STAT3 has been implicated in regulation of IgM expression (Faris, M. , et al., Immunology, 1997, 90, 350-357). BCL1 cells (1 x $10^6$) were electroporated essentially described in Example 3 with 5 nm or 15 nm of a STAT3 (ISIS 17152) or mismatch (ISIS 23177) oligonucleotide. Cells were incubated in 12-well plates in 2 mL complete RPMI following electroporation. The media was replaced with fresh media 24 hours post electroporation. After an additional 48 hours, growth media was harvested, centrifuged to remove cells, and assayed for IgM content using the OPT-EIA™ ELISA kit (Pharmingen, San Diego, CA) to capture and detect mouse IgM antibodies (Southern Biotechnology, Birmingham, AL).

Results are shown in Table 7. ISIS 17152 (SEQ ID NO: 99) significantly reduced IgM secretion as compared to mismatch control oligonucleotide (ISIS 23177).

**TABLE 7**

| Inhibition of BCL1 IgM Secretion by STAT3 Chimeric (deoxy gapped) Phosphorothioate Oligonucleotides | | | | | |
|---|---|---|---|---|---|
| ISIS # | SEQ ID NO: | ASO Gene Target | Dose | % IgM Expression | % IgM Inhibition |
| control | --- | --- | --- | 100% | --- |
| 17152 | 99 | 3'-UTC | 5 nM | 34.2% | 65.8-% |
| " | " | " | 15 nM | 23.1% | 76.9% |
| 23177 | 106 | control | 5 nM | 110.0% | --- |
| " | " | " | 15 nM | 80.8% | 19.2% |

**EXAMPLE 7: Induction of chemokines in BCL1 cells following treatment with STAT3 antisense chimeric (deoxy gapped) phosphorothioate oligonucleotide**

The effect of ISIS 17152 (SEQ ID. NO. 99) on chemokine levels in BCL 1 cells was determined. BCL1 cells were electroporated essentially as described in Example 3 with 5 nm, 10 nm or 20 nm of a STAT3 (ISIS 17152) or mismatch (ISIS 23177) oligonucleotide. Chemokine gene expression was induced in the electroporated BCL1 cells by addition of 10 uM of a CpG-containing oligonucleotide to the media 16 hours following electroporation. CpG-containing oligonucleotides are immuno-stimulatory molecules (Krieg, A.M., et al., Nature, 1995, 374, 546-549). Chemokine-levels were measured eight hours later using RNase protection assay by methods known to those skilled in the art with a mouse chemokine template set, Mck-5 (Pharmingen, San Diego, CA).

Results are shown in Table 8. ISIS 17152 (SEQ ID NO: 99) was able to induce the expression of the chemokines RANTES, MIP-1-alpha and MIP-1-beta whereas the mismatch control had minimal effect.

**TABLE 8**

| Induction of Chemokines in BCL1 Cells Following Treatment with STAT3 Chimeric (deoxy gapped) Phosphorothioate Oligonucleotides | | | | | | |
|---|---|---|---|---|---|---|
| ISIS # | SEQ ID NO: | ASO Gene Target | Dose | % RANTES mRNA | % MIPla mRNA | % MIPlb mRNA |
| control | --- | --- | --- | 100% | 100% | 100% |
| 17152 | 99 | 3'-UTR | 5 nM | 236% | 201% | 133% |
| " | " | " | 10 nM | 266% | 258% | 150% |
| " | " | " | 20 nM | 257% | 254% | 159% |
| 23178 | 107 | control | 5 nM | 96% | 123% | 96.5% |
| " | " | " | 10 nM | 70.2% | 116% | 87.1% |
| " | " | " | 20 nM | 56% | 106% | 73.3% |

**Example 8: Additional antisense oligonucleotides targeted to human STAT3**

An additional set of oligonucleotides targeted to SEQ ID NO: 1 was designed and synthesized as chimeric oligonucleotides ("gapmers") 20 nucleotides in length, composed of a central "gap" region consisting of ten 2'-deoxynucleotides, which is flanked on both sides (5' and 3' directions) by five-nucleotide "wings." The wings are composed of 2'-O-methoxyethyl (2'-MOE)nucleotides (shown in bold). The internucleoside (backbone) linkages are phosphorothioate (P=S) throughout the oligonucleotide. All 2'-MOE cytosines and 2'-deoxy cytosines were 5-methyl-cytosines. These oligonucleotide sequences are shown in Table 9.

**TABLE 9:**

| Nucleotide Sequences of Additional Chimeric (deoxy gapped) Phosphorothioate Oligonucleotides targeted to Human STAT3 | | | | |
|---|---|---|---|---|
| ISIS NO. | NUCLEOTIDE-SEQUENCE[1] (5' -> 3') | TARGET GENE NUCLEOTIDE CO-ORDINATES[2] | GENE TARGET REGION | SEQ ID NO: |
| 113169 | **ATGTG**ATTCTTTGCT**GGCCG** | 357 | 5' UTR | 108 |
| 113170 | **AGCTG**ATTCCATTGG**GCCAT** | 221 | AUG | 109 |
| 113171 | **CCAGG**AGATTATGAA**ACACC** | 385 | Coding | 110 |
| 113172 | **ACCGT**GTGTCAAGCT**GCTGT** | 241 | Coding | 111 |
| 113173 | **CCATT**GGGAAGCTGT**CACTG** | 286 | Coding | 112 |
| 113174 | **TGTGA**TTCTTTGCTG**GCCGC** | 356 | Coding | 113 |
| 113175 | **GCGGC**TATACTGCTG**GTCAA** | 411 | Coding | 114 |
| 113176 | **GCTCC**AGCATCTGCT**GCTTC** | 637 | Coding | 115 |
| 113177 | **GATTC**TTCCCACAGG**CACCG** | 539 | Coding | 116 |
| 113178 | **TGATT**CTTCCCACAG**GCACC** | 540 | Coding | 117 |
| 113179 | **ATCCT**GAAGGTGCTG**CTCCA** | 651 | Coding | 118 |
| 113180 | **CGGAC**ATCCTGAAGG**TGCTG** | 656 | Coding | 119 |
| 113181 | **CCCGC**CAGCTCACTC**ACGAT** | 869 | Coding | 120 |
| 113182 | **AGTCA**GCCAGCTCCT**CGTCC** | 928 | Coding | 121 |
| 113183 | **CCAGT**CAGCCAGCTC**CTCGT** | 930 | Coding | 122 |
| 113184 | **CGCCT**CTTCCAGTCA**GCCAG** | 938 | Coding | 123 |
| 113185 | **GGCCG**GTGCTGTACA**ATGGG** | 1109 | Coding | 124 |
| 113186 | **ATCCT**CTCCTCCAGC**ATCGG** | 1127 | Coding | 125 |
| 113187 | **CCGCT**CCACCACAAA**GGCAC** | 1176 | Coding | 126 |
| 113188 | **CGTCC**CCAGAGTCTT**TGTCA** | 1324 | Coding | 127 |
| 113189 | **TTGTG**TTTGTGCCCA**GAATG** | 1375 | Coding | 128 |
| 113190 | **GCTCG**GCCCCCATTC**CCACA** | 1472 | Coding | 129 |
| 113191 | **AGGCA**TTTGGCATCT**GACAG** | 1621 | Coding | 130 |
| 113192 | **CTTGG**GATTGTTGGT**CAGCA** | 1665 | Coding | 131 |
| 113193 | **CTCGG**CCACTTGGTCCC**AGG** | 1719 | Coding | 132 |
| 113194 | **CCCCG**CTTGGTGGTG**GACGA** | 1757 | Coding | 133 |
| 113195 | **CCCCC**GCTTGGTGGT**GGACG** | 1758 | Coding | 134 |
| 113196 | **GGAGA**AGCCCTTGCC**AGCCA** | 1881 | Coding | 135 |
| 113197 | **TTCAT**TCCAAAGGGCC**AAGA** | 1947 | Coding | 136 |
| 113198 | **CCCGC**TCCTTGCTGA**TGAAA** | 1981 | Coding | 137 |
| 113199 | **GTGCT**CAAGATGGCC**CGCTC** | 2000 | Coding | 138 |
| 113200 | **CCCAA**GTGAAAGTGA**CGCCT** | 2071 | Coding | 139 |
| 113201 | **ACCCA**AGTGAAAGTG**ACGCC** | 2072 | Coding | 140 |
| 113202 | **CCGAA**TGCCTCCTCC**TTGGG** | 2252 | Coding | 141 |
| 113203 | **GCCGA**CAATACTTCC**CGAAT** | 2266 | Coding | 142 |

(continued)

| Nucleotide Sequences of Additional Chimeric (deoxy gapped) Phosphorothioate Oligonucleotides targeted to Human STAT3 | | | | |
|---|---|---|---|---|
| ISIS NO. | NUCLEOTIDE-SEQUENCE[1] (5' -> 3') | TARGET GENE NUCLEOTIDE CO-ORDINATES[2] | GENE TARGET REGION | SEQ ID NO: |
| 113204 | **GATGC**TCCTGGCTCT**CTGGC** | 2284 | Coding | 143 |
| 113205 | **TCAAT**GAATCTAAAG**CGCGG** | 2404 | Coding | 144 |
| 113206 | **GACTC**AAACTGCCCT**CCTGC** | 2462 | Coding | 145 |
| 113207 | **ATCAC**CCACATTCAC**TCATT** | 2710 | 3' UTR | 146 |
| 113208 | **AAAA**GTGCCCAGAT**TGC** | 2682 | 3' UTR | 147 |
| 113209 | **AAAAG**TGCCCAGATT**GCTCA** | 2679 | 3' UTR | 148 |
| 113210 | **TAAAA**GTGCCCAGAT**TGCTC** | 2680 | 3' UTR | 149 |
| 113211 | **AAGCA**GATCACCCAC**ATTCA** | 2716 | 3' UTR | 150 |

These oligonucleotides were tested in U266 cells or their ability to reduce STAT3 expression at an oligonucleotide concentration of 2.5 μM - U266 human myeloma cell lines (originally obtained from American Type Culture Collection) were maintained under standard conditions in supplemented RPMI 1640 medium. Cells (15 x $10^6$ cells in PBS) were transfected with oligonucleotides at 200V with a single 6-millisecond pulse using a BTX Electro Square Porator T820 (Genetronics, San Diego CA). The cells were incubated for 24 hours before RNA extraction.

Total cellular RNA was isolated using the RNeasy® kit (Qiagen, Santa Clarita, CA). Northern blot analysis was perfomed on 15 μg of RNA using a cDNA probe prepared from MB-MDA 468 RNA by standard RT-PCR followed by a nested primer reaction. Signals were quantitated using a Molecular Dynamics Phosphorimager™.

Results for selected compounds (expressed as percent of control mRNA expression and percent inhibition of mRNA expression) are shown in Table 10.

**TABLE 10**

| Inhibition of Human STAT3 mRNA expression in U266 Cells by Chimeric (deoxy gapped) Phosphorothioate Oligonucleotides | | | | |
|---|---|---|---|---|
| **ISIS No:** | **SEQ ID NO:** | **GENE TARGET REGION** | **% mRNA EXPRESSION** | **% mRNA INHIBITION** |
| None | -- | -- | 100 | -- |
| 17148 | 95 | Coding | 95. 1 | 4.9 |
| 17152 | 99 | 3' UTR | 82.5 | 17.5 |
| 113170 | 109 | AUG | 89.6 | 10.4 |
| 113171 | 110 | Coding | 110.2 | -- |
| 113172 | 111 | Coding | 96.1 | 3.9 |
| 113173 | 112 | Coding | 119 | -- |
| 113175 | 114 | Coding | 75.8 | 24.2 |
| 113176 | 115 | Coding | 72.3 | 27.7 |
| 113178 | 117 | Coding | 143-9 | -- |
| 113181 | 120 | Coding | 105.4 | -- |
| 113184 | 123 | Coding | 104.3 | -- |
| 113187 | 126 | Coding | 55.9 | 44.1 |
| 113189 | 128 | Coding | 163.9 | -- |

(continued)

| Inhibition of Human STAT3 mRNA expression in U266 Cells by Chimeric (deoxy gapped) Phosphorothioate Oligonucleotides | | | | |
|---|---|---|---|---|
| **ISIS No:** | **SEQ ID NO:** | **GENE TARGET REGION** | **% mRNA EXPRESSION** | **% mRNA INHIBITION** |
| 113199 | 139 | Coding | 64.4 | 35.6 |
| 113207 | 146 | 3' UTR | 123.6 | -- |
| 113209 | 148 | 3' UTR | 71.4 | 28.6 |
| 113210 | 149 | 3' UTR | 72.2 | 27.8 |
| 113211 | 150 | 3' UTR | 116.5 | -- |

Dose-response experiments were conducted using ISIS 113176, 129987, 113187, 129991, 113209, 129995, 113210 and 129999 as well as ISIS 17148 and the mouse STAT3 oligo ISIS 114054. A series of mismatch oligonucleotides, ISIS 129987, ISIS 114505, ISIS 129991, ISIS 129995 and ISIS 129999 were used as controls. Results are shown in Table 11.

**Table 11**

| Percent inhibition of human STAT3 mRNA expression with antisense oligonucleotides- dose response | | | | |
|---|---|---|---|---|
| | | **Percent inhibition of STAT3 expression** | | |
| **ISIS #** | **SEQ ID NO:** | **Oligo concentration** | | |
| | | **2.5 μM** | **5 μM** | **10 μM** |
| 17148 | 95 | 8 | 54 | 60 |
| 114054 | 403 | 4 | 17 | 15 |
| 113176 | 115 | 33 | 67 | 79 |
| 129987 | 404 | 5 | 5 | 29 |
| 113187 | 126 | 44 | 56 | 75 |
| 129991 | 405 | 21 | 22 | 26 |
| 113209 | 148 | 43 | 54 | 73 |
| 129995 | 406 | 5 | 32 | 25 |
| 113210 | 149 | 36 | 50 | 76 |
| 129999 | 407 | 31 | 8 | -- |

ISIS 17148 (SEQ ID 95), 113176 (SEQ ID 115), 113187 (SEQ ID 126), 113209 (SEQ ID 148) and 113210 (SEQ ID 149) reduced STAT3 expression by over 50% at one or more oligonucleotide concentration. These compounds are therefore preferred.

**Example 9: Antisense inhibition of STAT3 causes apoptotic cell death in mouse melanoma cells**

Mouse B16 melanoma cells were grown under standard conditions in supplemented RPMI 1640 medium(Life Technologies, Inc., Grand Island, NY).

Cells were treated with ISIS 17152 (SEQ ID 99), targeted to mouse STAT3, or the 3-base mismatch control, ISIS 28084 (AAAAAGAGGCCTGATTGCCC; SEQ ID NO: 151). Cells were transfected with oligonucleotide using LipofectAMINE™ PLUSJ reagent (GibcoBRL). Oligonucleotide was pre-complexed with LipofectAMINE™ PLUSJ by mixing the oligonucleotide with 100 ul serum-free RPMI 1640 medium, and subsequently adding 6 ul LipofectAMINE™ PLUS reagent.

The sample was mixed well and incubated for 15 minutes at room temperature. An additional 4 ul of LipofectAMINE™ PLUSJ reagent was diluted to 100 ul in serum-free RPMI. This diluted Lipof ectAMINE™ PLUSJ was mixed

with the pre-complexed oligonucleotide/LipofectAMINE™ PLUSJ mixture and incubated for 15 minutes at room temperature. 800 l of serum-free RPMI 1640 was added, and the resulting oligonucleotide-LipofectAMINE PLUSJ-medium mixture (approximately 1 ml) was added to cells in a 6-well plate. After 3 hours incubation, 1 ml of RPMI 1640 supplemened with 20% fetal bovine serum was added. The final oligonucleotide concentrations were 200 nM or 300 nM.

Cells were counted 24 hours post-transfection to determine the effect of antisense treatment on cell viability. Cells were harvested at 24 hours post-transfection for western blot analysis, and at 48 hours post-transfection for Annexin-V staining for apoptosis. Effects of oligonucleotide on cell number are shown in Table 12.

**Table 12**

| Effect of antisense inhibition of STAT3 on cell number | | | | |
|---|---|---|---|---|
| Expt | 200 nM | | 300 nM | |
| | **ISIS 28084 (3 mismatch)** | **ISIS 17152** | **ISIS 28084 (3 mismatch)** | **ISIS 17152** |
| 1 | $10.2 \times 10^5$ | $3.8 \times 10^5$ | | |
| 2 | $5.0 \times 10^5$ | $6.8 \times 10^5$ | $9.1 \times 10^5$ | $3.5 \times 10^5$ |
| 3 | $3.5 \times 10^5$ | $1.8 \times 10^5$ | $3.3 \times 10^5$ | $2.2 \times 10^5$ |

The data show that treatment with STAT3 antisense oligonucleotide results in increased cell death as compared to treatment with a mismatch control oligo.

Apoptosis in B16 cells was measured 48 hours after antisense treatment by staining with Annexin V-PE (Clontech, Palo Alto, CA), followed by flow cytometry analysis. Positive staining for Annexin-V is an indicator of apoptosis. Mock-transfected and control oligonucleotide-treated cells had 11% and 10% Annexin-V positive cells, respectively. In contrast, 30% ISIS 17152-treated cells were Annexin-V positive, indicating a nearly threefold increase in the number of apoptotic cells. It should be noted that this apparent increase is likely an underestimate of the number of cells that underwent apoptosis in response to treatment with the STAT3 oligo as dead cells are washed off in processing of the cells in the apoptosis assay.

Western blot analysis was performed to detect STAT3 using methods known to those skilled in the art on cells harvested 24 hours after antisense treatment. Anti-STAT3 antibody was purchased from K15, Santa Cruz Biotechnology, Santa Cruz, CA. ISIS 17152 at 200nM or 300 nM significantly reduced STAT3 protein production in B16 cells.

**Example 10: Effect of STAT3 antisense oligonucleotides on leukemic large granular lymphocytes (LGL)**

LGL leukemia is a lymphoproliferative disease with autoimmune features and LGL cells are known to not undergo Fas-dependent apoptosis, despite high levels of Fas and FasL expression. (Lamy et al., Blood, 1998, 92, 4771-7). STAT3 antisense oligonucleotides were tested for their ability to sensitize LGL cells to the apoptotic signals in these cells.

LGL leukemic cells were obtained from patients who met the clinical criteria of T cell (CD3+) LGL leukemia with increased LGL counts and clonal TCR gene rearrangements. All patients had chronic disease not requiring treatment at the time of analysis. Purified leukemic LGL cells ($2 \times 10^6$) were plated in 24-well plates in 0.5mL of complete medium (supplemented RPMI-1640, from Gibco Life Technologies, Gaithersburg, MD). Cells were incubated with either ISIS 17148 antisense oligonucleotide (SEQ ID NO: 95) or the control oligonucleotide, ISIS 16094 (SEQ ID NO: 152). Antisense oligonucleotide was delivered to LGL leukemic cells was by passive uptake. No transfection reagents were used for delivery.

Extracts were prepared from the LGL cells each of three patients treated with a 1 uM dose of either STAT3 (ISIS 17148, SEQ ID 95) or control mismatch antisense oligonucleotides. Western blots were performed to determine STAT3 protein levels. A reduction in STAT3 protein levels ranged from 25 to 45% as compared to paied controls treated with the mismatch oligo.

Induction apoptosis in LGL cells treated with STAT3 (ISIS 17148, SEQ ID 95) or control mismatch antisense oligonucleotides at doses of 1, 2 and 5 uM was measured by flow cytometry. Apoptosis was significantly increased in the STAT 3 antisense treated cells and was dose dependent. Measurements of percent specific apoptosis in duplicate reactions revealed an increase in apoptosis from about 5% in untreated cells to levels of 6, 17 and 24% in STAT3 antisense-treated cells at 1, 2, and 5 uM, respectively. Levels of apoptosis in control oligonucleotide treated cells remained at about 6% at all doses. Apoptosis was believed to be activated via a Fas-mediated pathway.

**Example 11**

**Induction of apoptosis in the human myeloma cell line U266 following STAT3 antisense oligonucleotide treatment**

Mutilple myeloma (MM) is a hayperproliferative disorder. Previous studied demonstrated an increase in apoptosis in an MM cell line U266 in response to expression of a dominant negative STAT3 that lacks an intact transactivation domain.

Antisense STAT3 oligonucleotides ISIS 17148 (SEQ ID 95) and ISIS 113176 (SEQ ID 115) were tested for their ability to increase apoptosis in U266 cells as compared to a mismatch control oligonucleotide. Various amounts of oligonucleotide were delivered to cultured cells by transfection. Cells were harvested 48 hours post-transfection to determine STAT3 mRNA and protein levels by northern and western blot, respectively. A significant dose dependent reduction in STAT3 mRNA and proteing were observed in response to administration of both STAT3 antisense oligonucleotides. The mismatch control oligonucleotide had no significant effect on STAT3 mRNA or protein levels.

Cells were also assayed for an increase in apoptosis in response to the STAT3 antisense oligonucleotides by Annexin 5 staining and flow cytometry as described above.

A significant increase in apoptosis was seen in the cells transfected with both ISIS 17148 and ISIS 113176. No significant change in apoptosis was observed in the cells treated with the mismatch control oligonucleotide.

**Example 12**

**Antisense inhibition of human STAT3 by chimeric phosphorothioate oligonucleotides having 2'-MOE wings and a deoxy gap**

In accordance with the present invention, an additional series of oligonucleotides was designed to target different regions of the human STAT 3, using published sequences (GenBank® accession number L29277, incorporated herein as SEQ ID NO: 1, the complement of nucleotides 4189213 to 4263636 of the sequence with the GenBank® accession number NT_010755.13, incorporated herein as SEQ ID NO: 153 and GenBank® accession number NM_139276.1, incorporated herein as SEQ ID NO: 154). The oligonucleotides are shown in Table 13. "Target site" indicates the first (5'-most) nucleotide number on the particular target sequence to which the oligonucleotide binds. All compounds in Table 13 are chimeric oligonucleotides ("gapmers") 20 nucleotides in length, composed of a central "gap" region consisting of ten 2'-deoxynucleotides, which is flanked on both sides (5' and 3' directions) by five-nucleotide "wings". The wings are composed of 2'-O-methoxyethyl (2'-MOE)nucleotides. The internucleoside (backbone) linkages are phosphorothioate (P=S) throughout the oligonucleotide. All cytidine residues are 5-methylcytidines.

The compounds were analyzed for their effect on STAT3 mRNA levels in A549 cells. The human lung carcinoma cell line A549 was obtained from the American Type Culture Collection (ATCC) (Manassas, VA). A549 cells were routinely cultured in DMEM basal media (Invitrogen Life Technologies, Carlsbad, CA) supplemented with 10% fetal boving serum (Invitrogen Life Technologies, Carlsbad, CA), 100 units/mL penicillin, and 100 ug/mL streptomycin (Invitrogen Life Technologies, Carlsbad, CA). Cells were routinely passaged by trypsinization and dilution when they reached 90% confluence.

ISIS 18078 was used as a control oligonucleotide and was used at 75nM. ISIS 18078 (GTGCGCGCGAGCCCGAAATC, SEQ ID NO: 155) is an chimeric oligonucleotide ("gapmers") 20 nucleotides in length, composed of a central "gap" region consisting of nine 2'-deoxynucleotides, which is flanked on both sides (5' and 3' directions) by five-nucleotide and six-nucleotide "wings", respectively. The wings are composed of 2'-O-methoxyethyl (2'-MOE)nucleotides. The internucleoside (backbone) linkages are phosphorothioate (P=S) throughout the oligonucleotide. All cytidine residues are 5-methylcytidines.

When cells reached 65-75% confluency, they were treated with oligonucleotide. For cells grown in 96-well plates, wells were washed once with 100 L OPTI-MEM-1™ reduced-serum medium (Invitrogen Life Technologies, Carlsbad, CA) and then treated with 130 L of OPTI-MEM-1™ containing 3.75 g/mL LIPOFECTIN™ (Invitrogen Life Technologies, Carlsbad, CA) and 75 nM of the compounds in Table 13. Cells were treated and data were obtained in duplicate. Untreated cells served as controls. After 4-7 hours of treatment at 37°C, the medium was replaced with fresh medium. Cells were harvested 16-24 hours after oligonucleotide treatment. STAT3 mRNA levels in A549 cells were quantitated by real-time PCR as described by other methods herein.

Probes and primers to human STAT3 were designed to hybridize to a human STAT3 sequence, using published sequence information (incorporated herein as SEQ ID NO: 1). For primer-probe set 199 (PPS199) the PCR primers were: forward primer: ACATGCCACTTTGGTGTTTCATAA (SEQ ID NO: 156) reverse primer: TCTTCGTAGATTGTGCTGATAGAGAAC (SEQ ID NO: 157) and the PCR probe was: FAM-CAGTATAGCCGCTTCCTGCAAGAGTCGAA-TAMRA (SEQ ID NO: 158) where FAM is the fluorescent reporter dye and TAMRA is the quencher dye. Gene target quantities obtained by real time RT-PCR are normalized by quantifying total RNA using RiboGreen™ (Molecular Probes, Inc. Eugene, OR). In this assay, 170 μL of RiboGreen™ working reagent (RiboGreen™ reagent diluted 1:350 in 10mM Tris-HCl, 1 mM EDTA, pH 7.5) is pipetted into a 96-well plate containing 30 μL purified, cellular RNA. The plate is read in a CytoFluor® 4000 (PE Applied Biosystems, Foster City, CA) with excitation at 485nm and emission at 530nm.

The results of the antisense oligonucleotide treatments are the average of 2 experiments and are shown in Table 13. Data are expressed as percent inhibition relative to untreated control cells.

**Table 13**

| Inhibition of human STAT 3 mRNA levels by chimeric phosphorothioate oligonucleotides having 2'-MOE wings and a deoxy gap | | | | | | |
|---|---|---|---|---|---|---|
| **Isis #** | **Region** | **Target Seq ID No** | **Target Site** | **Sequence** | **% Inhib** | **Seq ID No** |
| 337245 | intron | 153 | 6814 | AGCCTCTGCACCCTCATGTT | 77 | 159 |
| 337246 | intron | 153 | 6868 | CTCCTAAATTAAGAACTTCT | 37 | 160 |
| 337247 | intron | 153 | 14801 | TTTTGCATGATGTAACCACT | 87 | 161 |
| 337248 | intron | 153 | 34820 | TATTGAAAATTATCTAATTC | 0 | 162 |
| 337249 | coding | 153 | 40369 | TTGGGCCATCCTGCTAAAAT | 48 | 163 |
| 337250 | exon: intron | 153 | 50156 | ATTCACTTGCCTCCTTGACT | 51 | 164 |
| 337251 | intron: exon | 153 | 51124 | ATGCCCTTACTCTCCGCATC | 74 | 165 |
| 337252 | exon: intron | 153 | 59140 | CTGAACTTACCCTCTGAGAG | 60 | 166 |
| 337253 | exon: intron | 153 | 64176 | AAATGCGGACCCAAGAGTTT | 49 | 167 |
| 337254 | 5'UTR | 1 | 56 | CTTGTTCCCTCGGCTGCGAC | 57 | 168 |
| 337255 | 5'UTR | 1 | 79 | GCCTGTCCAGGATCCGGTTG | 75 | 169 |
| 337256 | 5'UTR | 1 | 126 | GAAGGGCCTCTCCGAGCCGA | 67 | 170 |
| 337257 | 5'UTR | 1 | 148 | GGCGGCGAGGCTCCCTCAGG | 80 | 171 |
| 337258 | 5'UTR | 1 | 193 | TCCGGCAGAGGCCGAGAGGC | 56 | 172 |
| 337259 | 5'UTR | 154 | 225 | CCATCCTGCTAAAATCAGGG | 58 | 173 |
| 337260 | 5'UTR | 154 | 233 | CCATTGGGCCATCCTGCTAA | 62 | 174 |
| 337261 | coding | 1 | 235 | TGTCAAGCTGCTGTAGCTGA | 79 | 175 |
| 337262 | coding | 1 | 299 | AACTGCCGCAGCTCCATTGG | 74 | 176 |
| 337263 | coding | 1 | 326 | TCTTGACTCTCAATCCAAGG | 79 | 177 |
| 337264 | coding | 1 | 339 | CGCATATGCCCAATCTTGAC | 81 | 178 |
| 337265 | coding | 1 | 426 | CGACTCTTGCAGGAAGCGGC | 92 | 179 |
| 337266 | coding | 1 | 453 | TCGTAGATTGTGCTGATAGA | 61 | 180 |
| 337267 | coding | 1 | 470 | AGAAACTGCTTGATTCTTCG | 62 | 181 |
| 337268 | coding | 1 | 484 | GATACCTGCTCTGAAGAAAC | 75 | 182 |
| 337269 | coding | 1 | 491 | TTCTCAAGATACCTGCTCTG | 74 | 183 |
| 337270 | coding | 1 | 496 | TTGGCTTCTCAAGATACCTG | 89 | 184 |
| 337271 | coding | 1 | 541 | GTGATTCTTCCCACAGGCAC | 85 | 185 |
| 337272 | coding | 1 | 629 | ATCTGCTGCTTCTCCGTCAC | 74 | 186 |
| 337273 | coding | 1 | 634 | CCAGCATCTGCTGCTTCTCC | 73 | 187 |
| 337274 | coding | 1 | 647 | TGAAGGTGCTGCTCCAGCAT | 74 | 188 |
| 337275 | coding | 1 | 683 | TTCTGTTCTAGATCCTGCAC | 82 | 189 |
| 337276 | coding | 1 | 708 | CTGGAGATTCTCTACCACTT | 91 | 190 |

(continued)

| Inhibition of human STAT 3 mRNA levels by chimeric phosphorothioate oligonucleotides having 2'-MOE wings and a deoxy gap | | | | | | |
|---|---|---|---|---|---|---|
| **Isis #** | **Region** | **Target Seq ID No** | **Target Site** | **Sequence** | **% Inhib** | **Seq ID No** |
| 337277 | coding | 1 | 716 | AAGTCATCCTGGAGATTCTC | 79 | 191 |
| 337278 | coding | 1 | 721 | AATCAAAGTCATCCTGGAGA | 69 | 192 |
| 337279 | coding | 1 | 726 | GTTGAAATCAAAGTCATCCT | 78 | 193 |
| 337280 | coding | 1 | 731 | TTATAGTTGAAATCAAAGTC | 45 | 194 |
| 337281 | coding | 1 | 736 | GGGTTTTATAGTTGAAATCA | 16 | 195 |
| 337282 | coding | 1 | 741 | CTTGAGGGTTTTATAGTTGA | 58 | 196 |
| 337283 | coding | 1 | 746 | TGACTCTTGAGGGTTTTATA | 71 | 197 |
| 337284 | coding | 1 | 751 | CTCCTTGACTCTTGAGGGTT | 91 | 198 |
| 337285 | coding | 1 | 756 | CATGTCTCCTTGACTCTTGA | 78 | 199 |
| 337286 | coding | 1 | 768 | ATTCAGATCTTGCATGTCTC | 77 | 200 |
| 337287 | coding | 1 | 779 | TGGTTGTTTCCATTCAGATC | 82 | 201 |
| 337288 | coding | 1 | 790 | TGGTCACTGACTGGTTGTTT | 84 | 202 |
| 337289 | coding | 1 | 812 | TCCAGCTGCTGCATCTTCTG | 83 | 203 |
| 337290 | coding | 1 | 822 | GAGCATCTGTTCCAGCTGCT | 80 | 204 |
| 337291 | coding | 1 | 848 | CTTCTCCGCATCTGGTCCAG | 66 | 205 |
| 337292 | coding | 1 | 899 | TTCTGCACGTACTCCATCGC | 81 | 206 |
| 337293 | coding | 1 | 925 | CAGCCAGCTCCTCGTCCGTG | 92 | 207 |
| 337294 | coding | 1 | 935 | CTCTTCCAGTCAGCCAGCTC | 75 | 208 |
| 337295 | coding | 1 | 941 | TGCCGCCTCTTCCAGTCAGC | 82 | 209 |
| 337296 | coding | 1 | 999 | CCAGTTTTCTAGCCGATCTA | 80 | 210 |
| 337297 | coding | 1 | 1006 | ACGTTATCCAGTTTTCTAGC | 72 | 211 |
| 337298 | coding | 1 | 1025 | AGTTGAGATTCTGCTAATGA | 74 | 212 |
| 337299 | coding | 1 | 1030 | TCTGAAGTTGAGATTCTGCT | 80 | 213 |
| 337300 | coding | 1 | 1085 | CCTTTGTAGGAAACTTTTTG | 23 | 214 |
| 337301 | coding | 1 | 1162 | AGGCACTTTTCATTAAGTTT | 73 | 215 |
| 337302 | coding | 1 | 1262 | TTGACCAGCAACCTGACTTT | 61 | 216 |
| 337303 | coding | 1 | 1286 | AGCTGATAATTCAACTCAGG | 85 | 217 |
| 337304 | coding | 1 | 1291 | TTTTAAGCTGATAATTCAAC | 15 | 218 |
| 337305 | coding | 1 | 1297 | CTTTAATTTTAAGCTGATAA | 25 | 219 |
| 337306 | coding | 1 | 1302 | GCACACTTTAATTTTAAGCT | 77 | 220 |
| 337307 | coding | 1 | 1307 | TCAATGCACACTTTAATTTT | 53 | 221 |
| 337308 | coding | 1 | 1364 | CCCAGAATGTTAAATTTCCG | 70 | 222 |
| 337309 | coding | 1 | 1414 | AGAGGCTGCCGTTGTTGGAT | 73 | 223 |
| 337310 | coding | 1 | 1433 | AAGTGTTTGAATTCTGCAGA | 73 | 224 |
| 337311 | coding | 1 | 1452 | TCTCTGCTCCCTCAGGGTCA | 61 | 225 |

(continued)

| Inhibition of human STAT 3 mRNA levels by chimeric phosphorothioate oligonucleotides having 2'-MOE wings and a deoxy gap | | | | | | |
|---|---|---|---|---|---|---|
| Isis # | Region | Target Seq ID No | Target Site | Sequence | % Inhib | Seq ID No |
| 337312 | coding | 1 | 1517 | ATCAGGTGCAGCTCCTCAGT | 78 | 226 |
| 337313 | coding | 1 | 1522 | AGGTGATCAGGTGCAGCTCC | 61 | 227 |
| 337314 | coding | 1 | 1527 | CTCAAAGGTGATCAGGTGCA | 75 | 228 |
| 337315 | coding | 154 | 1571 | GAGGCCTTGGTGATACACCT | 46 | 229 |
| 337316 | coding | 154 | 1579 | TCAATCTTGAGGCCTTGGTG | 59 | 230 |
| 337317 | coding | 154 | 1584 | CTAGGTCAATCTTGAGGCCT | 55 | 231 |
| 337318 | coding | 1 | 1569 | GGTCTCTAGGTCAATCTTGA | 74 | 232 |
| 337319 | coding | 1 | 1577 | AAGGAGTGGGTCTCTAGGTC | 38 | 233 |
| 337320 | coding | 154 | 1602 | CTGGCAAGGAGTGGGTCTCT | 74 | 234 |
| 337321 | coding | 154 | 1609 | ACCACAACTGGCAAGGAGTG | 80 | 235 |
| 337322 | coding | 1 | 1609 | TCTGACAGATGTTGGAGATC | 69 | 236 |
| 337323 | coding | 1 | 1614 | TGGCATCTGACAGATGTTGG | 79 | 237 |
| 337324 | coding | 1 | 1619 | GCATTTGGCATCTGACAGAT | 79 | 238 |
| 337325 | coding | 1 | 1667 | TTCTTGGGATTGTTGGTCAG | 75 | 239 |
| 337326 | coding | 1 | 1778 | GTCAGCTGCTCGATGCTCAG | 78 | 240 |
| 337327 | coding | 154 | 1823 | TCCCAAGAGTTTCTCTGCCA | 84 | 241 |
| 337328 | coding | 1 | 1838 | CATGTGATCTGACACCCTGA | 85 | 242 |
| 337329 | coding | 1 | 1843 | TAGCCCATGTGATCTGACAC | 88 | 243 |
| 337330 | coding | 154 | 1885 | GCCATGTTTTCTTTGCAAAA | 59 | 244 |
| 337331 | coding | 1 | 1873 | CCTTGCCAGCCATGTTTTCT | 88 | 245 |
| 337332 | coding | 1 | 1878 | GAAGCCCTTGCCAGCCATGT | 91 | 246 |
| 337333 | coding | 154 | 1903 | AAGGAGAAGCCCTTGCCAGC | 90 | 247 |
| 337334 | coding | 154 | 1908 | CCCAGAAGGAGAAGCCCTTG | 85 | 248 |
| 337335 | coding | 154 | 1918 | TCCAGCCAGACCCAGAAGGA | 86 | 249 |
| 337336 | coding | 1 | 2048 | TCTTTGCTGCTTTCACTGAA | 79 | 250 |
| 337337 | coding | 1 | 2144 | ATGTTGTTCAGCTGCTGCTT | 76 | 251 |
| 337338 | coding | 1 | 2149 | ATGACATGTTGTTCAGCTGC | 80 | 252 |
| 337339 | coding | 1 | 2154 | AGCAAATGACATGTTGTTCA | 84 | 253 |
| 337340 | coding | 1 | 2159 | ATTTCAGCAAATGACATGTT | 72 | 254 |
| 337341 | coding | 1 | 2164 | TGATGATTTCAGCAAATGAC | 74 | 255 |
| 337342 | coding | 1 | 2174 | TTATAGCCCATGATGATTTC | 81 | 256 |
| 337343 | coding | 1 | 2179 | TGATCTTATAGCCCATGATG | 84 | 257 |
| 337344 | coding | 1 | 2184 | ATCCATGATCTTATAGCCCA | 90 | 258 |
| 337345 | coding | 1 | 2190 | GGTAGCATCCATGATCTTAT | 86 | 259 |
| 337346 | coding | 1 | 2232 | AATGTCAGGATAGAGATAGA | 55 | 260 |

(continued)

| Inhibition of human STAT 3 mRNA levels by chimeric phosphorothioate oligonucleotides having 2'-MOE wings and a deoxy gap | | | | | | |
|---|---|---|---|---|---|---|
| Isis # | Region | Target Seq ID No | Target Site | Sequence | % Inhib | Seq ID No |
| 337347 | coding | 1 | 2246 | GCCTCCTCCTTGGGAATGTC | 88 | 261 |
| 337348 | coding | 154 | 2273 | TCCGAATGCCTCCTCCTTGG | 92 | 262 |
| 337349 | coding | 154 | 2278 | TACTTTCCGAATGCCTCCTC | 65 | 263 |
| 337350 | coding | 154 | 2283 | GACAATACTTTCCGAATGCC | 84 | 264 |
| 337351 | coding | 1 | 2303 | CTACCTGGGTCAGCTTCAGG | 74 | 265 |
| 337352 | coding | 1 | 2333 | ATAAACTTGGTCTTCAGGTA | 80 | 266 |
| 337353 | coding | 1 | 2351 | GTCGTTGGTGTCACACAGAT | 81 | 267 |
| 337354 | coding | 1 | 2356 | TGCAGGTCGTTGGTGTCACA | 62 | 268 |
| 337355 | coding | 1 | 2361 | ATTGCTGCAGGTCGTTGGTG | 61 | 269 |
| 337356 | coding | 1 | 2366 | ATGGTATTGCTGCAGGTCGT | 75 | 270 |
| 337357 | coding | 1 | 2371 | GGTCAATGGTATTGCTGCAG | 71 | 271 |
| 337358 | coding | 1 | 2381 | GACATCGGCAGGTCAATGGT | 77 | 272 |
| 337359 | coding | 154 | 2423 | CAATGAATCTAAAGTGCGGG | 57 | 273 |
| 337360 | coding | 154 | 2428 | TGCATCAATGAATCTAAAGT | 71 | 274 |
| 337361 | coding | 1 | 2413 | CAAACTGCATCAATGAATCT | 61 | 275 |
| 337362 | coding | 1 | 2418 | ATTTCCAAACTGCATCAATG | 69 | 276 |
| 337363 | coding | 1 | 2456 | AACTGCCCTCCTGCTGAGGG | 63 | 277 |
| 337364 | coding | 1 | 2469 | GGTGAGGGACTCAAACTGCC | 70 | 278 |
| 337365 | 3'UTR | 1 | 2550 | CAGTCGTATCTTTCTGCAGC | 84 | 279 |
| 337366 | 3'UTR | 1 | 2658 | AGATAGCAGAAGTAGGAGAT | 66 | 280 |
| 337367 | 3'UTR | 1 | 2678 | AAAGTGCCCAGATTGCTCAA | 82 | 281 |
| 337368 | 3'UTR | 1 | 2684 | TTTTTAAAAGTGCCCAGATT | 59 | 282 |
| 337369 | 3'UTR | 1 | 2713 | CAGATCACCCACATTCACTC | 88 | 283 |
| 337370 | 3'UTR | 1 | 2729 | TGCATTTAGATAAAAGCAGA | 78 | 284 |
| 337371 | 3'UTR | 1 | 2744 | GAACACATCCTTATTTGCAT | 76 | 285 |
| 337372 | 3'UTR | 1 | 2759 | ATCATGGGTCTCAGAGAACA | 88 | 286 |
| 337373 | 3'UTR | 154 | 2790 | CACATCCCCTGATCATGGGT | 70 | 287 |
| 337374 | 3'UTR | 154 | 2826 | AGACATTTCCTTTTTCTCCC | 67 | 288 |
| 337375 | 3'UTR | 154 | 2908 | ACCAGGAGGCACTTGTCTAA | 89 | 289 |
| 337376 | 3'UTR | 154 | 2914 | GCAGGCACCAGGAGGCACTT | 83 | 290 |
| 337377 | 3'UTC | 154 | 2941 | GCTTACAGAAACAGGCAGAA | 78 | 291 |
| 337378 | 3'UTR | 154 | 2959 | AGGTGGCCTGTGGCATTTGC | 16 | 292 |
| 337379 | 3'UTR | 154 | 2971 | GTATGTAGCTATAGGTGGCC | 71 | 293 |
| 337380 | 3'UTR | 154 | 2983 | GCAATGCCAGGAGTATGTAG | 83 | 294 |
| 337381 | 3'UTR | 154 | 2992 | TTAAAAAGTGCAATGCCAGG | 86 | 295 |

(continued)

| Inhibition of human STAT 3 mRNA levels by chimeric phosphorothioate oligonucleotides having 2'-MOE wings and a deoxy gap | | | | | | |
|---|---|---|---|---|---|---|
| Isis # | Region | Target Seq ID No | Target Site | Sequence | % Inhib | Seq ID No |
| 337382 | 3'UTR | 154 | 3032 | GGCTTAGATAGTCCTATCTT | 84 | 296 |
| 337383 | 3'UTR | 154 | 3047 | TAAAAAGAAACCTAGGGCTT | 81 | 297 |
| 337384 | 3'UTR | 154 | 3108 | ATACAGAAAGGCTATGCTGA | 89 | 298 |
| 337385 | 3'UTR | 154 | 3121 | TTAAGTTTCTTAAATACAGA | 70 | 299 |

As shown in Table 13, SEQ ID Nos 159, 161, 165, 166, 169, 170, 171, 174, 175, 176, 177, 178, 179, 180, 181, 182, 183, 184, 185, 186, 187, 188, 189, 190, 191, 192, 193, 197, 198, 199, 200, 201, 202, 203, 204, 205, 206, 207, 208, 209, 210, 211, 212, 213, 215, 216, 217, 220, 222, 223, 224, 225, 226, 227, 228, 232, 234, 235, 236, 237, 238, 239, 240, 241, 242, 243, 245, 246, 247, 248, 249, 250, 251, 252, 253, 254, 255, 256, 257, 258, 259, 261, 262, 263, 264, 265, 266, 267, 268, 269, 270, 271, 272, 274, 275, 276, 277, 278, 279, 280, 281, 283, 284, 285, 286, 287, 288, 289, 290, 291, 293, 294, 295, 296, 297, 298 and 299 inhibited human STAT3 expression at least 60%.

**Example 13**

**Chimeric phosphorothioate oligonucleotides targeted to human STAT3 having 2'-MOE wings and a deox gap**

In accordance with the present disclosure, an additional series of oligonucleotides was designed to target different regions of the human STAT 3, using published sequences (GenBank® accession number L29277, incorporated herein as SEQ ID NO: 1, GenBank® accession number NM_139276.1, incorporated herein as SEQ ID NO: 154). The oligonucleotides are shown in Table 14. "Target site" indicates the first (5'-most) nucleotide number on the particular target sequence to which the oligonucleotide binds. All compounds in Table 14 are chimeric oligonucleotides ("gapmers") 20 nucleotides in length, composed of a central "gap" region consisting of ten 2'-deoxynucleotides, which is flanked on both sides (5' and 3' directions) by five-nucleotide "wings". The wings are composed of 2'-O-methoxyethyl (2'-MOE)nucleotides. The internucleoside (backbone) linkages are phosphorothioate (P=S) throughout the oligonucleotide. All cytidine residues are 5-methylcytidines.

The compounds were tested for their effects on human STAT3 expression in A549 cells. Cells were treated with 50 nM of antisense oligonucleotides mixed with 1.5 μL/mg LIPOFECTIN™. Cells were harvested approximately 20 hours following transfection and STAT3 mRNA expression was measured using real-time PCR. The data are the average of 2 experiments and are shown in Table 14 as percent inhibition relative to untreated control cells.

**Table 14**

| Chimeric phosphorothioate oligonucleotides targeted to human STAT3 having 2'-MOE wings and a deoxy gap | | | | | | |
|---|---|---|---|---|---|---|
| Isis # | Region | Target Seq ID No | Target Site | Sequence | % Inhib | Seq ID No |
| 345752 | Coding | 1 | 631 | GCATCTGCTGCTTCTCCGTC | 54 | 300 |
| 345753 | Coding | 1 | 633 | CAGCATCTGCTGCTTCTCCG | 40 | 301 |
| 345754 | Coding | 1 | 635 | TCCAGCATCTGCTGCTTCTC | 47 | 302 |
| 345755 | Coding | 1 | 636 | CTCCAGCATCTGCTGCTTCT | 22 | 303 |
| 345756 | Coding | 1 | 638 | TGCTCCAGCATCTGCTGCTT | 29 | 304 |
| 345757 | Coding | 1 | 641 | TGCTGCTCCAGCATCTGCTG | 37 | 305 |
| 345758 | Coding | 1 | 643 | GGTGCTGCTCCAGCATCTGC | 42 | 306 |
| 345759 | Coding | 1 | 645 | AAGGTGCTGCTCCAGCATCT | 35 | 307 |
| 345760 | Coding | 1 | 1663 | TGGGATTGTTGGTCAGCATG | 23 | 308 |

(continued)

| Chimeric phosphorothioate oligonucleotides targeted to human STAT3 having 2'-MOE wings and a deoxy gap | | | | | | |
|---|---|---|---|---|---|---|
| Isis # | Region | Target Seq ID No | Target Site | Sequence | % Inhib | Seq ID No |
| 345761 | Coding | 1 | 1668 | ATTCTTGGGATTGTTGGTCA | 42 | 309 |
| 345762 | Coding | 1 | 1670 | ACATTCTTGGGATTGTTGGT | 42 | 310 |
| 345763 | Coding | 1 | 1671 | CACATTCTTGGGATTGTTGG | 43 | 311 |
| 345764 | Coding | 1 | 1673 | TTCACATTCTTGGGATTGTT | 35 | 312 |
| 345765 | Coding | 1 | 1675 | AGTTCACATTCTTGGGATTG | 27 | 313 |
| 345766 | Coding | 1 | 1677 | GAAGTTCACATTCTTGGGAT | 0 | 314 |
| 345767 | Coding | 1 | 380 | AGATTATGAAACACCAAAGT | 18 | 315 |
| 345768 | Coding | 1 | 382 | GGAGATTATGAAACACCAAA | 7 | 316 |
| 345769 | Coding | 1 | 384 | CAGGAGATTATGAAACACCA | 38 | 317 |
| 345770 | Coding | 1 | 387 | TCCCAGGAGATTATGAAACA | 46 | 318 |
| 345771 | Coding | 1 | 388 | CTCCCAGGAGATTATGAAAC | 19 | 319 |
| 345772 | Coding | 1 | 390 | CTCTCCCAGGAGATTATGAA | 32 | 320 |
| 345773 | Coding | 1 | 392 | ATCTCTCCCAGGAGATTATG | 20 | 321 |
| 345774 | Coding | 1 | 1872 | CTTGCCAGCCATGTTTTCTT | 43 | 322 |
| 345775 | Coding | 1 | 1874 | CCCTTGCCAGCCATGTTTTC | 26 | 323 |
| 345776 | Coding | 1 | 1876 | AGCCCTTGCCAGCCATGTTT | 57 | 324 |
| 345777 | Coding | 1 | 1880 | GAGAAGCCCTTGCCAGCCAT | 60 | 325 |
| 345778 | Coding | 1 | 1882 | AGGAGAAGCCCTTGCCAGCC | 73 | 326 |
| 345779 | Coding | 154 | 1904 | GAAGGAGAAGCCCTTGCCAG | 60 | 327 |
| 345780 | Coding | 1 | 1877 | AAGCCCTTGCCAGCCATGTT | 61 | 328 |
| 345781 | Coding | 1 | 1879 | AGAAGCCCTTGCCAGCCATG | 74 | 329 |
| 345782 | Coding | 154 | 1905 | AGAAGGAGAAGCCCTTGCCA | 52 | 330 |
| 345783 | Coding | 154 | 1907 | CCAGAAGGAGAAGCCCTTGC | 39 | 331 |
| 345784 | Coding | 154 | 1909 | ACCCAGAAGGAGAAGCCCTT | 48 | 332 |
| 345785 | Coding | 1 | 2247 | TGCCTCCTCCTTGGGAATGT | 69 | 333 |
| 345786 | Coding | 1 | 2249 | AATGCCTCCTCCTTGGGAAT | 3 | 334 |
| 345787 | Coding | 1 | 2251 | CGAATGCCTCCTCCTTGGGA | 54 | 335 |
| 345788 | Coding | 154 | 2274 | TTCCGAATGCCTCCTCCTTG | 42 | 336 |
| 345789 | Coding | 154 | 2275 | TTTCCGAATGCCTCCTCCTT | 1 | 337 |
| 345790 | Coding | 154 | 2277 | ACTTTCCGAATGCCTCCTCC | 26 | 338 |
| 345791 | Coding | 1 | 420 | TTGCAGGAAGCGGCTATACT | 24 | 339 |
| 345792 | Coding | 1 | 422 | TCTTGCAGGAAGCGGCTATA | 42 | 340 |
| 345793 | Coding | 1 | 424 | ACTCTTGCAGGAAGCGGCTA | 51 | 341 |
| 345794 | Coding | 1 | 425 | GACTCTTGCAGGAAGCGGCT | 60 | 342 |
| 345795 | Coding | 1 | 427 | TCGACTCTTGCAGGAAGCGG | 42 | 343 |

(continued)

| Chimeric phosphorothioate oligonucleotides targeted to human STAT3 having 2'-MOE wings and a deoxy gap | | | | | | |
|---|---|---|---|---|---|---|
| Isis # | Region | Target Seq ID No | Target Site | Sequence | % Inhib | Seq ID No |
| 345796 | Coding | 1 | 428 | TTCGACTCTTGCAGGAAGCG | 51 | 344 |
| 345797 | Coding | 1 | 430 | CATTCGACTCTTGCAGGAAG | 50 | 345 |
| 345798 | Coding | 1 | 2176 | TCTTATAGCCCATGATGATT | 23 | 346 |
| 345799 | Coding | 1 | 2178 | GATCTTATAGCCCATGATGA | 36 | 347 |
| 345800 | Coding | 1 | 2180 | ATGATCTTATAGCCCATGAT | 30 | 348 |
| 345801 | Coding | 1 | 2182 | CCATGATCTTATAGCCCATG | 57 | 349 |
| 345802 | Coding | 1 | 2186 | GCATCCATGATCTTATAGCC | 51 | 350 |
| 345803 | Coding | 1 | 2188 | TAGCATCCATGATCTTATAG | 31 | 351 |
| 345804 | Coding | 1 | 2189 | GTAGCATCCATGATCTTATA | 11 | 352 |
| 345805 | 3'UTR | 154 | 3102 | AAAGGCTATGCTGATACAGT | 62 | 353 |
| 345806 | 3'UTR | 154 | 3104 | AGAAAGGCTATGCTGATACA | 46 | 354 |
| 345807 | 3'UTR | 154 | 3106 | ACAGAAAGGCTATGCTGATA | 56 | 355 |
| 345808 | 3'UTR | 154 | 3107 | TACAGAAAGGCTATGCTGAT | 46 | 356 |
| 345809 | 3'UTR | 154 | 3109 | AATACAGAAAGGCTATGCTG | 51 | 357 |
| 345810 | 3'UTR | 154 | 3110 | AAATACAGAAAGGCTATGCT | 27 | 358 |
| 345811 | 3'UTR | 154 | 3112 | TTAAATACAGAAAGGCTATG | 3 | 359 |
| 345812 | 3'UTR | 154 | 3114 | TCTTAAATACAGAAAGGCTA | 41 | 360 |
| 345813 | 3'UTR | 1 | 2753 | GGTCTCAGAGAACACATCCT | 56 | 361 |
| 345814 | 3'UTR | 1 | 2755 | TGGGTCTCAGAGAACACATC | 31 | 362 |
| 345815 | 3'UTR | 1 | 2757 | CATGGGTCTCAGAGAACACA | 68 | 363 |
| 345816 | 3'UTR | 1 | 2758 | TCATGGGTCTCAGAGAACAC | 42 | 364 |
| 345817 | 3'UTR | 1 | 2761 | TGATCATGGGTCTCAGAGAA | 65 | 365 |
| 345818 | 3'UTR | 1 | 2763 | CCTGATCATGGGTCTCAGAG | 22 | 366 |
| 345819 | 3'UTR | 1 | 2765 | CCCCTGATCATGGGTCTCAG | 38 | 367 |
| 345820 | Coding | 154 | 1912 | CAGACCCAGAAGGAGAAGCC | 40 | 368 |
| 345821 | Coding | 154 | 1914 | GCCAGACCCAGAAGGAGAAG | 52 | 90 |
| 345822 | Coding | 154 | 1916 | CAGCCAGACCCAGAAGGAGA | 49 | 369 |
| 345823 | Coding | 154 | 1917 | CCAGCCAGACCCAGAAGGAG | 64 | 370 |
| 345824 | Coding | 154 | 1919 | GTCCAGCCAGACCCAGAAGG | 54 | 371 |
| 345825 | Coding | 154 | 1920 | TGTCCAGCCAGACCCAGAAG | 43 | 372 |
| 345826 | Coding | 154 | 1922 | ATTGTCCAGCCAGACCCAGA | 29 | 373 |
| 345827 | Coding | 154 | 1924 | ATATTGTCCAGCCAGACCCA | 29 | 374 |
| 345828 | Coding | 1 | 2181 | CATGATCTTATAGCCCATGA | 40 | 375 |
| 345829 | Coding | 1 | 2183 | TCCATGATCTTATAGCCCAT | 26 | 376 |
| 345830 | Coding | 1 | 2185 | CATCCATGATCTTATAGCCC | 37 | 377 |

(continued)

| Chimeric phosphorothioate oligonucleotides targeted to human STAT3 having 2'-MOE wings and a deoxy gap | | | | | | |
|---|---|---|---|---|---|---|
| Isis # | Region | Target Seq ID No | Target Site | Sequence | % Inhib | Seq ID No |
| 345831 | Coding | 1 | 2187 | AGCATCCATGATCTTATAGC | 34 | 378 |
| 345832 | Coding | 1 | 2191 | TGGTAGCATCCATGATCTTA | 30 | 379 |
| 345833 | Coding | 1 | 2192 | TTGGTAGCATCCATGATCTT | 18 | 380 |
| 345834 | Coding | 1 | 2196 | GATATTGGTAGCATCCATGA | 21 | 381 |
| 106707 | Coding | 1 | 386 | CCCAGGAGATTATGAAACAC | 19 | 18 |
| 106712 | Coding | 1 | 639 | CTGCTCCAGCATCTGCTGCT | 45 | 23 |
| 106734 | Coding | 1 | 1669 | CATTCTTGGGATTGTTGGTC | 39 | 45 |
| 106748 | Coding | 1 | 2194 | TATTGGTAGCATCCATGATC | 18 | 59 |
| 106770 | 3' UTR | 1 | 2760 | GATCATGGGTCTCAGAGAAC | 34 | 81 |
| 113171 | Coding | 1 | 385 | CCAGGAGATTATGAAACACC | 47 | 110 |
| 113176 | Coding | 1 | 637 | GCTCCAGCATCTGCTGCTTC | 52 | 115 |
| 113192 | Coding | 1 | 1665 | CTTGGGATTGTTGGTCAGCA | 48 | 131 |
| 113196 | Coding | 1 | 1881 | GGAGAAGCCCTTGCCAGCCA | 68 | 135 |
| 113202 | Coding | 1 | 2252 | CCGAATGCCTCCTCCTTGGG | 66 | 141 |
| 337265 | Coding | 154 | 446 | CGACTCTTGCAGGAAGCGGC | 62 | 92 |
| 337325 | Coding | 154 | 1687 | TTCTTGGGATTGTTGGTCAG | 37 | 239 |
| 337332 | Coding | 154 | 1898 | GAAGCCCTTGCCAGCCATGT | 60 | 246 |
| 337333 | Coding | 154 | 1903 | AAGGAGAAGCCCTTGCCAGC | 58 | 247 |
| 337335 | Coding | 154 | 1918 | TCCAGCCAGACCCAGAAGGA | 59 | 249 |
| 337344 | Coding | 154 | 2204 | ATCCATGATCTTATAGCCCA | 46 | 258 |
| 337345 | Coding | 154 | 2210 | GGTAGCATCCATGATCTTAT | 36 | 259 |
| 337348 | Coding | 154 | 2273 | TCCGAATGCCTCCTCCTTGG | 68 | 262 |
| 337372 | 3' UTR | 154 | 2779 | ATCATGGGTCTCAGAGAACA | 47 | 286 |
| 337384 | 3' UTR | 154 | 3108 | ATACAGAAAGGCTATGCTGA | 44 | 298 |

**Example 14**

**Chimeric phosphorothioate oligonucleotides targeted to mouse STAT3, having 2'-MOE wings and a deoxy gap**

In accordance with the present invention, an additional series of oligonucleotides was designed to target different regions of the mouse STAT 3 RNA, using published sequences (GenEank® accession number U06922.1, incorporated herein as SEQ ID NO: 82, GenBank® accession number U30709.1, incorporated herein as SEQ ID NO: 382). The oligonucleotides are shown in Table 15. "Target site" indicates the first (5'-most) nucleotide number on the particular target sequence to which the oligonucleotide binds. All compounds in Table 15 are chimeric oligonucleotides ("gapmers") 20 nucleotides in length, composed of a central "gap" region consisting of ten 2'-deoxynucleotides, which is flanked on both sides (5' and 3' directions) by five-nucleotide "wings". The wings are composed of 2'-O-methoxyethyl (2'-MOE)nucleotides. The internucleoside (backbone) linkages are phosphorothioate (P=S) throughout the oligonucleotide. All cytidine residues are 5-methylcytidines.

**Table 15**

| Chimeric phosphorothioate oligonucleotides targeted to mouse STAT3 having 2'-MOE wings and a deoxy gap | | | | | |
|---|---|---|---|---|---|
| **Isis #** | **Region** | **Target Seq ID No** | **Target Site** | **Sequence** | **Seq ID No** |
| 29800 | coding | 82 | 2213 | TGGTATTGCTGCAGGTCGTT | 383 |
| 29801 | coding | 82 | 2224 | CGGCAGGTCAATGGTATTGC | 384 |
| 29802 | coding | 82 | 2230 | GGACATCGGCAGGTCAATGG | 385 |
| 29806 | 5'UTR | 382 | 11 | TTGTACCTCAGCGCGGACGC | 386 |
| 134027 | coding | 82 | 2309 | ACTCAAACTGCCCTCCTGCT | 95 |
| 337354 | coding | 82 | 2204 | TGCAGGTCGTTGGTGTCACA | 268 |
| 345821 | coding | 82 | 1742 | GCCAGACCCAGAAGGAGAAG | 90 |

In a further embodiment, an additional series of oligonucleotides was designed to target mouse STAT 3 RNA, using published sequences (GenBank® accession number U06922.1, incorporated herein as SEQ ID NO: 82). The compounds are shown in Table 16. "Target site" indicates the first (5'-most) nucleotide number on the particular sequence to which the compound binds. All compounds in Table 16 are chimeric oligonucleotides, composed of a "gap" region consisting of twelve 2'-deoxynucleotides, which is flanked on both sides (5' and 3' directions) by "wings" consisting of 2'-O-methoxyethyl (2'-MOE)nucleotides. The number of 2'-MOE nucleotides in the gaps vary from a length of 2 to 5 nucleotides, with the 2'- deoxynucleotides in plain type and the 2'-MOE nucleotides in bold type. The exact structure of each oligonucleotide is designated in Table 16 as the "wing" structure. A designation of 5~10~5, for example, indicates that the first and last 5 nucleotides are 2'-MOE nucleotides and the central 10 nucleotides are 2'deoxynucleotides. The internucleoside (backbone) linkages are phosphorothioate (P=S) throughout the oligonucleotide. Unmodified cytidine residues which are underscored; all other cytidine residues are 5-methylcytidines.

**Table 16**

| Chimeric phosphorothioate oligonucleotides targeted to mouse STAT3, having 2'-MOE wings and a deoxy gap | | | | | | |
|---|---|---|---|---|---|---|
| **ISIS #** | **Region** | **TARGET SEQ ID NO** | **TARGET SITE** | **SEQUENCE** | **WING STRUCTURE** | **SEQ ID NO** |
| 133003 | 3' UTR | 82 | 2527 | **AAAAA**GTGCCCAGATTGCCC | 5~12~5 | 99 |
| 346030 | 3' UTR | 82 | 2527 | **AAAA**GTGCCCAGATTQCCC | 4~10~5 | 387 |
| 346031 | 3' UTR | 82 | 2528 | **AAAA**GTGCCCAGATTGCC | 4~10~4 | 388 |
| 346032 | 3' UTR | 82 | 2528 | **AAA**GTGCCCAGATTGCC | 3~10~4 | 389 |

In a further embodiment of the present disclosure, an additional series of oligonucleotides was designed to target different regions of the mouse STAT 3 RNA, using published sequence (GenBank® accession number U06922.1, incorporated herein as SEQ ID NO: 82). The oligonucleotides are shown in Table 17. "Target site" indicates the first (5'-most) nucleotide number on the particular target sequence to which the oligonucleotide binds. All compounds in table 17 are uniformly composed of 2'-O-methoxyethyl (2'-MOE)nucleotides. The internucleoside (backbone) linkages are phosphorothioate (P=S) throughout the oligonucleotide, and all cytidine residues are 5-methylcytidines.

**Table 17**

| Phosphorothioated uniform 2'MOE oligonucleotides targeted to mouse STAT3 | | | | | |
|---|---|---|---|---|---|
| **Isis #** | **Region** | **Target Seq ID No** | **Target Site** | **Sequence** | **Seq ID No** |
| 29803 | coding | 82 | 2253 | ATCAATGAATCTAAAGTGCG | 93 |
| 29805 | coding | 82 | 2206 | GCTGCAGGTCGTTGGTGTCA | 390 |

**Example 15**

**Antisense inhibition of human STAT 3 by chimeric oligonucleotides having 2'-MOE wings and a deoxy gap: dose response**

In accordance with the present invention, a subset of the antisense oligonucleotides targeted to human STAT3 was further investigated in dose-response studies. The compounds were analyzed for their effect on human STAT 3 mRNA levels in T-24 cells.

The transitional cell bladder carcinoma cell line T-24 was obtained from the American Type Culture Collection (ATCC, Manassas, VA). T-24 cells were routinely cultured in complete McCoy's 5A basal medium supplemented with 10% fetal bovine, 100 units per mL penicillin, and 100 micrograms per mL streptomycin (medium and supplements from Invitrogen Life Technologies, Carlsbad, CA). Cells were routinely passaged by trypsinization and dilution when they reached 90% confluence. Cells were seeded into 96-well plates (Falcon-Primaria #3872, BD Biosciences, Bedford, MA) at a density of 7000 cells/well for use in antisense olignucleotide transfection experiments.

Control oligonucleotides used were ISIS 129695 (TTCTACCTCGCGCGATTTAC, SEQ ID NO: 391), ISIS 129694 (GTACAGTTATGCGCGGTAGA SEQ ID NO: 392), ISIS 129690 (TTAGAATACGTCGCGTTATG SEQ ID NO: 393), ISIS 129686 (CGTTATTAACCTCCGTTGAA SEQ ID NO: 394), ISIS 116847 (CTGCTAGCCTCTGGATTTGA, SEQ ID NO: 395) and ISIS 113529 (CTCTTACTGTGCTGTGGACA SEQ ID NO: 396). These oligonucleotides do not target STAT3 and are used as negative controls.

T-24 cells were treated with 18.75, 37.5, 75, or 150 nM of oligonucleotide mixed with 3 ug/mL LIPOFECTIN™ per 1.00 nM.oligonucleotide as described by other examples herein. Untreated cells served as controls. Following 16 hours of treatment, RNA was prepared from cells for subsequent real-time PCR analysis.

Human STAT3 mRNA expression levels were quantitated by real-time PCR using primer probe set PPS199 and gene target quantities were normalized using RiboGreen® as described in other examples herein. Data are averages from two experiments are shown in Table 18. A "-" or "+" designation indicates a decrease or increase of STAT 3 mRNA expression, respectively, relative to untreated control cells.

**Table 18**

| Inhibition of human STAT 3 mRNA levels by chimeric phosphorothioate oligonucleotides having 2'-MOE wings and a deoxy gap: dose response | | | | | |
|---|---|---|---|---|---|
| Percent change of STAT3 expression using PPS199 | | | | | |
| | | Oligonucleotide Concentration | | | |
| **Isis #** | **Seq ID No** | **18.75 nM** | **37.5 nM** | **75 nM** | **150 nM** |
| **106747** | **58** | -37 | -48 | -71 | -84 |
| **337247** | **161** | -23 | -43 | -62 | -75 |
| **337270** | **184** | -29 | -41 | -67 | -87 |
| **337276** | **190** | -40 | -61 | -76 | -81 |
| **337284** | **198** | -49 | -64 | -69 | -72 |
| **337293** | **207** | -26 | -49 | -66 | -79 |
| **337303** | **217** | -44 | -61 | -69 | -72 |
| **337332** | **246** | -63 | -79 | -87 | -92 |
| **337333** | **247** | -48 | -73 | -82 | -88 |
| **337344** | **258** | -27 | -47 | -63 | -77 |
| **337348** | **262** | -61 | -77 | -82 | -86 |
| **337384** | **298** | -40 | -55 | -71 | -80 |
| **129695** | **391** | +5 | +2 | +8 | 0 |
| **129694** | **392** | +4 | -3 | -4 | -10 |
| **129690** | **393** | +2 | +7 | +6 | +8 |
| **129686** | **394** | +2 | +1 | -5 | +1 |

(continued)

| Inhibition of human STAT 3 mRNA levels by chimeric phosphorothioate oligonucleotides having 2'-MOE wings and a deoxy gap: dose response | | | | | |
|---|---|---|---|---|---|
| Percent change of STAT3 expression using PPS199 | | | | | |
| | | Oligonucleotide Concentration | | | |
| **Isis #** | **Seq ID No** | **18.75 nM** | **37.5 nM** | **75 nM** | **150 nM** |
| **116847** | **395** | +7 | +4 | +8 | +5 |
| **113529** | **396** | +1 | -1 | -11 | -26 |

As shown in Table 18, the compounds tested inhibit human STAT3 mRNA expression in a dose-dependent manner.

The dose-response was repeated in T-24 cells and gene target quantities were measured using a different primer-probe set, called PPS2033 herein. PPS2033 comprises probes and primers to human STAT3 were designed to hybridize to a human STAT3 sequence, using published sequence information (incorporated herein as SEQ ID NO: 154). For PPS2033 the PCR primers were:
forward primer: GAGGCCCGCCCAACA (SEQ ID NO: 397) reverse primer: TTCTGCTAATGACGTTATCCAGTTTT (SEQ ID NO: 398) and the PCR probe was: FAM-CTGCCTAGATCGGC-MGB (SEQ ID NO: 399) where FAM is the fluorescent reporter dye and MGB is the quencher dye. Gene target quantities obtained by real-time PCR are normalized by quantifying total RNA using RiboGreen™ (Molecular Probes, Inc. Eugene, OR). Control oligonucleotides used were ISIS 129695 (SEQ ID NO: 391), ISIS 129694 (SEQ ID NO: 392), ISIS 129690 (SEQ ID NO: 393), ISIS 129686 (SEQ ID NO: 394), ISIS 116847 (SEQ ID NO: 395) and ISIS 113529 (SEQ ID NO: 396).

T-24 cells were treated with 18.75, 37.5, 75, or 150 nM of oligonucleotide mixed with 3 ug/mL LIPOFECTIN™ per 100 nM oligonucleotide as described by other examples herein. Untreated cells served as controls. Following 16 hours of treatment, RNA was prepared from cells for subsequent real-time PCR analysis.

Human STAT3 mRNA expression levels were quantitated by real-time PCR using primer probe set PPS2033 and gene target quantities were normalized using RiboGreen® as described in other examples herein. Data are averages from two experiments are shown in Table 19. A "-" or "+" designation indicates a decrease or increase of STAT 3 mRNA expression, respectively, relative to untreated control cells.

**Table 19**

| Inhibition of human STAT 3 mRNA levels by chimeric phosphorothioate oligonucleotides having 2'-MOE wings and a deoxy gap: dose response | | | | | |
|---|---|---|---|---|---|
| Percent change of STAT3 expression using PPS2033 | | | | | |
| | | Oligonucleotide Concentration | | | |
| **Isis #** | **Seq ID No** | **18.75 nM** | **37.5 nM** | **75 nM** | **150 nM** |
| **106747** | **58** | -32 | -48 | -62 | -76 |
| **337247** | **161** | +17 | -21 | -53 | -69 |
| **337270** | **184** | -16 | -27 | -67 | -87 |
| **337276** | **190** | -34 | -58 | -75 | -81 |
| **337284** | **198** | -49 | -62 | -66 | -68 |
| **337293** | **207** | -26 | -49 | -67 | -79 |
| **337303** | **217** | -47 | -59 | -69 | -71 |
| **337332** | **246** | -66 | -79 | -85 | -91 |
| **337333** | **247** | -46 | -70 | -82 | -90 |
| **337344** | **258** | -17 | -37 | -60 | -76 |
| **337348** | **262** | -53 | -76 | -83 | -86 |
| **337384** | **298** | -41 | -59 | -69 | -80 |

(continued)

| Inhibition of human STAT 3 mRNA levels by chimeric phosphorothioate oligonucleotides having 2'-MOE wings and a deoxy gap: dose response | | | | | |
|---|---|---|---|---|---|
| Percent change of STAT3 expression using PPS2033 | | | | | |
| | | Oligonucleotide Concentration | | | |
| **Isis #** | **Seq ID No** | **18.75 nM** | **37.5 nM** | **75 nM** | **150 nM** |
| **129695** | **391** | -4 | +2 | +8 | +3 |
| **129694** | **392** | +19 | -1 | +7 | +2 |
| **129690** | **393** | +4 | +10 | +8 | +11 |
| **129686** | **394** | +20 | +16 | +25 | +9 |
| **116847** | **395** | +45 | +33 | +22 | -2 |
| **113529** | **396** | +1 | +12 | -11 | -24 |

As shown in Table 19, measurement of target gene quantities using PPS2033 demonstrates that the compounds tested inhibit human STAT3 mRNA expression in a dose-dependent manner.

An additional dose-response experiment was preformed in A549 cells. A549 cells were treated with 18.75, 37.5, 75, or 150 nM of oligonucleotide mixed with 3 ug/mL LIPOFECTIN™ per 100 nM oligonucleotide as described by other examples herein. Control oligonucleotides used were ISIS 129686 (SEQ ID NO: 394) and ISIS 129690 (SEQ ID NO: 393). Untreated cells served as controls. Following 16 hours of treatment, RNA was prepared from cells for subsequent real-time PCR analysis.

Human STAT3 mRNA expression levels were quantitated by real-time PCR using primer probe set PPS199 and gene target quantities were normalized using Ribogreen™ as described in other examples herein. Data are averages from two experiments are shown in Table 20. A "-" or "+" designation in the dose response results indicates a decrease or increase of STAT 3 mRNA expression, respectively, relative to untreated control cells.

**Table 20**

| Inhibition of human STAT 3 mRNA levels by chimeric phosphorothioate oligonucleotides having 2'-MOE wings and a deoxy gap: dose response | | | | | |
|---|---|---|---|---|---|
| Percent change of STAT3 expression in A549 cells using PPS199 | | | | | |
| | | Oligonucleotide Concentration | | | |
| **Isis #** | **Seq ID No** | **18.75 nM** | **37.5 nM** | **75 nM** | **150 nM** |
| **106734** | **45** | -2 | -16 | -56 | -73 |
| **337332** | **246** | -31 | -61 | -77 | -87 |
| **337333** | **247** | -8 | -39 | -59 | -75 |
| **337348** | **262** | -26 | -43 | -55 | -77 |
| **129686** | **394** | +27 | +23 | +22 | +19 |
| **129690** | **393** | +30 | +27 | +16 | +27 |

As shown in Table 20, the compounds tested inhibit human STAT3 mRNA expression in A549 cells in a dose-dependent manner.

**Example 16**

**RNA Synthesis**

In general, RNA synthesis chemistry is based on the selective incorporation of various protecting groups at strategic intermediary reactions. Although one of ordinary skill in the art will understand the use of protecting groups in

organic synthesis, a useful class of protecting groups includes silyl ethers. In particular bulky silyl ethers are used to protect the 5'-hydroxyl in combination with an acid-labile orthoester protecting group on the 2'-hydroxyl. This set of protecting groups is then used with standard solid-phase synthesis technology. It is important to lastly remove the acid labile orthoester protecting group after all other synthetic steps. Moreover, the early use of the silyl protecting groups during synthesis ensures facile removal when desired, without undesired deprotection of the 2'-hydroxyl.

Following this procedure for the sequential protection of the 5'-hydroxyl in combination with protection of the 2'-hydroxyl by protecting groups that are differentially removed and are differentially chemically labile, RNA oligonucleotides were synthesized.

RNA oligonucleotides are synthesized in a stepwise fashion. Each nucleotide is added sequentially (3"- to 5'-direction) to a solid support-bound oligonucleotide. The first nucleoside at the 3'-end of the chain is covalently attached to a solid support. The nucleotide precursor, a ribonucleoside phosphoramidite, and activator are added, coupling the second base onto the 5'-end of the first nucleoside. The support is washed and any unreacted 5'-hydroxyl groups are capped with acetic anhydride to yield 5'-acetyl moieties. The linkage is then oxidized to the more stable and ultimately desired P(V) linkage. At the end of the nucleotide addition cycle, the 5'-silyl group is cleaved with fluoride. The cycle is repeated for each subsequent nucleotide.

Following synthesis, the methyl protecting groups on the phosphates are cleaved in 30 minutes utilizing 1 M disodium-2-carbamoyl-2-cyanoethylene-1,1-dithiolate trihydrate ($S_2Na_2$) in DMF. The deprotection solution is washed from the solid support-bound oligonucleotide using water. The support is then treated with 40% methylamine in water for 10 minutes at 55 °C. This releases the RNA oligonucleotides into solution, deprotects the exocyclic amines, and modifies the 2'- groups. The oligonucleotides can be analyzed by anion exchange HPLC at this stage.

The 2'-orthoester groups are the last protecting groups to be removed. The ethylene glycol monoacetate orthoester protecting group developed by Dharmacon Research, Inc. (Lafayette, CO), is one example of a useful orthoester protecting group which, has the following important properties. It is stable to the conditions of nucleoside phosphoramidite synthesis and oligonucleotide synthesis. However, after oligonucleotide synthesis the oligonucleotide is treated with methylamine which not only cleaves the oligonucleotide from the solid support but also removes the acetyl groups from the orthoesters. The resulting 2-ethyl-hydroxyl substituents on the orthoester are less electron withdrawing than the acetylated precursor. As a result, the modified orthoester becomes more labile to acid-catalyzed hydrolysis. Specifically, the rate of cleavage is approximately 10 times faster after the acetyl groups are removed. Therefore, this orthoester possesses sufficient stability in order to be compatible with oligonucleotide synthesis and yet, when subsequently modified, permits deprotection to be carried out under relatively mild aqueous conditions compatible with the final RNA oligonucleotide product.

Additionally, methods of RNA synthesis are well known in the art (Scaringe, S. A. Ph.D. Thesis, University of Colorado, 1996; Scaringe, S. A., et al., J. Am. Chem. Soc., 1998, 120, 11820-11821; Matteucci, M. D. and Caruthers, M. H. J. Am. Chem. Soc., 1981, 103, 3185-3191; Beaucage, S. L. and Caruthers, M. H. Tetrahedron Lett., 1981, 22, 1859-1862; Dahl, B. J., et al., Acta Chem. Scand,. 1990, 44, 639-641; Reddy, M. P., et al., Tetrahedrom Lett., 1994, 25, 4311-4314; Wincott, F. et al., Nucleic Acids Res., 1995, 23, 2677-2684; Griffin, B. E., et al., Tetrahedron, 1967, 23, 2301-2313; Griffin, B. E., et al., Tetrahedron, 1967, 23, 2315-2331).

RNA antisense compounds (RNA oligonucleotides) of the present invention can be synthesized by the methods herein or purchased from Dharmacon Research, Inc (Lafayette, CO). Once synthesized, complementary RNA antisense compounds can then be annealed by methods known in the art to form double stranded (duplexed) antisense compounds. For example, duplexes can be formed by combining 30 μl of each of the complementary strands of RNA oligonucleotides (50 uM RNA oligonucleotide solution) and 15 μl of 5X annealing buffer (100 mM potassium acetate, 30 mM HEPES-KOH pH 7.4, 2 mM magnesium acetate) followed by heating for 1 minute at 90°C, then 1 hour at 37°C. The resulting duplexed antisense compounds can be used in kits, assays, screens, or other methods to investigate the role of a target nucleic acid, or for diagnostic or therapeutic purposes.

**Example 17**

**Design and screening of duplexed antisense compounds targeting STAT3**

In accordance with the present disclosure, a series of nucleic acid duplexes comprising the antisense compounds of the present invention and their complements can be designed to target STAT3. The nucleobase sequence of the antisense strand of the duplex comprises at least a portion of an oligonucleotide targeted to STAT3 as disclosed herein. The ends of the strands may be modified by the addition of one or more natural or modified nucleobases to form an overhang. The sense strand of the dsRNA is then designed and synthesized as the complement of the antisense strand and may also contain modifications or additions to either terminus. For example, in one embodiment, both strands of the dsRNA duplex would be complementary over the central nucleobases, each having overhangs at one or both termini. The antisense and sense strands of the duplex comprise from about 17 to 25 nucleotides, or from about 19 to

23 nucleotides. Alternatively, the antisense and sense strands comprise 20, 21 or 22 nucleotides.

For example, a duplex comprising an antisense strand having the sequence CGAGAGGCGGACGGGACCG and having a two-nucleobase overhang of deoxythymidine(dT) would have the following structure:

```
  cgagaggcggacgggaccgTT    Antisense Strand
  |||||||||||||||||||
TTgctctccgcctgccctggc      Complement
```

Overhangs can range from 2 to 6 nucleobases and these nucleobases may or may not be complementary to the target nucleic acid. In another embodiment, the duplexes may have an overhang on only one terminus.

In another embodiment, a duplex comprising an antisense strand having the same sequence CGAGAGGCGGACGGGACCG may be prepared with blunt ends (no single stranded overhang) as shown:

```
cgagaggcggacgggaccg      Antisense Strand
|||||||||||||||||||
gctctccgcctgccctggc      Complement
```

The RNA duplex can be unimolecular or bimolecular; i.e, the two strands can be part of a single molecule or may be separate molecules.

RNA strands of the duplex can be synthesized by methods disclosed herein or purchased from Dharmacon Research Inc. (Lafayette, CO). Once synthesized, the complementary strands are annealed. The single strands are aliquoted and diluted to a concentration of 50 uM. Once diluted, 30 uL of each strand is combined with 15uL of a 5X solution of annealing buffer. The final concentration of said buffer is 100 mM potassium acetate, 30 mM HEPES-KOH pH 7.4, and 2mM magnesium acetate. The final volume is 75 uL. This solution is incubated for 1 minute at 90°C and then centrifuged for 15 seconds. The tube is allowed to sit for 1 hour at 37°C at which time the dsRNA duplexes are used in experimentation. The final concentration of the dsRNA duplex is 20 uM. This solution can be stored frozen (-20°C) and freeze-thawed up to 5 times.

Once prepared, the duplexed antisense compounds are evaluated for their ability to modulate STAT3.

When cells reached 80% confluency, they are treated with duplexed antisense compounds of the disclosure. For cells grown in 96-well plates, wells are washed once with 200 L OPTI-MEM-1™ reduced-serum medium (Gibco BRL) and then treated with 130 L of OPTI-MEM-1™ containing 12 g/mL LIPOFECTIN™ (Gibco BRL) and the desired duplex antisense compound at a final concentration of 200 nM (a ratio of 6 g/mL LIPOFECTIN™ per 100 nM duplex antisense compound). After 5 hours of treatment, the medium is replaced with fresh medium. Cells are harvested 16 hours after treatment, at which time RNA is isolated and target reduction measured by RT-PCR.

A series of nucleic acid duplexes comprising the antisense compounds of the present disclosure and their complements was designed to target STAT3 mRNA, using published sequence (GenDank® Accession number L29277, incorporated herein as SEQ ID NO: 1). The nucleobase sequence of the antisense strand of the duplex is 20 nucleotides in length. The sequences of the antisense strand are listed in Table 21. The sense strand of the dsRNA is designed and synthesized as the complement of the antisense strand.

All compounds in Table 21 are oligodeoxynucleotides, 21 nucleotides in length with the two nucleotides on the 3' end being the TT overhang and with phosphodiester internucleoside linkages (backbones) throughout. These sequences are shown to contain thymine (T) but one of skill in the art will appreciate that thymine (T) is generally replaced by uracil (U) in RNA sequences.

**Table 21**

| dsRNAs targeted to human STAT3 | | | | | |
|---|---|---|---|---|---|
| **ISIS #** | **REGION** | **TARGET SITE** | **TARGET SEQ ID** | **SEQUENCE** | **SEQ ID NO** |
| 330249 | coding | 1669 | 1 | ATTCTTGGGATTGTTGGTCTT | 400 |
| 330247 | coding | 637 | 1 | CTCCAGCATCTGCTGCTTCTT | 401 |

The compounds in Table 21 were tested for their effects on human STAT3 expression in A549 cells. The dsRNA comprising ISIS 330249 and its complement targets the same site as the antisense oligonucleotide ISIS 106734 (SEQ ID NO: 45) and the dsRNA comprising ISIS 330247 and its complement targets the same site as the antisense oligonucleotide ISIS 113176 (SEQ ID NO: 115); thus, ISIS 106734 and ISIS 113176 were also tested. A549 cells were treated with oligonucleotide mixed with LIPOFECTIN™ (Invitrogen Life Technologies, Carlsbad, CA) as described herein. Oligonucleotide concentrations used are indicated in Table 22. The control oligonucleotide used was ISIS 129698 (TTTGATCGAGGTTAGCCGTG, SEQ ID NO: 402). Cells were treated with oligonucleotide for 4 hours and harvested an additional 16 hours later. Untreated cells served as a control.

Human STAT3 mRNA expression levels were quantitated by real-time PCR using primer probe set PPS199 and gene target quantities were normalized using Ribogreen™ as described in other examples herein. Data are averages from two experiments are shown in Table 22. A "-" or "+" designation indicates a decrease or increase of STAT 3 mRNA expression, respectively, relative to untreated control cells. Where present, "N.D." indicates not determined.

**Table 22**

| Inhibition of STAT 3 mRNA levels by dsRNAs | | | | | | | |
|---|---|---|---|---|---|---|---|
| Percent change in STAT3 mRNA expression in A549 cells by duplex antisense compounds | | | | | | | |
| | Oligonucleotide Concentration | | | | | | |
| **Isis #** | **SEQ ID NO** | **12.5 nM** | **25 nM** | **50 nM** | **100 nM** | **200 nM** | **400 nM** |
| **330249** | 400 | -64 | -70 | -80 | -83 | -87 | -81 |
| **106734** | 45 | -5 | -5 | -40 | -56 | -67 | -77 |
| **330247** | 401 | +11 | -19 | -15 | -16 | -20 | -48 |
| **113176** | 115 | +8 | +17 | +6 | 0 | -22 | -34 |
| **129698** | 402 | N.D. | N.D. | +41 | +42 | +1 | +22 |

### Example 18

#### Inhibition of tumor growth in LNCaP mouse model of prostate carcinoma

The LNCaP murine model of human prostate carcinoma is described in Kiyama et al., Cancer Res. 63:3575-3584, 2003. Briefly, LNCaP human prostatic carcinoma cells were cultured and maintained in RPMI medium (Invitrogen Life Technologies, Carlsbad, CA) supplemented with 5% heat-inactivated fetal bovine serum. About 1 X $10^6$ LNCaP cells were inoculated subcutaneously with 0.1 ml of Matrigel (Becton Dickinson Labware, Franklin Lakes, NJ) in the flank region of 6-8 week old male athymic nude mice (Harlan Sprague Dawley, Inc., Indianapolis, IN) via a 27-gauge needle under methoxyfluorane anesthesia. Mice bearing tumors between 300 and 500 $mm^3$ in volume were castrated via a scrotal approach and randomly assigned to treatment with 10 mg/kg of either ISIS 113176 (SEQ ID NO: 115) human antisense or ISIS 129987 (SEQ ID NO: 404) human mismatch control STAT 3 oligonucleotide intraperitoneally five times per week for the first week followed by three times per week thereafter. Treatment commenced beginning one day after castration. Tumor volumes and serum prostate specific antigen (PSA) measurements were performed once weekly. Tumor volumes were calculated by the formula L X W X H X 0.5236 (Gleave et al., Cancer Res. 51:1598-1605, 1992). Blood samples were obtained from tail vein incisions of mice, and serum PSA levels were determined by an enzymatic immunoassay kit with a lower limit of sensitivity of 0.2 μg/liter (Abbott IMX, Montreal, Quebec, Canada) according to the manufacturer's protocol.

ISIS 113176 suppressed the induction of serum PSA levels and tumor growth in the LNCaP xenograft model in castrated nude mice. Similar treatment of mice with the mismatch control oligonucleotide ISIS 129987 had no effect. The observed STAT3 antisense oligonucleotide-mediated effects on PSA and tumor volume were significantly different from mismatch oligonucleotide ISIS 129987 or saline treated controls (student's t-test, $p \leq 0.50$). Treatment effects were demonstrated out to the end of the observation period (10 weeks post-castration).

To address the potential target-specific toxicity of this approach, normal mice were treated subcutaneously with an optimized murine STAT3 antisense oligonucleotide (up to 50 mg/kg three times per week tor 2 weeks) and pharmacodynamic and toxological effects were evaluated in the blood, liver and bone marrow. STAT3 antisense oligonucleotide treatment resulted in 85% liver mRNA reduction and significant inhibition of STAT3 protein in the bone marrow pre-monocytic subpopulation. No overt changes were observed in complete blood counts, liver histology or bone marrow subpopulations in animals treated with STAT3 antisense oligonucleotide. Liver and bone marrow expression of STAT3 was significantly reduced by treatment with STAT3 antisense oligonucleotide. Thus, antisense oligonucleotides to STAT 3 represent a therapeutic opportunity for treatment of prostate cancer.

**Example 19**

**Antisense inhibition of human STAT3 in primary human hepatocytes: dose response**

In a further embodiment, antisense oligonucleotides targeted to human STAT3 were selected for dose response studies in primary human hepatocytes. Primary human hepatocytes were purchased from InVitro Technologies (Baltimore, MD). Cells were plated in 24-well plates (Falcon-Primaria, BD Biosciences, Bedford, MA) at a density of 50,000 cells/well in high-glucose DMEM medium supplemented with 10% fetal bovine serum, 100 units/mL penicillin, and 100 μg/mL streptomycin (medium and supplements from Invitrogen Life Technologies, Carlsbad, CA). Cells are allowed to adhere overnight, and are treated with antisense oligonucleotides the following day.

The antisense oligonucleotides tested in this assay were ISIS 113196, ISIS 337332, ISIS 337333, ISIS 345778, ISIS 345781, ISIS 345794, ISIS 345815 and ISIS 345823. Cells were treated with 5, 10, 25, 50 and 150 nM of antisense oligonucleotides mixed with LIPOFECTIN™, at a concentration of 3 μg /mL LIPOFECTIN™ per 100 nM of oligonucleotide. Untreated cells served as the control to which data were normalized. After 4 hours, the culture medium was replaced and cells were cultured for an additional 20 hours. RNA was isolated and STAT3 mRNA levels were measured by real-time PCR, using PPS199, as described herein. The data were obtained in triplicate and averaged. The results are shown in Table 23 as percent mRNA expression relative to the untreated control.

For each antisense oligonucleotide tested, the IC50, the concentration of antisense oligonucleotide at which STAT3 mRNA expression is inhibited by 50%, is shown in Table 24.

**Table 23**

| Inhibition of Human STAT3 mRNA Expression in Primary Human Hepatocytes | | | | | | | |
|---|---|---|---|---|---|---|---|
| Treatment | SEQ ID NO | % Control | | | | | IC50 (nM) |
| | | Dose (nM) | | | | | |
| | | 5 | 10 | 25 | 50 | 150 | |
| 113196 | 135 | 88 | 80 | 73 | 33 | 28 | 36 |
| 337332 | 246 | 84 | 74 | 74 | 55 | 31 | 53 |
| 337333 | 247 | 72 | 81 | 90 | 49 | 31 | 51 |
| 345778 | 326 | 81 | 79 | 58 | 80 | 31 | 84 |
| 345781 | 329 | 72 | 89 | 61 | 77 | 35 | 82 |
| 345794 | 342 | 51 | 29 | 13 | 7 | 2 | 4 |
| 345815 | 363 | 67 | 80 | 86 | 50 | 29 | 49 |
| 345823 | 370 | 120 | 129 | 65 | 40 | 16 | 37 |

**Table 24**

| IC50 for STAT3 antisense inhibition | | |
|---|---|---|
| Treatment | SEQ ID NO | $IC_{50}$ (nM) |
| 113196 | 135 | 36 |
| 337332 | 246 | 53 |
| 337333 | 247 | 51 |
| 345778 | 326 | 84 |
| 345781 | 329 | 82 |
| 345794 | 342 | 4 |
| 345815 | 363 | 49 |
| 345823 | 370 | 37 |

As shown in Table 23, ISIS 113196, ISIS 337332, ISIS 345794, and ISIS 345823 reduced human STAT3 mRNA levels in primary human hepatocytes in a dose-dependent manner. As shown in Table 24, ISIS 345794 exhibits the lowest IC50.

Shown in Table 25 are expression levels of STAT1 in the cells described above. The STAT1 expression levels were measured by real-time PCR using primers and probe designed by methods routine in the art and described herein (e.g., Primer Express®, Applied Biosystems, Foster City, CA).

**Table 25**

| **Human STAT1 mRNA Expression in Primary Human Hepatocytes Treated with Antisense Oligonucleotides Targeting STAT3** | | | | | | |
|---|---|---|---|---|---|---|
| **Treatment** | **SEQ ID NO** | **% Control** | | | | |
| | | **Dose of STAT3 Antisense Oligonucleotide (nM)** | | | | |
| | | **5** | **10** | **25** | **50** | **150** |
| **113196** | 135 | 113 | 103 | 12 5 | 10 8 | 251 |
| **337332** | 246 | 102 | 102 | 14 2 | 13 6 | 349 |
| **337333** | 247 | 80 | 121 | 87 | 10 5 | 264 |
| **345778** | 326 | 92 | 190 | 18 7 | 16 8 | 582 |
| **345781** | 329 | 149 | 122 | 13 4 | 16 9 | 406 |
| **345794** | 342 | 232 | 138 | 19 0 | 21 2 | 438 |
| **345815** | 363 | 158 | 117 | 18 7 | 18 7 | 272 |
| **345823** | 370 | 119 | 110 | 85 | 18 7 | 305 |

As shown in Table 25, antisense oligonucleotides targeted to STAT3 did not cause substantial or dose-dependent inhibition of STAT1 expression. A basic local alignment search tools (BLAST) analysis, a gene analysis technique known in the art, of ISIS 345794 revealed no homologies to nucleic acid molecules other than STAT3.

**Example 20**

**Antisense inhibition of human STAT3 in HepG2 cells: dose response**

In a further embodiment, antisense oligonucleotides targeted to human STAT3 were selected for dose response studies in HepG2 cells.

The human hepatoblastoma cell line HepG2 was obtained from the American Type Culture Collection (Manassas, VA). HepG2 cells were routinely cultured in Eagle's MEM adjusted to contain 1.5 g/L sodium bicarbonate and supplemented with 10% fetal bovine serum, 0.1 mM nonessential amino acids, and 1 mM sodium pyruvate (Invitrogen Life Technologies, Carlsbad, CA). Cells were routinely passaged by trypsinization and dilution when they reached approximately 90% confluence. Multiwell culture plates are prepared for cell culture by coating with a 1:100 dilution of type 1 rat tail collagen (BD Biosciences, Bedford, MA) in phosphate-buffered saline. The collagen-containing plates were incubated at 37°C for approximately 1 hour, after which the collagen was removed and the wells were washed twice with phosphate-buffered saline. Cells were seeded into 96-well plates (Falcon-Primaria #3872, BD Biosciences, Bedford, MA) at a density of approximately 8,000 cells/well for use in oligomeric compound transfection experiments.

The antisense oligonucleotides tested in this assay were ISIS 113196, ISIS 337332, ISIS 337333, ISIS 345778, ISIS 345781, ISIS 345794, ISIS 345815 and ISIS 345823. Cells were treated with 5, 10, 25, 50 and 150 nM of antisense oligonucleotides mixed with LIPOFECTIN™, at a concentration of 3 μg/mL LIPOFECTIN™ per 100 nM of oligonucleotide. Untreated cells served as the control to which data were normalized. After 4 hours, the culture medium was replaced and cells were cultured for an additional 20 hours. RNA was isolated and STAT3 mRNA levels were measured by real-time PCR using the primer probe set PPS199, described herein. The data were obtained in triplicate and averaged. The results are shown in Table 26 as percent mRNA expression relative to the untreated control. The IC50, the concentration of antisense compound at which STAT3 mRNA expression is inhibited by 50%, is also shown.

Table 26

| Inhibition of Human STAT3 mRNA Expression in HepG2 cells | | | | | | | | |
|---|---|---|---|---|---|---|---|---|
| Treatment | SEQ ID NO | % Control | | | | | | IC50 (nM) |
| | | Dose (nM) | | | | | | |
| | | 1 | 10 | 25 | 50 | 100 | 200 | |
| **113196** | 135 | 75 | 40 | 26 | 12 | 11 | 12 | 7 |
| **337332** | 246 | 73 | 47 | 30 | 13 | 7 | 17 | 9 |
| **337333** | 247 | 91 | 47 | 29 | 15 | 15 | 16 | 10 |
| **345778** | 326 | 94 | 55 | 26 | 13 | 13 | 24 | 12 |
| **345781** | 329 | 66 | 52 | 19 | 7 | 6 | 14 | 11 |
| **345794** | 342 | 63 | 14 | 5 | 2 | 1 | 0 | 6 |
| **345815** | 363 | 52 | 23 | 19 | 11 | 12 | 12 | 5 |
| **345823** | 370 | 66 | 65 | 34 | 21 | 13 | 12 | 15 |

As shown in Table 26, ISIS 113916, ISIS 337333, ISIS 345794, ISIS 345815, and ISIS 345823 caused dose-dependent reduction in STAT3 mRNA expression in HepG2 cells.

Shown in Table 27 are expression levels of STAT1 in the cells treated with antisense oligonucleotides targeted to STAT3 described above. STAT1 mRNA levels were measured by real-time PCR using primers and probe designed by methods routine in the art and described herein.

Table 27

| Human STAT1 mRNA Expression in HepG2 Cells Treated with Antisense Oligonucleotides Targeting STAT3 | | | | | | | |
|---|---|---|---|---|---|---|---|
| Treatment | SEQ ID NO | % Control | | | | | |
| | | Dose (nM) | | | | | |
| | | 1 | 10 | 25 | 50 | 100 | 200 |
| **113196** | 135 | 87 | 94 | 96 | 10 1 | 89 | 79 |
| **337332** | 246 | 82 | 84 | 96 | 10 7 | 82 | 30 |
| **337333** | 247 | 91 | 87 | 10 9 | 83 | 87 | 56 |
| **345778** | 326 | 112 | 84 | 96 | 99 | 61 | 50 |
| **345781** | 329 | 93 | 114 | 13 5 | 14 3 | 93 | 40 |
| **345794** | 342 | 115 | 112 | 10 5 | 77 | 93 | 57 |
| **345815** | 363 | 84 | 113 | 11 2 | 93 | 58 | 61 |
| **345823** | 370 | 115 | 115 | 16 1 | 13 1 | 115 | 94 |

As shown in Table 27, STAT3 oligonucleotides did not cause substantial or dose-dependent inhibition of STAT1 expression.

STAT3 protein levels were also measured following transfection of HepG2 cells with antisense oligonucleotides. Cells were treated with 10nM, 50nM or 100nM doses of antisense oligonucleotides targeted to STAT3, and STAT3 protein levels were measured by western blot using a commercially available STAT3 antibody (Santa Cruz Biotechnology, Inc., Santa Cruz, CA), which was detected using ECLplus™ reagents (Amersham Biosciences, Piscataway, NJ) and quantitated using the Chemidoc™ EQ System (Bio-Rad Laboratories, Hercules, CA). The protein expressed by the housekeeping gene G3PDH was used to normalize for differences in protein levels among samples. The data are shown in Table 28, expressed as percent protein expression relative to untreated control cells.

Table 28

| Inhibition of STAT3 Protein Expression in HepG2 cells | | | | |
|---|---|---|---|---|
| Treatment | SEQ ID NO | % Control | | |
| | | Dose (nM) | | |
| | | 10 | 50 | 100 |
| **113196** | 135 | 100 | 28 | 13 |
| **337332** | 246 | 94 | 29 | 12 |
| **337333** | 247 | 104 | 23 | 12 |
| **345778** | 326 | 66 | 17 | 8 |
| **345781** | 329 | 83 | 17 | 7 |
| **345794** | 342 | 96 | 28 | 19 |
| **345815** | 363 | 82 | 32 | 19 |
| **345823** | 370 | 99 | 46 | 31 |

As shown in Table 28, treatment with ISIS 113196, ISIS 337332, ISIS 337333, ISIS 345778, ISIS 345781, ISIS 345794, ISIS 345815, and ISIS 345823 caused dose-dependent reduction of STAT3 protein levels in HepG2 cells.

**Example 21**

**Antisense inhibition of human STAT3 in A549 cells: dose response**

Antisense oligonucleotides targeted to human STAT3 were tested in a dose response study in A549 cells. Cells were treated with antisense oligonucleotides mixed with LIPOFECTIN™, at a concentration of 3 μg/mL LIPOFECTIN™ per 100 nM of oligonucleotide. Oligonucleotide concentrations are indicated in Tables 29 and 30. Untreated cells served as the control to which data were normalize. STAT3 mRNA expression levels were measured in duplicate, using two different primer probe sets, both described herein: PPS199 and PPS2033. The data using PPS2033 and PPS199 are shown in Tables 29 and 30, respectively, as percent expression relative to the untreated controls.

Table 29

| Antisense inhibition of human STAT3 in A549 cells measured using PPS2033 | | | | | |
|---|---|---|---|---|---|
| | | % Control | | | |
| | | Dose of oligonucleotide (nM) | | | |
| Treatment | SEQ ID NO | 9.375 | 18.75 | 37.5 | 75 |
| 106734 | 45 | 97 | 92 | 46 | 21 |
| 113176 | 115 | 91 | 78 | 58 | 27 |
| 113196 | 135 | 80 | 71 | 15 | 5 |
| 337332 | 246 | 104 | 70 | 21 | 10 |
| 337333 | 247 | 93 | 67 | 28 | 10 |
| 337348 | 262 | 84 | 73 | 30 | 17 |
| 345778 | 326 | 92 | 63 | 17 | 8 |
| 345781 | 329 | 97 | 50 | 19 | 8 |
| 345785 | 333 | 87 | 68 | 23 | 10 |
| 345815 | 363 | 85 | 70 | 35 | 20 |
| 345823 | 370 | 91 | 96 | 50 | 33 |
| 129690 | 393 | 83 | 88 | 96 | 103 |

(continued)

| Antisense inhibition of human STAT3 in A549 cells measured using PPS2033 | | | | | |
|---|---|---|---|---|---|
| | | % Control | | | |
| | | Dose of oligonucleotide (nM) | | | |
| Treatment | SEQ ID NO | 9.375 | 18.75 | 37.5 | 75 |
| 129691 | 410 | 122 | 105 | 109 | 119 |
| 129695 | 391 | 79 | 92 | 90 | 102 |
| 141923 | 409 | 98 | 106 | 95 | 98 |

As shown in Table 29, all of the antisense oligonucleotides targeted to human STAT3 inhibited expression in A459 cells, as measured using PPS2033.

**Table 30**

| Antisense inhibition of human STAT3 in A549 cells measured using PPS199 | | | | | |
|---|---|---|---|---|---|
| | | % Control | | | |
| | | Dose of olignucleotide (nM) | | | |
| Treatment | SEQ ID NO | 9.375 | 18.75 | 37.5 | 75 |
| 106734 | 45 | 85 | 62 | 26 | 10 |
| 113176 | 115 | 90 | 83 | 51 | 26 |
| 113196 | 135 | 97 | 70 | 17 | 8 |
| 337332 | 246 | 123 | 81 | 22 | 9 |
| 337333 | 247 | 76 | 51 | 14 | 4 |
| 337348 | 262 | 109 | 89 | 34 | 14 |
| 345778 | 326 | 76 | 36 | 6 | 2 |
| 345781 | 329 | 107 | 59 | 21 | 6 |
| 345785 | 333 | 79 | 45 | 9 | 2 |
| 345815 | 363 | 124 | 83 | 31 | 16 |
| 345823 | 370 | 136 | 111 | 70 | 35 |
| 129690 | 393 | 130 | 124 | 142 | 151 |
| 129691 | 410 | 101 | 126 | 128 | 107 |
| 129695 | 391 | 128 | 125 | 136 | 143 |
| 141923 | 409 | 88 | 92 | 84 | 76 |

As shown in Table 30, the antisense oligonucleotides targeted to human STAT3 inhibited expression in a dose-dependent manner in A549 cells, as measured using PS199.

An additional dose-response experiment was performed in the same manner, using an alternative range of antisense oligonucleotide concentrations. The data using primer probe sets PPS2O33 and PPS199 are shown in Tables 31 and 32, respectively, as percent of STAT3 mRNA expression relative to untreated control cells.

Table 31

| Antisense inhibition of human STAT3 in A549 cells measured using PPS2033 | | | | | |
|---|---|---|---|---|---|
| | | % Control | | | |
| | | Dose of oligonucleotide (nM) | | | |
| **Treatment** | **SEQ ID NO** | **18.75** | **37.5** | **75** | **150** |
| 106734 | 45 | 87 | 52 | 23 | 11 |
| 113176 | 115 | 114 | 68 | 24 | 20 |
| 113196 | 135 | 82 | 26 | 10 | 6 |
| 337332 | 246 | 88 | 49 | 14 | 7 |
| 337333 | 247 | 53 | 25 | 9 | 9 |
| 337348 | 262 | 56 | 35 | 10 | 2 |
| 345778 | 326 | 59 | 22 | 9 | 7 |
| 345781 | 329 | 83 | 39 | 7 | 3 |
| 345785 | 333 | 70 | 37 | 15 | 9 |
| 345815 | 363 | 50 | 27 | 13 | 7 |
| 345823 | 370 | 77 | 58 | 23 | 6 |
| 129690 | 393 | 102 | 110 | 111 | 103 |
| 129691 | 410 | 89 | 87 | 103 | 112 |
| 129695 | 391 | 108 | 112 | 121 | 129 |
| 141923 | 409 | 88 | 99 | 86 | 78 |

The data in Table 31 demonstrate that the additional concentrations tested also inhibited human STAT3in a dose-dependent manner.

Table 32

| Antisense inhibition of human STAT3 in A549 cells measured using PPS199 | | | | | |
|---|---|---|---|---|---|
| | | % Control | | | |
| | | Dose of oligonucleotide (nM) | | | |
| **Treatment** | **SEQ ID NO** | **18.75** | **37.5** | **75** | **150** |
| 106734 | 45 | 85 | 57 | 31 | 16 |
| 113176 | 115 | 101 | 64 | 33 | 19 |
| 113196 | 135 | 90 | 23 | 12 | 7 |
| 337332 | 246 | 76 | 47 | 14 | 5 |
| 337333 | 247 | 63 | 32 | 14 | 11 |
| 337348 | 262 | 67 | 45 | 13 | 5 |
| 345778 | 326 | 60 | 27 | 12 | 11 |
| 345781 | 329 | 94 | 50 | 12 | 5 |
| 345785 | 333 | 57 | 31 | 14 | 9 |
| 345815 | 363 | 44 | 25 | 13 | 7 |
| 345823 | 370 | 63 | 65 | 24 | 4 |
| 129690 | 393 | 99 | 102 | 116 | 98 |

(continued)

| Antisense inhibition of human STAT3 in A549 cells measured using PPS199 | | | | | |
|---|---|---|---|---|---|
| | | % Control | | | |
| | | Dose of oligonucleotide (nM) | | | |
| **Treatment** | **SEQ ID NO** | **18.75** | **37.5** | **75** | **150** |
| 129691 | 410 | 72 | 79 | 83 | 88 |
| 129695 | 391 | 104 | 105 | 142 | 137 |
| 141923 | 409 | 107 | 87 | 96 | 80 |

The data in Table 32 demonstrate that the additional concentrations tested also inhibited human STAT3 in a dose-dependent manner.

**Example 22**

**Antisense inhibition of human STAT3 in Hep3B cells: dose response**

Antisense inhibition of STAT3 was tested in a dose-response study in Hep3B cells.

The human hepatoma cell line Hep3B (Hep3B2.1-7) is obtained from the American Type Culture Collection (ATCC, Manassas, VA). This cell line was initially derived from a hepatocellular carcinoma of an 8-yr-old African-American male. The cells are epithelial in morphology and are tumorigenic in nude mice. Hep3B cells are routinely cultured in Minimum Essential Medium (MEM) with Earle's Balanced Salt Solution, 2 mM L-glutamine, 1.5 g/L sodium bicarbonate, 0.1 mM nonessential amino acids, 1.0 mM sodium pyruvate (ATCC #20-2003, Manassas, VA) and with 10% heat-inactivated fetal bovine serum (Invitrogen Life Technologies, Carlsbad, CA). Cells are routinely passaged by trypsinization and dilution when they reach 90% confluence.

Cells were treated with the antisense oligonucleotides ISIS 129688, ISIS 113196, ISIS 337333, ISIS 345794, ISIS 345815, or the control oligonucleotide ISIS 129688 (TTCGCGGCTGGACGATTCAG, incorporated herein as SEQ ID NO: 411), each mixed with LIPOFECTIN™, at a concentration of 3 μg/mL LIPOFECTINT™ per 100 nM of oligonucleotide. ISIS 129688, which does not target STAT3, is a 20 nucleotide "gapmer" consisting of ten 2'-deoxynucleotides flanked on both sides (5' and 3' directions) by five-nucleotide "wings" composed of 2'-O-methoxyethyl (2'-MOE)nucleotides. ISIS 129688 has a phosphorothioate backbone throughout the oligonucleotide, and all cytidine residues are 5-methylcytidines.

Oligonucleotide concentrations were 6.25nM, 25 nM, 50nM, and 100nM. Untreated cells served as the control to which data were normalized. STAT3 mRNA expression levels were measured in duplicate by real-time PCR, as described herein, using PPS2033. The data are shown in Table 33 as percent expression relative to the untreated controls.

**Table 33**

| Inhibition of STAT3 Expression in Hep3B Cells: Dose-response | | | | | |
|---|---|---|---|---|---|
| **Treatment** | **SEQ ID NO** | % Control | | | |
| | | Dose (nM) | | | |
| | | **100** | **50** | **25** | **6.25** |
| 113196 | 135 | 57 | 24 | 17 | 98 |
| 337333 | 247 | 50 | 35 | 25 | 23 |
| 345794 | 342 | 94 | 77 | 125 | 112 |
| 345815 | 363 | 74 | 99 | 89 | 104 |
| 129688 | 411 | 82 | 27 | 13 | 92 |

As shown in Table 33, treatment with ISIS 113926, ISIS 337333, ISIS 345815 or ISIS 345815 caused a dose-dependent reduction in STAT3 mRNA expression in Hep3B cells. ISIS 345794 reduced STAT3 mRNA expression at the 50 nM dose in Hep3B cells.

**Example 23**

**Antisense inhibition of human STAT3 in THLE-2 cells: dose response**

Antisense inhibition of STAT3 was tested in a dose-response study in THLE-2 cells.

The SV-40 transformed liver epithelial cell line THLE-2 was obtained from the American Type Culture Collection (ATCC; Manassas, VA) and cultured according to the supplier's instructions. Cells were plated at a density of approximately 10,000 cells per well in 96-well plates for use in oligonucleotide transfection experiments.

Cells were treated with the antisense oligonucleotides ISIS 129688, ISIS 113196, ISIS 337333, ISIS 345794, ISIS 345815, or the control oligonucleotide ISIS 129688, each mixed with LIPOFECTIN™, at a concentration of 3 μg/mL LIPOFECTIN™ per 100 nM of oligonucleotide. Oligonucleotide concentrations were 6.25nM, 25 nM, 50nM, and 100nM. Untreated cells served as the control to which data were normalized. STAT3 mRNA expression levels were measured in duplicate by real-time PCR, as described herein, using PPS2033. The data are shown in Table 34 as percent expression relative to the untreated controls.

**Table 34**

| Inhibition of STAT3 Expression in THLE-2 Cells: Dose-response | | | | | |
|---|---|---|---|---|---|
| Treatment | SEQ ID NO | % Control | | | |
| | | Dose (nM) | | | |
| | | **100** | **50** | **25** | **6.25** |
| 113196 | 135 | 93 | 13 | 6 | 5 |
| 337333 | 247 | 117 | 30 | 14 | 11 |
| 345794 | 342 | 111 | 32 | 16 | 21 |
| 345815 | 363 | 104 | 31 | 16 | 12 |
| 129688 | 411 | 93 | 95 | 83 | 72 |

As shown in Table 34, treatment with ISIS 113926, ISIS 337333, or ISIS 345815 caused a dose-dependent reduction in STAT3 mRNA expression in THLE-2 cells. ISIS 354794 also reduced STAT3 mRNA expression in THLE-2 cells.

**Example 24**

**Design and screening of dsRNAs targeting human STAT3**

In a further embodiment, a series of nucleic acid duplexes was designed to target STAT3 mRNA (GenBank® accession number NM_139276.2, incorporated herein as SEQ ID NO: 412) and is shown in Table 35. All compounds in Table 35 are oligoribonucleotides 19 nucleotides in length with phosphodiester internucleoside linkages (backbones) throughout. The compounds were prepared to have blunt ends. Table 35 shows the antisense strand of the dsRNA, and the sense strand is synthesized as the complement of the antisense strand. These sequences are shown to contain uracil (U) but one of skill in the art will appreciate that uracil (U) is generally replaced by thymine (T) in DNA sequences. "Target site" indicates the first (5'-most) nucleotide number on the particular target sequence to which the compound binds.

The compounds in Table 35 were tested for their effects on STAT3 mRNA in A549 cells. dsRNA controls that do not target human STAT3 included the duplex of ISIS 335449 (UUUGUCUCUGGUCCUUACUU; incorporated herein as SEQ ID NO: 413) and its complement, and the duplex of ISIS 359661 (UUAUCGCUUCUCGUUGCUU; incorporated herein as SEQ ID NO: 414) and its complement. ISIS 335449 is an oligoribonucleotide 20 nucleotides in length with phosphodiester internucleoside linkages (backbones) throughout the compound. ISIS 359661 is an oligoribonucleotide 19 nucleotides in length with phosphodiester internucleoside linkages (backbones) throughout the compound. Both ISIS 335449 and ISIS 359661 and their complements were prepared with blunt ends. ISIS 129700 (TAGTGCGGACCTACCCACGA, incorporated herein as SEQ ID NO: 415), which does not target STAT3, was used as a control single-stranded oligonucleotide. ISIS 129700 is a chimeric oligonucleotides ("gapmer") 20 nucleotides in length, composed of a central "gap" region consisting of 10 2'-deoxynucleotides, which is flanked on the 5' and 3' ends by a five-nucleotide "wings". The win-gs are composed of 2'-O-methoxyethyl (2'-MOE) nucleotides. The internucleoside (backbone) linkages are phosphorothioate (P=S) throughout the oligonucleotide. All cytidines are 5-methylcytidines.

A549 cells were treated with 150 nM of dsRNA compounds mixed with 15 g/mL LIPOFECTIN™ (Invitrogen Life Technologies, Carlsbad, CA) or 150 nM of single-strand oligonucleotides mixed with 15 g/mL LIPOFECTIN™ for a period of 4 hours, followed by 20 hours of culture in normal growth medium.

Human STAT3 mRNA expression was measured as described by other examples herein, using PPS2033. Results were normalized to untreated control cells, which were not treated with dsRNA compounds or single-strand oligonucleotides. Data are the average of 2 experiments and are presented in Table 35.

**Table 35**

| Inhibition of human STAT3 mRNA by dsRNAs in A549 cells | | | | | | |
|---|---|---|---|---|---|---|
| ISIS # | REGION | TARGET SEQ | TARGET SITE | SEQUENCE | % INHIB | SEQ ID NO |
| 36195 8 | Coding | 412 | 520 | CCAUUGGCUUCUCAAGAUA | 0 | 416 |
| 36195 9 | Coding | 412 | 892 | GCCCCGCCAGCUCACUCAC | 33 | 417 |
| 36196 0 | Coding | 412 | 925 | GAGUUUUCUGCACGUACUC | 36 | 418 |
| 36196 1 | Coding | 412 | 1075 | CCAGUUUCUUAAUUUGUUG | 21 | 419 |
| 36196 2 | Coding | 412 | 1096 | AAACUUUUUGCUGCAACUC | 66 | 420 |
| 36196 3 | Coding | 412 | 1171 | UUAAGUUUCUAAACAGCUC | 87 | 421 |
| 36196 4 | Coding | 412 | 1314 | AAUUUUAAGCUGAUAAUUC | 66 | 422 |
| 36196 5 | Coding | 412 | 1337 | GAGUCUUUGUCAAUGCACA | 63 | 423 |
| 36196 6 | Coding | 412 | 1424 | UUGUUGGAUUCUUCCAUGU | 59 | 424 |
| 36196 7 | Coding | 412 | 1593 | GUGGGUCUCUAGGUCAAUC | 38 | 425 |
| 36196 8 | Coding | 412 | 1605 | AACUGGCAAGGAGUGGGUC | 30 | 426 |
| 36196 9 | Coding | 412 | 1843 | CUGAAUAAUUCACACCAGG | 33 | 427 |
| 36197 0 | Coding | 412 | 1867 | AUUUAGCCCAUGUGAUCUG | 58 | 428 |
| 36197 1 | Coding | 412 | 1877 | UCUUUGCAAAAUUUAGCCC | 52 | 429 |
| 36197 2 | Coding | 412 | 1888 | CAGCCAUGUUUUCUUUGCA | 24 | 430 |
| 36197 3 | Coding | 412 | 1934 | AGGUCAAUGAUAUUGUCCA | 45 | 431 |
| 36197 4 | Coding | 412 | 1944 | CUUUUUCACAAGGUCAAUG | 53 | 432 |
| 36197 5 | Coding | 412 | 1954 | CCAGGAUGUACUUUUUCAC | 39 | 433 |
| 36197 6 | Coding | 412 | 2057 | UCACUGAAUCUUAGCAGGA | 20 | 434 |
| 36197 7 | 3' UTR | 412 | 2681 | AAAGAUAGCAGAAGUAGGA | 83 | 435 |
| 36197 8 | 3' UTR | 412 | 3010 | UUUGGAUGUCAGCAAGGUU | 76 | 436 |
| 36197 9 | 3' UTR | 412 | 3075 | CCCUUUAAUUGUUAUUAUU | 0 | 437 |
| 36198 0 | 3' UTR | 412 | 3501 | GGGAUUAUAUAAAUUACCA | 25 | 438 |
| 36198 1 | 3' UTR | 412 | 3625 | AAAUAAGUCUAUUUAUAAA | 11 | 439 |
| 36198 2 | 3' UTR | 412 | 3645 | GGCCAAUACAUUACAAAGG | 42 | 440 |
| 36198 3 | 3' UTR | 412 | 4010 | UAUCACCAAGAAACUGGCU | 42 | 441 |
| 36198 4 | 3' UTR | 412 | 4080 | GACUCAAGUUUAUCAGUAA | 0 | 442 |
| 36198 5 | 3' UTR | 412 | 4330 | AAUUCCACAGAAACUCUGA | 47 | 443 |
| 36198 6 | 3' UTR | 412 | 4350 | UAAUUUGAUUUAACAAACA | 21 | 444 |
| 36198 7 | 3' UTR | 412 | 4480 | UAUGUACUGAAGAGUGUUG | 62 | 445 |
| 36198 8 | 3' UTR | 412 | 4520 | UCUCACCUUUCUAAAUAUU | 44 | 446 |
| 36198 9 | 3' UTR | 412 | 4630 | AAGACCAGAUACAUGCUAC | 69 | 447 |

(continued)

| Inhibition of human STAT3 mRNA by dsRNAs in A549 cells | | | | | | |
|---|---|---|---|---|---|---|
| ISIS # | REGION | TARGET SEQ | TARGET SITE | SEQUENCE | % INHIB | SEQ ID NO |
| 36199 0 | 3' UTR | 412 | 4650 | CUUUUGCUACAAUCAGAGU | 38 | 448 |
| 36199 1 | 3' UTR | 412 | 4790 | CCACCUUAUAGGUAGGUAA | 38 | 449 |
| 36199 2 | 3' UTR | 412 | 4840 | GAAGUACACAUUGGAAUUU | 53 | 450 |
| 36199 3 | 3' UTR | 412 | 4930 | UGGAAGUUAAAGUAGAUAC | 57 | 451 |
| 36199 4 | 3' UTR | 412 | 4956 | UACGGUUCCUAUAUAACGU | 45 | 452 |

These data demonstrate that dsRNAs targeted to human STAT3 reduced mRNA expression in A549 cells. The dsRNAs comprising the antisense strands of SEQ ID NOs 417, 418, 420, 421, 422, 423, 424, 425, 426, 427, 428, 429, 431, 432, 433, 435, 436, 440, 441, 443, 445, 446, 447, 448, 449, 450, 451 and 452 and their complements demonstrated at least 30% inhibition of STAT3 in this assay.

**Example 25**

**Apoptosis in lung carcinoma cell lines following antisense inhibition of human STAT3**

Antisense oligonucleotides targeted to human STAT3 were tested for their effects on apoptosis in A549 and H460 cells, both of which are lung carcinoma cell lines.

A549 cells were obtained and cultured as described herein. The H-460 lung carcinoma cell line was obtained from the American Type Culture Collection (ATCC; Manassas, VA) and cultured in RPMI medium supplemented with 10% fetal bovine serum, 100 units/mL penicillin and 100 μg/mL streptomycin (medium and supplements from Invitrogen Life Technologies, Carlsbad, CA). Both cell types were plated at a density of 10,000 cells per well in 96-well plates.

Cells were treated with antisense oligonucleotides mixed with LIPOFECTIN™, at a concentration of 3 μg/mL LIPOFECTIN™ per 100 nM of oligonucleotide. Oligonucleotide concentrations were 10, 20, 40 and 80 nM. ISIS 29848 (NNNNNNNNNNNNNNNNNNNN, where N = A, T, C or G; SEQ ID NO: 453) is a randomized sequence and was used as a negative control. Untreated cells served as the control to which data were normalized. Cells were transfected with antisense oligonucleotides and STAT3 mRNA levels were measured 24 hours following treatment by real-time PCR using PPS2033.

To evaluate the effects of antisense inhibition of STAT3 on apoptosis, a caspase assay was performed 24 and 48 hours following antisense oligonucleotide treatment. Caspase-3 activity was evaluated with a fluorometric HTS Caspase-3 assay (Catalog #HTS02; EMD Biosciences, San Diego, CA) that detects cleavage after aspartate residues in the peptide sequence DEVD. The DEVD substrate is labeled with a fluorescent molecule, which exhibits a blue to green shift in fluorescence upon cleavage by caspase-3. Active caspase-3 in the oligomeric compound-treated cells was measured by this assay according to the manufacturer's instructions. Following antisense oligonucleotide treatment, 50 L of assay buffer containing 10 M dithiothreitol was added to each well, followed by addition 20 L of the caspase-3 fluorescent substrate conjugate. Fluorescence in wells was immediately detected (excitation/emission 400/505 nm) using a fluorescent plate reader (SpectraMAX™ GeminiXS™, Molecular Devices, Sunnyvale, CA). The plate was covered and incubated at 37 C for an additional three hours, after which the fluorescence was again measured (excitation/emission 400/505 nm). The value at time zero was subtracted from the measurement obtained at 3 hours. The measurement obtained from the untreated control cells was designated as 100% activity. Caspase-3 activity in cells treated with oligomeric compounds was normalized to that in untreated control cells. Values for caspase activity above or below 100% were considered to indicate that the compound has the ability to stimulate or inhibit caspase activity, respectively.

Apoptosis was also evaluated by detecting the cleavage of poly(ADP-ribose) polymerase (PARP) in A549 and H460 cells, which were plated at 250,000 cells per well in 6-well treated with 150 nM of antisense oligonucleotides targeted to human STAT3. Protein was isolated 48 hours following oligonucleotide treatment and subjected to western blot analysis. PARP was detected with a commercially available antibody (Cell Signaling, Beverly, MA), followed by detection with ECLplus™ (Amersham Biosciences, Piscataway, NJ). The signal was detected witch a Chemidoc™ EQ System (Bio-Rad Laboratories, Hercules, CA). Both full-length PARP and cleaved PARP were quantitated.

Apoptosis was further evaluated by measuring hypodiploidy in A549 and H460 cells, which were plated at 50,000 cells per well in 24 well plates and treated with 20, 80 and 150 nM of antisense oligonucleotides targeted to human STAT3. ISIS 226844 (GCCCTCCATGCTGGCACAGG, SEQ ID NO: 454) does not target STAT3 and was used as a negative control. ISIS 183891 (CCGAGCTCTCTTATCAACAG, SEQ ID NO: 455) is targeted to kinesin-like 1,

inhibition of which results in cell cycle arrest, and was used as a positive control at a dose of 100 nM. Cells were harvested 48 hours following treatment and routine procedures were used to prepare cells for flow cytometry analysis. Cells were stained with propidium iodide to generate a cell cycle profile using a flow cytometer. The cell cycle profile was analyzed with the ModFit program (Verity Software House, Inc., Topsham ME). Fragmentation of nuclear DNA is a hallmark of apoptosis and produces an increase in cells with a hypodiploid DNA content, which are categorized as "subG1". An increase in cells in G1 phase is indicative of a cell cycle arrest prior to entry into S phase; an increase in cells in S phase is indicative of cell cycle arrest during DNA synthesis; and an increase in cells in the G2/M phase is indicative of cell cycle arrest just prior to or during mitosis. Aneuploid (A) cells have a DNA content greater than that in diploid cells. Cell cycle profiles of cells treated with oligomeric compounds were normalized to those of untreated control cells. Values above or below 100% were considered to indicate an increase or decrease, respectively, in the proportion of cells in having the DNA content of the indicated cell cycle stage.

The data from these assays in A549 cells are shown in the following tables. STAT3 mRNA levels in A549 cells are shown in Table 36 as percent mRNA expression relative to untreated control cells. Caspase-3 activity at 24 and 48 hours following oligonucleotide treatment is presented in Table 37 as percentage of untreated control cells. PARP quantitation is shown in Table 38 as percentage relative to untreated control cells. DNA profiles are shown in Table 39 as percentage in each phase of the cell cycle.

**Table 36**

| Antisense inhibition of STAT3 in A549 cells | | | | | |
|---|---|---|---|---|---|
| | | % Control | | | |
| | | Dose of oligonucleotide (nM) | | | |
| **ISIS #** | **SEQ ID NO** | **10** | **20** | **40** | **80** |
| 106707 | 58 | 37 | 40 | 27 | 26 |
| 113176 | 115 | 42 | 35 | 21 | 16 |
| 113196 | 135 | 2 | 1 | 1 | 2 |
| 337332 | 246 | 24 | 3 | 5 | 18 |
| 337333 | 247 | 17 | 16 | 16 | 17 |
| 337348 | 262 | 44 | 19 | 10 | 12 |
| 345778 | 326 | 8 | 12 | 9 | 14 |
| 345781 | 329 | 6 | 1 | 2 | 8 |
| 345785 | 333 | 7 | 4 | 4 | 9 |
| 29848 | 453 | 76 | 75 | 71 | 70 |
| 129987 | 404 | 62 | 58 | 49 | 41 |

**Table 37**

| Caspase-3 activity following antisense inhibition of STAT3 in A549 cells | | | | | | | | | |
|---|---|---|---|---|---|---|---|---|---|
| | Caspase-3 Activity, % control 24 hours | | | | | Caspase-3 Activity, % control 48 hours | | | |
| ISIS # | Dose of Oligonucleotide (nM) | | | | | Dose of Oligonucleotide (nM) | | | |
| | 10 | 20 | 40 | 80 | | 10 | 20 | 40 | 80 |
| 106707 | 54 | 60 | 70 | 64 | | 67 | 73 | 83 | 71 |
| 113176 | 77 | 87 | 88 | 83 | | 56 | 38 | 124 | 125 |
| 113196 | 76 | 108 | 97 | 93 | | 165 | 506 | 674 | 597 |
| 337332 | 155 | 243 | 195 | 208 | | 494 | 1782 | 1608 | 766 |
| 337333 | 52 | 70 | 63 | 102 | | 118 | 121 | 147 | 200 |
| 337348 | 39 | 51 | 72 | 66 | | 73 | 82 | 2167 | 2649 |
| 345778 | 31 | 34 | 45 | 70 | | 146 | 134 | 124 | 207 |
| 345781 | 220 | 258 | 240 | 279 | | 1917 | 2766 | 3434 | 3428 |
| 345785 | 69 | 84 | 90 | 97 | | 516 | 889 | 1307 | 1364 |
| 226844 | 86 | 81 | 78 | 75 | | 153 | 400 | 2114 | 843 |
| 29848 | 72 | 70 | 85 | 68 | | 16 | 18 | 8 | 14 |
| 129987 | 58 | 52 | 66 | 66 | | 67 | 61 | 53 | 61 |

**Table 38**

| Full-length and cleaved PARP following antisense inhibition of STAT3 in A549 cells | | |
|---|---|---|
| | PARP % control | |
| ISIS # | Full-length | Cleaved |
| 106707 | 89 | 104 |
| 113176 | 87 | 97 |
| 113196 | 8 | 2171 |
| 337332 | 6 | 2532 |
| 337333 | 11 | 1828 |
| 337348 | 16 | 975 |
| 345778 | 7 | 665 |
| 345781 | 2 | 823 |
| 345785 | 1 | 313 |
| 29848 | 84 | 127 |
| 129987 | 106 | 119 |

**Table 39**

| DNA profiles following antisense inhibition of STAT3 in A549 cells | | | | | | |
|---|---|---|---|---|---|---|
| | | DNA Profiles % of cells per profile | | | | |
| Treatment | Oligonucleotide concentration (nM) | Sub G1 | G1 | G1/S | G2/M | A |
| UTC | none | 0 | 51 | 29 | 20 | 13 |

(continued)

| DNA profiles following antisense inhibition of STAT3 in A549 cells | | | | | | |
|---|---|---|---|---|---|---|
| | | DNA Profiles % of cells per profile | | | | |
| Treatment | Oligonucleotide concentration (nM) | Sub G1 | G1 | G1/S | G2/M | A |
| UTC | none | 0 | 49 | 32 | 19 | 14 |
| 106707 | 20 | 0 | 51 | 30 | 19 | 15 |
| | 80 | 0 | 53 | 28 | 18 | 17 |
| | 150 | 0 | 55 | 26 | 20 | 17 |
| 113176 | 20 | 0 | 54 | 28 | 18 | 16 |
| | 80 | 0 | 54 | 29 | 17 | 16 |
| | 150 | 5 | 53 | 29 | 18 | 15 |
| 113196 | 20 | 3 | 50 | 28 | 22 | 12 |
| | 80 | 34 | 67 | 21 | 13 | 7 |
| | 150 | 32 | 63 | 23 | 14 | 4 |
| 337332 | 20 | 10 | 47 | 29 | 24 | 10 |
| | 80 | 39 | 67 | 18 | 16 | 3 |
| | 150 | 42 | 69 | 18 | 13 | 5 |
| 337333 | 20 | 0 | 52 | 29 | 20 | 12 |
| | 80 | 22 | 56 | 30 | 14 | 7 |
| | 150 | 20 | 57 | 25 | 18 | 8 |
| 337348 | 20 | 0 | 54 | 28 | 19 | 16 |
| | 80 | 1 | 51 | 30 | 19 | 16 |
| | 150 | 27 | 53 | 24 | 23 | 12 |
| 345778 | 20 | 1 | 51 | 26 | 23 | 14 |
| | 80 | 25 | 63 | 21 | 16 | 5 |
| | 150 | 30 | 60 | 24 | 16 | 5 |
| 345781 | 20 | 20 | 47 | 28 | 25 | 13 |
| | 80 | 43 | 62 | 27 | 11 | 5 |
| | 150 | 39 | 61 | 30 | 9 | 5 |
| 345785 | 20 | 3 | 50 | 27 | 23 | 14 |
| | 80 | 33 | 61 | 18 | 21 | 8 |
| | 150 | 32 | 59 | 23 | 18 | 7 |
| 29848 | 20 | 0 | 52 | 30 | 18 | 16 |
| | 80 | 0 | 52 | 30 | 17 | 15 |
| | 150 | 2 | 58 | 22 | 20 | 12 |
| 129987 | 20 | 0 | 52 | 29 | 19 | 15 |
| | 80 | 0 | 49 | 31 | 20 | 16 |
| | 150 | 0 | 53 | 28 | 18 | 16 |
| 183891 | 100 | 2 | 7 | 7 | 86 | 11 |

It is evident from these data that antisense inhibition of STAT3 resulted in apoptosis in A549 cells, as measured by three different assays. For example, treatment with ISIS 345781 at 20 nM, which inhibited STAT3 mRNA expression by as much as 99% (Table 36), and dramatically increased caspase-3 activity at 48 hours to a level approximately 30 times greater (Table 37) than that observed in the untreated control. Additionally, full-length PARP was reduced to 2% of the control, and cleaved PARP was approximately 8 times that of the control (Table 38). Furthermore, the subG1 population of cells treated with ISIS 345781 was approximately 20 times greater than the control (Table 39), indicating that DNA fragmentation occurred. In general, the distribution of cells in other phases of the cell cycle, subG1, G1, G1/S and G2/M, was not markedly changed. ISIS 337332 caused an increase in the G1 population at the 80 and 150 nM doses, which is indicative of a G1 cell cycle arrest. Together, these data clearly demonstrate that antisense inhibition of STAT3 by ISIS 345781 induced apoptosis in A549 cells. Other antisense oligonucleotide treatments similarly caused a decrease in STAT3 expression concomitant with an induction of apoptosis.

The data from the real-time PCR, caspase-3, PARP and DNA profile assays in H-460 cells are shown in the following tables. STAT3 mRNA levels in H-460 cells are shown in Table 40 as percent mRNA expression relative to untreated control cells. Caspase-3 activity at 24 and 48 hours following oligonucleotide treatment is presented in Table 41 as percentage of untreated control cells. PARP quantitation is shown in Table 42 as percentage relative to untreated control cells. DNA profiles are shown in Table 43 as percentage in each phase of the cell cycle.

**Table 40**

| Antisense inhibition of STAT3 in H-460 cells | | | | | |
|---|---|---|---|---|---|
| | | % Control | | | |
| | | Dose of oligonucleotide (nM) | | | |
| ISIS # | SEQ ID NO | 10 | 20 | 40 | 80 |
| 106707 | 58 | 49 | 49 | 33 | 30 |
| 113176 | 115 | 52 | 50 | 22 | 17 |
| 113196 | 135 | 12 | 4 | 4 | 6 |
| 337332 | 246 | 21 | 18 | 12 | 15 |
| 337333 | 247 | 26 | 20 | 14 | 22 |
| 337348 | 262 | 41 | 25 | 16 | 12 |
| 345778 | 326 | 11 | 7 | 8 | 9 |
| 345781 | 329 | 13 | 6 | 5 | 9 |
| 345785 | 333 | 15 | 8 | 7 | 15 |
| 29848 | 453 | 85 | 83 | 85 | 68 |
| 129987 | 404 | 53 | 61 | 65 | 48 |

**Table 41**

| Caspase-3 activity following antisense inhibition of STAT3 in H-460 cells | | | | | | | | |
|---|---|---|---|---|---|---|---|---|
| | Caspase-3 Activity, % control 24 hours | | | | Caspase-3 Activity, % control 48 hours | | | |
| ISIS # | Dose of Oligonucleotide (nM) | | | | Dose of Oligonucleotide (nM) | | | |
| | 10 | 20 | 40 | 80 | 10 | 20 | 40 | 80 |
| 106707 | 83 | 81 | 70 | 83 | 37 | 24 | 27 | 57 |
| 113176 | 97 | 85 | 80 | 143 | 37 | 44 | 121 | 235 |
| 113196 | 241 | 291 | 285 | 264 | 101 | 130 | 163 | 140 |
| 337332 | 132 | 416 | 431 | 487 | 205 | 364 | 399 | 393 |
| 337333 | 84 | 84 | 88 | 87 | 93 | 113 | 111 | 116 |
| 337348 | 55 | 54 | 71 | 125 | 58 | 62 | 297 | 440 |
| 345778 | 65 | 83 | 91 | 119 | 111 | 125 | 139 | 170 |
| 345781 | 218 | 361 | 426 | 493 | 509 | 586 | 630 | 654 |
| 345785 | 76 | 84 | 112 | 154 | 210 | 237 | 279 | 313 |
| 226844 | 84 | 78 | 128 | 138 | 81 | 89 | 293 | 269 |
| 29848 | 112 | 86 | 90 | 92 | 59 | 41 | 46 | 55 |
| 129987 | 98 | 93 | 76 | 96 | 85 | 67 | 75 | 87 |

**Table 42**

| PARP cleavage following antisense inhibition of STAT3 in H-460 cells | | |
|---|---|---|
| | PARP % of control | |
| ISIS # | Full length | Cleaved |
| 106707 | 188 | 146 |
| 113176 | 144 | 112 |
| 113196 | 14 | 47 |
| 337332 | 29 | 97 |
| 337333 | 36 | 120 |
| 337348 | 74 | 247 |
| 345778 | 30 | 100 |
| 345781 | 4 | 13 |
| 345785 | 43 | 143 |
| 29848 | 129 | 100 |
| 129987 | 165 | 128 |

**Table 43**

| DNA profile following antisense inhibition of STAT3 in H-460 cells | | | | | | |
|---|---|---|---|---|---|---|
| | | DNA Profiles % of cells per profile | | | | |
| Treatment | Oligonucleotide concentration (nM) | Sub G1 | G1 | G1/S | G2/M | A |
| UTC | none | 2 | 52 | 31 | 17 | 16 |

(continued)

| DNA profile following antisense inhibition of STAT3 in H-460 cells | | | | | | |
|---|---|---|---|---|---|---|
| | | DNA Profiles % of cells per profile | | | | |
| Treatment | Oligonucleotide concentration (nM) | Sub G1 | G1 | G1/S | G2/M | A |
| UTC | none | 1 | 50 | 33 | 18 | 17 |
| 106707 | 20 | 2 | 50 | 32 | 18 | 20 |
| | 80 | 1 | 51 | 32 | 17 | 19 |
| | 150 | 2 | 52 | 30 | 18 | 20 |
| 113176 | 20 | 2 | 50 | 33 | 18 | 21 |
| | 80 | 8 | 47 | 35 | 17 | 19 |
| | 150 | 18 | 49 | 24 | 27 | 13 |
| 113196 | 20 | 11 | 42 | 41 | 17 | 16 |
| | 80 | 32 | 46 | 39 | 15 | 9 |
| | 150 | 27 | 47 | 37 | 16 | 11 |
| 337332 | 20 | 13 | 44 | 40 | 16 | 15 |
| | 80 | 28 | 41 | 45 | 15 | 7 |
| | 150 | 28 | 43 | 45 | 13 | 7 |
| 337333 | 20 | 7 | 42 | 40 | 17 | 18 |
| | 80 | 15 | 43 | 40 | 17 | 14 |
| | 150 | 14 | 43 | 41 | 15 | 14 |
| 337348 | 20 | 2 | 56 | 27 | 16 | 23 |
| | 80 | 11 | 46 | 38 | 16 | 19 |
| | 150 | 7 | 47 | 37 | 16 | 19 |
| 345778 | 20 | 22 | 49 | 35 | 16 | 12 |
| | 80 | 20 | 48 | 36 | 15 | 11 |
| | 150 | 13 | 44 | 40 | 15 | 17 |
| 345781 | 20 | 25 | 41 | 39 | 20 | 10 |
| | 80 | 26 | 43 | 39 | 18 | 8 |
| | 150 | 8 | 48 | 38 | 15 | 18 |
| 345785 | 20 | 22 | 47 | 39 | 14 | 10 |
| | 80 | 20 | 48 | 37 | 15 | 15 |
| | 150 | 2 | 49 | 34 | 17 | 19 |
| 29848 | 20 | 3 | 47 | 35 | 19 | 18 |
| | 80 | 10 | 41 | 41 | 17 | 15 |
| | 150 | 2 | 49 | 33 | 19 | 20 |
| 129987 | 20 | 2 | 50 | 32 | 18 | 21 |
| | 80 | 2 | 49 | 32 | 19 | 19 |
| | 150 | 16 | 17 | 44 | 39 | 16 |
| 183891 | 100 | 2 | 52 | 31 | 17 | 16 |

Antisense inhibition of STAT3 in H-460 cells induced apoptosis. For example, treatment with 20 nM of ISIS 345785 inhibited STAT3 expression by approximately 92% (Table 40). This reduction in target expression was accompanied by an approximately 2.4-fold increase in caspase-3 activity (Table 41), relative to the untreated control. Full-length PARP was reduced to 43% of the control, whereas cleaved PARP was increased to 143% of control (Table 42). Moreover, hypodiploidy in cells treated with 20 nM of ISIS 345785 was increased approximately 4 times that in control cells (Table 43). Thus, antisense inhibition of STAT3 by ISIS 3456785 in H-460 cells resulted in the induction of apoptosis. Other antisense oligonucleotide treatments similarly caused a decrease in STAT3 expression concomitant with an induction of apoptosis.

In addition to inducing apoptosis, antisense inhibition of STAT3 by ISIS 113176 resulted in an S phase arrest, indicating that DNA replication was blocked.

**Example 26**

**Inhibition of STAT3 and tumor growth in vivo in a model of ALCL**

NPM-ALK transgenic mice spontaneously develop T-cell lymphomas and plasma cell tumors (Chiarle, et al., Blood, 2003, 101, 1919-1927). Thus, they are used as in vivo models of anaplastic large cell lymphoma (ALCL) and multiple myeloma, both of which are associated with STAT3 activation. Additionally, when cells from the T-cell lymphomas and plasma cells tumors are injected into recipient mice, subcutaneous tumors develop in the recipient mice. Transplanted T-cell lymphomas and plasma cell tumors serve as animal models for ALCL and multiple myeloma, respectively.

Antisense oligonucleotides targeted to STAT3 were tested for their effects on STAT3 expression and tumor growth in ALCL *in vivo.* Approximately 2 X $10^6$ cells derived from mouse NPM-ALK T-cell tumors were injected into syngeneic (genetically identical) mice. 7 days following injection of the tumor cells, mice were given daily subcutaneous injections of ISIS 17152 (SEQ ID NO: 99) or a 3 base mismatch control oligonucleotide ISIS 28084 (SEQ ID NO: 151) at a dose of 20 mg/kg. Oligonucleotides were administered for 14 days to six animals per oligonucleotide treatment. Oligonucleotides were injected subcutaneously counterlaterally to the tumor sides. Tumor volumes were measured daily and are shown in Table 44.

**Table 44**

| Tumor volume in a model of ALCL: mouse NPM-ALK cells | | |
|---|---|---|
| | Tumor Volume $mm^3$ | |
| Day of treatment | STAT3 ASO | Control oligo |
| 1 | 30 | 30 |
| 2 | 30 | 80 |
| 3 | 50 | 500 |
| 4 | 90 | 1300 |
| 5 | 110 | 1480 |

Minimal or no growth of tumors was observed in animals treated with ISIS 17152. Conversely, in animals treated with the control oligonucleotide, large tumor masses developed in all 5 animals within 14 days of injection of the tumor cells. STAT3 protein was measured by western blot as described herein, using a monoclonal STAT3 antibody (Zymed Laboratories, Inc., South San Francisco, CA) which was detected with a chemiluminescence system (ECL, Amersham Biosciences, Piscataway, NJ) STAT3 protein was reduced in the tumor as well as in liver in animals treated with ISIS 17152. Furthermore, histological examination of residual tumor mass revealed that in addition to being substantially smaller, tumors from animals treated with ISIS 17152 displayed significant areas of necrosis and single-cell death.

A similar in vivo experiment was performed in which approximately 1 X $10^7$ human NPM-ALK tumor cells (provided by Dr. A Lorenzana, Van Eslander Cancer Center, Gross Pointe Woods, MI) were injected into immunocompromised mice. This study was performed as described for mouse NPM-ALK cells, using ISIS 113176 (SEQ ID NO: 115) or a mismatched control oligonucleotide (129987, SEQ ID NO: 404) at 20 mg/kg. Oligonucleotides were injected subcutaneously counterlaterally to the tumor sides. Oligonucleotides were administered for 14 days to groups containing 3 animals each. Tumor volumes were measured and are shown in Table 44.

Table 44

| Tumor volume in a model of ALCL using human NPM-ALK cells | | |
|---|---|---|
| | Tumor Volume $mm^3$ | |
| Day of treatment | STAT3 ASO | Control oligo |
| 0 | 50 | 50 |
| 2 | 100 | 100 |
| 4 | 100 | 240 |
| 6 | 100 | 520 |
| 8 | 260 | 1310 |
| 10 | 330 | 2670 |
| 12 | 360 | 3140 |
| 14 | 790 | 4810 |

Minimal or no growth of tumors was observed in animals treated with ISIS 113176. Conversely, tumor growth was seen in animals injected with a mismatched control oligonucleotide. These data demonstrate that antisense inhibition of STAT3 inhibits STAT3 protein expression in tumors and inhibits tumor growth *in vivo* in a model of ALCL. These findings demonstrate that STAT3 inhibition is a therapeutic approach in the treatment of human ALCL.

**Example 27**

**Inhibition of tumor growth in vivo in a model of multiple myeloma**

Multiple myeloma cells derived from NPM-ALK transgenic mice, which are characterized by the presence of constitutively activated STAT3, were subcutaneously injected into syngeneic mice. Mice received daily subcutaneous injections of ISIS 17152 (SEQ ID NO: 99) or a mismatched control oligonucleotide (SEQ ID NO: 151) at a dose of 20 mg/kg. Oligonucleotides were administered for 14 days. Oligonucleotides were injected subcutaneously counterlaterally to the tumor sides. Tumor volumes were measured every 2 days and are shown in Table 45.

Table 45

| Tumor volume in a model of ALCL using human NPM-ALK cells | | |
|---|---|---|
| | Tumor Volume $mm^3$ | |
| Day of treatment | STAT3 ASO | Control oligo |
| 0 | 80 | 170 |
| 2 | 80 | 830 |
| 4 | 80 | 1170 |
| 6 | 170 | 1670 |
| 8 | 250 | 3500 |
| 10 | 500 | 6670 |
| 12 | 500 | 8580 |
| 14 | 1170 | 11000 |
| 16 | 1330 | 13670 |

As shown in Table 45, ISIS 17152, targeted to STAT3, inhibted tumor growth in this model, whereas the control oligonucleotide did not. These data demonstrate that antisense inhibition of STAT3 prevents tumor growth *in vivo* in a model of multiple myeloma.

**Example 28**

**Effects of antisense inhibition of human STAT3 in ALCL cell lines**

Antisense inhibition of STAT3 was evaluated for its effects on cell growth and survival in human ALCL cells (TS ALCL cells or SU-DHL cells, provided by Dr. A. Lorenzana, Van Eslander Cancer Center, Gross Pointe Woods, MI). TS ALCL cells were transfected with 0.02, 0.3, 1.5, 3, 6, 12 and 15 M of ISIS 113176 (SEQ ID NO: 115) or the 3-base mismatched ISIS 129987 (SEQ ID NO: 404) by electroporation. 2 X $10^6$ cells were electroporated using an Amaxa® electroporation unit (Amaxa Biosystems, Gaithersburg, MD) according to the manufacturer's instructions. After 24 hours, cells were harvested. Cell viability was evaluated by trypan blue staining, which is taken up only by unhealthy or dead cells. Cell viability and STAT3 protein were not reduced in cells treated with the mismatched control. However, cells treated with ISIS 113176 displayed a dose-dependent reduction in cell viability, which was 97%, 87%, 67%, 60% and 29% of the control at doses of 0.03, 1.5, 3, 6 and 12 M. Cell viability was reduced to 36% of the control in cells receiving a 15 M dose of ISIS 113176. In addition to a reduction in cell viability, western blotting of treated cells, performed as described herein, revealed that STAT3 protein expression was decreased in a dose-dependent manner.

The incorporation of tritiated thymidine into newly synthesized DNA serves as a measure of cell proliferation. Cell growth following antisense inhibition of STAT3 was evaluated by measuring the amount of tritiated ($^3$H) thymidine incorporated into cells transfected with ISIS 113176 (SEQ ID NO: 115). SU-DHL cells were transfected with 7 μM of ISIS 113176. Cells were transfected with a constitutively activated STAT3, STAT3c, which has an amino acid substitution that causes STAT3 to spontaneously dimerize, bind to DNA and activate transcription (Bromberg, J.F., et al., Cell, 1999, 98, 295-303). Cells were cultured in the presence of 1 Ci of $^3$H-thymidine during the last 18 hours of culture. 24, 48 and 72 hours following oligonucleotide treatment, cells were harvested. $^3$H-thymidine incorporation was measured by routine scintillation counting and is shown as counts per minute (CPM) in Table 46. Jurkat cells (which can be obtained from ATCC, Manassas, VA) were used as a control for cells not affected by STAT3 activation or inhibition.

**Table 46**

| **Reduced cell proliferation following antisense inhibition in human ALCL cells** | | | | |
|---|---|---|---|---|
| | | **$^3$H-Thymidine incorporated, CPM** | | |
| | | **Time following tranfection (hours)** | | |
| **Cell type** | **Treatment** | **24** | **48** | **72** |
| SU-DHL | ISIS 113176 | 770 | 1530 | 1680 |
| | STAT3c | 2000 | 3150 | 3590 |
| | untreated | 1350 | 2530 | 3090 |
| Jurkat | ISIS 113176 | 2670 | 5330 | 8000 |
| | STAT3c | 1560 | 3780 | 5940 |
| | untreated | 1940 | 3830 | 6440 |

As shown in Table 46, antisense inhibition in human SU-DHL cells markedly inhibited cell proliferation. Jurkat cells, which do not express constutitively activated STAT3, were unaffected by antisense oligonucleotide treatment. Thus, the effects on cell proliferation are specific to antisense inhibition of STAT3.

In a similar assay, TS ALCL cells were tranfected with 7 μM of ISIS 113176. STAT3 expression and PARP cleavage were evaluated by western blot 24, 48 and 72 hours following oligonucleotide treatment, using procedures described herein. STAT3 protein expression was reduced and the cleavage of PARP, an indicator of apoptosis, was observed, both of which occured in a time-dependent manner. Cell growth was evaluated by measuring $^3$H-thymidine incorporation into these cells.

**Table 47**

| **Reduced cell proliferation following antisense inhibition of STAT3 in TS ALCL cells** | | | |
|---|---|---|---|
| | $^3$H-Thymidine incorporated, CPM | | |
| | Time following tranfection (hours) | | |
| Treatment | 24 | 48 | 72 |

(continued)

| Reduced cell proliferation following antisense inhibition of STAT3 in TS ALCL cells | | | |
|---|---|---|---|
| | $^3$H-Thymidine incorporated, CPM | | |
| | Time following tranfection (hours) | | |
| ISIS 113176 | 770 | 1530 | 1680 |
| STAT3c | 2000 | 3150 | 3590 |
| untreated | 1350 | 2530 | 3090 |

These data demonstrate that antisense inhibition of STAT3 reduced cell proliferation in anaplastic lymphoma cells.

**Example 29**

**Effects of antisense oligonucleotides targeted to STAT3 in normal mice: toxicity screen**

Antisense oligonucleotides targeted to STAT3 were evaluated for their effects in normal mice. C57B1/6 mice were injected subcutaneously with a 50 mg/kg dose of oligonucleotide, twice weekly for a period of 4 weeks. STAT3 antisense oligonucleotides administered were ISIS 337332, ISIS 337333, ISIS 345778, ISIS 345781, ISIS 113196, ISIS 345815, ISIS 345794, or ISIS 345823. ISIS 335099 (GAGTATCACTATGGCTGGCC, SEQ ID NO: 456) does not target STAT3 and was used as a control oligonucleotide. Animals receiving saline injections were also used as a control group. Each treatment group contained 5 animals. Blood samples were collected before treatment (Wk0) and after 2 (Wk2) and 4 (Wk4) weeks of treatment. Blood glucose, cholesterol and triglycerides were measured by routine clinical analysis. These data are shown in Table 48.

**Table 48**

| **Effect of Antisense Oligonucleotides Targeted to STAT3 on Plasma Glucose, Triglycerides, and Cholesterol in Normal Mice** | | | | | | | | | | |
|---|---|---|---|---|---|---|---|---|---|---|
| **Treatment** | **SEQ ID NO** | **Glucose (mg/dL)** | | | **Triglycerides (mg/dL)** | | | **Cholesterol (mg/dL)** | | |
| | | **Wk 0** | **Wk 2** | **Wk 4** | **Wk 0** | **Wk 2** | **Wk 4** | **Wk 0** | **Wk 2** | **Wk 4** |
| **Saline** | n/a | 175 | 206 | 192 | 112 | 70 | 124 | 68 | 65 | 73 |
| **113196** | 135 | 165 | 185 | 192 | 87 | 75 | 62 | 67 | 69 | 54 |
| **337332** | 246 | 170 | 184 | 217 | 110 | 59 | 64 | 75 | 115 | 10 3 |
| **337333** | 247 | 180 | 177 | 213 | 93 | 81 | 71 | 72 | 52 | 55 |
| **345778** | 326 | 168 | 182 | 187 | 133 | 104 | 89 | 73 | 62 | 55 |
| **345781** | 329 | 186 | 180 | 190 | 102 | 58 | 71 | 77 | 149 | 12 1 |
| **345794** | 342 | 186 | 193 | 201 | 81 | 100 | 84 | 81 | 91 | 94 |
| **345815** | 363 | 178 | 175 | 157 | 87 | 154 | 108 | 81 | 142 | 17 8 |
| **345823** | 370 | 183 | 194 | 239 | 123 | 99 | 106 | 81 | 81 | 96 |

The liver transaminases ALT and AST, increases in which can indicate toxicities, were also measured in brood by routine clinical analysis. These data are shown in Table 49.

Table 49

| Effect of Antisense Oligonucleotides Targeted to STAT3 on Liver Transaminase Levels in Normal Mice | | | | | | | |
|---|---|---|---|---|---|---|---|
| Treatment | SEQ ID NO | ALT (IU/L) | | | ALT (IU/L) | | |
| | | Wk 0 | Wk 2 | Wk 4 | Wk 0 | Wk 2 | Wk 4 |
| **Saline** | none | 39 | 30 | 33 | 57 | 44 | 64 |
| **113196** | 135 | 33 | 49 | 76 | 48 | 52 | 109 |
| **337332** | 246 | 34 | 191 | 123 | 48 | 168 | 159 |
| **337333** | 247 | 32 | 43 | 44 | 50 | 53 | 60 |
| **345778** | 326 | 36 | 44 | 102 | 47 | 53 | 106 |
| **345781** | 329 | 32 | 223 | 204 | 43 | 170 | 211 |
| **345794** | 342 | 30 | 34 | 48 | 40 | 40 | 63 |
| **345815** | 363 | 31 | 48 | 85 | 45 | 47 | 81 |
| **345823** | 370 | 33 | 37 | 44 | 44 | 44 | 57 |

After 4 weeks of treatment, the animals were sacrificed and RNA was prepared from liver tissue. STAT3 mRNA expression was measured by real-time PCR as described herein. The data are shown in Table 50 as percent inhibition of mRNA expression relative to saline-treated control mice.

Table 50

| STAT3 Target Reduction in Liver | | |
|---|---|---|
| Treatment | SEQ ID NO | % Inhibition |
| **Control** | | 0 |
| **113196** | 135 | 45 |
| **337332** | 246 | 75 |
| **337333** | 247 | 45 |
| **345778** | 326 | 45 |
| **345781** | 329 | 20 |
| **345794** | 342 | 55 |
| **345815** | 363 | 0 |
| **345823** | 370 | 35 |

As shown in Table 50, treatment with ISIS 113916, ISIS 337332, ISIS 337333, ISIS 345778, ISIS 345794, and ISIS 345823 caused a reduction in STAT3 mRNA in liver.

Liver, spleen, fat pad and kidney weights were measured at the end of the study. The average liver weight for each treatment group is given in Table 51.

Table 51

| Effect of STAT3 Antisense Oligonucleotides on Liver Weight | | |
|---|---|---|
| Treatment | SEQ ID NO | Weight (g) |
| **Saline** | n/a | 1.3 |
| **113196** | 135 | 1.7 |
| **337332** | 246 | 1.5 |
| **337333** | 247 | 1.5 |

(continued)

| Effect of STAT3 Antisense Oligonucleotides on Liver Weight | | |
|---|---|---|
| **Treatment** | **SEQ ID NO** | **Weight (g)** |
| **345778** | 326 | 1.8 |
| **345781** | 329 | 1.4 |
| **345794** | 342 | 1.7 |
| **345815** | 363 | 1.5 |
| **345823** | 370 | 1.5 |

Fat pad weights for saline-treated animals averaged about 0.33 g. The average fat pad weights for the ISIS 337333, ISIS 345778, ISIS 345815, ISIS 345794, or ISIS 345823 treatment groups ranged from about 0.29 g to 0.36 g, and thus were not significantly different as compared to those measured for the saline treatment group. The average fat pad weights for animals treated with ISIS 337333, ISIS 345781, and ISIS 113196 were slightly lower than saline-treated controls at about 0.23 g, 0.22 g, and 0.26 g, respectively.

Measurement of spleen weights of animals treated with ISIS 337332, ISIS 337333, ISIS 345778, and ISIS 113196 resulted in averages between about 0.09 and 0.1 g, which did not differ from the average for saline-treated animals (about 0.09 g). The average spleen weight for animals treated with ISIS 345815, ISIS 345794, or ISIS 345823 was about 0.08, 0.07, and 0.08, respectively. Thus, treatment with antisense oligonucleotides targeting STAT3 did not cause an increase in spleen weights of normal mice.

The average kidney weights measured for the treatment groups ranged from about 0.29 g to about 0.34 g. As compared to the average kidney weight measured for saline-treated controls (about 0.36 g), these data indicate that treatment with antisense oligonucleotides targeted to STAT3 did not cause an increase in kidney weight in normal mice.

Body weights were measured at weeks 0, 2 and 4. The average body weights measured for the saline-treated control animals ranged from about 24 g to 27 g, and animals showed weight gain over time. Animals treated with antisense oligonucleotides targeted to STAT3 showed similar increases in weight with time, and their average weights did not vary significantly from those of control animals.

The treatment of normal mice with antisense oligonucleotides targeted to human STAT3 did not result in significant adverse affects.

**Example 30**

**Evaluation of antisense oligonucleotides targeted to STAT3 as a treatment for cancer**

The inappropriate expression and/or activation of STAT3 is associated with a variety of cancers, including prostate cancer, squamous cell carcinomas of the head and neck, non-small cell lung carcinoma and hepatocellular carcinoma, multiple myeloma, anaplastic large cell lymphoma, chronic lymphocytic leukemia, acute myeloid leukemia and non-hodgkin's lymphoma. Thus, antisense oligonucleotides targeted to STAT3 are evaluated in patients with solid tumors or hematological malignancies. Treatment groups are approximately 30 to 40 patients. During a loading period, antisense oligonucleotides are administered intravenously three times in the first week, after which antisense oligonucleotides are administered weekly. Doses range from 2 mg/kg to 10 mg/kg. The effects of antisense oligonucleotide treatment are evaluated by assessing the response to treatment and by measuring biomarkers. Examples of biological markers in multiple myeloma patients include serum paraprotein and apoptosis in circulating multiple myeloma cells. STAT3 protein and phosphorylation are also measured in circulating myeloma cells.

Antisense oligonucleotides targeted to STAT3 are also tested in patients who are refractory to chemotherapeutic agents or who are experiencing a relapse of a cancer.

Antisense oligonucleotides targeted to STAT3 are also administered alone or in combination with other agents, for example, the chemotherapeutic agent Velcade®.

**Example 31**

**Apoptosis in a lung carcinoma cell line following antisense inhibition of human STAT3**

The cytokine IL6 is a known upstream regulator of STAT function and is also upregulated in lung cancer cells. As STAT3 is frequently implicated in tumor formation and survival in epithelial cells, it was of interest to delineate the

role of STAT3 in human nonsmall cell lung carcinoma cells. To this end, antisense inhibition of STAT3 was tested for its effects on cell growth and induction of apoptosis. The assays were performed as described (Song et al., Oncogene, 2003, 22, 4150-4165)

A549, H460, H358 and H 1299 cell lines, all of which can be obtained from ATCC (Manassas, VA), were grown in RPMI media supplemented with 5% fetal bovine serum, 2 mM glutamine and 1% penicillin/streptomycin (Invitrogen Life Technologies, Carlsbad, CA). A549, H358 and H1299 cells exhibit constitutively activated STAT3.

Oligonucleotides were transfected into human lung cancer cells using either encapsulated liposomes or electroporation. A549, H358 andH1299 cells (100,000-200,000 cells in a 60-mm dish) were transfected with 500 nM of oligonucleotides with 15 mg of LIPOFECTIN™ (Invitrogen Life Technologies, Carlsbad, CA) in 1.5 ml of OptiMEM™ media (Invitrogen Life Technologies, Carlsbad, CA). Cells were incubated with the oligonucleotides for 4 hours, the mixture was removed and the cells were washed once with RPMI plus 5% fetal bovine serum and then incubated in culture medium. For immunoblots, electrophoretic mobility shift assays and microscopy, cells were harvested 24 hours after transfection. For Apo-BRDU™ assays, cells were harvested at 48 hours post-transfection.

For electroporation of H460 cells, cells were grown to approximately 20-30 X $10^6$ in number, tyrpsinized, and incubated with oligonucleotides at a final concentration of 10 $\mu$m. Cells were electroporated in a Biorad electroporator (Bio-Rad Laboratories, Hercules, CA) at 1000 $\mu$F and 230V in OPTI-MEM, Eagle's media in a total volume of 400 mL. Cells were subsequently replated in 10 cm dishes in RPMI with 5% FBS and 1% penicillin/streptomycin and placed in a 5% $CO_2$ and 37°C incubator overnight. The next morning the cells were washed with PBS and new media with serum and antibiotics was added.

Apoptosis was assayed using an Apo-BRDU™ kit (Pharmingen, BD Biosciences, San Diego, CA) according to the manufacturer's instructions. Data from 10,000 cells per experimental condition were acquired and analysed for fluorescence on a FACScan™ Immunocytometry System using CELLQuest™ software (BD Biosciences, San Jose, CA). Apoptosis was also detected by measuring the cleavage of poly-(ADP-ribose) polymerase (PARP), which is indicative of apoptosis, using PARP antibody (Cell Signaling, Beverly, MA) to detect full-length PARP as well as the cleaved large fragment.

STAT DNA-binding assays were performed as described previously (Yu et al., Science, 1995, 269, 81-83; Garcia et al., Cell Growth Differ., 1997, 8, 1267-1276). Protein-DNA complexes were resolved by nondenaturing polyacrylamide gel electrophoresis (PAGE) and detected by autoradiography. Anti-STAT3 (C-20) polyclonal antibodies used to identify specific STAT3 complexes by electrophoretic mobility shift assay (EMSA) were obtained from Santa Cruz Biotechnologies. For use in supershift assays, 1 g of the C-20 antibody was incubated with nuclear extracts for 20 min at room temperature prior to addition of radiolabeled probe(s) and electrophoresis.

Western blots were performed as follows. Cell lysates were normalized for total protein content (50 mg) and subjected to SDS-PAGE. Primary antibodies used in these studies consisted of STAT3 (Transduction Laboratories, Lexington, KY), phosphorylated STAT3 Y705 (Cell Signaling, Beverly, MA) PARP (Cell Signaling, Beverly, MA), and beta-actin (Sigma, St. Louis, MO). Detection of proteins was accomplished using horseradish-peroxidase-conjugated secondary antibodies and enhanced chemiluminescence (ECL) kit from Amersham Biosciences (Piscataway, NJ).

A549 cells were transfected with no oligonucleotides, antisense, or mismatch oligonucleotides and 24 h later cells were collected and total protein and nuclear extracts were prepared. Western blotting revealed a reduction in the total STAT3 protein content of cells by ISIS 113176, while no reduction was seen in cells treated the mismatched control oligonucleotide or in untreated cells. The reduction in STAT3 levels was accompanied by the activation of apoptosis as evidenced by cleavage of (PARP) as early as 24 hours after transfection, while no cleavage of PARP was seen in mismatch control-treated or untreated cells. As assayed by EMSA, antisense inhibiton of STAT3 resulted in nearly complete inhibition of STAT3 DNA-binding activity, while mismatch oligonucleotide treatment had no affect on STAT3 DNA-binding activity. Photomicroscopic examination of cells treated with ISIS 113176 for 48 hours revealed reduced numbers and appearance of altered cell size and shape with accompanying increase in cellular debris and vacuolated cells. Conversely, cells treated the mismatch control oligonucleotide appeared to have no change in cellular number, size, shape, nor had increased cellular debris suggesting cell death. To provide additional evidence to support the role of STAT3 in controlling apoptosis in A549 cells, the cells were collected and assayed for DNA fragmentation by incorporation of Apo-BrdU. Results from three independent experiments were pooled and percent apoptosis determined by the percentage of cells incorporating Apo-BrdU. Cells treated with antisense STAT3 oligonuclotides had increased Apo-BrdU incorporation, approximately 16 times that of mismatch-control treated cells and approximately 48 times that of untreated control cells, results which are indicative of apoptosis. Untreated and mismatch control-treated cells had minimal evidence of nicked DNA. Therefore, antisense inhibition of STAT3 induced apoptosis, as indicated by PARP cleavage and DNA fragmentation, in A549 lung carcinoma cells.

To extend the findings with A549 cells, similar experiments were performed with H358 and H1299 lung carcinoma cells. Antisense inhibition of STAT3 resulted in loss of STAT3 protein in both H358 and H1299 cells compared to treatment with the mismatch control oligonucleotide. Activation of apoptotic pathways was demonstrated in H358 cells, evidenced by PARP cleavage in cells treated with ISIS 113176, while no evidence of apoptosis was seen in H1299 cells, despite

absent STAT3 protein.

The effect of STAT3 antisense oligonucleotide treatment was also assessed in H460 lung carcinoma cells that have minimal STAT3 DNA-binding activity. H460 cells were transfected with up to 10 m of ISIS 113176 or ISIS 129987 by electroporation. STAT3 activity as measured by EMSA showed minimal DNA-binding activity, which was reduced by STAT3 antisense inhibition but not by mismatch control oligonucleotide treatment. No effects on cellular morphology or cell number were evident, apoptosis was not increased (0.53% and 0.27% apoptotic cells following treatment with ISIS 129987 and ISIS 113176, respectively) and no increased incorporation of Apo-BrdU was observed. These data support the conclusion that antisense inhibition of STAT3 is specific and does not induce cell death in H460 cells, which have minimal STAT3 activity.

From these data, it is evident that antisense inhibition of STAT3 resulted in apoptosis in A549 and H358 cells, while no observation of apoptosis was seen in H1299 or H460 cells. These findings demonstrate that direct inhibition of STAT3 leads to apoptotic cell death and suggests that STAT3 controls apoptotic pathways in certain human lung cancer cells.

**Example 32**

**Antisense inhibition of human STAT3 in prostate cancer cells**

The pathogenesis of multiple myeloma involves tha activation of STAT3 by the cytokine IL-6, which prevents apoptosis. Owing to the association of cytokines, including IL-6, with prostate cancer, the function of STAT3 was investigated in prostate cancer cels. In a further embodiment, DU145 cells subjected to antisense inhibition of STAT3 were evaluated for STAT3 activity, apoptosis and proliferation. The procedures using in these assays are described in detail by Mora, et al. (Cancer Research, 2002, 62, 6659-6666).

DU145 prostate cancer cells were obtained from ATCC (Manassas, VA) and were cultured in RPMI medium supplemented with 10% fetal bovine serum. Cells were treated with 250 nM of ISIS 113176 (SEQ ID NO: 115) or ISIS 129987 (GCTCCAATACCCGTTGCTTC, SEQ ID NO: 404), a mismatched control oligonucleotide that does not target STAT3. Cells were seeded at a density of 2 X $10^6$ cells in a 10 cm plate 18 hours prior to transfection. Cells were transfected for 3 hours with Lipofectamine™ Plus (Invitrogen Life Technologies, Carlsbad, CA) alone, with Lipofectamine™ Plus and ISIS 113176 or with Lipofectamine™ Plus with ISIS 129987. Untreated cells served as a control.

After 24 hours of treatment, cells were harvested for protein isolation and nuclear extract preparation. The DNA-binding activity of STAT3 was evaluated by electrophoretic mobility shift assay (EMSA) in nuclear extracts. 5 ug of total cellular protein was incubated with a $^{32}P$-labeled oligonucleotide probe that binds to activated STAT3 proteins. Protein-DNA complexes were resolved by nondenaturing polyacrylamide electrophoresis and detected by autoradiography. STAT3 protein levels were evaluated by western blot, using an antibody from Santa Cruz Biotechnology (Santa Cruz, CA). Transfected cells were also collected and fixed in 95% ethanol for immunohistochemical staining of phosphorylated STAT3, using a rabbit human polyclonal antibody (Phospho-Tyr705-Stat3; Cell Signaling, Beverly, MA).

EMSA revealed greatly decreased STAT3 DNA-binding activity in the DU145 cells transfected with ISIS 113176, as compared to control cells (mismatched control oligonucleotide, Lipofectaminern Plus alone or no treatment). An antibody to STAT3 blocked and supershifted this DNA-binding activity, confirming that the activity corresponds to STAT3. STAT3 protein expression was greatly reduced in DU145 cells transfected with ISIS 113176, as compared to control cells. Furthermore, staining of phosphorylated STAT3 was abolished in transfected DU145 cells. These data demonstrate that antisense inhibition of STAT3 resulted in a reduction in STAT3 protein levels, including phosphorylated STAT3, and STAT3 DNA-binding activity.

Apoptosis in control and transfected DU145 cells was evaluated by measuring caspase-3 levels, using an antibody specific for activated caspase-3, according to the supplier's protocol (Becton Dickinson PharMingen, San Diego, CA). Staining was analyzed by flow cytometry. Cell proliferation was measured by counting the number of viable cells using a hemocytometer and trypan blue dye exclusion. Activated caspase-3 was approximately 5%, 7% and 18% of untreated controls in cells receiving Lipofectamine™ Plus alone, ISIS 129987 or ISIS 113176, respectively, demonstrating an increase in apoptosis following antisense inhibition of STAT3. Cell proliferation was also suppressed over a 48 hour period and did not rise above 3 X $10^6$ cells in ISIS 113176-treated samples. Cell number in mismatched control and Lipofectamine-Plus treated cells increased over the same 48 hour period, to approximately 5 X $10^6$ and 9 X $10^6$ cells, respectively.

**Example 33**

**Chimeric phosphorothioate oligonucleotides targeted to mouse STAT3 having 2'-MOE wings and a deoxy gap**

In accordance with the present disclosure, an additional series of oligonucleotides was designed to target

different regions of the mouse STAT 3, using published sequences (GenBank® accession number U06922, incorporated herein as SEQ ID NO: 82, the complement of nucleotides 12369484 to 12421464 of GenBank® accession number NT_039521.2, incorporated herein as SEQ ID NO: 461; GenBank® accession number NM_213659.1, incorporated herein as SEQ ID NO: 462; and GenBank® accession number NM_011486.3, incorporated herein as SEQ ID NO: 463). The oligonucleotides are shown in Table 52. "Target site" indicates the first (5'-most) nucleotide number on the particular target sequence to which the oligonucleotide binds. All compounds in Table 52 are chimeric oligonucleotides ("gapmers") 20 nucleotides in length, composed of a central "gap" region consisting of ten 2'-deoxynucleotides, which is flanked on both sides (5' and 3' directions) by five-nucleotide "wings". The wings are composed of 2'-O-methoxyethyl (2'-MOE)nucleotides. The internucleoside (backbone) linkages are phosphorothioate (P=S) throughout the oligonucleotide. All cytidine residues are 5-methylcytidines.

The compounds were analyzed for their effect on mouse STAT3 mRNA levels by quantitative real-time PCR as described in other examples herein. Data are averages from two experiments in which b.END cells were treated with 75 nM of the antisense oligonucleotides.

The mouse brain endothelial cell line b.END was obtained from Dr. Werner Risau at the Max Plank Institute (Bad Nauheim, Germany). b.END cells were routinely cultured in DMEM, high glucose (Invitrogen Life Technologies, Carlsbad, CA) supplemented with 10% fetal bovine serum (Invitrogen Life Technologies, Carlsbad, CA). Cells were routinely passaged by trypsinization and dilution when they reached approximately 90% confluence. Cells were seeded into 96-well plates (Falcon-Primaria #3872, BD Biosciences, Bedford, MA) at a density of approximately 3000 cells/well for use in oligomeric compound transfection experiments.

Probes and primers to mouse STAT3 were designed to hybridize to a mouse STAT3 sequence, using published sequence information (GenBank® accession number NMR_213659.1, incorporated herein as SEQ ID NO: 462). For mouse STAT3 the PCR primers were:
forward primer: GCCACGTTGGTGTTTCATAATCT (SEQ ID NO: 464) reverse primer: GATAGAGGACATTGGACTCTTGCA (SEQ ID NO: 465) and the PCR probe was: FAM-TTGGGTGAAATTGACCAGCAATATAGCCG-TAMRA (SEQ ID NO: 466) where FAM is the fluorescent dye and TAMRA is the quencher dye.

**Table 52**

| Chimeric phosphorothioate oligonucleotides targeted to mouse STAT3 having 2'-MOE wings and a deoxy gap | | | | | | |
|---|---|---|---|---|---|---|
| **Isis #** | **Region** | **Target SEQ ID NO** | **Target Site** | **Sequence** | **% Inhib** | **Seq ID No** |
| 106747 | Coding | 82 | 2017 | GCCCATGATGATTTCAGCAA | 70 | 58 |
| 133003 | 3' UTR | 82 | 2527 | AAAAAGTGCCCAGATTGCCC | 76 | 99 |
| 337269 | Coding | 82 | 339 | TTCTCAAGATACCTGCTCTG | 41 | 183 |
| 337270 | Coding | 82 | 344 | TTGGCTTCTCAAGATACCTG | 76 | 184 |
| 337287 | Coding | 82 | 627 | TGGTTGTTTCCATTCAGATC | 60 | 201 |
| 337289 | Coding | 82 | 660 | TCCAGCTGCTGCATCTTCTG | 65 | 203 |
| 337294 | Coding | 82 | 783 | CTCTTCCAGTCAGCCAGCTC | 51 | 208 |
| 337298 | Coding | 82 | 873 | AGTTGAGATTCTGCTAATGA | 35 | 212 |
| 337299 | Coding | 82 | 878 | TCTGAAGTTGAGATTCTGCT | 69 | 213 |
| 337302 | Coding | 82 | 1110 | TTGACCAGCAACCTGACTTT | 30 | 216 |
| 337303 | Coding | 82 | 1134 | AGCTGATAATTCAACTCAGG | 76 | 217 |
| 337304 | Coding | 82 | 1139 | TTTTAAGCTGATAATTCAAC | 1 | 218 |
| 337317 | Coding | 82 | 1412 | CTAGGTCAATCTTGAGGCCT | 50 | 231 |
| 337319 | Coding | 82 | 1425 | AAGGAGTGGGTCTCTAGGTC | 9 | 233 |
| 337320 | Coding | 82 | 1430 | CTGGCAAGGAGTGGGTCTCT | 55 | 234 |
| 337322 | Coding | 82 | 1457 | TCTGACAGATGTTGGAGATC | 32 | 236 |
| 337323 | Coding | 82 | 1462 | TGGCATCTGACAGATGTTGG | 54 | 237 |
| 337328 | Coding | 82 | 1686 | CATGTGATCTGACACCCTGA | 51 | 242 |

(continued)

| Chimeric phosphorothioate oligonucleotides targeted to mouse STAT3 having 2'-MOE wings and a deoxy gap | | | | | | |
|---|---|---|---|---|---|---|
| Isis # | Region | Target SEQ ID NO | Target Site | Sequence | % Inhib | Seq ID No |
| 337329 | Coding | 82 | 1691 | TAGCCCATGTGATCTGACAC | 52 | 243 |
| 337331 | Coding | 82 | 1721 | CCTTGCCAGCCATGTTTTCT | 57 | 245 |
| 337332 | Coding | 82 | 1726 | GAAGCCCTTGCCAGCCATGT | 77 | 246 |
| 337334 | Coding | 82 | 1736 | CCCAGAAGGAGAAGCCCTTG | 47 | 248 |
| 337337 | Coding | 82 | 1992 | ATGTTGTTCAGCTGCTGCTT | 62 | 251 |
| 337338 | Coding | 82 | 1997 | ATGACATGTTGTTCAGCTGC | 77 | 252 |
| 337339 | Coding | 82 | 2002 | AGCAAATGACATGTTGTTCA | 53 | 253 |
| 337340 | Coding | 82 | 2007 | ATTTCAGCAAATGACATGTT | 44 | 254 |
| 337341 | Coding | 82 | 2012 | TGATGATTTCAGCAAATGAC | 42 | 255 |
| 337342 | Coding | 82 | 2022 | TTATAGCCCATGATGATTTC | 10 | 256 |
| 337343 | Coding | 82 | 2027 | TGATCTTATAGCCCATGATG | 70 | 257 |
| 337344 | Coding | 82 | 2032 | ATCCATGATCTTATAGCCCA | 70 | 258 |
| 337375 | 3' UTR | 82 | 2704 | ACCAGGAGGCACTTGTCTAA | 59 | 89 |
| 367289 | Intron | 461 | 3664 | CTTAATTCTTAGAGATCCAC | 66 | 467 |
| 367290 | Intron 1 | 461 | 13469 | CACACTAATGCAAGATGCTA | 51 | 468 |
| 367291 | Intron | 461 | 24761 | AAAAAGTGATCTACCAACAG | 25 | 469 |
| 367292 | Exon 8: Intron 8 | 461 | 34889 | CAACTCTTACCAGTTTTCCA | 0 | 470 |
| 367293 | Coding | 82 | 2253 | ATCAATGAATCTAAAGTGCG | 18 | 471 |
| 367294 | Start Codon | 462 | 147 | CACTGAGCCATCCTGCTGCA | 50 | 472 |
| 367295 | Start Codon | 462 | 156 | AGCTGGTTCCACTGAGCCAT | 60 | 473 |
| 367296 | Coding | 462 | 380 | TGTGCTGATAGAGGACATTG | 70 | 474 |
| 367297 | Coding | 462 | 475 | AGACTCTTCCCACAGGCATC | 63 | 475 |
| 367298 | Coding | 462 | 864 | CAGTCAGCCAGCTCTTCATC | 52 | 476 |
| 367299 | Coding | 462 | 940 | AGTTATCCAGTTTTCCAGAC | 40 | 477 |
| 367300 | Coding | 462 | 990 | TCCAGTTTCTTAATTTGTTG | 44 | 478 |
| 367301 | Coding | 462 | 1016 | TGTAGGACACTTTCTGCTGC | 62 | 479 |
| 367302 | Coding | 462 | 1169 | ACTGGACACCAGTCTTGATG | 14 | 480 |
| 367303 | Coding | 462 | 1231 | TTTAATTTTAAGCTGATAAT | 0 | 481 |
| 367304 | Coding | 462 | 1236 | CACACTTTAATTTTAAGCTG | 40 | 482 |
| 367305 | Coding | 462 | 1241 | CAATGCACACTTTAATTTTA | 15 | 483 |
| 367306 | Coding | 462 | 1300 | GCCCAGAATGTTAAATTTCC | 54 | 484 |
| 367307 | Coding | 462 | 1379 | CCCTAAGGGTCAGGTGCTTG | 69 | 485 |
| 367308 | Coding | 462 | 1398 | CCATTCCCACATCTCTGCTC | 55 | 486 |
| 367309 | Coding | 462 | 1458 | AAGGTGATCAGGTGCAGCTC | 44 | 487 |
| 367310 | Coding | 462 | 1505 | GGGTCTCTAGGTCAATCTTG | 46 | 488 |

(continued)

| Chimeric phosphorothioate oligonucleotides targeted to mouse STAT3 having 2'-MOE wings and a deoxy gap | | | | | | |
|---|---|---|---|---|---|---|
| **Isis #** | **Region** | **Target SEQ ID NO** | **Target Site** | **Sequence** | **% Inhib** | **Seq ID No** |
| 367311 | Coding | 462 | 1525 | CACCACAACTGGCAAGGAGT | 75 | 489 |
| 367312 | Coding | 462 | 1588 | GGTCAGCATGTTATACCACA | 69 | 490 |
| 367313 | Coding | 462 | 1647 | ACTTGGTCCCAGGTTCCAAT | 54 | 491 |
| 367314 | Coding | 462 | 1763 | GACACCCTGAGTAGTTCACA | 58 | 492 |
| 367315 | Coding | 462 | 1803 | CCAGCCATGTTTTCTTTGCA | 50 | 493 |
| 367316 | Coding | 462 | 1842 | ATGATATTGTCTAGCCAGAC | 49 | 494 |
| 367317 | Coding | 462 | 1868 | CCAAGATATACTTTTTCACA | 30 | 495 |
| 367318 | Coding | 462 | 1883 | CTTCATTCCAAAGGGCCAAG | 23 | 496 |
| 367319 | Coding | 462 | 2005 | CCAAGTGAAAGTGACCCCTC | 25 | 497 |
| 367320 | Coding | 462 | 2023 | ACTGATGTCCTTTTCCACCC | 50 | 498 |
| 367321 | Coding | 462 | 2033 | GGGTCTTGCCACTGATGTCC | 50 | 499 |
| 367322 | Coding | 462 | 2043 | GACTGGATCTGGGTCTTGCC | 67 | 500 |
| 367323 | Coding | 462 | 2273 | CACAGATGAACTTGGTCTTC | 32 | 501 |
| 367324 | Coding | 462 | 2283 | GTTGGTGTCACACAGATGAA | 35 | 502 |
| 367325 | Coding | 462 | 2303 | CAATGGTATTGCTGCAGGTC | 50 | 503 |
| 367326 | Coding | 462 | 2308 | CAGGTCAATGGTATTGCTGC | 64 | 504 |
| 367327 | Coding | 462 | 2341 | CATCAATGAATCTAAAGTGC | 72 | 505 |
| 367328 | Coding | 462 | 2346 | AACTGCATCAATGAATCTAA | 50 | 506 |
| 367329 | Coding | 462 | 2351 | TTCCAAACTGCATCAATGAA | 40 | 507 |
| 367330 | Coding | 462 | 2356 | GTTATTTCCAAACTGCATCA | 46 | 508 |
| 367331 | Coding | 462 | 2423 | AGGTCAGATCCATGTCAAAC | 61 | 509 |
| 367332 | 3' UTR | 462 | 2893 | AGTCCTAGCTTCTATGTGGA | 70 | 510 |
| 367333 | 3' UTR | 463 | 2307 | ATCAATGAATGGTGTCACAC | 0 | 511 |

**Example 34**

**Antisense inhibition of STAT3 leads to cell death and tumor growth inhibition in a model of multiple myeloma**

In a further embodiment, the requirement for STAT3 in the development of multiple myeloma and in the growth of multiple myeloma-derived tumors was investigated. Transgenic NPM-ALK (NPM-ALK Tg) mice spontaneously develop multiple myeloma characterized by constitutively activated STAT3. To determine whether STAT3 is required for the development of multiple myeloma, STAT3 was genetically disrupted in the B-cells of NPM-ALK Tg mice. The mouse STAT3 locus was engineered to contain Cre recombinase recognition sites (loxP sites), as previously described (Raz et al., PNAS, 1999, 96, 2846-2851). When mice bearing this modified STAT3 locus are crossed with mice harboring a Cre recombinase transgene, the Cre recombinase excises the portion of the locus flanked by the loxP sites, resulting in a deleted STAT3 locus. NPM-ALK Tg mice were bred to introduce the conditionally disruptive STAT3 gene. These mice were then crossed to mice carrying a CD19+-specific Cre transgene, which express Cre under the control of the B lineage-restricted CD19 gene (Rickert et al., Nuc. Acids Res., 1997, 25, 1317). The resultant offspring harbor a B-cell specific deletion of the STAT3 gene. B-cells can be identified by the CD19 antigen, and mature into the plasma cells responsible for multiple myeloma, which are identified by the presence of both the CD19 and CD138 antigens.

Mice were crossed as described and genotyping of the offspring revealed that greater than 80% of all CD19+ or CD138+ cells (identified as previously described, Chiarle et al., Blood, 2003, 101, 1919-1927) underwent STAT3

deletion. Moreover, in 23 NPM-ALK Tg STAT3-deficient animals examined, no STAT3-negative multiple myeloma cells were detected. Nuclear extracts were prepared from these cells as previously described (Mora et al., Cancer Res. 62:6659, 2002). Detection of phosphorylated STAT3 in nuclear extracts by western blotting as described herein revealed that all CD138+ multiple myeloma cells in these mice contained detectable nuclear phosphorylated STAT3. As determined by southern blotting (as described in Raz et al., PNAS, 1999, 96, 2846-2851), the STAT3 gene in these cells was intact. These data demonstrate that STAT3 is absolutely required for the transformation of plasma cells.

The STAT3-positive CD19+ cells resulted in the development of multiple myeloma, and the multiple myeloma cells were transplanted into recipient mice. The effects of STAT3 antisense inhibition were tested in the resultant tumors. Multiple myeloma cells were injected subcutaneously ($2 X 10^6$ cells) or intravenously ($1 X 10^7$ cells) into nude or syngeneic mice (Balb/c mice). Animals with measurable multiple myeloma tumors (approximately 0.5 $mm^3$) were injected daily with ISIS 17152 (SEQ ID NO: 99) or the control oligonucleotide ISIS 28084 (SEQ ID NO: 151) at a dose of 20 mg/kg for 14 days. Injections were administered subcutaneously, counterlaterally to the tumors. Each treatment group included from 3 to 9 animals.

After 15 days of oligonucleotide treatment, tumor growth in ISIS 17152-injected animals was reduced to 15% of that in control oligonucleotide-injected animals. As judged by routine histological analysis, ISIS 17152-treated tumors displayed large necrotic areas throughout, as compared to control oligonucleotide-treated tumors, which showed only single apoptotic figures. Additionally, serum IgM levels, which are increased in multiple myeloma, were measured by ELISA using a commercially available kit (e.g., OPT-EIA™, BD Biosciences Pharmingen, San Diego, CA) and were found to be decreased in ISIS 17152-treated animals, compared to control oligonucleotide-treated animals. Furthermore, the overall survival rate of animals receiving ISIS 17152 treatment was significantly increased, relative to control animals.

A similar experiment was performed in mice in which 1 $cm^3$ tumors had developed, to test the effects of antisense inhibition of STAT3 on well-established tumors. Mice were treated daily for 14 days with 40 mg/kg ISIS 17152 or the control oligonucleotide. In this experiment, tumor necrosis and tumor cell cycle arrest were observed as a result of antisense inhibition of STAT3.

Apoptosis in tumors was evaluated by Annexin-V staining, using an Annexin-V staining kit (Clontech, Palo Alto, CA) according to the manufacturer's instructions. Briefly, the cells were resuspended in 0.2 mL of staining buffer (10 mM HEPES, pH 7.4, 140 mM NaCl, 5 mM $CaCl_2$) and 10 $\mu$L of propidium iodide (50 $\mu$g/ml) and 5 $\mu$L of Annexin-V reagent were added at 4°C for 10 minutes. The samples were then diluted with FacsFlow buffer and analyzed on a Becton Dickinson FACScan™ (Mountain View, CA). In tumor cells of mice receiving the antisense oligonucleotide targeted to STAT3, an approximately 2-fold increase in Annexin-V positive tumor cells was observed, as compared to the control. Apoptosis was also evaluated by TUNEL staining, performed using a commercially available kit (e.g., APO-DIRECT™, Pharmingen, BD Biosciences, San Diego, CA) according to the manufacturer's instructions. In tumor cells of mice treated with ISIS 17152, large areas of TUNEL-positive cells were present, in contrast to the control oligonucleotide treated tumors. These data demonstrate that antisense inhibition caused apoptosis in transplanted multiple myeloma tumors.

Cell cyle arrest was evaluated by flow cytometry. Cells were prepared as described herein. Cells from ISIS 17152-treated tumors (20 mg/kg dose) exhibited a 45% reduction in the S-phase population, compared to cells from control-treated tumors. Thus, antisense inhibition of STAT3 led to an arrest in the G1 phase of the cell cycle.

Primary multiple myeloma tumors and transplanted tumors were evaluated by western blot for the expression of STAT3, phosphorylated STAT3, Bcl-xL, survivin, ERK, phosphorylated ERK and c-myc. Protein lysates were prepared and subjected to western blot analysis, using commercially available antibodies (STAT3 and phosphorylated STAT3 antibodies obtained as described herein; other antibodies are available from Santa Cruz Biotechnology, Santa Cruz, CA). In both primary and transplanted multiple myeloma tumors, high levels of all of these proteins were observed, indicating that the transplanted tumor cells exhibit characteristics similar to the primary tumor cells.

Combination treatments were also tested in the mouse model of multiple myeloma. Animals bearing tumors originating from transplanted multiple myeloma cells were treated with ISIS 17152 in combination with bortezomib (known commercially as Velcade®, Millenium Pharmaceuticals, Cambridge, MA) or cyclophosphamide (known commercially as Endoxana®, AstaMedica, Frankfurt, Germany). One treatment group, consisting of at least 10 animals, receives a daily subcutaneous dose of ISIS 17152 at 20 mg/kg, in combination with bortezmib, for 14 days. Bortezemib is administered intravenously at doses ranging from 0.05 mg/kg to 1.0 mg/kg, twice weekly for the 14 day study period. An additional treatment group, consisting of at least 10 animals, receives a daily, subcutaneous dose of ISIS 17152 at 20 mg/kg, in combination with cyclophosphamide. Cyclophosphamide is administered intraperitoneally at a dose of 35 mg/kg, twice weekly for the 14 day study period. In the combination treatment groups, the following endpoints are evaluated: tumor growth, survival rate, serum IgM levels, apoptosis, cell cycle arrest, STAT3 and phosphorylated STAT3 protein levels, and tumor necrosis.

These data demonstrate that antisense inhibition of STAT3 led to G1 phase arrest, cell death and tumor growth inhibition in vivo. Thus, antisense oligonucleotides target to STAT3 are therapeutic agents with applications in the treatment of multiple myeloma.

## Claims

1. An antisense oligonucleotide 12 to 50 nucleobases in length, which specifically hybridises with a nucleic acid molecule encoding STAT3 and is capable of inhibiting STAT3 expression, and wherein the oligonucleotide is targeted to nucleotides 2983 to 3127 of SEQ ID NO:154.

2. The antisense oligonucleotide of claim 1, wherein the nucleic acid molecule encodes human STAT3.

3. The antisense oligonucleotide of any preceding claim, wherein the oligonucleotide is 100% complementary to the nucleic acid molecule encoding STAT3.

4. The antisense oligonucleotide of any preceding claim, which is:

   13 to 40 nucleobases in length; or
   15 to 30 nucleobases in length.

5. The antisense oligonucleotide of any preceding claim, wherein the antisense oligonucleotide comprises at least one modified internucleoside linkage, such as a phosphorothioate linkage.

6. The antisense oligonucleotide of any preceding claim, wherein the antisense oligonucleotide comprises at least one substituted sugar moiety, such as a 2'-O-(2-methoxyethyl) moiety.

7. The antisense oligonucleotide of any preceding claim, wherein the antisense oligonucleotide comprises at least one modified nucleobase, such as a 5-methyl cytosine.

8. The antisense oligonucleotide of any preceding claim which is a chimeric oligonucleotide.

9. The antisense oligonucleotide of claim 8, which is a gapmer.

10. The antisense oligonucleotide of claim 8 or 9 which is a chimeric oligonucleotide having a first region comprising deoxynucleotides and second and third regions flanking said first region, comprising at least one 2'-O-(2-methoxyethyl) nucleotide.

11. The antisense oligonucleotide of claim 10, wherein said first region is ten deoxynucleotides in length and said second and third regions are each five nucleotides in length.

12. A pharmaceutical composition comprising the antisense oligonucleotide of any preceding claim and a pharmaceutically acceptable carrier or diluent.

13. The antisense oligonucleotide of any of claims 1 to 11 for use in therapy or prophylaxis.

14. The antisense oligonucleotide of claim 13 for use in the treatment or prevention of abnormal inflammatory states or inflammatory diseases, such as rheumatoid arthritis.

15. The antisense oligonucleotide of claim 13 for use in the treatment or prevention of cancers, such as breast, prostrate, brain or head and neck cancer, melanomas, myelomas, leukemias or lymphomas.

## Patentansprüche

1. Antisense-Oligonukleotid mit einer Länge von 12 bis 50 Nukleobasen, das spezifisch mit einem STAT3 codierenden Nukleinsäuremolekül hybridisiert und zur Hemmung der STAT3-Expression fähig ist, und wobei das Oligonukleotid auf Nukleotid 2983 bis 3127 von SEQ ID NO:154 gerichtet ist.

2. Antisense-Oligonukleotid nach Anspruch 1, wobei das Nukleinsäuremolekül menschliches STAT3 codiert.

3. Antisense-Oligonukleotid nach einem vorhergehenden Anspruch, wobei das Oligonukleotid 100% komplementär zu dem STAT3 codierenden Nukleinsäuremolekül ist.

4. Antisense-Oligonukleotid nach einem vorhergehenden Anspruch, das eine Länge von:

   13 bis 40 Nukleobasen; oder
   15 bis 30 Nukleobasen
   aufweist.

5. Antisense-Oligonukleotid nach einem vorhergehenden Anspruch, wobei das Antisense-Oligonukleotid wenigstens eine modifizierte Internukleosid-Verknüpfung, wie etwa eine Phosphorthioat-Verknüpfung, umfasst.

6. Antisense-Oligonukleotid nach einem vorhergehenden Anspruch, wobei das Antisense-Oligonukleotid wenigstens eine substituierte Zuckergruppierung, wie etwa eine 2'-O-(2-Methoxyethyl)-Gruppierung, umfasst.

7. Antisense-Oligonukleotid nach einem vorhergehenden Anspruch, wobei das Antisense-Oligonukleotid wenigstens eine modifizierte Nukleobase, wie etwa ein 5-Methylcytosin, umfasst.

8. Antisense-Oligonukleotid nach einem vorhergehenden Anspruch, bei dem es sich um ein chimäres Oligonukleotid handelt.

9. Antisense-Oligonukleotid nach Anspruch 8, bei dem es sich um ein Gapmer handelt.

10. Antisense-Oligonukleotid nach Anspruch 8 oder 9, bei dem es sich um ein chimäres Oligonukleotid mit einem Desoxynukleotide umfassenden ersten Bereich und einem den ersten Bereich flankierenden zweiten und dritten Bereich, umfassend wenigstens ein 2'-O-(2-Methoxyethyl)-Nukleotid, handelt.

11. Antisense-Oligonukleotid nach Anspruch 10, wobei der erste Bereich eine Länge von zehn Desoxynukleotiden aufweist und der zweite und dritte Bereich jeweils eine Länge von fünf Nukleotiden aufweisen.

12. Pharmazeutische Zusammensetzung, umfassend das Antisense-Oligonukleotid nach einem vorhergehenden Anspruch und ein pharmazeutisch unbedenkliches Träger- oder Verdünnungsmittel.

13. Antisense-Oligonukleotid nach einem der Ansprüche 1 bis 11 zur Verwendung in der Therapie oder Prophylaxe.

14. Antisense-Oligonukleotid nach Anspruch 13 zur Verwendung bei der Behandlung oder Vorbeugung anomaler entzündlicher Zustände oder von Entzündungskrankheiten wie rheumatoider Arthritis.

15. Antisense-Oligonukleotid nach Anspruch 13 zur Verwendung bei der Behandlung oder Vorbeugung von Krebserkrankungen wie Brustkrebs, Prostatakrebs, Hirntumoren oder Krebs im Kopf- und Halsbereich, Melanomen, Myelomen, Leukämien oder Lymphomen.

## Revendications

1. Oligonucléotide antisens de 12 à 50 nucléobases de longueur, qui s'hybride spécifiquement avec une molécule d'acide nucléique codant pour STAT3 et est capable d'inhiber l'expression de STAT3, et l'oligonucléotide étant ciblé vers les nucléotides 2983 à 3127 de SEQ ID NO: 154.

2. Oligonucléotide antisens de la revendication 1, la molécule d'acide nucléique codant pour STAT3 humain.

**3.** Oligonucléotide antisens de l'une quelconque des revendications précédentes, l'oligonucléotide étant à 100 % complémentaire de la molécule d'acide nucléique codant pour STAT3.

**4.** Oligonucléotide antisens de l'une quelconque des revendications précédentes, qui est:

de 13 à 40 nucléobases de longueur ; ou
de 15 à 30 nucléobases de longueur.

**5.** Oligonucléotide antisens de l'une quelconque des revendications précédentes, l'oligonucléotide antisens comprenant au moins un lieur internucléosidique modifié, tel qu'un lieur phosphorothioate.

**6.** Oligonucléotide antisens de l'une quelconque des revendications précédentes, l'oligonucléotide antisens comprenant au moins un fragment glucidique substitué, tel qu'un fragment 2'-O-(2-méthoxyéthyle).

**7.** Oligonucléotide antisens de l'une quelconque des revendications précédentes, l'oligonucléotide antisens comprenant au moins une nucléobase modifiée, telle qu'une 5-méthylcytosine.

**8.** Oligonucléotide antisens de l'une quelconque des revendications précédentes qui est un oligonucléotide chimère.

**9.** Oligonucléotide antisens de la revendication 8, qui est un gapmère.

**10.** Oligonucléotide antisens de la revendication 8 ou 9 qui est un oligonucléotide chimère ayant une première région comprenant des désoxynucléotides et des deuxième et troisième régions flanquant ladite première région, comprenant au moins un 2'-O-(2-méthoxyéthyl)-nucléotide.

**11.** Oligonucléotide antisens de la revendication 10, dans lequel ladite première région est de dix désoxynucléotides de longueur et lesdites deuxième et troisième régions sont chacune de cinq nucléotides de longueur.

**12.** Composition pharmaceutique comprenant l'oligonucléotide antisens de l'une quelconque des revendications précédentes et un véhicule ou diluant pharmaceutiquement acceptable.

**13.** Oligonucléotide antisens de l'une quelconque des revendications 1 à 11 pour utilisation en thérapie ou prophylaxie.

**14.** Oligonucléotide antisens de la revendication 13 pour utilisation dans le traitement ou la prévention d'états inflammatoires ou maladies inflammatoires anormaux, tels que la polyarthrite rhumatoïde.

**15.** Oligonucléotide antisens de la revendication 13 pour utilisation dans le traitement ou la prévention de cancers, tels que les cancers du sein, de la prostate, du cerveau ou du cancer de la tête et du cou, les mélanomes, les myélomes, les leucémies ou les lymphomes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5719042 A
- US 5844082 A
- US 5731155 A, Schreiber, R.D.
- US 5582999 A
- US 5463023 A
- US 5716622 A, Darnell, J.E.
- WO 0061102 A
- US 5663153 A
- US 3687808 A
- US 4469863 A
- US 4476301 A
- US 5023243 A
- US 5177196 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541306 A
- US 5550111 A
- US 5563253 A
- US 5571799 A
- US 5587361 A
- US 5194599 A
- US 5565555 A
- US 5527899 A
- US 5721218 A
- US 5672697 A
- US 5625050 A
- US 5489677 A
- US 5602240 A
- US 5034506 A
- US 5166315 A
- US 5185444 A
- US 5214134 A
- US 5216141 A
- US 5235033 A
- US 5264562 A
- US 5264564 A
- US 5405938 A
- US 5434257 A
- US 5470967 A
- US 5541307 A
- US 5561225 A
- US 5596086 A
- US 5610289 A
- US 5608046 A
- US 5618704 A
- US 5623070 A
- US 5663312 A
- US 5633360 A
- US 5677437 A
- US 5792608 A
- US 5646269 A
- US 5677439 A
- US 5539082 A
- US 5714331 A
- US 5719262 A
- WO 9839352 A
- WO 9914226 A
- WO 98DK39319980914 A, Wengel
- US 5874553 A
- US 6127346 A
- US 4981957 A
- US 5118800 A
- US 5319080 A
- US 5359044 A
- US 5393878 A
- US 5446137 A
- US 5466786 A
- US 5514785 A
- US 5519134 A
- US 5567811 A
- US 5576427 A
- US 5591722 A
- US 5597909 A
- US 5610300 A
- US 5627053 A
- US 5639873 A
- US 5646265 A
- US 5658873 A
- US 5670633 A
- US 5792747 A
- US 5700920 A
- US 09130973 B
- US 09123108 B
- US 09349040 B
- US 6147200 A
- US 9917895 W

- US 10155920 B
- US 10013295 B
- US 6028183 A
- US 6007992 A
- US 4845205 A
- US 5130302 A
- US 5134066 A
- US 5175273 A
- US 5367066 A
- US 5432272 A
- US 5457187 A
- US 5459255 A
- US 5484908 A
- US 5502177 A
- US 5525711 A
- US 5552540 A
- US 5587469 A
- US 5594121 A
- US 5596091 A
- US 5614617 A
- US 5645985 A
- US 5750692 A
- US 5830653 A
- US 5763588 A
- US 6005096 A
- US 5681941 A
- US 09996292 B
- US 9209196 W
- US 09334130 B
- US 4828979 A
- US 4948882 A
- US 5218105 A
- US 5525465 A
- US 5541313 A
- US 5545730 A
- US 5552538 A
- US 5578717 A
- US 5580731 A
- US 5591584 A
- US 5109124 A
- US 5118802 A
- US 5138045 A
- US 5414077 A
- US 5486603 A
- US 5512439 A
- US 5578718 A
- US 4587044 A
- US 4605735 A
- US 4667025 A
- US 4762779 A
- US 4789737 A
- US 4824941 A
- US 4835263 A
- US 4876335 A
- US 4904582 A
- US 4958013 A
- US 5082830 A
- US 5112963 A
- US 5214136 A
- US 5245022 A
- US 5254469 A
- US 5258506 A
- US 5262536 A
- US 5272250 A
- US 5292873 A
- US 5317098 A
- US 5371241 A
- US 5391723 A
- US 5416203 A
- US 5451463 A
- US 5510475 A
- US 5512667 A
- US 5565552 A
- US 5567810 A
- US 5574142 A
- US 5585481 A
- US 5587371 A
- US 5595726 A
- US 5597696 A
- US 5599923 A
- US 5599928 A
- US 5688941 A
- WO 9726270 A, Wincott
- WO 03004602 A
- US 5013830 A
- US 5149797 A
- US 5220007 A
- US 5256775 A
- US 5366878 A
- US 5403711 A
- US 5491133 A
- US 5565350 A
- US 5623065 A
- US 5652355 A
- US 5652356 A
- US 5700922 A
- US 5760209 A
- US 5614621 A
- US 6051699 A
- US 6020475 A
- US 6326478 B
- US 6169177 B
- US 6121437 A
- US 6465628 B
- US 5108921 A
- US 5354844 A
- US 5416016 A
- US 5459127 A
- US 5521291 A
- US 5543158 A
- US 5547932 A
- US 5583020 A
- US 5591721 A
- US 4426330 A
- US 4534899 A
- US 5013556 A

- US 5213804 A
- US 5227170 A
- US 5264221 A
- US 5356633 A
- US 5395619 A
- US 5417978 A
- US 5462854 A
- US 5469854 A
- US 5512295 A
- US 5527528 A
- US 5534259 A
- US 5543152 A
- US 5556948 A
- US 5580575 A
- US 5595756 A
- WO 9324510 A, Gosselin
- WO 9426764 A
- US 5770713 A, Imbach
- US 5705188 A, Junichi
- WO 9730731 A, Lollo
- US 4837028 A
- WO 8804924 A
- WO 9713499 A, Lim
- EP 0445131 B1
- WO 9004384 A, Fisher
- EP 0496813 B1
- WO 9105545 A
- US 5225212 A
- WO 9420073 A, Zalipsky
- WO 9610391 A, Choi
- US 5540935 A, Miyazaki
- WO 9640062 A, Thierry
- US 5665710 A, Rahman
- WO 9704787 A, Love
- US 4501729 A
- US 5378825 A
- WO 9220823 A
- US 10773678 B
- US 10713139 B
- US 09758881 B
- US 0009054 W
- US 09288461 B

**Non-patent literature cited in the description**


- **DARNELL, JR., J.E. et al.** *Science,* 1994, vol. 264, 1415-1421
- **YANG, C.-H. et al.** *Proc. Natl. Acad. Sci. USA,* 1998, vol. 95, 5568-5572
- **JAIN, N. et al.** *Oncogene,* 1998, vol. 17, 3157-3167
- **ZHONG, Z. et al.** *Proc. Natl. Acad. Sci . USA,* 1994, vol. 91, 4806-4810
- **FUKADA, T. et al.** *Immunity,* 1996, vol. 5, 449-460
- **YU, C.-L. et al.** *Science,* 1995, vol. 269, 81-83
- **TURKSON, J. et al.** *Mol. Cell. Biol.,* 1998, vol. 18, 2545-2552
- **MIGONE, T.-S. et al.** *Science,* 1995, vol. 269, 79-83
- **NIU et al.** *Cancer Res.,* 1999, vol. 59, 5059-5063
- **SARTOR, C.I. et al.** *Cancer Res.,* 1997, vol. 57, 978-987
- **GARCIA, R. et al.** *Cell Growth and Differentiation,* 1997, vol. 8, 1267-1276
- **CATTANEO, E. et al.** *Anticancer Res.,* 1998, vol. 18, 2381-2387
- **NI et al.** *Cancer Res.,* 2000, vol. 60, 1225-1228
- **GOUILLEUX-GRUART, V. et al.** *Leuk. Lymphoma,* 1997, vol. 28, 83-88
- **YU, C.-L. et al.** *J. Immunol.,* 1997, vol. 159, 5206-5210
- **FRANK, D. A. et al.** *J. Clin. Invest.,* 1997, vol. 100, 3140-3148
- **SONG et al.** *Oncogene,* 2003, vol. 22, 4150
- **MORA et al.** *Cancer Res.,* 2002, vol. 62, 6659
- **CATLETT-FALCONE, R. et al.** *Immunity,* 1999, vol. 10, 105-115
- **SENGUPTA, T.K. et al.** *J. Exp. Med.,* 1995, vol. 181, 1015-1025
- **WANG, F. et al.** *J. Exp. Med.,* 1995, vol. 182, 1825-1831
- **CALDENHOVEN, E. et al.** *J. Biol. Chem.,* 1996, vol. 271, 13221-13227
- **GRANDIS, J. R. et al.** *J. Clin. Invest.,* 1998, vol. 102, 1385-1392
- **GUO ; KEMPHEUS.** *Cell,* 1995, vol. 81, 611-620
- **MONTGOMERY et al.** *Proc. Natl. Acad. Sci. USA,* 1998, vol. 95, 15502-15507
- **FIRE et al.** *Nature,* 1998, vol. 391, 806-811
- **TIJSTERMAN et al.** *Science,* 2002, vol. 295, 694-697
- **NIELSEN et al.** *Science,* 1991, vol. 254, 1497-1500
- **DWAINE A. BRAASCH ; DAVID R. COREY.** *Biochemistry,* 2002, vol. 41 (14), 4503-4510
- **WANG et al.** *J. Am. Chem. Soc.,* 2000, vol. 122, 8595-8602
- **WOUTERS ; HERDEWIJN.** *Bioorg. Med. Chem. Lett.,* 1999, vol. 9, 1563-1566
- **SINGH et al.** *Chem. Commun.,* 1998, vol. 4, 455-456
- **PETERSEN et al.** *J. Mol. Recognit.,* 2000, vol. 13, 44-53
- **WENGEL et al.** *Nucleosides Nucleotides,* 1999, vol. 18, 1365-1370

- **KOSHKIN et al.** *J. Am. Chem. Soc.,* 1998, vol. 120, 13252-13253
- **WAHLESTEDT et al.** *Proc. Natl. Acad. Sci. U. S. A.,* 2000, vol. 97, 5633-5638
- **KOSHKIN et al.** *Tetrahedron,* 1998, vol. 54, 3607-3630
- **KUMAR et al.** *Bioorg. Med. Chem. Lett.,* 1998, vol. 8, 2219-2222
- **SINGH et al.** *J. Org. Chem.,* 1998, vol. 63, 10035-10039
- **STEFFENS et al.** *Helv. Chim. Acta,* 1997, vol. 80, 2426-2439
- **STEFFENS et al.** *J. Am. Chem. Soc.,* 1999, vol. 121, 3249-3255
- **RENNEBERG et al.** *J. Am. Chem. Soc.,* 2002, vol. 124, 5993-6002
- **MARTIN et al.** *Helv. Chim. Acta,* 1995, vol. 78, 486-504
- The Concise Encyclopedia Of Polymer Science And Engineering. John Wiley & Sons, 1990, 858-859
- **ENGLISCH et al.** *Angewandte Chemie, International Edition,* 1991, vol. 30, 613
- **SANGHVI, Y.S.** Antisense Research and Applications. CRC Press, 1993, 289-302
- Antisense Research and Applications. CRC Press, 1993, 276-278
- **KURCHAVOV et al.** *Nucleosides and Nucleotides,* 1997, vol. 16, 1837-1846
- **LIN, K.-Y. ; JONES, R. J. ; MATTEUCCI, M.** *J. Am. Chem. Soc.,* 1995, vol. 117, 3873-3874
- **WANG, J. ; LIN, K.-Y. ; MATTEUCCI, M.** *Tetrahedron Lett.,* 1998, vol. 39, 8385-8388
- **LIN, K.-Y. ; MATTEUCCI, M.** *J. Am. Chem. Soc.,* 1998, vol. 120, 8531-8532
- **LIN, K-Y ; MATTEUCCI, M.** *J. Am. Chem. Soc.,* 1998, vol. 120, 8531-8532
- **FLANAGAN, W. M. ; WOLF, J.J. ; OLSON, P. ; GRANT, D. ; LIN, K.-Y. ; WAGNER, R. W. ; MATTEUCCI, M.** *Proc. Natl. Acad. Sci. USA,* 1999, vol. 96, 3513-3518
- **LETSINGER et al.** *Proc. Natl. Acad. Sci. USA,* 1989, vol. 86, 6553-6556
- **MANOHARAN et al.** *Bioorg. Med. Chem. Let.,* 1994, vol. 4, 1053-1060
- **MANOHARAN et al.** *Ann. N.Y. Acad. Sci.,* 1992, vol. 660, 306-309
- **MANOHARAN et al.** *Bioorg. Med. Chem. Let.,* 1993, vol. 3, 2765-2770
- **OBERHAUSER et al.** *Nucl. Acids Res.,* 1992, vol. 20, 533-538
- **SAISON-BEHMOARAS et al.** *EMBO J.,* 1991, vol. 10, 1111-1118
- **KABANOV et al.** *FEBS Lett.,* 1990, vol. 259, 327-330
- **SVINARCHUK et al.** *Biochimie,* 1993, vol. 75, 49-54
- **MANOHARAN et al.** *Tetrahedron Lett.,* 1995, vol. 36, 3651-3654
- **SHEA et al.** *Nucl. Acids Res.,* 1990, vol. 18, 3777-3783
- **MANOHARAN et al.** *Nucleosides & Nucleotides,* 1995, vol. 14, 969-973
- **MISHRA et al.** *Biochim. Biophys. Acta,* 1995, vol. 1264, 229-237
- **CROOKE et al.** *J. Pharmacol. Exp. Ther.,* 1996, vol. 277, 923-937
- **BEAUCAGE ; TYER.** *Tetrahedron,* 1925, vol. 49, 1925
- **WOLFGANG SANGER.** Principles of Nucleic Acid Structure. Springer-Verlag, 1984
- **GALLO et al.** *Tetrahedron,* 2001, vol. 57, 5707-5713
- **HARRY-O'KURU et al.** *J. Org. Chem.,* 1997, vol. 62 (6), 1754-1759
- **TANG et al.** *J. Org. Chem.,* 1999, vol. 64, 747-754
- **KAWASAKI et al.** *J. Med. Chem.,* 1993, vol. 36, 831-841
- **GUILLERM et al.** *Bioorganic and Medicinal Chemistry Letters,* 1995, vol. 5, 1455-1460
- **OWEN et al.** *J. Org. Chem.,* 1976, vol. 41, 3010-3017
- **JACOBSON et al.** *J. Med. Chem. Lett.,* 2000, vol. 43, 2196-2203
- **LEE et al.** *Bioorganic and Medicinal Chemistry Letters,* 2001, vol. 11, 1333-1337
- **MORITA et al.** *Bioorganic & Medicinal Chemistry Letters,* 2002, vol. 12, 73-76
- Chemistry of Nucleosides and Nucleotides. Plenum press, 1988, vol. 1-3
- **ARNOTT ; HUKINS.** *Biochem. Biophys. Res. Comm.,* 1970, vol. 47, 1504
- **SANGER et al.** Principles of Nucleic Acid Structure. Springer-Verlag, 1984
- **LESNIK et al.** *Biochemistry,* 1995, vol. 34, 10807-10815
- **CONTE et al.** *Nucleic Acids Res.,* 1997, vol. 25, 2627-2634
- **SEARLE et al.** *Nucleic Acids Res.,* 1993, vol. 21, 2051-2056
- **EGLI et al.** *Biochemistry,* 1996, vol. 35, 8489-8494
- **SANGER, W.** Principles of Nucleic Acid Structure. Springer-Verlag, 1984
- **BERGER.** *Nucleic Acids Research,* 1998, vol. 26, 2473-2480
- **LANE et al.** *Eur. J. Biochem.,* 1993, vol. 215, 297-306
- **FEDOROFF et al.** *J. Mol. Biol.,* 1993, vol. 233, 509-523
- **GONZALEZ et al.** *Biochemistry,* 1995, vol. 34, 4969-4982
- **HORTON et al.** *J. Mol. Biol.,* 1996, vol. 264, 521-533

- **BAKER et al.** *J. Biol. Chem.,* 1997, vol. 272, 11944-12000
- **MARTIN, P.** *Helv. Chim. Acta,* 1995, vol. 78, 486-504
- **ALTMANN et al.** *Chimia,* 1996, vol. 50, 168-176
- **ALTMANN et al.** *Biochem. Soc. Trans.,* 1996, vol. 24, 630-637
- **ALTMANN et al.** *Nucleosides Nucleotides,* 1997, vol. 16, 917-926
- **BERGE et al.** Pharmaceutical Salts. *J. of Pharma Sci.,* 1977, vol. 66, 1-19
- **VAN BERGE-HENEGOUWEN et al.** *Gastroenterol.,* 1977, vol. 73, 300
- **DISANTO.** Remington's Pharmaceutical Sciences. Mack Publishing Co, 1990, 1451-1458
- **MURANISHI.** Crit. Rev. Ther. Drug Carrier Systems. 1990, vol. 7, 1
- **LEE et al.** *Crit. Rev. Ther. Drug Carrier Systems,* 1991, vol. 8, 91
- **HARVEY.** Remington's Pharmaceutical Sciences. Mack Publishing Co, 1990, 711
- **HIRAHATA et al.** *Gan To Kagaku Ryoho,* 1992, vol. 19 (10), 1591
- **SOMOGYI et al.** *Pharm. Res.,* 1995, vol. 12, 149
- **AKAMO et al.** *Japanese J. Cancer Res.,* 1994, vol. 85, 652
- **INOUE et al.** *Artif. Organs,* 1997, vol. 21, 28
- **PENNINGTON et al.** *Ailment Pharmacol. Ther.,* 1995, vol. 9, 471
- **BENET et al.** Goodman & Gilman's The Pharmacological Basis of Therapeutics. McGraw-Hill, 1996
- **LEE et al.** *Critical Reviews in Therapeutic Drug Carrier Systems,* 1991, 92
- **TAKAHASHI et al.** *J. Pharm. Phamacol.,* 1988, vol. 40, 252
- **MURANISHI.** *Critical Reviews in Therapeutic Drug Carrier Systems,* 1990, vol. 7, 1
- **EL-HARIRI et al.** *J. Pharm. Pharmacol.,* 1992, vol. 44, 651
- **BRUNTON et al.** Goodman & Gilman's The Pharmacological Basis of Therapeutics. McGraw-Hill, 1996, 934-935
- **SWINYARD.** Remington's Pharmaceutical Sciences. Mack Publishing Co, 1990, 782-783
- **YAMAMOTO et al.** *J. Pharm. Exp. Ther.,* 1992, vol. 263, 25
- **YAMASHITA et al.** *J. Pharm. Sci.,* 1990, vol. 79, 579
- **JARRETT.** *J. Chromatogr.,* 1993, vol. 618, 315
- **BUUR et al.** *J. Control Rel.,* 1990, vol. 14, 43
- **YAMASHITA et al.** *J. Pharm. Pharmacol.,* 1987, vol. 39, 621
- **IDSON.** Pharmaceutical Dosage Forms. Marcel Dekker, Inc, 1988, vol. 1, 199
- **ROSOFF.** Pharmaceutical Dosage Forms. Marcel Dekker, Inc, 1988, vol. 1, 245
- **BLOCK.** Pharmaceutical Dosage Forms. Marcel Dekker, Inc, 1988, vol. 2, 335
- **HIGUCHI et al.** Remington's Pharmaceutical Sciences. Mack Publishing Co, 1985, 301
- **RIEGER.** Pharmaceutical Dosage Forms. Marcel Dekker, Inc, 1988, vol. 1, 285
- **BLOCK.** Pharmaceutical Dosage Forms. Marcel Dekker, Inc, 1988, vol. 1, 335
- **LEUNG ; SHAH.** Controlled Release of Drugs: Polymers and Aggregate Systems. VCH Publishers, 1989, 185-215
- **SCHOTT.** Remington's Pharmaceutical Sciences. Mack Publishing Co, 1985, 271
- **CONSTANTINIDES et al.** *Pharmaceutical Research,* 1994, vol. 11, 1385-1390
- **RITSCHEL.** *Meth. Find. Exp. Clin. Pharmacol.,* 1993, vol. 13, 205
- **CONSTANTINIDES et al.** *Pharmaceutical Research,* 1994, vol. 11, 1385
- **HO et al.** *J. Pharm. Sci.,* 1996, vol. 85, 138-143
- **WANG et al.** *Biochem. Biophys. Res. Commun.,* 1987, vol. 147, 980-985
- **ZHOU et al.** *Journal of Controlled Release,* 1992, vol. 19, 269-274
- **WEINER et al.** *Journal of Drug Targeting,* 1992, vol. 2, 405-410
- **DU PLESSIS et al.** *Antiviral Research,* 1992, 259-265
- **HU et al.** *S.T.P.Pharma. Sci.,* 1994, vol. 4 (6), 466
- **ALLEN et al.** *FEBS Letters,* 1987, vol. 223, 42
- **WU et al.** *Cancer Research,* 1993, vol. 53, 3765
- **PAPAHADJOPOULOS et al.** *Ann. N.Y. Acad. Sci.,* 1987, vol. 507, 64
- **GABIZON et al.** *Proc. Natl. Acad. Sci . U. S.A.,* 1988, vol. 85, 6949
- **SUNAMOTO et al.** *Bull. Chem. Soc. Jpn.,* 1980, vol. 53, 2778
- **ILLUM et al.** *FEBS Lett.,* 1984, vol. 167, 79
- **KLIBANOV et al.** *FEBS Lett.,* 1990, vol. 268, 235
- **BLUME et al.** *Biochimica et Biophysica Acta,* 1990, vol. 1029, 91
- **RIEGER.** Pharmaceutical Dosage Forms. Marcel Decker, Inc, 1988, 285
- **RIEGER.** Pharmaceutical Dosage Forms. Marcel Dekker, Inc, 1988, 285
- The Merck Manual of Diagnosis and Therapy. 1987, 1206-1228
- The Merck Manual of Diagnosis and Therapy. 1987, 2499-250646-49

- **MARTIN, P.** *Helv. Chim. Acta,* 1995, vol. 78, 486-506
- **SANGHVI et al.** *Nucl. Acids Res.,* 1993, vol. 21, 3197-3203
- **DE MESMAEKER et al.** *Acc. Chem. Res.,* 1995, vol. 28, 366-374
- **P.E. NIELSEN et al.** *Science,* 1991, vol. 254, 1497-1500
- **CHIANG et al.** *J. Biol. Chem.,* 1991, vol. 266, 18162
- **AKIRA, S. et al.** *Cell,* 1994, vol. 77, 63-71
- **CHIANG, M-Y. et al.** *J. Biol. Chem,* 1991, vol. 266, 18162-18171
- **KARRAS, J.G. et al.** *J. Exp. Med.,* 1997, vol. 185, 1035-1042
- **KARRAS, J.G. et al.** *J. Immunol.,* 1996, vol. 157, 2299
- **FRANCIS, D.A. et al.** *Int. Immunol.,* 1995, vol. 7, 151-161
- **FARIS, M. et al.** *Immunology,* 1997, vol. 90, 350-357
- **KRIEG, A.M. et al.** *Nature,* 1995, vol. 374, 546-549
- **LAMY et al.** *Blood,* 1998, vol. 92, 4771-7
- **SCARINGE, S. A.** *Ph.D. Thesis,* 1996
- **SCARINGE, S. A. et al.** *J. Am. Chem. Soc.,* 1998, vol. 120, 11820-11821
- **MATTEUCCI, M. D. ; CARUTHERS, M. H.** *J. Am. Chem. Soc.,* 1981, vol. 103, 3185-3191
- **BEAUCAGE, S. L. ; CARUTHERS, M. H.** *Tetrahedron Lett.,* 1981, vol. 22, 1859-1862
- **DAHL, B. J. et al.** *Acta Chem. Scand,* 1990, vol. 44, 639-641
- **REDDY, M. P. et al.** *Tetrahedrom Lett.,* 1994, vol. 25, 4311-4314
- **WINCOTT, F. et al.** *Nucleic Acids Res.,* 1995, vol. 23, 2677-2684
- **GRIFFIN, B. E. et al.** *Tetrahedron,* 1967, vol. 23, 2301-2313
- **GRIFFIN, B. E. et al.** *Tetrahedron,* 1967, vol. 23, 2315-2331
- **KIYAMA et al.** *Cancer Res.,* 2003, vol. 63, 3575-3584
- **GLEAVE et al.** *Cancer Res.,* 1992, vol. 51, 1598-1605
- **CHIARLE et al.** *Blood,* 2003, vol. 101, 1919-1927
- **BROMBERG, J.F. et al.** *Cell,* 1999, vol. 98, 295-303
- **SONG et al.** *Oncogene,* 2003, vol. 22, 4150-4165
- **YU et al.** *Science,* 1995, vol. 269, 81-83
- **GARCIA et al.** *Cell Growth Differ.,* 1997, vol. 8, 1267-1276
- **MORA et al.** *Cancer Research,* 2002, vol. 62, 6659-6666
- **RAZ et al.** *PNAS,* 1999, vol. 96, 2846-2851
- **RICKERT et al.** *Nuc. Acids Res.,* 1997, vol. 25, 1317